# EUROPEAN PATENT SPECIFICATION

(11) **EP 3 898 629 B1**
(45) Date of publication and mention of the grant of the patent: **18.01.2023**
(21) Application number: 19827682.6
(22) Date of filing: 16.12.2019
(51) Int. Cl.: C07D 487/04, A61K 31/4985, A61P 31/04

(54) **IMIDAZOPYRAZINE DERIVATIVES AS ANTIBACTERIALS**
IMIDAZOPYRAZINDERIVATE ALS ANTIBAKTERIELLE SUBSTANZEN
DÉRIVÉS D'IMIDAZOPYRAZINE EN TANT QU'ANTIBACTÉRIENS

(30) Priority: 17.12.2018 WO PCT/CN2018/121476; 07.11.2019 WO PCT/CN2019/116346
(43) Date of publication of application: 27.10.2021
(73) Proprietor: F. Hoffmann-La Roche AG, 4070 Basel (CH)
(72) Inventor: BLANC, Jean-Baptiste, 4070 Basel (CH); KHAN, Nawaz, Cambridge CB1 3LQ (GB); KRAMER, Christian, 4070 Basel (CH); LERNER, Christian, 4070 Basel (CH); PFLIEGER, Philippe, 4070 Basel (CH); STOLL, Theodor, 4070 Basel (CH); YANG, Song, Shanghai, 201203 (CN)
(74) Representative: Neuhaus, Christian Michel
(86) International application number: PCT/EP2019/085386
(87) International publication number: WO 2020/127075

(56) References cited:
- WO-A1-2012/168733
- WO-A1-2020/126952
- WO-A1-2020/126953
- WO-A1-2020/126954
- WO-A1-2020/126956
- NERMIN S. ABDOU ET AL: "Synthesis and in vitro antiproliferative activity of novel pyrazolo[3,4-d]pyrimidine derivatives", MEDCHEMCOMM, vol. 6, no. 8, 2015, pages 1518-1534, XP055671437, United Kingdom ISSN: 2040-2503, DOI: 10.1039/C5MD00127G

## Description

### Field of the Invention

The present invention relates to novel imidazopyrazine derivatives which exhibit antibacterial properties. The invention also relates to their use in the treatment or prevention of bacterial infections and resulting diseases, in particular for the treatment or prevention of infections with *Acinetobacter baumannii* and resulting diseases.

### Background of the Invention

*Acinetobacter baumannii* is a Gram-negative, aerobic, nonfermenting bacterium recognized over the last decades as an emergining pathogen with very limited treatment options.

*A. baumannii* is considered to be a serious threat by the US Centers for Disease Control and Prevention and belongs to the so called 'ESKAPE' pathogens (*Enterococcus faecium, Staphylococcus aureus, Klebsiella pneumoniae, Acinetobacter baumannii, Pseudomonas aeruginosa* and *Enterobacter species* & *E. coli)* that currently cause the majority of nosocomial infections and effectively "escape" the activity of antimicrobial agents.

*A. baumannii* is most often encountered in intensive care units and surgical wards, where extensive antibiotic use has enabled selection for resistance against all known antimicrobials and where it causes infections that include bacteremia, pneumonia, meningitis, urinary tract infection, and wound infection.

*A. baumannii* has an exceptional ability to upregulate and acquire resistance determinants and shows an environmental persistance that allows its survival and spread in the nosocomial setting, making this organism a frequent cause of outbreaks of infection and an endemic, health care-associated pathogen.

Due to increasing antibiotic resistance to most if not all available therapeutic options, Muti-Drug Resistant (MDR) *A. baumanniii* infections, especially those caused by Carbapenem resistant *A*. *baumannii,* are extremely difficult or even impossible to treat with high mortality rate as well as increased morbidity and length of stay in intensive care unit.

*Acinetobacter baumannii* has been defined and still remains "a prime example of a mismatch between unmet medical needs and the current antimicrobial research and development pipeline" according to the Antimicrobial Availability Task Force (AATF) of the Infectious Diseases Society of America (IDSA). Thus, there is a high demand and need to identify compounds suitable for the treatment of diseases and infections caused by *Acinetobacter baumannii.*

The present invention provides novel compounds which exhibit activity against drug-susceptible as well as drug-resistant strains of *Acinetobacter baumannii.*

### Summary of the Invention

In a first aspect, the present invention provides a compound of formula (I) 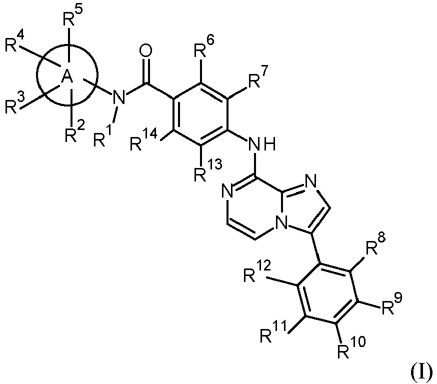 or a pharmaceutically acceptable salt thereof, wherein A and R¹ to R¹⁴ are as described herein.

In one aspect, the present invention provides a process of manufacturing the compounds of formula (I) described herein, comprising:
(i) reacting a carboxylic acid IVa, wherein R⁶ to R¹⁴ are as defined herein, 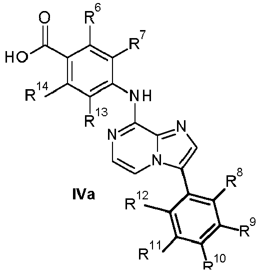 with an amine V, wherein A, L and R¹ to R⁵ are as defined herein, 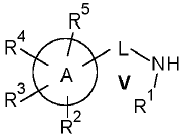 in the presence of a coupling reagent (such as HATU, TBTU, and the like) and a base (such as DIPEA, NEt₃, and the like), to form said compound of formula (I); or
(ii) reacting a compound VI, wherein R¹ to R⁷, R¹³, R¹⁴, L and A are as defined herein and X is halogen, 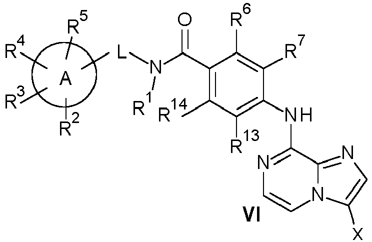 with a boronic acid VII, wherein R⁸ to R¹² are as defined herein and Y is a boronic acid or a boronic acid ester, 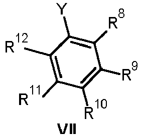 in the presence of a transition metal catalyst (such as PdCl₂(dppf)-CH₂Cl₂ adduct, Pd(PPh₃)₄, and the like) and a base (such as K₃PO₄, NaOtBu, and the like) to form said compound of formula (I).

In a further aspect, the present invention provides a compound of formula (I) as described herein, or a pharmaceutically acceptable salt thereof, for use as therapeutically active substance.

In a further aspect, the present invention provides a pharmaceutical composition comprising a compound of formula (I) as described herein, or a pharmaceutically acceptable salt thereof, and a therapeutically inert carrier.

In a further aspect, the present invention provides a compound of formula (I) as described herein, or a pharmaceutically acceptable salt thereof, for use as antibiotic.

In a further aspect, the present invention provides a compound of formula (I) as described herein, or a pharmaceutically acceptable salt thereof, for use in the treatment or prevention of nosocomial infections and resulting diseases.

In a further aspect, the present invention provides a compound of formula (I) as described herein, or a pharmaceutically acceptable salt thereof, for use in the treatment or prevention of infections and resulting diseases caused by Gram-negative bacteria.

In a further aspect, the present invention provides a compound of formula (I) as described herein, or a pharmaceutically acceptable salt thereof, for use in the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or E. *coli,* or a combination therof.

In a further aspect, the present invention provides the use of a compound of formula (I) as described herein, or a pharmaceutically acceptable salt thereof, for the preparation of medicaments useful for the treatment or prevention of infections and resulting diseases caused by *Enterococcus faecium, Staphylococcus aureus, Klebsiella pneumoniae, Acinetobacter baumannii, Pseudomonas aeruginosa, Enterobacter species* or *E. coli,* or a combination therof.

### Detailed Description of the Invention

### Definitions

Features, integers, characteristics, compounds, chemical moieties or groups described in conjunction with a particular aspect, embodiment or example of the invention are to be understood to be applicable to any other aspect, embodiment or example described herein, unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The term "alkyl" refers to a mono- or multivalent, e.g., a mono- or bivalent, linear or branched saturated hydrocarbon group of 1 to 6 carbon atoms ("C₁-C₆-alkyl"), e.g., 1, 2, 3, 4, 5, or 6 carbon atoms. In some embodiments, the alkyl group contains 1 to 3 carbon atoms, e.g., 1, 2 or 3 carbon atoms. Some non-limiting examples of alkyl include methyl, ethyl, propyl, 2-propyl (isopropyl), n-butyl, iso-butyl, sec-butyl, tert-butyl, and 2,2-dimethylpropyl. A particularly preferred, yet non-limiting example of alkyl is methyl.

The term "alkenyl" denotes a monovalent linear or branched hydrocarbon group of 2 to 6 carbon atoms with at least one double bond ("C₂-C₆-alkenyl"). In particular embodiments, alkenyl has 2 to 4 carbon atoms with at least one double bond. Examples of alkenyl include ethenyl, propenyl, prop-2-enyl, isopropenyl, n-butenyl and iso-butenyl. Particular alkenyl group is ethenyl.

The term "alkynyl" denotes a monovalent linear or branched hydrocarbon group of 2 to 6 carbon atoms with at least one triple bond ("C₂-C₆-alkynyl"). In particular embodiments, alkynyl has 2 to 4 carbon atoms with at least one triple bond. Examples of alkynyl include ethynyl, propynyl, n-butynyl or isobutynyl. Preferred alkenyl is propynyl.

The term "alkoxy" refers to an alkyl group, as previously defined, attached to the parent molecular moiety via an oxygen atom. Unless otherwise specified, the alkoxy group contains 1 to 6 carbon atoms ("C₁-C₆-alkoxy"). In some preferred embodiments, the alkoxy group contains contains 1 to 4 carbon atoms. In still other embodiments, the alkoxy group contains 1 to 3 carbon atoms. Some non-limiting examples of alkoxy groups include methoxy, ethoxy, n-propoxy, isopropoxy, n-butoxy, isobutoxy and tert-butoxy. A particularly preferred, yet non-limiting example of alkoxy is methoxy.

The term "alkynyloxy" refers to an alkynyl group, as previously defined, attached to the parent molecular moiety via an oxygen atom.

The term "halogen" or "halo" refers to fluoro (F), chloro (Cl), bromo (Br), or iodo (I). Preferably, the term "halogen" or "halo" refers to fluoro (F), chloro (Cl) or bromo (Br). Particularly preferred, yet non-limiting examples of "halogen" or "halo" are fluoro (F) and chloro (Cl).

The term "cycloalkyl" as used herein refers to a saturated or partly unsaturated monocyclic or bicyclic hydrocarbon group of 3 to 12 ring carbon atoms ("C₃-C₁₂-cycloalkyl"). In some preferred embodiments, the cycloalkyl group is a saturated monocyclic hydrocarbon group of 3 to 10 ring carbon atoms, in particular 3 to 8 ring carbon atoms. "Bicyclic cycloalkyl" refers to cycloalkyl moieties consisting of two saturated carbocycles having two carbon atoms in common, i.e., the bridge separating the two rings is either a single bond or a chain of one or two ring atoms, and to spirocyclic moieties, i.e., the two rings are connected via one common ring atom. Preferably, the cycloalkyl group is a saturated monocyclic hydrocarbon group of 3 to 6 ring carbon atoms, e.g., of 3, 4, 5 or 6 carbon atoms. Some non-limiting examples of cycloalkyl include cyclopropyl, cyclobutyl, cyclopentyl, cyclohexyl and cycloheptyl.

The term "cycloalkyloxy" refers to a group cycloalkyl-O-, i.e. a cycloalkyl group substituted with an oxy group and attached to the parent molecular moiety via said oxy group.

The term "cyanocycloalkyloxy" refers to a cycloalkyloxy group, wherein at least one of the hydrogen atoms of the cycloalkyloxy group has been replaced by a cyano group. Preferably, "cyanocycloalkyloxy" refers to a cycloalkyloxy group wherein 1, 2 or 3 hydrogen atoms of the cycloalkyloxy group have been replaced by a cyano group.

The term "aminoalkynyloxy" refers to an alkynyloxy group, wherein at least one of the hydrogen atoms of the alkynyloxy group has been replaced by an amino group. Preferably, "aminoalkynyloxy" refers to an alkynyloxy group wherein 1, 2 or 3 hydrogen atoms of the alkynyloxy group have been replaced by an amino group. A preferred, yet non-limiting example of aminoalkynyloxy is 3-aminoprop-1-ynyl.

The term "aminoalkoxy" refers to an alkoxy group, wherein at least one of the hydrogen atoms of the alkoxy group has been replaced by an amino group. Preferably, "aminoalkoxy" refers to an alkoxy group wherein 1, 2 or 3 hydrogen atoms of the alkoxy group have been replaced by an amino group. A preferred, yet non-limiting example of aminoalkoxy is aminomethoxy.

The term "aminoalkyl" refers to an alkyl group, wherein at least one of the hydrogen atoms of the alkyl group has been replaced by an amino group. Preferably, "aminoalkyl" refers to an alkyl group wherein 1, 2 or 3 hydrogen atoms of the alkyl group have been replaced by an amino group. A preferred, yet non-limiting example of aminoalkyl is aminomethyl.

The term "carboxyalkyl" refers to an alkyl group, wherein at least one of the hydrogen atoms of the alkyl group has been replaced by a carboxy group. Preferably, "carboxyalkyl" refers to an alkyl group wherein 1, 2 or 3 hydrogen atoms of the alkyl group have been replaced by a carboxy group. A preferred, yet non-limiting example of aminoalkyl is carboxymethyl.

The term "aminoalkoxyalkynyloxy" refers to an alkynyloxy group, wherein at least one of the hydrogen atoms of the alkynyloxy group has been replaced by an aminoalkoxy group. Preferably, "aminoalkoxyalkynyloxy" refers to an alkynyloxy group wherein 1, 2 or 3 hydrogen atoms of the alkynyloxy group have been replaced by an aminoalkoxy group.

The term "hydroxyalkynyloxy" refers to an alkynyloxy group, wherein at least one of the hydrogen atoms of the alkynyloxy group has been replaced by a hydroxy group. Preferably, "hydroxyalkynyloxy" refers to an alkynyloxy group wherein 1, 2 or 3 hydrogen atoms of the alkynyloxy group have been replaced by a hydroxy group. A preferred, yet non-limiting example of hydroxyalkynyloxy is 3-hydroxyprop-1-ynyl.

The terms "heterocycloalkyl" and "heterocyclyl" are used interchangeably and refer to a saturated or partly unsaturated mono- or bicyclic, preferably monocyclic ring system of 3 to 10 ring atoms, preferably 3 to 8 ring atoms, wherein 1, 2, or 3 of said ring atoms are heteroatoms selected from N, O and S, the remaining ring atoms being carbon. Preferably, 1 to 2 of said ring atoms are selected from N and O, the remaining ring atoms being carbon. "Bicyclic heterocyclyl" refers to heterocyclic moieties consisting of two cycles having two ring atoms in common, i.e., the bridge separating the two rings is either a single bond or a chain of one or two ring atoms, and to spirocyclic moieties, i.e., the two rings are connected via one common ring atom. Some non-limiting examples of monocyclic heterocyclyl groups include azetidin-3-yl, azetidin-2-yl, oxetan-3-yl, oxetan-2-yl, 2-oxopyrrolidin-1-yl, 2-oxopyrrolidin-3-yl, 5-oxopyrrolidin-2-yl, 5-oxopyrrolidin-3-yl, 2-oxo-1-piperidyl, 2-oxo-3-piperidyl, 2-oxo-4-piperidyl, 6-oxo-2-piperidyl, 6-oxo-3-piperidyl, 1-piperidinyl, 2-piperidinyl, 3-piperidinyl, 4-piperidinyl, morpholino, morpholin-2-yl and morpholin-3-yl.

The term "heterocyclylalkyl" refers to an alkyl group, wherein at least one of the hydrogen atoms of the alkyl group has been replaced by a heterocyclyl group. Preferably, "heterocyclylalkyl" refers to an alkyl group wherein 1, 2 or 3 hydrogen atoms, most preferably 1 hydrogen atom of the alkyl group have been replaced by a heterocyclyl group.

The term "aryl" refers to a monocyclic, bicyclic, or tricyclic carbocyclic ring system having a total of 6 to 14 ring members ("C₆-C₁₄-aryl"), preferably, 6 to 12 ring members, and more preferably 6 to 10 ring members, and wherein at least one ring in the system is aromatic. Some non-limiting examples of aryl include phenyl and 9H-fluorenyl (e.g. 9H-fluoren-9-yl). A particularly preferred, yet non-limiting example of aryl is phenyl.

The term "heteroaryl" refers to a mono- or multivalent, monocyclic or bicyclic, preferably bicyclic ring system having a total of 5 to 14 ring members, preferably, 5 to 12 ring members, and more preferably 5 to 10 ring members, wherein at least one ring in the system is aromatic, and at least one ring in the system contains one or more heteroatoms. Preferably, "heteroaryl" refers to a 5-10 membered heteroaryl comprising 1, 2, 3 or 4 heteroatoms independently selected from O, S and N. Most preferably, "heteroaryl" refers to a 5-10 membered heteroaryl comprising 1 to 2 heteroatoms independently selected from O and N. Some non-limiting examples of heteroaryl include 2-pyridyl, 3-pyridyl, 4-pyridyl, indol-1-yl, 1H-indol-2-yl, 1H-indol-3-yl, 1H-indol-4-yl, 1H-indol-5-yl, 1H-indol-6-yl, 1H-indol-7-yl, 1,2-benzoxazol-3-yl, 1,2-benzoxazol-4-yl, 1,2-benzoxazol-5-yl, 1,2-benzoxazol-6-yl, 1,2-benzoxazol-7-yl, 1H-indazol-3-yl, 1H-indazol-4-yl, 1H-indazol-5-yl, 1H-indazol-6-yl, 1H-indazol-7-yl, pyrazol-1-yl, 1H-pyrazol-3-yl, 1H-pyrazol-4-yl, 1H-pyrazol-5-yl, imidazol-1-yl, 1H-imidazol-2-yl, 1H-imidazol-4-yl, 1H-imidazol-5-yl, oxazol-2-yl, oxazol-4-yl and oxazol-5-yl. A particularly preferred, yet non-limiting example of heteroaryl is indolyl, in particular 1H-indol-3-yl.

The term "alkylheteroaryl" refers to a heteroaryl group, wherein at least one of the hydrogen atoms of the heteroaryl group has been replaced by an alkyl group. Preferably, "alkylheteroaryl" refers to a heteroaryl group wherein 1, 2 or 3 hydrogen atoms, most preferably 1 hydrogen atom of the heteroaryl group have been replaced by an alkyl group.

The term "heteroaryloxy" refers to a heteroaryl group attached to the parent molecular moiety via an oxygen atom.

The term "hydroxy" refers to an -OH group.

The term "amino" refers to an -NH₂ group.

The term "cyano" refers to a -CN (nitrile) group.

The term "sulfamoyl" refers to a -SO₂-NH₂ group.

The term "alkylsulfamoyl" refers to a -SO₂-NH(alkyl) group.

The term "dialkylsulfamoyl" refers to a -SO₂-N(alkyl)₂ group.

The term "alkylsulfonyl" refers to a -SO₂-alkyl group.

The term "alkylsulfonyloxy" refers to a -O-SO₂-alkyl group.

The term "alkylsulfanyl" refers to a -S-alkyl group.

The term "carboxy" refers to a -COOH group.

The term "carbamimidoyl" refers to a 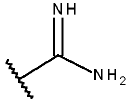 group.

The term "guanidino" refers to a 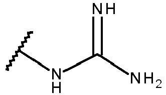 group.

The term "ureido" refers to a 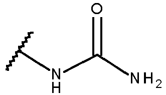 group.

The term "carbamoyl" refers to a -C(O)NH₂ group.

The term "carbonyl" refers to a -C(O)- group.

The term "alkoxycarbonyl" refers to a -C(O)-O-alkyl group (i.e., an alkyl ester).

The term "haloalkyl" refers to an alkyl group, wherein at least one of the hydrogen atoms of the alkyl group has been replaced by a halogen atom, preferably fluoro. Preferably, "haloalkyl" refers to an alkyl group wherein 1, 2 or 3 hydrogen atoms of the alkyl group have been replaced by a halogen atom, most preferably fluoro. Particularly preferred, yet non-limiting examples of haloalkyl are trifluoromethyl and trifluoroethyl.

The term "haloalkoxy" refers to an alkoxy group, wherein at least one of the hydrogen atoms of the alkoxy group has been replaced by a halogen atom, preferably fluoro. Preferably, "haloalkoxy" refers to an alkoxy group wherein 1, 2 or 3 hydrogen atoms of the alkoxy group have been replaced by a halogen atom, most preferably fluoro. A particularly preferred, yet non-limiting example of haloalkoxy is trifluoromethoxy (-OCF₃).

The term "cyanoalkoxy" refers to an alkoxy group, wherein at least one of the hydrogen atoms of the alkoxy group has been replaced by a cyano group. Preferably, "cyanoalkoxy" refers to an alkoxy group wherein 1, 2 or 3 hydrogen atoms of the alkoxy group have been replaced by a cyano group. A particularly preferred, yet non-limiting example of cyanoalkoxy is cyanomethoxy.

The term "cyanoalkyl" refers to an alkyl group, wherein at least one of the hydrogen atoms of the alkyl group has been replaced by a cyano group. Preferably, "cyanoalkyl" refers to an alkyl group wherein 1, 2 or 3 hydrogen atoms of the alkyl group have been replaced by a cyano group. A particularly preferred, yet non-limiting example of cyanoalkyl is cyanomethyl.

The term "alkoxyalkynyloxy" refers to an alkynyloxy group, wherein at least one of the hydrogen atoms of the alkynyloxy group has been replaced by an alkoxy group. Preferably, "alkoxyalkynyloxy" refers to an alkynyloxy group wherein 1, 2 or 3 hydrogen atoms of the alkynyloxy group have been replaced by an alkoxy group.

The term "cycloalkylalkoxy" refers to an alkoxy group, wherein at least one of the hydrogen atoms of the alkoxy group has been replaced by a cycloalkyl group. Preferably, "cycloalkylalkoxy" refers to an alkoxy group wherein 1, 2 or 3 hydrogen atoms, most preferably 1 hydrogen atom of the alkoxy group have been replaced by a cycloalkyl group. A particularly preferred, yet non-limiting example of cycloalkylalkoxy is cyclopropylmethoxy.

The term "cyanocycloalkylalkoxy" refers to a cycloalkylalkoxy group, wherein at least one of the hydrogen atoms of the cycloalkylalkoxy group has been replaced by a cyano group. Preferably, "cyanocycloalkylalkoxy" refers to a cycloalkylalkoxy group wherein 1, 2 or 3 hydrogen atoms, most preferably 1 hydrogen atom of the cycloalkylalkoxy group have been replaced by a cyano group.

The term "hydroxyalkyl" refers to an alkyl group, wherein at least one of the hydrogen atoms of the alkyl group has been replaced by a hydroxy group. Preferably, "hydroxyalkyl" refers to an alkyl group wherein 1, 2 or 3 hydrogen atoms, most preferably 1 hydrogen atom of the alkyl group have been replaced by a hydroxy group. Preferred, yet non-limiting examples of hydroxyalkyl are hydroxymethyl and hydroxyethyl (e.g. 2-hydroxyethyl). A particularly preferred, yet non-limiting example of hydroxyalkyl is hydroxymethyl.

The term "hydroxyalkoxy" refers to an alkoxy group, wherein at least one of the hydrogen atoms of the alkoxy group has been replaced by a hydroxy group. Preferably, "hydroxyalkoxy" refers to an alkoxy group wherein 1, 2 or 3 hydrogen atoms, most preferably 1 hydrogen atom of the alkoxy group have been replaced by a hydroxy group. Preferred, yet non-limiting examples of hydroxyalkoxy are hydroxymethoxy and hydroxyethoxy (e.g. 2-hydroxyethoxy). A particularly preferred, yet non-limiting example of hydroxyalkoxy is hydroxymethoxy.

The term "hydroxyalkoxyalkyl" refers to an alkyl group, wherein at least one of the hydrogen atoms of the alkyl group has been replaced by a hydroxyalkoxy group. Preferably, "hydroxyalkoxyalkyl" refers to an alkyl group wherein 1, 2 or 3 hydrogen atoms, most preferably 1 hydrogen atom of the alkyl group have been replaced by a hydroxyalkoxy group. A preferred, yet non-limiting example of hydroxyalkoxyalkyl is 2-hydroxyethoxymethyl.

The term "alkoxycarbonylalkoxy" refers to an alkoxy group, wherein at least one of the hydrogen atoms of the alkoxy group has been replaced by an alkoxycarbonyl group. Preferably, "alkoxycarbonylalkoxy" refers to an alkoxy group wherein 1, 2 or 3 hydrogen atoms, most preferably 1 hydrogen atom of the alkoxy group have been replaced by an alkoxycarbonyl group. A preferred, yet non-limiting example of alkoxycarbonylalkoxy is 2-methoxy-2-oxo-ethoxy.

The term "arylalkoxy" refers to an alkoxy group, wherein at least one of the hydrogen atoms of the alkoxy group has been replaced by an aryl group. Preferably, "arylalkoxy" refers to an alkoxy group wherein 1, 2 or 3 hydrogen atoms, most preferably 1 hydrogen atom of the alkoxy group have been replaced by an aryl group. A particularly preferred, yet non-limiting example of arylalkoxy is benzyloxy.

The term "heteroarylalkoxy" refers to an alkoxy group, wherein at least one of the hydrogen atoms of the alkoxy group has been replaced by a heteroaryl group. Preferably, "heteroarylalkoxy" refers to an alkoxy group wherein 1, 2 or 3 hydrogen atoms, most preferably 1 hydrogen atom of the alkoxy group have been replaced by a heteroaryl group.

The term "alkoxyalkyl" refers to an alkyl group, wherein at least one of the hydrogen atoms of the alkyl group has been replaced by an alkoxy group. Preferably, "alkoxyalkyl" refers to an alkyl group wherein 1, 2 or 3 hydrogen atoms, most preferably 1 hydrogen atom of the alkyl group have been replaced by an alkoxy group. A particularly preferred, yet non-limiting example of alkoxyalkyl is 2-methoxyethyl.

The term "pharmaceutically acceptable salt" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, trifluoroacetic acid, propionic acid, glycolic acid, pyruvic acid, oxalic acid, maleic acid, malonic acid, succinic acid, fumaric acid, tartaric acid, lactic acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are hydrochlorides, fumarates, lactates (in particular derived from L-(+)-lactic acid), tartrates (in particular derived from L-(+)-tartaric acid) and trifluoroacetates.

The term "protective group" (PG) denotes the group which selectively blocks a reactive site in a multifunctional compound such that a chemical reaction can be carried out selectively at another unprotected reactive site in the meaning conventionally associated with it in synthetic chemistry. Protective groups can be removed at the appropriate point. Exemplary protective groups are amino-protective groups, carboxy-protective groups or hydroxy-protective groups. Particular protective groups are the tert-butoxycarbonyl (Boc), benzyloxycarbonyl (Cbz), fluorenylmethoxycarbonyl (Fmoc) and benzyl (Bn). Further particular protective groups are the tert-butoxycarbonyl (Boc) and the fluorenylmethoxycarbonyl (Fmoc). More particular protective group is the tert-butoxycarbonyl (Boc). Exemplary protective groups and their application in organic synthesis are described, for example, in "Protective Groups in Organic Chemistry" by T. W. Greene and P. G. M. Wutts, 5th Ed., 2014, John Wiley & Sons, N.Y.

The compounds of formula (I) can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention, the asymmetric carbon atom can be of the "R" or "S" configuration.

The term "treatment" as used herein includes: (1) inhibiting the state, disorder or condition (e.g. arresting, reducing or delaying the development of the disease, or a relapse thereof in case of maintenance treatment, of at least one clinical or subclinical symptom thereof); and/or (2) relieving the condition (i.e., causing regression of the state, disorder or condition or at least one of its clinical or subclinical symptoms). The benefit to a patient to be treated is either statistically significant or at least perceptible to the patient or to the physician. However, it will be appreciated that when a medicament is administered to a patient to treat a disease, the outcome may not always be effective treatment.

The term "prophylaxis" as used herein includes: preventing or delaying the appearance of clinical symptoms of the state, disorder or condition developing in a mammal and especially a human that may be afflicted with or predisposed to the state, disorder or condition but does not yet experience or display clinical or subclinical symptoms of the state, disorder or condition.

The term "mammal" as used herein includes both humans and non-humans and includes but is not limited to humans, non-human primates, canines, felines, murines, bovines, equines, and porcines. In a particularly preferred embodiment, the term "mammal" refers to humans.

The term "nosocomial infection" refers to a hospital-acquired infection (HAI), which is an infection that is acquired in a hospital or other health care facility. To emphasize both hospital and nonhospital settings, it is sometimes instead called a health care-associated infection (HAI or HCAI). Such an infection can be acquired in hospitals, nursing homes, rehabilitation facilities, outpatient clinics, or other clinical settings.

### Compounds of the Invention

In a first aspect, the present invention provides a compound of formula (I) 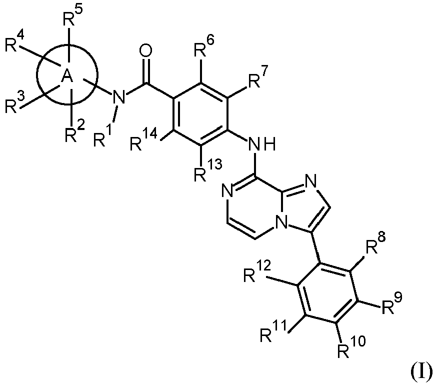 or a pharmaceutically acceptable salt thereof, wherein:
- A: is C₂-C₉-heterocycloalkyl, C₃-C₁₂-cycloalkyl, C₆-C₁₄-aryl or C₁-C₁₃-heteroaryl;
- R¹: is hydrogen, C₁-C₆-alkyl, halo-C₁-C₆-alkyl, cyano-C₁-C₆-alkyl, hydroxy-C₁-C₆-alkyl, amino-C₁-C₆-alkyl, C₁-C₆-alkyl-NH-C₁-C₆-alkyl-, (C₁-C₆-alkyl)₂N-C₁-C₆-alkyl-amino-C₁-C₆-alkyl-O-C₁-C₆-alkyl, C₁-C₆-alkyl-NH-C₁-C₆-alkyl-O-C₁-C₆-alkyl-, (C₁-C₆-alkyl)₂N-C₁-C₆-alkyl-O-C₁-C₆-alkyl- or C₃-C₁₂-cycloalkyl;
- R²: is hydrogen, hydroxy, carboxy, amino, C₁-C₆-alkyl-NH-, C₁-C₆-alkyl-C(O)-NH-, (C₁-C₆-alkyl)₂N-, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkyl-NH-C₂-C₆-alkenyl-NH-, amino-C₂-C₆-alkenyl-NH-, C₁-C₆-alkylimidoyl-NH-C₁-C₆-alkyl; C₁-C₆-alkyl or a group 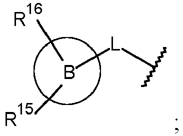
- each of R³, R⁴ and R⁵: is independently hydrogen or hydroxy;
- R⁶: is hydrogen, halogen, C₁-C₆-alkyl, halo-C₁-C₆-alkyl, hydroxy-C₁-C₆-alkyl, C₂-C₆-alkenyl, NO₂, CN, C₃-C₁₂-cycloalkyl, hydroxy-C₁-C₆-alkoxy-C₁-C₆-alkyl, C₁-C₆-alkoxy or alkoxy-C₁-C₆-alkyl;
- R⁷ and R¹³: are each independently hydrogen, halogen or C₁-C₆-alkyl;
- each of R⁸, R⁹, R¹⁰, R¹¹ and R¹²: is independently hydrogen, halogen, C₁-C₆-alkoxycarbonyl-C₁-C₆-alkoxy, amino, C₁-C₆-alkyl-NH-, (C₁-C₆-alkyl)₂N-, hydroxy, C₁-C₆-alkoxy, C₁-C₆-alkylsulfanyl, C₁-C₆-alkylsulfonyloxy, C₃-C₁₂-cycloalkyl-C₁-C₆-alkoxy, halo-C₁-C₆-alkoxy, C₆-C₁₄-aryl-C₁-C₆-alkoxy, C₁-C₁₃-heteroaryloxy, C₁-C₁₃-heteroaryl-C₁-C₆-alkoxy, cyano-C₁-C₆-alkoxy, C₃-C₁₂-cycloalkyloxy, C₂-C₆-alkynyloxy, C₁-C₆-alkoxy-C₂-C₆-alkynyloxy, cyano-C₃-C₁₂-cycloalkyloxy, cyano-C₃-C₁₂-cycloalkyl-C₁-C₆-alkoxy, amino-C₂-C₆-alkynyloxy, hydroxy-C₂-C₆-alkynyloxy, halo-C₁-C₆-alkyl, sulfamoyl, C₁-C₆-alkylsulfamoyl, C₁-C₆-alkyl, amino-C₁-C₆-alkoxy-C₂-C₆-alkynyloxy or amino-C₁-C₆-alkoxy;
- R¹⁴: is hydrogen, halogen or C₁-C₆-alkyl;
- R¹⁵ and R¹⁶: are each independently hydrogen, oxo or hydroxy;
- B: is C₂-C₉-heterocycloalkyl; and
- L: is a covalent bond, carbonyl, -NH-C(O)- or -C(O)-NH-.

In one embodiment, the present invention provides a compound of formula (I), or a pharmaceutically acceptable salt thereof, wherein:
- A: is C₂-C₉-heterocycloalkyl, C₃-C₁₂-cycloalkyl, C₆-C₁₄-aryl or C₁-C₁₃-heteroaryl;
- R¹: is hydrogen, C₁-C₆-alkyl, halo-C₁-C₆-alkyl, cyano-C₁-C₆-alkyl, hydroxy-C₁-C₆-alkyl, amino-C₁-C₆-alkyl, C₁-C₆-alkyl-NH-C₁-C₆-alkyl-, (C₁-C₆-alkyl)₂N-C₁-C₆-alkyl-amino-C₁-C₆-alkyl-O-C₁-C₆-alkyl, C₁-C₆-alkyl-NH-C₁-C₆-alkyl-O-C₁-C₆-alkyl-, (C₁-C₆-alkyl)₂N-C₁-C₆-alkyl-O-C₁-C₆-alkyl- or C₃-C₁₂-cycloalkyl;
- R²: is hydrogen, hydroxy, carboxy, amino, C₁-C₆-alkyl-NH-, (C₁-C₆-alkyl)₂N-, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkyl-NH-C₂-C₆-alkenyl-NH- amino-C₂-C₆-alkenyl-NH-, C₁-C₆-alkylimidoyl-NH-C₁-C₆-alkyl; C₁-C₆-alkyl or a group 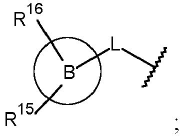
- each of R³, R⁴ and R⁵: is independently hydrogen or hydroxy;
- R⁶: is hydrogen, halogen, C₁-C₆-alkyl, halo-C₁-C₆-alkyl, hydroxy-C₁-C₆-alkyl, C₂-C₆-alkenyl, NO₂, CN, C₃-C₁₂-cycloalkyl, hydroxy-C₁-C₆-alkoxy-C₁-C₆-alkyl, C₁-C₆-alkoxy or alkoxy-C₁-C₆-alkyl;
- R⁷ and R¹³: are each independently hydrogen, halogen or C₁-C₆-alkyl;
- each of R⁸, R⁹, R¹⁰, R¹¹ and R¹²: is independently hydrogen, halogen, C₁-C₆-alkoxycarbonyl-C₁-C₆-alkoxy, amino, C₁-C₆-alkyl-NH-, (C₁-C₆-alkyl)₂N-, hydroxy, C₁-C₆-alkoxy, C₁-C₆-alkylsulfanyl, C₁-C₆-alkylsulfonyloxy, C₃-C₁₂-cycloalkyl-C₁-C₆-alkoxy, halo-C₁-C₆-alkoxy, C₆-C₁₄-aryl-C₁-C₆-alkoxy, C₁-C₁₃-heteroaryloxy, C₁-C₁₃-heteroaryl-C₁-C₆-alkoxy, cyano-C₁-C₆-alkoxy, C₃-C₁₂-cycloalkyloxy, C₂-C₆-alkynyloxy, C₁-C₆-alkoxy-C₂-C₆-alkynyloxy, cyano-C₃-C₁₂-cycloalkyloxy, cyano-C₃-C₁₂-cycloalkyl-C₁-C₆-alkoxy, amino-C₂-C₆-alkynyloxy, hydroxy-C₂-C₆-alkynyloxy, halo-C₁-C₆-alkyl, sulfamoyl, C₁-C₆-alkylsulfamoyl, C₁-C₆-alkyl, amino-C₁-C₆-alkoxy-C₂-C₆-alkynyloxy or amino-C₁-C₆-alkoxy;
- R¹⁴: is hydrogen, halogen or C₁-C₆-alkyl;
- R¹⁵ and R¹⁶: are each independently hydrogen, oxo or hydroxy;
- B: is C₂-C₉-heterocycloalkyl; and
- L: is a covalent bond, carbonyl or -C(O)-NH-.

In one embodiment, the present invention provides a compound of formula (I) as described herein, or a pharmaceutically acceptable salt thereof, wherein R¹ is hydrogen or C₁-C₆-alkyl.

In a preferred embodiment, the present invention provides a compound of formula (I) as described herein, or a pharmaceutically acceptable salt thereof, wherein R¹ is hydrogen.

In one embodiment, the present invention provides a compound of formula (I) as described herein, or a pharmaceutically acceptable salt thereof, wherein R² is hydrogen, hydroxy, carboxy, amino, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-,C₁-C₆-alkylimidoyl-NH-C₁-C₆-alkyl; C₁-C₆-alkyl or a group 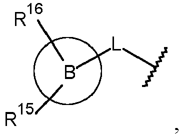 wherein R¹⁵, R¹⁶, B and L are as defined herein.

In a preferred embodiment, the present invention provides a compound of formula (I) as described herein, or a pharmaceutically acceptable salt thereof, wherein R² is amino, C₁-C₆-alkylimidoyl-NH-C₁-C₆-alkyl or a group 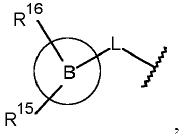 wherein R¹⁵, R¹⁶, B and L are as defined herein.

In one embodiment, the present invention provides a compound of formula (I) as described herein, or a pharmaceutically acceptable salt thereof, wherein R² is hydrogen, hydroxy, carboxy, amino, amino-C₁-C₆-alkyl, C₁-C₆-alkyl-C(O)-NH-, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-,C₁-C₆-alkylimidoyl-NH-C₁-C₆-alkyl; C₁-C₆-alkyl or a group 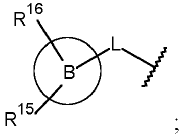 wherein:
- R¹⁵: is oxo or hydroxy;
- R¹⁶: is hydrogen or oxo;
- B: is C₂-C₉-heterocycloalkyl; and
- L: is a covalent bond, carbonyl or -C(O)-NH-.

In a preferred embodiment, the present invention provides a compound of formula (I) as described herein, or a pharmaceutically acceptable salt thereof, wherein R² is amino, C₁-C₆-alkylimidoyl-NH-C₁-C₆-alkyl or a group 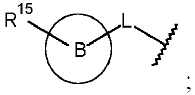 wherein:
- R¹⁵: is hydroxy;
- B: is C₂-C₉-heterocycloalkyl; and
- L: is carbonyl or -C(O)-NH-.

In a particularly preferred embodiment, the present invention provides a compound of formula (I) as described herein, or a pharmaceutically acceptable salt thereof, wherein R² is amino, (ethanimidoylamino)methyl or a group 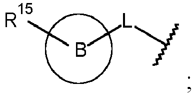 wherein:
- R¹⁵: is hydroxy;
- B: is pyrrolidine; and
- L: is carbonyl or -C(O)-NH-.

In a particularly preferred embodiment, the present invention provides a compound of formula (I) as described herein, or a pharmaceutically acceptable salt thereof, wherein R² is amino, 4-hydroxypyrrolidine-2-carbonyl, (4-hydroxypyrrolidine-2-carbonyl)amino or (ethanimidoylamino)methyl.

In one embodiment, the present invention provides a compound of formula (I) as described herein, or a pharmaceutically acceptable salt thereof, wherein R³ is hydrogen.

In one embodiment, the present invention provides a compound of formula (I) as described herein, or a pharmaceutically acceptable salt thereof, wherein R⁴ is hydrogen.

In one embodiment, the present invention provides a compound of formula (I) as described herein, or a pharmaceutically acceptable salt thereof, wherein R⁵ is hydrogen.

In one embodiment, the present invention provides a compound of formula (I) as described herein, or a pharmaceutically acceptable salt thereof, wherein R⁶ is hydrogen, halogen or C₁-C₆alkyl.

In a preferred embodiment, the present invention provides a compound of formula (I) as described herein, or a pharmaceutically acceptable salt thereof, wherein R⁶ is C₁-C₆-alkyl.

In a particularly preferred embodiment, the present invention provides a compound of formula (I) as described herein, or a pharmaceutically acceptable salt thereof, wherein R⁶ is ethyl or methyl.

In one embodiment, the present invention provides a compound of formula (I) as described herein, or a pharmaceutically acceptable salt thereof, wherein R⁷ is hydrogen.

In one embodiment, the present invention provides a compound of formula (I) as described herein, or a pharmaceutically acceptable salt thereof, wherein R⁸ is hydrogen or halogen.

In a preferred embodiment, the present invention provides a compound of formula (I) as described herein, or a pharmaceutically acceptable salt thereof, wherein R⁸ is halogen.

In a particularly preferred embodiment, the present invention provides a compound of formula (I) as described herein, or a pharmaceutically acceptable salt thereof, wherein R⁸ is fluoro.

In one embodiment, the present invention provides a compound of formula (I) as described herein, or a pharmaceutically acceptable salt thereof, wherein R⁹ is hydrogen or halogen.

In a preferred embodiment, the present invention provides a compound of formula (I) as described herein, or a pharmaceutically acceptable salt thereof, wherein R⁹ is halogen.

In a particularly preferred embodiment, the present invention provides a compound of formula (I) as described herein, or a pharmaceutically acceptable salt thereof, wherein R⁹ is fluoro.

In one embodiment, the present invention provides a compound of formula (I) as described herein, or a pharmaceutically acceptable salt thereof, wherein R¹⁰ is C₁-C₆-alkoxy or halo-C₁-C₆-alkoxy.

In a preferred embodiment, the present invention provides a compound of formula (I) as described herein, or a pharmaceutically acceptable salt thereof, wherein R¹⁰ is C₁-C₆-alkoxy.

In a particularly preferred embodiment, the present invention provides a compound of formula (I) as described herein, or a pharmaceutically acceptable salt thereof, wherein R¹⁰ is methoxy.

In one embodiment, the present invention provides a compound of formula (I) as described herein, or a pharmaceutically acceptable salt thereof, wherein R¹¹ is hydrogen.

In one embodiment, the present invention provides a compound of formula (I) as described herein, or a pharmaceutically acceptable salt thereof, wherein R¹² is hydrogen.

In one embodiment, the present invention provides a compound of formula (I) as described herein, or a pharmaceutically acceptable salt thereof, wherein R¹³ is hydrogen.

In one embodiment, the present invention provides a compound of formula (I) as described herein, or a pharmaceutically acceptable salt thereof, wherein R¹⁴ is hydrogen.

In one embodiment, the present invention provides a compound of formula (I) as described herein, or a pharmaceutically acceptable salt thereof, wherein R¹⁵ is oxo or hydroxy.

In a preferred embodiment, the present invention provides a compound of formula (I) as described herein, or a pharmaceutically acceptable salt thereof, wherein R¹⁵ is hydroxy.

In one embodiment, the present invention provides a compound of formula (I) as described herein, or a pharmaceutically acceptable salt thereof, wherein R¹⁶ is hydrogen or oxo.

In a preferred embodiment, the present invention provides a compound of formula (I) as described herein, or a pharmaceutically acceptable salt thereof, wherein R¹⁶ is hydrogen.

In one embodiment, the present invention provides a compound of formula (I) as described herein, or a pharmaceutically acceptable salt thereof, wherein A is C₂-C₉-heterocycloalkyl, C₃-C₁₂-cycloalkyl or C₆-C₁₄-aryl.

In a preferred embodiment, the present invention provides a compound of formula (I) as described herein, or a pharmaceutically acceptable salt thereof, wherein A is C₂-C₉-heterocycloalkyl or C₃-C₁₂-cycloalkyl.

In a particularly preferred embodiment, the present invention provides a compound of formula (I) as described herein, or a pharmaceutically acceptable salt thereof, wherein A is cyclopropyl, cyclobutyl, cyclopentyl or 4-piperidyl.

In one embodiment, the present invention provides a compound of formula (I) as described herein, or a pharmaceutically acceptable salt thereof, wherein B is pyrrolidin-2-yl.

In one embodiment, the present invention provides a compound of formula (I) as described herein, or a pharmaceutically acceptable salt thereof, wherein L is carbonyl or -C(O)-NH-.

In one embodiment, the present invention provides a compound of formula (I) as described herein, or a pharmaceutically acceptable salt thereof, wherein:
- A: is C₂-C₉-heterocycloalkyl, C₃-C₁₂-cycloalkyl or C₆-C₁₄-aryl;
- R¹: is hydrogen or C₁-C₆-alkyl;
- R²: is hydrogen, hydroxy, carboxy, amino, C₁-C₆-alkyl-C(O)-NH-, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkylimidoyl-NH-C₁-C₆-alkyl; C₁-C₆-alkyl or a group 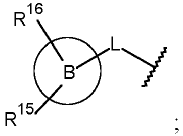
- each of R³, R⁴, R⁵, R⁷, R¹¹, R¹², R¹³ and R¹⁴: is hydrogen;
- R⁶: is hydrogen, halogen or C₁-C₆-alkyl;
- each of R⁸ and R⁹: is independently hydrogen or halogen;
- R¹⁰: is C₁-C₆-alkoxy or halo-C₁-C₆-alkoxy;
- R¹⁵: is oxo or hydroxy;
- R¹⁶: is hydrogen or oxo;
- B: is C₂-C₉-heterocycloalkyl; and
- L: is a covalent bond, carbonyl or -C(O)-NH-.

In one embodiment, the present invention provides a compound of formula (I) as described herein, or a pharmaceutically acceptable salt thereof, wherein:
- A: is C₂-C₉-heterocycloalkyl, C₃-C₁₂-cycloalkyl or C₆-C₁₄-aryl;
- R¹: is hydrogen or C₁-C₆-alkyl;
- R²: is hydrogen, hydroxy, carboxy, amino, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkylimidoyl-NH-C₁-C₆-alkyl; C₁-C₆-alkyl or a group 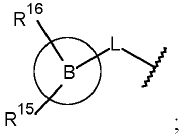
- each of R³, R⁴, R⁵, R⁷, R¹¹, R¹², R¹³ and R¹⁴: is hydrogen;
- R⁶: is hydrogen, halogen or C₁-C₆-alkyl;
- each of R⁸ and R⁹: is independently hydrogen or halogen;
- R¹⁰: is C₁-C₆-alkoxy or halo-C₁-C₆-alkoxy;
- R¹⁵: is oxo or hydroxy;
- R¹⁶: is hydrogen or oxo;
- B: is C₂-C₉-heterocycloalkyl; and
- L: is a covalent bond, carbonyl or -C(O)-NH-.

In a preferred embodiment, the present invention provides a compound of formula (I) as described herein, or a pharmaceutically acceptable salt thereof, wherein:
- A: is C₂-C₉-heterocycloalkyl or C₃-C₁₂-cycloalkyl;
each of R¹, R³, R⁴, R⁵, R⁷, R¹¹, R¹², R¹³, R¹⁴ and R¹⁶ is hydrogen;
- R²: is amino, C₁-C₆-alkylimidoyl-NH-C₁-C₆-alkyl or a group 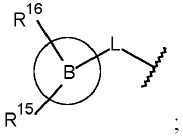
- R⁶: is C₁-C₆-alkyl;
- R⁸ and R⁹: are both halogen;
- R¹⁰: is C₁-C₆-alkoxy;
- R¹⁵: is hydroxy;
- B: is C₂-C₉-heterocycloalkyl; and
- L: is carbonyl or -C(O)-NH-.

In a particularly preferred embodiment, the present invention provides a compound of formula (I) as described herein, or a pharmaceutically acceptable salt thereof, wherein:
- A: is cyclopropyl, cyclobutyl, cyclopentyl or 4-piperidyl;
each of R¹, R3, R⁴, R⁵, R⁷, R¹¹, R¹², R¹³, R¹⁴ and R¹⁶ is hydrogen;
- R²: is amino, (ethanimidoylamino)methyl or a group 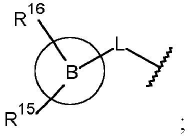
- R⁶: is ethyl or methyl;
- R⁸ and R⁹: are both fluoro;
- R¹⁰: is methoxy;
- R¹⁵: is hydroxy;
- B: is pyrrolidin-2-yl; and
- L: is carbonyl or -C(O)-NH-.

In one embodiment, the present invention provides a compound of formula (I) as described herein, or a pharmaceutically acceptable salt thereof, wherein:
- A: is C₂-C₉-heterocycloalkyl, C₃-C₁₂-cycloalkyl or C₆-C₁₄-aryl;
- R¹: is hydrogen or C₁-C₆-alkyl;
- R²: is hydrogen, hydroxy, carboxy, amino, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkylimidoyl-NH-C₁-C₆-alkyl; C₁-C₆-alkyl or a group
- each of R³, R⁴ and R⁵: is hydrogen; and
- B, L, R¹⁵ and R¹⁶: are as defined herein.

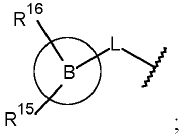

In a preferred embodiment, the present invention provides a compound of formula (I) as described herein, or a pharmaceutically acceptable salt thereof, wherein:
- A: is C₂-C₉-heterocycloalkyl or C₃-C₁₂-cycloalkyl;
- each of R¹, R³, R⁴ and R⁵: is hydrogen;
- R²: is amino, C₁-C₆-alkylimidoyl-NH-C₁-C₆-alkyl or a group 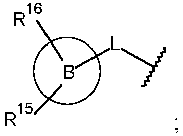 and

- B, L, R¹⁵ and R¹⁶: are as defined herein.

In a particularly preferred embodiment, the present invention provides a compound of formula (I) as described herein, or a pharmaceutically acceptable salt thereof, wherein:
- A: is cyclopropyl, cyclobutyl, cyclopentyl or 4-piperidyl;
- each of R¹, R³, R⁴ and R⁵: is hydrogen;
- R²: is amino, (ethanimidoylamino)methyl or a group 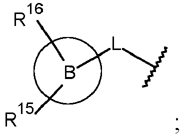 and
- B, L, R¹⁵ and R¹⁶: are as defined herein.

In one embodiment, the present invention provides a compound of formula (I) as described herein, or a pharmaceutically acceptable salt thereof, wherein:
- R⁶: is hydrogen, halogen or C₁-C₆-alkyl; and
- each of R⁷, R¹³, and R¹⁴: is hydrogen.

In a preferred embodiment, the present invention provides a compound of formula (I) as described herein, or a pharmaceutically acceptable salt thereof, wherein:
- R⁶: is C₁-C₆-alkyl; and each of R⁷, R¹³, and R¹⁴ is hydrogen.

In a particularly preferred embodiment, the present invention provides a compound of formula (I) as described herein, or a pharmaceutically acceptable salt thereof, wherein:
- R⁶: is ethyl or methyl; and each of R⁷, R¹³, and R¹⁴ is hydrogen.

In one embodiment, the present invention provides a compound of formula (I) as described herein, or a pharmaceutically acceptable salt thereof, wherein:
each of R⁸ and R⁹ is independently hydrogen or halogen;
- R¹⁰: is C₁-C₆-alkoxy or halo-C₁-C₆-alkoxy; and
- R¹¹ and R¹²: are both hydrogen.

In a preferred embodiment, the present invention provides a compound of formula (I) as described herein, or a pharmaceutically acceptable salt thereof, wherein:
each of R⁸ and R⁹ are both halogen;
- R¹⁰: is C₁-C₆-alkoxy; and
- R¹¹ and R¹²: are both hydrogen.

In a particularly preferred embodiment, the present invention provides a compound of formula (I) as described herein, or a pharmaceutically acceptable salt thereof, wherein:
each of R⁸ and R⁹ are both fluoro;
- R¹⁰: is methoxy; and
- R¹¹ and R¹²: are both hydrogen.

In one embodiment, the present invention provides a compound of formula (I) as described herein, or a pharmaceutically acceptable salt thereof, wherein:
- R¹⁵: is oxo or hydroxy;
- R¹⁶: is hydrogen or oxo;
- B: is C₂-C₉-heterocycloalkyl; and
- L: is a covalent bond, carbonyl or -C(O)-NH-.

In a preferred embodiment, the present invention provides a compound of formula (I) as described herein, or a pharmaceutically acceptable salt thereof, wherein:
- R¹⁵: is hydroxy;
- R¹⁶: is hydrogen;
- B: is C₂-C₉-heterocycloalkyl; and
- L: is a carbonyl or -C(O)-NH-.

In a particularly preferred embodiment, the present invention provides a compound of formula (I) as described herein, or a pharmaceutically acceptable salt thereof, wherein:
- R¹⁵: is hydroxy;
- R¹⁶: is hydrogen;
- B: is pyrrolidin-2-yl; and
- L: is a carbonyl or -C(O)-NH-.

In one embodiment, the present invention provides a compound of formula (I) as described herein, or a pharmaceutically acceptable salt thereof, wherein the group 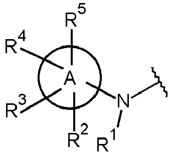 is selected from:
methyl-(1-methylpiperidin-4-yl)amino; methyl-(1-methylpiperidin-4-yl)amino; [(2-(R),3-(R),4-(R),5-(R),6-(R))-2,4,5-trihydroxy-6-(hydroxymethyl)oxan-3-yl]amino; methyl(piperidin-4-yl)amino; methyl(piperidin-4-yl)amino; 1-azabicyclo[2.2. 2]octan-3-ylamino; methyl(piperidin-4-yl)amino; [2-(aminomethyl)-1,3-dioxan-5-yl]amino; [(3-(S),4-(R))-4-hydroxypyrrolidin-3-yl]-methylamino; [1-(1,1-dioxothian-4-yl)piperidin-4-yl]amino; 4-carboxyanilino; [(3-(R),4-(R))-3-fluoropiperidin-4-yl]amino; [1-(aminomethyl)cyclopropyl]amino; [3-(hydroxymethyl)oxetan-3-yl]amino; [(3-(R))-pyrrolidin-3-yl]amino; [(3-(R))-pyrrolidin-3-yl]amino; [(3-(S))-2-oxopyrrolidin-3-yl]amino; [1-(aminomethyl)cyclopropyl]amino; azetidin-3-ylamino; piperidin-4-ylamino; (4-aminocyclohexyl)amino; (3-aminocyclobutyl)amino; (3-aminocyclobutyl)amino; [(1-(R),2-(R))-2-aminocyclohexyl]amino; [(1-(R),2-(R))-2-aminocyclopentyl]amino; [4-[[(2-(S))-2-amino-5-carbamimidamidopentanoyl]amino]cyclohexyl] amino; [3-[[(2-(S))-2-amino-5-carbamimidamidopentanoyl]amino]cyclobutyl]amino; [(1-(R),2-(R))-2-aminocyclopentyl] amino; [1-(carbamimidamidomethyl)cyclopropyl] amino; [1-[(ethanimidoylamino)methyl]cyclopropyl]amino; (4-aminocyclohexyl)amino; (3-aminocyclobutyl)amino; [4-[[(2-(S),4-(S))-4-hydroxypyrrolidine-2-carbonyl] amino] cyclohexyl] amino; [(3-(R))-pyrrolidin-3-yl]amino; [1-[(2-(S),4-(S))-4-hydroxypyrrolidine-2-carbonyl]piperidin-4-yl]amino; [4-[[1-(dimethylamino)-2-methylprop-1-enyl]amino]cyclohexyl]amino; (2-aminocyclobutyl)amino; [3-[[(2-(S),4-(S))-4-hydroxypyrrolidine-2-carbonyl]amino]cyclopentyl]amino; and (1-methylpiperidin-4-yl)amino.

In one embodiment, the present invention provides a compound of formula (I) as described herein, or a pharmaceutically acceptable salt thereof, wherein the compound of formula (I) is selected from:
N-[2-(aminomethyl)-1,3-dioxan-5-yl]-4-[[3-(4-methoxyphenyl)imidazo[1,2-a]pyrazin-8-yl] amino]-2-methyl-benzamide;
2-chloro-4-[[3-(3-fluoro-4-methoxy-phenyl)imidazo[1,2-a]pyrazin-8-yl]amino]-N-methyl-N-(4-piperidyl)benzamide;
4-[[3-(3-fluoro-4-methoxy-phenyl)imidazo[1,2-a]pyrazin-8-yl] amino] -N,2-dimethyl-N-(4-piperidyl)benzamide;
4-[[3-[4-(difluoromethoxy)-3-fluoro-phenyl]imidazo[1,2-a]pyrazin-8-yl]amino]-N,2-dimethyl-N-(4-piperidyl)benzamide;
4-[[3-(4-methoxyphenyl)imidazo[1,2-a]pyrazin-8-yl]amino]-2-methyl-N-(1-methyl-4-piperidyl)benzamide;
4-[[3-[4-(difluoromethoxy)phenyl]imidazo[1,2-a]pyrazin-8-yl] amino] -2-methyl-N-quinuclidin-3-yl-benzamide;
4-[[3-(4-methoxyphenyl)imidazo[1,2-a]pyrazin-8-yl]amino]-N,2-dimethyl-N-(1-methyl-4-piperidyl)benzamide;
4-[[3-(4-methoxyphenyl)imidazo[1,2-a]pyrazin-8-yl]amino]-N-methyl-N-(1-methyl-4-piperidyl)benzamide;
4-[[3-[4-(difluoromethoxy)phenyl]imidazo[1,2-a]pyrazin-8-yl] amino] -2-methyl-N-[(2S,3S,4R,5S,6R)-2,4,5-trihydroxy-6-(hydroxymethyl)tetrahydropyran-3-yl]benzamide;
N-[1-(1,1-dioxothian-4-yl)-4-piperidyl]-4-[[3-(3-fluoro-4-methoxy-phenyl)imidazo[1,2-a]pyrazin-8-yl]amino]-2-methyl-benzamide;
4-[[4-[[3-(3-fluoro-4-methoxy-phenyl)imidazo[1,2-a]pyrazin-8-yl]amino]-2-methylbenzoyl]amino]benzoic acid;
4-[[3-(3-fluoro-4-methoxy-phenyl)imidazo[1,2-a]pyrazin-8-yl]amino]-N-[(3R,4R)-4-hydroxypyrrolidin-3-yl]-N,2-dimethyl-benzamide;
4-[[3-(3-fluoro-4-methoxy-phenyl)imidazo[1,2-a]pyrazin-8-yl]amino]-N-[(3R,4S)-3-fluoro-4-piperidyl]-2-methyl-benzamide;
4-[[3-(2,3-difluoro-4-methoxy-phenyl)imidazo[1,2-a]pyrazin-8-yl]amino]-2-ethyl-N-[3-(hydroxymethyl)oxetan-3 -yl] benzamide;
4-[[3-(2,3-difluoro-4-methoxy-phenyl)imidazo[1,2-a]pyrazin-8-yl]amino]-2-ethyl-N-[(3R)-pyrrolidin-3-yl] benzamide;
4-[[3-(2,3-difluoro-4-methoxy-phenyl)imidazo[1,2-a]pyrazin-8-yl]amino]-2-ethyl-N-[(3S)-pyrrolidin-3-yl] benzamide;
N-[1-(aminomethyl)cyclopropyl]-4-[[3-[4-(difluoromethoxy)-2,3-difluoro-phenyl]imidazo[1,2-a] pyrazin-8-yl] amino]-2-ethyl-benzamide;
4-[[3-(2,3-difluoro-4-methoxy-phenyl)imidazo[1,2-a]pyrazin-8-yl]amino]-2-ethyl-N-[(3S)-2-oxopyrrolidin-3 -yl] benzamide;
N-(azetidin-3-yl)-4-[[3-(2,3-difluoro-4-methoxy-phenyl)imidazo[1,2-a]pyrazin-8-yl]amino]-2-ethyl-benzamide;
N-[(1S,2S)-2-aminocyclopentyl]-4-[[3-(2,3-difluoro-4-methoxy-phenyl)imidazo[1,2-a]pyrazin-8-yl] amino]-2-ethyl-benzamide;
N-[1-(aminomethyl)cyclopropyl]-4-[[3-(2,3-difluoro-4-methoxy-phenyl)imidazo[1,2-a]pyrazin-8-yl] amino]-2-ethyl-benzamide;
4-[[3-(2,3-difluoro-4-methoxy-phenyl)imidazo[1,2-a]pyrazin-8-yl]amino]-2-ethyl-N-(4-piperidyl)benzamide;
N-(4-aminocyclohexyl)-4-[[3-(2,3-difluoro-4-methoxy-phenyl)imidazo[1,2-a]pyrazin-8-yl] amino]-2-ethyl-benzamide;
N-(3-aminocyclobutyl)-4-[[3-(2,3-difluoro-4-methoxy-phenyl)imidazo[1,2-a]pyrazin-8-yl] amino]-2-ethyl-benzamide;
4-[[3-(2,3-difluoro-4-methoxy-phenyl)imidazo[1,2-a]pyrazin-8-yl]amino]-2-ethyl-N-[rac-(1R,2S)-2-aminocyclohexyl]benzamide;
N-[(1S,2R)-2-aminocyclopentyl]-4-[[3-(2,3-difluoro-4-methoxy-phenyl)imidazo[1,2-a]pyrazin-8-yl] amino]-2-ethyl-benzamide;
N-[4-[[(2S)-2-amino-5-guanidino-pentanoyl]amino]cyclohexyl]-4-[[3-(2,3-difluoro-4-methoxyphenyl)imidazo[1,2-a]pyrazin-8-yl]amino]-2-ethyl-benzamide;
N-(4-aminocyclohexyl)-4-[[3-(2,3-difluoro-4-methoxy-phenyl)imidazo[1,2-a]pyrazin-8-yl] amino]-2-methyl-benzamide;
4-[[3-(2,3-difluoro-4-methoxy-phenyl)imidazo[1,2-a]pyrazin-8-yl]amino]-N-[1-[(2S,4R)-4-hydroxypyrrolidine-2-carbonyl]-4-piperidyl]-2-methyl-benzamide;
4-[[3-(2,3-difluoro-4-methoxy-phenyl)imidazo[1,2-a]pyrazin-8-yl]amino]-N-[4-[[1-(dimethylamino)-2-methyl-prop-1-enyl]amino]cyclohexyl]-2-methyl-benzamide;
4-[[3-(2,3-difluoro-4-methoxy-phenyl)imidazo[1,2-a]pyrazin-8-yl]amino]-2-ethyl-N-[1-(guanidinomethyl)cyclopropyl]benzamide;
(2S,4R)-N-[4-[[4-[[3-(2,3-difluoro-4-methoxy-phenyl)imidazo[1,2-a]pyrazin-8-yl]amino]-2-methyl-benzoyl]amino]cyclohexyl]-4-hydroxy-pyrrolidine-2-carboxamide;
N-(3-aminocyclobutyl)-4-[[3-(2,3-difluoro-4-methoxy-phenyl)imidazo[1,2-a]pyrazin-8-yl] amino]-2-methyl-benzamide;
rac-(2S,4R)-N-[3-[[4-[[3-(2,3-difluoro-4-methoxy-phenyl)imidazo[1,2-a]pyrazin-8-yl]amino]-2-methyl-benzoyl]amino]cyclopentyl]-4-hydroxy-pyrrolidine-2-carboxamide;
N-[3-[[(2S)-2-amino-5-guanidino-pentanoyl]amino]cyclobutyl]-4-[[3-(2,3-difluoro-4-methoxyphenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-N-[1-[(ethanimidoylamino)methyl]cyclopropyl]-2-ethyl-benzamide;
N-(2-aminocyclobutyl)-4-[[3-(2,3-difluoro-4-methoxy-phenyl)imidazo[1,2-a]pyrazin-8-yl] amino]-2-ethyl-benzamide;
4-[[3-(2,3-difluoro-4-methoxy-phenyl)imidazo[1,2-a]pyrazin-8-yl]amino]-2-methyl-N-[(3R)-pyrrolidin-3-yl]benzamide;
4-[[3-(2,3-difluoro-4-methoxy-phenyl)imidazo[1,2-a]pyrazin-8-yl]amino]-2-ethyl-N-[rac-(1R,2S)-2-aminocyclopropyl]benzamide;
rac-(2S,4R)-N-[2-[[4-[[3-(2,3-difluoro-4-methoxy-phenyl)imidazo[1,2-a]pyrazin-8-yl]amino]-2-ethyl-benzoyl]amino]cyclopropyl]-4-hydroxy-pyrrolidine-2-carboxamide;
4-[[3-(2,3-difluoro-4-methoxy-phenyl)imidazo[1,2-a]pyrazin-8-yl]amino]-2-ethyl-N-[rac-(1S,5R)-3-azabicyclo[3.1.0]hexan-6-yl]benzamide;
N-[(1R,2S)-2-aminocyclopentyl]-4-[[3-(2,3-difluoro-4-methoxy-phenyl)imidazo[1,2-a]pyrazin-8-yl] amino]-2-ethyl-benzamide;
(2S,4R)-N-[3-[[4-[[3-(2,3-difluoro-4-methoxy-phenyl)imidazo[1,2-a]pyrazin-8-yl]amino]-2-ethyl-benzoyl]amino]-1-bicyclo[1.1.1]pentanyl]-4-hydroxy-pyrrolidine-2-carboxamide;
rac-(2S,4R)-N-[2-[[4-[[3-(2,3-difluoro-4-methoxy-phenyl)imidazo[1,2-a]pyrazin-8-yl]amino]-2-ethyl-benzoyl]amino]cyclobutyl]-4-hydroxy-pyrrolidine-2-carboxamide;
rac-(2S,4R)-N-[2-[[4-[[3-(2,3-difluoro-4-methoxy-phenyl)imidazo[1,2-a]pyrazin-8-yl]amino]-2-ethyl-benzoyl]amino]cyclopropyl]-4-hydroxy-pyrrolidine-2-carboxamide;
(2S,4R)-N-[3-[[4-[[3-(2,3-difluoro-4-methoxy-phenyl)imidazo[1,2-a]pyrazin-8-yl]amino]-2-methyl-benzoyl]amino]cyclobutyl]-4-hydroxy-pyrrolidine-2-carboxamide;
(2S,4R)-N-[3-[[4-[[3-(2,3-difluoro-4-methoxy-phenyl)imidazo[1,2-a]pyrazin-8-yl]amino]-2-methyl-benzoyl]amino]cyclobutyl]-4-hydroxy-pyrrolidine-2-carboxamide;
4-[[3-(2,3-difluoro-4-methoxy-phenyl)imidazo[1,2-a]pyrazin-8-yl]amino]-N-[1-[(2S,4R)-4-hydroxypyrrolidine-2-carbonyl]azetidin-3-yl]-2-methyl-benzamide;
4-[[3-(2,3-difluoro-4-methoxy-phenyl)imidazo[1,2-a]pyrazin-8-yl]amino]-N-[(3S)-1-[(2S,4R)-4-hydroxypyrrolidine-2-carbonyl]pyrrolidin-3-yl]-2-methyl-benzamide;
4-[[3-(2,3-difluoro-4-methoxy-phenyl)imidazo[1,2-a]pyrazin-8-yl]amino]-N-[(3R)-1-[(2S,4R)-4-hydroxypyrrolidine-2-carbonyl]pyrrolidin-3-yl]-2-methyl-benzamide;
(2S,4R)-N-[4-[[4-[[3-(2,3-difluoro-4-methoxy-phenyl)imidazo[1,2-a]pyrazin-8-yl]amino]-2-methyl-benzoyl]amino]cyclohexyl]-4-hydroxy-pyrrolidine-2-carboxamide;
N-(3-amino-1-bicyclo[1.1.1]pentanyl)-4-[[3-(2,3-difluoro-4-methoxy-phenyl)imidazo[1,2-a] pyrazin-8-yl] amino]-2-ethyl-benzamide;
4-[[3-(2,3-difluoro-4-methoxy-phenyl)imidazo[1,2-a]pyrazin-8-yl]amino]-2-methyl-N-[4-(2-methylpropanoylamino)cyclohexyl]benzamide;
4-[[3-(2,3-difluoro-4-methoxy-phenyl)imidazo[1,2-a]pyrazin-8-yl]amino]-2-methyl-N-[1-[rac-(2S,4R)-4-hydroxypyrrolidine-2-carbonyl]-3-piperidyl]benzamide;
4-[[3-(2,3-difluoro-4-methoxy-phenyl)imidazo[1,2-a]pyrazin-8-yl]amino]-2-ethyl-N-[rac-(1R,2R)-2-aminocyclopropyl]benzamide;
N-(3-aminocyclobutyl)-4-[[3-(2,3-difluoro-4-methoxy-phenyl)imidazo[1,2-a]pyrazin-8-yl] amino]-2-methyl-benzamide;
N-(azetidin-3-yl)-4-[[3-(2,3-difluoro-4-methoxy-phenyl)imidazo[1,2-a]pyrazin-8-yl]amino]-2-methyl-benzamide;
4-[[3-(2,3-difluoro-4-methoxy-phenyl)imidazo[1,2-a]pyrazin-8-yl]amino]-2-methyl-N-[(3S)-pyrrolidin-3-yl] benzamide;
4-[[3-(2,3-difluoro-4-methoxy-phenyl)imidazo[1,2-a]pyrazin-8-yl]amino]-2-methyl-N-[rac-(1S,3S)-3-aminocyclopentyl]benzamide;
(2S,4R)-N-[(1R,2S)-2-[[4-[[3-(2,3-difluoro-4-methoxy-phenyl)imidazo[1,2-a]pyrazin-8-yl]amino]-2-ethyl-benzoyl]amino]cyclopentyl]-4-hydroxy-pyrrolidine-2-carboxamide;
N-(4-aminocyclohexyl)-4-[[3-(2,3-difluoro-4-methoxy-phenyl)imidazo[1,2-a]pyrazin-8-yl] amino]-2-methyl-benzamide;
4-[[3-(2,3-difluoro-4-methoxy-phenyl)imidazo[1,2-a]pyrazin-8-yl]amino]-2-methyl-N-(3-piperidyl)benzamide;
4-[[3-(2,3-difluoro-4-methoxy-phenyl)imidazo[1,2-a]pyrazin-8-yl]amino]-2-methyl-N-(4-piperidyl)benzamide;
(2S,4R)-N-[2-[[4-[[3-(2,3-difluoro-4-methoxy-phenyl)imidazo[1,2-a]pyrazin-8-yl]amino]-2-ethyl-benzoyl]amino]cyclohexyl]-4-hydroxy-pyrrolidine-2-carboxamide;
4-[[3-(2,3-difluoro-4-methoxy-phenyl)imidazo[1,2-a]pyrazin-8-yl]amino]-2-ethyl-N-[1-[rac-(2S)-2-amino-5-guanidino-pentanoyl]-3-piperidyl]benzamide;
N-[(3R)-1-[(2S)-2-amino-5-guanidino-pentanoyl]pyrrolidin-3-yl]-4-[[3-(2,3-difluoro-4-methoxyphenyl)imidazo[1,2-a]pyrazin-8-yl]amino]-2-ethyl-benzamide;
4-[[3-(2,3-difluoro-4-methoxy-phenyl)imidazo[l,2-a]pyrazin-8-yl]amino]-2-ethyl-N-[3-[[rac-(2S)-2-amino-5 -guanidino-pentanoyl] amino] cyclopentyl] benzamide;
N-[4-[[(2S)-2-amino-5-guanidino-pentanoyl]amino]cyclohexyl]-4-[[3-(2,3-difluoro-4-methoxyphenyl)imidazo[l,2-a]pyrazin-8-yl]amino]-2-ethyl-benzamide;
N-[1-[(2S)-2-amino-5-guanidino-pentanoyl]-4-piperidyl]-4-[[3-(2,3-difluoro-4-methoxyphenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-[rac-(1S,3S)-3-aminocyclopentyl]benzamide;
N-(4-aminocyclohexyl)-4-[[3-(2,3-difluoro-4-methoxy-phenyl)imidazo[1,2-a]pyrazin-8-yl] amino]-2-ethyl-benzamide;
4-[[3-(2,3-difluoro-4-methoxy-phenyl)imidazo[1,2-a]pyrazin-8-yl]amino]-2-ethyl-N-(3-piperidyl)benzamide; and
N-[3-(aminomethyl)oxetan-3-yl]-4-[[3-(2,3-difluoro-4-methoxy-phenyl)imidazo[1,2-a]pyrazin-8-yl] amino]-2-ethyl-benzamide.

In a preferred embodiment, the present invention provides a compound of formula (I) as described herein, or a pharmaceutically acceptable salt thereof, wherein the compound of formula (I) is selected from:
4-[[3-(2,3-difluoro-4-methoxy-phenyl)imidazo[1,2-a]pyrazin-8-yl]amino]-N-[1-[(2S,4R)-4-hydroxypyrrolidine-2-carbonyl]-4-piperidyl]-2-methyl-benzamide;
rac-(2S,4R)-N-[3-[[4-[[3-(2,3-difluoro-4-methoxy-phenyl)imidazo[1,2-a]pyrazin-8-yl]amino]-2-methyl-benzoyl]amino]cyclopentyl]-4-hydroxy-pyrrolidine-2-carboxamide;
N-(3-aminocyclobutyl)-4-[[3-(2,3-difluoro-4-methoxy-phenyl)imidazo[1,2-a]pyrazin-8-yl]amino]-2-ethyl-benzamide; and
4-[[3-(2,3-difluoro-4-methoxy-phenyl)imidazo[1,2-a]pyrazin-8-yl]amino]-N-[1-[(ethanimidoylamino)methyl]cyclopropyl]-2-ethyl-benzamide.

In one embodiment, the present invention provides pharmaceutically acceptable salts of the compounds of formula (I) as described herein, especially pharmaceutically acceptable salts selected from hydrochlorides, fumarates, lactates (in particular derived from L-(+)-lactic acid), tartrates (in particular derived from L-(+)-tartaric acid) and trifluoroacetates. In yet a further particular embodiment, the present invention provides compounds according to formula (I) as described herein (i.e., as "free bases" or "free acids", respectively).

In some embodiments, the compounds of formula (I) are isotopically-labeled by having one or more atoms therein replaced by an atom having a different atomic mass or mass number. Such isotopically-labeled (i.e., radiolabeled) compounds of formula (I) are considered to be within the scope of this disclosure. Examples of isotopes that can be incorporated into the compounds of formula (I) include isotopes of hydrogen, carbon, nitrogen, oxygen, phosphorous, sulfur, fluorine, chlorine, and iodine, such as, but not limited to, ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Certain isotopically-labeled compounds of formula (I), for example, those incorporating a radioactive isotope, are useful in drug and/or substrate tissue distribution studies. The radioactive isotopes tritium, i.e. ³H, and carbon-14, i.e., ¹⁴C, are particularly useful for this purpose in view of their ease of incorporation and ready means of detection. For example, a compound of formula (I) can be enriched with 1, 2, 5, 10, 25, 50, 75, 90, 95, or 99 percent of a given isotope.

Substitution with heavier isotopes such as deuterium, i.e. ²H, may afford certain therapeutic advantages resulting from greater metabolic stability, for example, increased *in vivo* half-life or reduced dosage requirements.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of formula (I) can generally be prepared by conventional techniques known to those skilled in the art or by processes analogous to those described in the Examples as set out below using an appropriate isotopically-labeled reagent in place of the nonlabeled reagent previously employed.

### Processes of Manufacturing

The preparation of compounds of formula (I) of the present invention may be carried out in sequential or convergent synthetic routes. Syntheses of the compounds of the invention are shown in the following schemes. The skills required for carrying out the reactions and purifications of the resulting products are known to those skilled in the art. The substituents and indices used in the following description of the processes have the significance given herein before unless indicated to the contrary. In more detail, the compounds of formula (I) can be manufactured by the methods given below, by the methods given in the examples or by analogous methods. Appropriate reaction conditions for the individual reaction steps are known to a person skilled in the art. Also, for reaction conditions described in literature affecting the described reactions see for example: Comprehensive Organic Transformations: A Guide to Functional Group Preparations, 3rd Edition, Richard C. Larock. John Wiley & Sons, New York, NY. 2018*).* We find it convenient to carry out the reactions in the presence or absence of a solvent. There is no particular restriction on the nature of the solvent to be employed, provided that it has no adverse effect on the reaction or the reagents involved and that it can dissolve the reagents, at least to some extent. The described reactions can take place over a wide range of temperatures, and the precise reaction temperature is not critical to the invention. It is convenient to carry out the described reactions in a temperature range between -78 °C to reflux temperature. The time required for the reaction may also vary widely, depending on many factors, notably the reaction temperature and the nature of the reagents. However, a period of from 0.5 h to several days will usually suffice to yield the described intermediates and compounds. The reaction sequence is not limited to the one displayed in the schemes, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

The synthesis of the compound of formula (I) may, for example, be accomplished according to the general synthesis outlined in the following Scheme 1. 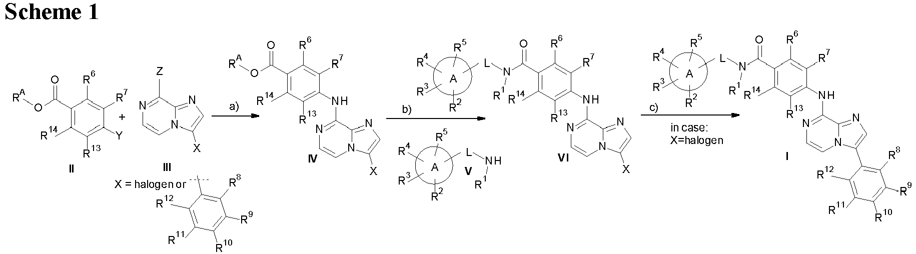
a) Acids or esters **II,** wherein Y is NH₂ or halogen and R^{A} is H or alkyl, are commercially available, can be accessed by methods known in the art or in literature and can conveniently be reacted with imidazopyrazine derivatives **III** to access intermediates **IV.** Depending on the varying substitution **(II:** Y= NH₂ or halogen) it is convenient to react acids / esters **II** with the appropriate imidazopyrazine derivative **III** (Z= NH₂ or halogen and X= halogen or appropriately substituted aryl moiety) under metal catalysis reaction conditions or nucleophilic aromatic substitution reaction conditions (as appropriate) to yield acids / esters **IV.**
b) Acid derivatives **IV** (R^{A} = H), can be accessed from esters **IV** (R^{A} = alkyl) upon saponification in the presence of a base. Examples of bases include: LiOH, NaOH and the like. Acid derivatives **IV** are conveniently reacted with an amine **V** under varying coupling reaction conditions (coupling reaction conditions include: HATU, TBTU, and the like in the presence of a base, such as DIPEA, NEt₃, and the like) to afford amides **VI.** Amines **V** (and their protected congeners) are commercially available, known in the art or prepared according to methods known in the art. In case X = appropriately substituted aryl ring, these derivatives **VI** might be the final desired imidazopyrazines derivatives **I,** or any protecting group might have to be cleaved under appropriate conditions to afford final imidazopyrazines derivatives **I.** These imidazopyrazines **I** might be the final desired compounds however might be further derivatised to yield final imidazopyrazines derivatives **I.**
c) Amides **VI** wherein X is a halogen are conveniently reacted under metal catalysis (catalysts include: PdCl₂(dppf)-CH₂Cl₂ adduct, Pd(PPh₃)₄, and the like and in the presence of a base, such as K₃PO₄, NaOtBu, and the like) with the appropriate boronic acid or ester to afford imidazopyrazines derivatives **I.** These imidazopyrazines derivatives **I** might be the final desired compounds however any protecting group will have to be cleaved under appropriate conditions to afford final imidazopyrazines **I.** These imidazopyrazines **I** might be the final desired compounds however might be further derivatised to yield final imidazopyrazines derivatives **I.**

In one aspect, the present invention provides a process of manufacturing the compounds of formula (I) described herein, comprising:
(i) reacting a carboxylic acid **IVa,** wherein R⁶ to R¹⁴ are as defined herein, 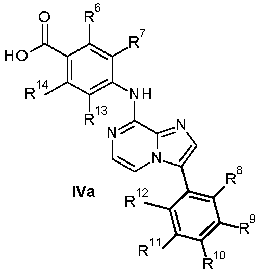 with an amine **V,** wherein A, L and R¹ to R⁵ are as defined herein, 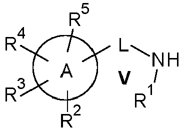 in the presence of a coupling reagent (such as HATU, TBTU, and the like) and a base (such as DIPEA, NEt₃, and the like), to form said compound of formula (I); or
(ii) reacting a compound **VI,** wherein R¹ to R⁷, R¹³, R¹⁴, L and A are as defined herein and X is halogen, 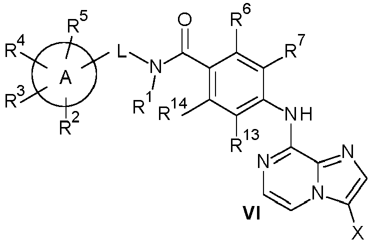 with a boronic acid **VII,** wherein R⁸ to R¹² are as defined herein and Y is a boronic acid or a boronic acid ester, 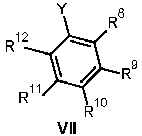 in the presence of a transition metal catalyst (such as PdCl₂(dppf)-CH₂Cl₂ adduct, Pd(PPh₃)₄, and the like) and a base (such as K₃PO₄, NaOtBu, and the like) to form said compound of formula (I).

In a further aspect, the present invention provides a compound of formula (I) as described herein, or a pharmaceutically acceptable salt thereof, when manufactured according to the processes disclosed herein.

### Using the Compounds of the Invention

As illustrated in the experimental section, the compounds of formula (I) and their pharmaceutically acceptable salts possess valuable pharmacological properties for the treatment or prevention of infections and resulting diseases, particularly bacteremia, pneumonia, meningitis, urinary tract infection, and wound infection, caused by pathogens, particularly by bacteria, more particularly by *Acinetobacter* species, most particularly by *Acinetobacter baumannii .*

The compounds of formula (I) and their pharmaceutically acceptable salts exhibit activity as antibiotics, particularly as antibiotics against *Acinetobacter* species, more particularly as antibiotics against *Acinetobacter baumannii,* most particularly as pathogen-specific antibiotics against *Acinetobacter baumannii.*

The compounds of formula (I) and their pharmaceutically acceptable salts can be used as antibiotics, i.e. as antibacterial pharmaceutical ingredients suitable in the treatment and prevention of bacterial infections, particularly in the treatment and prevention of bacterial infections caused by *Acinetobacter* species, more particularly in the treatment and prevention of bacterial infections caused by *Acinetobacter baumannii.*

The compounds of the present invention can be used, either alone or in combination with other drugs, for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In one aspect, the present invention provides compounds of formula (I) or their pharmaceutically acceptable salts as described herein for use as therapeutically active substances.

In a further aspect, the present invention provides a compound of formula (I) as described herein, or a pharmaceutically acceptable salt thereof, for use as antibiotic.

In a further aspect, the present invention provides a compound of formula (I) as described herein, or a pharmaceutically acceptable salt thereof, for use in the treatment or prevention of nosocomial infections and resulting diseases.

In a particular embodiment, said nosocomial infections and resulting diseases are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by Gram-negative bacteria.

In a particular embodiment, said infections and resulting diseases caused by Gram-negative bacteria are selected from bacteremia, pneumonia, meningitis, urinary tract infection and wound infection, or a combination thereof.

In a further aspect, the present invention provides a compound of formula (I) as described herein, or a pharmaceutically acceptable salt thereof, for use in the treatment or prevention of infections and resulting diseases caused by ***E**nterococcus faecium, **S**taphylococcus aureus, **K**lebsiella pneumoniae, **A**cinetobacter baumannii, **P**seudomonas aeruginosa, **E**nterobacter species* or E. *coli,* or a combination therof.

In a further aspect, the present invention provides compounds of formula (I) or their pharmaceutically acceptable salts as defined above for use in the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

In a further aspect, the present invention provides the use of compounds of formula (I) or their pharmaceutically acceptable salts as defined above for the preparation of medicaments for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.* Such medicaments comprise compounds of formula (I) or their pharmaceutically acceptable salts as defined above.

### Pharmaceutical Compositions and Administration

In one aspect, the present invention provides pharmaceutical compositions comprising compounds of formula (I) or their pharmaceutically acceptable salts as defined above and one or more pharmaceutically acceptable excipients. Exemplary pharmaceutical compositions are described in Examples 75 to 78.

In a further aspect, the present invention relates to pharmaceutical compositions comprising compounds of formula (I) or their pharmaceutically acceptable salts as defined above and one or more pharmaceutically acceptable excipients for the treatment or prevention of infections and resulting diseases, particularly bacteremia, pneumonia, meningitis, urinary tract infection, and wound infection, caused by pathogens, particularly by bacteria, more particularly caused by *Acinetobacter* species, most particularly by *Acinetobacter baumannii.*

The compounds of formula (I) and their pharmaceutically acceptable salts can be used as medicaments (e.g. in the form of pharmaceutical preparations). The pharmaceutical preparations can be administered internally, such as orally (e.g. in the form of tablets, coated tablets, dragées, hard and soft gelatin capsules, solutions, emulsions or suspensions), nasally (e.g. in the form of nasal sprays) or rectally (e.g. in the form of suppositories). However, the administration can also be effected parentally, such as intramuscularly or intravenously (e.g. in the form of injection solutions or infusion solutions).

The compounds of formula (I) and their pharmaceutically acceptable salts can be processed with pharmaceutically inert, inorganic or organic excipients for the production of tablets, coated tablets, dragées and hard gelatin capsules. Lactose, corn starch or derivatives thereof, talc, stearic acid or its salts etc. can be used, for example, as such excipients for tablets, dragées and hard gelatin capsules.

Suitable excipients for soft gelatin capsules are, for example, vegetable oils, waxes, fats, semisolid substances and liquid polyols, etc.

Suitable excipients for the production of solutions and syrups are, for example, water, polyols, saccharose, invert sugar, glucose, etc.

Suitable excipients for injection solutions are, for example, water, alcohols, polyols, glycerol, vegetable oils, etc.

Suitable excipients for suppositories are, for example, natural or hardened oils, waxes, fats, semisolid or liquid polyols, etc.

Moreover, the pharmaceutical preparations can contain preservatives, solubilizers, viscosityincreasing substances, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary in wide limits and will, of course, be fitted to the individual requirements in each particular case. In general, in the case of oral administration a daily dosage of about 0.1 mg to 20 mg per kg body weight, preferably about 0.5 mg to 4 mg per kg body weight (e.g. about 300 mg per person), divided into preferably 1-3 individual doses, which can consist, for example, of the same amounts, should be appropriate. It will, however, be clear that the upper limit given herein can be exceeded when this is shown to be indicated.

### Examples

The invention will be more fully understood by reference to the following examples. The claims should not, however, be construed as limited to the scope of the examples.

In case the preparative examples are obtained as a mixture of enantiomers, the pure enantiomers can be separated by methods described herein or by methods known to the man skilled in the art, such as e.g., chiral chromatography (e.g., chiral SFC) or crystallization.

All reaction examples and intermediates were prepared under an argon atmosphere if not specified otherwise.

The following abbreviations are used in the present text:
(R)-BINAP = (R)-2,2'-bis(diphenylphosphino)-1,1'-binaphthyl, ACN = acetonitrile, aq. = aqueous, Boc = tert-butyloxycarbonyl, Boc-Glu-OtBu = Boc-L-glutamic acid 1-tert-butyl ester, Boc-Glu(OtBu)-OH = N-α-t.-Boc-L-glutamic acid γ-t.-butyl ester, Boc-Orn(Z)-OH = Na-Boc-Nδ-Cbz-L-ornithine, Nα-Boc-Nδ-Z-L-ornithine, Nδ-Z-Nα-Boc-L-ornithine, BrettPhos-Pd-G3 = [(2-Di-cyclohexylphosphino-3,6-dimethoxy-2',4',6'- triisopropyl-1,1'-biphenyl)-2-(2'-amino-1,1' -biphenyl)]palladium(II) methanesulfonate methanesulfonate, CAS = chemical abstracts registration number, Cs₂CO₃ = cesium carbonate, DCM = dichloromethane, DIAD = diisopropyl azodicarboxylate, DIPEA = ethyl diisopropylamine, DMA = N,N-dimethylacetamide, DMAP = 4-(dimethylamino)-pyridine, DMF = N,N-dimethylformamide, DMSO = dimethylsulfoxide, DMSO-d6 = deuterated dimethylsulfoxide, EA = ethyl acetate, EDC = 1-ethyl-3-(3-dimethylaminopropyl)carbodiimide, EDCI = 1-ethyl-3-(3-dimethylaminopropyl)carbodiimide, EI = electron impact, ESI = electrospray ionization, ESI⁺ = electrospray ionization positive (mode), ESP = electrospray ionization positive (mode), Et₂O = diethylether, Et₃N = triethylamine, EtOAc = ethyl acetate, EtOH = ethanol, FA = formic acid, Fmoc-Agp(Boc)2-OH = N-α-Fmoc-N,NÆ-γ-di-t.-butoxycarbonyl-L-diaminobutanoic acid, Fmoc-Arg(Boc)2-OH = N-α-Fmoc-N-ω,N-ωÆ-bis-t-butoxycarbonyl-L-arginine, H₂ = hydrogen, h = hour(s), HATU = 1-[bis(dimethylamino)methylene]-1H-1,2,3-triazolo[4,5-b]pyridinium-3-oxide hexafluorophosphate, HCl = hydrochloric acid, HFIP = 1,1,1,3,3,3-hexafluoroisopropanol, H₂O = water, HOBt = 1-hydroxy-1H-benzotriazole, HPLC = high performance liquid chromatography, HV = high vacuum, ISN = ion spray negative (mode), K₂CO₃ = potassium carbonate, KI = potassium iodide, KOH = potassium hydroxide, K₃PO₄ = potassium phosphate tribasic, LC-MS = liquid chromatography coupled with mass spectroscopy, LiOH = lithium hydroxide, MeOH = methanol, MgSO₄ = magnesium sulphate, min = minute(s), mL = milliliter, MS = mass spectrometry, MTBE = tert.-butyl methyl ether, N₂ = nitrogen, Na₂CO₃ = sodium carbonate, Na₂SO₃,= sodium sulfite, Na₂SO₄ = sodium sulfate, Na₂S₂O₃ = sodium thiosulfate, NEt₃ = triethylamine, NaHCO₃ = sodium hydrogen carbonate, NaOH = sodium hydroxide, NH₄Cl = ammonium chloride, NiCl₂.6H₂O = nickel(II)chloride hexahydrate, NMO = N-methylmorpholine N-oxide, NMP = N-methyl-2-pyrrolidone, Pd/C = palladium on activated carbon, Pd₂(dba)₃ = tris(dibenzylideneacetone)dipalladium(0), PdCl₂(PPh₃)₂ = bis(triphenylphosphine)palladium(II) dichloride, Pd(dppf)Cl₂ = [1,1'-bis(diphenylphosphino)ferrocene]dichloropalladium(II), PdCl₂(dppf)-CH₂Cl₂ = [1,1'-bis(diphenylphosphino)ferrocene]dichloropalladium(II) dichloromethane complex, PE = petroleum ether, PhI(OAc)₂ = (diacetoxyiodo)benzene, PPA = polyphosphoric acid, pTsOH = para toluenesulfonic acid, Rf = retention factor, RM = reaction mixture, RT = room temperature, SOCl₂ = thionyl chloride, SFC = supercritical fluid chromatography, TBTU = 2-(1H-Benzotriazole-1-yl)-1,1,3,3-tetramethylaminium tetrafluoroborate, T₃P = propylphosphonic anhydride, t-Bu-X-phos = 2-di-tert-butylphosphino-2',4',6'-triisopropylbiphenyl, TEA = triethylamine, TEMPO = (2,2,6,6-tetramethylpiperidin-1-yl)oxyl, TFA = trifluoroacetic acid, THF = tetrahydrofurane, prep-TLC = preparative thin layer chromatography, UV = ultraviolet.

### Intermediate 1

### 4-((3-Iodoimidazo[1,2-a]pyrazin-8-yl)amino)-2-methylbenzoic acid

A mixture of 8-chloro-3-iodoimidazo[1,2-a]pyrazine (100 mg, 358 µmol) and 4-amino-2-methylbenzoic acid (108 mg, 716 µmol) in 1,4-dioxane (2 mL) and acetic acid (2 mL) was stirred at 90 °C for 48 h. The mixture was allowed to cool to room temperature and filtered. The residue was washed with diethyl ether and dried *in vacuo* to give the title compound (139 mg) as a white solid. MS (ESI, m/z): 395.1 [M+H]⁺.

### Intermediate 2

### 2-Chloro-4-((3-(3-fluoro-4-methoxyphenyl)imid azo [1,2-a] pyrazin-8-yl)amino)benzoic acid

To 8-chloro-3-(3-fluoro-4-methoxyphenyl)imidazo[1,2-a]pyrazine (Intermediate 3, 50 mg, 180 µmol) in acetonitrile (0.9 mL) and acetic acid (100 µL) was added 4-amino-2-chlorobenzoic acid (46.3 mg, 270 µmol), followed by stirring at 80 °C overnight. The reaction mixture was filtered to give the title compound (70 mg) as a light brown solid. MS (ESI, m/z): 411.3 [M-H]⁻.

The following intermediates were prepared in analogy:

| Int. | Name | MS ESI [M+H]⁺ | Starting Material |
|---|---|---|---|
| 4 | 4-((3-(4-methoxyphenyl)imidazo[1,2-a]pyrazin-8-yl)amino)-2-methylbenzoic acid | 375.2 | 4-amino-2-methylbenzoic acid and Intermediate 5 |
| 6 | 4-((3-(3-fluoro-4-methoxyphenyl)imidazo[1,2-a]pyrazin-8-yl)amino)-2-methylbenzoic acid | 393.3 | 4-amino-2-methylbenzoic acid and Intermediate 3 |
| 7 | 4-((3 -(4-(difluoromethoxy)phenyl)imidazo [1,2-a]pyrazin-8-yl)amino)-2-methylbenzoic acid | 411 | 4-amino-2-methylbenzoic acid and Intermediate 8 |
| 9 | 2-chloro-4-[[3-(4-methoxyphenyl)imidazo[1,2-a]pyrazin-8-yl]amino]benzoic acid | 395.2 | 4-amino-2-chlorobenzoic acid and Intermediate 5 |
| 10 | 2-bromo-4-((3-(4-(difluoromethoxy)phenyl)imidazo[1,2-a]pyrazin-8-yl)amino)benzoic acid | 475.1 | 4-amino-2-bromobenzoic acid and Intermediate 8 |
| 11 | 2-chloro-4-((3-(4-(difluoromethoxy)phenyl)imidazo[1,2-a]pyrazin-8-yl)amino)benzoic acid | 431.2 | 4-amino-2-chlorobenzoic acid and Intermediate 8 |
| 12 | 4-((3-(4-(difluoromethoxy)phenyl)imidazo [1,2-a]pyrazin-8-yl)amino)-2-ethylbenzoic acid | 425.1 | methyl 4-amino-2-ethylbenzoate and Intermediate 13 followed by ester hydrolysis |
| 14 | 4-((3-(3-chloro-4-methoxyphenyl)imidazo[1,2-a]pyrazin-8-yl)amino)-2-methylbenzoic acid | 409.3 | methyl 4-amino-2-methylbenzoate and Intermediate 15 followed by ester hydrolysis |
| 16 | 4-((3-(3-chloro-4-methoxyphenyl)imidazo[1,2-a]pyrazin-8-yl)amino)benzoic acid | 395.1 | 4-aminobenzoic acid and Intermediate 15 |
| 17 | 4-((3-(4-methoxyphenyl)imidazo[1,2-a]pyrazin-8-yl)amino)benzoic acid | 360 | 4-aminobenzoic acid and Intermediate 5 |
| 18 | 4-((3 -(4-(difluoromethoxy)phenyl)imidazo [1,2-a]pyrazin-8-yl)amino)-2-(trifluoromethyl)benzoic acid | 465.2 | 4-amino-2-(trifluoromethyl)benz oic acid and Intermediate 8 |
| 19 | 4-((3 -(4-(difluoromethoxy)phenyl)imidazo [1,2-a] pyrazin-8-yl) amino)-2-nitrobenzoic acid | 442.2 | 4-amino-2-nitrobenzoic acid and Intermediate 8 |
| 20 | 2-chloro-4-[[3-(2,3-difluoro-4-methoxyphenyl)imidazo[1,2-a]pyrazin-8-yl]amino]benzoic acid | 431 | 4-amino-2-chlorobenzoic acid and Intermediate 21 |
| 22 | 2-chloro-4-[[3-[4-(difluoromethoxy)-2,3-difluoro-phenyl]imidazo[1,2-a]pyrazin-8-yl]amino]benzoic acid | 467.1 | 4-amino-2-chlorobenzoic acid and Intermediate 23 |
| 24 | 2-chloro-4-[[3-[2-chloro-4-(cyanomethoxy)-3-fluoro-phenyl]imidazo[1,2-a]pyrazin-8-yl]amino]benzoic acid | 471.2 | 4-amino-2-chlorobenzoic acid and Intermediate 25 |
| 26 | 4-[[3-(2-chloro-5-fluoro-4-methoxyphenyl)imidazo[1,2-a]pyrazin-8-yl]amino]-2-methyl-benzoic acid | 427.1 | methyl 4-amino-2-methylbenzoate and Intermediate 27 |
| 28 | 4-[[3 -(2,3-difluoro-4- methoxy - phenyl)imidazo[1,2-a]pyrazin-8-yl]amino]-2-methyl-benzoic acid | 411.0 | 4-amino-2-methylbenzoic acid and Intermediate 21 |
| 20 | 2-chloro-4-[[3-(2,3-difluoro-4-methoxyphenyl)imidazo[1,2-a]pyrazin-8-yl]amino]benzoic acid | 431 | 4-amino-2-chlorobenzoic acid and Intermediate 21 |
| 29 | 2-chloro-4-[[3-[4-(cyanomethoxy)-2,3-difluorophenyl]imidazo[1,2-a]pyrazin-8-yl]amino]benzoic acid | 456.1 | 4-amino-2-chlorobenzoic acid and Intermediate 30 |
| 31 | 4-[[3-[4-(cyanomethoxy)-2,3-difluorophenyl]imidazo[1,2-a]pyrazin-8-yl]amino]-2-methyl-benzoic acid | 436.1 | Intermediate 30 and 4-amino-2-methylbenzoic acid |
| 32 | 4-[[3-[4-(cyanomethoxy)-2,3-difluorophenyl]imidazo[1,2-a]pyrazin-8-yl]amino]-2-ethyl-benzoic acid | 450.1 | Intermediate 33 and Intermediate 30 |
| 34 | 4-((3-(2-chloro-3-fluoro-4-methoxyphenyl)imidazo[1,2-a]pyrazin-8-yl)amino)-2-methylbenzoic acid | 427.2 | Intermediate 1 and 2-(2-chloro-3-fluoro-4-methox y-phenyl)-4,4,5,5-tetramethyl-1,3,2-dioxaborolane |
| 35 | 2-chloro-4-[[3-[3-chloro-4-(cyanomethoxy)-2-fluoro-phenyl]imidazo[1,2-a]pyrazin-8-yl]amino]benzoic acid | 472.0 | Intermediate 36 and 4-amino-2-chlorobenzoic acid |
| 37 | 2-chloro-4-[[3-[5-chloro-4-(cyanomethoxy)-2-fluoro-phenyl]imidazo[1,2-a]pyrazin-8-yl]amino]benzoic acid | 471.9 | Intermediate 38 and 4-amino-2-chlorobenzoic acid |
| 39 | 4-[[3-[5-chloro-4-(cyanomethoxy)-2-fluorophenyl]imidazo[1,2-a]pyrazin-8-yl] amino] -2-methyl-benzoic acid | 452.1 | Intermediate 38 and 4-amino-2-methylbenzoic acid |
| 40 | 4-[[3-[2-chloro-4-(cyanomethoxy)-3-fluorophenyl]imidazo[1,2-a]pyrazin-8-yl]amino]-2-ethyl-benzoic acid | 466.1 | Intermediate 41 and Intermediate 33 |
| 42 | methyl 2-ethyl-4-[(3-iodoimidazo[l,2-a]pyrazin-8-yl)amino]benzoate | 423.2 | 8-chloro-3-iodoimidazo[1,2-a]pyrazine and methyl 4-amino-2-ethyl-benzoate (CAS No 1211589-24-0) |
| 43 | 4-((3-(4-(difluoromethoxy)-3-fluorophenyl)imidazo[1,2-a]pyrazin-8-yl)amino)-2-methylbenzoic acid | 429.2 | Intermediate 44 and 4-amino-2-methylbenzoic acid |
| 45 | 2 -cyano- 4 - ((3-(4-(difluoromethoxy)phenyl)imidazo[1,2-a]pyrazin-8-yl)amino)benzoic acid | 422.2 | methyl 4-amino-2-cyanobenzoate and Intermediate 8 followed by ester hydrolysis with LiOH |
| 46 | 4-((3 -(4-(difluoromethoxy)phenyl)imidazo [1,2-a]pyrazin-8-yl)amino)-2-iodobenzoic acid | 523.1 | methyl 4-amino-2-iodobenzoate and Intermediate 8 followed by ester hydrolysis with LiOH |
| 47 | 4-((3-(4-(difluoromethoxy)phenyl)imidazo[1,2-a]pyrazin-8-yl)amino)-2-vinylbenzoic acid | 423.1 | Intermediate 48 and Intermediate 8 followed by ester hydrolysis with LiOH |
| 49 | methyl 4-((3-iodoimidazo[1,2-a]pyrazin-8-yl)amino)-2-methylbenzoate | 409.1 | From 8-chloro-3-iodoimidazo[1,2-a]pyrazine and methyl 4-amino-2-methyl benzoate |

### Intermediate 3

### 8-Chloro-3-(3-fluoro-4-methoxyphenyl)imidazo[1,2-a]pyrazine

To 8-chloro-3-iodoimidazo[1,2-a]pyrazine (500 mg, 1.79 mmol) in dioxane (6.5 mL) and water (3.25 mL) was added 2-(3-fluoro-4-methoxyphenyl)-4,4,5,5-tetramethyl-1,3,2-dioxaborolane (474 mg, 1.88 mmol), 1,1'-bis(diphenylphosphino)ferrocenedichloro palladium(II) dichloromethane complex (65.5 mg, 89.5 µmol) and sodium carbonate (379 mg, 3.58 mmol, Eq: 2) followed by stirring at 50 °C for 2 d. The reaction mixture was partitioned between ethyl acetate and water. The organic layers were dried over Na₂SO₄, filtered and concentrated to give a red solid, which was purified by column chromatography (silica gel, DCM/MeOH, 0-5%) to give the title compound (359 mg) as a pink-brown solid. MS (ESI, m/z): 278.1 [M+H]⁺.

The following intermediates were prepared in analogy to Intermediate 3:

| Int. | Name | ESI MS [M+H]⁺ | Starting Material |
|---|---|---|---|
| 5 | 8-Chloro-3-(4-methoxyphenyl)imidazo[1,2-a]pyrazine | 260.1 | (4-methoxyphenyl) boronic acid |
| 8 | 8-chloro-3-(4-(difluoromethoxy)phenyl)imidazo[1,2-a]pyrazine | 296 | 2-(4-(difluoromethoxy)p henyl)-4,4,5,5-tetramethyl-1,3,2-dioxaborolane |
| 50 | 4-(8-chloroimidazo[1,2-a]pyrazin-3-yl)phenol | 246.0 | 4-hydroxyphenylboro nic acid |
| 13 | 8-chloro-3-(4-chloro-2,3-difluorophenyl)imidazo[1,2-a]pyrazine | 299.9 | (4-chloro-2,3-difluorophenyl)boro nic acid |
| 15 | 8-chloro-3-(3-chloro-4-methoxyphenyl)imidazo[1,2-a]pyrazine | 294.1 | (3-chloro-4-methoxyphenyl) bor onic acid |
| 51 | 8-chloro-3-(2-chloro-4-methoxyphenyl)imidazo[1,2-a]pyrazine | 293.1 | (2-chloro-4-methoxyphenyl)boronic acid |
| 21 | 8-chloro-3-(2,3-difluoro-4-methoxyphenyl)imidazo[1,2-a]pyrazine | 296.0 | (2,3-difluoro-4-methoxyphenyl) bor onic acid |
| 30 | 2-[4-(8-chloroimidazo[1,2-a]pyrazin-3-yl)-2,3-difluoro-phenoxy] acetonitrile | 321.0 | Intermediate 52 |
| 36 | 2-[2-chloro-4-(8-chloroimidazo[1,2-a]pyrazin-3-yl)-3-fluoro-phenoxy] acetonitrile | 337.0 | Intermediate 53 |
| 38 | 2-[5-chloro-4-(8-chloroimidazo[1,2-a]pyrazin-3-yl)-2-fluoro-phenoxy] acetonitrile | 337.0 | Intermediate 53 |
| 44 | 8-chloro-3-[4-(difluoromethoxy)-3-fluorophenyl]imidazo[1,2-a]pyrazine | 314.0 | 2-[4-(difluoromethoxy)-3-fluoro-phenyl]-4,4,5,5-tetramethyl-1,3,2-dioxaborolane |
| 25 | 2-[3-chloro-4-(8-chloroimidazo[1,2-a]pyrazin-3-yl)-2-fluoro-phenoxy] acetonitrile | 339.0 | 2-[3-chloro-2-fluoro-4-(4,4,5,5-tetramethyl-1,3,2-dioxaborolan-2-yl)phenoxy] acetonit rile |
| 54 | 4-(8-chloroimidazo[1,2-a]pyrazin-3-yl)-2,3-difluoro- phenol | 281.0 | (2,3-difluoro-4-hydroxyphenyl)bor onic acid |

### Intermediate 52

### 2-[2,3-difluoro-4-(4,4,5,5-tetramethyl-1,3,2-dioxaborolan-2-yl)phenoxy]acetonitrile

Step 1:

### 2-(4-bromo-2,3-difluoro-phenoxy)acetonitrile

To a solution of 4-bromo-2,3-difluorophenol (5.2 g, 25 mmol, Eq: 1), bromoacetonitrile (6.0 g, 50 mmol, Eq: 2) in DMF (25 mL) was added potassium carbonate (6.9 g, 50 mmol, Eq: 2) and then the resultant mixture was stirred overnight at room temperature.

The mixture was poured into water (50 mL) and the aqueous solution was extracted with ethyl acetate (100 mL×2). The organic layers were combined and washed with water and brine, dried over anhydrous Na₂SO₄ and concentrated under reduced pressure. The residue was purified by prep. HPLC to give the title compound (5.2 g, 84 % yield) as white solid.

MS (ESI, m/z): 248.0 [M+H]+.

### Step 2:

### 2-[2,3-difluoro-4-(4,4,5,5-tetramethyl-1,3,2-dioxaborolan-2-yl)phenoxy]acetonitrile

To a solution of 2-(4-bromo-2,3-difluorophenoxy)acetonitrile (6.2 g, 25 mmol, Eq: 1) in dioxane (50 mL) and was added (4,4,4',4',5,5,5',5'-octamethyl-2,2'-bi(1,3,2-dioxaborolane) (6.35 g, 25 mmol, Eq: 1), Pd(dppf)Cl2 (1.6g, 2 mmol, Eq: 0.08) and potassium acetate (4.9 g, 50 mmol, Eq: 2) and then the resultant mixture was degassed for 5 min with nitrogen and then stirred overnight at 80°C. After cooling to room temperature, the mixture was poured into water (100 mL) and the aqueous solution was extracted with ethyl acetate (100 mL×2). The organic layers were combined and washed with water and brine, dried over anhydrous Na₂SO₄and concentrated under reduced pressure to give a red oil which was purified by silica gel column chromatography to provide the desired compound (4 g, 54% yield) as an off-white solid.

### Intermediate 55

### 2-[3-chloro-2-fluoro-4-(4,4,5,5-tetramethyl-1,3,2-dioxaborolan-2-yl)phenoxy]acetonitrile

Prepared in analogy to Intermediate 52 starting from 4-bromo-3-chloro-2-fluoro-phenol [CAS#1360745-16-9].

### Intermediate 33

### 4-amino-2-ethyl-benzoic acid

### Step 1: methyl 4-nitro-2-vinyl-benzoate & ethyl 4-nitro-2-vinyl-benzoate

A mixture of methyl 2-bromo-4-nitro-benzoate (5.2 g, 20 mmol, 1 eq), 2,4,6-trivinylcyclotriboroxane pyridine complex (5.78 g, 24 mmol, 1.2 eq), tetrakis(triphenylphosphine)palladium(0) (1.16 g, 1 mmol, 0.050 eq) and potassium carbonate (11.05 g, 79.99 mmol, 4 eq) in toluene (50 mL) and ethanol (50 mL) was stirred at 90 °C under nitrogen for 2 h. The mixture was filtered over Celite. The filtrate was concentrated to dryness. To the crude was added water (50 mL). The mixture was extracted with ethyl acetate (50 mL × 3). The combined organic layers were concentrated to dryness. The crude was then purified by flash column chromatography eluting 10% ethyl acetate in petrol ether to afford methyl 4-nitro-2-vinyl-benzoate (1.58 g) as a brown oil.

### Step 2: ethyl 4-amino-2-ethyl-benzoate

A mixture of ethyl 4-nitro-2-vinyl-benzoate (392.0 mg, 1.77 mmol, 1 eq) and Pd/C (10%) (50.0 mg) in MeOH (10 mL) was stirred at 25 °C for 5 h under a hydrogen atmosphere. The mixture was filtered over Celite to afford ethyl 4-amino-2-ethyl-benzoate (331 mg, 1.71 mmol, 96.66% yield) as brown oil. MS (ESI⁺): 194.1 [(M+H)⁺].

### Step 3: 4-amino-2-ethyl-benzoic acid

To a solution of methyl 4-amino-2-ethylbenzoate (540 mg, 3.0 mmol) in THF (5 mL) and methanol (25 mL) was added 2.0 M LiOH (3.0 mL) aqueous solution. The resultant mixture was stirred for 15 h at room temperature and then acidified to pH=5-6 with 3.0 M hydrochloric acid.

The resulting suspension was filtered, the solid was washed with water and then dried to give the title compound (0.3 g, 60.5% yield) as a white solid

MS (ESI, m/z): 166.0 [M+H]+.

### Intermediate 56

### 2-(dimethylamino)-1-piperazin-1-yl-ethanone di-trifluoroacetate

### Step 1: tert-butyl 4-[2-(dimethylamino)acetyl]piperazine-1-carboxylate

To a solution of tert-butyl piperazine-1-carboxylate ( 500mg, 2.68 mmol) in DMF (20mL) was added dimethylglycine (277 mg, 2.68 mmol), triethylamine (815 mg, 1.12 mL, 8.05 mmol) and 1-propanephosphonic anhydride (1.71 g, 5.37 mmol,), the reaction was stirred for 20 minutes at room temperature. The reaction mixture was quenched with water and washed with brine. The mixture was extracted in DCM. The organic layer was concentrated in vacuum to give crude product (530 mg), which was used in the next step without further purification. MS (ESI, m/z): [Ms+1]⁺ 272

### Step 2: 2-(dimethylamino)-1-piperazin-1-yl-ethanone di-trifluoroacetate

A solution of tert-butyl 4-(dimethylglycyl)piperazine-1-carboxylate (530 mg) in DCM (5 mL) and TFA (5 mL) was stirred for one hour at room temperature. The reaction mixture was concentrated *in vacuo* to give the crude product (680 mg), which was used without further purification. MS (ESI, m/z): [M+H]⁺ 172

### Intermediate 57

### 4-(3-aminopropyl)piperazin-2-one

### Step 1:

### 2-[3-(3-oxopiperazin-1-yl)propyl]isoindoline-1,3-dione

A mixture of 3-(1,3-dioxoisoindolin-2-yl)propyl methanesulfonate (1.34 g, 5 mmol, Eq: 1), piperazin-2-one (600 mg, 6 mmol, Eq: 1.2) and potassium carbonate (1.38 g, 10 mmol, Eq: 2) in N,N-dimethylformamide (25 mL) was stirred at room temperature overnight. The mixture was diluted with H₂O and extracted with DCM. The DCM layer was dried and concentrated *in vacuo* to give a yellow oil, which was purified by flash column chromatography to provide the desired compound (1.2 g, 83.5 % yield) as a white solid. MS (ESI, m/z): 278.1 [M+H]+.

### Step 2:

### 4-(3-aminopropyl)piperazin-2-one

To a mixture of 2-(3-(3-oxopiperazin-1-yl)propyl)isoindoline-1,3-dione (1.15 g, 4 mmol) in EtOH (25 mL) was added hydrazine hydrate (2.0 mL) and then the mixture was stirred at room temperature overnight. The suspension was filtered and the filtrate was concentrated to give the title compound (0.5 g, 80 % yield) as a yellow oil. MS (ESI, m/z): 158.1 [M+H]⁺.

The following intermediates were prepared in analogy to intermediate 57

| Int. | Name | ESI MS [M+H]⁺ | Starting Material |
|---|---|---|---|
| 58 | 4-(3-aminopropyl)-1-methyl-piperazin-2-one | 172.1 | 1-methylpiperazin-2-one |
| 59 | tert-butyl 4-(3-aminopropyl)-3-oxo-piperazine-1-carboxylate | 258.1 | tert-butyl 3-oxopiperazine-1-carboxylate |
| 60 | 1-tert-butyl 2-methyl 4-(3-aminopropyl)piperazine-1,2-dicarboxylate | 302.2 | 1-tert-butyl 2-methyl piperazine-1,2-dicarboxylate |
| 61 | 2-(2-imidazol- 1 -ylethoxy)ethanamine | 156.1 | Intermediate 62 and imidazole |

### Intermediate 63

### 2-ethyl-4-[(3-iodoimidazo[1,2-a]pyrazin-8-yl)amino] benzoic acid

### Step 1: methyl 2-ethyl-4-[(3-iodoimidazo[1,2-a]pyrazin-8-yl)amino]benzoate

To a solution of 8-chloro-3-iodo-imidazo[1,2-a]pyrazine (6.0 g, 21.47 mmol, 1 eq) in ACN (60 mL) was added methyl 4-amino-2-ethyl-benzoate [CAS#1211589-24-0] (4.72 g, 26.31 mmol, 1.23 eq) and acetic acid (6.0 mL, 21.47 mmol, 1 eq). The reaction mixture was stirred at 80 °C for 60 h. After cooling to room temperature, the reaction mixture was filtered and washed with (ACN:MeOH=10:1, V:V), and then dried to provide methyl 2-ethyl-4-[(3-iodoimidazo[1,2-a]pyrazin-8-yl)amino]benzoate (9.37 g, crude) as off-white solid. ¹H NMR (400 MHz, DMSO-d6) δ 10.14 (br s, 1H), 7.98-8.06 (m, 2H), 7.89 (s, 1H), 7.86 (d, J=4.77 Hz, 1H), 7.82 (d, J=8.53 Hz, 1H), 7.62 (d, J=4.77 Hz, 1H), 3.80 (s, 3H), 2.93 (q, J=7.40 Hz, 2H), 1.18 (t, J=7.40 Hz, 3H)

### Step 2: 2-ethyl-4-[(3-iodoimidazo[1,2-a]pyrazin-8-yl)amino]benzoic acid

To a solution of methyl 2-ethyl-4-[(3-iodoimidazo[1,2-a]pyrazin-8-yl)amino]benzoate (9.37 g, 22.19 mmol, 1 eq) in THF (80 mL) was added sodium hydroxide (80.0 mL, 320 mmol, 14.42 eq) and then stirred at 60 °C for 60 h. The reaction mixture was adjusted to pH=1~2 by 3N HCl, filtered and dried to 2-ethyl-4-[(3-iodoimidazo[1,2-a]pyrazin-8-yl)amino]benzoic acid (8.2 g, 20.09 mmol, 90.52 % yield) as white solid. ¹H NMR (400 MHz, DMSO-d6) δ 12.48 (br s, 1H), 9.86 (s, 1H), 8.05 (dd, J=2.13, 8.66 Hz, 1H), 7.99 (d, J=2.01 Hz, 1H), 7.78-7.86 (m, 3H), 7.61 (d, J=4.64 Hz, 1H), 2.95 (q, J=7.40 Hz, 2H), 1.18 (t, J=7.47 Hz, 3H).

The following intermediates were prepared in analogy to Intermediate 63

| Int. | Name | ESI MS [M+H]⁺ | Starting Material |
|---|---|---|---|
| 64 | 2-chloro-4-[(3-iodoimidazo[1,2-a]pyrazin-8-yl)amino]benzoic acid | 415.1 | methyl 4-amino-2-chlorobenzoate |

### Reference Example 1

### 1-(4-((3-(4-([1,2-a]pyrazin-8-yl)amino)-2-methylbenzoyl)piperidine-4-carboxylic acid

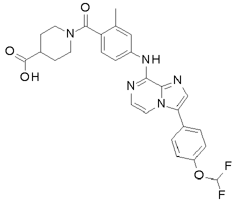

### Step 1

### methyl 1-(4-((3-(4-(difluoromethoxy)phenyl)imidazo[1,2-a]pyrazin-8-yl)amino)-2-methylbenzoyl)piperidine-4-carboxylate

A mixture of Intermediate 7, DIPEA (94.5 mg, 128 µL, 731 µmol) and HATU (185 mg, 487 µmol) in DMF (2 mL) was stirred for 30 min. Methyl piperidine-4-carboxylate (52.3 mg, 366 µmol) was added and stirring continued overnight. The mixture was purified by prep. HPLC to yield the title compound as a light brown solid (93 mg).

MS (ESI, m/z): 536.3 [M+H]⁺.

### Step 2

### 1-(4-((3-(4-([1,2-a]pyrazin-8-yl)amino)-2-methylbenzoyl)piperidine-4-carboxylic acid

A mixture of methyl 1-(4-((3-(4-(difluoromethoxy)phenyl)imidazo[1,2-a]pyrazin-8-yl)amino)-2-methylbenzoyl)piperidine-4-carboxylate (93 mg), 1M aq LiOH (0.8 mL) in THF(1 mL)/water (0.5 mL) was stirred at 60 °C for 5 h. The reaction mixture was concentrated and acidified by addition of 1M aq HCl. Water (1 mL) was added and the mixture was extracted with DCM. The combined organic layers were dried over sodium sulphate and then concentrated *in vacuo* to give the title compound (91 mg) as a white solid.

MS (ESI, m/z): 522.2 [M+H]⁺.

The following Examples and Intermediates were prepared in analogy to Reference Example 1

| Ex. | Name | Structure | ESI MS [M+H]⁺ | Starting Material |
|---|---|---|---|---|
| REF 2 | 1-(4-((3-(3-fluoro-4-methoxyphenyl)imidaz o[1,2-a]pyrazin-8-yl)amino)-2-methylbenzoyl)piperidi ne-4-carboxylic acid | 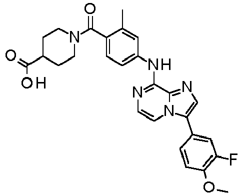 | 504.2 | Intermediate 6 |
| 65 | 1-[4-[[3-(2,3-difluoro-4-methoxyphenyl)imidazo[1,2-a]pyrazin-8-yl]amino]-2-methyl-benzoyl]piperidine-4-carboxylic acid | 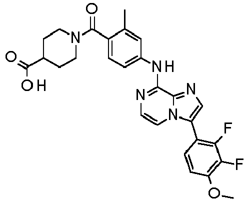 | 522.4 | Intermediate 28 |
| 66 | 1-(2-chloro-4-((3-(3-fluoro-4-methoxyphenyl)imidaz o[1,2-a]pyrazin-8-yl)amino)benzoyl)piper idine-4-carboxylic acid | 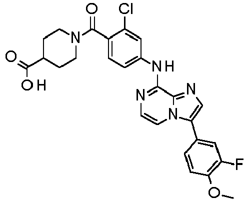 | 524.3 | Intermediate 2 |
| 67 | 1-[2-chloro-4-[(3-iodoimidazo[1,2-a]pyrazin-8-yl)amino]benzoyl]piper idine-4-carboxylic acid | 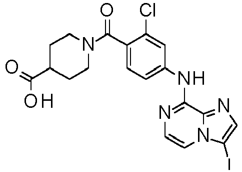 | 526.3 | Intermediate 64 |
| 68 | 1-[4-[(3-iodoimidazo[1,2-a]pyrazin-8-yl)amino]- 2-methylbenzoyl]piperidine-4-carboxylic acid | 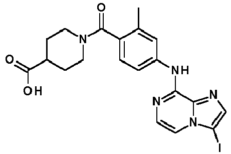 | 506.1 | Intermediate 1 |
| 69 | 1-(4-((3-(4-(cyanomethoxy)-2,3-difluorophenyl)imidazo [1,2-a]pyrazin-8-yl)amino)-2-methylbenzoyl)piperidi ne-4-carboxylic acid | 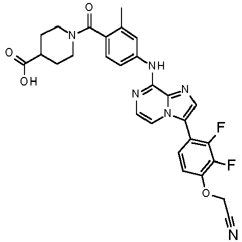 | 547.3 | Intermediate 31 and piperidine-4-carboxylic acid (no hydrolysis step) |

***Reference Example 3***

### 4-((3-(2,3-difluoro-4-methoxyphenyl)imidazo[1,2-a]pyrazin-8-yl)amino)-N,N,2-trimethylbenzamide

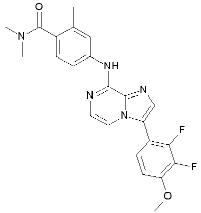

### Step 1:

### 4-amino-N,N,2-trimethyl-benzamide

To a solution 4-amino-2-methylbenzoic acid (2.7g, 18 mmol), dimethylamine hydrochloride (1.76g, 21.6 mmol) in DCM (350 mL) was added TEA (3.6 g, 36 mmol) and then the resultant mixture was stirred for 30 min at room temperature, EDCI (4 g, 21 mmol) was added in the mixture and stirred for extra 10 h. The mixture was poured into water (500 mL) and the aqueous solution was extracted with DCM (100 mL×2). The organic layers were combined and washed with water and brine, dried over anhydrous sodium sulphate and concentrated under reduced pressure to give a yellow oil which was purified by flash column chromatography to provide the desired compound (2.5 g, 78% yield) as an off-white solid

MS (ESI, m/z): 179.1 [M+H]⁺.

### Step 2:

### 4-((3-(2,3-difluoro-4-methoxyphenyl)imidazo[1,2-a]pyrazin-8-yl)amino)-N,N,2-trimethylbenzamide

To a solution of Intermediate 21 (295 mg, 1 mmol) in acetonitrile (10 mL) and acetic acid (1 mL) was added 4-amino-N,N,2-trimethyl-benzamide (178 mg, 1 mmol). The mixture was stirred overnight at 85 °C. The mixture was poured into water (50 mL) and the aqueous solution was extracted with DCM (75 mL×2). The organic layers were combined and washed with water and brine, dried over anhydrous sodium sulphate and concentrated under reduced pressure to give a red oil which was purified by prep. HPLC to provide the desired compound (200 mg, 45.7 % yield) as an off-white solid.

MS (ESI, m/z): 438.1 [M+H]⁺.

### Reference Example 4

### 4-(2-chloro-4-((3-(2,3-difluoro-4-methoxyphenyl)imidazo [1,2-a]pyrazin-8-yl)amino)benzoyl)piperazine-2-carboxylic acid

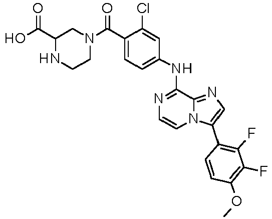

### Step 1:

### 1-tert-butyl 2-methyl 4-[2-chloro-4-[[3-(2,3-difluoro-4-methoxy-phenyl)imidazo[1,2-a] pyrazin-8-yl] amino] benzoyl] piperazine-1,2-dicarboxylate

To a solution of 2-chloro-4-((3-(2,3-difluoro-4-methoxyphenyl)imidazo[1,2-a]pyrazin-8-yl)amino)benzoic acid (215 mg, 0.5 mmol), 1-tert-butyl 2-methyl piperazine-1,2-dicarboxylate (146 mg, 0.6 mmol) in anhydrous DMF (5 mL) was added DIPEA (129 mg, 1.0 mmol) and then the resultant mixture was stirred for 30 min at room temperature, HATU (380 mg, 1.0 mmol) was added in the mixture and stirred for extra 10 h. The mixture was poured into water (50 mL) and the aqueous solution was extracted with ethyl acetate (50 mL×2). The organic layers were combined and washed with water and brine, dried and concentrated under reduced pressure to give a red oil, which was used in next step without purification. MS (ESI, m/z): 657.1 [M+H]⁺.

### Step 2:

### methyl 4-[2-chloro-4-[[3-(2,3-difluoro-4-methoxy-phenyl)imidazo[1,2-a]pyrazin-8-yl]amino]benzoyl]piperazine-2-carboxylate

To a solution of 1-tert-butyl 2-methyl 4-(2-chloro-4-((3-(2,3-difluoro-4-methoxyphenyl)imidazo[1,2-a]pyrazin-8-yl)amino)benzoyl)piperazine-1,2-dicarboxylate (200 mg, 0.3 mmol) in ethyl acetate (5 mL) was added 1 M hydrochloric acid in ethyl acetate (5.0 mL) at room temperature. The resultant mixture was stirred for 4 h and then adjusted to pH=7-8 with 2M aq. Na₂CO₃. The mixture was extracted with DCM (75 mL×2), the combined organic layers were washed with water and brine, dried and concentrated to give a red solid, which was used in the next step without purification.

MS (ESI, m/z): 557.1 [M+H]⁺.

### Step 3:

### 4-[2-chloro-4-[[3-(2,3-difluoro-4-methoxy-phenyl)imidazo[1,2-a]pyrazin-8-yl]amino]benzoyl]piperazine-2-carboxylicacid

To a solution of methyl 4-(2-chloro-4-((3-(2,3-difluoro-4-methoxyphenyl)imidazo[1,2-a]pyrazin-8-yl)amino)benzoyl)piperazine-2-carboxylate (167 mg, 0.3 mmol) in THF (5 mL)and MeOH ethyl acetate (5 mL) was added 1M aq. LiOH (3 mL) dropwise at room temperature. The resultant mixture was stirred for 4 h, and then acidified to pH 5-6 with 2 M hydrochloric acid. The mixture was extracted with DCM (50 mL × 2), and the combined organic layers were washed with brine, and then dried and then concentrated to give a light yellow oil, which was purified by prep. HPLC to provide the desired compound (200 mg, 45.7 % yield) as an off-white solid.

MS (ESI, m/z): 438.1 [M+H]⁺

The following Examples were prepared in analogy to Reference Example 4

| Ex. | Name | Structure | ESI MS [M+H]⁺ | Starting Material |
|---|---|---|---|---|
| REF 5 | 4-(3-(4-((3-(3-fluoro-4-methoxyphenyl)imidaz o[1,2-a]pyrazin-8-yl)amino)-2-methylbenzamido)prop yl)piperazine-2-carboxylic acid | 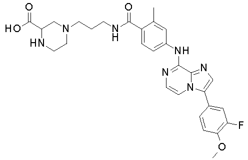 | 562.2 | Intermediate 6 and Intermediate 60 |
| REF 6 | 4-(1-(2-chloro-4-((3-(3-fluoro-4-methoxyphenyl)imidaz o[1,2-a]pyrazin-8-yl)amino)benzoyl)piper idine-4-carbonyl)piperazine-2-carboxylic acid, formate salt | 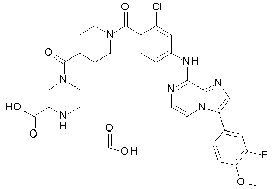 | 636.4 | Intermediate 66 and 1-(tert-butyl) 2-methyl piperazine-1,2-dicarboxylate |

### Intermediate 23

### 8-chloro-3-[4-(difluoromethoxy)-2,3-difluoro-phenyl]imidazo[1,2-a]pyrazine

### Step 1: 1-bromo-4-(difluoromethoxy)-2,3-difluoro-benzene

A mixture of 4-bromo-2,3-difluorophenol (1 g, 4.78 mmol), sodium chlorodifluoroacetate (1.09 g, 7.18 mmol) and potassium carbonate (1.32 g, 9.57 mmol) in DMF (10 mL) was heated to 100 °C with stirring overnight. The mixture was diluted with saturated aq. NaHCO₃ solution and extracted with DCM. The DCM layer was dried and concentrated. The residue was purified by column chromatography (eluting with PE/EA=50/1) to give the title compound (1 g) as colorless oil.

### Step 2: 2-[4-(difluoromethoxy)-2,3-difluoro-phenyl]-4,4,5,5-tetramethyl-1,3,2-dioxaborolane

A mixture of 1-bromo-4-(difluoromethoxy)-2,3-difluorobenzene (850 mg), bis(pinacolato)diboron (833 mg, 3.28 mmol), potassium acetate (644 mg, 6.56 mmol) and PdCl₂(PPh₃)₂ (115 mg, 164 µmol) in dioxane (20 mL) was heated to 100 °C with stirring overnight. The mixture was concentrated in vacuo and the residue was purified by column chromatography (eluting with PE/EA=30/1) to give the title compound (800 mg, 2.61 mmol) as colorless oil.

### Step 3: 8-chloro-3-[4-(difluoromethoxy)-2,3-difluoro-phenyl]imidazo[1,2-a]pyrazine

A mixture of 8-chloro-3-iodoimidazo[1,2-a]pyrazine (730 mg, 2.61 mmol), 2-(4-(difluoromethoxy)-2,3-difluorophenyl)-4,4,5,5-tetramethyl-1,3,2-dioxaborolane (800 mg, 2.61 mmol), PdCl₂(dppf)-CH₂Cl₂ adduct (95.6 mg, 131 µmol) and K₃PO₄ (1.66 g, 7.84 mmol) in THF (40 mL) and H₂O (10 mL) was heated to 50 °C with stirring overnight. The mixture was diluted with H₂O and extracted with DCM. The DCM layer was dried and concentrated. The residue was purified by silica gel column chromatography (eluting with PE/EA=5/1) to give the title compound (400 mg, 1.21 mmol) as a brown solid. MS (ESI, m/z): 332.2 [M+H]⁺

### Intermediate 41

### 2- [3-chloro-4-(8-chloroimidazo [1,2-a] pyrazin-3-yl)-2-fluoro-phenoxy] acetonitrile

### Step 1: 4-bromo-3-chloro-2-fluoro-phenol

To a stirred solution of 3-chloro-2-fluorophenol (10.00 g, 68.24 mmol) in DCM (200 mL) was added bromine (13.09 g, 81.88 mmol) dropwise at -10 °C. The reaction mixture was warmed up to 20 °C and stirred for 16 h. The reaction was quenched with sat. aq. Na₂SO₃ (100 mL) and extracted with DCM (150 mL). The organic phase was washed with sat. NaHCO₃ (100 mL) and brine (100 mL), dried and concentrated under reduced pressure to give 4-bromo-3-chloro-2-fluoro-phenol (13.7 g) as a white solid . ¹H NMR (400 MHz, CDCl3) □: 7.31 (dd, 1H), 6.86 (t, 1H)

### Step 2: 2-(4-bromo-3-chloro-2-fluoro-phenoxy)acetonitrile

A mixture of 4-bromo-3-chloro-2-fluoro-phenol (13.70 g, 60.77 mmol), potassium carbonate (12.60 g, 91.16 mmol) and bromoacetonitrile (8.75 g, 72.92 mmol) in acetonitrile (200 mL) was stirred at 60 °C for 16 h. The reaction mixture was cooled and filtered. The filtrate was concentrated under reduced pressure, purified by the flash column chromatography (eluting with PE/EA=10/1) to give 2-(4-bromo-3-chloro-2-fluoro-phenoxy) acetonitrile (13.0 g) as a white solid. ¹H NMR (400 MHz, CDCl3) □: 7.43 (dd, 1H), 6.96 (dd, 1H), 4.84 (s, 2H)

### Step 3: 2-[3-chloro-2-fluoro-4-(4,4,5,5-tetramethyl-1,3,2-dioxaborolan-2-yl)phenoxy]aceto nitrile

A mixture of 2-(4-bromo-3-chloro-2-fluoro-phenoxy)acetonitrile (13.00 g, 49.15 mmol), bis(pinacolato)diboron (14.98 g, 58.98 mmol), potassium acetate (14.47 g, 147.46 mmol) and Pd(dppl)Cl₂ ·CH₂Cl₂ adduct (3.60 g, 4.92 mmol) in 1,4-dioxane (280 mL) was stirred at 70 °C under nitrogen for 16 h. The reaction was cooled and the mixture was filtered. The filtrate was concentrated under reduced pressure and the residue was purified by flash column chromatography (eluting with PE: EA=10:1) to afford desired product (11.6 g) as a light yellow solid.

### Step 4: 2-[3-chloro-4-(8-chloroimidazo[1,2-a]pyrazin-3-yl)-2-fluoro-phenoxy]acetonitrile Intermediate 41

A mixture of 8-chloro-3-iodo-imidazo[1,2-a]pyrazine (6.50 g, 23.26 mmol), 2-[3-chloro-2-fluoro-4-(4,4,5,5-tetramethyl-1,3,2-dioxaborolan-2-yl)phenoxy] acetonitrile (11.59 g, 23.26 mmol), sodium carbonate (7.40 g, 69.77 mmol) and Pd(dppf)Cl₂·CH₂Cl₂ adduct (1.7 g, 2.33 mmol) in 1,4-dioxane (150 mL) and water (30 mL) was stirred under nitrogen at 60 °C for 16 h. The reaction mixture was cooled and filtered. The filtrate was concentrated and the residue was diluted with H₂O (100 mL) and extracted with DCM (200 mLx3). The organic phase was washed with brine (100 mL), concentrated under reduced pressure and purified by flash column chromatography (PE/EA=1/1) to give crude product. It was re-purified by trituration (PE/EA=3/1) and dried in vacuo to afford the title compound (5.0 g) as a light red solid.

MS obsd. (ESI⁺) [(M+H)⁺]: 337.2

### Intermediate 27

### 8-chloro-3-(2-chloro-5-fluoro-4-methoxy-phenyl)imidazo[1,2-a]pyrazine

### Step 1: 1-chloro-4-fluoro-5-methoxy-2-nitro-benzene

Sodium (712 mg, 30.97 mmol) was added to MeOH (50 mL) and the mixture was stirred for 10 min. To the resulting mixture was added a solution of 1-chloro-4,5-difluoro-2-nitro-benzene (5.0 g, 25.83 mmol) in MeOH (20 mL) at 0 °C. The reaction was stirred at 15 °C for 2 h. The mixture was quenched with water (30 mL) and then extracted with DCM (100 mL). The DCM layer was washed with brine (30 mL), dried and concentrated under reduced pressure to give 1-chloro-4-fluoro-5-methoxy-2-nitro-benzene (4.2 g) as a yellow solid. ¹H NMR (400 MHz, CDCl3) □: 7.86 (d, 1H), 7.08 (d, 1H), 4.00 (s, 3H)

### Step 2: 2-chloro-5-fluoro-4-methoxy-aniline

To a stirred suspension of nickel(ii) chloride hexahydrate (2.44 g, 10.25 mmol) and sodium borohydride (380 mg, 10.04 mmol) in methanol (100 mL) was added a solution of 1-chloro-4-fluoro-5-methoxy-2-nitro-benzene (4.2 g, 20.43 mmol) in THF (40 mL) at 0 °C drop wise. Then additional sodium borohydride (2.31 g, 61.06 mmol) was added at 0 °C and the reaction mixture was stirred for 1 h at 15 °C. The reaction was quenched by addition of water (20 mL). The solid was filtered and the filtrate was extracted with DCM. The organic phase was washed with brine, dried and concentrated under reduced pressure. The residue was purified by column chromatography (eluting with PE/EA=5/1) to give the title compound (2.8 g) as a yellow solid.

### Step 3: 1-bromo-2-chloro-5-fluoro-4-methoxy-benzene

To a solution of 2-chloro-5-fluoro-4-methoxy-aniline (1.7 g, 9.68 mmol) in aq. HBr (20 mL) was added sodium nitrite (735 mg, 10.65 mmol) in water (8 mL) at 0 °C. The mixture was stirred at 0 °C for 30 min. Then a solution of copper (I) bromide (2.08 g, 14.52 mmol) and copper (II) bromide (3.24 g, 14.52 mmol) in aq. HBr (20 mL) was added to the mixture. The reaction was stirred at 60 °C for 2 h. The mixture was diluted with DCM (100 mL), washed with water (30 mL) and brine (30 mL), dried and concentrated under reduced pressure. The residue was purified by column chromatography (PE/EA=20/1) to give the title compound (530 mg) as a white solid.

### Step 4: 2-(2-chloro-5-fluoro-4-methoxy-phenyl)-4, 4, 5, 5-tetramethyl-1, 3, 2-dioxaborolane

A mixture of 1-bromo-2-chloro-5-fluoro-4-methoxy-benzene (530 mg, 2.21 mmol), bis(pinacolato)diboron (843 mg, 3.32 mmol), potassium acetate (652 mg, 6.64 mmol) and Pd(dppf)Cl₂ · CH₂Cl₂ adduct (181 mg, 0.22 mmol) in 1,4-dioxane (2 mL) was stirred at 80 °C under nitrogen for 16 h. The reaction was cooled and the mixture was filtered. The filtrate was concentrated under reduced pressure and the residue was purified by flash column chromatography (PE/EA=100/1) to give desired compound (250 mg) as a white solid.

### Step 5: 8-chloro-3-(2-chloro-5-fluoro-4-methoxy-phenyl)imidazo[1,2-a]pyrazine

A mixture of intermediate 8-chloro-3-iodo-imidazo[1,2-a]pyrazine (400 mg, 1.43 mmol), 2-(2-chloro-5- fluoro-4-methoxy-phenyl)-4,4,5,5-tetramethyl-1,3,2-dioxaborolane (420 mg, 1.47 mmol), sodium carbonate (455 mg, 4.29 mmol) and Pd(dppf)Cl₂ ·CH₂Cl₂ adduct (117 mg, 0.14 mmol) in 1,4-dioxane (8 mL) and water (2 mL) was stirred under nitrogen at 50 °C for 16 h. The reaction mixture was cooled and filtered. The filtrate was concentrated under reduced pressure and the residue was purified by flash column chromatography (PE/EA=3/1) to give the desired product (375 mg) as a brown solid. MS obsd. (ESI⁺) [(M+H)⁺]: 312.2

### Intermediate 70

### 8-chloro-3-(2-chloro-3-fluoro-4-methoxy-phenyl)imidazo[1,2-a]pyrazine

### Step 1: 1-bromo-2-chloro-3-fluoro-4-methoxy-benzene

To a stirred solution of 1-chloro-2-fluoro-3-methoxy-benzene (2.00 g, 12.46 mmol) in chloroform (20 mL) was added bromine (1.89 g, 11.83 mmol) drop wise. The reaction mixture was stirred at 15 °C for 2 h. The reaction was quenched with aq. Na₂SO₃ solution and extracted with DCM. The organic phase was washed with brine (20 mL), dried over anhydrous sodium sulphate, concentrated under reduced pressure to give the desired compound (2.00 g) as a white solid.

### Step 2: 2-(2-chloro-3-fluoro-4-methoxy-phenyl)-4,4,5,5-tetramethyl-1,3,2-dioxaborolane

A mixture of 1-bromo-2-chloro-3-fluoro-4-methoxy-benzene (1.00 g, 2.8 mmol), bis(pinacolato)diboron (710 mg, 2.8 mmol), potassium acetate (824 mg, 8.39 mmol) and Pd (dppf)Cl₂· CH₂Cl₂ adduct (228 mg, 0.28 mmol) in 1,4-dioxane (20 mL) was stirred at 80 °C under nitrogen for 2 h. The reaction was cooled and the mixture was filtered. The filtrate was concentrated under reduced pressure and the residue was purified by flash column chromatography (eluting with PE/EA=50/1) to give the desired compound (200 mg) as a white solid.

### Step 3: 8-chloro-3-(2-chloro-3-fluoro-4-methoxy-phenyl)imidazo[1,2-a]pyrazine

A mixture of 2-(2-chloro-3-fluoro-4-methoxy-phenyl)-4,4,5,5-tetramethyl-1,3,2-dioxaborolane (195 mg, 0.68 mmol), 8-chloro-3-iodo-imidazo[1,2-a]pyrazine (190 mg, 0.68 mmol), sodium carbonate (216 mg, 2.04 mmol) and Pd(dppf)Cl₂ ·CH₂Cl₂ adduct (55 mg, 0.07 mmol) in 1,4-dioxane (4 mL) and water (1 mL) was stirred under nitrogen at 50 °C for 16 h. The reaction was cooled to RT and concentrated under reduced pressure. The residue was purified by prep-TLC (PE/EA=2/1) to afford desired compound (67 mg) as a white solid. MS obsd. (ESI⁺) [(M+H)⁺] : 312.

### Intermediate 71

### 2-[5-chloro-2-fluoro-4-(4,4,5,5-tetramethyl-1,3,2-dioxaborolan-2-yl)phenoxy]acetonitrile

### Step 1: 5-chloro-2-fluoro-4-nitro-phenol

A mixture of 1-chloro-4,5-difluoro-2-nitro-benzene (5.0 g, 25.83 mmol) and 15% aqueous KOH (2.9 g, 7.75 mmol) was stirred at 100 °C for 14 h. The mixture was added HCl (1N) until pH 4~5 and extracted with DCM (100 mL × 3). The mixture was then concentrated to dryness and purified by flash column chromatography (PE/EA = 100% ~ 10%) to afford 5-chloro-2-fluoro-4-nitro-phenol (4.1 g, 21.41 mmol) as a yellow solid. MS obsd. (ESI⁻): 190.0 [(M-H)-].

### Step 2: 4-amino-5-chloro-2-fluoro-phenol

To a mixture of 5-chloro-2-fluoro-4-nitro-phenol (4.0 g, 20.88 mmol) and ammonium chloride (5.59 g, 104.42 mmol) in ethanol (60 mL) and water (30 mL) was added iron (5.83 g, 104.42 mmol). The mixture was stirred at 25 °C for 2 h. The mixture was filtered by celite. The filtrate was concentrated *in vacuo* to remove EtOH. The mixture was extracted with EA (30 mL × 3). The combined organic layers were concentrated to dryness. The crude product was purified by flash column chromatography to (PE/EA = 100% to 90%) afford 4-amino-5-chloro-2-fluorophenol (1.68 g) as a brown solid. MS obsd. (ESI⁻): 162.1 [(M-H)⁻].

### Step 3: 4-bromo-5-chloro-2-fluoro-phenol

To a mixture of 4-amino-5-chloro-2-fluoro-phenol (1.55 g, 9.57 mmol) in hydrobromic acid (19.69 mL, 145.02 mmol) was added a solution of sodium nitrite (0.79 g, 11.48 mmol) in water (8 mL) at 0 °C. The mixture was kept at the same temperature for 30 min. Then a mixture of copper(II) bromide (0.67 mL, 14.35 mmol) and copper(I) bromide (2.06 g, 14.35 mmol) in hydrobromic acid (19.69 mL, 145.02 mmol) was added. The mixture was stirred at 60 °C for 14 h. The mixture was diluted with water (50 mL) and extracted with DCM (50 mL×3). The combined organic layers were concentrated to dryness. The crude was purified by flash column chromatography (PE/EA = 100% to 90%) to afford 4-bromo-5-chloro-2-fluoro-phenol (1.89 g) as a white solid.

MS obsd. (ESI⁻): 223.0 [(M-H)⁻].

### Step4: 2-(4-bromo-5-chloro-2-fluoro-phenoxy)acetonitrile

A mixture of 4-bromo-5-chloro-2-fluoro-phenol (1.89 g, 8.38 mmol) and potassium carbonate (3.48 g, 25.15 mmol) in acetone (150 mL) was stirred at 25 °C for 10 min. Then bromoacetonitrile (0.63 mL, 10.06 mmol) was added. The mixture was then stirred at 25 °C for 14 h. The mixture was concentrated to dryness and added water (20 mL). The mixture was extracted with ethyl acetate (10 mL × 3). The combined organic layers were concentrated to dryness. The crude was purified by flash column chromatography (EA/PE = 10%) to afford 2-(4-bromo-5-chloro-2-fluoro-phenoxy)acetonitrile (1.96 g) as a white solid. ¹H NMR (400 MHz, CDCl3) □:7.44 (d, 1H), 7.23 (d, 1H), 4.83 (s, 2H)

### Step 5: 2-[5-chloro-2-fluoro-4-(4,4,5,5-tetramethyl-1,3,2-dioxaborolan-2-yl)phenoxy] acetonitrile

A mixture of 2-(4-bromo-5-chloro-2-fluoro-phenoxy)acetonitrile (1.96 g, 7.41 mmol), bis(pinacolato)diboron (2.26 g, 8.89 mmol), potassium acetate (1.39 mL, 22.23 mmol) and [1,1'-bis(diphenylphosphino)ferrocene]dichloropalladium(II) (542.26 mg, 0.740 mmol) in 1,4-dioxane (20 mL) was stirred at 100 °C under nitrogen for 14 h. The mixture was filtered over celite. The filtrate was concentrated to dryness. The crude product was then purified by flash column chromatography (EA/PE = 5%) to afford 2-[5-chloro-2-fluoro-4-(4,4,5,5-tetramethyl-1,3,2-dioxaborolan-2-yl)phenoxy]acetonitrile (2.12 g) as a white solid.

¹H NMR (400 MHz, CDCl₃) *δ* ppm: 1.36 (s, 12H) 4.84 (s, 2H) 7.07 (d, *J* = 7.0 Hz, 1H) 7.49 (d, *J* = 11.3 Hz, 1H)

### Intermediate 72

### 2-(2-(2-hydroxyethoxy)ethyl)isoindoline-1,3-dione

A mixture of 2-(2-aminoethoxy)ethanol (3.51 g, 33.4 mmol) and isobenzofuran-1,3-dione (4.5 g, 30.4 mmol) in toluene (40 mL) was heated to 110 °C with stirring overnight. The mixture was concentrated *in vacuo.* The residue was diluted with water and extracted with DCM. The DCM layer was dried and concentrated to give crude 2-(2-(2-hydroxyethoxy)ethyl)isoindoline-1,3-dione (5.8 g, 81 % yield) as yellow solid which was used in next step directly. MS obsd. (ESI⁺) [(M+H)⁺] : 236.

### Intermediate 62

### 2-[2-(1,3-dioxoisoindolin-2-yl)ethoxy]ethyl 4-methylbenzenesulfonate

To a solution of 2-(2-(2-hydroxyethoxy)ethyl)isoindoline-1,3-dione (2.5 g, 10.6 mmol) and TEA (2.15 g, 2.96 mL, 21.2 mmol) in DCM (40 mL) cooled at 0 °C was added 4-methylbenzene-1-sulfonyl chloride (4.05 g, 21.3 mmol). The mixture was warmed slowly to RT and stirred at RT overnight. The mixture was purified by column chromatography (eluting with PE/EA=2/1) to give 2-(2-(1,3-dioxoisoindolin-2-yl)ethoxy)ethyl 4-methylbenzenesulfonate (3.2 g, 78 % yield) as white solid. MS obsd. (ESI⁺) [(M+H)⁺]: 390.

### Intermediate 73

### tert-butyl N-[[1-[4-[(3-iodoimidazo[1,2-a]pyrazin-8-yl)amino]-2-methyl-benzoyl]-4-piperidyl] methyl] carbamate

A mixture of 4-(Boc-aminomethyl)piperidine (1.77 g, 8.25 mmol), 4-((3-iodoimidazo[1,2-a]pyrazin-8-yl)amino)-2-methylbenzoic acid (Intermediate 1, 2.5 g, 6.34 mmol), HATU (3.62 g, 9.51 mmol) and Et₃N (1.93 g, 2.65 mL, 19 mmol) in DMF (30 mL) was stirred at room temperature overnight. The mixture was poured into water. The aqueous phase was extracted with DCM. The organic phase was washed with saturated NaCl solution and water. The organic phase was dried and concentrated *in vacuo.* The residue was purified by flash column to afford the title compound (3 g) as an orange oil. MS (ESI, m/z): 591 [M+H]+

The following intermediates were prepared in analogy:

| Int. | Name | MS ESI [M+H]⁺ | Starting Material |
|---|---|---|---|
| 74 | tert-butyl rac-(3R)-3-[[1-[2-chloro-4-[(3-iodoimidazo[1,2-a]pyrazin-8-yl)amino]benzoyl]piperidine-4-carbonyl]amino]pyrrolidine-1-carboxylate | 694.4 | Intermediate 67 and tert-butyl (R)-3-aminopyrrolidine-1- carboxylate |
| 75 | tert-butyl (2S,4R)-4-hydroxy-2-(4-(4-((3-iodoimidazo[1,2-a]pyrazin-8-yl)amino)-2-methylbenzoyl)piperazine-1-carbonyl)pyrrolidine-1-carboxylate | 676.2 | Intermediate 76 and (2S,4R)-1-(tert-butoxycarbonyl)-4-hydroxypyrrolidine-2-carboxylic acid |

### Reference Example 7

### 2-chloro-4-[[3-(2,3-difluoro-4-methoxyphenyl)imidazo[1,2-a]pyrazin-8-yl]amino]-N-[2-(2-piperazin-1-ylethoxy)ethyl]benzamide;2,2,2-trifluoroacetic acid

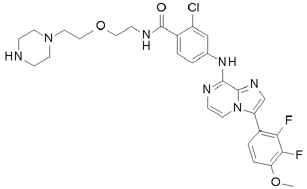

### Step 1: tert-butyl 4-[2-[2-(1,3-dioxoisoindolin-2-yl)ethoxy]ethyl]piperazine-1-carboxylate

A mixture of Intermediate 62 (650 mg, 1.67 mmol), *tert*-butyl piperazine-1-carboxylate (466 mg, 2.5 mmol) and potassium carbonate (461 mg, 3.34 mmol) in DMF (10 mL) was stirred at RT overnight. The mixture was diluted with H₂O and extracted with DCM. The DCM layer was combined and washed with brine, concentrated and the residue was purified by column (silica gel, eluting with PE/EA=3/1) to give tert-butyl 4-(2-(2-(1,3-dioxoisoindolin-2-yl)ethoxy)ethyl)piperazine-1-carboxylate (700 mg) which was used in next step directly.

### Step 2: tert-butyl 4-[2-(2-aminoethoxy)ethyl]piperazine-1-carboxylate

To a solution of tert-butyl 4-(2-(2-(1,3-dioxoisoindolin-2-yl)ethoxy)ethyl)piperazine-1-carboxylate (700 mg, 1.73 mmol) in EtOH (10 mL) was added hydrazine hydrate (510 mg, 0.5 mL, 10.2 mmol). The mixture was stirred at rt overnight. The volatiles were removed and the residue was suspended in DCM and an insoluble solid was filtered off. The filtrate was concentrated *in vacuo* to give crude tert-butyl 4-(2-(2-aminoethoxy)ethyl)piperazine-1-carboxylate (500 mg) as light yellow oil which was used in next step directly.

### Step 3: tert-butyl 4-[2-[2-[[2-chloro-4-[[3-(2,3-difluoro-4-methoxy-phenyl)imidazo[1,2-a] pyrazin-8-yl] amino] benzoyl] amino] ethoxy] ethyl] piperazine-1-carboxylate

A mixture of tert-butyl 4-(2-(2-aminoethoxy)ethyl)piperazine-1-carboxylate (127 mg, 464 µmol), intermediate 20 (100 mg, 232 µmol), HATU (177 mg, 464 µmol) and TEA (363 mg, 0.5 mL) in DMF (5 mL) was stirred at rt overnight. The reaction was diluted with H₂O (50 mL) and extracted with DCM. The DCM layer was dried and concentrated *in vacuo* to give crude tert-butyl 4-(2-(2-(2-chloro-4-((3-(2,3-difluoro-4-methoxyphenyl)imidazo[1,2-a]pyrazin-8-yl)amino)benzamido)ethoxy)ethyl)piperazine-1-carboxylate (200 mg) as yellow oil which was used in the next step directly.

### Step 4: 2-chloro-4-[[3-(2,3-difluoro-4-methoxy-phenyl)imidazo[1,2-a]pyrazin-8-yl]amino]-N-[2-(2-piperazin-1-ylethoxy)ethyl]benzamide

To a solution of tert-butyl 4-(2-(2-(2-chloro-4-((3-(2,3-difluoro-4-methoxyphenyl)imidazo[1,2-a]pyrazin-8-yl)amino)benzamido)ethoxy)ethyl)piperazine-1-carboxylate (200 mg, 291 µmol) in MeOH (10 mL) was added TFA (2.96 g, 2 mL, 26 mmol). The mixture was heated to 50 °C with stirring overnight. The volatiles were removed and the residue was purified by prep-HPLC to give 2-chloro-4-((3-(2,3-difluoro-4-methoxyphenyl)imidazo[1,2-a]pyrazin-8-yl)amino)-N-(2-(2-(piperazin-1-yl)ethoxy)ethyl)benzamide (30 mg) as light yellow solid. MS obsd. (ESI+) [(M+H)+] : 586.

### Reference Example 8

### 2-chloro-4-[[3-(2,3-difluoro-4-methoxy-phenyl)imidazo[1,2-a]pyrazin-8-yl]amino]-N-[2-[2-(dimethylamino)ethoxy]ethyl]benzamide;2,2,2-trifluoroacetic acid

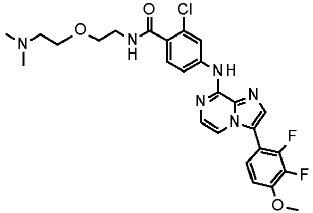

### Step 1: 2-[2-[2-(dimethylamino)ethoxy]ethyl]isoindoline-1,3-dione

A mixture of Intermediate 62 (400 mg, 1.03 mmol), dimethylamine (770 µL, 1.54 mmol) and potassium carbonate (284 mg, 2.05 mmol) in acetonitrile (10 mL) was stirred at RT overnight. The mixture was diluted with H₂O and extracted with DCM. The DCM layer was combined and washed with brine, concentrated to give crude 2-(2-(2-(dimethylamino)ethoxy)ethyl)isoindoline-1,3-dione (300 mg) which was used in next step directly.

### Step 2: 2-[2-(dimethylamino)ethoxy]ethanamine

To a soluion of 2-(2-(2-(dimethylamino)ethoxy)ethyl)isoindoline-1,3-dione (300 mg, 1.14 mmol) in EtOH (10 mL) was added hydrazine hydrate (57.3 mg, 1.14 mmol). The reaction was stirred at RT overnight. The solid was filtered and the filtrate was concentrated *in vacuo* to give 2-(2-aminoethoxy)-N,N-dimethylethanamine (150 mg) as light yellow oil which was used in next step directly.

### Step 3: 2-chloro-4-[[3-(2,3-difluoro-4-methoxy-phenyl)imidazo[1,2-a]pyrazin-8-yl]amino]-N-[2-[2-(dimethylamino)ethoxy]ethyl]benzamide

A mixture of Intermediate 20 (100 mg, 232 µmol, 3-(2-(dimethylamino)ethoxy)propan-1-amine (33.9 mg, 232 µmol), HATU (177 mg, 464 µmol) and TEA (363 mg, 0.5 mL, 3.59 mmol) in DMF (5 mL) was stirred at rt overnight. The mixture was diluted with H₂O (50 mL) and extracted with DCM (50 mL) for three times. The DCM layer was dried and concentrated in vacuo. The residue was purified by prep-HPLC to give the title compound (40 mg) as light yellow solid. MS (ESI+) [M+H]⁺: 545.1.

### Reference Example 9

### 4-[[3-(2,3-difluoro-4-methoxy-phenyl)imidazo[1,2-a]pyrazin-8-yl]amino]-2-methyl-N-[2-[2-(3-oxopiperazin-1-yl)ethoxy]ethyl]benzamide;2,2,2-trifluoroacetic acid

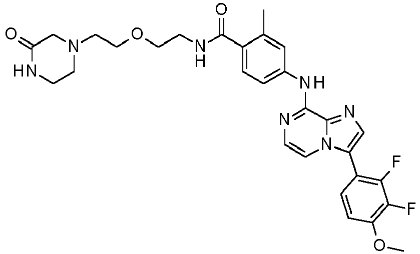

### Step 1: 2-[2-(1,3-dioxoisoindolin-2-yl)ethoxy]ethyl methanesulfonate

To a solution of Intermediate 72 (2 g, 8.5 mmol) and TEA (1.45 g, 2 mL) in CH₂Cl₂ (50 mL) cooled at 0 °C was added MsCl (1.07 g, 9.35 mmol). The mixture was warmed to RT and stirred at RT for 4 h. The mixture was concentrated *in vacuo* and the residue was purified by column chromatography (eluting with PE/EA=1/1) to give 2-(2-(1,3-dioxoisoindolin-2-yl)ethoxy)ethyl methanesulfonate

### Step 2: 2-[2-[2-(3-oxopiperazin-1-yl)ethoxy]ethyl]isoindoline-1,3-dione

A mixture of 2-(2-(1,3-dioxoisoindolin-2-yl)ethoxy)ethyl methanesulfonate (500 mg, 1.6 mmol), piperazin-2-one (192 mg, 1.91 mmol) and K₂CO₃ (441 mg, 3.19 mmol) in DMF (5 mL) was heated to 100 °C with stirring overnight. The mixture was diluted with H₂O and extracted with DCM. The DCM layer was dried and concentrated *in vacuo* to give crude 2-(2-(2-(3-oxopiperazin-1-yl)ethoxy)ethyl)isoindoline-1,3-dione (550 mg) as yellow oil which was used in next step directly.

### Step 3: 4-[2-(2-aminoethoxy)ethyl]piperazin-2-one

A mixture of crude 2-(2-(2-(3-oxopiperazin-1-yl)ethoxy)ethyl)isoindoline-1,3-dione (550 mg, 1.73 mmol, Eq: 1) and hydrazine monohydrate (104 mg, 2.08 mmol) in EtOH (5 mL) was stirred at RT overnight. The mixture was concentrated *in vacuo* and the solid residue was suspended in DCM. The mixture was stirred at RT for 30 min and filtered. The filtrate was concentrated *in vacuo* to give crude 4-(2-(2-aminoethoxy)ethyl)piperazin-2-one (350 mg) as a yellow oil which was used in next step directly.

### Step 4: 4-[[3-(2,3-difluoro-4-methoxy-phenyl)imidazo[1,2-a]pyrazin-8-yl]amino]-2-methyl-N-[2-[2-(3-oxopiperazin-1-yl)ethoxy]ethyl]benzamide

A mixture of Intermediate 28 (150 mg, 366 µmol), 4-(2-(2-aminoethoxy)ethyl)piperazin-2-one (137 mg, 731 µmol, TEA (363 mg, 0.5 mL) and HATU (278 mg, 731 µmol) in DMF (5 mL) was stirred at rt. The mixture was diluted with H₂O (30 mL) and extracted with ethyl acetate. The ethyl acetate layer was concentrated and the residue was purified by prep-HPLC to give the title compound (36 mg) as light yellow solid. (ESI+) [(M+H)⁺]: 580.

### Reference Example 10

### 4-[[3-(3-fluoro-4-methoxy-phenyl)imidazo[1,2-a]pyrazin-8-yl]amino]-N,2-dimethyl-N-(3-oxo-3-piperazin-1-yl-propyl)benzamide

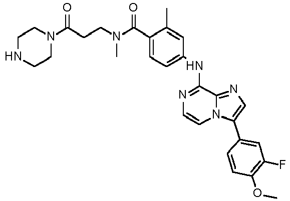

### Step 1: ethyl 3-[[4-[[3-(3-fluoro-4-methoxy-phenyl)imidazo[1,2-a]pyrazin-8-yl]amino]-2-methyl-benzoyl]-methyl-amino]propanoate

To a stirred solution of ethyl 3-(methylamino) propanoate (100 mg, 0.76 mmol), Intermediate 6 (200 mg, 0.51 mmol) and triethylamine (0.2 mL, 1.53 mmol) in DMF (3 mL) was added 1-propanephosphonic anhydride (487 mg, 0.76 mmol, 50% in ethyl acetate) slowly. The reaction was stirred at 15 °C for 4 h. The reaction mixture was diluted with H₂O (10 mL) and extracted with ethyl acetate. The organic phase was washed with brine, dried over anhydrous sodium sulphate and concentrated under reduced pressure to give the title compound (250 mg) as a yellow oil. MS (ESI, m/z): 506 [M+H]⁺.

### Step 2: 3-[[4-[[3-(3-fluoro-4-methoxy-phenyl)imidazo[1,2-a] pyrazin-8-yl] amino]-2-methyl-benzoyl]-methyl-amino]propanoic acid

To a stirred solution of ethyl 3-[[4-[[3-(3-fluoro-4-methoxy-phenyl)imidazo[1,2-a]pyrazin-8-yl]amino]-2-methyl-benzoyl]-methyl-amino]propanoate (250 mg, 0.49 mmol) in ethanol (3 mL) was added a solution of sodium hydroxide (40 mg, 0.99 mmol) in water (0.5 mL) slowly. The reaction was stirred at 30 °C for 4 h. Aq. HCl (1.0 M) was added drop wise until pH=4-5. The reaction mixture was concentrated under reduced pressure and the residue was purified by prep-HPLC to afford the title compound (59.4 mg) as a white solid. MS obsd. (ESI+) [(M+H)⁺] : 478

### Step 3: tert-butyl 4-[3-[[4-[[3-(3-fluoro-4-methoxy-phenyl)imidazo[1,2-a]pyrazin-8-yl]amino]-2-methyl-benzoyl]-methyl-amino]propanoyl]piperazine-1-carboxylate

To a stirred mixture of 3-[[4-[[3-(3-fluoro-4-methoxy-phenyl)imidazo[1,2-a]pyrazin-8-yl]amino]-2-methyl-benzoyl]-methyl-amino]propanoic acid (140 mg, 0.29 mmol), Boc-piperazine hydrochloride (98 mg, 0.44 mmol) and triethylamine (0.12 mL, 0.88 mmol) in DMF (2 mL) was added 1-propanephosphonic anhydride (280 mg, 0.44 mmol, 50% in ethyl acetate) slowly at 15 °C. The reaction was stirred for 4 h. The reaction mixture was diluted with H₂O (10 mL) and extracted with ethyl acetate. The organic phase was washed with brine (10 mL), dried and concentrated under reduced pressure to give the title compound (170 mg) as a yellow oil.

MS obsd. (ESI⁺) [(M+H)⁺] : 646

### Step 4: 4-[[3-(3-fluoro-4-methoxy-phenyl)imidazo[1,2-a] pyrazin-8-yl]amino]-N,2-dimethyl-N-(3-oxo-3-piperazin-1-yl-propyl)benzamide

A mixture of tert-butyl 4-[3-[[4-[[3-(3-fluoro-4-methoxy-phenyl)imidazo[1,2-a]pyrazin-8-yl]amino]-2-methyl-benzoyl]-methyl-amino]propanoyl]piperazine-1-carboxylate (170 mg, 0.26 mmol) and a solution of HCl in MeOH (0.2 mL, 0.79 mmol) in methanol (2 mL) was stirred at 15 °C for 4 h. The reaction mixture was concentrated under reduced pressure and purified by prep-HPLC to give the title compound (7.7 mg) as a white solid. MS obsd. (ESI+)

[(M+H)+]: 546.1.

### Reference Example 11

### 4-[[3-(3-fluoro-4-methoxy-phenyl)imidazo[1,2-a]pyrazin-8-yl]amino]-N,2-dimethyl-N-(4-oxo-4-piperazin-1-yl-butyl)benzamide

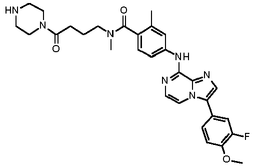

Reference Example 11 was prepared using same procedure as for Reference Example 10, changing ethyl 3-(methylamino) propanoate to methyl 4-(methylamino) butanoate hydrochloride. The title compound was purified by prep-HPLC. MS (ESI, m/z): 560.1 [M+H]⁺.

### Intermediate 77:

### 2-[[4-[[3-(3-fluoro-4-methoxy-phenyl)imidazo[1,2-a]pyrazin-8-yl]amino]-2-methyl-benzoyl]-methyl-amino]acetic acid

### Step 1: methyl 2-[methyl-(2-methyl-4-nitro-benzoyl)amino] acetate

A mixture of 2-methyl-4-nitro-benzoic acid (2.00 g, 11.04 mmol), EDC hydrochloride (3.17 g, 16.56 mmol), HOBt (2.24 g, 16.56 mmol) and DIPEA (5.77 mL, 33.12 mmol) in DMF (40 mL) was stirred at 15 °C for 0.5 h. Then sarcosine methyl ester hydrochloride (2.31 g, 16.56 mmol) was added and the mixture was stirred at 15°C for 16 h. The reaction mixture was diluted with H₂O (50 mL) and extracted with ethyl acetate. The organic phase was washed with brine, dried, concentrated under reduced pressure and purified by flash column chromatography (eluting with PE:EA=3:1) to afford the title compound (1.00 g) as brown oil. MS obsd. (ESI⁺) [M+H]⁺ : 267

### Step 2: methyl 2- [(4-amino-2-methyl-benzoyl)-methyl-amino] acetate

A mixture of methyl 2-[methyl-(2-methyl-4-nitro-benzoyl)amino]acetate (1.00 g, 3.76 mmol) and palladium (200 mg, 1.88 mmol, 10 wt% on charcoal) in methanol (20 mL) was stirred under hydrogen (15 psi) at 15 °C for 16 h. The mixture was filtered and the filtrate was concentrated under reduced pressure to give the title compound (850 mg) as a crude product which was used directly in the next step.

### Step 3: methyl 2-[[4-[[3-(3-fluoro-4-methoxy-phenyl)imidazo[1,2-a]pyrazin-8-yl]amino]-2-methyl-benzoyl]-methyl-amino]acetate

A mixture of Intermediate 3 (950 mg, 3.42 mmol) and methyl 2-[[4-[[3-(3-fluoro-4-methoxyphenyl)imidazo[1,2-a]pyrazin-8-yl]amino]-2-methyl-benzoyl]-methyl-amino]acetate (808 mg, 3.42 mmol) in acetonitrile (18 mL) and acetic acid (2 mL) was stirred at 100 °C for 4 h. The reaction was cooled and concentrated under reduced pressure. The residue was purified by flash column chromatography (eluting with DCM/MeOH=50/1) to afford the title compound (1.20 g) as a yellow solid. MS obsd. (ESI⁺) [(M+H)⁺] : 478

### Step 4: 2-[[4-[[3-(3-fluoro-4-methoxy-phenyl)imidazo[1,2-a]pyrazin-8-yl]amino]-2-methyl-benzoyl]-methyl-amino]acetic acid

Into a stirred solution of methyl 2-[[4-[[3-(3-fluoro-4-methoxy-phenyl)imidazo[1,2-a]pyrazin-8-yl]amino]-2-methyl-benzoyl]-methyl-amino]acetate (1.10 g, 2.3 mmol) in methanol (20 mL) was added a solution of sodium hydroxide (276 mg, 6.91 mmol) in water (3.5 mL). The reaction was stirred at 30 °C for 4 h and then cooled and concentrated. The residue was diluted with H₂O (20 mL) and acidified with aq. HCl (1.0 M) until pH=5-6. The precipitate was collected by filtration and then triturated (acetonitrile) to afford the title compound (1.02 g) as a white solid, which was used without further purification in the subsequent steps. (ESI⁺) [(M+H)⁺] : 464.1

### Reference Example 12

### 2-[[4-[[3-(3-fluoro-4-methoxy-phenyl)imidazo[1,2-a]pyrazin-8-yl]amino]-2-methylphenyl]methyl-methyl-amino]-1-piperazin-1-yl-ethanone hydrochloride

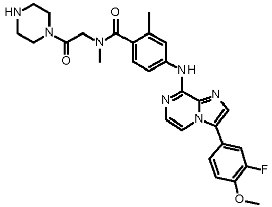

### Step 1: tert-butyl 4-[2-[[4-[[3-(3-fluoro-4-methoxy-phenyl)imidazo[1,2-a]pyrazin-8-yl]amino]-2-methyl-benzoyl]-methyl-amino]acetyl]piperazine-1-carboxylate

Into a stirred solution of Intermediate 77 (206. mg, 0.44 mmol), Boc-piperazine hydrochloride (119 mg, 0.53 mmol) and triethylamine (0.19 mL, 1.33 mmol) in DMF (3 mL) was added 1-propanephosphonic anhydride (425 mg, 0.67 mmol, 50% in ethyl acetate) slowly. The reaction was stirred at 15 °C for 4h. The reaction mixture was diluted with H₂O (10 mL) and extracted with ethyl acetate. The organic phase was washed with brine, dried over anhydrous sodium sulphate and concentrated under reduced pressure to give the title compound (300 mg) as a yellow oil which was used directly in the next step. MS obsd. (ESI⁺) [(M+H)⁺] : 632

### Step 2: 2-[[4-[[3-(3-fluoro-4-methoxy-phenyl)imidazo[1,2-a]pyrazin-8-yl]amino]-2-methyl-phenyl]methyl-methyl-amino]-1-piperazin-1-yl-ethanone hydrochloride

A mixture of tert-butyl 4-[2-[[4-[[3-(3-fluoro-4-methoxy-phenyl)imidazo[1,2-a]pyrazin-8-yl]amino]-2-methyl-benzoyl]-methyl-amino]acetyl]piperazine-1-carboxylate (200 mg, 0.32 mmol) and a solution of HCl in 1,4-dioxane (0.4 mL, 1.58 mmol) in methanol (2 mL) was stirred at 15 °C for 4 h. The reaction mixture was concentrated under reduced pressure and the residue was purified by prep-HPLC to afford the title compound (88 mg) as a white solid. MS obsd.

(ESI⁺) [(M+H)⁺]: 532

The following intermediates were prepared in analogy:

| Ex# | Name | Structure | MS ESI [M+H]+ | Starting Material |
|---|---|---|---|---|
| REF 13 | 4-[2-[[4-[[3-(3-fluoro-4-methoxyphenyl)imidazo[1,2-a]pyrazin-8-yl]amino]-2-methyl-phenyl]methyl-methyl-amino]acetyl] piperazin-2-one | 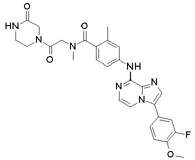 | 546.1 | Intermediate 77 and piperazin-2-one |
| REF 14 | N-[2-(dimethylamino)-2-oxo-ethyl]-4-[[3-(3-fluoro-4-methoxyphenyl)imidazo[1,2-a]pyrazin-8-yl]amino]-N,2-dimethyl-benzamide | 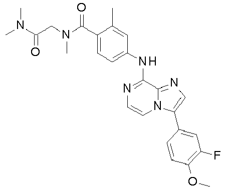 | 491.1 | Intermediate 77 and dimethyl amine |
| REF 15 | 4-[[3-(3-fluoro-4-methoxyphenyl)imidazo[1,2-a]pyrazin-8-yl]amino]-N,2-dimethyl-N-(2-morpholino-2-oxo-ethyl)benzamide | 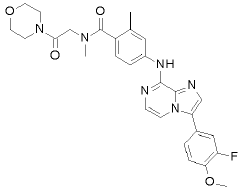 | 533.1 | Intermediate 77 and morpholine |
| REF 16 | N-[2-[3-[(dimethylamino)methyl]pyr rolidin-1-yl]-2-oxo-ethyl]-4-[[3-(3-fluoro-4-methoxyphenyl)imidazo[1,2-a]pyrazin-8-yl]amino]-N,2-dimethyl-benzamide | 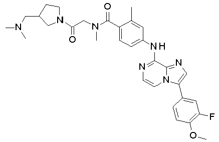 | 574.1 | Intermediate 77 and N, N-dimethyl-1-pyrro lidin-3-ylmethanamine dihydrochloride |

### Intermediate 78

### tert-Butyl N-[2-[2-[(4-amino-2-methyl-benzoyl)amino]ethoxy]ethyl]carbamate

### Step 1

### tert-Butyl N-[2-[2-[(2-methyl-4-nitro-benzoyl)amino]ethoxy]ethyl]carbamate

To a mixture of 2-methyl-4-nitro-benzoic acid (3.45 g, 19.04 mmol, 1 eq), N-Boc-2-(2-amino-ethoxy)-ethylamine (3.89 g, 19.04 mmol, 1 eq) and triethylamine (7.96 mL, 57.13 mmol, 3 eq) in THF (50 mL) was added 1-propanephosphonic anhydride in ethyl acetate (18.18 g, 28.57 mmol, 1.5 eq) at 25 °C. The mixture was stirred at 25 °C for 16 h. The reaction was concentrated to dryness and the residue was taken up in ethyl acetate (50 mL) and washed with 2 × 50 mL water then 1 × 50 mL brine. The combined organic layers were then separated and dried (MgSO₄) before concentration to dryness to afford the crude product. The product was purified by silica gel column chromatography (30% ethyl acetate / PE) to afford the desired product (5.08 g) as a colorless oil.

### Step 2

### tert-Butyl N-[2-[2-[(4-amino-2-methyl-benzoyl)amino]ethoxy]ethyl]carbamate

A mixture of tert-butyl N-[2-[2-[(2-methyl-4-nitro-benzoyl)amino]ethoxy]ethyl]carbamate (2.0 g, 4.35 mmol, 1 eq) and palladium/C (1.5 mmol, 0.350 eq) in methanol (20 mL) was stirred under H₂ (775 mmHg) at 25 °C for 16 h. The mixture was filtered and purified by flash column chromatography to afford the title product (1.12 g) as a light yellow oil. MS (ESI, m/z): 238 [M+H-Boc]⁺.

### Intermediate 79

### tert-Butyl (2-(2-(4-((3-iodoimidazo[1,2-a]pyrazin-8-yl)amino)-2-methylbenzamido)ethoxy)ethyl)carbamate

The title compound was prepared in analogy to Reference Example 17 step 1 from Intermediate 1 and tert-butyl (2-(2-aminoethoxy)ethyl)carbamate. MS (ESI, m/z): 581.3[M+H]⁺

### Reference Example 18

### N-(2-(2-aminoethoxy)ethyl)-4-((3-(2,3-difluoro-4-methoxyphenyl)imidazo[1,2-a]pyrazin-8-yl)amino)-2-methylbenzamide hydrochloride

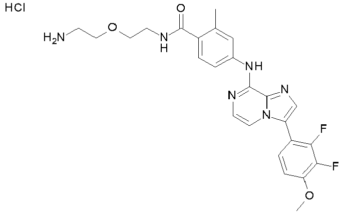

### Step 1:

### tert-Butyl (2-(2-(4-((3-iodoimidazo[1,2-a]pyrazin-8-yl)amino)-2-methylbenzamido)ethoxy)ethyl) carbamate

tert-Butyl (2-(2-aminoethoxy)ethyl)carbamate (104 mg, 510 µmol), diisopropylethylamine (132 mg, 178 µl, 1.02 mmol) and HATU (259 mg, 680 µmol) were added to a solution of 4-((3-iodoimidazo[1,2-a]pyrazin-8-yl)amino)-2-methylbenzoic acid (intermediate 1, 134 mg, 340 µmol) in DMF (5 mL). The mixture was stirred overnight at room temperature. The reaction mixture was poured into 5 mL H₂O and extracted with acetonitrile. The organic layers were dried over sodium sulphate and concentrated *in vacuo.* The crude material was purified by flash chromatography (silica gel, 50% to 100% ethyl acetate in heptane) to give the title compound (112 mg) as a yellow solid. MS (ESI, m/z): 581.3 [M+H]⁺.

### Step 2:

### tert-Butyl (2-(2-(4-((3-(2,3-difluoro-4-methoxyphenyl)imidazo[1,2-a]pyrazin-8-yl)amino)-2-methylbenzamido)ethoxy)ethyl)carbamate

tert-butyl (2-(2-(4-((3-iodoimidazo[1,2-a]pyrazin-8-yl)amino)-2-methylbenzamido)ethoxy)ethyl) carbamate (50 mg, 86.1 µmol), (2,3-difluoro-4-methoxyphenyl)boronic acid (24.3 mg, 129 µmol), Na₂CO₃ (18.3 mg, 172 µmol) and 1,1'-bis(diphenylphosphino)ferrocenedichloro palladium(II) dichloromethane complex (7.03 mg, 8.61 µmol) in dioxane (1000 µl) and water (100 µl) was heated in a microwave at 80 °C for 30 min. The crude reaction mixture was purified by prep. HPLC to give the title compound (28 mg) as a white solid. MS (ESI, m/z): 597.4

[M+H]⁺.

### Step 3:

### N-(2-(2-aminoethoxy)ethyl)-4-((3-(2,3-difluoro-4-methoxyphenyl)imidazo [1,2-a]pyrazin-8-yl)amino)-2-methylbenzamide hydrochloride

tert-Butyl (2-(2-(4-((3-(2,3-difluoro-4-methoxyphenyl)imidazo[1,2-a]pyrazin-8-yl)amino)-2-methylbenzamido)ethoxy)ethyl)carbamate (28 mg, 46.9 µmol) was combined with 3M HCl in MeOH (235 µl, 704 µmol) to give a light yellow solution. The reaction mixture was stirred at room temperature overnight. After removal of the volatiles, the solid obtained was dried *in vacuo* to give the title product (23.3 mg) as a light yellow solid. MS (ESI, m/z): 497.2 [M+H]⁺.

### Reference Example 17

### N-(2-(2-aminoethoxy)ethyl)-2-chloro-4-((3-(3-fluoro-4-methoxyphenyl)imidazo[1,2-a]pyrazin-8-yl)amino)benzamide hydrochloride

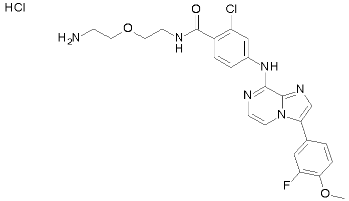

### Step 1:

### tert-Butyl (2-(2-(2-chloro-4-((3-(3-fluoro-4-methoxyphenyl)imidazo[1,2-a]pyrazin-8-yl)amino)benzamido)ethoxy)ethyl) carbamate.

To 2-chloro-4-((3-(3-fluoro-4-methoxyphenyl)imidazo[1,2-a]pyrazin-8-yl)amino)benzoic acid (intermediate 2, 35 mg, 84.8 µmol) in DMF (1 mL) was added tert-butyl (2-(2-aminoethoxy)ethyl)carbamate (26 mg, 127 µmol), HATU (64.5 mg, 170 µmol) and diisopropylethylamine (32.9 mg, 44.4 µL, 254 µmol) followed by stirring at room temperature for 1 h. The crude reaction mixture was purified by prep. HPLC to give the title compound (31 mg) as an orange solid. MS (ESI, m/z): 599.4 [M+H]⁺.

### Step 2:

### N-(2-(2-aminoethoxy)ethyl)-2-chloro-4-((3-(3-fluoro-4-methoxyphenyl)imidazo[1,2-a]pyrazin-8-yl)amino)benzamide hydrochloride

The title compound was obtained as a white solid (31 mg) in analogy to Reference Example 18, step 3 from tert-butyl (2-(2-(2-chloro-4-((3-(3-fluoro-4-methoxyphenyl)imidazo[1,2-a]pyrazin-8-yl)amino)benzamido)ethoxy)ethyl) carbamate. MS (ESI, m/z): 500.3 [M+H]⁺.

The following examples were prepared in analogy to Reference Example 17, the deprotection step 2 was only applied for intermediates derived from Boc-protected amines.

| Ex. | Name | Structure | MS ESI [M+H]⁺ | Starting Material |
|---|---|---|---|---|
| REF 19 | N-(6-aminohexyl)-4-((3-(4-methoxyphenyl)imid azo[1,2-a]pyrazin-8-yl)amino)-2-methylbenzamide hydrochloride | 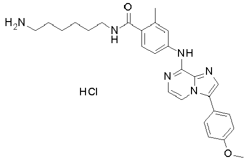 | 473.3 | Intermediate 4 and tert-butyl (6-aminohexyl)carbamat e hydrochloride |
| REF 20 | (4-(2-Aminoethyl)piperidi n-1-yl)(4-((3-(4-(difluoromethoxy)ph enyl)imidazo[1,2-a]pyrazin-8-yl)amino)-2-methylphenyl)metha none hydrochloride | 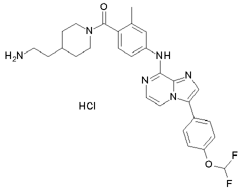 | 521.1 | Intermediate 7 and tert-butyl (2-(piperidin-4-yl)ethyl)carbamate |
| REF 21 | 4-((3-(3-Fluoro-4-methoxyphenyl)imid azo[1,2-a]pyrazin-8-yl)amino)-N,2-dimethyl-N-(2-(piperazin-1-yl)ethyl)benzamide hydrochloride | 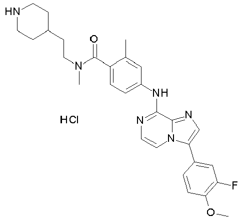 | 517.3 | Intermediate 6 and tert-butyl 4-(2-(methylamino)ethyl)p iperidine-1-carboxylate |
| REF 22 | (2-Chloro-4-((3-(3-fluoro-4-methoxyphenyl)imid azo[1,2-a]pyrazin-8-yl)amino)phenyl)(4-(2-(dimethylamino)ethy l)piperazin-1-yl)methanone | 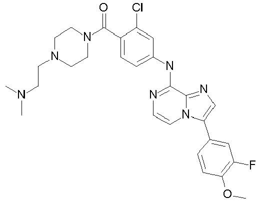 | 553.3 | Intermediate 2 and N,N-dimethyl-2-(piperazin-1-yl)ethanamine |
| REF 23 | 2-Chloro-4-((3-(3-fluoro-4-methoxyphenyl)imid azo[1,2-a]pyrazin-8-yl)amino)-N-methyl-N-(2-(piperazin-1-yl)ethyl)benzamide hydrochloride | 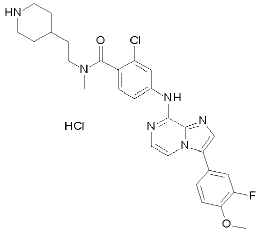 | 537.0 | Intermediate 2 and tert-butyl 4-(2-(methylamino)ethyl)p iperazine-1-carboxylate |
| REF 24 | (4-(Aminomethyl)piper idin-1-yl)(2-chloro-4-((3-(3-fluoro-4-methoxyphenyl)imid azo[1,2-a]pyrazin-8-yl)amino)phenyl)met hanone hydrochloride | 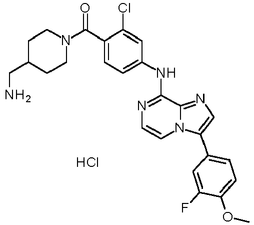 | 510.1 | Intermediate 2 and tert-butyl (piperidin-4-ylmethyl)carbamate |
| REF 25 | N-(2-(2-aminoethoxy)ethyl)-2-chloro-4-((3-(4-methoxyphenyl)imid azo[1,2-a]pyrazin-8-yl)amino)benzamide hydrochloride | 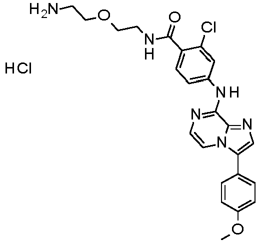 | 481.2 | Intermediate 9 and tert-butyl (2-(2-aminoethoxy)ethyl)ca rbamate |
| REF 26 | 1-(4-((3-(4-(difluoromethoxy)ph enyl)imidazo[1,2-a]pyrazin-8-yl)amino)-2-methylbenzoyl)piper idine-4-carboxamide | | 521.7 | Intermediate 7 and piperidine-4-carboxamide |
| REF 27 | (4-((3-(4-methoxyphenyl)imid azo[1,2-a]pyrazin-8-yl)amino)-2-methylphenyl)(4-methylpiperazin-1- yl)methanone | 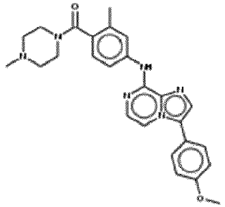 | 456 | Intermediate 4 and 1-methyl piperazine |
| REF 28 | N-(2-((2-hydroxyethyl)amino) ethyl)-4-((3-(4-methoxyphenyl)imid azo[1,2-a]pyrazin-8-yl)amino)-2-methylbenzamide | 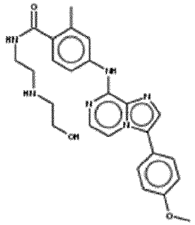 | 460 | Intermediate 4 and 2-((2-aminoethyl)amino)et hanol |
| REF 29 | 4-((3-(4-methoxyphenyl)imid azo[1,2-a]pyrazin-8-yl)amino)-N,N,2-trimethylbenzamide | 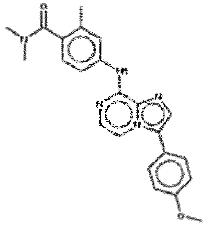 | 401 | Intermediate 4 and dimethylamine hydrochloride |
| REF 30 | (4-((3-(4-methoxyphenyl)imid azo[1,2-a]pyrazin-8-yl)amino)-2-methylphenyl)(morp holino)methanone | 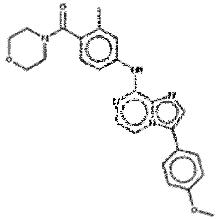 | 443 | Intermediate 4 and morpholine |
| REF 31 | (4-hydroxypiperidin-1-yl)(4-((3-(4-methoxyphenyl)imid azo[1,2-a]pyrazin-8-yl)amino)-2-methylphenyl)metha none | 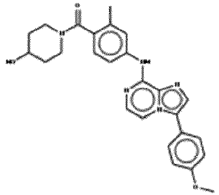 | 457 | Intermediate 4 and piperidin-4-ol hydrochloride |
| REF 32 | 4-((3-(4-methoxyphenyl)imid azo[1,2-a]pyrazin-8-yl)amino)-N,2-dimethylbenzamide | 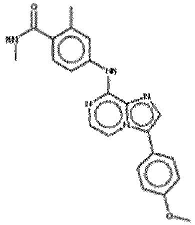 | 387 | Intermediate 4 and methanamine hydrochloride |
| 1 | 4-((3-(4-methoxyphenyl)imid azo[1,2-a]pyrazin-8-yl)amino)-2-methyl-N-(1-methylpiperidin-4-yl)benzamide | 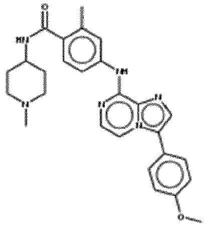 | 470 | Intermediate 4 and 1-methylpiperidin-4-amine hydrochloride |
| REF 33 | 4-((3-(4-methoxyphenyl)imid azo[1,2-a]pyrazin-8-yl)amino)-2-methyl-N-(2-(methylamino)-2-oxoethyl) benzamide | 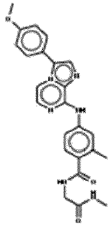 | 444 | Intermediate 4 and 2-amino-N-methyl acetamide hydrochloride |
| REF 34 | N-(2-(dimethylamino)-2-oxoethyl)-4-((3-(4-methoxyphenyl)imid azo[1,2-a]pyrazin-8-yl)amino)-2-methylbenzamide | 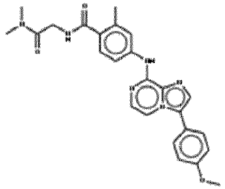 | 458 | Intermediate 4 and 1,1-dimethylurea hydrochloride |
| REF 35 | N-(2-amino-2-oxoethyl)-4-((3-(4-methoxyphenyl)imid azo[1,2-a]pyrazin-8-yl)amino)-2-methylbenzamide | 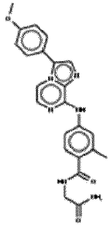 | 430 | Intermediate 4 and 2-aminoacetamide hydrochloride |
| REF 36 | N-(2-(dimethylamino)ethy 1)-4-((3-(4-methoxyphenyl)imid azo[1,2-a]pyrazin-8-yl)amino)-N,2-dimethylbenzamide | 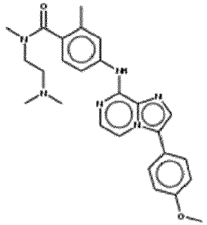 | 458 | Intermediate 4 and N1,N1,N2-trimethylethane-1,2-diamine |
| REF 37 | N-(2-hydroxyethyl)-4-((3-(4-methoxyphenyl)imid azo[1,2-a]pyrazin-8-yl)amino)-N,2-dimethylbenzamide | 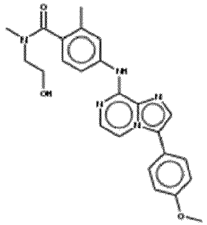 | 431 | Intermediate 4 and 2-(methylamino)ethano 1 |
| REF 38 | N-(2-(4-hydroxypiperidi-1-yl)-2-oxoethyl)-4-((3-(4-methoxyphenyl)imid azo[1,2-a]pyrazin-8-yl)amino)-2-methylbenzamide | 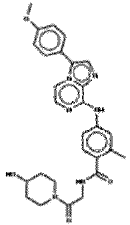 | 514 | Intermediate 4 and 2-amino-1-(4-hydroxypiperidin-1-yl)ethanone |
| REF 39 | N-(6-aminohexyl)-4-((3-(4-(difluoromethoxy)ph enyl)imidazo[1,2-a]pyrazin-8-yl)amino)-2-methylbenzamide hydrochloride | | 509.2 | Intermediate 7 and tert-butyl (6-aminohexyl)carbamat e |
| REF 40 | (4-(1H-1,2,4-triazol-1-yl)piperidin-1-yl)(4-((3-(4-(difluoromethoxy)ph enyl)imidazo[1,2-a]pyrazin-8-yl)amino)-2-methylphenyl)metha none | 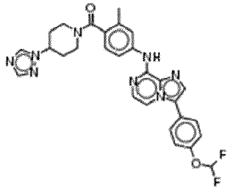 | 545.6 | Intermediate 7 and 4-(1H-1,2,4-triazol-1-yl)piperidine |
| REF 41 | 1-(1-(4-((3-(4-(difluoromethoxy)ph enyl)imidazo[1,2-a]pyrazin-8-yl)amino)-2-methylbenzoyl)piper idin-4-yl)imidazolidin-2-one | 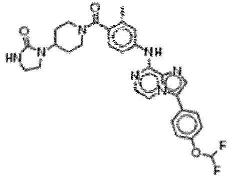 | 562.7 | Intermediate 7 and 1-(piperidin-4-yl)imidazolidin-2-one |
| REF 26 | 1-(4-((3-(4-(difluoromethoxy)ph enyl)imidazo[1,2-a]pyrazin-8-yl)amino)-2-methylbenzoyl)piper idine-4-carboxamide | 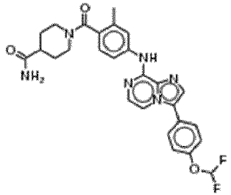 | 521.7 | Intermediate 7 and piperidine-4-carboxamide |
| REF 42 | 1-(4-((3-(4-(difluoromethoxy)ph enyl)imidazo[1,2-a]pyrazin-8-yl)amino)-2-methylbenzoyl)piper idine-3-carboxamide | 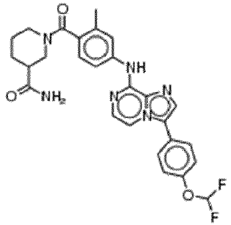 | 521.2 | Intermediate 7 and piperidine-3-carboxamide |
| REF 43 | ethyl (1-(4-((3-(4-(difluoromethoxy)ph enyl)imidazo[1,2-a]pyrazin-8-yl)amino)-2-methylbenzoyl)piper idin-4-yl)carbamate | 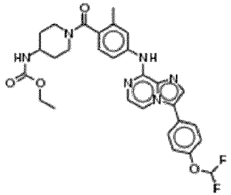 | 565.4 | Intermediate 7 and ethyl piperidin-4-ylcarbamate |
| REF 44 | (4-((3-(4-(difluoromethoxy)ph enyl)imidazo[1,2-a]pyrazin-8-yl)amino)-2-methylphenyl)(4-(pyrimidin- 2-yloxy)piperidin-1-yl)methanone | 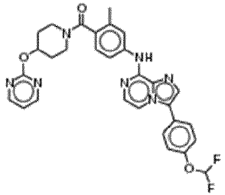 | 572.5 | Intermediate 7 and 2-(piperidin-4-yloxy)pyrimidine dihydrochloride |
| REF 45 | (4-((3-(4-(difluoromethoxy)ph enyl)imidazo[1,2-a]pyrazin-8-yl)amino)-2-methylphenyl)(4-(4-methyl-4H-1,2,4-triazol-3-yl)piperidin-1-yl)methanone | 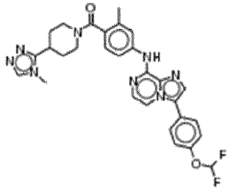 | 559.5 | Intermediate 7 and 4-(4-methyl-4H-1,2,4-triazol-3-yl)piperidine |
| REF 46 | 2-chloro-4-((3-(3-fluoro-4-methoxyphenyl)imid azo[1,2-a]pyrazin-8-yl)amino)-N-methylbenzamide | | 426.3 | Intermediate 2 and methylamine hydrochloride |
| REF 47 | (1-(4-((3-(3-fluoro-4-methoxyphenyl)imid azo[1,2-a]pyrazin-8-yl)amino)-2-methylbenzoyl)piper idin-4-yl)(piperazin-1-yl)methanone hydrochloride | | 572.3 | Intermediate 6 and tert-butyl piperazine-1-carboxylate |
| REF 48 | (4-(aminomethyl)piperi din-1-yl)(2-bromo-4-((3-(4-(difluoromethoxy)ph enyl)imidazo[1,2-a]pyrazin-8-yl)amino)phenyl)met hanone hydrochloride | | 573.3 | Intermediate 10 and tert-butyl (piperidin-4-ylmethyl)carbamate |
| REF 49 | 2-chloro-4-((3-(4-(difluoromethoxy)ph enyl)imidazo[1,2-a]pyrazin-8-yl)amino)-N-methyl-N-(2-(piperidin-4-yl)ethyl)benzamide hydrochloride | | 555.2 | Intermediate 11 and tert-butyl 4-(2-(methylamino)ethyl)p iperidine-1-carboxylate |
| REF 50 | 1-(4-((3-(4-(difluoromethoxy)ph enyl)imidazo[1,2-a]pyrazin-8-yl)amino)-2-methylbenzoyl)-N-methylpiperidine-4-carboxamide | 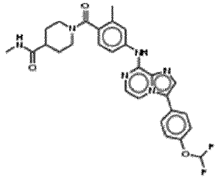 | 535.3 | Intermediate 7 and N-methylpiperidine-4-carboxamide |
| REF 51 | N-((1-carbamimidoylpiperi din-4-yl)methyl)-4-((3-(4-(difluoromethoxy)ph enyl)imidazo[1,2-a]pyrazin-8-yl)amino)-2-methylbenzamide | 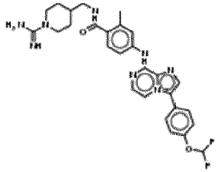 | 549.3 | Intermediate 7 and 4-(aminomethyl)piperid ine-1-carboximidamide hydrochloride |
| REF 52 | (4-((1H-pyrazol-1-yl)methyl)piperidin-1-yl)(4-((3-(4-(difluoromethoxy)ph enyl)imidazo[1,2-a]pyrazin-8-yl)amino)-2-methylphenyl)metha none | 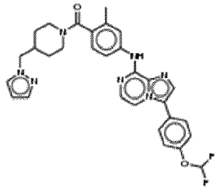 | 558.2 | Intermediate 7 and 4-((1H-pyrazol-1-yl)methyl)piperidine |
| REF 53 | (4-((3-(4-(difluoromethoxy)ph enyl)imidazo[1,2-a]pyrazin-8-yl)amino)-2-methylphenyl)(1-oxa-4,9-diazaspiro[5.5]undec an-9-yl)methanone | 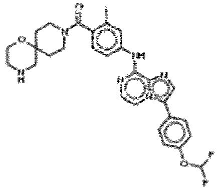 | 549.2 | Intermediate 7 and 1-oxa-4,9-diazaspiro[5.5]undec ane |
| 2 | 4-((3-(4-(difluoromethoxy)ph enyl)imidazo[1,2-a]pyrazin-8-yl)amino)-2-methyl-N-(quinuclidin-3-yl)benzamide | 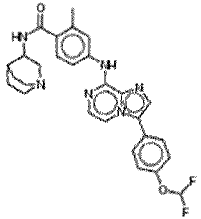 | 519.3 | Intermediate 7 and quinuclidin-3-amine dihydrochloride |
| REF 54 | N-(2-(2-chloroethoxy) ethyl)-4-((3-(4-methoxyphenyl)imid azo[1,2-a]pyrazin-8-yl)amino)-2-methyl benzamide | 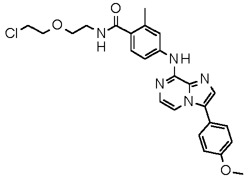 | 480.3 | Intermediate 4 and 2-(2-chloroethoxy)ethana mine hydrochloride |
| REF 55 | (4-(aminomethyl)piperi din-1-yl)(4-((3-(4-(difluoromethoxy)ph enyl)imidazo [1,2-a]pyrazin-8-yl)amino)-2-methylphenyl)metha none hydrochloride | 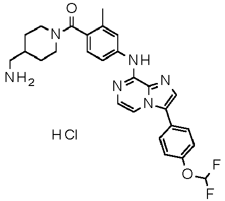 | 505.4 [M-H]⁻ | Intermediate 7 and tert-butyl (piperidin-4-ylmethyl)carbamate |
| REF 56 | (4-(aminomethyl)piperi din-1-yl)(4-((3-(4-methoxyphenyl)imid azo[1,2-a]pyrazin-8-yl)amino)-2-methylphenyl)metha none hydrochloride | 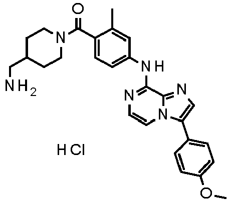 | 508.0 | Intermediate 4 and tert-butyl (piperidin-4-ylmethyl)carbamate |
| REF 57 | (4-(azetidin-3-yl)piperidin-1-yl)(4-((3-(4-(difluoromethoxy)ph enyl)imidazo[1,2-a]pyrazin-8-yl)amino)-2-methylphenyl)metha none hydrochloride | 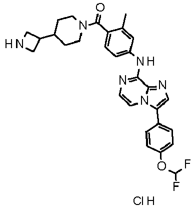 | 533.2 | Intermediate 7 and tert-butyl 3-(piperidin-4-yl)azetidine-1-carboxylate |
| REF 58 | N-(2-(2-aminoethoxy)ethyl)-4-((3-(4-chloro-2,3-difluorophenyl)imid azo[1,2-a]pyrazin-8-yl)amino)-2-ethylbenzamide hydrochloride | | 515.1 | Intermediate 12 and tert-butyl (2-(2-aminoethoxy)ethyl)ca rbamate |
| REF 59 | 4-((3-(4-(difluoromethoxy)ph enyl)imidazo[1,2-a]pyrazin-8-yl)amino)-2-methyl-N-(5-morpholinopentyl)be nzamide | | 565.2 | Intermediate 7 and 5-morpholinopentan-1-amine |
| REF 60 | N-(2-(2-amino-2-oxoethoxy)ethyl)-4-((3-(4-(difluoromethoxy)ph enyl)imidazo[1,2-a]pyrazin-8-yl)amino)-2-methylbenzamide | 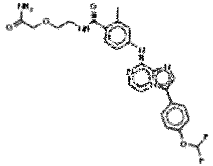 | 511.4 | Intermediate 7 and 2-(2-aminoethoxy)acetami de |
| REF 61 | 4-((3-(4-(difluoromethoxy)ph enyl)imidazo[1,2-a]pyrazin-8-yl)amino)-2-methyl-N-(2-(2-(methylamino)-2-oxoethoxy)ethyl)ben zamide | 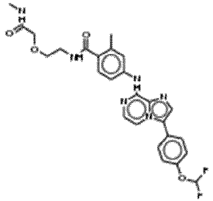 | 525.4 | Intermediate 7 and 2-(2-aminoethoxy)-N-methyl acetamide |
| REF 62 | 4-((3-(4-(difluoromethoxy)ph enyl)imidazo[1,2-a]pyrazin-8-yl)amino)-N-(2-(2-(2,4-dioxooxazolidin-3-yl)ethoxy)ethyl)-2-methylbenzamide | 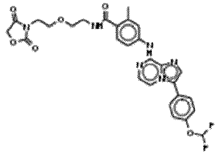 | 581.4 | Intermediate 7 and 3-(2-(2-aminoethoxy)ethyl)o xazolidine-2,4-dione |
| REF 63 | 4-((3-(4-(difluoromethoxy)ph enyl)imidazo[1,2-a]pyrazin-8-yl)methyl)-N,2-dimethylbenzamide | | 422 [M-H]⁻ | Intermediate 7 and methylamine hydrochloride |
| REF 64 | 4-((3-(3-chloro-4-methoxyphenyl)imid azo[1,2-a]pyrazin-8-yl)amino)-N,2-dimethylbenzamide | | 422.3 | Intermediate 14 and methylamine (2M in THF) |
| REF 65 | (4-((3-(3-chloro-4-methoxyphenyl)imid azo[1,2-a]pyrazin-8-yl)amino)-2-methyl phenyl)(morp holino)methanone | | 478.2 | Intermediate 14 and morpholine |
| REF 66 | 4-((3-(3-chloro-4-methoxyphenyl)imid azo[1,2-a]pyrazin-8-yl)amino)-N-(2-(dimethylamino)ethy 1)-N-methylbenzamide | | 479.7 | Intermediate 16 and N1,N1,N2-trimethylethane-1,2-diamine |
| REF 67 | N-(2-aminoethyl)-4-[[3-(3-chloro-4-methoxyphenyl)imid azo[1,2-a]pyrazin-8-yl]amino]benzamide ;hydrochloride | 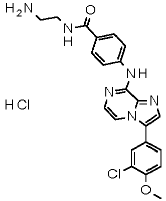 | 435.3 | Intermediate 16 and tert-butyl (2-aminoethyl)carbamat e |
| REF 68 | 4-[[3-(3-chloro-4-methoxyphenyl)imid azo[1,2-a]pyrazin-8-yl]amino]-*N*-[2-(methylamino)ethyl] benzamide;hydrochl oride | 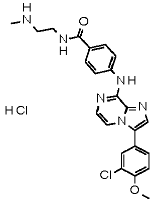 | 449.3 | Intermediate 16 and tert-butyl (2-aminoethyl)(methyl)c arbamate |
| REF 69 | 4-((3-(3-chloro-4-methoxyphenyl)imid azo[1,2-a]pyrazin-8-yl)amino)-N-(2-(piperazin-1-yl)ethyl)benzamide hydrochloride | 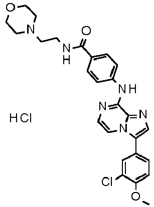 | 506.2 | Intermediate 16 and tert-butyl 4-(2-aminoethyl)piperazin e-1-carboxylate |
| REF 70 | (4-((3-(3-chloro-4-methoxyphenyl)imid azo[1,2-a]pyrazin-8-yl)amino)-2-methylphenyl)(4-methylpiperazin-1-yl)methanone | 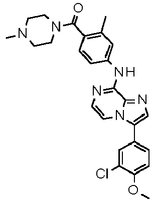 | 389.4 [M-H]⁻ | Intermediate 14 and 1-methyl piperazine |
| REF 71 | N-(2-aminoethyl)-4-((3-(4-methoxyphenyl)imid azo[1,2-a]pyrazin-8-yl)amino)-2-methylbenzamide | 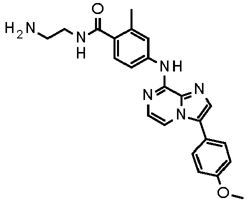 | 417.2 | Intermediate 4 and tert-butyl (2-aminoethyl)carbamat e |
| REF 72 | N-(2-aminoethyl)-4-((3-(4-methoxyphenyl)imid azo[1,2-a]pyrazin-8-yl)amino)-N,2-dimethylbenzamide | 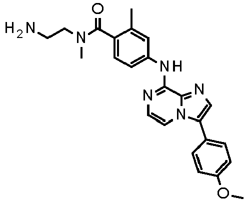 | 431.2 | Intermediate 4 and tert-butyl (2-(methylamino)ethyl)c arbamate |
| REF 73 | N-(3-aminopropyl)-4-((3-(4-methoxyphenyl)imid azo[1,2-a]pyrazin-8-yl)amino)-N-methylbenzamide hydrochloride | 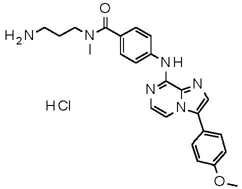 | 431.2 | Intermediate 17 and tert-butyl (3-(methylamino)propyl )carbamate hydrochloride |
| REF 74 | N-(3-aminopropyl)-4-((3-(4-methoxyphenyl)imid azo[1,2-a]pyrazin-8-yl)amino)-N,2-dimethylbenzamide hydrochloride | 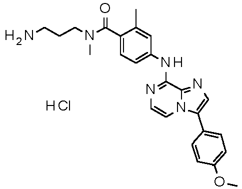 | 445.2 | Intermediate 4 and tert-butyl (3-(methylamino)propyl )carbamate hydrochloride |
| REF 75 | 4-[[3-(3-fluoro-4-methoxyphenyl)imidazo[1,2-a]pyrazin-8-yl]amino]-N,2-dimethyl-N-(tetrahydropyran-4-ylmethyl)benzamide ;2,2,2-trifluoroacetic acid | 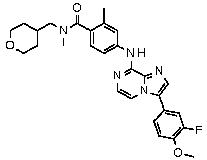 | 504.1 | Intermediate 6 and N-methyl- 1 -(tetrahydro-2H-pyran-4-yl)methanamine |
| REF 76 | [2-[(dimethylamino)me thyl]morpholin-4-yl]-[4-[[3-(3-fluoro-4-methoxyphenyl)imidazo[1,2-a]pyrazin-8-yl]amino]-2-methylphenyl] methanone ;2,2,2-trifluoroacetic acid | 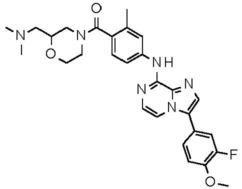 | 519.1 | Intermediate 6 and N,N-dimethyl-1-(morpholin- 2-yl)methanamine |
| REF 77 | N-[3-(1,1-dioxo-1,4-thiazinan-4-yl)propyl]-4-[[3-(3-fluoro-4-methoxyphenyl)imidazo[1,2-a]pyrazin-8-yl]amino]-2-methylbenzamide;2,2,2-trifluoroacetic acid | 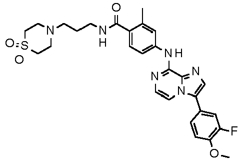 | 567.2 | Intermediate 6 and 4-(3-aminopropyl)thiomor pholine 1,1-dioxide |
| REF 78 | 4-[[3-(3-fluoro-4-methoxyphenyl)imidazo[1,2-a]pyrazin-8-yl]amino]-2-methyl-N-(2-tetrahydropyran-4-ylethyl)benzamide; 2, 2,2-trifluoroacetic acid | 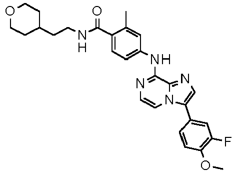 | 504.2 | Intermediate 6 and 2-(tetrahydro-2H-pyran-4-yl)ethanamine |
| REF 79 | 4-[[3-(3-fluoro-4-methoxyphenyl)imidazo[1,2-a]pyrazin-8-yl]amino]-N,2-dimethyl-N-(3-pyridylmethyl)benza mide | 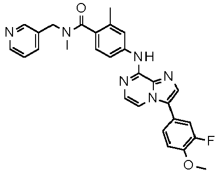 | 497.3 | Intermediate 6 and N-methyl-N-(3-pyridylmethyl)amine |
| REF 80 | 4-[[3-(3-fluoro-4-methoxyphenyl)imidazo[1,2-a]pyrazin-8-yl]amino]-N,2-dimethyl-N-(pyrimidin-4-ylmethyl)benzamide | 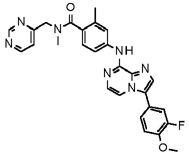 | 498.0 | Intermediate 6 and N-methyl-1-pyrimidin-4-yl-methanamine |
| REF 81 | 2-chloro-4-[[3-(2,3-difluoro-4-methoxyphenyl)imidazo[1,2-a]pyrazin-8-yl]amino]-N-(tetrahydropyran-4-ylmethyl)benzamide ;2,2,2-trifluoroacetic acid | 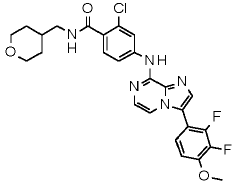 | 528.1 | Intermediate 20 and (tetrahydro-2H-pyran-4-yl)methanamine |
| REF 82 | 2-chloro-4-[[3-(2,3-difluoro-4-methoxyphenyl)imidazo[1,2-a]pyrazin-8-yl]amino]-N-methyl-N-(tetrahydropyran-4-ylmethyl)benzamide;2,2,2-trifluoroacetic acid | 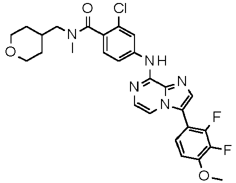 | 542.1 | Intermediate 20 and N-methyl-1-(tetrahydro-2H-pyran-4-yl)methanamine |
| REF 83 | 2-chloro-4-[[3-(2,3-difluoro-4-methoxyphenyl)imidazo[1,2-a]pyrazin-8-yl]amino]-N-(tetrahydrothiopyran -4-ylmethyl)benzamide | 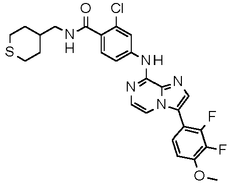 | 544.2 | Intermediate 20 and (tetrahydro-2H-thiopyran-4-yl)methanamine |
| REF 84 | [4-(2-aminoethyl)piperazi n-1-yl]-[4-[[3-[4-(difluoromethoxy)ph enyl]imidazo[1,2-a]pyrazin-8-yl]amino]-2-methylphenyl]methanone | 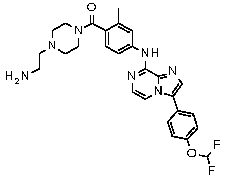 | 522.2 | Intermediate 7 and 2-(piperazin-1-yl)ethanamine |
| REF 85 | 2-chloro-4-[[3-[4-(difluoromethoxy)-2,3-difluorophenyl]imidazo[1,2-a]pyrazin-8-yl]amino]-N-(4-piperidylmethyl) benzamide;2,2,2-trifluoroacetic acid | 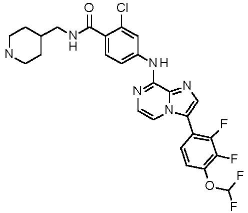 | 563.1 | Intermediate 22 and piperidin-4-ylmethanamine |
| REF 86 | 2-chloro-4-[[3-(2,3-difluoro-4-methoxyphenyl)imidazo[1,2-a]pyrazin-8-yl]amino]-N-methyl-N-(2-piperazin-1-ylethyl)benzamide; 2, 2,2-trifluoroacetic acid | 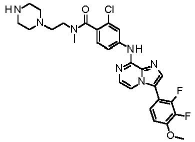 | 556.2 | Intermediate 20 and tert-butyl 4-(2-(methylamino)ethyl)p iperazine-1-carboxylate |
| REF 87 | 4-[[3-[4-(difluoromethoxy)ph enyl]imidazo[1,2-a]pyrazin-8-yl]amino]-N,2-dimethyl-N-(2-piperazin-1-ylethyl)benzamide | 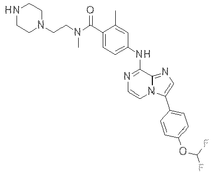 | 536.3 | Intermediate 7 and tert-butyl 4-(2-(methylamino)ethyl)p iperazine-1-carboxylate |
| REF 88 | tert-butyl 4-[2-[[4-[[3-[4-(difluoromethoxy)ph enyl]imidazo[1,2-a]pyrazin-8-yl] amino] -2-methylbenzoyl]amino]ethyl ]piperazine-1-carboxylate | 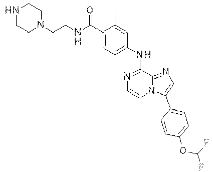 | 522.2 | Intermediate 7 and tert-butyl 4-(2-aminoethyl)piperazin e-1-carboxylate |
| REF 89 | tert-butyl 3-[[[4-[[3-[4-(difluoromethoxy)ph enyl]imidazo[1,2-a]pyrazin-8-yl]amino]-2-methyl-benzoyl]amino]methyl]pyrrolidine-1-carboxylate | 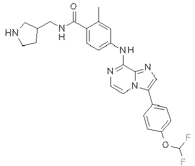 | 492.3 | Intermediate 7 and tert-butyl 3-(aminomethyl)pyrroli dine-1-carboxylate |
| REF 90 | 2-[3-chloro-4-[8-[3-chloro-4-[4-[(dimethylamino)me thyl]piperidine-1-carbonyl]anilino]imi dazo[1,2-a]pyrazin-3-yl]-2-fluoro-phenoxy] acetonitrile; formic acid | 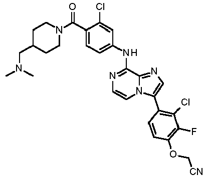 | 596.2 | Intermediate 80 and N,N-dimethyl-1-(4-piperidyl) methanamine |
| REF 91 | 2-[3-chloro-4-[8-[3-chloro-4-[4-[2-(dimethylamino)ethy l]piperazine-1-carbonyl] anilino]imidazo[1,2-a]pyrazin-3-yl]-2-fluoro-phenoxy] acetonitrile | 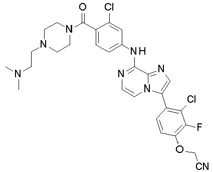 | 611.2 | Intermediate 80 and 1-(2-dimethylaminoethyl) piperazine |
| REF 92 | [4-(aminomethyl)-1-piperidyl]-[4-[[3-(2-chloro-5-fluoro-4-methoxyphenyl)imidazo[1,2-a]pyrazin-8-yl]amino]-2-methyl-phenyl]methanone hydrochloride | 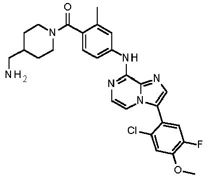 | 523.2 | Intermediate 26 and 4-(tert-butoxy carbonyl amino methyl) piperidine |
| REF 93 | N-(2-(2-aminoethoxy)ethyl)-4-((3-(4-methoxyphenyl)imid azo[1,2-a]pyrazin-8-yl)amino)-2-methylbenzamide hydrochloride | 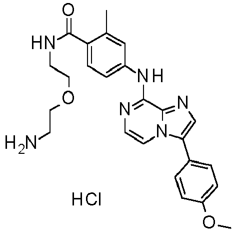 | 461.2 | Intermediate 4 and tert-butyl (2-(2-aminoethoxy)ethyl)ca rbamate |
| REF 94 | N-(2-(2-aminoethoxy)ethyl)-4-((3-(4-methoxyphenyl)imid azo[1,2-a]pyrazin-8-yl)amino)benzamide hydrochloride | 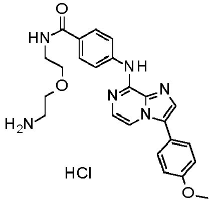 | 447.2 | Intermediate 17 and tert-butyl (2-(2-aminoethoxy)ethyl)ca rbamate |
| REF 95 | 4-((3-(4-methoxyphenyl)imid azo[1,2-a]pyrazin-8-yl)methyl)-2-methyl-N-(2-(1-methylpiperidin-4-yl)ethyl)benzamide | 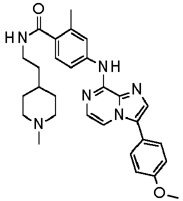 | 497 [M-H]⁻ | Intermediate 4 and 2-(1 -methylpiperidin-4-yl)ethanamine |
| REF 96 | 4-((3-(4-methoxyphenyl)imid azo[1,2-a]pyrazin-8-yl)methyl)-2-methyl-N-(2-(4-methylpiperazin-1-yl)ethyl)benzamide | 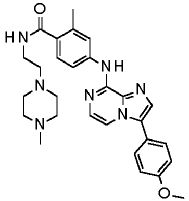 | 500 | Intermediate 4 and 2-(4-methylpiperazin-1-yl)ethanamine hydrochloride |
| REF 97 | 4-((3-(4-(difluoromethoxy)ph enyl)imidazo[1,2-a]pyrazin-8-yl)amino)-N-(2-(dimethylamino)ethy 1)-N,2-dimethylbenzamide | 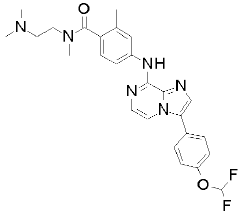 | 495 | Intermediate 7 and N1,N1,N2-trimethylethane-1,2-diamine |
| REF 98 | N-(3-aminopropyl)-4-((3-(4-methoxyphenyl)imid azo[1,2-a]pyrazin-8-yl)amino)-2-methylbenzamide hydrochloride | 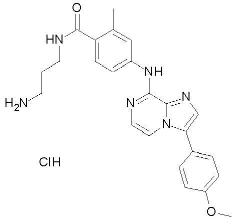 | 431 | Intermediate 4 and tert-butyl (3-aminopropyl)carbam ate |
| REF 99 | 4-[[3-(4-methoxyphenyl)imid azo[1,2-a]pyrazin-8-yl]amino]-2-methyl-N-[(1-methylpiperidin-4-yl)methyl]benzamid e | 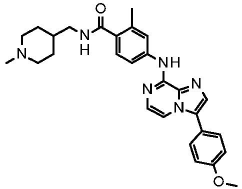 | 483.3 [M-H]⁻ | Intermediate 4 and N,1-dimethylpiperidin-4-amine |
| REF 100 | 4-((3-(4-(difluoromethoxy)ph enyl)imidazo [1,2-a]pyrazin-8-yl)amino)-N-methyl-2-(trifluoromethyl)ben zamide | 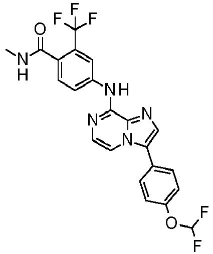 | 448.3 | Intermediate 18 and methanamine hydrochloride |
| REF 101 | 4-((3-(4-(difluoromethoxy)ph enyl)imidazo[1,2-a]pyrazin-8-yl)amino)-N-methyl-2-nitrobenzamide | 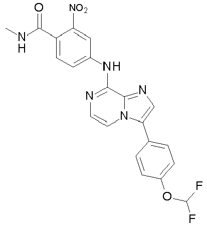 | 455.3 | Intermediate 19 and methanamine hydrochloride |
| REF 102 | N-(2-(2-(2-aminoethoxy)ethoxy )ethyl)-4-((3-(4-(difluoromethoxy)ph enyl)imidazo[1,2-a]pyrazin-8-yl)amino)-2-methyl benzamide | 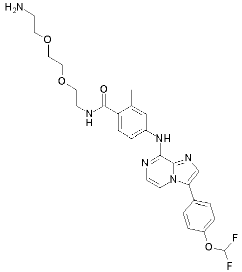 | 539.5 [M-H]⁻ | Intermediate 7 and tert-butyl (2-(2-(2-aminoethoxy)ethoxy) ethyl) carbamate |
| REF 103 | N-(2-(2-(2-(2-aminoethoxy)ethoxy )ethoxy)ethyl)-4-((3-(4-(difluoromethoxy)ph enyl)imidazo[1,2-a]pyrazin-8-yl)amino)-2-methyl benzamide | 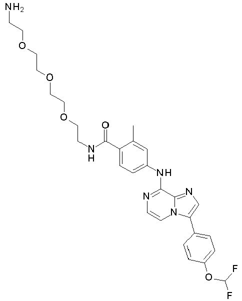 | 583.5 [M-H]⁻ | Intermediate 7 and tert-butyl (2-(2-(2-(2-aminoethoxy)ethoxy) ethoxy)ethyl)carbama te |
| REF 104 | 4-[[3-(2,3-difluoro-4-methoxyphenyl)imidazo[1,2-a]pyrazin-8-yl]amino]-2-methyl-N-(4-piperidylmethyl)ben zamide | 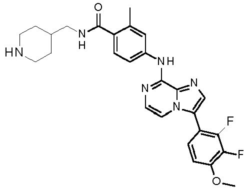 | 507.2 | Intermediate 28 and tetrahydropyridtert-butyl 4-(aminomethyl)piperi dine-1 -carboxylate |
| REF 105 | 4-((3 -(2,3-difluoro-4-methoxyphenyl)imid azo[1,2-a]pyrazin-8-yl)amino)-2-methyl-N-(2-(piperazin-1-yl)ethyl)benzamide hydrochloride | 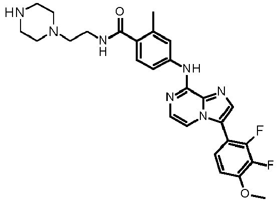 | 522.2 | Intermediate 28 and 2-(piperazin-1-yl)ethanamine |
| REF 106 | 4-[[3-(2,3-difluoro-4-methoxyphenyl)imidazo[1,2-a]pyrazin-8-yl] amino] -2-methyl-N-(pyrrolidin-3-ylmethyl)benzamide | 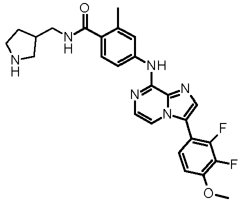 | 493.2 | Intermediate 28 and tert-butyl 3-(aminomethyl)pyrroli dine-1-carboxylate |
| REF 107 | 2-chloro-4-[[3-(2,3-difluoro-4-methoxyphenyl)imidazo[1,2-a]pyrazin-8-yl]amino]-N-(4-piperidylmethyl)ben zamide | 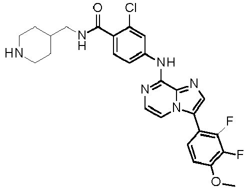 | 527.1 | Intermediate 20 and tert-butyl 4-(aminomethyl)piperid ine-1-carboxylate |
| REF 108 | 2-chloro-4-[[3-(2,3-difluoro-4-methoxyphenyl)imidazo[1,2-a]pyrazin-8-yl]amino]-N-(2-piperazin-1-ylethyl)benzamide | 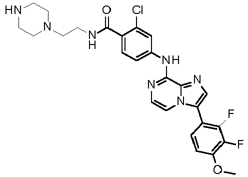 | 542.1 | Intermediate 20 and tert-butyl 4-(2-aminoethyl)piperazin e-1-carboxylate |
| REF 109 | 2-chloro-4-[[3-(2,3-difluoro-4-methoxyphenyl)imidazo[1,2-a]pyrazin-8-yl]amino]-N-(pyrrolidin-3-ylmethyl)benzamide | 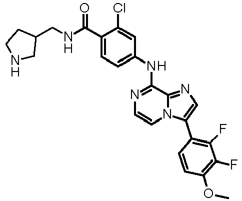 | 513.1 | Intermediate 20 and tert-butyl 3-(aminomethyl)pyrroli dine-1-carboxylate |
| REF 110 | (4-(2-aminoethyl)piperidin -1-yl)(2-chloro-4-((3-(2,3-difluoro-4-methoxyphenyl)imid azo[1,2-a]pyrazin-8-yl)amino)phenyl)met hanone | 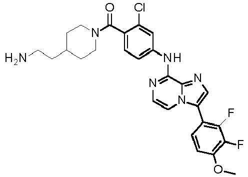 | 541.1 | Intermediate 20 and tert-butyl (2-(piperidin-4-yl)ethyl)carbamate |
| REF 111 | (4-(aminomethyl)piperi din-1-yl)(2-chloro-4-((3 -(2,3-difluoro-4-methoxyphenyl)imid azo[1,2-a]pyrazin-8-yl)amino)phenyl)met hanone | 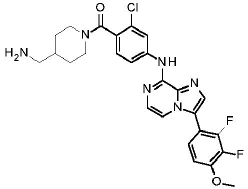 | 527.1 | Intermediate 20 and piperidin-4-ylmethanamine |
| REF 112 | 4-((3 -(2,3-difluoro-4-methoxyphenyl)imid azo[1,2-a]pyrazin-8-yl)amino)-N,2-dimethyl-N-(2-(piperazin-1-yl)ethyl)benzamide | 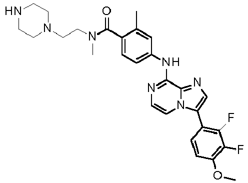 | 536.2 | Intermediate 28 and tert-butyl 4-(2-(methylamino)ethyl)p iperazine-1-carboxylate |
| REF 113 | (4-((3-(2,3-difluoro-4-methoxyphenyl)imid azo[1,2-a]pyrazin-8-yl)amino)-2-methylphenyl)(4-(2-(dimethylamino)ethy l)piperazin-1-yl)methanone | 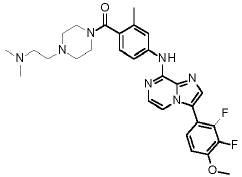 | 550.2 | Intermediate 28 and N,N-dimethyl-2-(piperazin-1-yl)ethanamine |
| REF 114 | (4-(aminomethyl)piperi din-1-yl)(4-((3-(2,3-difluoro-4-methoxyphenyl)imid azo[1,2-a]pyrazin-8-yl)amino)-2-methylphenyl)metha none | 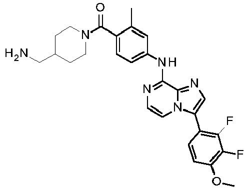 | 507.2 | Intermediate 28 and piperidin-4-ylmethanamine |
| REF 115 | N-(2-(2-aminoethoxy)ethyl)-2-chloro-4-((3-(2,3-difluoro-4-methoxyphenyl)imid azo[1,2-a]pyrazin-8-yl)amino)benzamide | 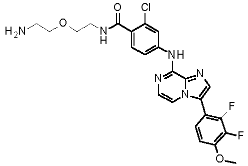 | 517.1 | Intermediate 20 and piperidin-4-ylmethanamine |
| REF 116 | (4-((3-(2,3-difluoro-4-methoxyphenyl)imid azo[1,2-a]pyrazin-8-yl)amino)-2-methylphenyl)(4-((dimethylamino)me thyl)piperidin-1-yl)methanone | 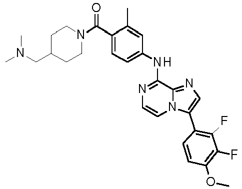 | 535.2 | Intermediate 28 and N,N-Dimethyl-1-(piperidin-4-yl)methanamine dihydrochloride |
| REF 117 | 4-((3-(2,3-difluoro-4-methoxyphenyl)imid azo[1,2-a]pyrazin-8-yl)amino)-2-methyl-N-((1-methylpiperidin-4-yl)methyl)benzamid e | 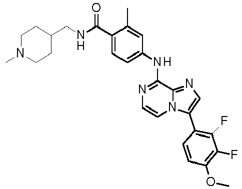 | 521.2 | Intermediate 28 and (1-methylpiperidin-4-yl)methanamine |
| REF 118 | aziridin-1-yl(2-chloro-4-((3-(2,3-difluoro-4-methoxyphenyl)imid azo[1,2-a]pyrazin-8-yl)amino)phenyl)met hanone | 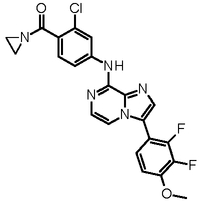 | 456.0 | Intermediate 20 and 2-chloroethanamine hydrochloride |
| REF 119 | 4-((3 -(2,3-difluoro-4-methoxyphenyl)imid azo[1,2-a]pyrazin-8-yl)amino)-N,2-dimethyl-N-(2-(piperidin-4-yl)ethyl)benzamide | 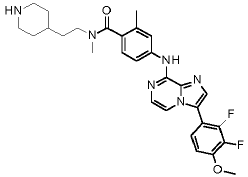 | 535.2 | Intermediate 28 and tert-butyl 4-[2-(methyl amino)ethyl]p iperidine-1-carboxylate |
| REF 120 | N-(3-aminopropyl)-4-((3-(2,3-difluoro-4-methoxyphenyl)imid azo[1,2-a]pyrazin-8-yl)amino)-2-methyl benzamide | 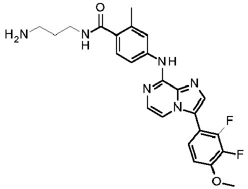 | 467.1 | Intermediate 28 and tert-butyl (3-aminopropyl)carbam ate |
| REF 121 | 4-((3-(2,3-difluoro-4-methoxyphenyl)imid azo[1,2-a]pyrazin-8-yl)amino)-2-methyl-N-(3-(3-oxopiperazin-1-yl)propyl)benzamide | 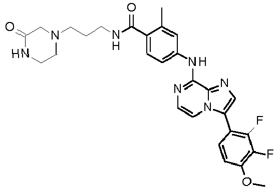 | 550.2 | Intermediate 28 and Intermediate 57-P1 |
| REF 122 | 4-((3-(2,3-difluoro-4-methoxyphenyl)imid azo[1,2-a]pyrazin-8-yl)amino)-2-methyl-N-(3-(4-methyl-3-oxopiperazin-1-yl)propyl)benzamide | 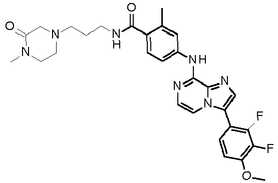 | 564.2 | Intermediate 28 and Intermediate 58 |
| REF 123 | 4-((3-(2,3-difluoro-4-methoxyphenyl)imid azo[1,2-a]pyrazin-8-yl)amino)-2-methyl-N-(3-(piperazin-1-yl)propyl)benzamide | 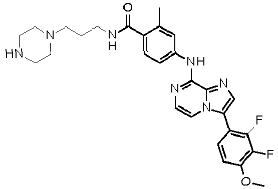 | 536.2 | Intermediate 28 and tert-butyl 4-(3-aminopropyl)piperazi ne-1-carboxylate |
| REF 124 | 4-((3-(2,3-difluoro-4-methoxyphenyl)imid azo[1,2-a]pyrazin-8-yl)amino)-2-methyl-N-(3-(piperazin-1-ylsulfonyl)propyl)be nzamide | 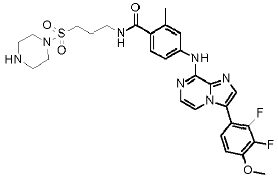 | 600.2 | Intermediate 28 and tert-butyl 4-((3-aminopropyl)sulfonyl )piperazine-1-carboxylate |
| REF 125 | 4-((3-(2,3-difluoro-4-methoxyphenyl)imid azo[1,2-a]pyrazin-8-yl)amino)-2-methyl-N-(3-(2-oxopiperazin-1-yl)propyl)benzamide | 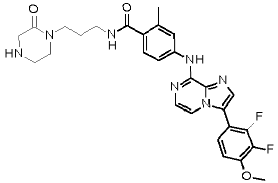 | 550.2 | Intermediate 28 and Intermediate 59 |
| REF 126 | 4-((3-(3-fluoro-4-methoxyphenyl)imid azo[1,2-a]pyrazin-8-yl)amino)-2-methyl-N-((tetrahydrofuran-3-yl)methyl)benzamid e | 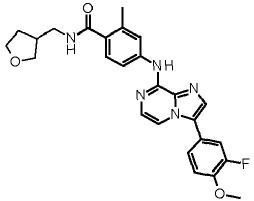 | 476.2 | Intermediate 6 and (tetrahydrofuran-3-yl)methanamine |
| REF 127 | 4-((3-(3-fluoro-4-methoxyphenyl)imid azo[1,2-a]pyrazin-8-yl)amino)-N,2-dimethyl-N-((tetrahydrofuran-3- yl)methyl)benzamid e | 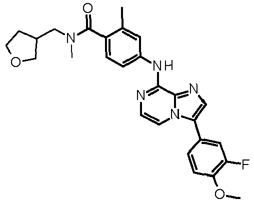 | 490.2 | Intermediate 6 and N-methyl-1-(tetrahydrofuran-3-yl)methanamine |
| REF 128 | 4-((3-(3-fluoro-4-methoxyphenyl)imid azo[1,2-a]pyrazin-8-yl)amino)-2-methyl-N-((tetrahydro-2H-pyran-3-yl)methyl)benzamid e | 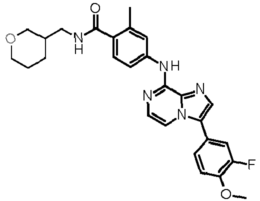 | 490.2 | Intermediate 6 and (tetrahydrofuran-3-yl)methanamine |
| REF 129 | 4-((3-(3-fluoro-4-methoxyphenyl)imid azo[1,2-a]pyrazin-8-yl)amino)-N,2-dimethyl-N-(pyridin-4-ylmethyl)benzamide | 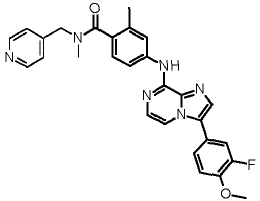 | 497.2 | Intermediate 6 and N-methyl-1 -(pyridin-4-yl)methanamine |
| REF 130 | 2-(4-(8-((3-chloro-4-(4-(2-(dimethylamino)ethy l)piperazine-1-carbonyl)phenyl)ami no)imidazo[1,2-a]pyrazin-3-yl)-2,3-difluorophenoxy)ace tonitrile | 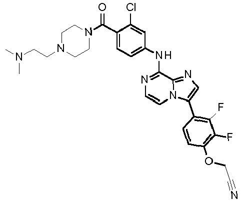 | 595.2 | Intermediate 29 and N,N-dimethyl-2-(piperazin-1-yl)ethanamine |
| REF 131 | 2-chloro-4-((3-(4-(cyanomethoxy)-2,3-difluorophenyl)imid azo[1,2-a]pyrazin-8-yl)amino)-N-methyl-N-(2-(piperazin-1-yl)ethyl)benzamide | 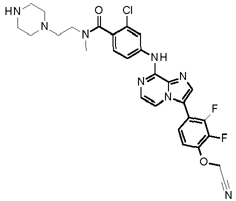 | 581.2 | Intermediate 29 and tert-butyl 4-(2-(methylamino)ethyl)p iperazine-1-carboxylate |
| REF 132 | 2-chloro-4-((3-(4-(cyanomethoxy)-2,3-difluorophenyl)imid azo[1,2-a]pyrazin-8-yl)amino)-N-(2-(pyridin-4-yl)ethyl)benzamide | 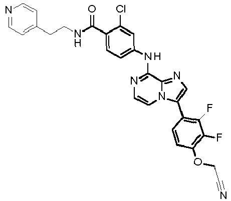 | 560.1 | Intermediate 29 and 2-(pyridin-4-yl)ethanamine |
| REF 133 | 2-chloro-4-[[3-[4-(cyanomethoxy)-2,3-difluorophenyl]imidazo[1,2-a]pyrazin-8-yl]amino]-N-[3-(4-pyridyl)propyl]benza mide;2,2,2-trifluoroacetic acid | 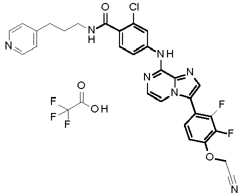 | 574.1 | Intermediate 29 and 3-(pyridin-4-yl)propan-1-amine |
| REF 134 | 2-[4-[8-[3-chloro-4-[4-(1H-imidazol-5-yl)piperidine-1-carbonyl]anilino]imi dazo[1,2-a]pyrazin-3-yl]-2,3-difluoro-phenoxy]acetonitrile ;2,2,2-trifluoroacetic acid | 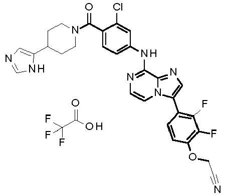 | 589.1 | Intermediate 29 and 4-(1H-imidazol-5-yl)piperidine |
| REF 135 | 2-chloro-4-((3-(4-(cyanomethoxy)-2,3-difluorophenyl)imid azo[1,2-a]pyrazin-8-yl)amino)-N-(3-(piperazin-1-yl)propyl)benzamide | 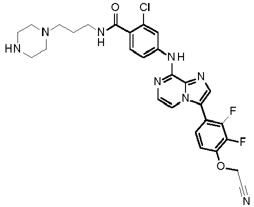 | 581.1 | Intermediate 29 and tert-butyl 4-(3-aminopropyl)piperazi ne-1-carboxylate |
| REF 136 | N-(2-(2-(1H-imidazol-1-yl)ethoxy)ethyl)-2-chloro-4-((3-(4-(cyanomethoxy)-2,3-difluorophenyl)imid azo[1,2-a]pyrazin-8-yl)amino)benzamide | 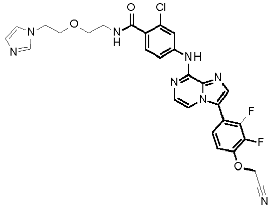 | 593.1 | Intermediate 29 and 61 |
| REF 137 | 2-(4-(8-((4-(4-(2-(dimethylamino)ethy 1)piperazine-1-carbonyl)-3-ethylphenyl)amino)i midazo[1,2-a]pyrazin-3-yl)-2,3-difluorophenoxy)ace tonitrile | 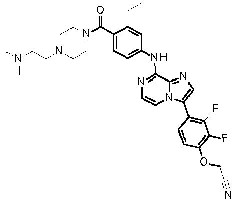 | 589.2 | Intermediate 32 and N,N-dimethyl-2-(piperazin-1-yl)ethanamine |
| REF 138 | 2-(4-(8-((4-(4-(aminomethyl)piperi dine-1-carbonyl)-3- chlorophenyl)amino) imidazo[1,2-a]pyrazin-3-yl)-2,3-difluorophenoxy)ace tonitrile | 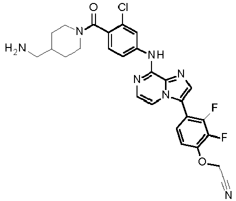 | 552.1 | Intermediate 29 and tert-butyl (piperidin-4-ylmethyl)carbamate |
| REF 139 | 2-[4-[8-[4-[4-(aminomethyl)piperi dine-1-carbonyl]-3-methyl-anilino]imidazo[1,2-a]pyrazin-3-yl]-2,3-difluoro-phenoxy] acetonitrile ;2,2,2-trifluoroacetic acid | 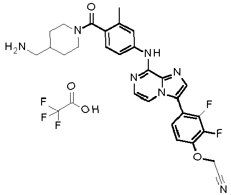 | 532.2 | Intermediate 31 and tert-butyl (piperidin-4-ylmethyl)carbamate |
| REF 140 | 2-chloro-4-[[3-[4-(cyanomethoxy)-2,3-difluorophenyl]imidazo[1,2-a]pyrazin-8-yl]amino]-N-methyl-N-[2-(4-piperidyl)ethyl]benzamide;2,2,2-trifluoroacetic acid | 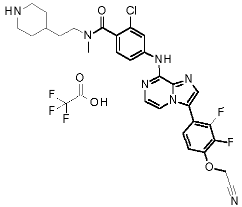 | 580.1 | Intermediate 29 and tert-butyl 4-(2-(methylamino)ethyl)p iperidine-1-carboxylate |
| REF 141 | 2-[4-[8-[3-chloro-4-[4-(1H-tetrazol-5-yl)piperidine-1-carbonyl]anilino]imi dazo[1,2-a]pyrazin-3-yl]-2,3-difluoro-phenoxy] acetonitrile ;2,2,2-trifluoroacetic acid | 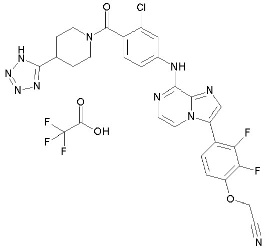 | 591.1 | Intermediate 29 and 4-(2H-tetrazol-5-yl)piperidine hydrochloride |
| REF 142 | 2-[4-[8-[3-chloro-4-[4-[2-(dimethylamino)acet yl]piperazine-1-carbonyl]anilino]imi dazo[1,2-a]pyrazin-3-yl]-2,3-difluoro-phenoxy]acetonitrile ;2,2,2-trifluoroacetic acid | 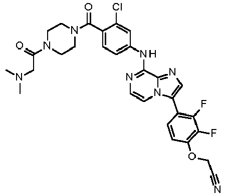 | 609.2 | Intermediate 29 and intermediate 56 |
| REF 143 | 2-chloro-4-[[3-[4-(cyanomethoxy)-2,3-difluorophenyl]imidazo[1,2-a]pyrazin-8-yl]amino]-N-(4-piperidylmethyl)ben zamide | 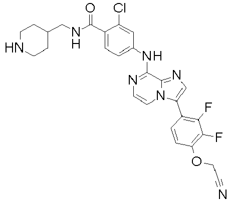 | 552.1 | Intermediate 29 and 4-piperidyl methanamine |
| REF 144 | 2-chloro-4-[[3-[3-chloro-4-(cyanomethoxy)-2-fluorophenyl]imidazo[1,2-a]pyrazin-8-yl]amino]-N-[2-(4-pyridyl)ethyl]benza mide | 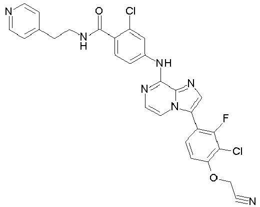 | 576.2 | Intermediate 35 and 2-(pyridin-4-yl)ethanamine |
| REF 145 | N-[2-(2-aminoethoxy)ethyl]-4-[[3-[4-(cyanomethoxy)-2,3-difluorophenyl]imidazo[1,2-a]pyrazin-8-yl]amino]-2-ethyl-benzamide;2,2,2-trifluoroacetic acid | 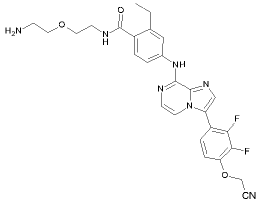 | 536.2 | Intermediate 32 and N-BOC-2-(2-amino-ethoxy)-ethyl amine |
| REF 146 | 4-[[3-[4-(cyanomethoxy)-2,3-difluorophenyl]imidazo[1,2-a]pyrazin-8-yl]amino]-N-[2-[2-(dimethylamino)etho xy]ethyl]-2-ethyl-benzamide | 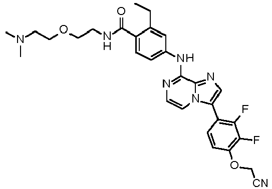 | 564.2 | Intermediate 32 and 2-[2-(dimethylamino) ethoxy] ethanamine |
| REF 147 | 4-[[3-[2-chloro-4-(cyanomethoxy)-3- fluorophenyl]imidazo[1,2-a]pyrazin-8-yl]amino]-N-[2-[2-(dimethylamino)ethoxy]ethyl]-2-ethyl-benzamide | 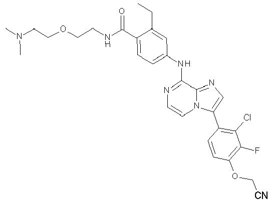 | 580.2 | Intermediate 40 and 2-[2-(dimethylamino) ethoxy] ethanamine |
| REF 148 | 2-[5-chloro-4-[8-[3-chloro-4-[4-[3-(hydroxymethyl)pipe razine-1-carbonyl]piperidine-1-carbonyl] anilino] imi dazo[1,2-a]pyrazin-3-yl]-2-fluoro-phenoxy] acetonitrile ; 2,2,2-trifluoroacetic | 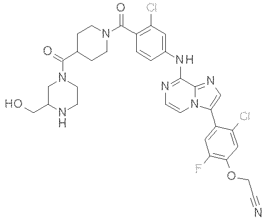 | 681.1 | Intermediate 37 and 81 |
| REF 149 | 2-[5-chloro-2-fluoro-4-[8-[4-[4-[3-(hydroxymethyl)pipe razine-1-carbonyl]piperidine-1-carbonyl]-3-methyl-anilino]imidazo[1,2-a]pyrazin-3-yl]phenoxy]acetonitr ile; 2,2,2-trifluoroacetic acid | 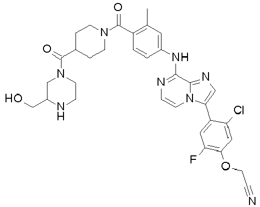 | 661.3 | Intermediate 39 and 81 |
| REF 150 | (1-(4-((3-(4-(difluoromethoxy)ph enyl)imidazo[1,2-a]pyrazin-8-yl)amino)-2-methylbenzoyl)piper idin-4-yl)((3R,4R,5R)-3,4-dihydroxy-5-(hydroxymethyl)pipe ridin-1-yl)methanone | 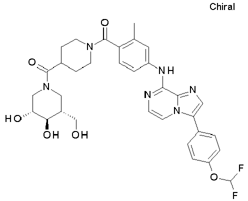 | 651.5 | Intermediate 7 and (3R,4R,5R)-5-(hydroxymethyl)pipe ridine-3,4-diol hydrochloride |
| REF 151 | (4-(aminomethyl)piperi din-1-yl)(4-((3-(3-fluoro-4-methoxyphenyl)imid azo[1,2-a]pyrazin-8-yl)amino)-2-methylphenyl)metha none hydrochloride | 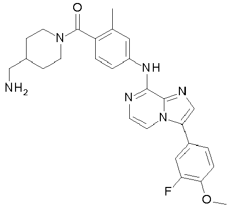 | 489.2 | Intermediate 6 and tert-butyl (piperidin-4-ylmethyl)carbamate |
| REF 152 | (4-(2-(dimethylamino)ethy l)piperazin-1-yl)(4-((3-(3-fluoro-4-methoxyphenyl)imid azo[1,2-a]pyrazin-8-yl)amino)-2-methylphenyl)metha none | 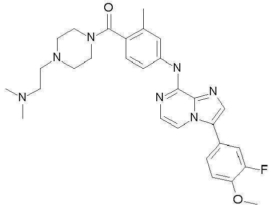 | 532.6 | Intermediate 6 and N,N-dimethyl-2-(piperazin-1-yl)ethanamine |
| REF 153 | (4-((3-(4-(difluoromethoxy)ph enyl)imidazo[1,2-a]pyrazin-8-yl)amino)-2-methylphenyl)(4-(4-methylpiperazin-1- yl)piperidin-1-yl)methanone | 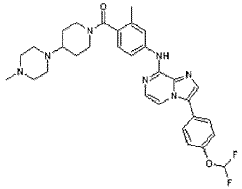 | 576.2 | Intermediate 7 and 1-methyl-4-(piperidin-4-yl)piperazine |
| REF 154 | 2-amino-1-(4-(4-((3-(4-(difluoromethoxy)ph enyl)imidazo[1,2-a]pyrazin-8-yl)amino)-2-methylbenzoyl)piper azin-1-yl)ethanone hydrochloride | | 536.2 | Intermediate 7 and tert-butyl (2-oxo-2-(piperazin-1-yl)ethyl)carbamate |
| REF 155 | 1-(4-(4-((3-(4-(difluoromethoxy)ph enyl)imidazo[1,2-a]pyrazin-8-yl)amino)-2-methylbenzoyl)piper azin-1-yl)-2-(dimethylamino)etha none | 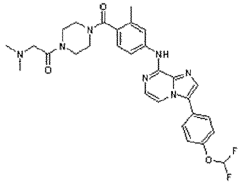 | 564.1 | Intermediate 7 and 2-(dimethylamino)-1-(piperazin-1-yl)ethanone dihydrochloride |
| REF 156 | (4-(2-(aminomethyl)morp holine-4-carbonyl)piperidin-1-yl)(4-((3-(3-fluoro-4-methoxyphenyl)imid azo[1,2-a]pyrazin-8-yl)amino)-2-methylphenyl)metha none hydrochloride | 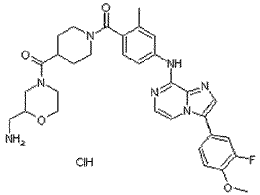 | 602.3 | Intermediate 6 and tert-butyl (morpholin-2-ylmethyl)carbamate |
| REF 157 | 1-(4-((3-(3-fluoro-4-methoxyphenyl)imid azo[1,2-a]pyrazin-8-yl)amino)-2-methylbenzoyl)-N-(2-(methylamino)ethyl) piperidine-4-carboxamide hydrochloride | 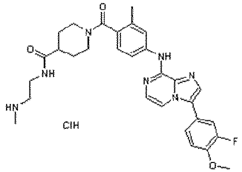 | 560.3 | Intermediate 6 and tert-butyl (2-aminoethyl)(methyl)c arbamate |
| REF 158 | N-(2-((2-aminoethyl)(methyl) amino)ethyl)-4-((3-(3-fluoro-4-methoxyphenyl)imid azo[1,2-a]pyrazin-8-yl)amino)-2-methylbenzamide | 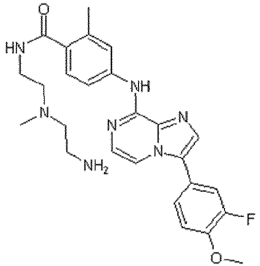 | 492.3 | Intermediate 6 and N1-(2-aminoethyl)-N1-methylethane-1,2-diamine |
| REF 159 | (4-((3-(3-fluoro-4-methoxyphenyl)imid azo[1,2-a]pyrazin-8-yl)amino)-2-methylphenyl)(4-(1-methyl-1H-imidazol-2-yl)piperazin-1-yl)methanone | 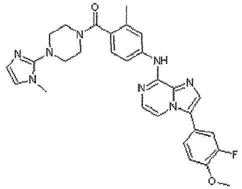 | 541.3 | Intermediate 6 and 1-(1-methyl-1H-imidazol-2-yl)piperazine |
| REF 160 | 1-(2-chloro-4-((3-(3-fluoro-4-methoxyphenyl)imid azo[1,2-a]pyrazin-8-yl)amino)benzoyl)-N-(2-(methyl amino)ethyl) piperidine-4-carboxamide hydrochloride | 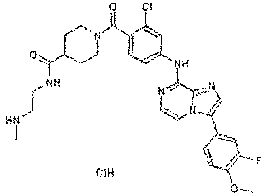 | 580.2 | Intermediate 2 and tert-butyl (2-aminoethyl)(methyl)c arbamate |
| REF 161 | (4-((3-(3-fluoro-4-methoxyphenyl)imid azo[1,2-a]pyrazin-8-yl)amino)-2-methylphenyl)(2-(hydroxymethyl)pipe razin-1-yl)methanone | 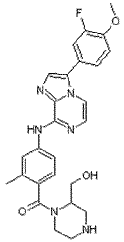 | 489.4 | Intermediate 6 and tert-butyl 2-(hydroxymethyl)pipe razine-1-carboxylate |
| REF 162 | (4-(2-(aminomethyl)morp holine-4-carbonyl)piperidin-1-yl)(4-((3-(4-(difluoromethoxy)ph enyl)imidazo[1,2-a]pyrazin-8-yl)amino)-2-methylphenyl)metha none hydrochloride | | 678.7 | Reference Example 1 and tert-butyl (2-aminoethyl)(methyl)c arbamate |
| REF 163 | (4-(6-cyclopropyl-2,6-diazaspiro[3.3]hepta ne-2-carbonyl)piperidin-1-yl)(4-((3-(4-(difluoromethoxy)ph enyl)imidazo [1,2-a]pyrazin-8-yl)amino)-2-methylphenyl)metha none | 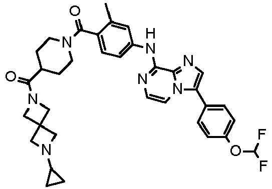 | 642.5 | Reference Example 1 and 2-cyclopropyl-2,6-diazaspiro[3.3]heptan e |
| REF 164 | (4-((3-(4-(difluoromethoxy)ph enyl)imidazo [1,2-a]pyrazin-8-yl)amino)-2-methylphenyl)(4-(piperazin-1-yl)piperidin-1-yl)methanone hydrochloride | 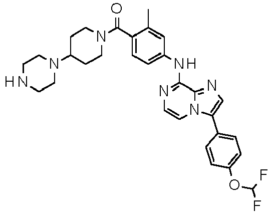 | 562.3 | Intermediate 7 and tert-butyl 4-(piperidin-4-yl)piperazine-1-carboxylate |
| REF 165 | N-[3-(dimethylamino)pro pyl]-1-[4-[[3-(3-fluoro-4-methoxyphenyl)imid azo[1,2-a]pyrazin-8-yl]amino]-2-methylbenzoyl]piper idine-4-carboxamide | 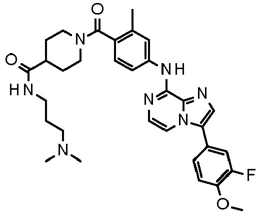 | 588.3 | Reference Example 2 and N1,N1-dimethylpropane-1,3-diamine |
| REF 166 | N-[2-(dimethylamino)ethy 1]-1-[4-[[3-(3-fluoro-4-methoxyphenyl)imid azo[1,2-a]pyrazin-8-yl]amino]-2-methylbenzoyl] piper idine-4-carboxamide | 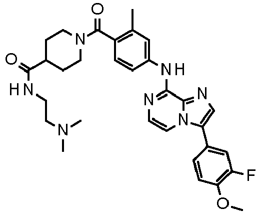 | 574.7 | Reference Example 2 and N1,N1-dimethylethane-1,2-diamine |
| REF 167 | 4-[1-[4-[[3-(3-fluoro-4-methoxyphenyl)imid azo[1,2-a]pyrazin-8-yl]amino]-2-methylbenzoyl]piper idine-4-carbonyl]piperazine-2-carboxylic acid;hydrochloride | 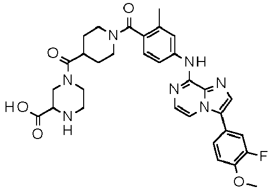 | 616.3 | Reference Example 2 and 1-tert-butyl 2-methyl piperazine-1,2-dicarboxylate, the intermediate ester was hydrolyzed with LiOH in THF/MeOH/H2O as described previously |
| REF 168 | 1-(4-((3-(3-fluoro-4-methoxyphenyl)imid azo[1,2-a]pyrazin-8-yl)amino)-2-methylbenzoyl)-N- (3-(methylamino)propy l)piperidine-4-carboxamide hydrochloride | 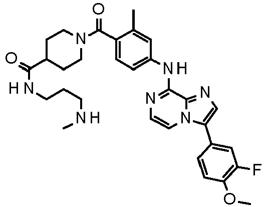 | 574.4 | Reference Example 2 and tert-butyl (3-aminopropyl)(methyl )carbamate |
| REF 169 | (R)-(1-(4-((3-(3-fluoro-4-methoxyphenyl)imid azo[1,2-a]pyrazin-8-yl)amino)-2-methylbenzoyl)piper idin-4-yl)(3-(methylamino)pyrrol idin-1-yl)methanone hydrochloride | 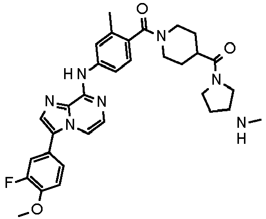 | 586.4 | Reference Example 2 and (R)-tert-butyl methyl(pyrrolidin-3-yl)carbamate |
| REF 170 | 1-(4-((3-(3-fluoro-4-methoxyphenyl)imid azo[1,2-a]pyrazin-8-yl)amino)-2-methylbenzoyl)-N- (4-(methylamino)butyl) piperidine-4-carboxamide hydrochloride | 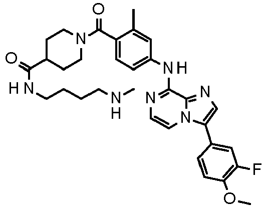 | 588.5 | Reference Example 2 and tert-butyl (4-aminobutyl)(methyl)c arbamate |
| REF 171 | N-(3-aminopropyl)-1-(4-((3-(3-fluoro-4-methoxyphenyl)imid azo[1,2-a]pyrazin-8-yl)amino)-2-methylbenzoyl)piper idine-4-carboxamide hydrochloride | 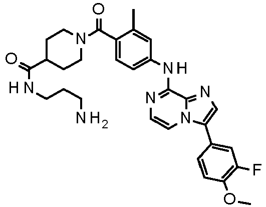 | 560.4 | Reference Example 2 and tert-butyl (3-aminopropyl)carbam ate |
| REF 172 | (1-(4-((3-(3-fluoro-4-methoxyphenyl)imid azo[1,2-a]pyrazin-8-yl)amino)-2-methylbenzoyl)piper idin-4-yl)(3-(hydroxymethyl)pipe razin-1-yl)methanone 2,2,2-trifluoroacetate | 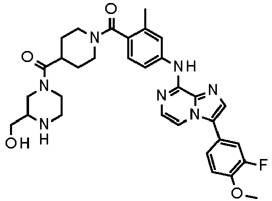 | 602.4 | Reference Example 2 and tert-butyl 2-(hydroxymethyl)pipe razine-1-carboxylate |
| REF 173 | 1-(4-((3-(3-fluoro-4-methoxyphenyl)imid azo[1,2-a]pyrazin-8-yl)amino)-2-methylbenzoyl)-N- ((3-hydroxypyrrolidin-3-yl)methyl)piperidine -4-carboxamide 2,2,2-trifluoroacetate | 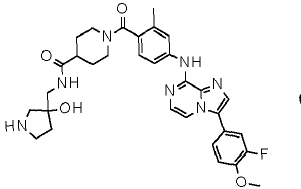 | 602.4 | Reference Example 2 and tert-butyl 3-(aminomethyl)-3-hydroxypyrrolidine-1-carboxylate |
| REF 174 | N-(3-amino-2-hydroxypropyl)-1-(4-((3-(3-fluoro-4-methoxyphenyl)imid azo[1,2-a]pyrazin-8-yl)amino)-2-methylbenzoyl)piper idine-4-carboxamide 2,2,2-trifluoroacetate | 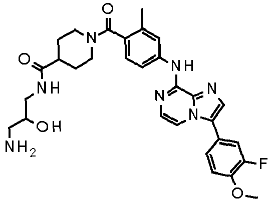 | 576.3 | Reference Example 2 and tert-butyl (3-amino-2-hydroxypropyl)carba mate |
| REF 175 | 1-(4-((3-(3-fluoro-4-methoxyphenyl)imid azo[1,2-a]pyrazin-8-yl)amino)-2-methylbenzoyl)-N-((3R,4R)-4-hydroxypyrrolidin-3-yl)piperidine-4-carboxamide 2,2,2-trifluoroacetate | 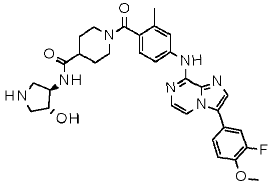 | 588.3 | Reference Example 2 and (3R,4R)-tert-butyl 3-amino-4-hydroxypyrrolidine-1-carboxylate |
| REF 176 | N-(azetidin-3-yl)-1-(4-((3-(3-fluoro-4-methoxyphenyl)imid azo[1,2-a]pyrazin-8-yl)amino)-2-methylbenzoyl)piperidine-4-carboxamide 2,2,2-trifluoroacetate | 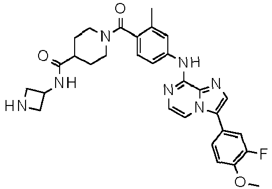 | 558.3 | Reference Example 2 and tert-butyl 3-aminoazetidine-1-carboxylate |
| REF 177 | 1-(4-((3-(3-fluoro-4-methoxyphenyl)imid azo[1,2-a]pyrazin-8-yl)amino)-2-methylbenzoyl)-N- ((3-hydroxyazetidin-3-yl)methyl)piperidine -4-carboxamide 2,2,2-trifluoroacetate | 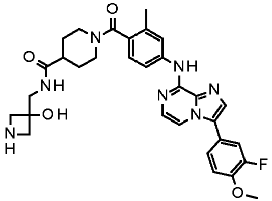 | 588.3 | Reference Example 2 and tert-butyl 3-(aminomethyl)-3- hydroxyazetidine-1- carboxylate |
| REF 178 | (R)-1-(4-((3-(3-fluoro-4-methoxyphenyl)imid azo[1,2-a]pyrazin-8-yl)amino)-2-methylbenzoyl)-N- (pyrrolidin-3-yl)piperidine-4-carboxamide 2,2,2-trifluoroacetate | 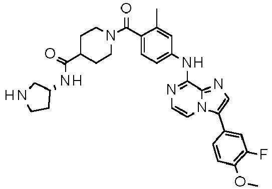 | 572.3 | Reference Example 2 and (R)-tert-butyl 3-aminopyrrolidine-1-carboxylate |
| REF 179 | N-(azetidin-3-ylmethyl)-1-(4-((3-(3-fluoro-4-methoxyphenyl)imid azo[1,2-a]pyrazin-8-yl)amino)-2-methylbenzoyl)piper idine-4-carboxamide 2,2,2-trifluoroacetate | 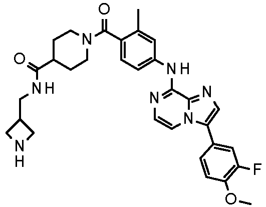 | 572.4 | Reference Example 2 and tert-butyl 3-(aminomethyl)azetidi ne-1-carboxylate |
| REF 180 | N-(2-(azetidin-1-yl)ethyl)-1-(4-((3-(3-fluoro-4-methoxyphenyl)imid azo[1,2-a]pyrazin-8-yl)amino)-2-methylbenzoyl)piper idine-4-carboxamide | 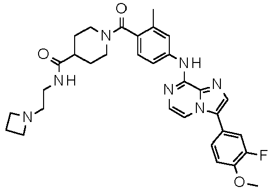 | 586.3 | Reference Example 2 and 2-(azetidin-1-yl)ethanamine |
| REF 181 | N-(3-(azetidin-1-yl)propyl)-1-(4-((3-(3-fluoro-4-methoxyphenyl)imid azo[1,2-a]pyrazin-8-yl)amino)-2-methylbenzoyl)piper idine-4-carboxamide | 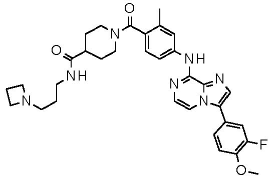 | 600.3 | Reference Example 2 and 3-(azetidin-1-yl)propan-1-amine |
| REF 182 | (R)-1-(4-((3-(2,3-difl uoro-4-methoxyphenyl)imid azo[1,2-a]pyrazin-8-yl)amino)-2-methylbenzoyl)-N- (pyrrolidin-3-yl)piperidine-4-carboxamide hydrochloride | 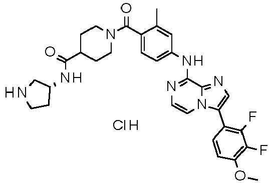 | 590.3 | Intermediate 65 and tert-butyl (R)-3-aminopyrrolidine-1-carboxylate |
| REF 183 | (R)-1-(2-chloro-4-((3-(3-fluoro-4-methoxyphenyl)imid azo[1,2-a]pyrazin-8-yl)amino)benzoyl)-N-(pyrrolidin-3-yl)piperidine-4-carboxamide hydrochloride | 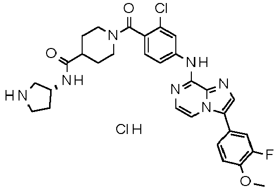 | 593.3 | Intermediate 66 and tert-butyl (R)-3-aminopyrro lidine-1-carboxylate |
| REF 184 | 1-(4-(2-chloro-4-((3-(3-fluoro-4-methoxyphenyl)imid azo[1,2-a]pyrazin-8-yl)amino)benzoyl)pi perazin-1-yl)-2-(methylamino)ethan-1-one hydrochloride | 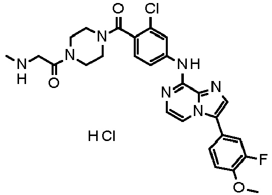 | 553.3 | Intermediate 2 and tert-butyl methyl(2-oxo-2-(piperazin-1-yl)ethyl)carbamate |
| REF 185 | 2-(dimethylamino)-1-(4-(4-((3-(3-fluoro-4-methoxyphenyl)imid azo[1,2-a]pyrazin-8-yl)amino)-2-methylbenzoyl)piper azin-1-yl)ethanone | 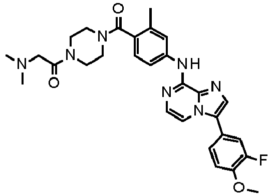 | 546.3 | Intermediate 6 and 2-(dimethylamino)-1-(piperazin-1-yl)ethanone dihydrochloride |
| REF 186 | N-(azetidin-3-ylmethyl)-1-(2-chloro-4-((3-(3-fluoro-4-methoxyphenyl)imid azo[1,2-a]pyrazin-8-yl)amino)benzoyl)pi peridine-4-carboxamide | 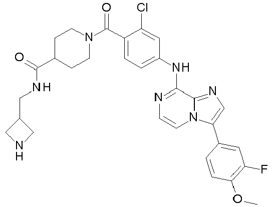 | 590.6 (M-H) | Intermediate 66 and tert-butyl 3-(aminomethyl)azetidi ne-1-carboxylate. |
| REF 187 | N-(azetidin-3-ylmethyl)-1-(4-((3-(3-fluoro-4-methoxyphenyl)imid azo[1,2-a]pyrazin-8-yl)amino)-2-methylbenzoyl)piper idine-4-carboxamide | 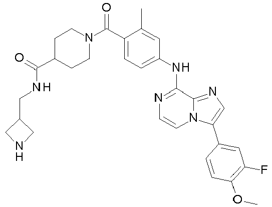 | 572.5 | Reference Example 2 and tert-butyl 3-(aminomethyl)azetidi ne-1-carboxylate. |
| REF 188 | (S)-1-(4-((3-(3-fluoro-4-methoxyphenyl)imid azo[1,2-a]pyrazin-8-yl)amino)-2-methylbenzoyl)-N- (pyrrolidin-3-yl)piperidine-4-carboxamide hydrochloride | 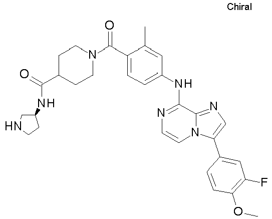 | 572.3 | Reference Example 2 and tert-butyl (S)-3-aminopyrrolidine-1-carboxylate |
| REF 189 | (4-((3-(4-(difluoromethoxy)ph enyl)imidazo[1,2-a]pyrazin-8-yl)amino)-2-methylphenyl)(4-(4,5-dihydro-1H-imidazol-2-yl)piperazin-1-yl)methanone | 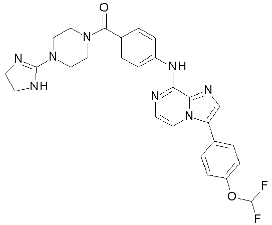 | 547.2 | Intermediate 7 and 1-(4,5-dihydro-1H-imidazol-2-yl)piperazine hydroiodide (CAS 295341-59-2) |
| REF 190 | N-(azetidin-3-ylmethyl)-1-(4-((3-(4-(difluoromethoxy)ph enyl)imidazo [1,2-a]pyrazin-8-yl)amino)-2-methylbenzoyl)piper idine-4-carboxamide | 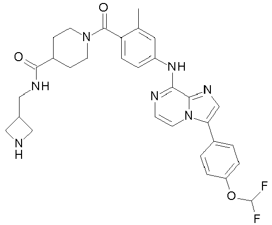 | 590.4 | Reference Example 1 and tert-butyl 3-(aminomethyl)azetidi ne-1-carboxylate |
| REF 191 | 4-[[3-[4-(difluoromethoxy)ph enyl]imidazo[1,2-a]pyrazin-8-yl]amino]-N,2-dimethyl-N-[2-(4-piperidyl)ethyl]benz amide;hydrochloride | 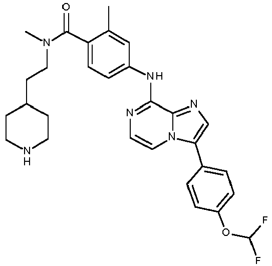 | 535.5 | Intermediate 7 and tert-butyl 4-(2-(methylamino)ethyl)p iperidine-1-carboxylate |
| REF 192 | N-((1-carbamimidoylpiperi din-4-yl)methyl)-4-((3-(3-chloro-4-methoxyphenyl)imid azo[1,2-a]pyrazin-8-yl)amino)-2-methyl benzamide | 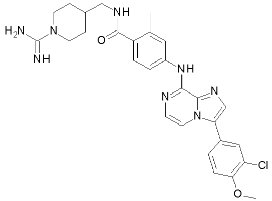 | 547.4 | Reference Example 2 and 4-(aminomethyl)piperid ine-1-carboximidamide |
| REF 193 | 4-(4-((3-(4-(difluoromethoxy)ph enyl)imidazo[1,2-a]pyrazin-8-yl)amino)-2-methylbenzoyl)piper azine-1-carboximidamide | 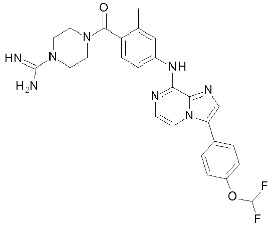 | 521.2 | Intermediate 7 and piperazine-1-carboximidamide |
| REF 194 | 4-((3-(4-(difluoromethoxy)ph enyl)imidazo[1,2-a]pyrazin-8-yl)amino)-N-(4-guanidinobutyl)-2-methylbenzamide | 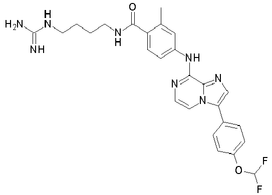 | 523.2 | Intermediate 7 and 1-(4-amino butyl) guanidin e sulfate |
| REF 195 | 1-(4-((3-(3-fluoro-4-methoxyphenyl)imid azo[1,2-a]pyrazin-8-yl)amino)-2-methylbenzoyl)-N- ((1-methylazetidin-3-yl)methyl)piperidine -4-carboxamide | 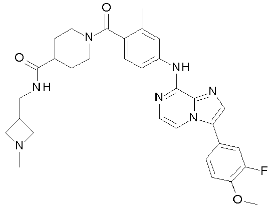 | 586.3 | Reference Example 2 and (1-methylazetidin-3-yl)methanamine |
| REF 196 | (2-chloro-4-((3-(3-fluoro-4-methoxyphenyl)imid azo[1,2-a]pyrazin-8-yl)amino)phenyl)(4-(2-(methylamino)ethyl) piperazin-1-yl)methanone | 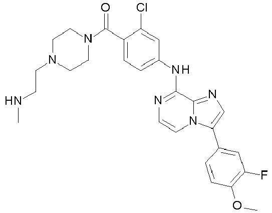 | 538.2 | Intermediate 2 and (9H-fluoren-9-yl)methyl methyl(2-(piperazin-1-yl)ethyl)carbamate hydrochloride. In situ deprotection with piperidine. |
| REF 133 | 2-chloro-4-[[3-[4-(cyanomethoxy)- 2,3-difluorophenyl]imidazo[1,2-a]pyrazin-8-yl]amino]-N-[3-(4-pyridyl)propyl]benza mide;2,2,2-trifluoroacetic acid | 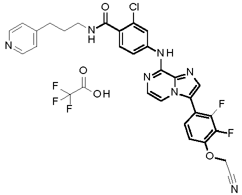 | 574.1 | Intermediate 29 and 3-(pyridin-4-yl)propan-1-amine |
| REF 197 | 2-[4-[8-[3-chloro-4-[4-(4-pyridyl)piperidine-1-carbonyl]anilino]imi dazo[1,2-a]pyrazin-3-yl]-2,3-difluoro-phenoxy] acetonitrile | 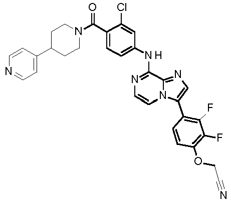 | 600.1 | Intermediate 29 and 4-(piperidin-4-yl)pyridine |
| REF 198 | 2-chloro-4-[[3-[2-chloro-4-(cyanomethoxy)-3-fluorophenyl]imidazo[1,2-a]pyrazin-8-yl] amino]-N-[2-(4-pyridyl)ethyl]benza mide | 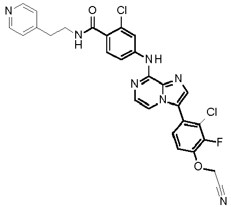 | 576.1 | Intermediate 24 and 2-(pyridin-4-yl)ethan-1-amine |
| REF 199 | 2-[4-[8-[3-chloro-4-(4-pyrimidin-2-ylpiperazine-1-carbonyl)anilino]imi dazo[1,2-a]pyrazin-3-yl]-2,3-difluoro-phenoxy]acetonitrile | 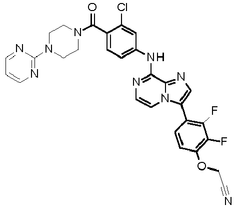 | 602.1 | Intermediate 29 and 2-(piperazin-1-yl)pyrimidine hydrochloride |
| REF 200 | 2-[4-[8-[3-chloro-4-[4-(4-methyl-1,2,4-triazol-3-yl)piperidine-1-carbonyl]anilino] imi dazo[1,2-a]pyrazin-3-yl]-2,3-difluoro-phenoxy] acetonitrile ;2,2,2-trifluoroacetic acid | 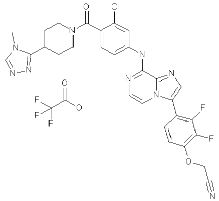 | 604.1 | Intermediate 29 and 4-(4-methyl-4H-1,2,4-triazol-3-yl)piperidine hydrochloride |
| REF 201 | 2-chloro-4-[[3-[4-(cyanomethoxy)-2,3-difluorophenyl]imidazo[1,2-a]pyrazin-8-yl]amino]-N-[(2-oxo-1H-pyridin-4-yl)methyl]benzamid e | 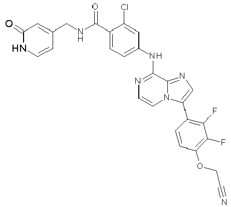 | 562.1 | Intermediate 29 and 4-(aminomethyl)pyridi n-2(1H)-one hydrochloride |
| REF 202 | 2-chloro-4-[[3-[4-(cyanomethoxy)-2,3-difluorophenyl]imidazo[1,2-a]pyrazin-8-yl]amino]-N-[(1-methyl-2-oxo-4-pyridyl)methyl]benz amide | 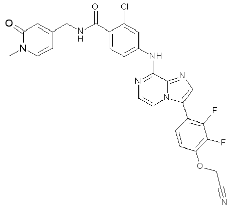 | 576.1 | Intermediate 29 and 4-(aminomethyl)-1-methylpyridin-2(1H)-one hydrochloride |
| REF 203 | 2-chloro-4-[[3-[4-(cyanomethoxy)-2,3-difluorophenyl]imidazo[1,2-a]pyrazin-8-yl]amino]-N-[(1-methyl-6-oxo-3-pyridyl)methyl]benz amide | 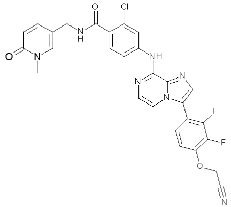 | 576.1 | Intermediate 29 and 5-(aminomethyl)-1-methylpyridin-2(1H)-one hydrochloride |
| REF 204 | 2-[4-[8-[3-chloro-4-[4-(1,2,4-triazol-4-yl)piperidine-1-carbonyl]anilino]imi dazo[1,2-a]pyrazin-3-yl]-2,3-difluoro-phenoxy]acetonitrile | 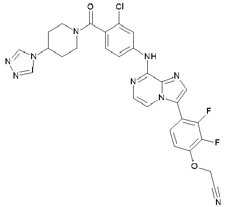 | 590.1 | Intermediate 29 and 4-(4H-1,2,4-triazol-4-yl)piperidine |
| REF 134 | 2-[4-[8-[3-chloro-4-[4-(1H-imidazol-4-yl)piperidine-1-carbonyl]anilino]imi dazo[1,2-a]pyrazin-3-yl]-2,3-difluoro-phenoxy] acetonitrile ;2,2,2-trifluoroacetic acid | 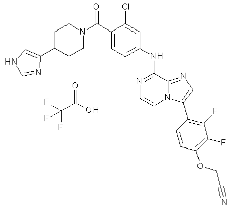 | 589.1 | Intermediate 29 and 4-cyclohexyl-1H-imidazole |
| REF 205 | 2-[3-chloro-4-[8-[3-chloro-4-[4-[2-(dimethylamino)ethy lamino]piperidine-1-carbonyl]anilino]imi dazo[1,2-a]pyrazin-3-yl]-2-fluoro-phenoxy] acetonitrile ;2,2,2-trifluoroacetic acid | 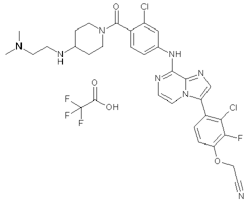 | 625.1 | Intermediate 24 and N,N-dimethyl-3-(piperazin-1-yl)propan-1-amine |
| REF 206 | 2-[4-[8-[3-chloro-4-[4-(2-pyrrolidin-1-ylethyl)piperazine-1-carbonyl] anilino] imi dazo[1,2-a]pyrazin-3-yl]-2,3-difluoro-phenoxy] acetonitrile ;2,2,2-trifluoroacetic acid | 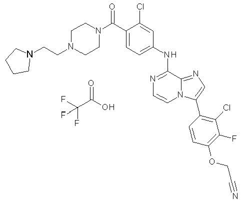 | 637.2 | Intermediate 24 and 1-(2-(pyrrolidin-1-yl)ethyl)piperazine |
| REF 207 | N-(2-(2-aminoethoxy)ethyl)-4-((3-(4-methoxyphenyl)imid azo[1,2-a]pyrazin-8-yl)amino)-N,2-dimethylbenzamide hydrochloride | 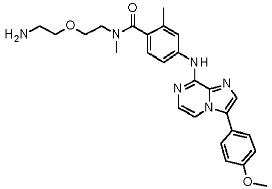 | 475.3 | Intermediate 4 and tert-butyl (2-(2-(methylamino)ethoxy )ethyl)carbamate hydrochloride |
| REF 208 | 4-((3-(4-chlorophenyl)imidaz o[1,2-a]pyrazin-8-yl)amino)-N,2-dimethyl-N-(2-(piperidin-4-yl)ethyl)benzamide hydrochloride | 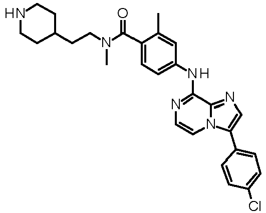 | 503.4 | Intermediate 82 and tert-butyl 4-(2-(methylamino)ethyl)p iperidine-1-carboxylate |
| REF 209 | 4-((3-(4-(difluoromethoxy)-3-fluorophenyl)imidaz o[1,2-a]pyrazin-8-yl)amino)-N,2-dimethyl-N-(2-(piperidin-4-yl)ethyl)benzamide hydrochloride | 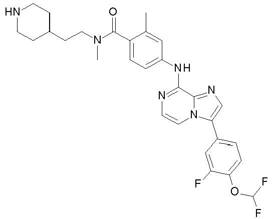 | 551.5( M-H) | Intermediate 43 tert-butyl 4-(2-(methylamino)ethyl)p iperidine-1-carboxylate |
| REF 210 | 2-chloro-4-((3-(3-fluoro-4-methoxyphenyl)imid azo[1,2-a]pyrazin-8-yl)amino)-N-methyl-N-(2-(piperazin-1-yl)ethyl)benzamide hydrochloride | 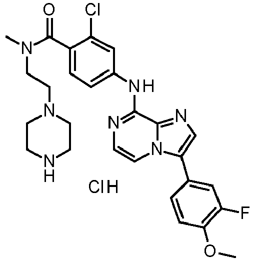 | 539.7 | Intermediate 2 and tert-butyl 4-(2-(methylamino)ethyl)p iperazine-1-carboxylate |
| REF 211 | 2-(4-(aminomethyl)piperi dine-1-carbonyl)-5 - ((3-(4-(difluoromethoxy)ph enyl)imidazo[1,2-a]pyrazin-8-yl)amino)benzonitrile | 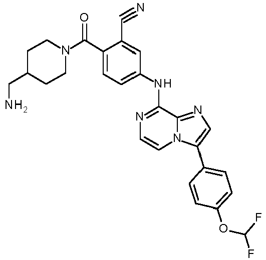 | 518.2 | Intermediate 45 and tert-butyl (piperidin-4-ylmethyl)carbamate (Deprotection with TFA) |
| REF 212 | 4-((3-(4-(difluoromethoxy)ph enyl)imidazo[1,2-a]pyrazin-8-yl)amino)-2-iodo-N-methylbenzamide | 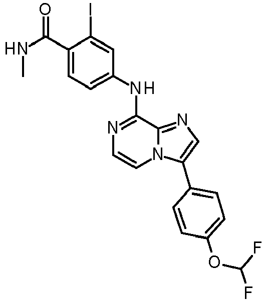 | 536 | Intermediate 46 and methanamine hydrochloride |
| REF 213 | 4-((3-(4-(difluoromethoxy)ph enyl)imidazo[1,2-a]pyrazin-8-yl)amino)-N-methyl-2-vinylbenzamide | 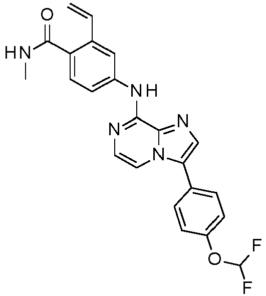 | 436.2 | Intermediate 47 and methanamine hydrochloride |
| REF 542 | 2-bromo-4-((3-(4-(difluoromethoxy)ph enyl)imidazo[1,2-a]pyrazin-8-yl)amino)-N-methylbenzamide | 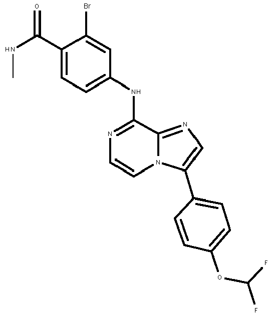 | 488.1 | Intermediate 10 and methanamine hydrochloride |
| REF 214 | [4-(aminomethyl)-4-hydroxypiperidin-1-yl]-[4-[[3-(3-fluoro-4-methoxyphenyl)imid azo[1,2-a]pyrazin-8-yl]amino]-2-methylphenyl]metha none;2,2,2-trifluoroacetic acid | 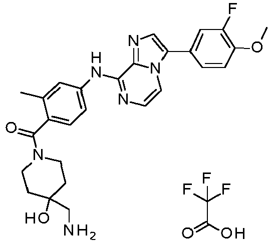 | 505.3 | Intermediate 6 and tert-butyl ((4-hydroxypiperidin-4-yl)methyl)carbamate |
| REF 215 | (4-((1H-imidazol-4-yl)methyl)piperidin-1-yl)(4-((3-(4-(difluoromethoxy)ph enyl)imidazo[1,2-a]pyrazin-8-yl)amino)-2-methylphenyl)metha none | 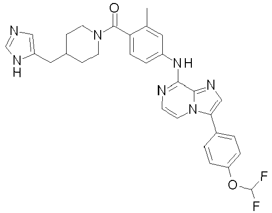 | 558.1 | Intermediate 7 and 4-((1H-imidazol-4-yl)methyl)piperidine dihydrobromide |
| REF 216 | N-(2-((2-aminoethyl)thio)ethy 1)-4-((3-(3-fluoro-4-methoxyphenyl)imid azo[1,2-a]pyrazin-8-yl)amino)-2-methylbenzamide | 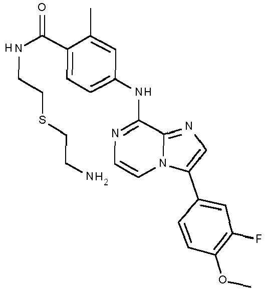 | 495.2 | Intermediate 6 and 2,2'-thiodiethanamine |
| REF 217 | (4-(azetidin-3-yl)piperazin-1-yl)(4-((3-(3-fluoro-4-methoxyphenyl)imid azo[1,2-a]pyrazin-8-yl)amino)-2-methylphenyl)metha none hydrochloride | 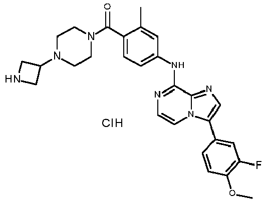 | 516.3 | Intermediate 6 and tert-butyl 3-(piperazin-1-yl)azetidine-1-carboxylate |
| 84 | N-(2-((2-aminoethyl)thio)ethy 1)-4-((3-(4-(difluoromethoxy)ph enyl)imidazo[1,2-a]pyrazin-8-yl)amino)-2-methyl benzamide | 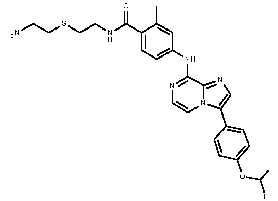 | 513.3 | Intermediate 7 and 2,2'-thiodiethanamine |
| REF 218 | N-(4-aminobutyl)-4-((3-(4-(difluoromethoxy)-2,3-difluorophenyl)imid azo[1,2-a]pyrazin-8-yl)amino)-2-ethyl benzamide hydrochloride | 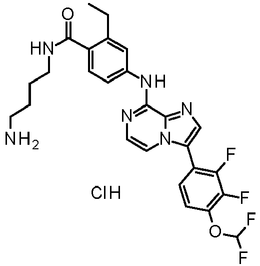 | 531.3 | Intermediate 85 and tert-butyl (4-aminobutyl) carbamat e |
| REF 219 | 4-(4-((3-(4-(difluoromethoxy)ph enyl)imidazo[1,2-a]pyrazin-8-yl)amino)-2-methylbenzoyl)piper azine-1-carboxamide | 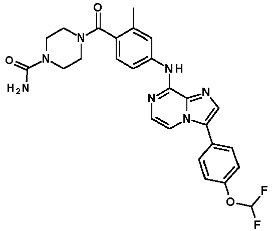 | 522.2 | Intermediate 7 and piperazine-1-carboximidamide |
| REF 220 | N-(azetidin-3-ylmethyl)-1-(4-((3-(2,3-difluoro-4-methoxyphenyl)imid azo[1,2-a]pyrazin-8-yl)amino)-2-methylbenzoyl)piper idine-4-carboxamide hydrochloride | 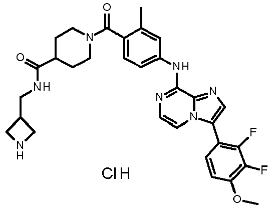 | 590.3 | Intermediate 65 and benzyl 3-(aminomethyl)azetidi ne-1-carboxylate |
| REF 221 | (2-chloro-4-((3-(3-fluoro-4-methoxyphenyl)imid azo[1,2-a]pyrazin-8-yl)amino)phenyl)(4-(2-(dimethylamino)ethy 1)-4-hydroxypiperidin-1-yl)methanone | 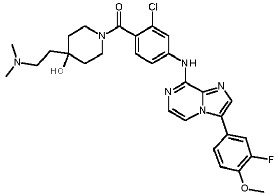 | 567.2 | Intermediate 2 and 4-(2-(dimethylamino)ethyl )piperidin-4-ol |
| REF 222 | 4-((3-(4-(difluoromethoxy)-2,3-difluorophenyl)imid azo[1,2-a]pyrazin-8-yl)amino)-2-ethyl-N-(3-(pyrrolidin-1-yl)propyl)benzamide | 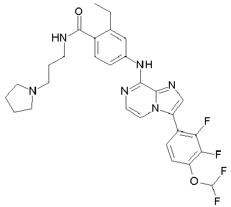 | 571.4 | Intermediate 85 and 3-(pyrrolidin-1-yl)propan-1-amine |
| REF 223 | N-(5 -aminopentyl)-4-((3-(4-(difluoromethoxy)-2,3-difluorophenyl)imid azo[1,2-a]pyrazin-8-yl)amino)-2-ethylbenzamide hydrochloride | 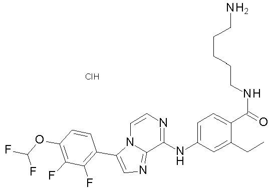 | 545.3 | Intermediate 85 and tert-butyl (5-aminopentyl)carbamate |
| 3 | (R)-4-((3-(2,3-difluoro-4-methoxyphenyl)imid azo[1,2-a]pyrazin-8-yl)amino)-2-ethyl-N-(pyrrolidin-3-yl)benzamide hydrochloride | 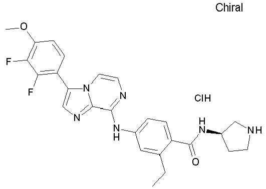 | | Intermediate 86 and rac-tert-butyl (R)-3-aminopyrrolidine-1-carboxylate |
| REF 224 | (S)-N-(4-aminobutan-2-yl)-4-((3-(2,3-difluoro-4-methoxyphenyl)imid azo[1,2-a]pyrazin-8-yl)amino)-2-ethylbenzamide hydrochloride | 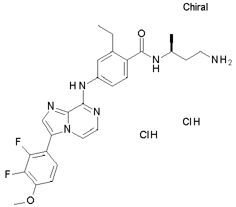 | | Intermediate 86 and rac-tert-butyl (R)-(3-aminobutyl)carbamat e |
| REF 225 | N-(3-amino-2,2-dimethylpropyl)-4-((3-(2,3-difluoro-4-methoxyphenyl)imid azo[1,2-a]pyrazin-8-yl)amino)-2-ethyl benzamide hydrochloride | 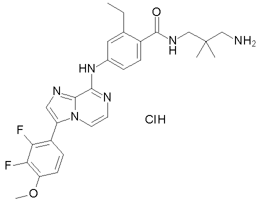 | 509.4 | Intermediate 86 and tert-butyl (3-amino-2,2-dimethylpropyl)carba mate |
| REF 226 | N-(1-amino-2-methylpropan-2-yl)-4-((3-(2,3-difluoro-4-methoxyphenyl)imid azo[1,2-a]pyrazin-8-yl)amino)-2-ethylbenzamide hydrochloride | 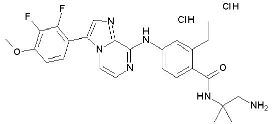 | 495.4 | Intermediate 86 and tert-butyl (2-amino-2-methylpropyl)carbam ate hydrochloride |
| REF 227 | N-(4-amino-2-methylbutan-2-yl)-4-((3-(4-(difluoromethoxy)-2,3-difluorophenyl)imid azo[1,2-a]pyrazin-8-yl)amino)-2-ethylbenzamide hydrochloride | 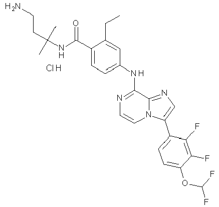 | 545.2 | Intermediate 85 and tert-butyl (3-amino-3-methylbutyl)carbama te |
| 4 | N-(1-(aminomethyl)cyclo propyl)-4-((3-(2,3-difluoro-4-methoxyphenyl)imid azo[1,2-a]pyrazin-8-yl)amino)-2-ethylbenzamide hydrochloride | 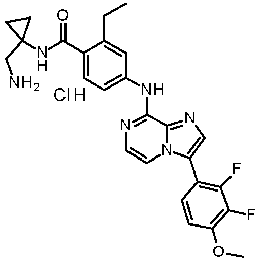 | 493.3 | Intermediate 86 and tert-butyl ((1-aminocyclopropyl)m ethyl) carbamate |
| REF 228 | (R)-N-(1-aminopropan-2-yl)-4-((3-(2,3-difluoro-4-methoxyphenyl)imid azo[1,2-a]pyrazin-8-yl)amino)-2-ethylbenzamide dihydrochloride | 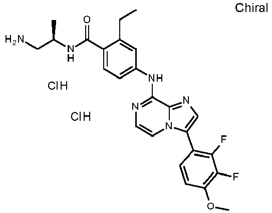 | 481.3 | Intermediate 86 and tert-butyl (R)-(2-aminopropyl)carbam ate |
| REF 229 | (S)-N-(1-aminopropan-2-yl)-4-((3-(2,3-difluoro-4-methoxyphenyl)imid azo[1,2-a]pyrazin-8-yl)amino)-2-ethylbenzamide dihydrochloride | 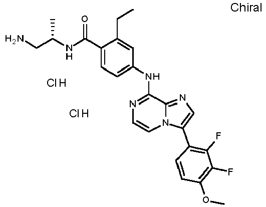 | 481.3 | Intermediate 86 and tert-butyl (S)-(2-aminopropyl)carbam ate |
| REF 230 | N-(3-(2-aminoacetamido)pro pyl)-4-((3-(2,3-difluoro-4-methoxyphenyl)imid azo[1,2-a]pyrazin-8-yl)amino)-2-ethylbenzamide hydrochloride | 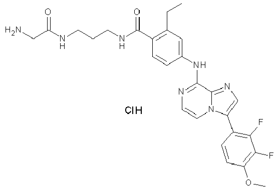 | 538.3 | Intermediate 87 and (tert-butoxycarbonyl)glyci ne |
| REF 231 | (S)-(4-(4-((3-(2,3-difluoro-4-methoxyphenyl)imid azo[1,2-a]pyrazin-8-yl)amino)-2-methylbenzoyl)piper azin-1-yl)(4,4-dimethylpyrrolidin-2-yl)methanone hydrochloride | 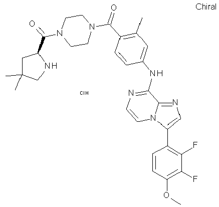 | 604.4 | Intermediate REF 232 and (S)-1-(tert-butoxycarbonyl)-4,4-dimethylpyrrolidine-2-carboxylic acid |
| REF 233 | (S)-(4-(4-((3-(2,3-difl uoro-4-methoxyphenyl)imid azo[1,2-a]pyrazin-8-yl)amino)-2-methylbenzoyl)piper azin-1-yl)(5,5-dimethylpyrrolidin-2-yl)methanone hydrochloride | 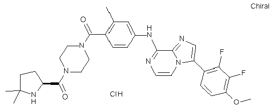 | 604.4 | Intermediate REF 232 and (S)-1-(tert-butoxycarbonyl)-5,5-dimethylpyrrolidine-2-carboxylic acid |
| REF 234 | (4-(4-((3-(2,3-difl uoro-4-methoxyphenyl)imid azo[1,2-a]pyrazin-8-yl)amino)-2-methylbenzoyl)piper azin-1-yl)((2S,3R)-3-hydroxypyrrolidin-2-yl)methanone hydrochloride | 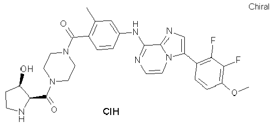 | 592.4 | Intermediate REF 232 and (2S,3R)-1-(tert-butoxycarbonyl)-3-hydroxypyrrolidine-2-carboxylic acid |
| REF 235 | [4-[[3-(2,3-difluoro-4-methoxyphenyl)imid azo[1,2-a]pyrazin-8-yl]amino]-2-methylphenyl]-[4-[(2#R!,3#S!)-3-hydroxypyrrolidine-2-carbonyl]piperazin-1-yl]methanone;hydro chloride | 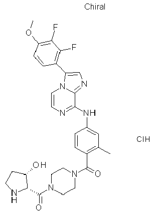 | 592.4 | Intermediate REF 232 and (2R,3S)-1-(tert-butoxycarbonyl)-3-hydroxypyrrolidine-2-carboxylic acid |
| REF 236 | (4-(4-((3-(2,3-difl uoro-4-methoxyphenyl)imid azo[1,2-a]pyrazin-8-yl)amino)-2-methylbenzoyl)piper azin-1-yl)(2-methylpyrrolidin-2-yl)methanone hydrochloride | 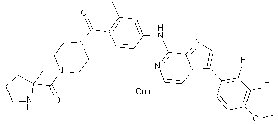 | 590.4 | Intermediate REF 232 and 1-(tert-butoxycarbonyl)-2-methylpyrrolidine-2-carboxylic acid |
| REF 237 | (4-(4-((3-(2,3-difl uoro-4-methoxyphenyl)imid azo[1,2-a]pyrazin-8-yl)amino)-2-methylbenzoyl)piper azin-1-yl)((2S,4R)-4-fluoropyrrolidin-2-yl)methanone hydrochloride | 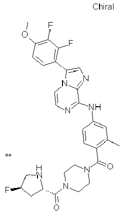 | 594.4 | Intermediate REF 232 and (2S,4R)-1-(tert-butoxycarbonyl)-4-fluoropyrrolidine-2-carboxylic acid |
| REF 238 | (4-((1R,2S,5S)-3-azabicyclo[3.1.0]hex ane-2-carbonyl)piperazin-1-yl)(4-((3-(2,3-difluoro-4-methoxyphenyl)imid azo[1,2-a]pyrazin-8-yl)amino)-2-methylphenyl)metha none hydrochloride | 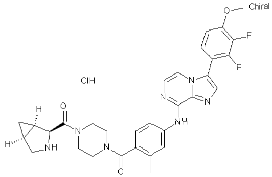 | 588.4 | Intermediate REF 232 and rac-(1R,2S,5S)-3-(tert-butoxycarbonyl)-3 - azabicyclo[3.1.0]hex ane-2-carboxylic acid |
| REF 239 | (4-(4-((3-(2,3-difl uoro-4-methoxyphenyl)imid azo[1,2-a]pyrazin-8-yl)amino)-2-methylbenzoyl)piper azin-1-yl)((2S,4S)-4-fluoropyrrolidin-2-yl)methanone hydrochloride | 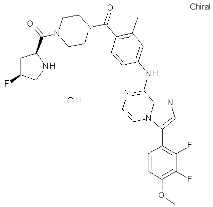 | 594.4 | Intermediate REF 232 and (2S,4S)-1-(tert-butoxycarbonyl)-4-fluoropyrrolidine-2-carboxylic acid |
| REF 240 | (4-(4-((3-(2,3-difl uoro-4-methoxyphenyl)imid azo[1,2-a]pyrazin-8-yl)amino)-2-methylbenzoyl)piper azin-1-yl)((2S,4S)-4-(methoxymethyl)pyr rolidin-2-yl)methanone hydrochloride | 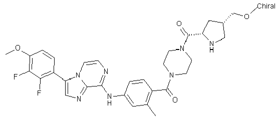 | 620.4 | Intermediate REF 232 and (2S,4S)-1-(tert-butoxycarbonyl)-4-(methoxymethyl)pyrr olidine-2-carboxylic acid |
| REF 241 | [4-[(2S,4R)-4-aminopyrrolidine-2-carbonyl] piperazin-1-yl]-[4-[[3-(2,3difluoro-4-methoxyphenyl)imid azo[1,2-a]pyrazin-8-yl]amino]-2-methylphenyl]metha none | 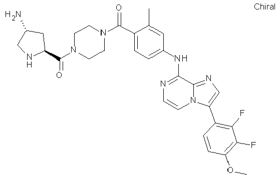 | 591.4 | Intermediate REF 232 and (2S,4R)-4-((((9H-fluoren-9-yl)methoxy)carbonyl) amino)-1-(tert-butoxycarbonyl)pyrrolidine-2-carboxylic acid. Final Fmoc removal with 4-methyl piperidine |
| REF 242 | (4-aminopiperidin-4-yl)(4-(4-((3-(2,3-difluoro-4-methoxyphenyl)imid azo[1,2-a]pyrazin-8-yl)amino)-2-methylbenzoyl)piper azin-1-yl)methanone | 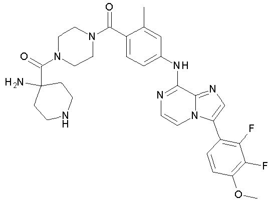 | 605.3 | Intermediate REF 232 and 4-((((9H-fluoren-9-yl)methoxy)carbonyl) amino)-1-(tert-butoxycarbonyl)piper idine-4-carboxylic acid. Final Fmoc removal with piperidine |
| REF 243 | 2-(4-(8-((4-(4-(2,6-diazaspiro[3.3]hepta ne-2-carbonyl)piperidine-1-carbonyl)-3-methylphenyl)amino )imidazo[1,2-a]pyrazin-3-yl)-2,3-difluorophenoxy)ace tonitrile | 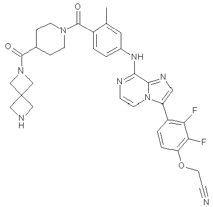 | 625.4( M-H) | Intermediate 69 and tert-butyl 2,6-diazaspiro[3.3]heptan e-2-carboxylate |
| REF 244 | 1-(4-((3-(2,3-difluoro-4-methoxyphenyl)imid azo[1,2-a]pyrazin-8-yl)amino)-2-methylbenzoyl)-N- (2-hydroxy-3-ureidopropyl)piperid ine-4-carboxamide | 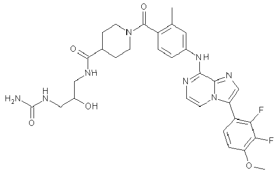 | 637.3 | Intermediate 65 and 1-(3-amino-2-hydroxypropyl)urea |
| | | | | |
| REF 245 | 1-(4-((3-(2,3-difl uoro-4-methoxyphenyl)imid azo[1,2-a]pyrazin-8-yl)amino)-2-methylbenzoyl)-N- (3-(dimethylamino)-2-hydroxypropyl)piper idine-4-carboxamide | 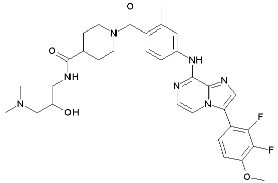 | 622.2 | Intermediate 65 and 1-amino-3-(dimethylamino)prop an-2-ol |
| REF 246 | 1-(4-((3-(2,3-difluoro-4-methoxyphenyl)imid azo[1,2-a]pyrazin-8-yl)amino)-2-methylbenzoyl)-N- (2-hydroxy-3-(piperazin-1-yl)propyl)piperidine-4-carboxamide hydrochloride | 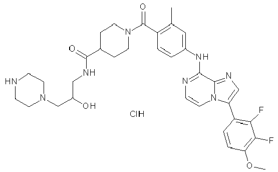 | 663.3 | Intermediate 65 and tert-butyl 4-(3-amino-2-hydroxypropyl)pipera zine-1-carboxylate |

### Reference Example 247 and Reference Example 248

### 2-chloro-4-[[3-[4-(cyanomethoxy)-2,3-difluoro-phenyl]imidazo[1,2-a]pyrazin-8-yl]amino]-N-[3-(dimethylamino)propylcarbamoyl]-N-ethyl-benzamide and 2-chloro-4-[[3-[4-(cyanomethoxy)-2,3-difluoro-phenyl]imidazo[1,2-a]pyrazin-8-yl]amino]-N-[2-(1H-tetrazol-5-yl)ethyl] benzamide

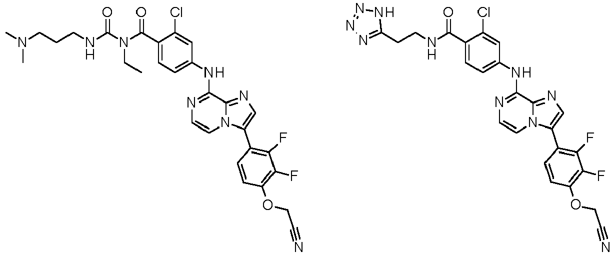

To a solution of Intermediate 29 (230 mg, 0.5 mmol), 4-(4-methyl-4H-1,2,4-triazol-3-yl)piperidine hydrochloride(122 mg, 0.6 mmol) in anhydrous DMF (10 mL) was added DIPEA (129 mg,1.0 mmol) and DMAP (73 mg,0.6 mmol), Followed by the resultant mixture was stirred for 30 min at room temperature, EDCI (115 mg, 0.6 mmol) was added in the mixture and stirred for extra 10 h.

The mixture was poured into water (50 mL) and the aqueous solution was extracted with DCM (50 mL×2). The organic layers were combined and washed with water and brine, dried over anhydrous sodium sulphate and concentrated under reduced pressure to give a red oil, which was purified by prep.HPLC to give Reference Example 247 (50 mg, 16% yield) as a white powder MS (ESI, m/z): 611.2 [M+H]+ and Reference Example 248 (60 mg, 21.3% yield) as a white powder.MS (ESI, m/z): 551.1 [M+H]+

### Reference Example 249

### N-[[1-[4-[[3-[4-(cyanomethoxy)-2,3-difluoro-phenyl]imidazo[1,2-a]pyrazin-8-yl]amino]-2-methyl-benzoyl]-4-piperidyl]methyl]-2-hydroxy-acetamide

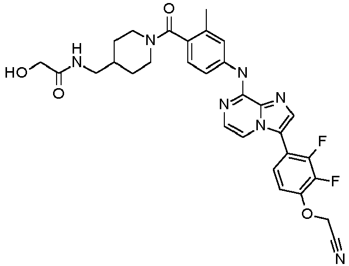

To a solution of Reference Example 139 (106 mg, 0.2 mmol), 2-hydroxyacetic acid (16 mg, 0.2 mmol) in anhydrous DMF (5 mL) was added DIPEA (52 mg,0.4 mmol) and then the resultant mixture was stirred for 30 min at room temperature, HATU (152 mg, 0.4 mmol) was added in the mixture and stirred for extra 10 h. The mixture was poured into water (50 mL) and the aqueous solution was extracted with DCM (50 mL x2). The organic layers were combined and washed with water and brine, dried over anhydrous sodium sulphate and concentrated under reduced pressure to give a red oil, which was purified by prep.HPLC to give Reference Example 249 (5 mg, 4.2 % yield) as a white powder MS (ESI, m/z): 590.2 [M+H]+

The following example was prepared in analogy to Reference Example 249

| Ex. | Name | Structure | ESI MS [M+H]+ | Starting Material |
|---|---|---|---|---|
| RE F 25 0 | N-[[1-[2-chloro-4-[[3-[4-(cyanomethoxy)-2,3-difluorophenyl]imidazo[1,2-a]pyrazin-8-yl]amino]benzoyl]-4-piperidyl]methyl]pyridin e-4-carboxamide 2,2,2-trifluoroacetate | 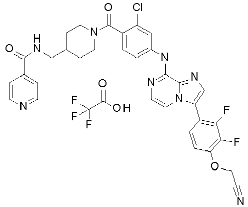 | 657.1 | Reference Example 138 and isonicotinic acid |

### Intermediate 81:

### tert-butyl 2-(hydroxymethyl)-4-(piperidine-4-carbonyl)piperazine-1-carboxylate

### Step 1: tert-butyl 4-(1-benzyloxycarbonylpiperidine-4-carbonyl)-2-(hydroxymethyl)piperazine-1-carboxylate

A mixture of 1-[(benzyloxy)carbonyl]piperidine-4-carboxylic acid (2.89 g, 10.99 mmol, 1.1 eq), tert-butyl 2-(hydroxymethyl)piperazine-1-carboxylate (2.16 g, 9.99 mmol, 1 eq), O-(7-azabenzotriazol-1-yl)-N,N,N',N'-tetramethyluronium hexafluorophosphate (5.7 g, 14.98 mmol, 1.5 eq) and N,N-diisopropylethylamine (5.22 mL, 29.96 mmol, 3 eq) in DMF (25 mL) was stirred at 25 °C for 14 h. The mixture was added water (50 mL) and extracted with ethyl acetate (50 mL × 3). The combined organic layers were washed with saturated NH₄Cl solution (50 mL) and concentrated to dryness. The crude product was purified by prep. HPLC. To the desired fractions were added NaHCO₃ (s) until pH 7~8 and extracted with ethyl acetate (100 mL × 3). The combined organic layers were dried over sodium sulphateand concentrated *in vacuo* to afford tert-butyl 4-(1-benzyloxycarbonylpiperidine-4-carbonyl)-2-(hydroxymethyl)piperazine-1-carboxylate (2.28 g) as a brown oil. MS obsd. (ESI⁺): 461.9 [(M+H)⁺].

### Step 2: tert-butyl 2-(hydroxymethyl)-4-(piperidine-4-carbonyl)piperazine-1-carboaylate

A mixture of tert-butyl 4-(1-benzyloxycarbonylpiperidine-4-carbonyl)-2-(hydroxyntethyl)piperazine-1-carboxylate (2.08 g, 4.51 mmol, 1 eq) and Pd/C (10%, 300 mg) in ethyl acetate (20 mL) was stirred at 25 °C for 72 h under hydrogen atmosphere. The mixture was filtered over celite and the filtrate was concentrated to dryness to afford tert-butyl 2-(hydroxymethyl)-4-(piperidine-4-carbonyl)piperazine-1-carboxylate (Intermediate 81) (1.29 g, 3.94 mmol, 87.43 % yield) as black oil. The crude product was used in next step without any purification.

MS obsd. (ESI⁺): 328.2 [(M+H)⁺].

### Reference Example 251:

### 4-[[3-(3-fluoro-4-methoxy-phenyl)imidazo[1,2-a] pyrazin-8-yl] amino]-N-(2-imidazol-1-ylethyl)-N,2-dimethyl-benzamide

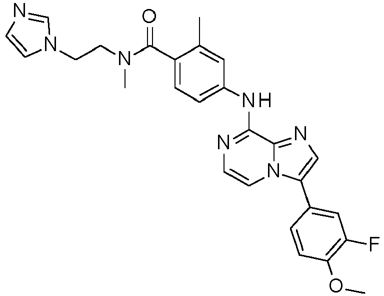

### Step 1: 2-imidazol-1-ylethyl methanesulfonate

A mixture of 1-(2-hydroxyethyl)imidazole (1.0 g, 8.92 mmol) in DCM (10 mL) was added methanesulfonyl chloride (1.02 g, 8.92 mmol) and triethylamine (2.5 mL, 17.84 mmol). After stirring at 20 °C for 4h, the reaction was quenched with H₂O (10 mL) and concentrated to dryness. The residue was diluted with EA (30 mL) and washed with water (30 mL) and brine (30 mL), dried over anhydrous sodium sulphate, concentrated under reduced pressure to afford the crude title product (500 mg) as yellow oil which was used in next step directly.

### Step 2: 2-imidazol-1-yl-N-methyl-ethanamine

A mixture of 2-imidazol-1-ylethyl methanesulfonate (250 mg, 1.31 mmol) and a solution of monomethylamine in EtOH (2 mL) was stirred at 70 °C for 12 h. The reaction mixture was concentrated under reduced pressure to afford crude product (150 mg) as yellow oil which was used directly in next step.

### Step 3: 4-[[3-(3-fluoro-4-methoxy-phenyl)imidazo[1,2-a]pyrazin-8-yl]amino]-N-(2-imidazol-1-ylethyl)-N,2-dimethyl-benzamide

Into a stirred solution of intermediate 6 (200 mg, 0.51 mmol), 2-imidazol-1-yl-N-methyl-ethanamine (96 mg, 0.76 mmol) and triethylamine (0.2 mL, 1.53 mmol) in DMF (2 mL) was added 1-propanephosphonic anhydride (486 mg, 0.76 mmol) slowly. The reaction was stirred at 25°C for 12h and then concentrated to dryness. The resiude was diluted with ethyl acetate (10 mL) and the resulting mixture was washed with water (3 mL) and brine (3 mL), dried over anhydrous sodium sulphate, concentrated under reduced pressure to afford crude product. The residue was purified by prep-HPLC to afford the title compound (50 mg) as yellow solid. MS obsd. (ESI⁺) [(M+H)⁺]: 500.3

### Reference Example 252

### 2-chloro-4-[[3-(2,3-difluoro-4-methoxy-phenyl)imidazo[1,2-a]pyrazin-8-yl]amino]-N-[(1,1-dioxothian-4-yl)methyl]benzamide;2,2,2-trifluoroacetic acid

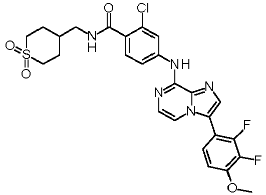

A mixture of Reference Example 83 (200 mg, 368 µmol) and oxone (678 mg, 1.1 mmol) in DMF (5 mL) was stirred at rt for 4 hrs. The mixture was diluted with H₂O (40 mL) and extracted with DCM. The organic layer was dried and concentrated *in vacuo.* The residue was purified by prep-HPLC to give the title compound (56 mg) as light yellow solid. MS (ESI, m/z): 576.1.

### Reference Example 253

### N-[2-(2-Aminoethoxy)ethyl]-2-methyl-4-[[3-(4-prop-2-ynoxyphenyl)imidazo[1,2-a]pyrazin-8-yl]amino]benzamide; formic acid

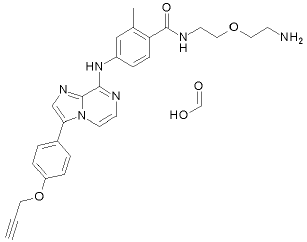

### Step 1:

### 8-Chloro-3-(4-prop-2-ynoxyphenyl)imidazo[1,2-a]pyrazine

A mixture of 4-(8-chloroimidazo[1,2-a]pyrazin-3-yl)phenol (intermediate 50, 100 mg, 0.410 mmol, 1 eq) and potassium carbonate (169 mg, 1.22 mmol, 3 eq) in DMF (3 mL) was added propargyl bromide (145 mg, 1.22 mmol, 3 eq) at 20 °C and stirred at 20 °C for 16 h . The mixture was filtered, poured into water, extracted with ethyl acetate, concentrated and purified by prep-TLC (PE/ethyl acetate=1:1) to afford the desired product (61 mg) as a yellow solid.

### Step 2:

### tert-Butyl N-[2-[2-[[2-methyl-4-[[3-(4-prop-2-ynoxyphenyl)imidazo[1,2-a]pyrazin-8-yl]amino]benzoyl]amino]ethoxy]ethyl]carbamate

A mixture of tert-butyl N-[2-[2-[(4-amino-2-methyl-benzoyl)amino]ethoxy]ethyl]carbamate (intermediate 78, 45.0 mg, 0.130 mmol, 1 eq), 8-chloro-3-(4-prop-2-ynoxyphenyl)imidazo[1,2-a]pyrazine (37.84 mg, 0.130 mmol, 1 eq), cesium carbonate (130.36 mg, 0.400 mmol, 3 eq), tris(dibenzylideneacetone)dipalladium (0) (12.21 mg, 0.010 mmol, 0.100 eq) and 9,9-dimethyl-4,5-bis(diphenylphosphino)xanthene (7.72 mg, 0.010 mmol, 0.100 eq) in 1,4-dioxane (5 mL) was stirred under N₂ at 115 °C on microwave for 2 h. The mixture was filtered and concentrated, purified by prep-TLC(DCM/MeOH/MeCN=10:1:1) to afford product (20 mg) as a light yellow solid.

**Step 3:**

### N-[2-(2-Aminoethoxy)ethyl]-2-methyl-4-[[3-(4-prop-2-ynoxyphenyl)imidazo[1,2-a]pyrazin-8-yl]amino]benzamide formate

A solution of tert-butyl N-[2-[2-[[2-methyl-4-[[3-(4-prop-2-ynoxyphenyl)imidazo[1,2-a]pyrazin-8-yl]amino]benzoyl]amino]ethoxy]ethyl]carbamate (50.0 mg, 0.090 mmol, 1 eq) in DCM (4 mL) was added trifluoroacetic acid (0.39 mL, 5.11 mmol, 59.78 eq) and stirred at 20 °C for 16 h. The solution was concentrated and purified by prep-HPLC to afford 13.4 mg product as white solid. MS (ESI, m/z): 485.4

### Reference Example 254

### N-[2-(2-aminoethoxy)ethyl]-4-[[3-[4-(4-hydroxybut-2-ynoxy)phenyl]imidazo[1,2-a]pyrazin-8-yl]amino]-2-methyl-benzamide formate

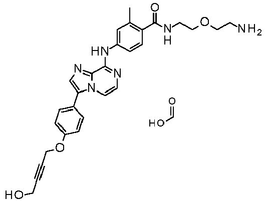

The title compound was obtained in analogy to Reference Example 253 using 4-hydroxybut-2-ynyl methanesulfonate instead of propargyl bromide. MS (ESI, m/z): 515.3

### Reference Example 255

### N-(2-(2-aminoethoxy)ethyl)-4-((3-(3-chloro-4-methoxyphenyl)imidazo[1,2-a]pyrazin-8-yl)amino)-2-methylbenzamide hydrochloride

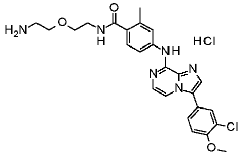

To Intermediate 79 (24 mg) in dioxane (900 µl) and water (100 µl) was added (3-chloro-4-methoxyphenyl)boronic acid (11.6 mg), 1,1'-bis(diphenylphosphino)ferrocenedichloro palladium(II) dichloromethane complex (3.03 mg, 4.13 µmol) and potassium carbonate (14.3 mg, 103 µmol) followed by stirring at 105 °C overnight. The reaction mixture was concentrated and purified by prep. HPLC to give a Boc-protected intermediate, which was deprotected to the title compound (11 mg, colorless solid) by addition of 4M HCl in dioxane (1 h), followed by concentration and drying *in vacuo.* MS (ESI, m/z): 495.3

The following examples were prepared in analogy to Reference Example 255:

| Ex. | Name | Structure | MS ESI [M+H]⁺ | Starting Material |
|---|---|---|---|---|
| REF 256 | N-(2-(2-aminoethoxy)ethyl)-4-((3-(4-fluorophenyl)imidaz o[1,2-a]pyrazin-8-yl)amino)-2-methylbenzamide hydrochloride | 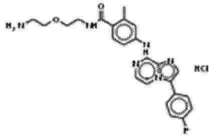 | 449.7 | Intermediate 79 and (4-fluorophenyl)boronic acid |
| REF 257 | N-(2-(2-aminoethoxy)ethyl)-4-((3-(4-chloro-3-fluorophenyl)imidaz o[1,2-a]pyrazin-8-yl)amino)-2-methylbenzamide hydrochloride | 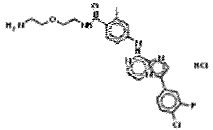 | 483.1 | Intermediate 79 and (4-chloro-3-fluorophenyl)boronic acid |
| REF 258 | N-(2-(2-aminoethoxy)ethyl)-4-((3-(3,4-difluorophenyl)imid azo[1,2-a]pyrazin-8-yl)amino)-2-methylbenzamide hydrochloride | 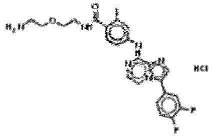 | 467.9 | Intermediate 79 and (3,4-difluorophenyl)boronic acid |
| REF 259 | N-(2-(2-aminoethoxy)ethyl)-4-((3-(2-fluoro-4-methoxyphenyl)imid azo[1,2-a]pyrazin-8-yl)amino)-2-methylbenzamide hydrochloride | 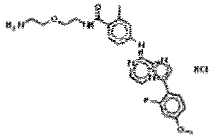 | 479.4 | Intermediate 79 and (2-fluoro-4-methoxyphenyl)boronic acid |
| REF 260 | N-(2-(2-aminoethoxy)ethyl)-4-((3-(2,4-difluorophenyl)imid azo[1,2-a]pyrazin-8-yl)amino)-2-methylbenzamide hydrochloride | 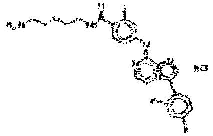 | 467.2 | Intermediate 79 and (2,4-difluorophenyl)boronic acid |
| REF 261 | N-(2-(2-aminoethoxy)ethyl)-4-((3-(3-fluoro-4-methoxyphenyl)imid azo[1,2-a]pyrazin-8-yl)amino)-2-methylbenzamide hydrochloride | 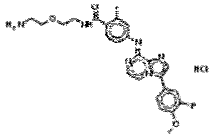 | 479.1 | Intermediate 79 and (3-fluoro-4-methoxyphenyl)boronic acid |
| REF 262 | N-(2-(2-aminoethoxy)ethyl)-2-methyl-4-((3-(2,3,4-trifluorophenyl)imid azo[1,2-a]pyrazin-8-yl)amino)benzamide hydrochloride | 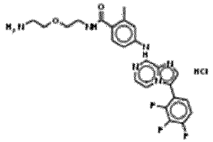 | 485.2 | Intermediate 79 and (2,3,4-trifluorophenyl)boronic acid |
| REF 263 | (4-(4-((3-(3-chloro-4-methoxyphenyl)imid azo[1,2-a]pyrazin-8-yl)amino)-2-methylbenzoyl)piper azin-1-yl)((2S,4R)-4-hydroxypyrrolidin-2-yl)methanone hydrochloride | | 590.2 | Intermediate 75 and (3-chloro-4-methoxyphenyl) boronic acid |
| REF 264 | (4-(4-((3-(3-chloro-2-fluoro-4-methoxyphenyl)imid azo[1,2-a]pyrazin-8-yl)amino)-2-methylbenzoyl)piper azin-1-yl)((2S,4R)-4-hydroxypyrrolidin-2-yl)methanone hydrochloride | 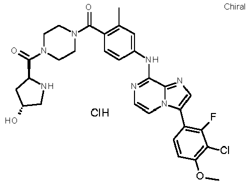 | 609.1 | Intermediate 75 and (3-chloro-2-fluoro-4-methoxyphenyl) boronic acid |
| REF 265 | 2-(2-chloro-4-(8-((4-(4-((2S,4R)-4-hydroxypyrrolidin-2-carbonyl)piperazine-1-carbonyl)-3-methylphenyl)amino )imidazo[1,2-a]pyrazin-3-yl)phenoxy)acetonitr ile | 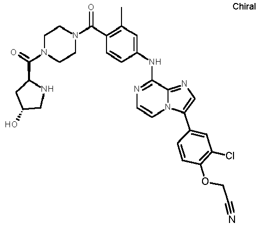 | 615.2 | Intermediate 75 and (2-(2-chloro-4-(4,4,5,5-tetramethyl-1,3,2-dioxaborolan-2-yl)phenoxy)acetonitrile (Intermediate 88) |

### Reference Example 266

### 4-[[3-(2,3-difluoro-4-methoxy-phenyl)imidazo[1,2-a]pyrazin-8-yl]amino]-N,N-dimethyl-benzamide

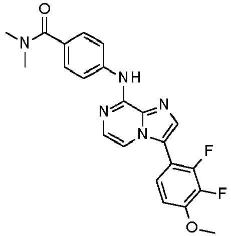

A mixture of 4-((3-iodoimidazo[1,2-a]pyrazin-8-yl)amino)-N,N-dimethylbenzamide (150 mg, 368 µmol), (2,3-difluoro-4-methoxyphenyl)boronic acid (69.2 mg, 368 µmol), K₃PO₄ (235 mg, 1.11 mmol) and PdCl₂(dppf)-CH₂Cl₂ adduct (13.5 mg, 18.4 µmol) in THF (5 mL) and H₂O (1 mL) was heated to 50 °C with stirring overnight. The reaction mixture was diluted with H₂O and extracted with DCM (30 mL) twice. The combined DCM layer was dried and concentrated in vacuo. The residue was purified by prep-HPLC to give 4-((3-(2,3-difluoro-4-methoxyphenyl)imidazo[1,2-a]pyrazin-8-yl)amino)-N,N-dimethylbenzamide (20 mg) as white solid. (ESI+) [(M+H)⁺] : 424.

### Reference Example 267

### N-[2-[2-[(dimethylamino)methyl]morpholin-4-yl]ethyl]-4-[[3-(3-fluoro-4-methoxy-phenyl)imidazo[1,2-a]pyrazin-8-yl]amino]-N,2-dimethyl-benzamide; formic acid

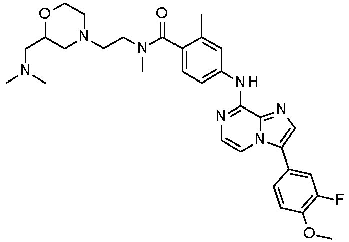

### Step 1: N-(2-chloroethyl)-N,2-dimethyl-4-nitro-benzamide

To a solution of (2-chloroethyl)methylamine (310 mg, 3.31 mmol), 2-methyl-4-nitro-benzoic acid (500 mg, 2.76 mmol), triethylamine (1.15 mL, 8.28 mmol) in DMF (8 mL) was added T3P (2.63 g, 4.14 mmol). The mixture was stirred at 20 °C for 16 h. The mixture was diluted with ethyl acetate (100 mL), washed with water (30 mL), brine (30 mL), dried over sodium sulphateand concentrated. The residue was purified by column chromatography to give N-(2-chloroethyl)-N,2-dimethyl-4-nitro-benzamide (480 mg) as a colorless oil.

### Step 2: N-[2-[2-[(dimethylamino)methyl]morpholin-4-yl]ethyl]-N,2-dimethyl-4-nitro-benzamide

A mixture of N,N-dimethyl-2-morpholinmethanamine (148 mg, 1.03 mmol), N-(2-chloroethyl)-N,2-dimethyl-4-nitro-benzamide (220 mg, 0.860 mmol), N,N-diisopropylethylamine (0.6 mL, 3.43 mmol) in DMSO (3 mL) was stirred at 100 °C for 16 h. After cooling to room temperature, the mixture was diluted with ethyl acetate (100 mL), washed with water (30 mL), brine (30 mL × 2), dried over sodium sulphateand concentrated under reduced pressure. The residue was purified by prep-HPLC (TFA). The fraction was concentrated. The residue was neutralized by aq. NaHCO₃, extracted with ethyl acetate (100 mL), dried over sodium sulphateand concentrated to give N-[2-[2-[(dimethylamino)methyl]morpholin-4-yl]ethyl]-N,2-dimethyl-4-nitro-benzamide (60 mg) as a light-yellow oil.

### Step 3: 4-amino-N-[2-[2-[(dimethylamino)methyl]morpholin-4-yl]ethyl]-N,2-dimethyl-benzamide

To a solution of N-[2-[2-[(dimethylamino)methyl]morpholin-4-yl]ethyl]-N,2-dimethyl-4-nitrobenzamide (60 mg, 0.160 mmol) in ethyl acetate (3 mL) was added Pd/C (10%, 20 mg). The mixture was stirred at 20 °C under H₂ for 16 h. The mixture was filtered and the filtrate was concentrated under reduced pressure to give crude 4-amino-N-[2-[2-[(dimethylamino)methyl]morpholin-4-yl]ethyl]-N,2-dimethyl-benzamide (50 mg) as a yellow oil, which was used directly for the next step without further purification.

### Step 4: N-[2-[2-[(dimethylamino)methyl]morpholin-4-yl]ethyl]-4-[[3-(3-fluoro-4-methoxy-phenyl)imidazo[1,2-a]pyrazin-8-yl]amino]-N,2-dimethyl-benzamide; formic acid

A mixture of 8-chloro-3-(3-fluoro-4-methoxy-phenyl)imidazo[1,2-a]pyrazine (50 mg, 0.180 mmol, 3), 4-amino-N-[2-[2-[(dimethylamino)methyl]morpholin-4-yl]ethyl]-N,2-dimethylbenzamide (50 mg, 0.150 mmol), Brettphos Pd G3 (14 mg, 0.020 mmol, CAS# 1470372-59-8 ), potassium carbonate (62 mg, 0.450 mmol) in tert-butanol (1 mL) was stirred at 100 °C for 16 h. The mixture was diluted with ethyl acetate (100 mL), washed with water (30 mL), brine (30 mL), dried over sodium sulphateand concentrated under reduced pressure. The residue was purified by prep-TLC (DCM:MeOH=10:1), then further purified by prep-HPLC (FA) to give N-[2-[2-[(dimethylamino)methyl]morpholin-4-yl]ethyl]-4-[[3-(3-fluoro-4-methoxyphenyl)imidazo[1,2-a]pyrazin-8-yl]amino]-N,2-dimethyl-benzamide (25.5 mg) as a white solid. MS obsd. (ESI⁺) [(M+H)⁺]: 576.2

### Reference Example 268

### 4-[[3-(3-fluoro-4-methoxy-phenyl)imidazo[1,2-a]pyrazin-8-yl]amino]-N,2-dimethyl-N-[2-[2-(methylaminomethyl)morpholin-4-yl]ethyl]benzamide hydrochloride

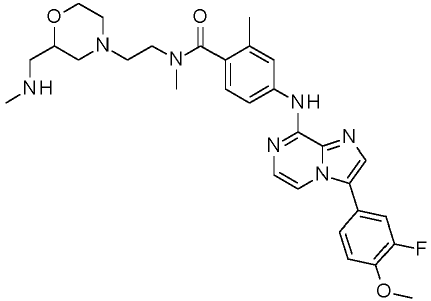

### Step 1: tert-butyl N-methyl-N-[[4-[2-[methyl-(2-methyl-4-nitrobenzoyl)amino]ethyl]morpholin-2-yl]methyl]carbamate

A mixture of N-(2-chloroethyl)-N,2-dimethyl-4-nitro-benzamide (230 mg, 0.900 mmol), tert-butyl N-methyl-N-(morpholin-2-ylmethyl)carbamate (250 mg, 1.09 mmol), N,N-diisopropylethylamine (0.62 mL, 3.58 mmol) in DMSO (5 mL) was stirred at 100 °C for 16 h. After cooling to room temperature, the mixture was diluted with ethyl acetate (100 mL), washed with water (30 mL), brine (30 mL × 2), dried over sodium sulphateand concentrated under reduced pressure. The residue was purified by prep-HPLC to give tert-butyl N-methyl-N-[[4-[2-[methyl-(2-methyl-4-nitro-benzoyl)amino]ethyl]morpholin-2-yl]methyl]carbamate (160 mg) as a light-yellow oil.

### Step 2: tert-butyl N-[[4-[2-[(4-amino-2-methyl-benzoyl)-methyl-amino]ethyl]morpholin-2-yl]methyl]-N-methyl-carbamate

To a solution of N-methyl-N-[[4-[2-[methyl-(2-methyl-4-nitro-benzoyl)amino]ethyl]morpholin-2-yl]methyl]carbamate (160 mg, 0.360 mmol) in ethyl acetate (3 mL) was added Pd/C (10%, 20 mg). The mixture was hydrogenated at 20 °C under H₂ for 16 h. The mixture was filtered and the filtrate was concentrated under reduced pressure to give crude tert-butyl N-[[4-[2-[(4-amino-2-methyl-benzoyl)-methyl-amino]ethyl]morpholin-2-yl]methyl]-N-methyl-carbamate (120 mg) as a yellow solid.

### Step 3: tert-butyl N-[[4-[2-[[4-[[3-(3-fluoro-4-methoxy-phenyl)imidazo[1,2-a]pyrazin-8-yl] amino]-2-methyl-benzoyl] -methyl-amino] ethyl] morpholin-2-yl] methyl] -N-methyl-carbamate

A mixture of 8-chloro-3-(3-fluoro-4-methoxy-phenyl)imidazo[1,2-a]pyrazine (80 mg, 0.290 mmol), N-[[4-[2-[(4-amino-2-methyl-benzoyl)-methyl-amino]ethyl]morpholin-2-yl]methyl]-N-methyl-carbamate (120 mg, 0.290 mmol), Brettphos Pd G3 (27 mg, 0.030 mmol, cas# 1470372-59-8), potassium carbonate (118 mg, 0.850 mmol) in t-BuOH (1.5 mL) was stirred at 100 °C for 16 h. The mixture was diluted with ethyl acetate (100 mL), washed with water (30 mL), brine (30 mL), dried over sodium sulphateand concentrated under reduced pressure. The residue was purified by prep-TLC (DCM/MeOH=15/1) to give tert-butyl N-[[4-[2-[[4-[[3-(3-fluoro-4-methoxy-phenyl)imidazo[1,2-a]pyrazin-8-yl]amino]-2-methyl-benzoyl]-methyl-amino]ethyl]morpholin-2-yl]methyl]-N-methyl-carbamate (120 mg) as a yellow oil.

### Step 4: 4-[[3-(3-fluoro-4-methoxy-phenyl)imidazo[1,2-a]pyrazin-8-yl]amino]-N,2-dimethyl-N-[2-[2-(methylaminomethyl)morpholin-4-yl]ethyl]benzamide

To a solution of tert-butyl N-[[4-[2-[[4-[[3-(3-fluoro-4-methoxy-phenyl)imidazo[1,2-a]pyrazin-8-yl]amino]-2-methyl-benzoyl]-methyl-amino]ethyl]morpholin-2-yl]methyl]-N-methyl-carbamate (120 mg, 0.180 mmol) in DCM (3 mL) was added HCl in dioxane (1.6 mL, 6.4 mmol). The mixture was stirred at 20 °C for 16 h. The mixture was concentrated under reduced pressure. The residue was purified by prep-HPLC to give the title compound (21 mg) as a white solid. MS obsd. (ESI⁺) [(M+H)⁺]: 562.1

### Reference Example 269

### N-(2-(2-(4-hydroxypiperidin-1-yl)ethoxy)ethyl)-4-((3-(4-methoxyphenyl)imidazo[1,2-a]pyrazin-8-yl)amino)-2-methylbenzamide

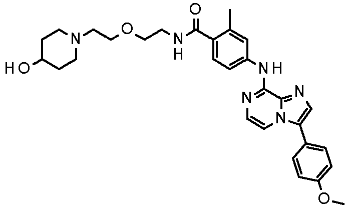

A mixture of N-(2-(2-chloroethoxy)ethyl)-4-((3-(4-methoxyphenyl)imidazo[1,2-a]pyrazin-8-yl)amino)-2-methylbenzamide (Reference Example 54, 30 mg, 62.5 µmol, Eq: 1), piperidin-4-ol (9.5 mg), sodium carbonate (9.94 mg, 93.8 µmol, Eq: 1.50) and potassium iodide (519 µg, 3.13 µmol, Eq: 0.05) in n-BuOH (0.5 mL) was heated at 105 °C for 48 h. Water was added to the reaction mixture and extracted with DCM. The combined organic layers were dried over sodium sulphateand concentrated to an oil. The product was purified by prep. HPLC to give the title compound (19 mg). MS (ESI, m/z): 547.4.

The following examples were prepared in analogy to Reference Example 269, Boc-protected intermediates were deprotected using HCl (4M in dioxane).

### Intermediate 89

### N-(2-(2-chloroethoxy)ethyl)-4-((3-(4-(difluoromethoxy)phenyl)imidazo[1,2-a]pyrazin-8-yl)amino)-2-methylbenzamide

The title compound was prepared in analogy to Reference Example 54 from Intermediate 7 and 2-(2-chloroethoxy)ethanamine hydrochloride. MS (ESI⁺) [(M+H)⁺]: 516.3

The following examples were prepared in analogy to Reference Example 269

| Ex. | Name | Structure | MS ESI [M+H]⁺ | Starting Material |
|---|---|---|---|---|
| REF 270 | 4-((3-(4-methoxyphenyl)imid azo[1,2-a]pyrazin-8-yl)amino)-2-methyl-N-(2-(2-(methylamino)ethox y)ethyl)benzamide | 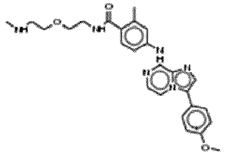 | 475.4 | Reference Example 54 and methanamine |
| REF 271 | N-(2-(2-((2-hydroxyethyl)amino) ethoxy)ethyl)-4-((3-(4-methoxyphenyl)imid azo[1,2-a]pyrazin-8-yl)amino)-2-methylbenzamide | 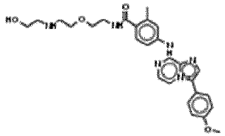 | 505.4 | Reference Example 54 and 2-aminoethanol |
| REF 272 | N-(2-(2-((2,2-difluoroethyl)amino) ethoxy)ethyl)-4-((3-(4-methoxyphenyl)imid azo[1,2-a]pyrazin-8-yl)amino)-2-methylbenzamide | 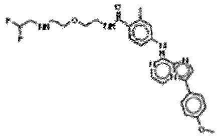 | 525.4 | Reference Example 54 and 2,2-difluoroethanamine |
| REF 273 | N-(2-(2-(2-oxa-6-azaspiro[3.3]heptan-6-yl)ethoxy)ethyl)-4-((3-(4-methoxyphenyl)imid azo[1,2-a]pyrazin-8-yl)amino)-2-methylbenzamide | 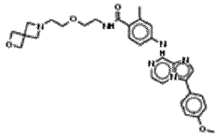 | 543.5 | Reference Example 54 and 2-oxa-6-azaspiro[3.3]heptan e |
| REF 274 | N-(2-(2-(4-fluoropiperidin-1-yl)ethoxy)ethyl)-4-((3-(4-methoxyphenyl)imid azo[1,2-a]pyrazin-8-yl)amino)-2-methylbenzamide | 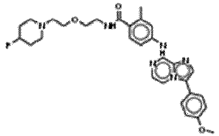 | 547.4 | Reference Example 54 and 4-fluoropiperidine |
| REF 275 | 4-[[3-(4-methoxyphenyl)imid azo[1,2-a]pyrazin-8-yl]amino]-2-methyl-N-[2-(2-piperazin-1-ylethoxy)ethyl]benza mide;dihydrochlorid e | 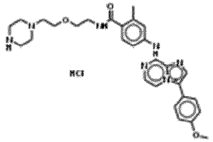 | 530.5 | Reference Example 54 and tert-butyl piperazine-1-carboxylate |
| REF 276 | 4-((3-(4-(difluoromethoxy)ph enyl)imidazo[1,2-a]pyrazin-8-yl)amino)-2-methyl-N-(2-(2-(methylamino)ethox y)ethyl)benzamide | 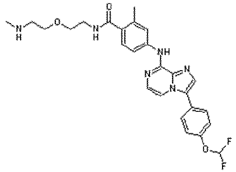 | 511.4 | Intermediate 89 and methylamine |

### Intermediate 90

### 1-(4-amino-2-chloro-benzoyl)piperidine-4-carboxylic acid

### Step 1:

### methyl 1-(4-amino-2-chloro-benzoyl)piperidine-4-carboxylate

To a solution of 4-amino-2-chlorobenzoic acid (1.70 g, 10 mmol), methyl piperidine-4-carboxylate(2.85 g, 20 mmol) in anhydrous DCM (50 mL) was added DIPEA (2.58 g, 20 mmol) and then the resultant mixture was stirred for 30 min at room temperature, HATU (7.6 g, 20 mmol) was added in the mixture and stirred for extra 10 h. The mixture was poured into water (100 mL) and the aqueous solution was extracted with DCM (100 mL×2). The organic layers were combined and washed with water and brine, dried over anhydrous sodium sulphate and concentrated under reduced pressure to give a red oil, which was purified by flash column chromatography to to provide the desired compound (1.82 g, 61.3 % yield) as a white solid. MS (ESI, m/z): 297.1 [M+H]+.

### Step 2:

### 1-(4-amino-2-chloro-benzoyl)piperidine-4-carboxylic acid

To a solution of methyl 1-(4-amino-2-chlorobenzoyl)piperidine-4-carboxylate (890 mg, 3.0 mmol) in THF (5 mL) and methanol (25 mL) was added 2.0 M aq. LiOH (3.0 mL). The resultant mixture was stirred for 15 h at room temperature and then acidified to pH=5-6 with 3.0 M hydrochloric acid. The resulting suspension was filtered, the solid was washed with water and then dried to give the title compound (0.6 g, 70.7% yield) as a white solid. MS (ESI, m/z): 283.0 [M+H]+.

### Intermediate 91

### 1-(4-amino-2-methyl-benzoyl)piperidine-4-carboxylic acid

### Step 1:

### methyl 1-(2-methyl-4-nitro-benzoyl)pipetidine-4-carboxylate

To a solution of 2-methyl-4-nitrobenzoic acid (1.8 g, 10 mmol), methyl piperidine-4-carboxylate(2.85 g, 20 mmol) in anhydrous DCM (50 mL) was added DIPEA (2.58 g,20 mmol) and then the resultant mixture was stirred for 30 min at room temperature, HATU (7.6 g, 20 mmol) was added in the mixture and stirred for extra 10 h. The mixture was poured into water (100 mL) and the aqueous solution was extracted with DCM (100 mL×2). The organic layers were combined and washed with water and brine, dried over anhydrous sodium sulphate and concentrated under reduced pressure to give a red oil, which was purified by flash column chromatography to provide the desired compound (2.86 g, 93.4 % yield) as a yellow solid. MS (ESI, m/z): 307.1 [M+H]+.

### Step 2:

### methyl 1-(4-amino-2-methyl-benzoyl)piperidine-4-carboxylate

To a solution of methyl 1-(2-methyl-4-nitro-benzoyl)piperidine-4-carboxylate (3.0 g, 9.3 mmol) in EtOH (50 mL) was added palladium on carbon (254 mg, 0.1 mol). The mixture was degassed and charged with a H₂ balloon. The reaction was stirred at room temperature overnight. The catalyst was filtered off and the filtrate was concentrated. The residue was purified by column chromatography to give the final compound (1.93 g, 70 % yield) as a red oil. MS (ESI, m/z): 277.1 [M+H]+.

### Step 3:

### 1-(4-amino-2-methyl-benzoyl)piperidine-4-carboxylic acid

To a solution of methyl 1-(4-amino-2-methylbenzoyl)piperidine-4-carboxylate (830 mg, 3.0 mmol) in THF (5 mL) and methanol (25 mL) was added 2.0 M aq LiOH (6.0 mL). The resultant mixture was stirred for 15 h at room temperature and then acidified to pH=5-6 with 3.0 M hydrochloric acid. The resulting suspension was filtered, the solid was washed with water and then dried to give the title compound (0.6 g, 76.2% yield) as a white solid. MS (ESI, m/z): 263.1 [M+H]+.

### Intermediate 92

### 1- [2-chloro-4-[[3-[4-(cyanomethoxy)-2,3-difluoro-phenyl] imidazo [1,2-a] pyrazin-8-yl]amino]benzoyl]piperidine-4-carboxylic acid

To a solution of intermediate 30 (0.96 g, 3.0 mmol) in acetonitrile (30 mL) and acetic acid (3.0 mL) was added intermediate 90 (0.85 g, 3.0 mmol) and then stirred overnight at 95 °C. The mixture was poured into water (50 mL) and the resulting suspension filtered. The solid was washed with acetonitrile and water, dried to give the title compound (1.0 g, 58.8 % yield) as a light red solid which was used in next step without purification. MS (ESI, m/z): 567.1 [M+H]+.

The following intermediates were prepared in analogy to intermediate 92

| Int. | Name | ESI MS [M+H]⁺ | Starting Material |
|---|---|---|---|
| 93 | 1-[2-chloro-4-[[3-[4-(cyanomethoxy)-2,3-difluoro-phenyl]imidazo[1,2-a]pyrazin-8-yl]amino]benzoyl]piperidine-4-carboxylic acid | 583.1 | Intermediate 90 and intermediate 41 |
| 94 | 1-[4-[[3-[4-(cyanomethoxy)-2,3-difluorophenyl]imidazo[1,2-a]pyrazin-8-yl]ammo]-2-methyl-benzoyl]piperidine-4-carboxylic acid | 547.1 | Intermediate 91 and intermediate 30 |
| 95 | 1-[4-[[3-[2-chloro-4-(cyanomethoxy)-3-fluorophenyl]imidazo[1,2-a]pyrazin-8-yl]amino]-2-methyl-benzoyl]piperidine-4-carboxylic acid | 563.1 | Intermediate 90 and intermediate 41 |

### Reference Example 277

### 2- [2,3-difluoro-4- [8- [4- [4- [3-(hydroxymethyl)piperazine-1-carbonyl]piperidine-1-carbonyl]-3-methyl-anilino]imidazo[1,2-a] pyrazin-3-yl] phenoxy]acetonitrile;2,2,2-trifluoroacetic acid

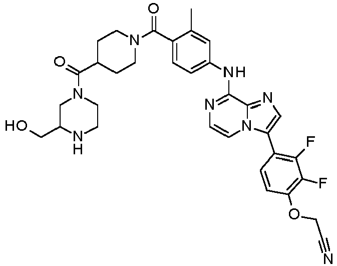

### Step 1:

### tert-butyl 4-[1-[4-[[3-[4-(cyanomethoxy)-2,3-difluoro-phenyl]imidazo[1,2-a]pyrazin-8-yl]amino]-2-methyl-benzoyl]piperidine-4-carbonyl]-2-(hydroxymethyl)piperazine-1-carboxylate

To a solution of intermediate 94 (273 mg, 0.5 mmol), tert-butyl 2-(hydroxymethyl)piperazine-1-carboxylate (130 mg, 0.6 mmol) in anhydrous DMF (10 mL) was added DIPEA (258 mg, 2.0 mmol) and then the resultant mixture was stirred for 30 min at room temperature, T₃P (0.5 mL, 0.75 mmol) was added to the mixture and stirred for extra 10 h. The mixture was poured into water (50 mL) and the aqueous solution was extracted with DCM (50 mL×2). The organic layers were combined and washed with water and brine, dried over anhydrous sodium sulphate and concentrated under reduced pressure to give a red oil which was used in next step without purification. MS (ESI, m/z): 745.3 [M+H]⁺.

**Step 2:**

### 2-[2,3-difluoro-4-[8-[4-[4-[3-(hydroxymethyl)piperazine-1-carbonyl]piperidine-1-carbonyl]-3-methyl-anilino]imidazo[1,2-a] pyrazin-3-yl] phenoxy]acetonitrile;2,2,2-trifluoroacetic acid

To a solution of tert-butyl 4-(1-(4-((3-(4-(cyanomethoxy)-2,3-difluorophenyl)imidazo[1,2-a]pyrazin-8-yl)amino)-2-methylbenzoyl)piperidine-4-carbonyl)-2-(hydroxymethyl)piperazine-1-carboxylate (300 mg, 0.4 mmol) in THF (5 mL) was added 3M hydrochloric acid (2.0 mL) at room temperature. The resultant mixture was stirred for 10 h and then adjusted to pH=7-8 with 2M Na₂CO₃ aqueous solution. The mixture was extracted with DCM (50 mL×2), the combined organic layers were washed with water and brine, dried over anhydrous sodium sulphate, and concentrated to give a red oil which was purified by prep. HPLC to provide the desired compound (215 mg, 79.2 % yield) as an off-white powder. MS (ESI, m/z): 645.2 [M+H]+.

The following examples were prepared in analogy to Reference Example 277

| Int. | Name | ESI MS [M+H]⁺ | Starting Material |
|---|---|---|---|
| REF 278 | piperazin-2-ylmethyl 1-[2-chloro-4-[[3-[2-chloro-4-(cyanomethoxy)-3-fluoro-phenyl]imidazo[1,2-a]pyrazin-8-yl]amino]benzoyl]piperidine-4-carboxylate trifluoroacetate | 681.1 | Intermediate 93 and tert-butyl 3-(hydroxymethyl)pip erazine-1-carboxylate |
| REF 113 | piperazin-2-ylmethyl 1-[2-chloro-4-[[3-[4-(cyanomethoxy)-2,3-difluorophenyl]imidazo[1,2-a]pyrazin-8-yl]amino]benzoyl]piperidine-4-carboxylate trifl uoroacetate | 665.2 | Intermediate 92 and tert-butyl 3-(hydroxymethyl)pip erazine-1-carboxylate |
| REF 279 | 2-[4-[8-[3-chloro-4-[4-[2-(hydroxymethyl)piperazine-1-carbonyl]piperidine-1- carbonyl]anilino]imidazo[1,2-a]pyrazin-3-yl]-2,3-difluoro-phenoxy] acetonitrile trifluoroacetate | 665.2 | Intermediate 92 and tert-butyl 3-(hydroxymethyl)pip erazine-1-carboxylate |
| REF 280 | 2-[3-chloro-2-fluoro-4-[8-[4-[4-[3-(hydroxymethyl)piperazine-1-carbonyl]piperidine-1-carbonyl]-3-methyl-anilino]imidazo[1,2-a]pyrazin-3-yl]phenoxy]acetonitrile trifluoroacetate | 661.2 | Intermediate 95 and tert-butyl 2-(hydroxymethyl)pip erazine-1-carboxylate |
| REF 281 | piperazin-2-ylmethyl 1-[2-chloro-4-[[3-[4-(cyanomethoxy)-2,3-difluorophenyl]imidazo[1,2-a]pyrazin-8-yl]amino]benzoyl]piperidine-4-carboxylate trifluoroacetate | 661.2 | Intermediate 95 and tert-butyl 3-(hydroxymethyl)pip erazine-1-carboxylate |
| REF 282 | 2-[3-chloro-4-[8-[3-chloro-4-[4-[2-(hydroxymethyl)piperazine-1-carbonyl]piperidine-1- carbonyl]anilino]imidazo[1,2-a]pyrazin-3-yl]-2-fluoro-phenoxy]acetonitrile trifluoroacetate | 681.1 | Intermediate 93 and tert-butyl 3-(hydroxymethyl)pip erazine-1-carboxylate |
| REF 277 | 2-[2,3-difluoro-4-[8-[4-[4-[3-(hydroxymethyl)piperazine-1-carbonyl]piperidine-1-carbonyl]-3-methyl-anilino]imidazo[1,2-a]pyrazin-3-yl]phenoxy]acetonitrile trifluoroacetate | 645.2 | Intermediate 94 and tert-butyl 2-(hydroxymethyl)pip erazine-1-carboxylate |
| REF 283 | 1-[2-chloro-4-[[3-[4-(cyanomethoxy)-2,3-difluoro-phenyl]imidazo[1,2-a]pyrazin-8-yl] amino]benzoyl]-N-[2-(methylamino)ethyl]piperidine-4-carboxamide trifluoroacetate | 623.2 | Intermediate 92 and tert-butyl (2-aminoethyl)(methyl )carbamate |
| REF 284 | 1-[4-[[3-[4-(cyanomethoxy)-2,3-difluorophenyl]imidazo[1,2-a]pyrazin-8-yl]amino]-2-methyl-benzoyl]-N-[2-(1H-tetrazol-5-yl)ethyl]piperidine-4-carboxamide trifluoroacetate | 642.2 | Intermediate 94 and 2-(2H-tetrazol-5-yl)ethan-1-amine hydrochloride |
| REF 285 | 1-[4-[[3-[4-(cyanomethoxy)-2,3-difluorophenyl]imidazo[1,2-a]pyrazin-8-yl]amino]-2-methyl-benzoyl]-N-methylsulfonyl-piperidine-4-carboxamide trifluoroacetate | 624.1 | Intermediate 94 and methanesulfonami de |
| REF 286 | N-(2-amino-3-hydroxypropyl)-1-(4-((3-(3-fluoro-4-methoxyphenyl)imidazo[1,2-a]pyrazin-8-yl)amino)-2-methylbenzoyl)piperidine-4-carboxamide hydrochloride | 576.3 | Reference Example 2 and tert-butyl (1-amino-3-hydroxypropan-2-yl)carbamate |

### Intermediate 96

### 2-[4-[8-[3-chloro-4-(piperazine-1-carbonyl)anilino]imidazo[1,2-a]pyrazin-3-yl]-2,3-difluoro-phenoxy] acetonitrile

### Step 1:

### tert-butyl 4-[1-[4-[[3-[4-(cyanomethoxy)-2,3-difluoro-phenyl]imidazo[1,2-a]pyrazin-8-yl]amino]-2-methyl-benzoyl]piperidine-4-carbonyl]-2-(hydroxymethyl)piperazine-1-carboxylate

To a solution of intermediate 29 (1.82 g, 4 mmol), tert-butyl piperazine-1-carboxylate (0.9 g, 4.8 mmol) in anhydrous DMF (35 mL) was added DIPEA (2.6 g, 20 mmol) and then the resultant mixture was stirred for 30 min at room temperature, T₃P (4 mL, 6.4 mmol) was added in the mixture and stirred for extra 10 h. The mixture was poured into water (50 mL) and the aqueous solution was extracted with DCM (50 mL×2). The organic layers were combined and washed with water and brine, dried over anhydrous sodium sulphate and concentrated under reduced pressure to give a red oil which was used in next step without purification. MS (ESI, m/z): 624.1 [M+H]+.

**Step 2:**

### 2-[4-[8-[3-chloro-4-(piperazine-1-carbonyl)anilino]imidazo[1,2-a]pyrazin-3-yl]-2,3-difluoro-phenoxy] acetonitrile

To a solution of tert-butyl 4-[1-[4-[[3-[4-(cyanomethoxy)-2,3-difluoro-phenyl]imidazo[1,2-a]pyrazin-8-yl]amino]-2-methyl-benzoyl]piperidine-4-carbonyl]-2-(hydroxymethyl)piperazine-1-carboxylate (1.8 g, 3 mmol) in THF (15 mL) was added 3M hydrochloric acid aqueous solution (10 mL) at room temperature. The resultant mixture was stirred for 10 h and then adjusted to pH=7-8 with ammonia solution. The mixture was poured into water (25 mL) and then extracted with dichloromethane / isopropanol (100 /10 mL), the organic layer was concentrated to give a red oil, which was purified by prep. HPLC to provide the title compound (1.2 g, 79.4 % yield) as a light red solid. MS (ESI, m/z): 524.1 [M+H]+.

The following intermediates were prepared in analogy to intermediate 96

| Int. | Name | ESI MS [M+H]⁺ | Starting Material |
|---|---|---|---|
| 97 | 2-[3-chloro-4-[8-[3-chloro-4-(piperazine-1-carbonyl)anilino]imidazo[1,2-a]pyrazin-3-yl]-2-fluoro-phenoxy] acetonitrile | 539.1 | Intermediate 24 and tert-butyl piperazine-1-carboxylate |
| 98 | [2-chloro-4-[[3-(2,3-difluoro-4-methoxyphenyl)imidazo[1,2-a]pyrazin-8-yl]amino]phenyl]-piperazin-1-yl-methanone | 499.1 | Intermediate 20 and tert-butyl piperazine-1-carboxylate |
| 76 | (4-((3-iodoimidazo[1,2-a]pyrazin-8-yl)amino)-2-methylphenyl)(piperazin-1-yl)methanone hydrochloride | 463.3 | Intermediate 1 and tert-butylpiperazine-1-carboxylate |

### Reference Example 287

### 2-[3-chloro-4-[8-[3-chloro-4-[4-[(2S,4R)-4-hydroxypyrrolidine-2-carbonyl]piperazine-1-carbonyl]anilino]imidazo[1,2-a]pyrazin-3-yl]-2-fluoro-phenoxy]acetonitrile formate

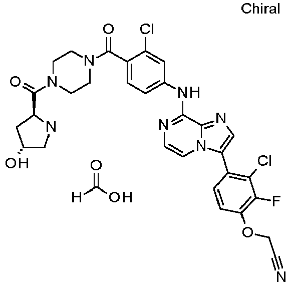

### Step 1:

### tert-butyl (2S,4R)-2-[4-[2-chloro-4-[[3-[2-chloro-4-(cyanomethoxy)-3-fluorophenyl] imidazo [1,2-a]pyrazin-8-yl] amino] benzoyl] piperazine-1-carbonyl]-4-hydroxy-pyrrolidine-1-carboxylate

To a solution of intermediate 97 (162 mg, 0.3 mmol), (2S,4R)-1-(tert-butoxycarbonyl)-4-hydroxypyrrolidine-2-carboxylic acid (83 mg, 0.36 mmol) was added DIPEA (78 mg, 0.6 mmol), the resultant mixture was stirred for 10 min at room temperature, and then HATU (228 mg, 0.6 mmol) was added in the mixture and stirred for extra 10 h at room temperature. The mixture was poured into water (50 mL) and the aqueous solution was extracted with DCM (50 mL×2). The organic layers were combined and washed with water and brine, dried over anhydrous sodium sulphate and concentrated under reduced pressure to give a red oil which was used in next step without purification. MS (ESI, m/z): 753.2 [M+H]+.

### Step 2:

### 2-[3-chloro-4-[8-[3-chloro-4-[4-[(2S,4R)-4-hydroxypyrrolidine-2-carbonyl]piperazine-1-carbonyl]anilino]imidazo[1,2-a]pyrazin-3-yl]-2-fluoro-phenoxy]acetonitrile formate

To a solution of tert-butyl (2S,4R)-2-(4-(2-chloro-4-((3-(2-chloro-4-(cyanomethoxy)-3-fluorophenyl)imidazo[1,2-a]pyrazin-8-yl)amino)benzoyl)piperazine-1-carbonyl)-4-hydroxypyrrolidine-1-carboxylate (200 mg, 0.265 mmol) in THF (5 mL) was added 3M hydrochloric acid aqueous solution (1 mL) at room temperature. The resultant mixture was stirred for 10 h and then adjusted to pH=7-8 with ammonia solution. The mixture was poured into water (25 mL) and then extracted with dichloromethane / isopropanol *(50*/*5* mL), the organic layer was concentrated to give a red oil, which was purified by prep. HPLC to provide the title compound (20 mg, 11.3 % yield) as a white powder. MS (ESI, m/z): 653.2 [M+H]+.

The following examples were prepared in analogy to Reference Example 287

| Ex. | Name | Structure | ESI MS [M+H]+ | Starting Material |
|---|---|---|---|---|
| REF 288 | 2-[4-[8-[3-chloro-4-[4-[(2S,4R)-4-hydroxypyrrolidine-2-carbonyl]piperazine-1-carbonyl]anilino]imidaz o[1,2-a]pyrazin-3-yl]-2,3-difluoro-phenoxy] acetonitrile formate | 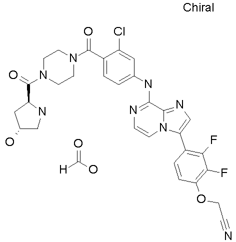 | 637.1 | Intermediate 96 and (2S,4R)-1-(tert-butoxycarbonyl)-4-hydroxypyrrolidine-2-carboxylic acid |
| REF 289 | [2-chloro-4-[[3-(2,3-difluoro-4-methoxyphenyl)imidazo[1,2-a]pyrazin-8-yl]amino]phenyl]-[4-[(2S,4R)-4-hydroxypyrrolidine-2-carbonyl]piperazin-1-yl]methanone formate | 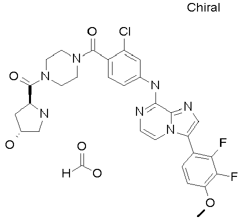 | 612.1 | Intermediate 98 and (2S,4R)-1-(tert-butoxycarbonyl)-4-hydroxypyrrolidine-2-carboxylic acid |

### Reference Example 290

### 2-[4-[8-[3-chloro-4-[4-[(3R)-3-(hydroxymethyl)piperazine-1-carbonyl]piperazine-1-carbonyl] anilino]imidazo[1,2-a] pyrazin-3-yl] -2,3-difluoro-phenoxy] acetonitrile

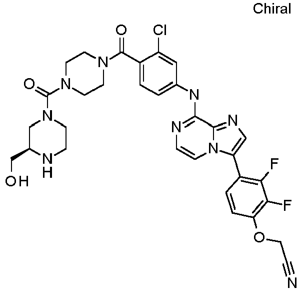

### Step 1:

### tert-butyl (2R)-4- [4- [2-chloro-4- [[3-[4-(cyanomethoxy)-2,3-difluoro-phenyl] imidazo [1,2-a] pyrazin-8-yl] amino] benzoyl]piperazine-1-carbonyl]-2-(hydroxymethyl)piperazine-1-carboxylate

To a solution of intermediate 96 (210 mg, 0.4 mmol), DIPEA (258 mg, 2.0 mmol) in anhydrous DCM (10 mL) was added triphosgene (104 mg, 0.2 mmol) and then the resultant mixture was stirred for 1.0 h at 0°C, and then treated with tert-butyl (R)-2-(hydroxymethyl)piperazine-1-carboxylate (104 mg, 0.48 mmol), the reaction mixture was allowed to warm to room temperature. The mixture was poured into saturated aq. sodium bicarbonate (50 mL) and the aqueous solution was extracted with DCM (50 mL×2). The organic layers were combined and washed with water and brine, dried over anhydrous sodium sulphateand concentrated under reduced pressure to give a red oil which was used in next step without purification. MS (ESI, m/z): 766.2 [M+H]+.

### Step 2:

### 2- [4- [8- [3-chloro-4- [4- [(3R)-3-(hydroxymethyl)piperazine-1-carbonyl]piperazine-1-carbonyl] anilino]imidazo[1,2-a]pyrazin-3-yl] -2,3-difluoro-phenoxy] acetonitrile

To a solution of tert-butyl (2R)-4-[4-[2-chloro-4-[[3-[4-(cyanomethoxy)-2,3-difluorophenyl]imidazo[1,2-a]pyrazin-8-yl]amino]benzoyl]piperazine-1-carbonyl]-2-(hydroxymethyl)piperazine-1-carboxylate (153 mg, 0.2 mmol) in THF (10 mL) was added 3M hydrochloric acid (2 mL) at room temperature. The resultant mixture was stirred for 10 h and then adjusted to pH=7-8 with ammonia solution. The mixture was poured into water (30 mL) and then extracted with dichloromethane / isopropanol (100/10 mL), the organic layer was concentrated to give a red oil, which was purified by prep. HPLC to provide the title compound (18 mg, 13.3 % yield) as a white powder. MS (ESI, m/z): 666.2 [M+H]+.

The following example was prepared in analogy to Reference Example 290

| Ex. | Name | Structure | ESI MS [M+H]+ | Starting Material |
|---|---|---|---|---|
| REF 291 | 2-[4-[8-[3-chloro-4-[4-[(3S)-3-(hydroxymethyl)pipera zine-1-carbonyl]piperazine-1-carbonyl]anilino]imida zo[1,2-a]pyrazin-3-yl]-2,3-difluoro-phenoxy]acetonitrile formate | 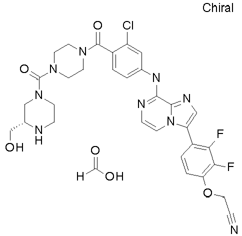 | 666.2 | Intermediate 96 and tert-butyl (S)-2-(hydroxymethyl)pip erazine-1-carboxylate |

### Reference Example 292

### N- [[1-[4-[[3-[4-(cyanomethoxy)-2,3-difluoro-phenyl]imidazo[1,2-a] pyrazin-8-yl] amino] -2-methyl-benzoyl]-4-piperidyl]methyl]-2-(methylamino)acetamide

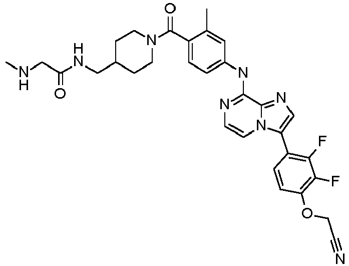

### Step 1:

### tert-butyl N-[2-[[1-[4-[[3-[4-(cyanomethoxy)-2,3-difluoro-phenyl]imidazo[1,2-a]pyrazin-8-yl]amino]-2-methyl-benzoyl]-4-piperidyl]methylamino]-2-oxo-ethyl]-N-methyl-carbamate

To a solution of intermediate REF 139 (106 mg, 0.2 mmol), N-(tert-butoxycarbonyl)-N-methylglycine (57 mg, 0.3 mmol) in anhydrous DMF (10 mL) was added DIPEA (52 mg,0.4 mmol) and then the resultant mixture was stirred for 30 min at room temperature, HATU (152 mg, 0.4 mmol) was added in the mixture and stirred for extra 10 h. The mixture was poured into water (50 mL) and the aqueous solution was extracted with DCM (50 mL×2). The organic layers were combined and washed with water and brine, dried over anhydrous sodium sulphateand concentrated under reduced pressure to give a red oil, which was used in next step without purification. MS (ESI, m/z): 703.2 [M+H]+.

### Step 2:

### N- [[1-[4-[[3-[4-(cyanomethoxy)-2,3-difluoro-phenyl]imidazo[1,2-a]pyrazin-8-yl] amino] -2-methyl-benzoyl]-4-piperidyl]methyl]-2-(methylamino)acetamide

To a solution of tert-butyl (2-(((1-(4-((3-(4-(cyanomethoxy)-2,3-difluorophenyl)imidazo[1,2-a]pyrazin-8-yl)amino)-2-methylbenzoyl)piperidin-4-yl)methyl)amino)-2-oxoethyl)(methyl)carbamate(140 mg, 0.2 mmol) in THF (5 mL) was added 3M aq. hydrochloric acid (2.0 mL) at room temperature. The resultant mixture was stirred for 10 h and then adjusted to pH=7-8 with aq. ammonia.The mixture was poured into water (25 mL) and then extracted with dichloromethane / isopropanol (50/5 mL), the organic layer was concentrated to give a red oil, which was purified by prep. HPLC to provide the title compound (25 mg, 20.3 % yield) as a white powder. MS (ESI, m/z): 603.2 [M+H]⁺.

### Reference Example 293

### N-((1-(4-((3-(4-methoxyphenyl)imidazo[1,2-a]pyrazin-8-yl)amino)-2-methylbenzoyl)piperidin-4-yl)methyl)acetamide

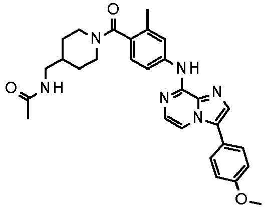

tert-Butyl ((1-(4-((3-(4-methoxyphenyl)imidazo[1,2-a]pyrazin-8-yl)amino)-2-methylbenzoyl)piperidin-4-yl)methyl)carbamate (Intermediate obtained in the preparation of Reference Example 56, 200 mg) was treated with 1.05eq acetyl chloride (0.032 mL) in 5 mL AcOEt/EtOH (9/1) the mixture was stirred overnight at room temperature. A mixture of Reference Example 56 and the title compound was obtained, which was separated by prep. HPLC. White powder (44 mg), MS (ESI, m/z): 513.4.

### Reference Example 294

### (4-((3-(3-chloro-4-methoxyphenyl)imidazo [1,2-a] pyrazin-8-yl)amino)phenyl)(morpholino)methanone

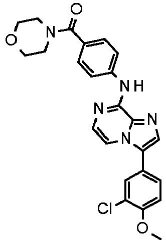

A mixture of (4-aminophenyl)(morpholino)methanone (31 mg), Intermediate 15 (29.4 mg), potassium carbonate (27.6 mg), t-Bu-X-phos (2 mg) and Pd₂(dba)₃ (1 mg) in dioxane was stirred at 100 °C overnight. DMSO was added, the mixture was filtered over Celite and purified by prep. HPLC to give the title compound (9 mg) as a colorless solid.

MS (ESI, m/z): 464.2

The following examples were prepared in analogy:

| Ex. | Name | Structure | MS ESI [M+H]⁺ | Starting material |
|---|---|---|---|---|
| REF 295 | 2-chloro-4-((3-(4-(difluoromethoxy)ph enyl)imidazo[1,2-a]pyrazin-8-yl)amino)-N-methylbenzamide | 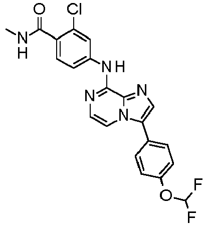 | 444.3 | Intermediate 8 and 4-amino-2-chloro-N-methylbenzamide |
| REF 296 | 2-chloro-4-((3-(4-(difluoromethoxy)ph enyl)imidazo[1,2-a]pyrazin-8-yl)amino)-N-(pyridin-2-ylmethyl)benzamide | 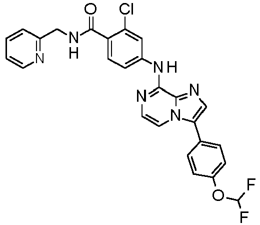 | 521.2 | Intermediate 8 and 4-amino-2-chloro-N-(pyridin-2-ylmethyl)benzamide |

### Reference Example 297

### [4-(aminomethyl)-1-piperidyl]-[4-[[3-(2-chloro-3-fluoro-4-methoxy-phenyl)imidazo[1,2-a] pyrazin-8-yl] amino]-2-methyl-phenyl]methanone hydrochloride

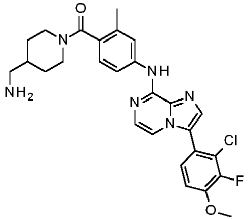

### Step 1: tert-butyl N-[[1-(2-methyl-4-nitro-benzoyl)-4-piperidyl]methyl]carbamate

A mixture of 2-methyl-4-nitro-benzoic acid (1.00 g, 5.52 mmol), HATU (2.52 g, 6.62 mmol) and DIPEA (2.88 mL, 16.56 mmol) in DMF (25 mL) was stirred at 15°C for 0.5h. Then 4-(tert-butoxycarbonylaminomethyl) piperidine (1.42 g, 6.62 mmol) was added and the reaction was stirred at 15 °C for 16 h. The reaction mixture was diluted with H₂O (50 mL) and extracted with ethyl acetate. The organic phase was washed with brine, dried over anhydrous sodium sulphate, concentrated under reduced pressure and purified by flash column chromatography (eluting with DCM/MeOH=50/1) to afford desired compound (2.00 g) as a light yellow solid. MS obsd. (ESI⁺) [(M+Na)⁺]: 400

### Step 2: tert-butyl N-[[1-(4-amino-2-methyl-benzoyl)-4-piperidyl]methyl]carbamate

A mixture oftert-butyl N-[[1-(2-methyl-4-nitro-benzoyl)-4-piperidyl]methyl]carbamate (1.00 g, 2.65 mmol) and palladium on charcoal (100 mg, 10 wt.%) in methanol (10 mL) was stirred at 15 °C under H₂ for 16 h. The catalyst was filtered off and the filtrate was concentrated under reduced pressure to give desired compound (900 mg) as a red oil which was used directly for the next step.

### Step 3: tert-butyl N-[[1-[4-[[3-(2-chloro-3-fluoro-4-methoxy-phenyl)imidazo[1,2-a]pyrazin-8-yl] amino]-2-methyl-benzoyl] -4-piperidyl] methyl] carbamate

A mixture of intermediate 70 (50 mg, 0.16 mmol) and tert-butyl N-[[1-(4-amino-2-methylbenzoyl)-4-piperidyl]methyl]carbamate (100 mg, 0.29 mmol) in acetonitrile (0.9 mL) and acetic acid (0.1 mL) was stirred at 90 °C for 16 h. The mixture was cooled to RT and concentrated under reduced pressure. The residue was purified by prep-TLC (DCM/MeOH=20/1) to afford desired compound (20 mg) as a white oil. MS obsd. (ESI⁺) [(M+H)⁺] : 623.1

### Step 4: [4-(aminomethyl)-1-piperidyl]-[4-[[3-(2-chloro-3-fluoro-4-methoxyphenyl)imidazo[1,2-a]pyrazin-8-yl]amino]-2-methyl-phenyl]methanone hydrochloride

To a stirred solution of tert-butyl N-[[1-[4-[[3-(2-chloro-3-fluoro-4-methoxyphenyl)imidazo[1,2-a]pyrazin-8-yl] amino]-2-methyl-benzoyl]-4-piperidyl]methyl] carbamate (20 mg, 0.03 mmol) in methanol (0.5 mL) was added a solution of HCl in dioxane (0.04 mL 4.0 M) drop wise. The reaction mixture was stirred at 15 °C for 2 h. The reaction mixture was concentrated under reduced pressure and the residue was purified by prep. HPLC to give the title compound (5.8 mg) as a white solid. MS obsd. (ESI⁺) [(M+H)⁺]: 523.2.

### Reference Example 298

### 4-[[3-(3-fluoro-4-methoxy-phenyl)imidazo[1,2-a] pyrazin-8-yl] amino]-N-[2-(1H-imidazol-2-yl)ethyl]-N,2-dimethyl-benzamide

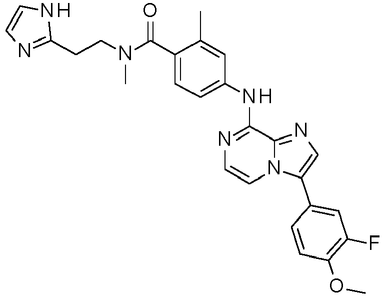

### Step 1: N-(3-hydroxypropyl)-N,2-dimethyl-4-nitro-benzamide

To a solution of 2-methyl-4-nitro-benzoic acid (1.0 g, 5.52 mmol) in DMF (10 mL) was added HATU (2.73 g, 7.18 mmol), 3-(methylamino)propan-1-ol (590 mg, 6.62 mmol) and triethylamine (1.68 g, 16.6 mmol). The mixture was stirred at 25 °C for 12 h and then diluted with ethyl acetate. The resulting mixture was washed with water and brine successively, dried over anhydrous sodium sulphate, concentrated under reduced pressure. The residue was purified by flash column chromatography (eluted with DCM/MeOH=20) to give the title compound (1.2 g) as a light yellow oil.

### Step 2: N,2-dimethyl-4-nitro-N-(3-oxopropyl)benzamide

To a stirred solution of N-(3-hydroxypropyl)-N,2-dimethyl-4-nitro-benzamide (900 mg, 3.57 mmol) and TEMPO (56 mg, 0.36 mmol) in DCM (10 mL) was added PhI(OAc)₂ (1.38 g, 4.28 mmol) slowly. The reaction was stirred at 20 °C for 1 h and then quenched with sat. Na₂SO₃ solution. The resulting mixture was extracted with DCM. The DCM layer was washed with brine, dried over anhydrous sodium sulphate, concentrated under reduced pressure and purified by flash column chromatrography to afford the title compound (460 mg) as a light yellow oil.

### Step 3: N-[2-(1H-imidazol-2-yl)ethyl]-N,2-dimethyl-4-nitro-benzamide

To a stirred solution of N,2-dimethyl-4-nitro-N-(3-oxopropyl)benzamide (450 mg, 1.8 mmol) and ammonium hydroxide (1.76 g, 12.6 mmol) in methanol (5 mL) was added glyoxal (230 mg, 3.96 mmol) slowly. The reaction was stirred at 20 °C for 12 h. The mixture was diluted with H₂O (20 mL) and extracted with DCM (30 mL). The organic phase was washed with brine, dried over anhydrous sodium sulphate, concentrated under reduced pressure to afford the title compound (450 mg) as a light yellow oil.

### Step 4: 4-amino-N-[2-(1H-imidazol-2-yl)ethyl]-N,2-dimethyl-benzamide

Into a stirred solution of N-[2-(1H-imidazol-2-yl)ethyl]-N,2-dimethyl-4-nitro-benzamide (200 mg, 0.69 mmol) in methanol (5 mL) was added Pd on charcoal (74 mg, 10 wt.%). The reaction was stirred under H₂ balloon at 20 °C for 1 h. The catalyst was filtered off and the filtrate was concentrated under reduced pressure to afford the title compound (60 mg) as a light yellow oil which was used directly in next step.

### Step 5: 4-[[3-(3-fluoro-4-methoxy-phenyl)imidazo[1,2-a]pyrazin-8-yl]amino]-N-[2-(1H-imidazol-2-yl)ethyl]-N,2-dimethyl-benzamide

To a solution of Intermediate 3 (60 mg, 0.22 mmol) in tert-butanol (2 mL) was added BrettPhos-Pd-G3 (196 mg, 0.22 mmol, CAS#1470372-59-8), 4-amino-N-[2-(1H-imidazol-2-yl)ethyl]-N,2-dimethyl-benzamide (59 mg, 0.23 mmol), and potassium carbonate (30 mg, 0.22 mmol). The mixture was stirred at 100 °C for 12 h under N₂. After cooled to RT, the reaction mixture was diluted with ethyl acetate (100 mL). The resulting mixture was washed with water and brine successively, dried over anhydrous Na₂SO₄, concentrated under reduced pressure to afford crude product as a yellow oil. It was purified by prep-HPLC to give the title compound (37 mg) as a white solid. MS obsd. (ESI⁺) [(M+H)⁺]: 500.1

### Reference Example 299:

### N-[2-[4-(2,2-difluoroethyl)piperazin-1-yl]ethyl]-4-[[3-(3-fluoro-4-methoxyphenyl)imidazo[1,2-a]pyrazin-8-yl]amino]-N,2-dimethyl-benzamide; formic acid

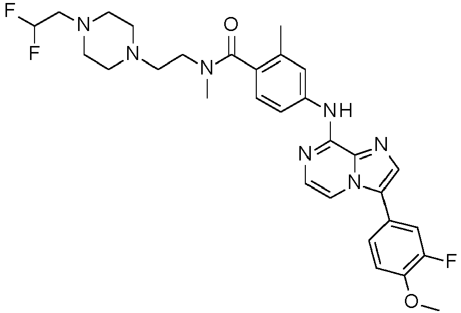

To a solution of REF 300 (50 mg, 0.09 mmol) in DMF (1 mL) was added potassium carbonate (37 mg, 0.27 mmol) and 1,1-difluoro-2-iodoethane (21 mg, 0.11 mmol). The reaction was stirred at 50 °C for 12 h and then was diluted with ethyl acetate. The resulting mixture was washed with water and brine successively, dried over anhydrous Na₂SO₄ and concentrated under reduced pressure to afford crude product as a yellow oil. It was purified by prep-HPLC to give the title compound (11 mg) as a white solid. MS obsd. (ESI⁺) [(M+H)⁺]: 582.

### Reference Example 301:

### N- [2-(1-diethoxyphosphoryl-4-piperidyl)ethyl]-4-[[3-[4-(difluoromethoxy)phenyl]imidazo[1,2-a]pyrazin-8-yl] amino]-N,2-dimethyl-benzamide

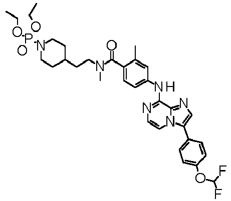

Into a stirred solution of Reference Example 191 (200 mg, 0.35 mmol) and triethylamine (0.15 mL, 1.05 mmol) in DCM (4 mL) was added diethyl chlorophosphate (200 mg, 1.16 mmol) slowly. The reaction was stirred at 15 °C for 2 h. The reaction was quenched with H₂O (5 mL) and extracted with DCM (10 mL × 3). The organic phase was washed with brine (10 mL), dried over anhydrous Na₂SO₄ , concentrated under reduced pressure and purified by prep-HPLC to afford the title compound (81.4 mg) as a white solid. MS obsd. (ESI⁺) [(M+23)⁺] : 671.1.

### Reference Example 302:

### 2-[2-(dimethylamino)ethyl]-6-[[3-(3-fluoro-4-methoxy-phenyl)imidazo[1,2-a]pyrazin-8-yl]amino]-3,4-dihydroisoquinolin-1-one

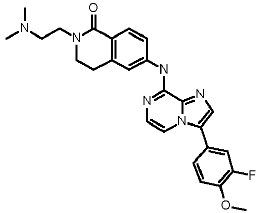

### Step 1: 6-bromo-2-[2-(dimethylamino)ethyl]-3,4-dihydroisoquinolin-1-one

Into a stirred solution of 6-bromo-3, 4-dihydro-2H-isoquinolin-1-one (200 mg, 0.88 mmol) in DMF (5 mL) was added sodium hydride (53 mg, 1.33 mmol, 60 wt%) portion wise at 15 °C. The mixture was stirred for 0.5 h. Then (2-bromoethyl)dimethylamine hydrobromide (309 mg, 1.33 mmol) was added and the reaction was stirred at 15 °C for 16 h. Sat. aq. NH₄Cl was added to quench the reaction. The obtained mixture was diluted with H₂O (10 mL) and extracted with ethyl acetate. The organic phase was washed with brine, dried over anhydrous Na₂SO₄, concentrated under reduced pressure and purified by prep-TLC (DCM/MeOH=20, Rf=0.1) to give the title compound (120 mg) as a light yellow oil.

### Step 2: 3-(3-fluoro-4-methoxy-phenyl)imidazo[1,2-a]pyrazin-8-amine

A mixture of 8-chloro-3-iodo-imidazo[1,2-a]pyrazine (500 mg, 1.8 mmol) and a solution of ammonium hydroxide (10 mL, 17.83 mmol) in 1,4-dioxane (5 mL) was stirred at 100 °C for 16 h in a sealed vessel. The reaction mixture was cooled and concentrated under reduced pressure. The residue was diluted with H₂O (20 mL) and extracted with DCM. The organic phase was dried over anhydrous Na₂SO₄ and concentrated under reduced pressure to give 3-(3-fluoro-4-methoxy-phenyl)imidazo[1,2-a]pyrazin-8-amine (300 mg) as a brown solid.

### Step 3: 2-[2-(dimethylamino)ethyl]-6-[[3-(3-fluoro-4-methoxy-phenyl)imidazo[1,2-a] pyrazin-8-yl] amino]-3,4-dihydroisoquinolin-1-one

A mixture of 3-(3-fluoro-4-methoxy-phenyl)imidazo[1,2-a]pyrazin-8-amine (100 mg, 0.39 mmol), 6-bromo-2-[2-(dimethylamino)ethyl]-3,4-dihydroisoquinolin-1-one (115 mg, 0.39 mmol), cesium carbonate (378 mg, 1.16 mmol), (R)-BINAP (48 mg, 0.08 mmol) and Pd₂(dba)₃ (22 mg, 0.04 mmol) in 1,4-dioxane (3 mL) was stirred under nitrogen at 100 °C for 16 h. The reaction mixture was cooled and filtered. The filtrate was concentrated under reduced pressure and purified by prep-TLC (DCM/MeOH=20, R_{f} = 0.2) to get a crude product. It was re-purified by trituration (CH₃OH, 2 mL) to afford the title compound (17.9 mg) as a white solid. MS obsd. (ESI⁺) [(M+H)⁺] : 475.1.

### Reference Example 303

### 7-[[3-(3-fluoro-4-methoxy-phenyl)imidazo[1,2-a]pyrazin-8-yl]amino]-2,3,4,5-tetrahydro-2-benzazepin-1-one hydrochloride

### Step 1: 6-aminotetralin-1-one oxime

A mixture of 6-amino-1,2,3,4-tetrahydronaphthalen-1-one (1.0 g, 6.2 mmol), hydroxylamine hydrochloride (474 mg, 6.82 mmol), sodium acetate (1.12 g, 13.65 mmol) in ethanol (10 mL) and water (3.3 mL) was stirred at 90 °C for 4 h. The mixture was cooled to RT and diluted with H₂O (20 mL). The precipitate was collected by filtration and washed with water and dried over high vacuum to give 6-aminotetralin-1-one oxime (880 mg) as a white solid.

### Step 2: 7-amino-2,3,4,5-tetrahydro-2-benzazepin-1-one

A mixture of 6-aminotetralin-1-one oxime (880 mg, 4.99 mmol) in PPA (10 mL) was stirred at 120 °C for 2 h. The mixture was cooled to 90 °C and then poured onto ice. The resulting mixture was neutralized with 4N aq. NaOH and extracted with ethyl acetate. The ethyl acetate layer was washed with brine, dried over Na₂SO₄ and concentrated under reduced pressure to give crude compound (850 mg) (mixed of another isomer) as brown solid.

### Step 3: 7- [[3-(3-fluoro-4-methoxy-phenyl)imidazo[1,2-a] pyrazin-8-yl] amino]-2,3,4,5-tetrahydro-2-benzazepin-1-one hydrochloride

A mixture of Intermediate 3 (150 mg, 0.540 mmol), crude 7-amino-2,3,4,5-tetrahydro-2-benzazepin-1-one (105 mg, 0.600 mmol) in acetonitrile (1.8 mL) and acetic acid (0.200 mL) was stirred at 90 °C for 16 h. The mixture was concentrated under reduced pressure and the residue was purified by prep. HPLC to give 7-[[3-(3-fluoro-4-methoxy-phenyl)imidazo[1,2-a]pyrazin-8-yl]amino]-2,3,4,5-tetrahydro-2-benzazepin-1-one (18.7 mg) as a red solid. (ESI⁺) [(M+H)⁺]: 418

### Reference Example 304

### 2-[4-[8-[4-[4-(aminomethyl)piperidine-1-carbonyl]-3-methyl-anilino]imidazo[1,2-a]pyrazin-3-yl]-2,3-difluoro-phenoxy]-2-methyl-propanenitrile;formic acid

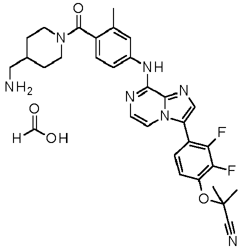

### Step 1:

### Ethyl 2-(4-bromo-2,3-difluoro-phenoxy)-2-methyl-propanoate

4-Bromo-2,3-difluorophenol (6 g, 28.7 mmol), ethyl 2-bromo-2-methylpropanoate (6.72 g, 34.5 mmol), Cs₂CO₃ (9.35 g, 28.7 mmol) and tetrabutylammoniumiodide (530 mg, 1.44 mmol) were suspended in DMF (30 mL). The resulting mixture was heated at 80 °C overnight. Then the mixture was cooled, diluted with water and extracted with ethyl ether. The combined organic phases were dried and concentrated. The residue was purified by flash column chromatography to give the title compound (6 g, 64.7 % yield).

### Step 2:

### 2-(4-bromo-2,3-difluoro-phenoxy)-2-methyl-propanoic acid

Ethyl 2-(4-bromo-2,3-difluorophenoxy)-2-methyl-propanoate (4.3 g, 13.3 mmol) and NaOH (1.06 g, 26.6 mmol) was dissolved in a mixed solution of MeOH (36 mL), THF (18 mL) and water (12 mL). The reaction solution was stirred at room temperature for 2 h. Then the solution was acidified by 12 N HCl aqueous solution to pH 2-3. The water layer was extracted with ethyl acetate, dried over anhydrous MgSO₄ and concentrated to give the title compound as a white solid (3.5 g, 89.1 % yield).

### Step 3:

### 2-(4-bromo-2,3-difluoro-phenoxy)-2-methyl-propanamide

A mixtue of 2-(4-bromo-2,3-difluorophenoxy)-2-methyl-propanoic acid (3.3 g, 11.2 mmol), 1-(3-dimethylaminopropyl)-3-ethylcarbodiimide hydrochloride (2.57 g, 13.4 mmol), 1-hydroxybenzotriazole (2.27 g, 16.8 mmol) and DIPEA (2.17 g, 2.93 mL, 16.8 mmol) in THF (30 mL) was stirred at room temperature for 2 h. Then aq. 25% NH₃ (10 mL) was added. The mixture was stirred overnight and then quenched with water. The aqueous layer was extracted with DCM. The combined organic layers were washed with saturated aq. NaHCO₃, brine, dried and concentrated. The residue was purified by flash column chromatography to give the title compound as a yellow solid (2 g, 60.8 % yield).

### Step 4:

### 2-(4-bromo-2,3-difluoro-phenoxy)-2-methyl-propanenitrile

To a solution of 2-(4-bromo-2,3-difluorophenoxy)-2-methyl-propanamide (2 g, 6.8 mmol) and Et₃N (4.13 g, 5.69 mL, 40.8 mmol) in dichloromethane (40 mL) was added trifluoroacetic anhydride (8.57 g, 5.76 mL, 40.8 mmol) at 0 °C. After the addition, the solution was allowed to reach room temperature and stirred for 2 h. Then the mixture was heated at 70 °C overnight. The reaction was concentrated and diluted with water. The water phase was adjusted to pH 8-9 by NaHCO₃ aqueous solution. The water phase was extracted with DCM, dried and concentrated. The residue was used into next step reaction without further purification.

### Step 5:

### 2-[2,3-difluoro-4-(4,4,5,5-tetramethyl-1,3,2-dioxaborolan-2-yl)phenoxy]-2-methylpropanenitrile

A mixture of 4,4,4',4',5,5,5',5'-octamethyl-2,2'-bi(1,3,2-dioxaborolane) (2.48 g, 9.78 mmol), 2-(4-bromo-2,3-difluorophenoxy)-2-methylpropanenitrile (1.8 g, 6.52 mmol), 1,1'-Bis(diphenylphosphino)ferrocene-palladium(II)dichloride dichloromethane complex (532 mg, 652 µmol) and potassium acetate (1.28 g, 13 mmol) in 1,4-dioxane (20 mL) was stirred at 80 °C overnight. Then the mixture was filtered and then concentrated. The residue was used in the next step reaction directly without further purification.

### Step 6:

### tert-butyl N-[[1-[4-[[3-[4-(1-cyano-1-methyl-ethoxy)-2,3-difluoro-phenyl]imidazo[1,2-a] pyrazin-8-yl] amino]-2-methyl-benzoyl]-4-piperidyl] methyl] carbamate

To a solution of tert-butyl ((1-(4-((3-iodoimidazo[1,2-a]pyrazin-8-yl)amino)-2-methylbenzoyl)piperidin-4-yl)methyl)carbamate (intermediate 73, 600 mg, 1.02 mmol) in water (5 mL) and THF (10 mL) was added 2-(2,3-difluoro-4-(4,4,5,5-tetramethyl-1,3,2-dioxaborolan-2-yl)phenoxy)-2-methylpropanenitrile (the crude product from step 5), 1,1'-Bis(diphenylphosphino)ferrocene-palladium(II) dichloride dichloromethane complex (166 mg, 203 µmol) and potassium phosphate tribasic (647 mg, 616 µl, 3.05 mmol) and then the mixture was degassed for 5 min with nitrogen and then stirred overnight at 70 °C. The mixture was filtered. The solution was concentrated and the water layer was extracted with DCM. The organic layer was concentrated and the residue was purified by prep. HPLC to give the title compound (400 mg). MS (ESI, m/z): 660.3 [M+H]⁺

### Step 7:

### 2-[4-[8-[4-[4-(aminomethyl)piperidine-1-carbonyl]-3-methyl-anilino]imidazo[1,2-a]pyrazin-3-yl]-2,3-difluoro-phenoxy]-2-methyl-propanenitrile formate

tert-butyl ((1-(4-((3-(4-((2-cyanopropan-2-yl)oxy)-2,3-difluorophenyl)imidazo[1,2-a]pyrazin-8-yl)amino)-2-methylbenzoyl)piperidin-4-yl)methyl)carbamate (400 mg, 606 µmol) in TFA (5 mL) and DCM (10 mL) was stirred at room temperature for 2 h. Then the mixture was neutralized by NaHCO₃ aqueous solution. The water layer was extracted with DCM. The organic layer was dried and concentrated. The residue was purified by prep-HPLC to give the title compound as a white powder (120 mg). MS (ESI, m/z): 560.3 [M+H]⁺

### Intermediate 99

### 1-[2,3-Difluoro-4-(4,4,5,5-tetramethyl-1,3,2-dioxaborolan-2-yl)phenoxy] cyclopropanecarbonitrile

### Step 1:

### methyl 4-bromo-2-(4-bromo-2,3-difluoro-phenoxy)butanoate

K₂CO₃ (6.61 g, 47.8 mmol) was added into a solution of 4-bromo-2,3-difluorophenol (5 g, 23.9 mmol) in dry DMF (20 mL). The mixture was stirred at RT for 10 min. To the mixture was added methyl 2,4-dibromobutanoate (6.22 g, 23.9 mmol) dropwise. The resulting mixture was stirred at RT for 3h. The mixture was diluted with ethyl acetate (60 mL), removed inorganic solid by filtration, then washed with water and brine. The organic phase was dried over flash column chromatography and concentrated. The residue was purified by flash column chromatography to give the title compound as an oil (4.7 g, 50 % yield).

### Step 2:

### Methyl 1-(4-bromo-2,3-difluoro-phenoxy)cyclopropanecarboxylate

Methyl 4-bromo-2-(4-bromo-2,3-difluorophenoxy)butanoate (4.7 g, 12.1 mmol) was dissolved in dry THF (30 mL) under N₂ protection, cooled with ice-acetone bath. To the mixture was added solid potassium tert-butoxide (1.36 g, 12.1 mmol) in portions. The resulting mixture was stirred at -10°C for 30 min, then at room temperature for 2 h. The reaction was dried *in vacuo,* the residue was directly purified by flash column chromatography to afford the title compound (2 g, 53.8 % yield).

### Step 3:

### 1- [2,3-difluoro-4-(4,4,5,5-tetramethyl-1,3,2-dioxaborolan-2-yl)phenoxy] cyclopropanecarbonitrile

The title compound was prepared in similar procedures to the step2, step 3, step 4 and step 5 of Reference Example 304 using methyl 1-(4-bromo-2,3-difluoro-phenoxy)cyclopropanecarboxylate as the starting materials.

### Intermediate 100

### 2- [[2,3-difluoro-4-(4,4,5,5-tetramethyl-1,3,2-dioxaborolan-2-yl)phenoxy]methyl]cyclopropanecarbonitrile

### Step 1:

### ethyl 2-(p-tolylsulfonyloxymethyl)cyclopropanecarboxylate

To a solution of ethyl 2-(hydroxymethyl)cyclopropane-1-carboxylate (4 g, 27.7 mmol) in DCM (30 mL) was added Et₃N (5.62 g, 7.73 mL, 55.5 mmol), DMAP (339 mg, 2.77 mmol) and 4-methylbenzenesulfonyl chloride (6.35 g, 33.3 mmol) at 0 °C. The yellow reaction mixture was stirred for 3 hs at room temperature. Then the reaction mixture was poured on aqueous HCl (30 mL) and DCM (30 mL) and the layers were separated. The aqueous layer was extracted with DCM. The organic layer was concentrated and the residue was purified by flash column chromatography to give the title compound as an oil (4.7 g, 56.8 % yield).

### Step 2:

### ethyl 2-[(4-bromo-2,3-difluoro-phenoxy)methyl]cyclopropanecarboxylate

4-bromo-2,3-difluorophenol (9 g, 43.1 mmol), ethyl 2-((tosyloxy)methyl)cyclopropane-1-carboxylate (12.8 g, 43.1 mmol) and Cs₂CO₃ (14 g, 43.1 mmol) was suspended in DMF (40 mL). The resulting mixture was heated at 65 °C overnight. Then the mixture was allowed to cool, diluted with water and extracted with ethyl ether. The combined organic phases were washed with Na₂CO₃ aqueous solution and concentrated. The residue was purified by flash column chromatography to give the title compound as an oil (8.5 g, 58.9% yield).

### Step 3:

### 2- [[2,3-difluoro-4-(4,4,5,5-tetramethyl-1,3,2-dioxaborolan-2-yl)phenoxy]methyl]cyclopropanecarbonitrile

The title compound was prepared in similar procedures to the step2, step 3, step 4 and step 5 of Reference Example 304 using ethyl 2-[(4-bromo-2,3-difluoro-phenoxy)methyl]cyclopropanecarboxylate as the starting materials.

### Intermediate 101

### 2-(3-fluoro-4-methylsulfanyl-phenyl)-4,4,5,5-tetramethyl-1,3,2-dioxaborolane

A mixture of 4,4,4',4',5,5,5',5'-octamethyl-2,2'-bi(1,3,2-dioxaborolane) (2.54 g, 10 mmol), 4-bromo-2-fluoro-1-methylsulfanyl-benzene (2.2 g, 10 mmol), 1,1'-bis(diphenylphosphino)ferrocene-palladium(II)dichloride dichloromethane complex (653 mg, 0.8 mmol) and potassium acetate (1.96 g, 20 mmol) in 1,4-dioxane (20 mL) was stirred at 80 °C overnight. Then the mixture was poured into water and extracted with ethyl acetate. The organic layer was dried and concentrated. The residue was purified by flash column chromatography to afford the title compound as an oil (1.6 g, 61 % yield).

### Reference Example 305

### (4-(aminomethyl)piperidin-1-yl)(4-((3-(2,5-difluoro-4-methoxyphenyl)imidazo[1,2-a] pyrazin-8-yl)amino)-2-methylphenyl)methanone

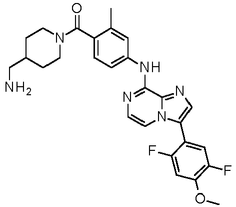

### Step1:

### tert-butyl N-[[1-[4-[[3-(2,5-difluoro-4-methoxy-phenyl)imidazo[1,2-a]pyrazin-8-yl] amino]-2-methyl-benzoyl]-4-piperidyl] methyl] carbamate

To a solution of tert-butyl N-[[1-[4-[(3-iodoimidazo[1,2-a]pyrazin-8-yl)amino]-2-methylbenzoyl]-4-piperidyl]methyl]carbamate (intermediate 73, 200 mg, 339 µmol) in water (2.5mL) and THF (5 mL) was added (2,5-difluoro-4-methoxyphenyl)boronic acid (82.8 mg, 440 µmol), 1,1'-bis(diphenylphosphino)ferrocene-palladium(II)dichloride dichloromethane complex (55.3 mg, 67.7 µmol) and potassium phosphate tribasic (216 mg,1.02 mmol). Then the mixture was degassed for 5 min with nitrogen and then stirred overnight at 80 °C. After cooling to room temperature, the mixture was concentrated and DCM was added. The organic layer was dried over Na₂SO₄ and concentrated *in vacuo.* The residue was used into next step reaction directly. MS (ESI, m/z): 607 [M+H]⁺

**Step2:**

### (4-(aminomethyl)piperidin-1-yl)(4-((3-(2,5-difluoro-4-methoxyphenyl)imidazo[1,2-a] pyrazin-8-yl)amino)-2-methylphenyl)methanone

To a solution of tert-butyl N-[[1-[4-[[3-(2,5-difluoro-4-methoxy-phenyl)imidazo[1,2-a]pyrazin-8-yl]amino]-2-methyl-benzoyl]-4-piperidyl]methyl]carbamate from step 1 in DCM (5 mL) was added CF₃COOH (5 mL). The mixture was stirred for 2h at room temperature. Then the mixture was concentrated and NaHCO₃ aqueous solution was added to neutralize the solution to pH 8-9. The water phase was extracted with DCM. The organic phase was concentrated *in vacuo* and the residue was purified by prep-HPLC to afford the title compound (37 mg) as a solid. MS (ESI, m/z): 507.1 [M+H]⁺

The following examples were prepared in analogy to Reference Example 305:

| Ex. | Name | Structure | MS ESI [M+H]⁺ | Starting Material |
|---|---|---|---|---|
| REF 306 | (4-(aminomethyl)piperi din-1-yl)(4-((3-(5-chloro-2-fluoro-4-methoxyphenyl)imid azo[1,2-a]pyrazin-8-yl)amino)-2-methylphenyl)metha none | 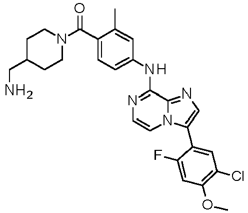 | 523.1 | Intermediate 73 and (5-chloro-2-fluoro-4-methoxyphenyl) boro nic acid |
| REF 307 | (4-(aminomethyl)piperi din-1-yl)(4-((3-(2,4-dichlorophenyl)imid azo[1,2-a]pyrazin-8-yl)amino)-2-methylphenyl)metha none | 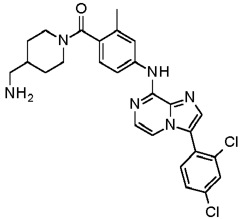 | 509.1 | Intermediate 73 and (2,4-dichlorophenyl)boro nic acid |
| REF 308 | (4-(aminomethyl)piperi din-1-yl)(4-((3-(2-chloro-4-methoxyphenyl)imid azo[1,2-a]pyrazin-8-yl)amino)-2-methy lphenyl)metha none | 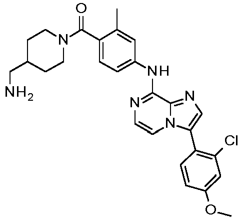 | 505.1 | Intermediate 73 and (2-chloro-4-methoxyphenyl) boro nic acid |
| REF 309 | (4-(aminomethyl)piperi din-1-yl)(4-((3-(3-chloro-4-ethoxy-2-fluorophenyl)imidaz o[1,2-a]pyrazin-8-yl)amino)-2-methylphenyl)metha none formate | 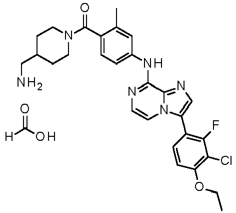 | 537.2 | Intermediate 73 and (3-chloro-4-ethoxy-2-fluorophenyl) boronic acid |
| REF 310 | (4-(aminomethyl)piperi din-1-yl)(2-methyl-4-((3-(3,4,5-trifluorophenyl)imid azo[1,2-a]pyrazin-8-yl)amino)phenyl)met hanone | 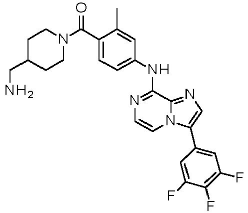 | 495.3 | Intermediate 73 and (3,4,5-trifluorophenyl)boro nic acid |
| REF 311 | (R)-1-(2-chloro-4-((3-(2,3-difluoro-4-methoxyphenyl)imid azo[1,2-a]pyrazin-8-yl)amino)benzoyl)-N-(pyrrolidin-3-yl)piperidine-4-carboxamide hydrochloride | 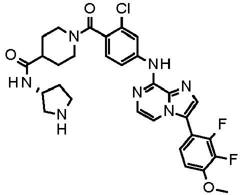 | 611.5 | Intermediate 74 and (2,3-difluoro-4-methoxyphenyl) boro nic acid |
| REF 312 | 1-(4-((3-(3-chloro-4-methoxyphenyl)imid azo[1,2-a]pyrazin-8-yl)amino)-2-methylbenzoyl)-N- (2-(methylamino)ethyl) piperidine-4-carboxamide hydrochloride | 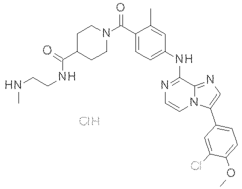 | 576.2 | Intermediate 102 and (3-chloro-4-methoxyphenyl) boro nic acid |
| REF 313 | [4-(aminomethyl)-1-piperidyl]-[4-[[3-(2,3-dichloro-4-methoxyphenyl)imidazo[1,2-a]pyrazin-8-yl]amino]-2-methylphenyl] methanone formate | 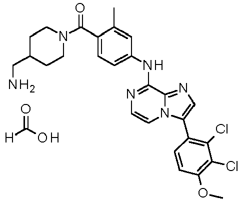 | 539.2 | Intermediate 73 and (2,3-dichloro-4-methoxyphenyl) boro nic acid |
| REF 314 | 2-[[4-[8-[4-[4-(aminomethyl)piperi dine-1-carbonyl]-3- methyl-anilino]imidazo[1,2-a]pyrazin-3-yl]-2,3-difluoro-phenoxy]methyl]cyc lopropanecarbonitril e formate | 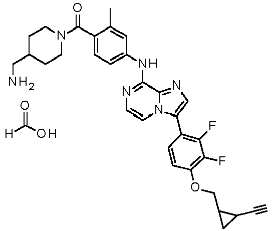 | 572.6 | Intermediate 73 and intermediate 100 |
| REF 315 | 1-[4-[8-[4-[4-(aminomethyl)piperi dine-1-carbonyl]-3- methyl-anilino]imidazo[1,2-a]pyrazin-3-yl]-2,3-difluoro-phenoxy] cyclopropa necarbonitrile formate | 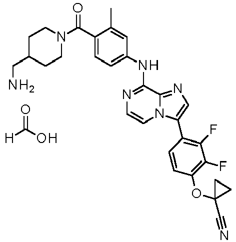 | 558.3 | Intermediate 73 and intermediate 99 |
| REF 316 | [4-(aminomethyl)-1-piperidyl]-[4-[[3-(3-fluoro-4-methylsulfanyl-phenyl)imidazo[1,2-a]pyrazin-8-yl]amino]-2-methyl-phenyl]methanone formate | 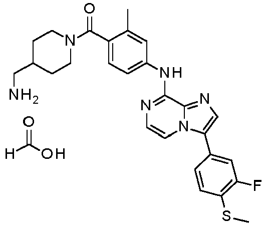 | 505.2 | Intermediate 73 and intermediate 101 |

### Reference Example 317

### [4-(aminomethyl)-1-piperidyl]-[4-[[3-[3-chloro-4-(cyclopropoxy)phenyl]imidazo[1,2-a]pyrazin-8-yl]amino]-2-methyl-phenyl]methanone formate

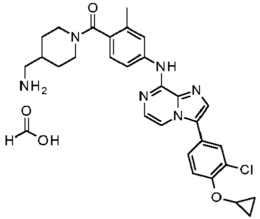

### Step 1:

### 4-bromo-2-chloro-1-(cyclopropoxy)benzene

Bromocyclopropane (8.75 g, 72.3 mmol) was added dropwise over 10 mins to a stirred solution of 4-bromo-2-chlorophenol (3 g, 14.5 mmol) and Cs₂CO₃ (11.8 g, 36.2 mmol) in dimethylacetamide (45 mL). The mixture was heated to 150 °C and stirred at this temperature for 16 h. Then the mixture was poured into water. The water layer was extracted with ethyl acetate. The combined organic layers were dried over anhydrous sodium sulphateand concentrated *in vacuo.* The residue was purified by flash column chromatography to give the title compound (3 g). MS (ESI, m/z): 247 [M+H]⁺

### Step 2:

### 2-[3-chloro-4-(cyclopropoxy)phenyl]-4,4,5,5-tetramethyl-1,3,2-dioxaborolane

Under N₂ atmosphere, a mixture of 4,4,4',4',5,5,5',5'-octamethyl-2,2'-bi(1,3,2-dioxaborolane) (3.69 g, 14.5 mmol), 4-bromo-2-chloro-1-cyclopropoxybenzene (3g, 12.1 mmol), potassium acetate (2.38 g, 24.2 mmol) and 1,1'-bis(diphenylphosphino)ferrocene-palladium(II)dichloride dichloromethane complex (990 mg, 1.21 mmol) in 1,4-dioxane (50 mL) was stirred at 80 °C overnight. After cooling to room temperature, the mixture was concentrated and the residue was dissolved in DCM. The organic phase was washed with water, dried and concentrated. The residue was purified by flash column to give the title compound (2.6 g) as a solid.

### Step 3:

### tert-butyl N-[[1-[4-[[3-[3-chloro-4-(cyclopropoxy)phenyl]imidazo[1,2-a] pyrazin-8-yl] amino]-2-methyl-benzoyl]-4-piperidyl] methyl] carbamate

To a solution of tert-butyl N-[[1-[4-[(3-iodoimidazo[1,2-a]pyrazin-8-yl)amino]-2-methylbenzoyl]-4-piperidyl]methyl]carbamate (300 mg, 508 µmol) in water (2.5 mL) and THF (5 mL) was added 2-(3-chloro-4-cyclopropoxyphenyl)-4,4,5,5-tetramethyl-1,3,2-dioxaborolane (195 mg, 660 µmol), 1,1'-bis(diphenylphosphino)ferrocene-palladium(II)dichloride dichloromethane complex (41.5 mg, 50.8 µmol) and potassium phosphate tribasic (324 mg, 308 µl, 1.52 mmol). Then the mixture was degassed for 5 min with nitrogen and then stirred overnight at 70 °C. After cooling to room temperature, the mixture was conentrated. The water phase was extracted with DCM. The organic phase was dried and concentrated to give the crude product (300 mg). The crude product was used into next step reaction directly without further purification. MS (ESI, m/z): 631 [M+H]⁺

### Step 4:

### (4-(aminomethyl)piperidin-1-yl)(4-((3-(3-chloro-4-cyclopropoxyphenyl)imidazo [1,2-a] pyrazin-8-yl)amino)-2-methylphenyl)methanone formate

A solution of tert-butyl N-[[1-[4-[[3-[3-chloro-4-(cyclopropoxy)phenyl]imidazo[1,2-a]pyrazin-8-yl]amino]-2-methyl-benzoyl]-4-piperidyl]methyl]carbamate (300 mg, 475 µmol) in TFA (5 mL) and DCM (5 mL) was stirred at room temperature for 2 h. Then the solution was concentrated and the residue was diluted with water and DCM. The mixture solution was basified to pH 8-9 with K₂CO₃ aqueous solution. The water layer was extracted with DCM. The combined organic phases were dried and concentraed. The residue was purified by prep-HPLC to give the title compound (14 mg) as a solid. MS (ESI, m/z): 531.2 [M+H]⁺

### Reference Example 318

### (4-(aminomethyl)piperidin-1-yl)(4-((3-(4-(cyclopropylmethoxy)-3-fluorophenyl)imidazo[1,2-a]pyrazin-8-yl)amino)-2-methylphenyl)methanone

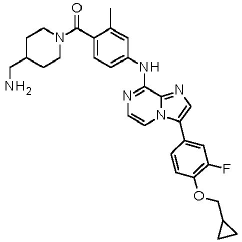

The title compound was obtained in analogy to Reference Example 317 using (bromomethyl)cyclopropane instead of bromocyclopropane and 4-bromo-2-fluoro-phenol instead of 4-bromo-2-chlorophenol. MS (ESI, m/z): 529.3 [M+H]⁺

### Reference Example 319

### 2-[4-[8-[4-[4-(aminomethyl)piperidine-1-carbonyl]-3-methyl-anilino]imidazo[1,2-a]pyrazin-3-yl]-2,3-difluoro-phenoxy]propanenitrile;2,2,2-trifluoroacetic acid

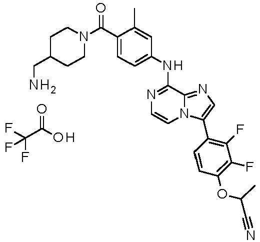

The title compound was obtained in analogy to Reference Example 317 using 2-bromopropanenitrile instead of bromocyclopropane and 4-bromo-2,3-difluoro-phenol instead of 4-bromo-2-chlorophenol. MS (ESI, m/z): 546.5 [M+H]⁺

### Reference Example 320

### 4-(4-(8-((4-(4-(aminomethyl)piperidine-1-carbonyl)-3-methylphenyl)amino)imidazo[1,2-a] pyrazin-3-yl)-2,3-difluorophenoxy)butanenitrile

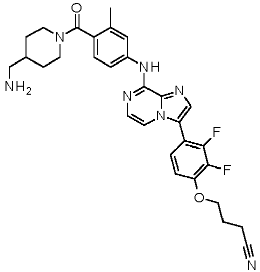

The title compound was obtained in analogy to Reference Example 317 using 4-bromobutanenitrile instead of bromocyclopropane and 4-bromo-2,3-difluoro-phenol instead of 4-bromo-2-chlorophenol. MS (ESI, m/z): 560.4 [M+H]⁺

### Reference Example 321

### [4-(aminomethyl)-1-piperidyl]-[4-[[3-(2,3-difluoro-4-prop-2-ynoxy-phenyl)imidazo[1,2-a]pyrazin-8-yl]amino]-2-methyl-phenyl]methanone;formic acid

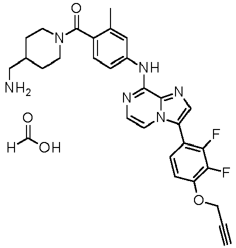

The title compound was obtained in analogy to Reference Example 317 using 3-bromoprop-1-yne instead of bromocyclopropane and 4-bromo-2,3-difluoro-phenol instead of 4-bromo-2-chlorophenol. MS (ESI, m/z): 531.1 [M+H]⁺

### Reference Example 322

### [4-(aminomethyl)-1-piperidyl]-[4-[[3-[2,3-difluoro-4-(2-pyridyloxy)phenyl] imidazo [1,2-a]pyrazin-8-yl]amino]-2-methyl-phenyl]methanone formate

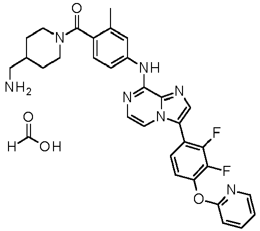

### Step 1:

### 2-(4-bromo-2,3-difluoro-phenoxy)pyridine

A mixture of 4-bromo-2,3-difluorophenol (3.5 g, 16.7 mmol), 2-fluoropyridine (2.44 g, 25.1 mmol) and K₂CO₃ (5.79 g, 41.9 mmol) in DMSO (20 mL) was heated at 120 °C for 3 days. Then the mixture was poured into water and extracted with DCM. The organic layer was dried and concentrated. The residue was purified by flash column chromatography to give the title compound as an oil (700 mg, 14.6 % yield).

### Step 2:

### 2-[2,3-difluoro-4-(4,4,5,5-tetramethyl-1,3,2-dioxaborolan-2-yl)phenoxy]pyridine

Under N₂, a mixture of 4,4,4',4',5,5,5',5'-octamethyl-2,2'-bi(1,3,2-dioxaborolane) (746 mg, 2.94 mmol), 2-(4-bromo-2,3-difluorophenoxy)pyridine (0.7 g, 2.45 mmol), potassium acetate (480 mg, 4.89 mmol) and 1,1'-bis(diphenylphosphino)ferrocene-palladium(II)dichloride dichloromethane complex (200 mg, 245 µmol) in 1,4-dioxane (10 mL) was stirred at 80 °C overnight. The reaction solution was filtered and concentrated. The residue was used into next step reaction directly without further purification.

### Step 3:

### tert-butyl N- [[1-[4-[[3- [2,3-difluoro-4-(2-pyridyloxy)phenyl] imidazo [1,2-a] pyrazin-8-yl] amino]-2-methyl-benzoyl]-4-piperidyl] methyl] carbamate

A mixture of 2-(2,3-difluoro-4-(4,4,5,5-tetramethyl-1,3,2-dioxaborolan-2-yl)phenoxy)pyridine (the crude product from step 2), tert-butyl ((1-(4-((3-iodoimidazo[1,2-a]pyrazin-8-yl)amino)-2-methylbenzoyl)piperidin-4-yl)methyl)carbamate (intermediate 73, 400 mg, 677 µmol), potassium phosphate (288 mg, 1.35 mmol) and 1,1'-bis(diphenylphosphino)ferrocene-palladium(II)dichloride dichloromethane complex (55.3 mg, 67.7 µmol) in 1,4-dioxane (10 mL) and water (5 mL) was heated at 95 °C for 1 h in a microwave tube. Then the mixture was concentrated and the water layer was extracted with DCM. The organic layer was dried and concentrated. The residue was used into next step reaction directly without further purification.

### Step 4:

### [4-(aminomethyl)-1-piperidyl]-[4-[[3-[2,3-difluoro-4-(2-pyridyloxy)phenyl] imidazo [1,2-a]pyrazin-8-yl]amino]-2-methyl-phenyl]methanone;formic acid

tert-butyl ((1-(4-((3-(2,3-difluoro-4-(pyridin-2-yloxy)phenyl)imidazo[1,2-a]pyrazin-8-yl)amino)-2-methylbenzoyl)piperidin-4-yl)methyl)carbamate (the crude product from step 3) was dissolved in DCM (5 mL) and TFA (5 mL). The solution was stirred for 1 h. Then the solution was concentrated and the residue was dissolved in DCM. Water (10 mL) was added. The solution was alkalized by addition of K₂CO₃ to pH 8-9. The water phase was extracted with DCM. The organic phase was concentrated and the residue was purified by prep-HPLC to give the title compound. MS (ESI, m/z):570.2 [M+H]⁺

### Reference Example 323

### [4-(aminomethyl)-1-piperidyl]-[4-[[3-[2,3-difluoro-4-(4-pyridyloxy)phenyl] imidazo [1,2-a] pyrazin-8-yl] amino]-2-methyl-phenyl] methanone

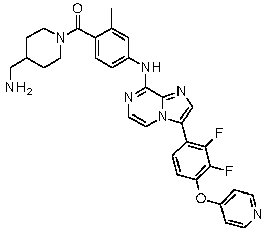

### Step 1:

### 4-(4-bromo-2,3-difluoro-phenoxy)pyridine

4-bromo-2,3-difluorophenol (3.0 g, 14.4 mmol) in DMA (20 mL) was added potassium tert-butoxide (3.22 g, 28.7 mmol) at 0 °C. The colorless solution was stirred for 1 h at room temperature. Then 4-fluoropyridine hydrochloride (1.92 g, 14.4 mmol) was added. The organic solution was heated at 100 °C overnight. The mixture was poured into water. The water layer was extracted with ethyl acetate. The combined organic layers were washed with saturated NaCl aqueous solution and concentrated. The residue was purified by flash column to give the title compound as an oil (3.3 g, 80% yield).

### Step 2:

### [4-(aminomethyl)-1-piperidyl]-[4-[[3-[2,3-difluoro-4-(4-pyridyloxy)phenyl] imidazo [1,2-a] pyrazin-8-yl] amino]-2-methyl-phenyl] methanone

The title compound was obtained in similar procedures to step 2, step 3 and step 4 of Reference Example 322 using 4-(4-bromo-2,3-difluoro-phenoxy)pyridine as the starting material. MS (ESI, m/z): 570.2 [M+H]⁺

### Reference Example 324

### [4-(aminomethyl)-1-piperidyl]-[4-[[3-[2,3-difluoro-4-(3-pyridylmethoxy)phenyl]imidazo[1,2-a]pyrazin-8-yl]amino]-2-methyl-phenyl]methanone formate

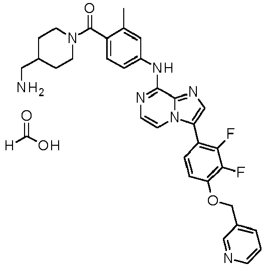

### Step 1: 3-[(4-bromo-2,3-difluoro-phenoxy)methyl]pyridine

4-bromo-2,3-difluorophenol (2.09 g, 10 mmol), 3-(chloromethyl)pyridine (1.64 g, 10 mmol), Cs₂CO₃ (3.25 g, 10 mmol) and tetrabutylammoniumiodide (185 mg, 0.5 mmol) was suspended in DMF (15 mL). The resulting mixture was heated to 60 °C overnight. Then the mixture was cooled, diluted with water and extracted with ethyl ether. The combined organic phases were dried and concentrated. The residue was purified by flash column chromatography to give the title compound as a yellow solid (1.6 g, 53% yield).

### Step 2:

### 3-[[2,3-difluoro-4-(4,4,5,5-tetramethyl-1,3,2-dioxaborolan-2-yl)phenoxy]methyl]pyridine

Under N₂, a mixture of 4,4,4',4',5,5,5',5'-octamethyl-2,2'-bi(1,3,2-dioxaborolane) (1.36 g, 5.3 mmol), 3-[(4-bromo-2,3-difluoro-phenoxy)methyl]pyridine (1.6 g, 5.3 mmol), potassium acetate (1.04 g, 10.6 mmol) and 1,1'-bis(diphenylphosphino)ferrocene-palladium(II)dichloride dichloromethane complex (346 mg, 0.424 mmol) in 1,4-dioxane (10 mL) was stirred at 80 °C overnight. The reaction solution was cooled and poured into water. The water phase was extracted with ethyl acetate. The organic phase was concentrated and purified by flash column chromatography to give the title compound as a solid (0.86 g).

### Step 3:

### [4-(aminomethyl)-1-piperidyl]-[4-[[3-[2,3-difluoro-4-(3-pyridylmethoxy)phenyl]imidazo[1,2-a]pyrazin-8-yl]amino]-2-methyl-phenyl]methanone formate

The title compound was prepared in analogy to Reference Example 305 using intermediate 73 and 3-[[2,3-difluoro-4-(4,4,5,5-tetramethyl-1,3,2-dioxaborolan-2-yl)phenoxy]methyl]pyridine as the starting materials. MS (ESI, m/z): 584.2 [M+H]⁺

### Reference Example 325

### [4-(aminomethyl)-1-piperidyl]-[4-[[3-[2,3-difluoro-4-(2-pyridylmethoxy)phenyl]imidazo[1,2-a]pyrazin-8-yl] amino]-2-methylphenyl]methanone;formic acid

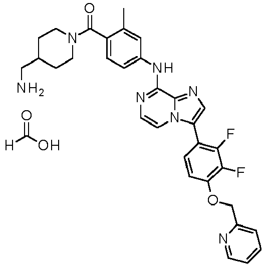

The title compound was obtained in analogy to Reference Example 324 using 2-(chloromethyl)pyridine instead of 3-(chloromethyl)pyridine. MS (ESI, m/z): 584.3 [M+H]⁺

### Reference Example 326

### [4-(aminomethyl)-1-piperidyl]-[4-[[3-(4-benzyloxy-2,3-difluoro-phenyl)imidazo[1,2-a]pyrazin-8-yl]amino]-2-methyl-phenyl]methanone formate

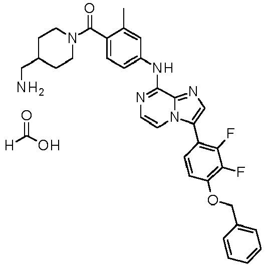

The title compound was obtained in analogy to Reference Example 324 using chloromethylbenzene instead of 3-(chloromethyl)pyridine. MS (ESI, m/z): 583.2 [M+H]⁺

### Reference Example 327

### [4-(aminomethyl)-1-piperidyl]-[4-[[3-[2,3-difluoro-4-(4-pyridylmethoxy)phenyl]imidazo[1,2-a]pyrazin-8-yl]amino]-2-methyl-phenyl]methanone formate

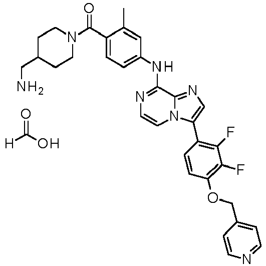

The title compound was obtained in analogy to Reference Example 324 using 4-(chloromethyl)pyridine instead of 3-(chloromethyl)pyridine. MS (ESI, m/z): 584.3 [M+H]⁺

### Reference Example 328

### [4- [[3-[4-(difluoromethoxy)phenyl] imidazo[1,2-a] pyrazin-8-yl] amino]-2-methyl-phenyl] - [4-(4-methylpiperazine-1-carbonyl)piperazin-1-yl]methanone formate

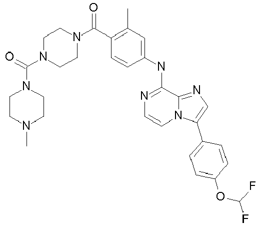

To a solution of [4-[[3-[4-(difluoromethoxy)phenyl]imidazo[1,2-a]pyrazin-8-yl]amino] -2-methyl-phenyl]-piperazin-1-yl-methanone (Reference Example 329) (100.0 mg, 0.210 mmol, 1 eq.) in ACN (3 mL) was added N,N-diisopropylethylamine (0.11 mL, 0.630 mmol, 3 eq.) and N.N'-carbonyldiimidazole (37.28 mg, 0.230 mmol, 1.1 eq.), then the reaction was stirred at 20 °C for 3 h. 1-methylpiperazine (41.87 mg, 0.420 mmol, 2 eq) was added and then stirred at 80 °C for 12 h. After concentration, NMP (3 mL) was added and then stirred at 120 °C for 12 h. The reaction mixture was purified by prep-HPLC to give product [4-[[3-[4-(difluoromethoxy)phenyl]imidazo[1, 2-a]pyrazin-8-yl]amino]-2-methyl-phenyl]-[4-(4-methylpiperazine-1-carbonyl)piperazin-1-yl] methanone formate (25.1 mg, 0.040 mmol, 18% yield) as yellow solid.

LC-MS: [M+H]⁺: 605.2

### Reference Example 329

### [4- [[3-[4-(difluoromethoxy)phenyl] imidazo[1,2-a]pyrazin-8-yl]amino]-2-methyl-phenyl]-piperazin-1-yl-methanone hydrochloride

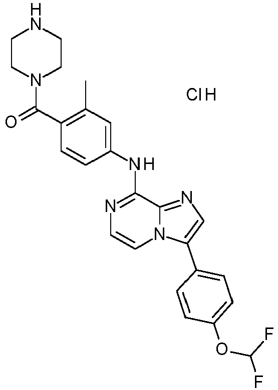

### Step 1: tert-butyl 4-[4-[[3-[4-(difluoromethoxy)phenyl]imidazo[1,2-a]pyrazin-8-yl]amino]-2-methyl-benzoyl] piperazine-1-carboxylate

To a solution of 4-[[3-[4-(difluoromethoxy)phenyl]imidazo[1,2-a]pyrazin-8-yl]amino]-2-methyl-benzoic acid (Intermediate 7) (2.4 g, 5.84 mmol, 1 eq.) in DMF (20 mL) was added1-BOC-piperazine (1.64 g, 8.78 mmol, 1.5 eq.), N,N-diisopropylethylamine (3.06 mL, 17.54 mmol, 3 eq.) and HATU (4.44 g, 11.7 mmol, 2 eq.), then the reaction was stirred at 25 °C for 12 h. 80 mL of water were added and extracted with ethyl acetate (3×100 mL). The combined organic layers were washed with brine (3x80 mL), dried over Na₂SO₄, filtered and concentrated. 40 mL of MTBE was added to the residue and stirred for 1 h. The suspension was filtered and dried to give tert-butyl 4-[4-[[3-[4-(difluoromethoxy)phenyl]imidazo[1,2-a]pyrazin-8-yl]amino]-2-methyl-benzoyl]piperazine-1-carboxylate (3.4 g, 5.88 mmol, 90 % yield) as yellow solid.

LC-MS: [M+H]⁺: 579.3

### Step 2) Reference Example 329

### [4-[[3-[4-(difluoromethoxy)phenyl]imidazo[1,2-a]pyrazin-8-yl]amino]-2-methyl-phenyl]-piperazin-1-yl-methanone;hydrochloride

In a 150 mL round-bottomed flask, tert-butyl 4-(4-((3-(4-(difluoromethoxy)phenyl)imidazo[1,2-a]pyrazin-8-yl)amino)-2-methylbenzoyl)piperazine-1-carboxylate (Step 1) (3.087 g, 5.18 mmol, Eq: 1) was combined with dioxane (25 mL) to give a light brown suspension. Heating, sonicating and addition of 1.0 mL MeOH were necessary to get a proper solution. Then hydrogen chloride (4M solution in dioxane) (12.9 mL, 51.8 mmol, Eq: 10) was added slowly. Again 5 mL dioxane were added and the reaction mixture was stirred overnight. Diethylether was added, the suspension sonicated in an ultra sonic bath, filtered and washed with diethylether and dried in high vacuum, leading to the target compound as an off-white solid (2.7 g, yield: 100%). LC-MS: [M+H]⁺: 479.3

The following Examples and Intermediates were prepared in analogy to Reference Example 329:

| Ex. | Name | Structure | MS ESI [M+H]⁺ | Starting Material |
|---|---|---|---|---|
| REF 330 | [4-[[3-(3-fluoro-4-methoxyphenyl)imidazo[1,2-a]pyrazin-8-yl]amino]-2-methylphenyl]-piperazin-1-yl-methanone hydrochloride | 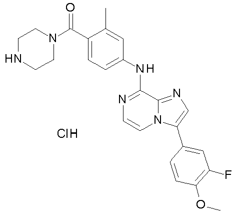 | 461.1 | Intermediate 6. After Step 1, purification by flash chromatography (silica gel, 50g, 0% to 100% DCM/MeOH/NH4OH (95/5/1) |
| REF 331 | N-(2-aminoethyl)-4-[[3-(2,3-difluoro-4-methoxyphenyl)imidazo[1,2-a]pyrazin-8-yl]amino]-2-ethyl-benzamide;2,2,2-trifluoroacetic acid | 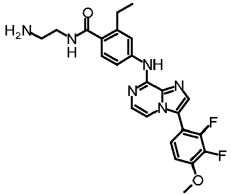 | 467.3 | Intermediate 86 and tert-butyl (2-aminoethyl)carbamate |
| REF 332 | N-(3-aminopropyl)-4-[[3-(2,3-difluoro-4-methoxyphenyl)imidazo[1,2-a]pyrazin-8-yl]amino]-2-ethyl-benzamide;2,2,2-trifluoroacetic acid | 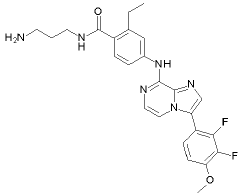 | 481.4 | Intermediate 86 and tert-butyl (3-aminopropyl)carbamate |
| REF 333 | N-(3-aminopropyl)-4-[[3-(2,3-difluoro-4-methoxyphenyl)imidazo[1,2-a]pyrazin-8-yl]amino]-2-ethyl-benzamide hydrochloride | 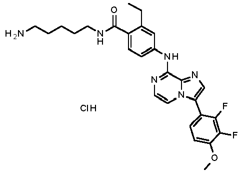 | 507.5 | Intermediate 86 and tert-butyl (5-aminopentyl)carbamate |
| 103 | [4-[[3-(2-chloro-3-fluoro-4-methoxyphenyl)imidazo[1,2-a]pyrazin-8-yl]amino]-2-methylphenyl]-piperazin-1-yl-methanone hydrochloride | 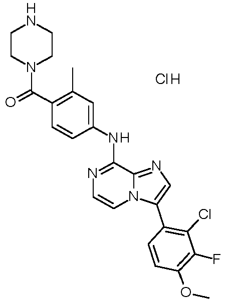 | 495.1 | Intermediate 34 |
| 104 | 4-[[3-(2,3-difluoro-4-methoxyphenyl)imidazo[1,2-a]pyrazin-8-yl]amino]-2-ethyl-N-[(2R)-2-aminopropyl]benza mide | 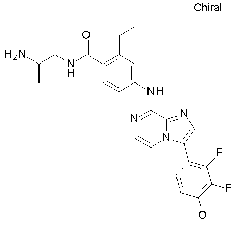 | 481.3 | Intermediate 86 and tert-butyl N-[(1R)-2-amino-1-methylethyl] carbamate |
| 105 | 4-[[3-(2,3-difluoro-4-methoxyphenyl)imidazo[1,2-a]pyrazin-8-yl]amino]-2-ethyl-N-[(2S)-2-aminopropyl]benza mide | 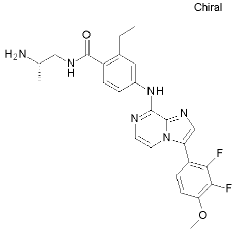 | 481.3 | Intermediate 86 and tert-butyl N-[(1S)-2-amino-1-methylethyl] carbamate |

### Intermediate 86

### 4-[[3-(2,3-difluoro-4-methoxy-phenyl)imidazo[1,2-a]pyrazin-8-yl] amino] -2-ethyl-benzoic acid

### Step 1) Methyl 4-((3-(2,3-difluoro-4-methoxyphenyl)imidazo[1,2-a]pyrazin-8-yl)amino)-2-ethylbenzoate

Under Ar, methyl 2-ethyl-4-((3-iodoimidazo[1,2-a]pyrazin-8-yl)amino)benzoate (Intermediate 42) (2 g, 4.36 mmol, Eq: 1) and (2,3-difluoro-4-methoxyphenyl)boronic acid [CAS#170981-41-6] (860 mg, 4.58 mmol, Eq: 1.05) were combined in dioxane (30 mL). A solution containing Na₂CO₃ [CAS#497-19-8] (1.02 g, 9.59 mmol, Eq: 2.2) in water (3 mL) was added and the off white suspension was degased with Ar. [1,1'-bis(diphenylphosphino)ferrocene]dichloropalladium(II), complex with dichloromethane [CAS#95464-05-4] (53.4 mg, 65.4 µmol, Eq: 0.015) was added and the orange suspension was stirred at 110 °C overnight. At RT, the suspension was filtered. The vessel and the filter cake were washed with ethyl acetate. Isolute was charged into the black suspension. The solvent was evaporated and the crude material was purified by flash chromatography (silica gel, 100 g, 0% to 50% ethyl acetate then 0% to 50% DCM/MeOH/25% aq.NH₃ (95:5:1) in DCM). The target compound was obtained as a light yellow solid (1.53 g, yield: 80%). LC-MS (ESP): m/z=439.3 [M+H]⁺.

### Step 2) Intermediate 86

### 4-[[3-(2,3-difluoro-4-methoxy-phenyl)imidazo[1,2-a]pyrazin-8-yl] amino] -2-ethyl-benzoic acid

Under Ar, methyl 4-((3-(2,3-difluoro-4-methoxyphenyl)imidazo[1,2-a]pyrazin-8-yl)amino)-2-ethylbenzoate (Step 1) (0.765 g, 1.74 mmol, Eq: 1) was suspended in ethanol (9.79 mL). 1M LiOH solution (3.59 mL, 3.59 mmol, Eq: 2.06) was added and the reaction mixture was stirred at 80°C overnight. The solvent was evaporated and the residue was partitioned between water (pH=12 with 1M NaOH) and ethyl acetate. The aqueous phase was acidified with 1M HCl to pH = 1. The resulting off-white suspension was filtered and the cake was washed with water, leading to the target compound as an off-white solid (574 mg, yield:78%). LC-MS (ESP): m/z=525.3 [M+H]⁺.

The following intermediates were prepared in analogy to Intermediate 86:

| Int. | Name | MS ESI [M+H]⁺ | Starting Material |
|---|---|---|---|
| 106 | 2-ethyl-4-((3-(3-fluoro-4-methoxyphenyl)imidazo[1,2-a]pyrazin-8-yl)amino)benzoic acid | 407.3 | Intermediate 42 and (3-fluoro-4-methoxyphenyl) boroni c acid |
| 85 | 4-((3-(4-(difluoromethoxy)-2,3-difluorophenyl)imidazo[1,2-a]pyrazin-8-yl)amino)-2-ethylbenzoic acid | 461.3 | Intermediate 42 and 107 |
| 108 | 4-((3-(2,3-difluoro-4-methoxyphenyl)imidazo[1,2-a]pyrazin-8-yl)amino)-2-methylbenzoic acid | 411.3 | Intermediate 49 and (2,3-difluoro-4-methoxyphenyl) boroni c acid |

### Intermediate 109

### 2- [2,3-difluoro-4- [8- [3-methyl-4-(piperazine-1-carbonyl)anilino]imidazo [1,2-a]pyrazin-3-yl] phenoxy] acetonitrile

### Step 1) tert-butyl 4-(4-((3-iodoimidazo[1,2-a]pyrazin-8-yl)amino)-2-methylbenzoyl)piperazine-1-carboxylate:

In a 50 mL round-bottomed flask, 4-((3-iodoimidazo[1,2-a]pyrazin-8-yl)amino)-2-methylbenzoic acid (Intermediate 1) (1.880 g, 4.15 mmol, Eq: 1), tert-butyl piperazine-1-carboxylate [CAS#57260-71-6] (1.16 g, 6.22 mmol, Eq: 1.5) and HATU (3.15 g, 8.29 mmol, Eq: 2.0) were combined with DMF (20 mL) (fresh bottle) to give a skin colored emulsion. The reaction mixture was sonicated to break some of the remaining solids. The reaction mixture was stirred at room temperature and DIPEA (2.68 g, 3.62 mL, 20.7 mmol, Eq: 5.0) was added. Vigorous stirring at room temperature was continued for 2 h and then DMF was mostly evaporated in high vacuum at 50 °C. The dark brown oil was diluted with DCM/MeOH (9:1) and charged with Isolute. Volatile solvents were evaporated in vacuum, remaining DMF was distilled off in HV at 50 °C. The crude material was purified by flash chromatography (silica gel, 120 g, 0% to 100% DCM/MeOH/25% aq. NH₃ (95/5/1), solid loading), leading to tert-butyl 4-(4-((3-iodoimidazo[1,2-a]pyrazin-8-yl)amino)-2-methylbenzoyl)piperazine-1-carboxylate (2.449 g, 4.14 mmol, 99.7 % yield) as a white solid. LC-MS (ESP): m/z=563.1 [M+H]⁺.

### Step 2) tert-butyl 4-(4-((3-(4-(cyanomethoxy)-2,3-difluorophenyl)imidazo[1,2-a]pyrazin-8-yl)amino)-2-methylbenzoyl)piperazine-1-carboxylate:

In a 100 mL four-necked flask, tert-butyl 4-(4-((3-iodoimidazo[1,2-a]pyrazin-8-yl)amino)-2-methylbenzoyl)piperazine-1-carboxylate (obtained in Step 1) (1 g, 1.69 mmol, Eq: 1) was combined with 2-(2,3-difluoro-4-(4,4,5,5-tetramethyl-1,3,2-dioxaborolan-2-yl)phenoxy)acetonitrile (523 mg, 1.77 mmol, Eq: 1.05), sodium carbonate (394 mg, 3.72 mmol, Eq: 2.2) and dioxane (15 mL). The resulting suspension was stirred and sparged with argon for two minutes. Water (1.5 mL) was added and [1,1'-bis(diphenylphosphino)ferrocene]dichloropalladium(II), complex with dichloromethane [CAS#95464-05-4] (20.7 mg, 25.3 µmol, Eq: 0.015) was added thereafter. The reaction mixture was refluxed for 48 hrs under argon atmosphere. The reaction mixture was diluted with ethyl acetate, filtered and the vessel as well as the filter cake were washed with plenty ethyl acetate and the obtained black solution was concentrated in vacuum. The crude material was purified by flash chromatography (silica gel, 40 g, 40% ethyl acetate in heptane isocratic directly followed by 0%-50% DCM/MeOH/NH3 (95/5/1), solid loading). The title compound tert-butyl 4-(4-((3-(4-(cyanomethoxy)-2,3-difluorophenyl)imidazo[1,2-a]pyrazin-8-yl)amino)-2-methylbenzoyl)piperazine-1-carboxylate (716 mg, 1.16 mmol, 68.8 % yield) was obtained as a brown waxy solid. LC-MS (ESP): m/z=604.3 [M+H]+.

### Step 3) 2- [2,3-difluoro-4- [8- [3-methyl-4-(piperazine-1-carbonyl)anilino]imidazo[1,2-a] pyrazin-3-yl] phenoxy] acetonitrile:

In a 50 mL round-bottomed flask, tert-butyl 4-(4-((3-(4-(cyanomethoxy)-2,3-difluorophenyl)imidazo[1,2-a]pyrazin-8-yl)amino)-2-methylbenzoyl)piperazine-1-carboxylate (obtained in Step 2) (716 mg, 1.19 mmol, Eq: 1) was combined with DCM (10 mL) to give a brown solution. TFA (1.48 g, 1 mL, 13 mmol, Eq: 10.9) was added and the reaction mixture was stirred at RT for 6 h and quenched with 5 mL of saturated aqueous sodium bicarbonate solution and 5 mL of water. Phases were separated and the separation funnel was washed with DCM/MeOH (9:1) to dissolve the precipitate. The organic phases were combined, dried with MgSO₄ monohydrate and filtered. The resulting light brown solution was evaporated *in vacuo.* The crude material was purified by flash chromatography (silica gel, 40 g, 0% to 100% DCM/MeOH/25% aq. NH₃ (90/10/1), solid loading) leading to 2-(2,3-difluoro-4-(8-((3-methyl-4-(piperazine-1-carbonyl)phenyl)amino)imidazo[1,2-a]pyrazin-3-yl)phenoxy)acetonitrile (417 mg, 812 µmol, 68.4 % yield) as an off-white solid. LC-MS (ESP) m/z=504.2 [M+H]+.

The following intermediates were prepared in analogy to Intermediate 109:

| Int. | Name | MS ESI [M+H ]⁺ | Starting Material |
|---|---|---|---|
| 110 | [4-[[3-(2,3-difluoro-4-methoxyphenyl)imidazo[1,2-a]pyrazin-8-yl]amino]-2-ethyl-phenyl]-piperazin-1-yl-methanone | 493.1 | Intermediate 63 and (2,3-difluoro-4-methoxyphenyl) boronic acid (Step 2) |
| 111 | 2-[3-chloro-2-fluoro-4-[8-[3-methyl-4-(piperazine-1-carbonyl)anilino]imidazo[1,2-a]pyrazin-3-yl]phenoxy]acetonitrile | 520.2 | Intermediate 1 and 2-[3-chloro-2-fluoro-4-(4,4,5,5-tetramethyl-1,3,2-dioxaborolan-2-yl)phenyl] acetonitrile |
| 112 | 2-[4-[8-[3-ethyl-4-(piperazine-1-carbonyl)anilino]imidazo[1,2-a]pyrazin-3-yl]-2,3-difluoro-phenoxy]acetonitrile | 518.3 | Intermediate 63 and 2-[2,3-difluoro-4-(4,4,5,5-tetramethyl-1,3,2-dioxaborolan-2-yl)phenoxy] acetonitrile (Step 2) |

### Intermediate 113

### tert-butyl N-[2-[[2-ethyl-4-[(3-iodoimidazo[1,2-a] pyrazin-8 yl)amino] benzoyl] amino]ethyl] carbamate

A mixture of 2-ethyl-4-((3-iodoimidazo[1,2-a]pyrazin-8-yl)amino)benzoic acid hydrochloride (Intermediate 63) (4.45 g, 0.01 mol, Eq: 1), HATU (5.7 g, 15 mmol, Eq: 1.5) and DIPEA (6.46 g, 8.73 mL, 50 mmol, Eq: 5) in DMF (40 mL) was stirred for 15 min. at rt. Then tert-butyl (2-aminoethyl)carbamate (2.45 g, 2.41 mL, 15 mmol, Eq: 1.5) was added and the resulting solution was stirred at RT for 1 1/2h. The reaction mixture was concentrated to dryness. To the liquid was added 100mL H₂O and extracted with ethyl acetate. The organic layer was washed with brine, dried over MgSO₄ and evaporated to dryness. The crude product (7.48g) was purified over 100g SiO₂ 60 in DCM/DCM:MeOH 9:1 (0-100%) by flash chromatography. The obtained material (4.5 g) was triturated with 10 mL Et₂O. The mixture was stirred for 1/2 h, filtered, the solid washed with Et₂O and dried, yielding 3.57 g of the title compound as off-white solid (yield: 65 %). MS (ESP) m/z=551.2 [M+H]+.

### Intermediate 102

### tert-butyl (2-(1-(4-((3-iodoimidazo[1,2-a]pyrazin-8-yl)amino)-2-methylbenzoyl)piperidine-4-carboxamido)ethyl)(methyl)carbamate

The title compound was prepared in analogy to Intermediate 113 from Intermediate 68. LC/MS: [M+H]⁺= 662.3

The following intermediate was prepared in analogy to Intermediate 113:

| Int. | Name | MS ESI [M+H]⁺ | Starting Material |
|---|---|---|---|
| 114 | tert-butyl N-[2-[[4-[(3-iodoimidazo[1,2-a]pyrazin-8-yl)amino]-2-methylbenzoyl]amino]ethyl]carbamate | 479.4 | Intermediate 1 and N-BOC-ethylenediamine |
| 115 | tert-butyl N-[3-[[4-[(3-iodoimidazo[1,2-a]pyrazin-8-yl)amino]-2-methylbenzoyl]amino]propyl]carbamate | 551.0 | Intermediate 1 and N-BOC-1,3-diaminopropane |

### Intermediate 116

### N-(2-aminoethyl)-4-[[3-[4-(cyanomethoxy)-2,3-difluoro-phenyl]imidazo[1,2-a]pyrazin-8-yl] amino]-2-ethyl-benzamide

### Step 1)

### tert-butyl N-[2-[[4-[[3-[4-(cyanomethoxy)-2,3-difluoro-phenyl]imidazo[1,2-a]pyrazin-8-yl] amino]-2-ethyl-benzoyl] amino] ethyl] carbamate

To a solution of tert-butyl N-[2-[[2-ethyl-4-[(3-iodoimidazo[1,2-a]pyrazin-8-yl)amino]benzoyl]amino]ethyl]carbamate (Intermediate 113) (0.5 g, 0.910 mmol, 1 eq) in water (2 mL)/1,4-dioxane (20 mL) was added 2-[2,3-difluoro-4-(4,4,5,5-tetramethyl-1,3,2-dioxaborolan-2-yl)phenoxy]acetonitrile (0.8 g, 2.73 mmol, 3 eq), 1,1'-bis(diphenylphosphino)ferrocene-palladium(II)dichloride dichloromethane complex (0.04 g, 0.050 mmol, 0.050 eq) and sodium carbonate (0.19 g, 1.82 mmol, 2 eq) at 25 °C, the mixture was stirred at 80 °C for 12 h. The mixture was poured into water (50 mL), and extracted with DCM (50 mL x3), the combined organic phases were washed with brine (50 mL x 3), dried over anhydrous Na₂SO₄, and concentrated, the crude product was purified by flash column (PE:EA:DCM=1:1:1) to give tert-butyl N-[2-[[4-[[3-[4-(cyanomethoxy)-2,3-difluorophenyl]imidazo[1,2-a]pyrazin-8-yl]amino]-2-ethyl-benzoyl]amino]ethyl]carbamate (400 mg, 0.680 mmol, 74.43 % yield) (PE:Ethyl acetate=1:1, Rf=0.1) as yellow solid. LC/MS: [M+H]⁺=592.3

### Step 2) Intermediate 116:

### N-(2-aminoethyl)-4-[[3-[4-(cyanomethoxy)-2,3-difluoro-phenyl]imidazo[1,2-a]pyrazin-8-yl] amino]-2-ethyl-benzamide

To a solution of tert-butyl N-[2-[[4-[[3-[4-(cyanomethoxy)-2,3-difluoro-phenyl]imidazo[1,2-a]pyrazin-8-yl]amino]-2-ethyl-benzoyl]amino]ethyl]carbamate obtained in step 1 (200.0 mg, 0.340 mmol, 1 eq) in DCM (20 mL) was addedtrifluoroacetic acid (2.0 mL, 25.96 mmol, 76.79 eq) at 0 °C, the mixture was stirred at 20 °C for 2 h. The mixture was concentrated to give N-(2-aminoethyl)-4-[[3-[4-(cyanomethoxy)-2,3-difluoro-phenyl]imidazo[1,2-a]pyrazin-8-yl]amino]-2-ethyl-benzamide (160 mg, 0.330 mmol, 96.3 % yield) as a brown gum. LC/MS: [M+H]⁺=492.3

The following intermediates were prepared in analogy to Intermediate 116:

| Int. | Name | MS ESI [M+H]⁺ | Starting Material |
|---|---|---|---|
| 117 | N-(2-aminoethyl)-4-[[3-(2-chloro-3-fluoro-4-methoxyphenyl)imidazo[1,2-a]pyrazin-8-yl] amino]-2-ethyl-benzamide | 483.2 | Intermediate 113 and 2-(2-chloro-3-fluoro-4-methoxy-phenyl)-4,4,5,5-tetramethyl-1,3,2-dioxaborolane |
| 118 | N-(2-aminoethyl)-4-[[3-[2-chloro-4-(cyanomethoxy)-3- fluorophenyl]imidazo[1,2-a]pyrazin-8-yl]amino]-2-ethyl-benzamide | 508.2 | Intermediate 113 and Intermediate 55: 2-[3-chloro-2-fluoro-4-(4,4,5,5-tetramethyl-1,3,2-dioxaborolan-2-yl)phenoxy] acetonitril e |
| 119 | N-(2-aminoethyl)-4-[[3-(4-chloro-2,3-difluorophenyl)imidazo[1,2-a]pyrazin-8-yl]amino]-2-ethyl-benzamide | 471.2 | Intermediate 113 and 2-(4-chloro-2,3-difluoro-phenyl)-4,4,5,5-tetramethyl-1,3,2-dioxaborolane |
| 120 | N-(2-aminoethyl)-4-[[3-[4-(difluoromethoxy)-2,3-difluorophenyl]imidazo[1,2-a]pyrazin-8-yl]amino]-2-ethyl-benzamide | 503.2 | Intermediate 113 and Intermediate 107 |
| 121 | N-(2-aminoethyl)-4-[[3-[4-(cyanomethoxy)-2,3-difluorophenyl]imidazo[1,2-a]pyrazin-8-yl]amino]-2-methylbenzamide | 478.2 | Intermediate 114 and 2-[2,3-difluoro-4-(4,4,5,5-tetramethyl-1,3,2-dioxaborolan-2-yl)phenoxy] acetonitril e |
| 122 | N-(3-aminopropyl)-4-[[3-[4-(cyanomethoxy)-2,3-difluorophenyl]imidazo[1,2-a]pyrazin-8-yl]amino]-2-methylbenzamide | 492.2 | Intermediate 115 and 2-[2,3-difluoro-4-(4,4,5,5-tetramethyl-1,3,2-dioxaborolan-2-yl)phenoxy]acetonitrile |

### Intermediate 123

### (2-chloro-4-((3-(3-fluoro-4-methoxyphenyl)imidazo[1,2-a]pyrazin-8-yl)amino)phenyl)(piperazin-1-yl)methanone hydrochloride

The title compound was prepared in analogy to Intermediate REF 17 from Intermediate 2 and tert-butyl piperazine-1-carboxylate.

MS obsd. (ESI⁻) [(M-H)]⁻: 479.4

### Reference Example 334

### N-(2-aminoethyl)-4-[4-[[3-[4-(difluoromethoxy)phenyl]imidazo[1,2-a]pyrazin-8-yl]amino]-2-methyl-benzoyl] piperazine-1-carboxamide hydrochloride

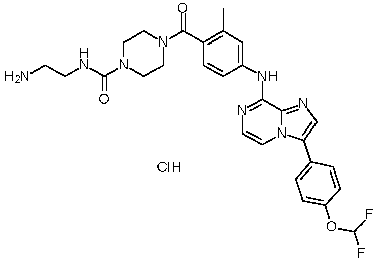

### Step 1)

To a solution of [4-[[3-[4-(difluoromethoxy)phenyl]imidazo[1,2-a]pyrazin-8-yl] amino]-2-methyl-phenyl]-piperazin-1-yl-methanone (Reference Example 329) (100.0 mg, 0.210 mmol, 1 eq) in DCM (5 mL) was added N,N-diisopropylethylamine (0.18 mL, 1.04 mmol, 5 eq) and bis(trichloromethyl)carbonate (24.81 mg, 0.080 mmol, 0.400 eq) at 0 °C, the mixture was stirred at 0 °C for 1 h, then N-BOC-ethylenediamine (100.45 mg, 0.630 mmol, 3 eq) was added, the mixture was stirred at 25 °C for 12 h, LC-MS showed the reaction was completed.

The mixture was concentrated and purified by prep-HPLC (TFA) to give tert-butyl N-[2-[[4-[4-[[3-[4-(difluoromethoxy)phenyl]imidazo[1,2-a]pyrazin-8-yl]amino]-2-methyl-ben zoyl]piperazine-1-carbonyl]amino]ethyl]carbamate (80 mg, 0.120 mmol, 57.59 % yield) as a yellow solid.

LC-MS: [M+H]⁺: 665.3

### Step 2)

To a solution of tert-butyl N-[2-[[4-[4-[[3-[4-(difluoromethoxy)phenyl]imidazo [1,2-a]pyrazin-8-yl]amino]-2-methyl-benzoyl]piperazine-1-carbonyl]amino]ethyl]carbamate (obtained in step 1) (80.0 mg, 0.120 mmol, 1 eq) in methanol (20 mL) was added hydrochloric acid in MeOH (0.77 mL, 3.1 mmol, 25.72 eq) and then stirred at 25 °C for 2 h. LC-MS showed the reaction was complete. After concentration, 100 mL of saturated NaHCO₃ aqueous were added and extracted with DCM (100 mL × 3). The combined organic layers were washed with brine (100 mL), dried over Na₂SO₄, filtered and concentrated. The residue was purified by prep-HPLC (HCl) to give N-(2-aminoethyl)-4-[4-[[3-[4-(difluoromethoxy)phenyl]imidazo[1,2-a]pyrazin-8-yl]amino]-2-methyl-benzoyl]piperazine-1-carboxamide hydrochloride (28 mg, 0.040 mmol, 36.24 % yield) as yellow solid.

LC-MS: [M+H]⁺: 565.1

The following examples were prepared in analogy to Reference Example 334 (Step 2 only required if protecting group needs removal):

| Ex. | Name | Structure | MS ESI [M+H ]⁺ | Starting Material |
|---|---|---|---|---|
| REF 335 | [4-[[3-[4-(difluoromethoxy)ph enyl]imidazo[1,2-a]pyrazin-8-yl]amino]-2-methylphenyl]-[4-[2-[(dimethylamino)me thyl]morpholine-4-carbonyl]piperazin-1-yl]methanone | 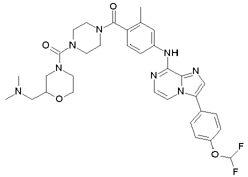 | 649.3 | Reference Example 329 and N,N-dimethyl-2-morpholinmethanamine |
| REF 336 | [4-[[3-[4-(difluoromethoxy)ph enyl]imidazo[1,2-a]pyrazin-8-yl]amino]-2-methylphenyl]-[4-[3-(hydroxymethyl)pipe razine-1-carbonyl] piperazin-1-yl]methanone hydrochloride | 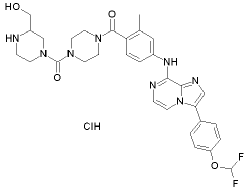 | 621.3 | Reference Example 329 and tert-butyl 2-(hydroxymethyl)piperaz ine-1-carboxylate |
| REF 337 | [4-[2-(aminomethyl)morp holine-4-carbonyl]piperazin-1-yl]-[4-[[3-[4-(difluoromethoxy)ph enyl]imidazo[1,2-a]pyrazin-8-yl]amino]-2-methylphenyl]methanone hydrochloride | 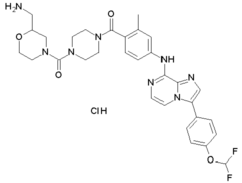 | 621.2 | Reference Example 329 and tert-butyl N-(morpholin- 2-ylmethyl)carbamate |
| REF 338 | 4-[4-[[3-[4-(difluoromethoxy)ph enyl]imidazo[1,2-a]pyrazin-8-yl]amino]-2-methylbenzoyl]-N,N-dimethyl-piperazine-1-carboxamide | 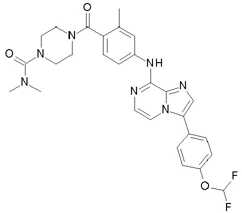 | 550.2 | Reference Example 329 and dimethylamine |
| REF 339 | [4-[[3-[4-(difluoromethoxy)ph enyl]imidazo[1,2-a]pyrazin-8-yl]amino]-2-methylphenyl]-[4-[2-(methylaminomethyl )morpholine-4-carbonyl]piperazin-1-yl]methanone hydrochloride | 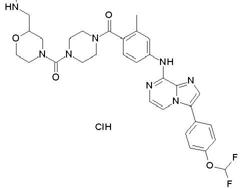 | 635.1 | Reference Example 329 and Carbamic acid, methyl(2-morpholinylmethyl)-, 1, 1-dimethylethyl ester [CAS# 185692-04-0] |
| REF 340 | 2-[2,3-difluoro-4-[8-[3-methyl-4-[4-(piperazine-1-carbonyl)piperazine-1-carbonyl] anilino]imi dazo[1,2-a]pyrazin-3-yl]phenoxy] acetonitr ile formate | 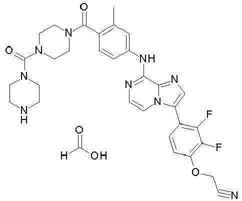 | 616.2 | Intermediate 109 and tert-butyl piperazine-1-carboxylate [CAS#57260-71-6]. Purification: prep HPLC with formic acid |
| REF 341 | 4-[4-[[3-[4-(difluoromethoxy)ph enyl]imidazo[1,2-a]pyrazin-8-yl]amino]-2-methylbenzoyl]-N-[(3S)-pyrrolidin-3-yl]piperazine-1-carboxamide hydrochloride | 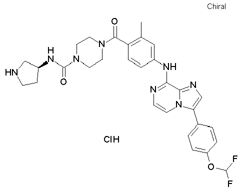 | 591.1 | Reference Example 329 and (S)-3-amino-1-N-BOC-pyrrolidine |
| | | | | |
| REF 342 | N-(2-aminoethyl)-4-[4-[[3-[4-(cyanomethoxy)-2,3-difluorophenyl]imidazo[1,2-a]pyrazin-8-yl] amino]-2-methylbenzoyl] piperazine-1-carboxamide formate | 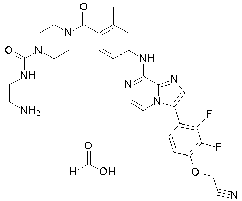 | 590.2 | Intermediate 109 and N-BOC-ethylenediamine Purification: prep HPLC with formic acid |
| REF 343 | [4-[(2R)-2-(aminomethyl)morp holine-4-carbonyl] piperazin-1-yl]-[4-[[3-(2,3-difluoro-4-methoxyphenyl)imidazo[1,2-a]pyrazin-8-yl] amino] -2-methylphenyl] methanone formate | 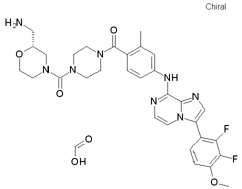 | 621.2 | Reference Example 232 and tert-butyl N-[[(2S)-morpholin-2-yl]methyl] carbamate Purification: prep HPLC with formic acid |
| REF 344 | 4-[4-[[3-[4-(difluoromethoxy)ph enyl]imidazo[1,2-a]pyrazin-8-yl]amino]-2-methylbenzoyl]-N-[(3R)-pyrrolidin-3-yl]piperazine-1-carboxamide hydrochloride | 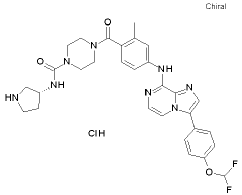 | 591.3 | Reference Example 329 and (R)-(+)-1-BOC-3-aminopyrrolidine |
| REF 345 | 4-[4-[[3-[4-(difluoromethoxy)ph enyl]imidazo[1,2-a]pyrazin-8-yl] amino] -2-methylbenzoyl]-N-(4-piperidyl)piperazine-1-carboxamide hydrochloride | 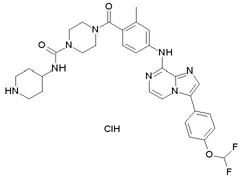 | 605.3 | Reference Example 329 and 4-amino-1-boc-piperidine |
| REF 346 | N-(azetidin-3-ylmethyl)-4-[4-[[3-[4-(difluoromethoxy)ph enyl]imidazo[1,2-a]pyrazin-8-yl]amino]-2-methylbenzoyl] piperazine-1-carboxamide formate | 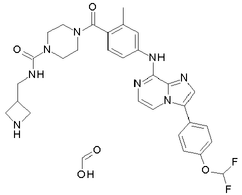 | 591.1 | Reference Example 329 and 1-BOC-3-(aminomethyl) azetidine. Purification: prep HPLC with formic acid |
| REF 347 | [4-[(2S)-2-(aminomethyl)morp holine-4-carbonyl]piperazin-1-yl]-[4-[[3-(2,3-difluoro-4-methoxyphenyl)imidazo[1,2-a]pyrazin-8-yl]amino]-2-methylphenyl] methanone formate | 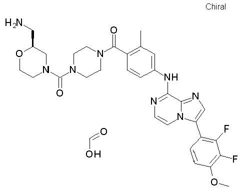 | 621.3 | Reference Example 232 and 1,1-Dimethylethyl [(2R)-2-morpholinylmethyl] carb amate [CAS# 186202-57-3]. Purification: prep HPLC with formic acid |
| REF 348 | [4-[(2S)-2-(aminomethyl)morp holine-4-carbonyl] piperazin-1-yl]-[4-[[3-[4-(difluoromethoxy)phenyl]imidazo[1,2-a]pyrazin-8-yl]amino]-2-methylphenyl]methanone formate | 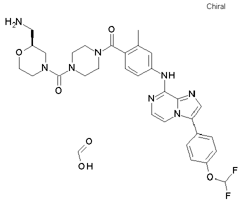 | 621.3 | Reference Example 329 and 1,1-Dimethylethyl [(2R)-2-morpholinylmethyl] carb amate [CAS# 186202-57-3]. Purification: prep HPLC with formic acid |
| REF 349 | N-[(1S)-2-amino-1-methyl-ethyl]-4-[4-[[3-(2,3-difluoro-4-methoxyphenyl)imidazo[1,2-a]pyrazin-8-yl]amino]-2-methylbenzoyl]piperazine-1-carboxamide hydrochloride | 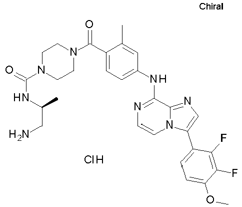 | [M-H]-: 577.6 | Reference Example 232 and tert-butyl (S)-(2-aminopropyl)carbamate [CAS#121103-15-9]. Deprotection with 4M HCl in dioxane. |
| REF 350 | N-[(1R)-2-amino-1-methyl-ethyl]-4-[4-[[3-(2,3-difluoro-4-methoxyphenyl)imidazo[1,2-a]pyrazin-8-yl]amino]-2-methyl-benzoyl]piperazine-1-carboxamide hydrochloride | 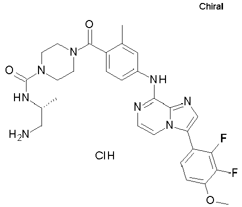 | 579.3 | Reference Example 232 and tert-butyl (R)-(2-aminopropyl)carbamate [CAS#333743-54-7]. Deprotection with 4M HCl in dioxane. |
| REF 351 | N-[(2S)-2-aminopropyl]-4-[4-[[3-(2,3-difluoro-4-methoxyphenyl)imidazo[1,2-a]pyrazin-8-yl]amino]-2-methyl-benzoyl]piperazine-1-carboxamide | 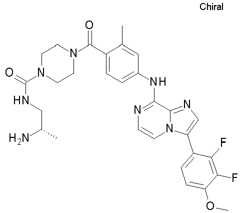 | 579.3 | Reference Example 232 and tert-butyl (S)-(1-aminopropan-2-yl)carbamate [CAS#146552-71-8]. Deprotection with 4M HCl in dioxane. |
| REF 352 | N-[(2R)-2-aminopropyl]-4-[4-[[3-[4-(difluoromethoxy)ph enyl]imidazo[1,2-a]pyrazin-8-yl]amino]-2-methyl-benzoyl]piperazine-1-carboxamide hydrochloride | 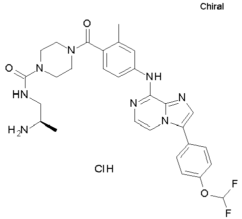 | [M-H]-: 577.3 | Reference Example 329 and tert-butyl (R)-(1-aminopropan-2-yl)carbamate hydrochloride [CAS#100927-10-4]. Deprotection with 4M HCl in dioxane. |
| REF 353 | N-[(2S)-2-aminopropyl]-4-[4-[[3-[4-(difluoromethoxy)ph enyl]imidazo[1,2-a]pyrazin-8-yl]amino]-2-methyl-benzoyl] piperazine-1-carboxamide hydrochloride | 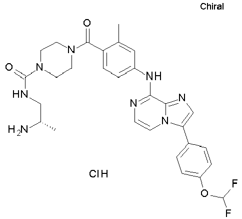 | 579.3 | Reference Example 329 and tert-butyl (S)-(1-aminopropan-2-yl)carbamate hydrochloride [CAS#146552-71-8] |
| REF 354 | N-[(2R)-2-aminopropyl]-4-[4-[[3-(2,3-difluoro-4-methoxyphenyl)imidazo[1,2-a]pyrazin-8-yl]amino]-2-methyl-benzoyl] piperazine-1-carboxamide hydrochloride | 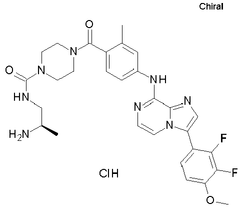 | 579.3 | Reference Example 232 and tert-butyl (R)-(1-aminopropan-2-yl)carbamate hydrochloride [CAS#100927 -10-4]. |
| REF 355 | N-(2-aminoethyl)-4-[4-[[3-(2-chloro-3-fluoro-4-methoxyphenyl)imidazo[1,2-a]pyrazin-8-yl]amino]-2-methyl-benzoyl]piperazine-1-carboxamide formate | 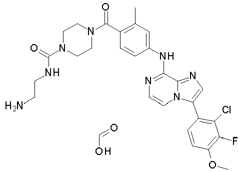 | 581.2 | Intermediate 103 ( [4-[[3-(2-chloro-3-fluoro-4-methoxyphenyl)imidazo[1,2-a]pyrazin-8-yl]amino]-2-methyl-phenyl]-piperazin-1-yl-methanone) and N-BOC-ethylenediamine. Purification: prep HPLC with formic acid |
| REF 356 | N-[(2R)-2-aminopropyl]-4-[4-[[3-(2-chloro-3-fluoro-4-methoxyphenyl)imidazo[1,2-a]pyrazin-8-yl]amino]-2-methyl-benzoyl]piperazine-1-carboxamide formate | 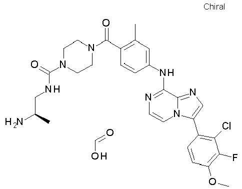 | 595.2 | Intermediate 103 and tert-butyl (R)-(1-aminopropan-2-yl)carbamate hydrochloride [CAS#100927-10-4]. Purification: prep HPLC with formic acid |
| REF 357 | N-[(2S)-2-aminopropyl]-4-[4-[[3-(2-chloro-3-fluoro-4-methoxyphenyl)imidazo[1,2-a]pyrazin-8-yl]amino]-2-methyl-benzoyl]piperazine-1-carboxamide formate | 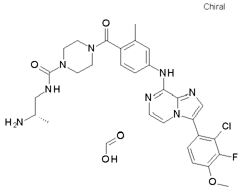 | 595.2 | Intermediate 103 and tert-butyl (S)-(1-aminopropan-2-yl)carbamate hydrochloride [CAS#146552-71-8]. Purification: prep HPLC with formic acid |
| REF 358 | 2-[4-[8-[4-[4-[(2R)-2-(aminomethyl)morp holine-4-carbonyl]piperazine-1-carbonyl]-3-methyl-anilino]imidazo[1,2-alpyrazin-3-yl]-3-chloro-2-fluoro-phenoxy]acetonitrile formate | 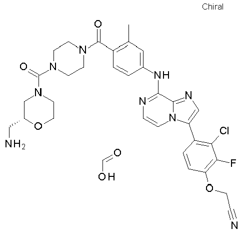 | 662.4 | Intermediate 111 and tert-butyl N-[[(2S)-morpholin-2-yl]methyl] carbamate. Deprotection in DCM:TFA 10:2, 20°C. Purification: prep HPLC with formic acid |
| REF 359 | 2-[4-[8-[4-[4-[(2S)-2-(aminomethyl)morp holine-4-carbonyl]piperazine-1-carbonyl]-3-methyl-anilino]imidazo[1,2-a]pyrazin-3-yl]-3-chloro-2-fluoro-phenoxy]acetonitrile formate | 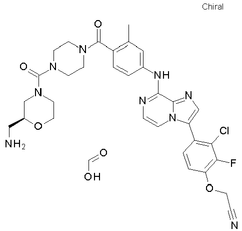 | 662.4 | Intermediate 111 and tert-butyl N-[[(2R)-morpholin-2-yl]methyl] carbamate. Deprotection in DCM:TFA 10:2, 20°C. Purification: prep HPLC with formic acid |
| REF 360 | N-(2-aminoethyl)-4-[4-[[3-[2-chloro-4-(cyanomethoxy)-3-fluorophenyl]imidazo[1,2-a]pyrazin-8-yl]amino]-2-methyl-benzoyl]piperazine-1-carboxamide formate | 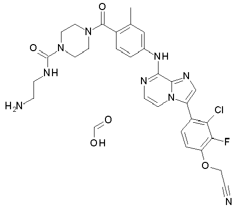 | 606.2 | Intermediate 111 and N-BOC-ethylenediamine. Deprotection in DCM:TFA 10:2, 20°C. Purification: prep HPLC with formic acid |
| REF 361 | N-[(1R)-2-amino-1-methyl-ethyl]-4-[4-[[3-[2-chloro-4-(cyanomethoxy)-3-fluorophenyl]imidazo[1,2-a]pyrazin-8-yl]amino]-2-methyl-benzoyl]piperazine-1-carboxamide formate | 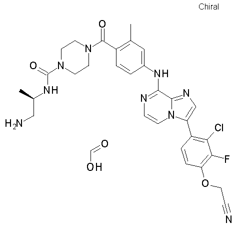 | 620.3 | Intermediate 111 and tert-butyl (R)-(2-aminopropyl)carbamate [CAS#333743-54-7]. Deprotection in DCM:TFA 10:2, 20°C. Purification: prep HPLC with formic acid |
| REF 362 | N-[(1S)-2-amino-1-methyl-ethyl]-4-[4-[[3-[2-chloro-4-(cyanomethoxy)-3-fluorophenyl]imidazo[1,2-a]pyrazin-8-yl]amino]-2-methyl-benzoyl] piperazine-1-carboxamide formate | 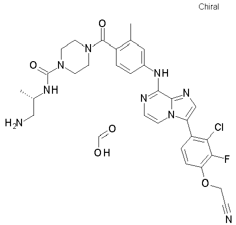 | 620.3 | Intermediate 111 and tert-butyl (R)-(2-aminopropyl)carbamate [CAS#333743-54-7]. Deprotection in DCM:TFA 10:2, 20°C. Purification: prep HPLC with formic acid |
| REF 363 | N-[(2R)-2-aminopropyl]-4-[4-[[3-[2-chloro-4-(cyanomethoxy)-3-fluorophenyl]imidazo[1,2-a]pyrazin-8-yl]amino]-2-methyl-benzoyl]piperazine-1-carboxamide formate | 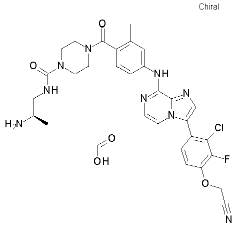 | 620.3 | Intermediate 111 and tert-butyl (R)-(1-aminopropan-2-yl)carbamate hydrochloride [CAS#100927-10-4]. Deprotection in DCM:TFA 10:2, 20°C. Purification: prep HPLC with formic acid |
| REF 364 | N-[(2S)-2-aminopropyl]-4-[4-[[3-[2-chloro-4-(cyanomethoxy)-3-fluorophenyl]imidazo[1,2-a]pyrazin-8-yl]amino]-2-methylbenzoyl]piperazine-1-carboxamide formate | 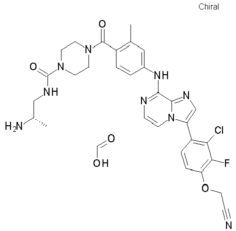 | 620.4 | Intermediate 111 and tert-butyl (S)-(1-aminopropan-2-yl)carbamate hydrochloride [CAS#146552-71-8]. Deprotection in DCM:TFA 10:2, 20°C. Purification: prep HPLC with formic acid |
| REF 365 | N-[(1R)-2-amino-1-methyl-ethyl]-4-[4-[[3-(2-chloro-3-fluoro-4-methoxyphenyl)imidazo[1,2-a]pyrazin-8-yl]amino]-2-methyl-benzoyl] piperazine-1-carboxamide formate | 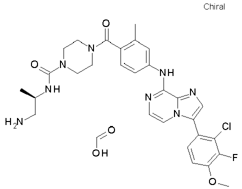 | 595.1 | Intermediate 103 and tert-butyl (R)-(2-aminopropyl)carbamate [CAS#333743-54-7]. Purification: prep HPLC with formic acid |
| REF 366 | [4-[(2R)-2-(aminomethyl)morp holine-4-carbonyl] piperazin-1-yl]-[4-[[3-(2-chloro-3-fluoro-4-methoxyphenyl)imidazo[1,2-a]pyrazin-8-yl]amino]-2-methylphenyl] methanone formate | 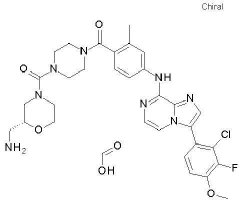 | 637.2 | Intermediate 103 and tert-butyl N-[[(2S)-morpholin-2-yl]methyl] carbamate. Deprotection in DCM:TFA 2:1, rt. Purification: prep HPLC with formic acid |
| REF 367 | N-[3-(2-aminoethylcarbamoy lamino)propyl]-4-[[3-(2,3-difluoro-4-methoxyphenyl)imidazo[1,2-a]pyrazin-8-yl]amino]-2-ethyl-benzamide | 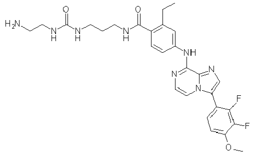 | 567.3 | Reference Example 332 and N-BOC-ethylenediamine |
| REF 368 | N-[2-(azetidin-1-yl)ethyl]-4-[4-[[3-[4-(difluoromethoxy)ph enyl]imidazo[1,2-a]pyrazin-8-yl]amino]-2-methyl-benzoyl]piperazine-1-carboxamide formate | 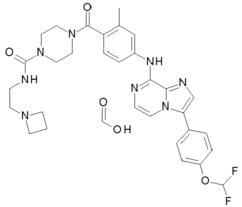 | 605.3 | Reference Example 329 and 1-Azetidineethanamine |
| REF 369 | N-(azetidin-3-yl)-4-[4-[[3-[4-(difluoromethoxy)ph enyl]imidazo[1,2-a]pyrazin-8-yl]amino]-2-methyl-benzoyl]piperazine-1-carboxamide trifluoroacetate | 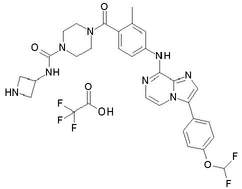 | 577.3 | Reference Example 329 and 1-BOC-3-(amino)azetidine Deprotection: 2h at rt in a 10/1 DCM/TFA mixture. |
| REF 370 | 4-[4-[[3-[4-(difluoromethoxy)ph enyl]imidazo[1,2-a]pyrazin-8-yl]amino]-2-methyl-benzoyl]-N-[rac-(3R,4R)-4-hydroxypyrrolidin-3-yl]piperazine-1-carboxamide hydrochloride | 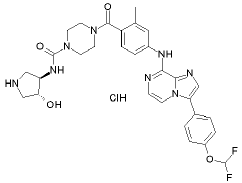 | 607.3 | Reference Example 329 and rac-tert-butyl (3R,4R)-3-amino-4-hydroxypyrrolidine-1-carboxylate hydrochloride [CAS# 148214-90-8] |
| REF 371 | 4-[4-[[3-[4-(difluoromethoxy)ph enyl]imidazo[1,2-a]pyrazin-8-yl]amino]-2-methyl-benzoyl]-N-[rac-(3R,4R)-4-methoxypyrrolidin-3-yl]piperazine-1-carboxamide hydrochloride | 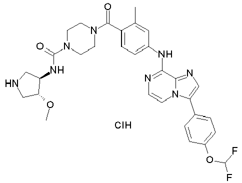 | 621.3 | Reference Example 329 and rac-tert-butyl (3R,4R)-3-amino-4-methoxypyrrolidine-1- carboxylate [CAS#429673-79-0] |
| REF 372 | N-[3-(3-aminopropylcarbam oylamino)propyl]-4-[[3-(2,3-difluoro-4-methoxyphenyl)imidazo[1,2-a]pyrazin-8-yl]amino]-2-ethyl-benzamide | 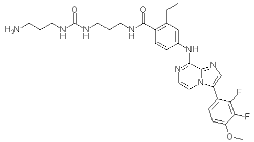 | 581.2 | Reference Example 332 and N-BOC-1,3-diaminopropane |
| REF 373 | (R)-(4-(4-((3-(2,3-difl uoro-4-methoxyphenyl)imid azo[1,2-a]pyrazin-8-yl)amino)-2-methylbenzoyl)piper azin-1-yl)(3-(hydroxymethyl)pipe razin-1-yl)methanone hydrochloride | 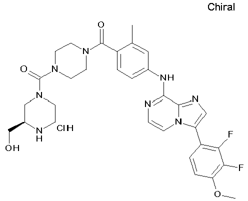 | 621.4 | From Reference Example 232 and tert-butyl (R)-2-(hydroxymethyl)piperaz ine-1-carboxylate |

### Example REF 374:

### [4-[[3-[4-(difluoromethoxy)phenyl]imidazo[1,2-a] pyrazin-8-yl] amino]-2-methyl-phenyl] - [4-(piperidine-4-carbonyl)piperazin-1-yl]methanone hydrochloride

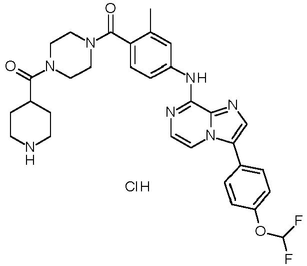

### Step 1) tert-butyl 4-[4-[4-[[3-[4-(difluoromethoxy)phenyl]imidazo[1,2-a]pyrazin-8-yl]amino]-2-methyl-benzoyl]piperazine-1-carbonyl]piperidine-1-carboxylate

In a 25 mL vial, (4-((3-(4-(difluoromethoxy)phenyl)imidazo[1,2-a]pyrazin-8-yl)amino)-2-methylphenyl)(piperazin-1-yl)methanone hydrochloride; Reference Example 329 (250 mg, 243 µmol, Eq: 1) and DIPEA (157 mg, 212 µL, 1.21 mmol, Eq: 5.0) were combined with DMF (5 mL) to give a light yellow suspension, that was stirred until most solids were dissolved. Then 1-(tert-butoxycarbonyl)piperidine-4-carboxylic acid (83.5 mg, 364 µmol, Eq: 1.5) and HATU (185 mg, 485 µmol, Eq: 2.0) were added. The walls of the tube were washed down with some DMF and the reaction mixture was stirred at RT for 2 h. The reaction mixture was poured into 10 mL ethyl acetate and extracted once with 0.1 M aq. NaOH. The organic phase was washed with brine, dried with magnesium sulfate monohydrate, filtered and the resulting solution was concentrated *in vacuo.* The crude material was purified by flash chromatography (silica gel, 25g, 0% to 75% DCM/MeOH/aq. 25% NH₄OH (95/5/1)) leading to 101 mg off-white solid. MS: [M+H]+; 690.4

### Step 2) Reference Example 374

### [4-[[3-[4-(difluoromethoxy)phenyl]imidazo[1,2-a]pyrazin-8-yl]amino]-2-methyl-phenyl]-[4-(piperidine-4-carbonyl)piperazin-1-yl]methanone hydrochloride

In a 50 mL round-bottomed flask, tert-butyl 4-(4-(4-((3-(4-(difluoromethoxy)phenyl)imidazo[1,2-a]pyrazin-8-yl)amino)-2-methylbenzoyl)piperazine-1-carbonyl)piperidine-1-carboxylate (Step 1) (100 mg, 145 µmol, Eq: 1) was combined with dioxane (1 mL) to give a colorless solution. Hydrogen chloride (4M in dioxane) (181 µl, 725 µmol, Eq: 5) was added. Stirring was continued and hydrogen chloride (4M in dioxane) (181 µl, 725 µmol, Eq: 5) was added again. The reaction mixture was stirred overnight. The reaction mixture was diluted with anhydrous ether, stirred and then filtered. The filter cake was washed with ether several times and dried in HV leading to the target compound as an off-white solid (93 mg, yield: 93 %). MS (ISN): [M-H]⁻; 588.5

The following examples were prepared in analogy to Reference Example 374 (Step 2 only required if protecting group needs removal):

| Ex. | Name | Structure | MS ESI [M+H]⁺ | Starting Material |
|---|---|---|---|---|
| REF 375 | [4-[[3-(3-fluoro-4-methoxyphenyl)imidazo[1,2-a]pyrazin-8-yl] amino]-2-methylphenyl]-[4-(pyrrolidine-2-carbonyl)piperazin-1-yl]methanone | 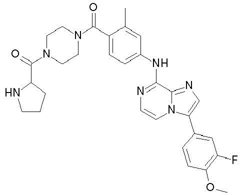 | [M-H]-;556.6 | Reference Example 330 and 1-(tert-butoxycarbonyl)pyrroli dine-2-carboxylic acid. Deprotection: 45 min at rt. Purified by flash chromatography (silica gel, 0% to 100% DCM/MeOH/NH4OH (90/10/1)) |
| REF 376 | [4-[(2S)-azetidine-2-carbonyl]piperazin- 1-yl]-[4-[[3-(3-fluoro-4-methoxyphenyl)imidazo[1,2-a]pyrazin-8-yl]amino]-2-methyl-phenyl]methanone | 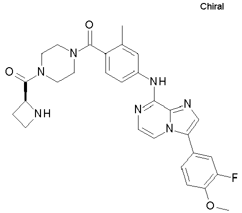 | [M-H]-; 542.6 | Reference Example 330 and (S)-1-(tert-butoxycarbonyl)azetidi ne-2-carboxylic acid. Deprotection: 45 min at rt. Purified by flash chromatography (silica gel, 0% to 100% DCM/MeOH/NH4OH (90/10/1)) |
| REF 377 | [4-[(2S)-azetidine-2-carbonyl] piperazin- 1-yl]-[4-[[3-[4-(difluoromethoxy)phe nyl]imidazo[1,2-a]pyrazin-8-yl]amino]-2-methyl-phenyl]methanone hydrochloride | 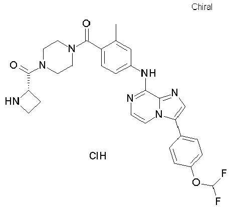 | [M-H]-; 560.2 | Reference Example 329 and (S)-1-(tert-butoxycarbonyl) azetidi ne-2-carboxylic acid. |
| REF 378 | [4-[[3-[4-(difluoromethoxy)phe nyl]imidazo[1,2-a]pyrazin-8-yl]amino]-2-methylphenyl]-[4-(pyrrolidine-2-carbonyl)piperazin-1-yl]methanone hydrochloride | 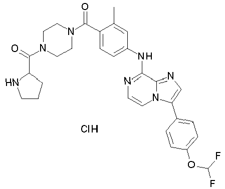 | [M-H]-;556.6 | Reference Example 329 and 1-(tert-butoxycarbonyl)pyrroli dine-2-carboxylic acid. |
| REF 379 | [4-[[3-(3-fluoro-4-methoxyphenyl)imidazo[1,2-a]pyrazin-8-yl]amino]-2-methylphenyl]-[4-[(2S)-pyrrolidine-2-carbonyl]piperazin-1-yl]methanone hydrochloride | 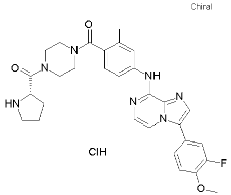 | [M-H]-; 556.4 | Reference Example 330 and (S)-1-(tert-butoxycarbonyl)pyrroli dine-2-carboxylic acid. |
| REF 380 | [4-[[3-(3-fluoro-4-methoxyphenyl)imidazo[1,2-a]pyrazin-8-yl] amino]-2-methylphenyl]-[4-[(2R)-pyrrolidine-2-carbonyl]piperazin-1-yl]methanone hydrochloride | 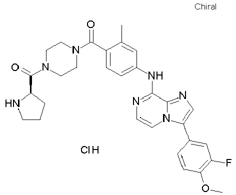 | [M-H]-; 556.4 | Reference Example 330 and (R)-1-(tert-butoxycarbonyl)pyrroli dine-2-carboxylic acid. |
| REF 381 | [4-[[3-[4-(difluoromethoxy)phe nyl]imidazo[1,2-a]pyrazin-8-yl]amino]-2-methyl-phenyl]-[4-[(2S,4R)-4-hydroxypyrrolidine-2-carbonyl]piperazin-1-yl]methanone hydrochloride | 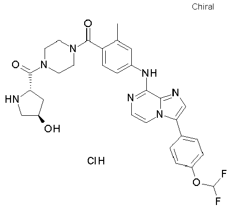 | [M-H]-; 590.4 | Reference Example 329 and (2S,4R)-1-(tert-butoxycarbonyl)-4-hydroxypyrrolidine-2-carboxylic acid. |
| REF 382 | [4-[[3-[4-(difluoromethoxy)phe nyl]imidazo[1,2-a]pyrazin-8-yl]amino]-2-methyl-phenyl]-[4-[2-(hydroxymethyl)piper azine-1-carbonyl]-1-piperidyl]methanone hydrochloride | 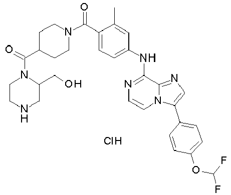 | [M+H] +; 620.2 | Reference Example 329 and tert-butyl 3-(hydroxymethyl)pipera zine-1-carboxylate |
| REF 383 | [4-[[3-[4-(difluoromethoxy)phe nyl]imidazo[1,2-a]pyrazin-8-yl] amino]-2-methylphenyl]-[4-[(2S,4S)-4-hydroxypyrro lidine- 2-carbonyl]piperazin-1-yl]methanone hydrochloride hydrochloride | 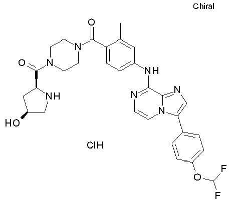 | [M-H]-; 590.5 | Reference Example 329 and (2S,4S)-1-(tert -butoxycarbonyl)-4-hydroxypyrrolidine-2-carboxylic acid. Deprotection: 5eq HCl in dioxane (4M) in diethylether/MeOH 5/2 at rt overnight. |
| REF 384 | [4-[[3-[4-(difluoromethoxy)phe nyl]imidazo[1,2-a]pyrazin-8-yl]amino]-2-methyl-phenyl]-[4-[3-(hydroxymethyl)piper azine-1-carbonyl]-1-piperidyl]methanone hydrochloride | 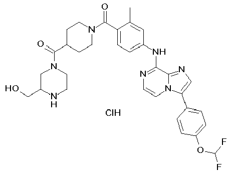 | 620.5( M+H) | Reference Example 1 and tert-butyl 2-(hydroxymethyl)pipera zine-1-carboxylate. Deprotection with 10 eq HCl 4M in dioxane, 45 min at rt. |
| REF 385 | [4-[[3-(2,3-difluoro-4-methoxyphenyl)imidazo[1,2-a]pyrazin-8-yl]amino]-2-methyl-phenyl]-[4-[(2S,4R)-4-hydroxypyrrolidine-2-carbonyl]piperazin-1-yl]methanone hydrochloride | 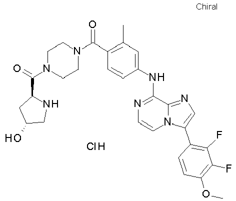 | [M-H]-; 590.5 | Reference Example 232 and (2S,4R)-1-(tert-butoxycarbonyl)-4-hydroxypyrrolidine-2-carboxylic acid [CAS# 13726-69-7] |
| REF 386 | [4-[[3-(2,3-difluoro-4-methoxyphenyl)imidazo[1,2-a]pyrazin-8-yl]amino]-2-ethyl-phenyl]-[4-[(2S,4R)-4-hydroxypyrrolidine-2-carbonyl]piperazin-1-yl]methanone hydrochloride | 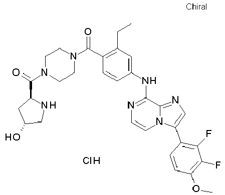 | 606.4 | Intermediate 110 and (2S,4R)-1-(tert-butoxycarbonyl)-4-hydroxypyrrolidine-2-carboxylic acid [CAS#13726-69-7]. Purification: prep HPLC with HCl |
| REF 387 | [4-[[3-(3-fluoro-4-methoxyphenyl)imidazo[1,2-a]pyrazin-8-yl] amino]-2-methylphenyl]-[4-[(3S)-pyrrolidine-3-carbonyl] piperazin- 1-yl]methanone hydrochloride | 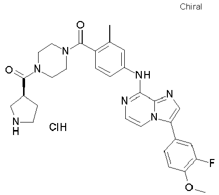 | 558.5 | Reference Example 330 and (3S)-1-(tert-Butoxycarbonyl)-3-pyrrolidinecarboxylic acid |
| REF 388 | [4-(azetidine-3-carbonyl)piperazin-1-yl]-[4-[[3-(3-fluoro-4-methoxyphenyl)imidazo[1,2-a]pyrazin-8-yl]amino]-2-methyl-phenyl]methanone hydrochloride | 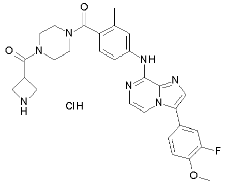 | 544.5 | Reference Example 330 and 1-Boc-Azetidine- 3-carboxylic acid |
| REF 389 | 2-[2,3-difluoro-4-[8-[4-[4-[(3R,4R)-3-hydroxypiperidine-4-carbonyl]piperazine-1-carbonyl]-3-methyl-anilino]imidazo[1,2-a]pyrazin-3-yl]phenoxy]acetonitril e formate | 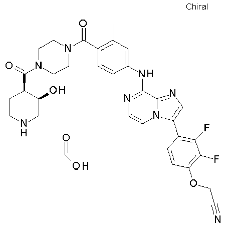 | 631.1 | Intermediate 109 and (3R,4R)-1-tert-butoxycarbonyl-3-hydroxy-piperidine-4-carboxylic acid [CAS# 1932301-36-4]. Deprotection: 2h at rt in a 80/20 DCM/TFA mixture (95 eq TFA), then neutralized with sat. Na₂CO₃ to pH8. Purification: prep HPLC with formic acid |
| REF 390 | 2-[2,3-difluoro-4-[8-[4-[4-[(3S,4S)-3-hydroxypiperidine-4-carbonyl] piperazine-1-carbonyl]-3-methyl-anilino]imidazo[1,2-a]pyrazin-3-yl]phenoxy]acetonitril e formate | 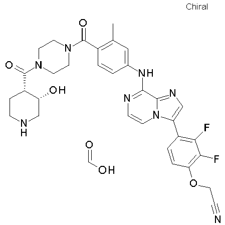 | 631.1 | Intermediate 109 and (3S,4S)-1-tert-butoxycarbonyl-3-hydroxy-piperidine-4-carboxylic acid, obtained by saponification of 1,4-Piperidinedicarboxylic acid, 3-hydroxy-, 1-(1, 1-dimethylethyl) 4-methyl ester, (3S,4S)-[CAS# 2166250-53-7] Deprotection: 2h at rt in a 80/20 DCM/TFA mixture (95 eq TFA), then neutralized with sat. Na₂CO₃ to pH 8. Purification: prep. HPLC in presence of formic acid |
| REF 391 | 2-[2,3-difluoro-4-[8-[4-[4-[(3S,4R)-3-hydroxypiperidine-4-carbonyl]piperazine-1-carbonyl]-3-methyl-anilino]imidazo[1,2-a]pyrazin-3-yl]phenoxy]acetonitril eformate | 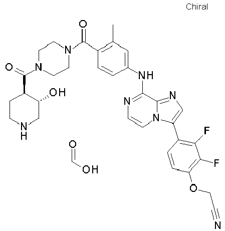 | 631.1 | Intermediate 109 and (3S,4R)-1-tert-butoxycarbonyl-3-hydroxy-piperidine-4-carboxylic acid obtained by saponification of 1,4-Piperidinedicarboxylic acid, 3-hydroxy-, 1-(1, 1-dimethylethyl) 4-ethyl ester, (3S,4R)-[CAS# 1805790-50-4]. Deprotection: 2h at rt in a 80/20 DCM/TFA mixture (95 eq TFA), then neutralized with sat. Na2CO3 to pH8. Purification: prep HPLC with formic acid as additive |
| REF 392 | 2-[2,3-difluoro-4-[8-[4-[4-[(3R,4S)-3-hydroxypiperidine-4-carbonyl]piperazine-1-carbonyl]-3-methyl-anilino]imidazo[1,2-a]pyrazin-3-yl]phenoxy]acetonitril e formate | 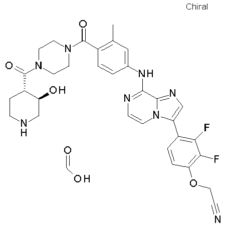 | 631.1 | Intermediate 109 and 1, 4-Piperidinedicarboxyl ic acid, 3-hydroxy-, 1-(1,1-dimethyl ethyl) ester, (3R,4S)- [CAS# 1821775-81-8]. Deprotection: 2h at rt in a 80/20 DCM/TFA mixture (95 eq TFA), then neutralized with sat. Na2CO3 to pH8. Purification: prep HPLC with formic acid as additive |
| REF 393 | [4-[[3-(2,3-difluoro-4-methoxyphenyl)imidazo[1,2-a]pyrazin-8-yl]amino]-2-methyl-phenyl]-[4-[(3R,4S)-3-hydroxypiperidine-4-carbonyl]piperazin-1-yl]methanone hydrochloride | 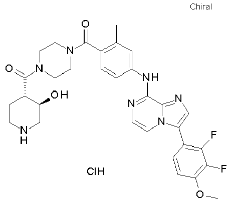 | 606.3 | Reference Example 232 and 1,4-Piperidinedicarboxylic acid, 3-hydroxy-, 1-(1,1-dimethyl ethyl) ester, (3R,4S)- [CAS# 1821775-81-8]. |
| REF 394 | [4-[[3-(2,3-difluoro-4-methoxyphenyl)imidazo[1,2-a]pyrazin-8-yl]amino]-2-methyl-phenyl]-[4-[(3S,4R)-3-hydroxypiperidine-4-carbonyl]piperazin-1-yl]methanone hydrochloride | 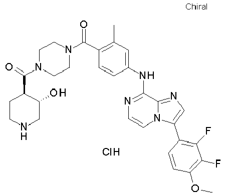 | 606.3 | Reference Example 232 and (3S,4R)-1-tert-butoxycarbonyl-3-hydroxy-piperidine-4-carboxylic acid obtained by saponification of 1,4-Piperidinedicarboxylic acid, 3-hydroxy-, 1-(1,1-dimethylethyl) 4-ethyl ester, (3S,4R)-[CAS# 1805790-50-4]. |
| REF 395 | 2-[4-[8-[3-ethyl-4-[4-(pi peridine-4-carbonyl)piperazine-1-carbonyl] anilino] imid azo[1,2-a]pyrazin-3-yl]-2,3-difluoro-phenoxy] acetonitrile formate | 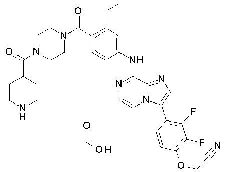 | 629.1 | Intermediate 112 and 1-(tert-butoxycarbonyl)piperid ine-4-carboxylic acid. Deprotection: 0.5h at rt in a 80/20 DCM/TFA mixture (13 eq TFA), then neutralized with sat. Na2CO3 to pH8. Purification: prep HPLC with formic acid as additive |
| REF 396 | [4-[[3-(2,3-difluoro-4-methoxyphenyl)imidazo [1,2-a]pyrazin-8-yl]amino] -2-methylphenyl]-[4-[rac-(3S,4S)-3-hydroxypiperidine-4-carbonyl]piperazin-1-yl]methanone | | 606.3 | Reference Example 232 and (3S,4S)-1-tert-butoxycarbonyl-3-hydroxy-piperidine-4-carboxylic acid, obtained by saponification of 1,4-Piperidinedicarboxylic acid, 3-hydroxy-, 1-(1,1-dimethylethyl) 4-methyl ester, (3S,4S)-[CAS# 2166250-53-7] Purification: prep HPLC |
| REF 397 | N-[2-[[(2S)-2-amino-5-guanidino-pentanoyl] amino]ethyl ]-4-[[3-(2,3-difluoro-4-methoxyphenyl)imidazo[1,2-a]pyrazin-8-yl]amino]-2-ethyl-benzamide trifl uoroacetate | | 623.3 | Reference Example 331 and FMOC-ARG-OH. Deprotection using 22 eq piperidine at 0-20°C for 12h. Purification by prep HPLC (TFA as additive). |
| REF 398 | 1-(4-(2-chloro-4-((3-(3-fluoro-4-methoxyphenyl)imida zo[1,2-a]pyrazin-8-yl)amino)benzoyl)pip erazin-1-yl)-2-(methyl amino)ethanon e | 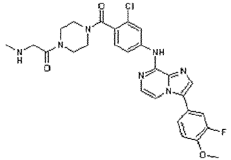 | 552.2 | Intermediate 123 and 2-((tert-butoxycarbonyl)(methy l)amino)acetic acid |
| REF 399 | 2-[2,3-difluoro-4-[8-[3-methyl-4-[4-[rac-(3R,4R)-3-hydroxypiperidine-4-carbonyl] piperazine-1-carbonyl] anilino]imid azo[1,2-a]pyrazin-3-yl]phenoxy] acetonitril e formate | 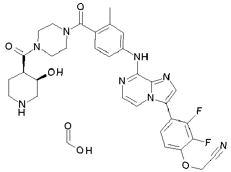 | 631.3 | Intermediate 109 and 1, 4-Piperidinedicarboxyl ic acid, 3-hydroxy-, 1-(1,1-dimethylethyl) ester, (3R,4R)-rel-[CAS# 1932301-36-4]. Deprotection: 1h at rt in a 80/20 DCM/TFA mixture (13 eq TFA), then neutralized with sat. Na2CO3 to pH8. Purification: prep HPLC with formic acid as additive |
| REF 400 | 2-[2,3-difluoro-4-[8-[3-methyl-4-[4-(piperidine-4-carbonyl)piperazine-1-carbonyl] anilino] imid azo[1,2-a]pyrazin-3-yl]phenoxy]acetonitril e formate | 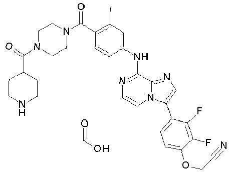 | 615.3 | Intermediate 109 and N-BOC-isonipecotic acid. Deprotection: 1h at rt in a 80/20 DCM/TFA mixture (13 eq TFA), then neutralized with sat. Na2CO3 to pH8. Purification: prep. HPLC with formic acid as additive |
| REF 401 | 2-[2,3-difluoro-4-[8-[3-methyl-4-[4-[(3R)-pyrrolidine-3-carbonyl] piperazine-1-carbonyl] anilino] imid azo[1,2-a]pyrazin-3-yl]phenoxy] acetonitril e formate | 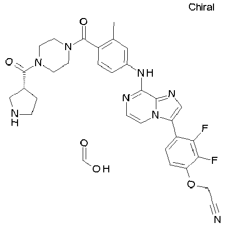 | 601.3 | Intermediate 109 and (R)-1-BOC-pyrrolidine-3-carboxylic acid. Deprotection: 1h at rt in a 80/20 DCM/TFA mixture (60 eq TFA), then neutralized with sat. Na2CO3 to pH8. Purification: prep. HPLC with formic acid as additive |
| REF 402 | 2-[4-[8-[3-ethyl-4-[4-[rac-(3S,4S)-3-hydroxypiperidine-4-carbonyl] piperazine-1-carbonyl] anilino] imid azo[1,2-a]pyrazin-3-yl]-2,3-difluoro-phenoxy] acetonitrile formate | 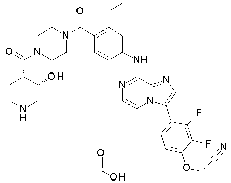 | 645.3 | Intermediate 112 and (3S,4S)-1-tert-butoxycarbonyl-3-hydroxy-piperidine-4-carboxylic acid, obtained by saponification of 1,4-Piperidinedicarboxylic acid, 3-hydroxy-, 1-(1,1-dimethylethyl) 4-methyl ester, (3S,4S)-[CAS# 2166250-53-7] Deprotection: 1h at rt in a 80/20 DCM/TFA mixture (95 eq TFA), then neutralized with sat. Na2CO3 to pH8. Purification: prep HPLC |
| REF 403 | 2-[2,3-difluoro-4-[8-[4-[4-[(2S,4R)-4-hydroxypyrrolidine-2-carbonyl]piperazine-1-carbonyl]-3-methyl-anilino]imidazo[1,2-a]pyrazin-3-yl]phenoxy]acetonitril e formate | 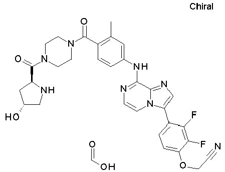 | 617.3 | Intermediate 109 and (2R,4S)-1-tert-butoxycarbonyl-4-hydroxy-pyrrolidine-2-carboxylic acid. Deprotection: 1h at rt in a 80/20 DCM/TFA mixture (62 eq TFA), then neutralized with sat. Na2CO3 to pH8. Purification: prep HPLC |
| REF 404 | 2-[2,3-difluoro-4-[8-[3-methyl-4-[4-[(3 S)-pyrrolidine-3-carbonyl] piperazine-1-carbonyl] anilino] imid azo[1,2-a]pyrazin-3-yl]phenoxy]acetonitril e;formic acid | 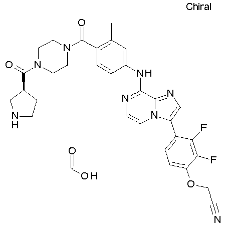 | 601.1 | Intermediate 109 and (S)-1-BOC-pyrrolidine-3-carboxylic acid. Deprotection: 1h at rt in a 100/10 DCM/TFA mixture (160 eq TFA), Purification: prep HPLC |
| REF 405 | (R)-(4-(4-((3-(2,3-difluoro-4-methoxyphenyl)imida zo[1,2-a]pyrazin-8-yl)amino)-2-methylbenzoyl)pipera zin-1-yl)(pyrrolidin-3-yl)methanone hydrochloride | 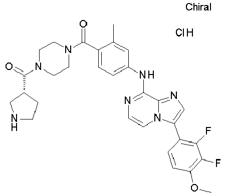 | 576.5 | Reference Example 232 and (R)-1-BOC-pyrrolidine-3-carboxylic acid |
| REF 406 | [4-(azetidine-3-carbonyl)piperazin-1-yl]-[4-[[3-(2,3-difluoro-4-methoxyphenyl)imidazo[1,2-a]pyrazin-8-yl] amino]-2-methylphenyl]methanone;hy drochloride | 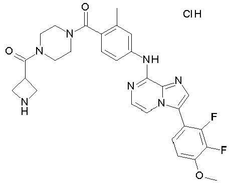 | 562.7 | Reference Example 232 and 1-(tert-butoxycarbonyl) azetidi ne-3-carboxylic acid |
| REF 407 | [4-[[3-(2,3-difluoro-4-methoxyphenyl)imidazo[1,2-a]pyrazin-8-yl] amino]-2-methylphenyl]-[4-(4-hydroxypiperidine-4-carbonyl)piperazin-1-yl]methanone hydrochloride | 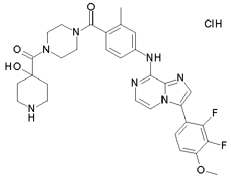 | 606.5 | Reference Example 232 and 1-(tert-butoxycarbonyl)-4-hydroxypiperidine-4-carboxylic acid |
| REF 408 | [4-(3-azabicyclo[3.2.1]octan e-8-carbonyl)piperazin-1-yl]-[4-[[3-(2,3-difluoro-4-methoxyphenyl)imidazo[1,2-a]pyrazin-8-yl] amino]-2-methylphenyl] methanone hydrochloride | 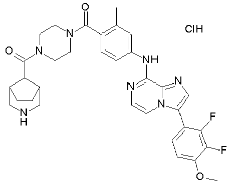 | 616.4 | Reference Example 232 and 3-(tert-butoxycarbonyl) bicyclo [3.2.1]octane-8-carboxylic acid |
| REF 409 | 2-[2,3-difluoro-4-[8-[4-[4-(4-hydroxypiperidine-4-carbonyl)piperazine-1-carbonyl]-3-methyl-anilino]imidazo[1,2-a]pyrazin-3-yl] phenoxy] acetonitril e formate | 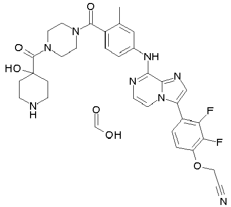 | 631.3 | Intermediate 109 and 1-(tert-butoxycarbonyl)-4-hydroxypiperidine-4-carboxylic acid |
| REF 410 | N-[(2R)-2-[[(2S)-2-amino-5 -guanidino-pentanoyl]amino]prop yl]-4-[[3-(2,3-difluoro-4-methoxyphenyl)imidazo[1,2-a]pyrazin-8-yl]amino]-2-ethyl-benzamide formate | 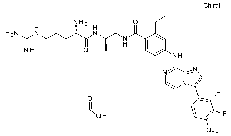 | 637.1 | Intermediate 104 and FMOC-ARG-OH. Deprotection using 22 eq piperidine at 0-20°C for 12h. Purification by prep HPLC (formic acid as additive). |
| REF 411 | N-[(2S)-2-[[(2S)-2-amino-5-guanidino-pentanoyl] amino]prop yl]-4-[[3-(2,3-difluoro-4-methoxy-phenyl)imidazo[1,2-a]pyrazin-8-yl]amino]-2-ethyl-benzamide formate | 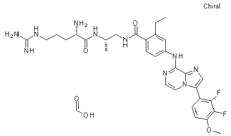 | 637.1 | Intermediate 105 and FMOC-ARG-OH. Deprotection using 22 eq piperidine at 0-20°C for 12h. Purification by prep HPLC (formic acid as additive). |
| REF 412 | N-[2-[[(2S)-2-amino-5-guanidino-pentanoyl] amino] ethyl ]-4-[[3-[4-(cyanomethoxy)-2,3-difluorophenyl]imidazo[1,2-a]pyrazin-8-yl] amino]-2-ethyl-benzamide formate | 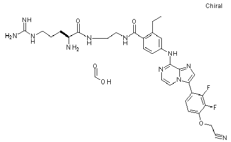 | 648.2 | Intermediate 116 and BOC-ARG-OH. Deprotection: 2h at rt in a 100/20 DCM/TFA mixture (200 eq TFA), then neutralized with sat. Na2CO3 to pH8. Purification: prep HPLC |
| REF 413 | 2-[3-chloro-2-fluoro-4-[8-[4-[4-[(2R)-2-(hydroxymethyl)piper azine-1-carbonyl] piperidine- 1-carbonyl]-3-methyl-anilino]imidazo[1,2-a]pyrazin-3-yl] phenoxy] acetonitril e | 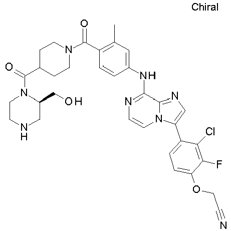 | 661.3 | Intermediate 111 and 124. Deprotection: 1h at rt in a 100/10 DCM/TFA mixture (160 eq TFA), Purification: prep HPLC |
| REF 414 | 2-[3-chloro-2-fluoro-4-[8-[4-[4-[(2S)-2-(hydroxymethyl)piper azine-1-carbonyl] piperidine-1-carbonyl]-3-methyl-anilino]imidazo[1,2-a]pyrazin-3-yl] phenoxy] acetonitril e formate | 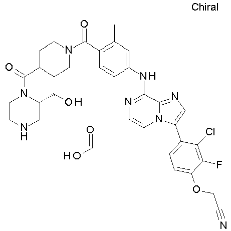 | 661.2 | Intermediate 111 and Intermediate 125. Deprotection: 1 h at rt in a 100/10 DCM/TFA mixture (160 eq TFA), Purification: prep HPLC |
| REF 415 | N-[2-[[(2R)-2-amino-5-guanidino-pentanoyl] amino] ethyl ]-4-[[3-(2,3-difluoro-4-methoxyphenyl)imidazo[1,2-a]pyrazin-8-yl]amino]-2-ethyl-benzamide formate | 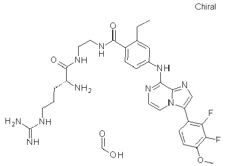 | 623.1 | Reference Example 331 and FMOC-D-ARG-OH. Deprotection using 22 eq piperidine at 0-20 °C for 12h. Purification by prep HPLC. |
| REF 416 | N-[2-[[(2S)-2-amino-5-guanidino-pentanoyl]amino]ethyl ]-4-[[3-(2-chloro-3-fluoro-4-methoxyphenyl)imidazo[1,2-a]pyrazin-8-yl]amino]-2-ethyl-benzamide formate | 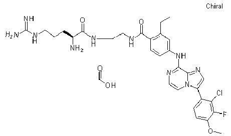 | 639.3 | Intermediate 117 and BOC-ARG-OH. Deprotection: 2h at rt in a 100/10 DCM/TFA mixture (38 eq TFA). Purification: prep HPLC |
| REF 417 | 2-[3-chloro-2-fluoro-4-[8-[3-methyl-4-[4-(piperidine-4-carbonyl)piperazine-1-carbonyl] anilino]imid azo[1,2-a]pyrazin-3-yl]phenoxy] acetonitril e formate | 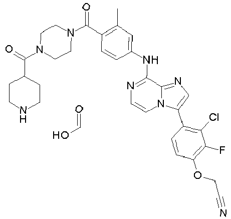 | 631.3 | Intermediate 111 and N-BOC-isonipecotic acid. Deprotection: 1h at rt in a 100/10 DCM/TFA mixture. Purification: prep HPLC. |
| REF 418 | N-[2-[[(2R)-2-amino-5-guanidino-pentanoyl]amino]ethyl ]-4-[[3-[2-chloro-4-(cyanomethoxy)-3 - fluorophenyl]imidazo[1,2-a]pyrazin-8-yl]amino]-2-ethyl-benzamide formate | 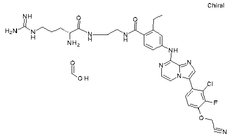 | 664.1 | Intermediate 118 and FMOC-D-ARG-OH. Deprotection using 22 eq piperidine at 0-20°C for 12h. Purification by prep HPLC. |
| REF 419 | [4-[[3-(2-chloro-3-fluoro-4-methoxyphenyl)imidazo[1,2-a]pyrazin-8-yl]amino]-2-methylphenyl]-[4-[(3S)-pyrrolidine-3-carbonyl]piperazin-1-yl]methanone formate | 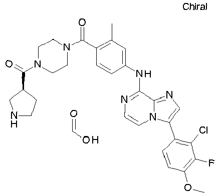 | 592.1 | Intermediate 103 and (S)-1-BOC-pyrrolidine-3-carboxylic acid. Deprotection: 1h at rt in a 100/10 DCM/TFA mixture. Purification: prep HPLC |
| REF 420 | 2-[3-chloro-2-fluoro-4-[8-[4-[4-[(2S,4R)-4-hydroxypyrrolidine- 2-carbonyl]piperazine-1-carbonyl]-3-methyl-anilino] imidazo[1,2-a]pyrazin-3-yl]phenoxy]acetonitril e formate | 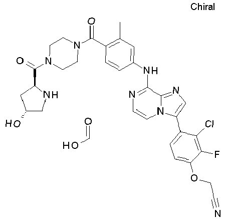 | 633.3 | Intermediate 111 and (2R,4S)-1-tert-butoxycarbonyl-4-hydroxy-pyrrolidine-2-carboxylic acid. Deprotection: 1h at rt in a 10/1 DCM/TFA mixture (95 eq TFA). Purification: prep. HPLC |
| REF 421 | 2- [3-chloro-2-fluoro-4-[8-[3-methyl-4-[4-[(3R)-pyrrolidine-3-carbonyl] piperazine-1-carbonyl] anilino] imid azo[1,2-a]pyrazin-3-yl]phenoxy] acetonitril e formate | 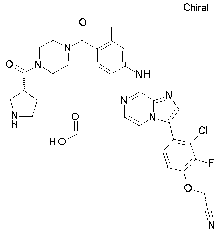 | 617.3 | Intermediate 111 and (R)-1-BOC-pyrrolidine-3-carboxylic acid. Deprotection: 1h at rt in a 10/1 DCM/TFA mixture Purification: prep. HPLC |
| REF 422 | 2-[3-chloro-2-fluoro-4-[8-[3-methyl-4-[4-[(3 S)-pyrrolidine-3-carbonyl] piperazine-1-carbonyl]anilino]imid azo[1,2-a]pyrazin-3-yl]phenoxy] acetonitril e formate | 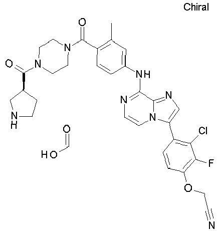 | 617.3 | Intermediate 111 and (S)-1-BOC-pyrrolidine-3-carboxylic acid. Deprotection: 1h at rt in a 10/1 DCM/TFA mixture Purification: prep. HPLC |
| REF 423 | N-[2-[[(2S)-2-amino-5-(4,5-dihydro-1H-imidazol-2-ylamino)pentanoyl]am ino]ethyl]-4-[[3-(2,3-difluoro-4-methoxyphenyl)imidazo[1,2-a]pyrazin-8-yl]amino]-2-ethyl-benzamide formate | 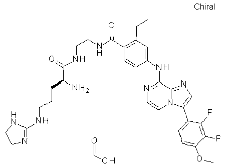 | 649.4 | Reference Example 331 and (2S)-2-(tert-butoxycarbonylamino)-5-(4, 5-dihydro-1H-imidazol-2-ylamino)pentanoic acid. Purification: prep. HPLC |
| REF 424 | N-[2-[[(2R)-2-amino-5-guanidino-pentanoyl]amino] ethyl ]-4-[[3-(2-chloro-3 - fluoro-4-methoxyphenyl)imidazo[1,2-a]pyrazin-8-yl] amino]-2-ethyl-benzamide formate | 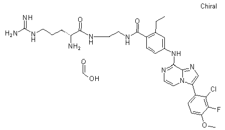 | 639.4 | Intermediate 117 and FMOC-D-ARG-OH. Deprotection using 22 eq piperidine at 20°C for 12h. Purification by prep HPLC. |
| REF 425 | N-[2-[[(2R)-2-amino-5-guanidino-pentanoyl] amino] ethyl ]-4-[[3-(4-chloro-2,3-difluorophenyl)imidazo[1,2-a]pyrazin-8-yl]amino]-2-ethyl-benzamide formate | 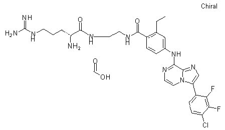 | 627.3 | Intermediate 119 and FMOC-D-ARG-OH. Deprotection using 22 eq piperidine at 20°C for 12h. Purification by prep HPLC. |
| REF 426 | 2-[3-chloro-2-fluoro-4-[8-[4-[4-(4-hydroxypiperidine-4-carbonyl)piperazine-1-carbonyl]-3-methyl-anilino]imidazo[1,2-a]pyrazin-3-yl] phenoxy] acetonitril e formate | 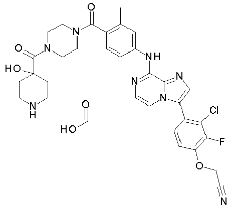 | 647.3 | Intermediate 111 and 1-(tert-butoxycarbonyl)-4-hydroxypiperidine-4-carboxylic acid. Deprotection: 1h at rt in a 10/1 DCM/TFA mixture Purification: prep. HPLC. |
| REF 427 | N-[2-[[(2S)-2-amino-4-guanidino-butanoyl]amino]ethyl] -4-[[3-(2,3-difluoro-4-methoxyphenyl)imidazo[1,2-a]pyrazin-8-yl]amino]-2-ethyl-benzamide formate | 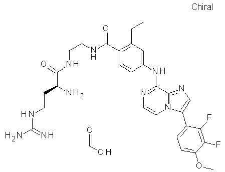 | 609.2 | Reference Example 331 and (2S)-2-(tert-butoxycarbonylamino)-4-guanidino-butanoic acid. Purification: prep. HPLC |
| REF 428 | [4-[[3-(2-chloro-3-fluoro-4-methoxyphenyl)imidazo[1,2-a]pyrazin-8-yl] amino]-2-methylphenyl]-[4-(4-hydroxypiperidine-4-carbonyl)piperazin-1-yl]methanone formate | 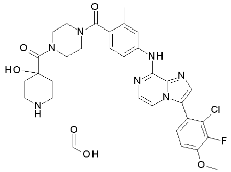 | 622.1 | Intermediate 103 and 1-(tert-butoxycarbonyl)-4-hydroxypiperidine-4-carboxylic acid. Purification: prep. HPLC |
| REF 429 | [4-[[3-(2-chloro-3-fluoro-4-methoxyphenyl)imidazo[1,2-a]pyrazin-8-yl]amino]-2-methyl-phenyl]-[4-[(3S,4R)-3-hydroxypiperidine-4-carbonyl]piperazin-1-yl]methanone formate | 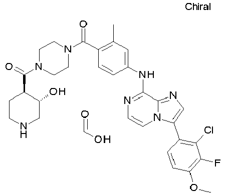 | 622.2 | Intermediate 103 and (3S,4R)-1-tert-butoxycarbonyl-3-hydroxy-piperidine-4-carboxylic acid obtained by saponification of 1,4-Piperidinedicarboxylic acid, 3-hydroxy-, 1-(1,1-dimethylethyl) 4-ethyl ester, (3S,4R)-[CAS# 1805790-50-4]. |
| REF 430 | [4-[[3-(2-chloro-3-fluoro-4-methoxyphenyl)imidazo[1,2-a]pyrazin-8-yl]amino]-2-methyl-phenyl]-[4-[(3S,4S)-3-hydroxypiperidine-4-carbonyl]piperazin-1-yl]methanone | 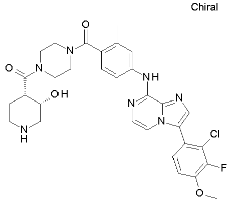 | 622.2 | Intermediate 103 and (3S,4S)-1-tert-butoxycarbonyl-3-hydroxy-piperidine-4-carboxylic acid, obtained by saponification of 1,4-piperidinedicarboxylic acid, 3-hydroxy-, 1-(1,1-dimethylethyl) 4-methyl ester, (3S,4S)-[CAS# 2166250-53-7] |
| REF 431 | [4-[[3-(2-chloro-3-fluoro-4-methoxyphenyl)imidazo[1,2-a]pyrazin-8-yl]amino]-2-methyl-phenyl]-[4-[(3R,4R)-3-hydroxypiperidine-4-carbonyl]piperazin-1-yl]methanone | 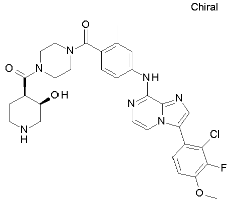 | 622.2 | Intermediate 103 and 1,4-piperidinedicarboxylic acid, 3-hydroxy-, 1-(1, 1-dimethylethyl) ester, (3R,4R)-rel- [CAS# 206111-42-4]. |
| REF 432 | [4-[[3-(2-chloro-3-fluoro-4-methoxyphenyl)imidazo[1,2-a]pyrazin-8-yl] amino]-2-methylphenyl]-[4-[(3R,4S)-3-hydroxypiperidine-4-carbonyl]piperazin-1-yl]methanone formate | 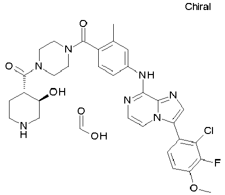 | 622.2 | Intermediate 103 and 1,4-piperidinedicarboxylic acid, 3-hydroxy-, 1-(1,1-dimethylethyl) ester, (3R,4S)- [CAS# 1821775-81-8]. Purification: prep. HPLC |
| REF 433 | N-[2-[[(2R)-2-amino-5-guanidino-pentanoyl]amino]ethyl ]-4-[[3-[4-(difluoromethoxy)-2,3-difluorophenyl]imidazo[1,2-a]pyrazin-8-yl] amino]-2-ethyl-benzamide formate | 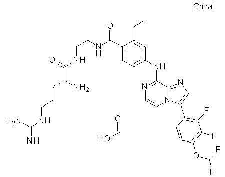 | 659.2 | Intermediate 120 and (2R)-2-(tert-butoxycarbonylamino)-5-guanidino-pentanoic acid hydrate hydrochloride |
| REF 434 | 2-[3-chloro-2-fluoro-4-[8-[4-[4-[(3R,4S)-3-hydroxypiperidine-4-carbonyl]piperazine-1-carbonyl]-3-methyl-anilino]imidazo[1,2-a]pyrazin-3-yl] phenoxy] acetonitril e formate | 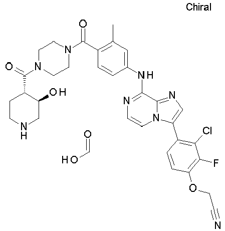 | 647.2 | Intermediate 111 and 1,4-Piperidinedicarboxylic acid, 3-hydroxy-, 1-(1,1-dimethyl ethyl) ester, (3R,4S)- [CAS# 1821775-81-8]. Deprotection: 1h at rt in a 10/1 DCM/TFA mixture Purification: prep. HPLC |
| REF 435 | 2-[3-chloro-2-fluoro-4-[8-[4-[4-[(3S,4R)-3-hydroxypiperidine-4-carbonyl]piperazine-1-carbonyl]-3-methyl-anilino]imidazo[1,2-a]pyrazin-3-yl]phenoxy]acetonitril e formate | 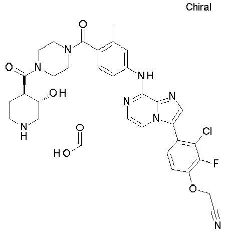 | 647.2 | Intermediate 111 and (3S,4R)-1-tert-butoxycarbonyl-3-hydroxy-piperidine-4-carboxylic acid obtained by saponification of 1,4-Piperidinedicarboxylic acid, 3-hydroxy-, 1-(1,1-dimethylethyl) 4-ethyl ester, (3S,4R)-[CAS# 1805790-50-4]. Deprotection: 1h at rt |
| | | | | in a 10/1 DCM/TFA mixture Purification: prep HPLC (FA as additive) |
| REF 436 | 2-[3-chloro-2-fluoro-4-[8-[4-[4-[(3S,4S)-3-hydroxypiperidine-4-carbonyl]piperazine-1-carbonyl]-3-methyl-anilino]imidazo[1,2-a]pyrazin-3-yl]phenoxy]acetonitril e formate | 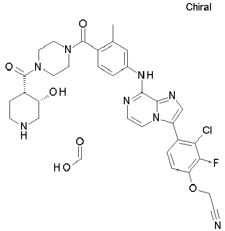 | 647.2 | Intermediate 111 and (3S,4S)-1-tert-butoxycarbonyl-3-hydroxy-piperidine-4-carboxylic acid, obtained by saponification of 1,4-Piperidinedicarboxylic acid, 3-hydroxy-, 1-(1,1-dimethylethyl) 4-methyl ester, (3S,4S)-[CAS# 2166250-53-7]. Deprotection: 1h at rt in a 10/1 DCM/TFA mixture Purification: prep HPLC (FA as additive) |
| REF 437 | 2-[3-chloro-2-fluoro-4-[8-[4-[4-[(3R,4R)-3-hydroxypiperidine-4-carbonyl]piperazine-1-carbonyl]-3-methyl-anilino]imidazo [1,2-a]pyrazin-3-yl]phenoxy]acetonitril e formate | 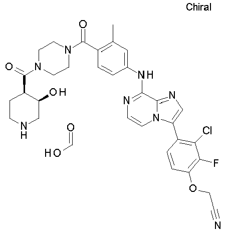 | 647.2 | Intermediate 111 and 1,4-Piperidinedicarboxylic acid, 3-hydroxy-, 1-(1,1-dimethyl ethyl) ester, (3R,4R)-rel-[CAS# 206111-42-4]. Deprotection: 1h at rt in a 10/1 DCM/TFA mixture Purification: prep HPLC (FA as additive) |
| REF 438 | 1-(4-(4-((3-(3-fluoro-4-methoxyphenyl)imida zo[1,2-a]pyrazin-8-yl)amino)-2-methylbenzoyl)pipera zin-1-yl)-2-(methyl amino)ethanon e hydrochloride | 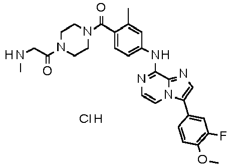 | 532.2 | Reference Example 330 and 2-((tert-butoxycarbonyl)(methy l)amino)acetic acid |
| REF 439 | 1-(3-(4-(4-((3-(4-(difluoromethoxy)phe nyl)imidazo[1,2-a]pyrazin-8-yl)amino)-2-methylbenzoyl)pipera zin-1-yl)-3-oxopropyl)guanidine | 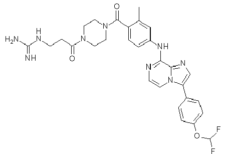 | 592.3 | Reference Example 329 and 3-guanidinopropanoic acid |
| REF 440 | (2S)-2-amino-1-[4-[4-[[3-[4-(difluoromethoxy)phe nyl]imidazo[1,2-a]pyrazin-8-yl] amino]-2-methylbenzoyl]piperazin-1-yl]propan-1-one hydrochloride | 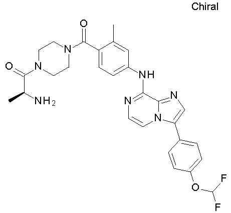 | [M-H]-; 584.4 | Reference Example 329 and N-Boc-L-Alanine |
| REF 441 | (2S,4S)-N-[2-[[4-[[3-[4-(cyanomethoxy)-2,3-difluorophenyl]imidazo[1,2-a]pyrazin-8-yl]amino]-2-methyl-benzoyl]amino]ethyl]-4-hydroxy-4-methyl-pyrrolidine-2-carboxamide formate | 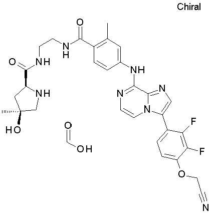 | 605.2 | Intermediate 121 and 1,2-Pyrrolidinedicarboxylic acid, 4-hydroxy-4-methyl-, 1-(1,1-dimethyl ethyl) ester, (2S,4S)-[CAS#1199793-52-6] |
| REF 442 | (2S,4S)-N-[3-[[4-[[3-[4-(cyanomethoxy)-2,3-difluorophenyl]imidazo[1,2-a]pyrazin-8-yl] amino] -2-methylbenzoyl]amino]propyl ]-4-hydroxy-4-methyl-pyrrolidine-2-carboxamide formate | 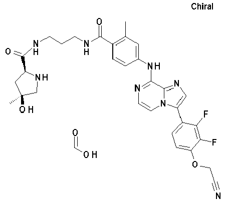 | 619.1 | Intermediate 122 and 1,2-Pyrrolidinedicarboxylic acid, 4-hydroxy-4-methyl-, 1-(1,1-dimethyl ethyl) ester, (2S,4S)-[CAS#1199793-52-6] |
| REF 443 | [4-[[3-(2,3-difluoro-4-methoxyphenyl)imidazo[1,2-a]pyrazin-8-yl] amino]-2-methylphenyl]-[4-[(2S,4S)-4-hydroxypyrro lidine- 2-carbonyl] piperazin-1-yl]methanone | 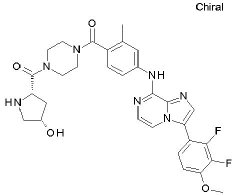 | [M-H]-; 590.0 | Reference Example 232 and (2S,4S)-1-(tert-butoxycarbonyl)-4-hydroxypyrrolidine-2-carboxylic acid [CAS#87691-27-8] |
| REF 444 | [4-[[3-(2,3-difluoro-4-methoxyphenyl)imidazo[1,2-a]pyrazin-8-yl] amino]-2-methylphenyl]-[4-[rac-(2R,4S)-4-hydroxypyrro lidine- 2-carbonyl] piperazin- 1-yl]methanone | 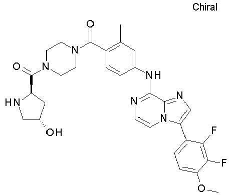 | [M-H]-; 590.4 | Reference Example 232 and (2R,4S)-1-(tert-butoxycarbonyl)-4-hydroxypyrrolidine-2-carboxylic acid [CAS# 147266-92-0] |
| REF 445 | 2-[4-[8-[4-[4-[(2S,4S)-4-ethyl-4-hydroxy-pyrrolidine-2-carbonyl] piperazine-1-carbonyl]-3-methyl-anilino]imidazo[1,2-a]pyrazin-3-yl]-2,3-difluoro-phenoxy]acetonitrile formate | 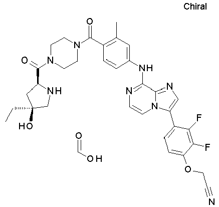 | 645.5 | Intermediate 109 and Intermediate 126 |
| REF 446 | 2-[2,3-difluoro-4-[8-[4-[4-[(2S,4S)-4-hydroxy-4-methyl-pyrrolidine-2-carbonyl]piperazine-1-carbonyl]-3-methyl-anilino]imidazo[1,2-a]pyrazin-3-yl] phenoxy] acetonitril e formate | 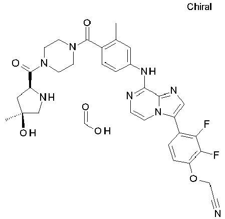 | 631.2 | Intermediate 109 and 1, 2-pyrro lidinedicarboxy lic acid, 4-hydroxy-4-methyl-, 1-(1,1-dimethylethyl) ester, (2S,4S)-[CAS#1199793-52-6] |
| REF 447 | 2-[2,3-difluoro-4-[8-[4-[4-[(2S,4R)-4-hydroxy-4-methyl-pyrrolidine-2-carbonyl]piperazine-1-carbonyl]-3-methyl-anilino]imidazo[1,2-a]pyrazin-3-yl] phenoxy] acetonitril e formate | 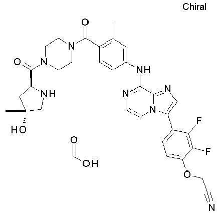 | 631.4 | Intermediate 109 and 1, 2-Pyrro lidinedicarboxy lic acid, 4-hydroxy-4-methyl-, 1-(1,1-dimethylethyl) ester, (2S,4R)-[CAS# 1365970-67-7] |
| REF 448 | 2-[4-[8-[4-[4-[(2S,4R)-4-ethyl-4-hydroxy-pyrrolidine-2-carbonyl]piperazine-1-carbonyl]-3-methyl-anilino]imidazo[1,2-a]pyrazin-3-yl]-2,3-difluoro-phenoxy] acetonitrile formate | 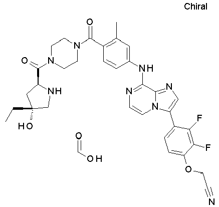 | 645.4 | Intermediate 109 and Intermediate 127 |
| REF 449 | N-[2-[[(2S)-2-amino-3-hydroxy-propanoyl]amino]ethy 1]-4-[[3-[4-(cyanomethoxy)-2,3-difluorophenyl]imidazo[1,2-a]pyrazin-8-yl]amino]-2-ethyl-benzamide formate | 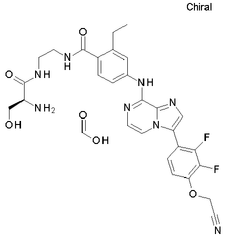 | 579.4 | Intermediate 116 and BOC-SER-OH |
| REF 450 | N-[2-[(2-aminoacetyl)amino] et hyl]-4-[[3-[4-(cyanomethoxy)-2,3-difluorophenyl]imidazo[1,2-a]pyrazin-8-yl] amino]-2-ethyl-benzamide formate | 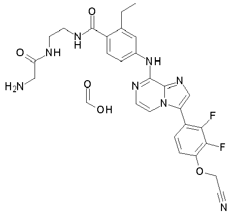 | 549.4 | Intermediate 116 and BOC-glycine |
| REF 451 | N-[2-(3-aminopropanoylamino )ethyl]-4-[[3-[4-(cyanomethoxy)-2,3-difluorophenyl]imidazo[1,2-a]pyrazin-8-yl]amino]-2-ethyl-benzamide formate | 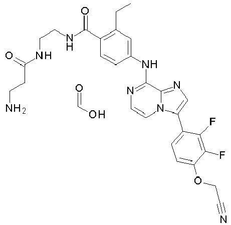 | 563.3 | Intermediate 116 and BOC-BETA-ALA-OH |
| REF 452 | N-[2-[[(2S,3R)-2-amino-3-hydroxy-butanoyl] amino] ethyl] -4-[[3-[4-(cyanomethoxy)-2,3-difluorophenyl]imidazo[1,2-a]pyrazin-8-yl]amino]-2-ethyl-benzamide formate | 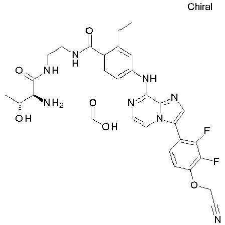 | 593.3 | Intermediate 116 and BOC-THR-OH |
| REF 453 | 4-[[3-(2,3-difluoro-4-methoxyphenyl)imidazo[1,2-a]pyrazin-8-yl]amino]-2-ethyl-N-[2-(5-guanidinopentanoyla mino)ethyl]benzamide formate | 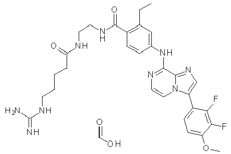 | 608.1 | Reference Example 331 and 5-guanidinopentanoic acid |
| REF 454 | 4-[[3-[4-(cyanomethoxy)-2,3-difluorophenyl]imidazo[1,2-a]pyrazin-8-yl]amino]-N-[2-[[(2S)-2,5-diaminopentanoyl] ami no]ethyl]-2-ethyl-benzamide formate | 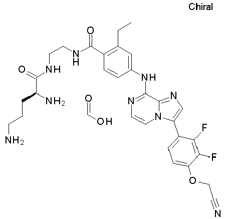 | 606.2 | Intermediate 116 and BOC-ORN(BOC)-OH |
| REF 455 | N-[2-(4-aminobutanoylamino) ethyl]-4-[[3-[4-(cyanomethoxy)-2,3-difluorophenyl]imidazo[1,2-a]pyrazin-8-yl]amino]-2-ethyl-benzamide formate | 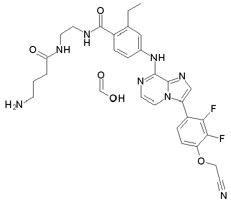 | 577.5 | Intermediate 116 and BOC-gamma-abu-OH |
| REF 456 | N-[2-[(4-amino-3-hydroxy-butanoyl)amino] ethyl] -4-[[3-[4-(cyanomethoxy)-2,3-difluorophenyl]imidazo[1,2-a]pyrazin-8-yl]amino]-2-ethyl-benzamide formate | 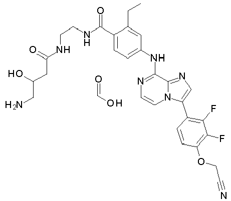 | 593.4 | Intermediate 116 and 4-(tert-butoxycarbonylamino)-3-hydroxy-butanoic acid |
| REF 457 | 4-[[3-[4-(cyanomethoxy)-2,3-difluorophenyl]imidazo[1,2-a]pyrazin-8-yl]amino]-N-[2-[[(2S)-2,6-diaminohexanoyl]ami no]ethyl]-2-ethyl-benzamide formate | 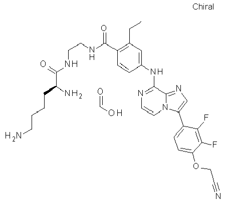 | 620.4 | Intermediate 116 and BOC-LYS(BOC)-OH |
| REF 458 | 4-[[3-[4-(cyanomethoxy)-2,3-difluorophenyl]imidazo[1,2-a]pyrazin-8-yl]amino]-N-[2-[[(2S)-2,4-diaminobutanoyl] ami n o]ethyl]-2-ethyl-benzamide formate | 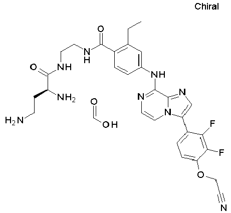 | 592.4 | Intermediate 116 and (2S)-2,4-bis(tert-butoxycarbonylamino) butanoic acid |
| REF 459 | 4-[[3-[4-(cyanomethoxy)-2,3-difluorophenyl]imidazo[1,2-a]pyrazin-8-yl]amino]-N-[2-[[(2S)-2,3-diaminopropanoyl] am ino]ethyl]-2-ethyl-benzamide formate | 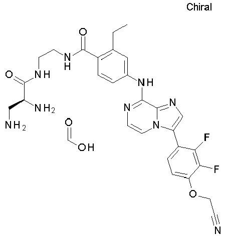 | 578.4 | Intermediate 116 and (2S)-2,3-bis(tert-butoxycarbonylamino) propanoic acid |
| REF 460 | N-[3-(3-aminopropanoylamino )-2-hydroxy-propyl]-4-[[3-(2,3-difluoro-4-methoxyphenyl)imidazo[1,2-a]pyrazin-8-yl] amino]-2-ethyl-benzamide hydrochloride | 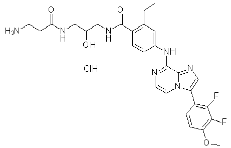 | 568.3 | Reference Example 543 and 3-((tert-butoxycarbonyl)amino) propanoic acid [CAS#3303-84-2] |
| 5 | N-[4-[[(2S)-2-amino-5-guanidino-pentanoyl]amino]cycl ohexyl]-4-[[3-(2,3-difluoro-4-methoxyphenyl)imidazo[1,2-a]pyrazin-8-yl] amino]-2-ethyl-benzamide trifluoroacetate | 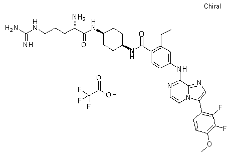 | 677.4 | Example 6 and L-arginine. Deprotection with TFA/DCM 2/1 at rt for 2 h, then precipitated from the reaction mixture by the addition of diethylether. |
| REF 461 | N-[2-[[(2R)-2-amino-5-guanidino-pentanoyl]amino]ethyl ]-4-[[3-[4-(cyanomethoxy)-2,3-difluoro-phenyl]imidazo[1,2-a]pyrazin-8-yl]amino]-2-ethyl-benzamide formate | 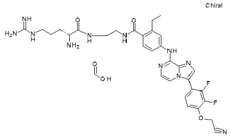 | 648.1 | Intermediate 116 and FMOC-D-ARG-OH. Deprotection with 10 eq piperidine in DCM at rt fro 12h. Purification with prep HPLC (FA) |

### Intermediate 126

### (2S,4S)-1-tert-butoxycarbonyl-4-ethyl-4-hydroxy-pyrrolidine-2-carboxylic acid And Intermediate 127

### (2S,4R)-1-tert-butoxycarbonyl-4-ethyl-4-hydroxy-pyrrolidine-2-carboxylic acid

Ethylmagnesium bromide (7.27 mL, 21.81 mmol, 2.5 eq) was added dropwise to a THF (50 mL) solution of (2S)-1-tert-butoxycarbonyl-4-oxo-pyrrolidine-2-carboxylic acid [CAS#84348-37-8] (2.0 g, 8.72 mmol, 1 eq) at - 20°C under nitrogen atmosphere. The resulting mixture was stirred at the same temperature for 1 h and then further stirred at 0 °C for 10 h. The reaction mixture was poured into 1 N aqueous hydrochloric acid solution (100 mL) under ice cooling, followed by extraction with ethyl acetate. The organic layer was washed with brine and dried over anhydrous Na₂SO₄. The solvent was evaporated under reduced pressure and the crude product was purified by prep. HPLC(FA as additive) to deliver (2S,4S)-1-tert-butoxycarbonyl-4-ethyl-4-hydroxy-pyrrolidine-2-carboxylic acid (Intermediate 126) (0.800 g, 3.09 mmol, 35.36% yield) as off white solid and (2S,4R)-1-tert-butoxycarbonyl-4-ethyl-4-hydroxy-pyrrolidine-2-carboxylic acid (Intermediate 127) (0.200 g, 0.770 mmol, 8.84 % yield) as off white solid.

### Reference Example 462

### ((2S,3R,4S)-3,4-dihydroxypyrrolidin-2-yl)(4-(4-((3-(3-fluoro-4-methoxyphenyl)imidazo[1,2-a]pyrazin-8-yl)amino)-2-methylbenzoyl)piperazin-1-yl)methanone hydrochloride

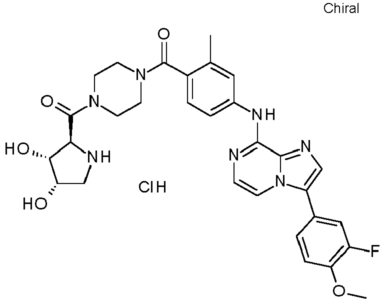

### Step 1: tert-butyl (S)-2-(4-(4-((3-(3-fluoro-4-methoxyphenyl)imidazo[1,2-a]pyrazin-8-yl)amino)-2-methylbenzoyl)piperazine-1-carbonyl)-2,5-dihydro-1H-pyrrole-1-carboxylate

The title compound was prepared in analogy to Reference Example 374 from Reference Example 330 and (S)-1-(tert-butoxycarbonyl)-2,5-dihydro-1H-pyrrole-2-carboxylic acid without cleavage of the Boc-protective group.

MS (ESI) [M+H]⁺: 656.5

### Step 2: tert-butyl (2S,3R,4S)-2-(4-(4-((3-(3-fluoro-4-methoxyphenyl)imidazo[1,2-a]pyrazin-8-yl)amino)-2-methylbenzoyl)piperazine-1-carbonyl)-3,4-dihydroxypyrrolidine-1-carboxylate

tert-butyl (S)-2-(4-(4-((3-(3-fluoro-4-methoxyphenyl)imidazo[1,2-a]pyrazin-8-yl)amino)-2-methylbenzoyl)piperazine-1-carbonyl)-2,5-dihydro-1H-pyrrole-1-carboxylate (50 mg, 76.3 µmol, Eq: 1) was dissolved in a mixture of tert-BuOH (750 µl), tetrahydrofuran (200 µl) and water (50 µl). Osmium tetroxide in water (4%) (48.5 mg, 59.8 µL, 7.63 µmol, Eq: 0.1) was added, followed by 4-methyolmorpholine N-oxide (13.4 mg, 114 µmol, Eq: 1.5). The mixture was stirred overnight. Then additional osmium tetroxide in water (4 %) (48.5 mg, 59.8 µl, 7.63 µmol, Eq: 0.1) and 4-methyolmorpholine N-oxide (13.4 mg, 114 µmol, Eq: 1.5) were added and the mixture was stirred over 72 h. The reaction was quenched by addition of sat. aq. Na₂S₂O₃ and then extracted with 2-MeTHF. The combined organic layers were washed with sat. aq. Na₂S₂O₃ and brine and then concentrated *in vacuo.* The residue was purified by prep. HPLC to obtain the title compound (52.6 mg) as a light brown solid.

MS (ESI) [M+H]⁺: 690.4

### Step 3: ((2S,3R,4S)-3,4-dihydroxypyrrolidin-2-yl)(4-(4-((3-(3-fluoro-4-methoxyphenyl)imidazo[1,2-a]pyrazin-8-yl)amino)-2-methylbenzoyl)piperazin-1-yl)methanone hydrochloride

4M HCl in dioxane (50 µl) was added to tert-butyl (2S,3R,4S)-2-(4-(4-((3-(3-fluoro-4-methoxyphenyl)imidazo[1,2-a]pyrazin-8-yl)amino)-2-methylbenzoyl)piperazine-1-carbonyl)-3,4-dihydroxypyrrolidine-1-carboxylate (8.3 mg, 12 µmol, Eq: 1) in DCM (200 µL). The reaction mixture was stirred overnight and then concentrated *in vacuo* to give the title compound (8.9 mg) as a white solid.MS (ESI) [M+H]⁺: 590.3

The following examples were prepared in analogy to Reference Example 462

| Ex. | Name | Structure | MS ESI [M+H]⁺ | Starting Material |
|---|---|---|---|---|
| REF 463 | (4-(4-((3-(2,3-difl uoro-4-methoxyphenyl)imid azo[1,2-a]pyrazin-8-yl)amino)-2-methylbenzoyl)piper azin-1-yl)((2S,3R,4S)-3,4-dihydroxypyrrolidin-2-yl)methanone | 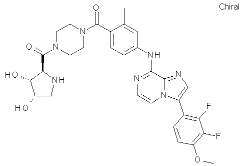 | 608.2 | from Reference Example 232 and (S)-1-(tert- butoxycarbonyl)-2,5-dihydro-1H-pyrrole-2-carboxylic acid |
| REF 464 | (4-(4-((3-(2,3-difl uoro-4-methoxyphenyl)imid azo[1,2-a]pyrazin-8-yl)amino)-2-methylbenzoyl)piper azin-1-yl)((2R,3S,4R)-3,4-dihydroxypyrrolidin-2-yl)methanone | 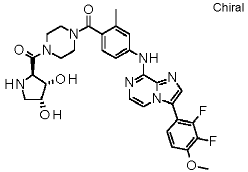 | 608.3 | from Reference Example 232 and (R)-1-(tert-butoxycarbonyl)-2,5-dihydro-1H-pyrrole-2-carboxylic acid |

### Reference Example 465

### rel-(4-(4-((3-(2,3-difluoro-4-methoxyphenyl)imidazo[1,2-a]pyrazin-8-yl)amino)-2-methylbenzoyl)piperazin-1-yl)((3R,4S)-3,4-dihydroxypiperidin-3-yl)methanone

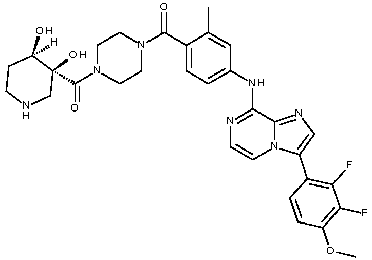

### Step 1: 1-(tert-butoxycarbonyl)-1,2,5,6-tetrahydropyridine-3-carboxylic acid

1,2,5,6-tetrahydropyridine-3-carboxylic acid hydrochloride (1 g, 6.11 mmol, Eq: 1) was combined with dioxane (7.8mL) and water (7.8 mL) to give a orange solution. Then di-tert-butyl dicarbonate (1.47 g, 6.72 mmol, Eq: 1.1) was slowly added as, a solution in dioxane (7.8 mL). After 15min, NaOH (8mL, 8 mmol, Eq: 1.31) was added and the RM stirred at RT overnight. The volatiles were removed, the reaction mixture was poured into 50 mL tBuOMe and extracted with 1 M HCl (2 × 25 mL). The aqueous layer was back-extracted with tBuOMe (2 × 25 mL). The organic layers were combined, washed with sat NaCl (2 x 25 mL), then dried over MgSO₄, filtered and concentrated in vacuo, the crude intermediate was used in the next step without further purification. MS (ESI) [M+H]⁺: 228.0

### Step 2: 1-tert-butyl 5-O-methyl 3,6-dihydro-2H-pyridine-1,5-dicarboxylate

1-(tert-Butoxycarbonyl)-1,2,5,6-tetrahydropyridine-3-carboxylic acid (409 mg, 1.8 mmol, Eq: 1) was dissolved in DMF (9 mL). potassium carbonate (298 mg, 2.16 mmol, Eq: 1.2) and Mel (511 mg, 225 µL, 3.6 mmol, Eq: 2) were successively added and RM was stirred at RT overnight. The RM was concentrated under HV. Residue was dissolved in ethyl acetate, filtered and concentrated under vacuum. MS (ESI) [M+H]⁺: 186.1 (carbamic acid, M-55)

### Step 3: 1-tert-butyl 3-O-methyl (3R,4S)-3,4-dihydroxypiperidine-1,3-dicarboxylate

1-(tert-butyl) 3-methyl 5,6-dihydropyridine-1,3(2H)-dicarboxylate (434 mg, 1.8 mmol, Eq: 1) was dissolved in tBuOH (20 mL). NMO (211 mg, 1.8 mmol, Eq: 1) and 4% osmium tetroxide in water (1.14 g, 1.41 mL, 180 µmol, Eq: 0.1) were successively added. Sodium thiosulfate (1.42 g, 8.99 mmol, Eq: 5) was added to quench the reaction but insoluble in tBuOH. The minimum amount of saturated Na₂S₂O₃ solution was added to solubilize the salt and RM was stirred for 1 h. RM was filtered through a pad of celite and concentrated under vacuum. Purification by combiflash. MS (ESI) [M+H]⁺: 176.1 (M-Boc)

### Step 4: 5-(tert-butyl) 3a-methyl (3aR,7aS)-2,2-dimethyldihydro-[1,3]dioxolo[4,5-c]pyridine-3a,5(4H,6H)-dicarboxylate

To a solution of rac-1-(tert-butyl) 3-methyl (3R,4S)-3,4-dihydroxypiperidine-1,3-dicarboxylate (320 mg, 1.16 mmol, Eq: 1) in DMF (1.16 mL) was added successively 2,2-dimethoxypropane (484 mg, 570 µl, 4.65 mmol, Eq: 4) and pTsOH (22.1 mg, 116 µmol, Eq: 0.1). RM was stirred and heated at 40 °C for 8 h and at 30 °C for 48 h. Purification by column chromatography, solid loaded with 1.2 g of silica, 12 g, heptane/ethyl acetate. Enantiomers were separated by SFC.

MS (ESI) [M+H]⁺: 260.2 (M-tBu)

### Step 5: (3aS,7aR)-5-(tert-butoxycarbonyl)-2,2-dimethyltetrahydro-[1,3]dioxolo[4,5-c] pyridine-3a(4H)-carboxylic acid

To a solution of 5-(tert-butyl) 3a-methyl (3aS,7aR)-2,2-dimethyldihydro-[1,3]dioxolo[4,5-c]pyridine-3a,5(4H,6H)-dicarboxylate (100 mg, 317 µmol, Eq: 1) in THF (1 mL)/MeOH (500 µL) was added LiOH (1 mL, 2 mmol, Eq: 6.31). The RM was stirred at RT for 2 h. Volatiles were removed under vacuum and mixture was put in the freezer overnight. DCM was added and mixture was stirred. Aqueous phase was acidified with ammonium chloride and then with HCl 1M until pH 4. Phases were separated and extraction with 2x 10 mL of DCM. Organic layers were combined, filtered through a pad of MgSO₄, concentrated *in vacuo* to provide an oil. MS (ESI) [M-H]⁻: 300.3

### Step 6: rel-(4-(4-((3-(2,3-difluoro-4-methoxyphenyl)imidazo[1,2-a]pyrazin-8-yl)amino)-2-methylbenzoyl)piperazin-1-yl)((3R,4S)-3,4-dihydroxypiperidin-3-yl)methanone

The title compound was prepared in analogy to Reference Example 374 from Reference Example 232 and rel-(3aR,7aS)-5-(tert-butoxycarbonyl)-2,2-dimethyltetrahydro-[1,3]dioxolo[4,5-c]pyridine-3a(4H)-carboxylic acid. MS (ESI) [M+H]⁺: 622.4

### Intermediate 124:

### tert-butyl (3R)-3-[[tert-butyl(dimethyl)silyl]oxymethyl]piperazine-1-carboxylate

To a solution of tert-butyl (3R)-3-(hydroxymethyl)piperazine-1-carboxylate [CAS# 278788-66-2] (200.0 mg, 0.920 mmol, 1 eq) and imidazole (75.54 mg, 1.11 mmol, 1.2 eq) in DCM (2 mL) was added tert-butyldimethylchlorosilane (153.31 mg, 1.02 mmol, 1.1 eq). The reaction was stirred at 20 °C for 12 h. The reaction was concentrated. The residue was purified by prep-TLC(PE:EtOAc=0:1) to give tert-butyl (3R)-3-[[tert-butyl(dimethyl)silyl]oxymethyl]piperazine-1-carboxylate (180 mg, 0.540 mmol, 58.89 % yield) as colorless oil.

The following Intermediate was prepared in analogy to Intermediate 124

| Int. | Name | MS ESI [M+H]⁺ | Starting Material |
|---|---|---|---|
| 12 5 | tert-butyl (3S)-3-[[tert-butyl(dimethyl)silyl]oxymethyl]piperazine-1-carboxylate | | tert-butyl (3S)-3-(hydroxymethyl)pipera zine-1-carboxylate |

### Intermediate 129

### 2-(4-(8-chloroimidazo[1,2-a]pyrazin-3-yl)-2,3-difluorophenoxy)propanenitrile

To a solution of Intermediate 54 (500mg, 1.78 mmol) in MeCN (30 mL) was added potassium carbonate (491 mg, 3.55 mmol) and 3-bromobutanenitrile (263 mg, 1.78 mmol), the reaction was stirred for 16 h at 60 °C. The reaction mixture was filtered and the filtrate was concentrated in vacuum. The residue was washed with brine and extracted in DCM. The organic layer was dried over anhydrous Na₂SO₄ and concentarted under reduced pressure to give 2-(4-(8-chloroimidazo[1,2-a]pyrazin-3-yl)-2,3-difluorophenoxy)propanenitrile (530 mg, 1.58 mmol, 89.2 % yield) which was directly used for the next step without further purification.

### Intermediate 130

### 4-((3-(4-(1-Cyanoethoxy)-2,3-difluorophenyl)imidazo[1,2-a]pyrazin-8-yl)amino)-2-methylbenzoic acid

To a solution of Intermediate 129 (520mg, 1.55 mmol) in the mixture solvent of MeCN (20mL) and acetic acid (4 mL) was added 4-amino-2-methylbenzoic acid (235 mg, 1.55 mmol), the reaction was stirred for 15 hours at 90 °C. The reaction mixture was cooled to room temperature and filtered. The filter cake was dried in vacuum to give 4-((3-(4-(1-cyanoethoxy)-2,3-difluorophenyl)imidazo[1,2-a]pyrazin-8-yl)amino)-2-methylbenzoic acid (560 mg, 1.25 mmol, 80.2 % yield). MS (ESI) [M+H]⁺: 450.1

### Intermediate 131

### 2-chloro-4-((3-(4-(1-cyanoethoxy)-2,3-difluorophenyl)imidazo[1,2-a]pyrazin-8-yl)amino)benzoic acid

To a solution of Intermediate 129 ( 500mg, 1.49 mmol) in the mixture solvent of MeCN (20mL) and acetic acid (4 mL) was added 4-amino-2-chlorobenzoic acid (256 mg, 1.49 mmol), the reaction was stirred for 15 hours at 90 °C. The reaction mixture was cooled to room temperature and filtered. The filter cake was dried in vacuum to give 2-chloro-4-((3-(4-(1-cyanoethoxy)-2,3-difluorophenyl)imidazo[1,2-a]pyrazin-8-yl)amino)benzoic acid (530mg, 1.13 mmol, 75.5 % yield). MS (ESI) [M+H]⁺: 470.3

### Example REF 466

### 2-Chloro-4-[[3-[4-(1-cyanoethoxy)-2,3-difluoro-phenyl]imidazo[1,2-a]pyrazin-8-yl] amino]-N-(2-piperazin-1-ylethyl)benzamide formate

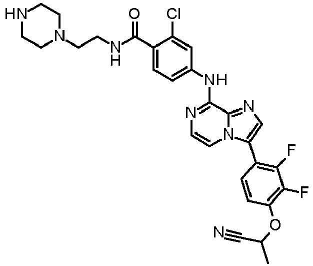

### Step 1: tert-butyl 4-[2-[[2-chloro-4-[[3-[4-(1-cyanoethoxy)-2,3-difluoro-phenyl]imidazo[1,2-a]pyrazin-8-yl]amino]benzoyl]amino]ethyl]piperazine-1-carboxylate

To a solution of Intermediate 131 (100mg, 213 µmol) in DMF (3 mL) was added tert-butyl 4-(2-aminoethyl)piperazine-1-carboxylate (48.8 mg, 213 µmol), triethylamine (43.1 mg, 426 µmol) and 2,4,6-tripropyl-1,3,5,2,4,6-trioxatriphosphinane 2,4,6-trioxide (102 mg, 319 µmol). The reaction was stirred for 30 minutes at room temperature. The mixture was poured into water and filtered. The filter cake was dried in vacuum to give tert-butyl 4-[2-[[2-chloro-4-[[3-[4-(1-cyanoethoxy)-2,3-difluoro-phenyl]imidazo[1,2-a]pyrazin-8-yl]amino]benzoyl]amino]ethyl]piperazine-1-carboxylate (135 mg).

### Step 2: 2-chloro-4-[[3-[4-(1-cyanoethoxy)-2,3-difluoro-phenyl]imidazo[1,2-a]pyrazin-8-yl]amino]-N-(2-piperazin-1-ylethyl)benzamide formate

To a solution of tert-butyl 4-(2-(2-chloro-4-((3-(4-(1-cyanoethoxy)-2,3-difluorophenyl)imidazo[1,2-a]pyrazin-8-yl)amino)benzamido)ethyl)piperazine-1-carboxylate ( 135mg) in THF (10 mL) was added concentrated HCl (2 mL), the reaction was stirred for two hours at room temperature. The reaction mixture was cooled to 0 °C and basified with ammonia. The mixture was extracted in ethyl acetate and the organic layer was concentrated in vacuum. The residue was purified by preparative HPLC to give 2-chloro-4-[[3-[4-(1-cyanoethoxy)-2,3-difluoro-phenyl]imidazo[1,2-a]pyrazin-8-yl]amino]-N-(2-piperazin-1-ylethyl)benzamide (45 mg). MS obsd. (ESI⁺) [(M+H)⁺]: 581

The following examples were prepared in analogy to Example REF 466, the deprotection step 2 was only applied for intermediates derived from Boc-protected amines.

| Ex^{#} | Name | Structure | ESI [M+H]⁺ | Starting Material |
|---|---|---|---|---|
| REF 467 | 2-[4-[8-[4-[4-[3-(dimethylamino)pro pyl]piperazine-1-carbonyl]-3-methyl-anilino]imidazo[1,2-a]pyrazin-3-yl]-2,3-difluoro-phenoxy]propanenitr ile formate | 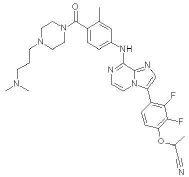 | 603.4 | Intermediate 130 and N,N-dimethyl-3-(piperazin-1-yl)propan-1-amine |
| REF 468 | 2-[4-[8-[4-[4-[2-(dimethylamino)ethy l]piperazine-1-carbonyl]-3-methyl-anilino]imidazo[1,2-a]pyrazin-3-yl]-2,3-difluoro-phenoxy] propanenitr ile; 2,2,2-trifluoroacetic acid | 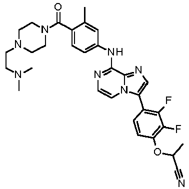 | 589.4 | Intermediate 130 and N,N-dimethyl-2-(piperazin-1-yl)ethan-1-amine |
| REF 469 | 2-[4-[8-[3-chloro-4-[4-[2-(dimethylamino)ethy l]piperazine-1-carbonyl] anilino] imi dazo[1,2-a]pyrazin-3-yl]-2,3-difluoro-phenoxy] propanenitr ile formate | 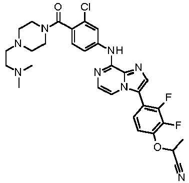 | 609.5 | Intermediate 131 and N,N-dimethyl-2-(piperazin-1-yl)ethan-1-amine |
| REF 470 | 2-[4-[8-[3-chloro-4-[4-[3-(dimethylamino)pro pyl]piperazine-1-carbonyl]anilino]imi dazo[1,2-a]pyrazin-3-yl]-2,3-difluoro-phenoxy]propanenitr ile | 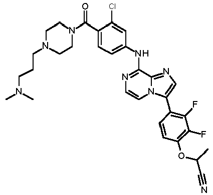 | 623.5 | Intermediate 131 and N,N-dimethyl-3-(piperazin-1-yl)propan-1-amine |
| REF 471 | (2R)-2-[4-[8-[4-[4-[2-(dimethylamino)ethy l]piperazine-1-carbonyl]-3-methyl-anilino]imidazo[1,2-a]pyrazin-3-yl]-2,3-difluoro-phenoxy]propanenitr ile formate | 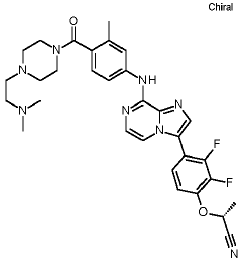 | 589.4 | The compound obtained by chiral separation of Reference Example 468 |
| REF 472 | ((2S)-2-[4-[8-[4-[4-[2-(dimethylamino)ethy l]piperazine-1-carbonyl]-3-methyl-anilino]imidazo[1,2-a]pyrazin-3-yl]-2,3-difluoro-phenoxy] propanenitr ile formate | 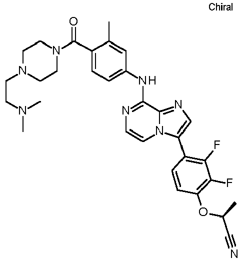 | 589.4 | The compound was obtained by chiral separation of Reference Example 468 |

### Reference Example 473

### 3-(4-(2-(4-((3-(4-(difluoromethoxy)phenyl)imidazo[1,2-a]pyrazin-8-yl)amino)-N,2-dimethylbenzamido)ethyl)piperidin-1-yl)propanoic acid

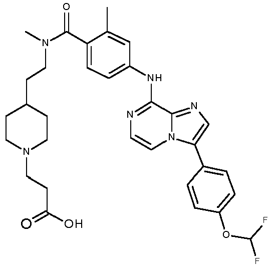

### Step 1)

### N-[2-[1-(2-cyanoethyl)-4-piperidyl]ethyl]-4-[[3-[4-(difluoromethoxy)phenyl]imidazo[1,2-a]pyrazin-8-yl]amino]-N,2-dimethyl-benzamide

4-((3-(4-(difluoromethoxy)phenyl)imidazo[1,2-a]pyrazin-8-yl)amino)-N,2-dimethyl-N-(2-(piperidin-4-yl)ethyl)benzamide (Reference Example 191, 96 mg, 180 µmol, Eq: 1), acrylonitrile (95.3 mg, 1.8 mmol, Eq: 10) and DIPEA (116 mg, 157 µL, 898 µmol, Eq: 5) were combined with dioxane (3mL) and stirred at 100 °C overnight. The reaction mixture was concentrated to dryness and purified by flash chromatography to give a brown viscous oil (53 mg, yield: 54 %). MS (ESI): [M+H]⁺: 588.5

### Step 2)

### 3- [4- [2- [[4-[[3-[4-(difluoromethoxy)phenyl]imidazo[1,2-a]pyrazin-8-yl]amino]-2-methylbenzoyl]-methyl-amino]ethyl]-1-piperidyl]propanoic acid

N-(2-(1-(2-cyanoethyl)piperidin-4-yl)ethyl)-4-((3-(4-(difluoromethoxy)phenyl)imidazo[1,2-a]pyrazin-8-yl)amino)-N,2-dimethylbenzamide (25 mg, 42.5 µmol, Eq: 1) was combined with dioxane (0.25 mL). 2M aq NaOH (425 µL, 851 µmol, Eq: 20) was added, and the reaction mixture was stirred at 100 °C overnight. After cooling down to RT, the reaction mixture was directly acidified with 2M aq HCl solution. The crude product obtained was purified by preparative HPLC. Finally the product was lyophilized to give the target compound as a light brown solid (3.7 mg, yield: 14%). MS (ESI): [M-H]⁻: 605.8

### Intermediate 132

### [4-(2-chloroethyl)piperazin-1-yl]-[2-chloro-4-[[3-(3-fluoro-4-methoxy-phenyl)imidazo[1,2-a] pyrazin-8-yl] amino] phenyl] methanone

2-chloro-4-((3-(3-fluoro-4-methoxyphenyl)imidazo[1,2-a]pyrazin-8-yl)amino)benzoic acid (Intermediate 2) (200 mg, 484 µmol, Eq: 1) was combined with DMF (6mL). DIPEA (188 mg, 254 µl, 1.45 mmol, Eq: 3) and HATU (368 mg, 969 µmol, Eq: 2.00) were added, followed, after stirring at RT for 15 minutes, by addition of 1-(2-chloroethyl)piperazine [CAS 61308-25-6] (108 mg, 727 µmol, Eq: 1.5). After stirring for 3 h at RT, the reaction mixture was poured into 25 mL H₂O and extracted with ethyl acetate. The crude product was used without further purification. MS (ESI): [M+H]⁺: 544.3

### Reference Example 474

### (2-Chloro-4-((3-(3-fluoro-4-methoxyphenyl)imidazo [1,2-a]pyrazin-8-yl)amino)phenyl)(4-(2-((2-hydroxyethyl)amino)ethyl)piperazin-1-yl)methanone

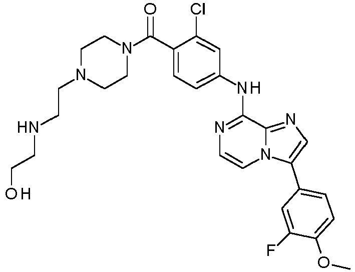

(2-chloro-4-((3-(3-fluoro-4-methoxyphenyl)imidazo[1,2-a]pyrazin-8-yl)amino)phenyl)(4-(2-chloroethyl)piperazin-1-yl)methanone (Intermediate 132) (40 mg, 73.6 µmol, Eq: 1), 2-aminoethan-1-ol (6.72 mg, 110 µmol, Eq: 1.50), sodium carbonate (11.7 mg, 110 µmol, Eq: 1.50), potassium iodide (611 µg, 3.68 µmol, Eq: 0.05) were combined with BuOH (800 µl) and stirred at 105 °C for 24 h. After extraction with DCM/water, the crude material was purified via prep HPLC to give the target compound (6.9 mg, yield: 16 %). MS (ESI): [M+H]⁺: 568.2

### Intermediate 48

### Methyl 4-amino-2-vinylbenzoate

A mixture of methyl 4-amino-2-bromobenzoate (500 mg, 2.17 mmol, Eq: 1), 4,4,5,5-tetramethyl-2-vinyl-1,3,2-dioxaborolane (502 mg, 553 µL, 3.26 mmol, Eq: 1.5), Na₂CO₃ (461 mg, 4.35 mmol, Eq: 2) and 1,1'-bis(diphenylphosphino)ferrocenedichloro palladium(II) dichloromethane complex (159 mg, 217 µmol, Eq: 0.1) in dioxane (6 mL) and water (600 µL) was heated in a microwave at 100 °C for 30 min. The reaction mixture was then poured into 30 mL H₂O and extracted with ethyl acetate (3 × 50 mL). The organic layers were dried over MgSO₄ and concentrated in vacuo. The crude material was purified by flash chromatography.

MS (ESI): [M+H]⁺: 178.2

### Reference Example 475

### (E)-4-((3-(4-(Difluoromethoxy)phenyl)imidazo[1,2-a]pyrazin-8-yl)amino)-N-methyl-2-(prop-1-en-1-yl)benzamide

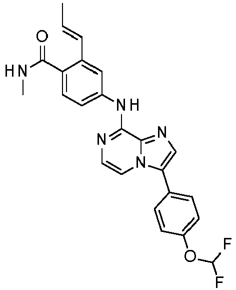

2-Bromo-4-((3-(4-(difluoromethoxy)phenyl)imidazo[1,2-a]pyrazin-8-yl)amino)-N-methylbenzamide (Reference Example 542) (50 mg, 102 µmol, Eq: 1), (E)-prop-1-en-1-ylboronic acid (13.2 mg, 154 µmol, Eq: 1.5), Na₂CO₃ (21.7 mg, 205 µmol, Eq: 2) and 1,1'-bis(diphenylphosphino)ferrocenedichloro palladium(II) dichloromethane complex (7.49 mg, 10.2 µmol, Eq: 0.1) were combined in dioxane (1.5 mL) and water (150 µL) and heated in the microwave at 90 °C for 30 min. The reaction mixture was poured into 50 mL H₂O and extracted with ethyl acetate ( 3 × 75 mL).The organic layers were dried over MgSO₄ and concentrated in vacuo. The crude material was purified by prep HPLC to give the target compound (68 %). MS (ESI): [M+H]⁺: 450.2

### Reference Example 476

### 4-((3-(4-(Difluoromethoxy)phenyl)imidazo[1,2-a]pyrazin-8-yl)amino)-N-methyl-2-propylbenzamide

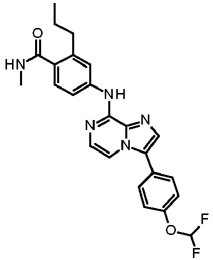

4-((3-(4-(Difluoromethoxy)phenyl)imidazo[1,2-a]pyrazin-8-yl)amino)-N-methyl-2-propylbenzamide (Reference Example 475) (20.8 mg, 46.1 µmol, 76.7 % yield) was dissolved in MeOH and palladium on carbon was added. The reaction was stirred under hydrogen. The reaction mixture was carefully filtered under argon through Celite. MS (ESI): [M+H]⁺: 452.1

### Intermediate 133

### N-(2,2-Diethoxyethyl)-4-((3-(4-methoxyphenyl)imidazo[1,2-a]pyrazin-8-yl)amino)-N,2-dimethylbenzamide

4-((3-(4-methoxyphenyl)imidazo[1,2-a]pyrazin-8-yl)amino)-2-methylbenzoic acid (Intermediate 4) (150 mg, 401 µmol, Eq: 1) and 2,2-diethoxy-N-methylethanamine (70.8 mg, 481 µmol, Eq: 1.20) were combined with DMF (4.5 mL). HATU (305 mg, 801 µmol, Eq: 2.00) and DIPEA (155 mg, 210 µl, 1.2 mmol, Eq: 3.00) were added, and the reaction mixture was stirred at RT. The reaction mixture was directly purified by flash chromatography (reverse phase, 20 g, 0% to 100% acetonitrile in water). MS (ESI): [M+H]⁺: 504.3

### Reference Example 477

### N-((5-Amino-1,3-dioxan-2-yl)methyl)-4-((3-(4-methoxyphenyl)imidazo[1,2-a]pyrazin-8-yl)amino)-N,2-dimethylbenzamide

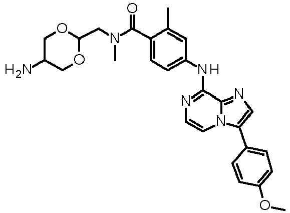

N-(2,2-Diethoxyethyl)-4-((3-(4-methoxyphenyl)imidazo[1,2-a]pyrazin-8-yl)amino)-N,2-dimethylbenzamide (Intermediate 133) (100 mg, 199 µmol, Eq: 1) and benzyl (1,3-dihydroxypropan-2-yl)carbamate (47 mg, 209 µmol, Eq: 1.05) were combined with toluene (1.5 mL), pTsOH (1.89 mg, 9.93 µmol, Eq: 0.05) was added, and the reaction mixture was stirred at reflux overnight. After cooling down to RT, the reaction mixture was concentrated to dryness, and purified by flash chromatography. MS (ESI): [M+H]⁺: 637.4.

The product obtained was dissolved in MeOH, palladium on carbon 10% was added and the reaction mixture obtained was stirred under hydrogen. The crude was purified by flash chromatography. MS (ESI): [M+H]⁺: 503.3

The following examples were prepared in analogy to Example REF 477

| Exa mple | Name | Structure | MS ESI [M+H]⁺ | Starting Material |
|---|---|---|---|---|
| 7 | N-(2-(aminomethyl)-1,3-dioxan-5-yl)-4-((3-(4-methoxyphenyl)imid azo[1,2-a]pyrazin-8-yl)amino)-2-methyl benzamide | 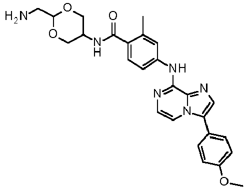 | 489.2 | Intermediate 142 and benzyl (2,2-diethoxyethyl)carbam ate |

### Intermediate 142

### N-(1,3-Dihydroxypropan-2-yl)-4-((3-(4-methoxyphenyl)imidazo[1,2-a]pyrazin-8-yl)amino)-2-methylbenzamide

4-((3-(4-Methoxyphenyl)imidazo[1,2-a]pyrazin-8-yl)amino)-2-methylbenzoic acid (Intermediate 4) (150 mg, 401 µmol, Eq: 1) and 2-aminopropane-1,3-diol (36.5 mg, 401 µmol, Eq: 1.50) were combined with DMF (3 mL) to give a light yellow suspension. HATU (152 mg, 401 µmol, Eq: 1.50) and DIPEA (104 mg, 140 µl, 801 µmol, Eq: 3.00) were added, and the reaction mixture was stirred at RT. The reaction mixture was directly purified by flash chromatography (reverse phase, 20g, 0% to 100% acetonitrile in water). MS (ESI): [M+H]⁺: 448.2.

### Reference Example 478

### 1-(2-Ethyl-4-((3-(3-fluoro-4-methoxyphenyl)imidazo[1,2-a]pyrazin-8-yl)amino)benzoyl)piperidine-4-carboxylic acid

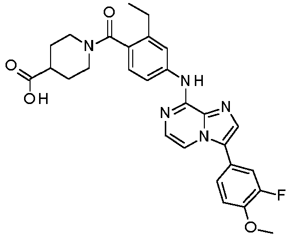

This example was prepared in analogy to Reference Example 1 from Intermediate 106. MS (ESI): [M+H]⁺: 518.3

### Reference Example 479

### N-(2-((2-Aminoethyl)sulfonyl)ethyl)-4-((3-(4-(difluoromethoxy)phenyl)imidazo[1,2-a]pyrazin-8-yl)amino)-2-methylbenzamide

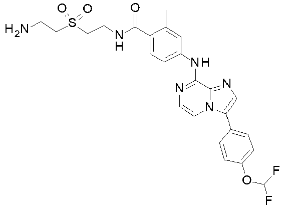

A solution of 3-chloroperoxybenzoic acid (19.9 mg, 80.7 µmol, Eq: 2.2) in DCM (2 mL) was added slowly to a solution of N-(2-((2-aminoethyl)thio)ethyl)-4-((3-(4-(difluoromethoxy)phenyl)imidazo[1,2-a]pyrazin-8-yl)amino)-2-methylbenzamide (Intermediate 84) (18.8 mg, 36.7 µmol, Eq: 1) in DCM (2 mL) at -78 °C under Ar. The mixture was stirred at - 78 °C for 1h and then warmed to RT. The reaction mixture was poured into 5 mL 1 M NaOH and extracted with DCM (5 × 20 mL).The organic layers were dried over sodium sulphate and concentrated in vacuo. MS (ESI): [M+H]⁺: 545.2

### Reference Example 480

### N-(2-((2-Aminoethyl)sulflnyl)ethyl)-4-((3-(4-(difluoromethoxy)phenyl)imidazo[1,2-a]pyrazin-8-yl)amino)-2-methylbenzamide

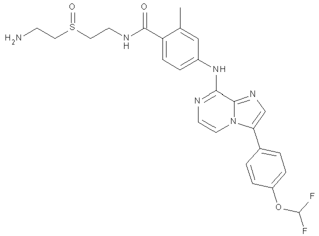

A solution of 3-chloroperoxybenzoic acid (7.4 mg, 33 µmol, Eq: 0.9) in DCM (2 mL) was added slowly to a solution of N-(2-((2-aminoethyl)thio)ethyl)-4-((3-(4-(difluoromethoxy)phenyl)imidazo[1,2-a]pyrazin-8-yl)amino)-2-methylbenzamide (84-001) (18.8 mg, 36.7 µmol, Eq: 1) in DCM (2 mL) at -78 °C under Ar. The mixture was stirred at -78 °C for 1h. The RM was quenched at -78 °C with NaOH. The reaction mixture was poured into 5 mL 1 M NaOH and extracted with DCM (3 × 20 mL). The organic layers were dried over Na₂SO₄ and concentrated *in vacuo.* MS (ESI): [M+H]⁺: 529.2

### Intermediate 107

### 2-(4-(Difluoromethoxy)-2,3-difluorophenyl)-4,4,5,5-tetramethyl-1,3,2-dioxaborolane

### Step1: 1-bromo-4-(difluoromethoxy)-2,3-difluorobenzene

4-bromo-2,3-difluorophenol (500 mg, 2.39 mmol, Eq: 1), sodium 2-chloro-2,2-difluoroacetate (730 mg, 4.78 mmol, Eq: 2) and potassium carbonate (397 mg, 2.87 mmol, Eq: 1.2) were dissolved in DMF (6 mL) and water (1.5 mL). The reaction mixture was heated to 100 °C and stirred for 3 h. The reaction mixture was poured into 20 mL sat NaHCO₃ and extracted with DCM (5 × 40 mL). The organic layers were dried over Na₂SO₄ and concentrated in vacuo. The crude material was purified by flash chromatography.

### Step2: 2-(4-(difluoromethoxy)-2,3-difluorophenyl)-4,4,5,5-tetramethyl-1,3,2-dioxaborolane

1-bromo-4-(difluoromethoxy)-2,3-difluorobenzene (240 mg, 927 µmol, Eq: 1), 4,4,4',4',5,5,5',5'-octamethyl-2,2'-bi(1,3,2-dioxaborolane) (282 mg, 1.11 mmol, Eq: 1.2), potassium acetate (182 mg, 1.85 mmol, Eq: 2) and dichlorobis(triphenylphosphine)palladium(II) (32.5 mg, 46.3 µmol, Eq: 0.05) were dissolved in dioxane (1 mL). The reaction mixture was heated to 100 °C and stirred for O/N. The crude material was purified by flash chromatography (silica gel, 40 g, 0% to 35% ethyl acetate in heptane). MS (ESI): [M+H]⁺: 306.1

### Intermediate 88

### 2-(2-chloro-4-(4,4,5,5-tetramethyl-1,3,2-dioxaborolan-2-yl)phenoxy)acetonitrile

2-(4-bromo-2-chlorophenoxy)acetonitrile (500 mg, 2.03 mmol, Eq: 1), 4,4,4',4',5,5,5',5'-octamethyl-2,2'-bi(1,3,2-dioxaborolane) (567 mg, 2.23 mmol, Eq: 1.10), potassium acetate (398 mg, 4.06 mmol, Eq: 2.00) and bis(triphenylphosphine)palladium(II)chloride (71.2 mg, 101 µmol, Eq: 0.05) were combined with dioxane (7.5 mL). After degassing with N₂, the reaction mixture was heated to 100 °C and stirred overnight. The reaction mixture was cooled to RT, adsorbed on Isolute HM-N and after evaporation to dryness, the crude material was purified by flash chromatography (silica gel, 40 g, 0% to 80% ethyl acetate in heptane). MS (ESI): [M+H]⁺: 293.9

### Reference Example 481

### (2-(4-((3-(2,3-difluoro-4-methoxyphenyl)imidazo[1,2-a]pyrazin-8-yl)amino)-2-ethylbenzamido)ethyl)glycine compound with 2,2,2-trifluoroacetic acid

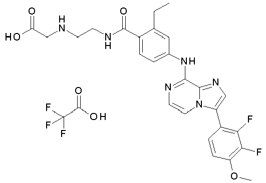

### Step1: tert-butyl (2-(4-((3-(2,3-difluoro-4-methoxyphenyl)imidazo[1,2-a]pyrazin-8-yl)amino)-2-ethylbenzamido)ethyl)glycinate

N-(2-aminoethyl)-4-((3-(2,3-difluoro-4-methoxyphenyl)imidazo[1,2-a]pyrazin-8-yl)amino)-2-ethylbenzamide hydrochloride (Intermediate REF 331) (40 mg, 79.5 µmol, Eq: 1) was combined with DMF (600 µl). TEA (32.2 mg, 44.3 µl, 318 µmol, Eq: 4.00) was added dropwise, followed by addtion of tert-butyl 2-chloroacetate (13.2 mg, 12.5 µL, 87.5 µmol, Eq: 1.10) . The reaction mixture was stirred at RT for 24h. The crude material was purified by prep HPLC. MS (ESI): [M+H]⁺: 581.4

### Step2: 2 (2-(4-((3-(2,3-difluoro-4-methoxyphenyl)imidazo[1,2-a]pyrazin-8-yl)amino)-2-ethylbenzamido)ethyl)glycine compound trifluoroacetate

tert-Butyl (2-(4-((3-(2,3-difluoro-4-methoxyphenyl)imidazo[1,2-a]pyrazin-8-yl)amino)-2-ethylbenzamido)ethyl)glycinate (15 mg, 25.8 µmol, Eq: 1) was combined with DCM (200 µL). TFA (29.5 mg, 19.9 µL, 258 µmol, Eq: 10.0) was added, and the reaction mixture was stirred at RT. The reaction mixture was concentrated to dryness, and lyophilized.

MS (ESI): [M+H]⁺: 525.2

The following examples were prepared in analogy to Reference Example 481

| Ex# | Name | Structure | MS ESI [M+H]⁺ | Starting Material |
|---|---|---|---|---|
| REF 482 | (3-(4-((3-(2,3-difluoro-4-methoxyphenyl)imi dazo[1,2-a]pyrazin-8-yl)amino)-2-ethyl benzamido)pr opyl)glycine compound trifluoroacetate | 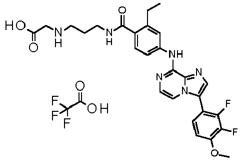 | 539.2 | From Intermediate 87 and tert-butyl 2-chloroacetate |
| REF 483 | (5-(4-((3-(2,3-difluoro-4-methoxyphenyl)imi dazo[1,2-a]pyrazin-8-yl)amino)-2-ethylbenzamido)pe ntyl)glycine hydrochloride | 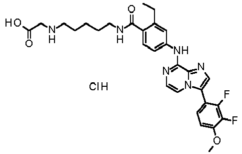 | 567.2 | From Intermediate REF 333 and tert-butyl 2-chloroacetate |

### Intermediate 135

### N-(2-(2-chloroethoxy)ethyl)-4-((3-(4-(difluoromethoxy)-2,3-difluorophenyl)imidazo[1,2-a]pyrazin-8-yl)amino)-2-ethylbenzamide

To a solution of 4-((3-(4-(difluoromethoxy)-2,3-difluorophenyl)imidazo[1,2-a]pyrazin-8-yl)amino)-2-ethylbenzoic acid (75 mg, 163 µmol, Eq: 1) in DMF (815 µl) was added DIPEA (84.2 mg, 114 µl, 652 µmol, Eq: 4) and HATU (124 mg, 326 µmol, Eq: 2). RM was stirred for 15 min. Then 2-(2-chloroethoxy)ethan-1-amine hydrochloride (26.1 mg, 163 µmol, Eq: 1) was added and the RM was stirred overnight. The RM was purified via prep HPLC. MS (ESI): [M+H]⁺: 566.3

The following intermediates were prepared in analogy to Intermediate 135

| Int. | Name | MS ESI [M+H ]⁺ | Starting Material |
|---|---|---|---|
| 13 6 | N-(2-(2-chloroethoxy)ethyl)-4-((3-(2,3-difluoro-4-methoxyphenyl)imidazo**[**1,2-a]pyrazin-8-yl)amino)-2-ethylbenzamide | 530.2 | From 86 and 2-(2-chloroethoxy)ethan-1 - amine hydrochloride |

### Reference Example 484

### N-(2-(2-(4-((3-(4-(Difluoromethoxy)-2,3-difluorophenyl)imidazo[1,2-a]pyrazin-8-yl)amino)-2-ethylbenzamido)ethoxy)ethyl)-N-methylglycine trifluoroacetate

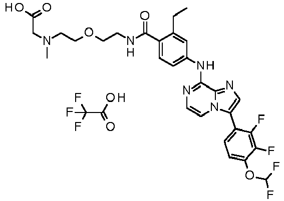

### Step1: tert-Butyl N-(2-(2-(4-((3-(4-(difluoromethoxy)-2,3-difluorophenyl)imidazo[1,2-a]pyrazin-8-yl)amino)-2-ethylbenzamido)ethoxy)ethyl)-N-methylglycinate

A mixture of N-(2-(2-chloroethoxy)ethyl)-4-((3-(4-(difluoromethoxy)-2,3-difluorophenyl)imidazo[1,2-a]pyrazin-8-yl)amino)-2-ethylbenzamide (Intermediate 135) (50 mg, 88.3 µmol, Eq: 1), tert-butyl methylglycinate (25.7 mg, 177 µmol, Eq: 2), K₂CO₃ (24.4 mg, 177 µmol, Eq: 2) and KI (14.7 mg, 88.3 µmol, Eq: 1) in MeCN/dioxane was heated at 90 °C for 2 days. Purification by flash chromatography. MS (ESI): [M+H]⁺: 675.4

### Step2: N-(2-(2-(4-((3-(4-(difluoromethoxy)-2,3-difluorophenyl)imidazo[1,2-a]pyrazin-8-yl)amino)-2-ethylbenzamido)ethoxy)ethyl)-N-methylglycine compound trifluoroacetate

2,2,2-Trifluoroacetic acid (298 mg, 200 µL, 2.61 mmol, Eq: 36.7) was added to a solution of tert-butyl N-(2-(2-(4-((3-(4-(difluoromethoxy)-2,3-difluorophenyl)imidazo[1,2-a]pyrazin-8-yl)amino)-2-ethylbenzamido)ethoxy)ethyl)-N-methylglycinate (48 mg, 71.1 µmol, Eq: 1) in DCM (200 µL). RM was stirred for 24 h. The reaction mixture was concentrated to dryness, and lyophilized. MS (ESI): [M+H]⁺: 619.4

The following examples were prepared in analogy to Reference Example 484

| Ex# | Name | Structure | MS ESI [M+H]⁺ | Starting Material |
|---|---|---|---|---|
| REF 485 | N-(2-(2-(4-((3-(2,3-difluoro-4-methoxyphenyl)imi dazo[1,2-a]pyrazin-8-yl)amino)-2-ethylbenzamido)eth oxy)ethyl)-N-methyl glycine hydrochloride | 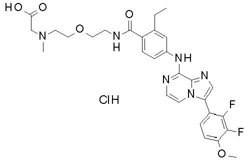 | 583.3 | From Intermediate 136 and tert-butyl methylglycinate. Deprotection with HCl |

### Reference Example 486

### 1-(4-((3-(4-(cyanomethoxy)-2,3-difluorophenyl)imidazo[1,2-a]pyrazin-8-yl)amino)-2-methylbenzoyl)-N-(3-(methylamino)propyl)piperidine-4-carboxamide

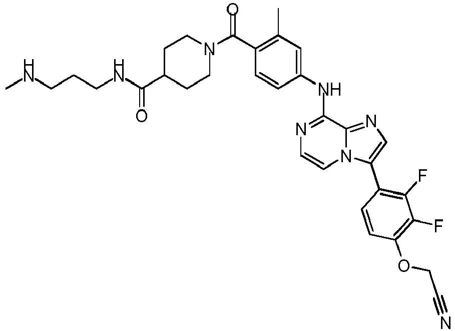

### Step 1: benzyl 4-((3-((tert-butoxycarbonyl)(methyl)amino)propyl)carbamoyl)piperidine-1-carboxylate

To a stirred solution of 1-[(benzyloxy)carbonyl]piperidine-4-carboxylic acid (500.0 mg, 1.9 mmol, 1 eq) and N-(3-aminopropyl)-N-methylcarbamic acid tert-butyl ester (357.53 mg, 1.9 mmol, 1 eq) in DMF (5 mL) was added O-(7-azabenzotriazol-1-yl)-N,N,N',N'-tetramethyluronium hexafluorophosphate (HATU) (1444.15 mg, 3.8 mmol, 2 eq) and N,N-diisopropylethylamine (1.32 mL, 7.6 mmol, 4 eq) in 20 °C and the mixture stirred at 20 °C for 4 h.The reaction was quenched by water and extracted with EA (20 mLx2), and the combined organic layers were concentrated under reduce pressure. The residue was purified by flash chromatography to get the product benzyl 4-[3-[tert-butoxycarbonyl(methyl)amino]propylcarbamoyl]piperidine-1-carboxylate (640 mg, 1.48 mmol, 77.73% yield) as a yellow oil. (ESI⁺) [(M+23)⁺]: 456.3

### Step 2: tert-butyl methyl(3-(piperidine-4-carboxamido)propyl)carbamate

To a solution of benzyl 4-[3-[tert-butoxycarbonyl(methyl)amino]propylcarbamoyl]piperidine-1-carboxylate (500.0 mg, 1.15 mmol, 1 eq) in methanol (10 mL) was added Pd/C (50.0 mg, 1.15 mmol, 1 eq) slowly at 20 °C, the mixture was stirred at 20 °C for 16 h under H₂ atmosphere, the mixture was filtered and concentrated to get the product tert-butyl N-methyl-N-[3-(piperidine-4-carbonylamino)propyl]carbamate (360 mg, 1.2 mmol, 88.62 % yield) as a yellow oil in crude form. (ESI⁺) [(M+1)⁺]: 300.2

### Step 3: tert-butyl methyl(3-(1-(2-methyl-4-nitrobenzoyl)piperidine-4-carboxamido)propyl)carbamate

To a solution of 2-methyl-4-nitro-benzoic acid (254.11 mg, 1.4 mmol, 1.2 eq) and 2-methyl-4-nitro-benzoic acid (254.11 mg, 1.4 mmol, 1.2 eq) in DMF (5 mL), was added N,N-diisopropylethylamine (0.2 mL, 1.17 mmol, 1 eq) and O-(7-azabenzotriazol-1-yl)-N,N,N',N'-tetramethyluronium hexafluorophosphate (444.48 mg, 1.17 mmol, 1 eq) at 20 °C, the mixture was stirred at 20 °C for 4 h, the mixture was concentrated to get a residue, which was purified by flash chromatography to get the title compound (300 mg, 0.650 mmol, 47.16% yield) as a white solid. (ESI⁺) [(M+23)⁺] : 485.2

### Step 4: tert-butyl (3-(1-(4-amino-2-methylbenzoyl)piperidine-4-carboxamido)propyl)(methyl)carbamate

To a solution of tert-butyl N-methyl-N-[3-[[1-(2-methyl-4-nitro-benzoyl)piperidine-4-carbonyl]amino]propyl]carbamate (50.0 mg, 0.110 mmol, 1 eq) in methanol (10 mL) was added Pd/C (5.0 mg, 0.110 mmol, 1 eq) at 20 °C, the mixture was stirred at 20 °C for 16 h under H₂. The mixture was filtered and concentrated and the residue was purified by prep-TLC (DCM:MeOH = 10:1) to give the title compound (25 mg, 0.060 mmol, 53.47% yield) as a colorless oil. (ESI⁺) [(M+23)⁺] : 455.2

### Step 5: 2-(4-bromo-2,3-difluorophenoxy)acetonitrile

To a mixture of bromoacetonitrile (2.3 g, 19.14 mmol, 2 eq) and potassium carbonate (2.65 g, 19.14 mmol, 2 eq) in DMF (25 mL) was added bromoacetonitrile (2.3 g, 19.14 mmol, 2 eq) and the mixture was stirred for 12 h at 25 °C. The reaction was diluted with water (100 mL) and extracted with ethyl acetate (75 mLx2). The combined organic layers were washed with 50 mL water and 50mL saturated brine sequentially, dried by MgSO₄ and concentrated to dryness. The crude product was then purified by flash column chromatography eluting 20% ethyl acetate in petroleum ether to give the desired product as light yellow oil. ¹H NMR (400 MHz, CHLOROFORM-d) δ = 7.39 - 7.31 (m, 1H), 6.91 - 6.81 (m, 1H), 4.87 (d, *J*=1.3 Hz, 2H) ppm.

### Step 6: 2-(2,3-difluoro-4-(4,4,5,5-tetramethyl-1,3,2-dioxaborolan-2-yl)phenoxy)acetonitrile

The title compound was obtained in analogy to **step 4** in the preparation of Intermediate 27 using 2-(4-bromo-2,3-difluorophenoxy)acetonitrile, used in crude form.

### Step 7: 2-(4-(8-chloroimidazo[1,2-a]pyrazin-3-yl)-2,3-difluorophenoxy)acetonitrile

The title compound was obtained in analogy to **step 5** in the preparation of Intermediate 27 using 2-(2,3-difluoro-4-(4,4,5,5-tetramethyl-1,3,2-dioxaborolan-2-yl)phenoxy)acetonitrile. (ESI⁺) [(M+1)⁺] : 321.0

### Step 8: tert-butyl (3-(1-(4-((3-(4-(cyanomethoxy)-2,3-difluorophenyl)imidazo[1,2-a]pyrazin-8-yl)amino)-2-methylbenzoyl)piperidine-4-carboxamido)propyl)(methyl)carbamate

To a solution of 2-[4-(8-chloroimidazo[1,2-a]pyrazin-3-yl)-2,3-difluoro-phenoxy]acetonitrile (18.53 mg, 0.060 mmol, 1 eq) and tert-butyl N-[3-[[1-(4-amino-2-methyl-benzoyl)piperidine-4-carbonyl]amino]propyl]-N-methyl-carbamate (25.0 mg, 0.060 mmol, 1 eq) in tert-butanol (2 mL) was added potassium carbonate (15.98 mg, 0.120 mmol, 2 eq) and Brettphos Pd G3 (2.62 mg, 0 mmol, 0.050 eq) at 20 °C, the mixture was stirred at 80 °C for 4 h, the mixture was filtered and concentrated to give the title compound (20 mg, 0.030 mmol, 44.42 % yield) as a yellow oil. (ESI⁺) [(M+1)⁺] : 717.3

### Step 9: 1-(4-((3-(4-(cyanomethoxy)-2,3-difluorophenyl)imidazo[1,2-a]pyrazin-8-yl)amino)-2-methylbenzoyl)-N-(3-(methylamino)propyl)piperidine-4-carboxamide

The title compound was obtained in analogy to step 2, Reference Example 12 using tert-butyl (3-(1-(4-((3-(4-(cyanomethoxy)-2,3-difluorophenyl)imidazo[1,2-a]pyrazin-8-yl)amino)-2-methylbenzoyl)piperidine-4-carboxamido)propyl)(methyl)carbamate. (ESI⁺) [(M+1)]⁺: 617.2

### Reference Example 487

### N-(2-(2-aminoethoxy)ethyl)-4-((3-(4-(cyanomethoxy)-2,3-difluorophenyl)imidazo[1,2-a]pyrazin-8-yl)amino)-2-ethylbenzamide

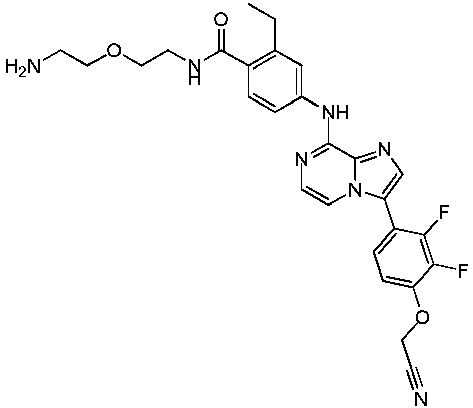

### Step 1: methyl 4-nitro-2-vinylbenzoate

A solution of methyl 2-bromo-4-nitro-benzoate (15.0 g, 57.68 mmol, 1 eq), vinylboronic acid pinacol ester (13.33 g, 86.53 mmol, 1.5 eq), potassium phosphate (14.33 mL, 173.05 mmol, 3 eq) and 1,1'-bis(di-tert-butylphosphino)ferrocene palladium dichloride (3.76 g, 5.77 mmol, 0.100 eq) in toluene (150 mL) and water (5 mL) was heated to 100 °C for 15 h under nitrogen atmosphere. The reaction mixture was concentrated and the residue was purified by silica gel chromatography eluting with petroleum ether/ethyl acetate = 50:1 to afford product methyl 4-nitro-2-vinyl-benzoate (6.2 g, 29.93 mmol, 51.88% yield) as a light yellow solid.

### Step 2: 4-nitro-2-vinylbenzoic acid

To a solution of methyl 4-nitro-2-vinyl-benzoate (2.0 g, 9.65 mmol, 1 eq) in THF (25 mL) and water (5 mL) was added lithium hydroxide hydrate (0.81 g, 19.31 mmol, 2 eq). The resulting mixture was stirred at 20 °C for 15 h. Then most solvent was removed, the mixture was neutralized with 3 N HCl and extracted with DCM, the obtained organic layer was dried over Na₂SO₄ and concentrated to afford 4-nitro-2-vinyl-benzoic acid (1.68 g, 8.7 mmol, 90.1 % yield) as a yellow solid, which was used directly in next step without further purification.

### Step 3: tert-butyl (2-(2-(4-nitro-2-vinylbenzamido)ethoxy)ethyl)carbamate

The title compound was obtained in analogy to step 3, Reference Example 486 using tert-butyl (2-(2-aminoethoxy)ethyl)carbamate and 4-nitro-2-vinylbenzoic acid. (ESI⁺) [(M+23)⁺] : 402.3

### Step 4: tert-butyl (2-(2-(4-amino-2-ethylbenzamido)ethoxy)ethyl)carbamate

The title compound was obtained in analogy to step 4 in Reference Example 486 using tert-butyl (2-(2-(4-nitro-2-vinylbenzamido)ethoxy)ethyl)carbamate. (ESI⁺) [(M+23)]⁺: 374.1

### Step 5: tert-butyl (2-(2-(4-((3-(4-(cyanomethoxy)-2,3-difluorophenyl)imidazo[1,2-a]pyrazin-8-yl)amino)-2-ethylbenzamido)ethoxy)ethyl)carbamate

The title compound was obtained in analogy to step 8 in Reference Example 486 using 2-(4-(8-chloroimidazo[1,2-a]pyrazin-3-yl)-2,3-difluorophenoxy)acetonitrile and tert-butyl (2-(2-(4-amino-2-ethylbenzamido)ethoxy)ethyl)carbamate. (ESI⁺) [(M+1)⁺] : 636.1

### Step 6: N-(2-(2-aminoethoxy)ethyl)-4-((3-(4-(cyanomethoxy)-2,3-difluorophenyl)imidazo[1,2-a]pyrazin-8-yl)amino)-2-ethylbenzamide

The title compound was obtained in analogy to step 2, Reference Example 12 using tert-butyl (2-(2-(4-((3-(4-(cyanomethoxy)-2,3-difluorophenyl)imidazo[1,2-a]pyrazin-8-yl)amino)-2-ethylbenzamido)ethoxy)ethyl)carbamate. (ESI⁺) [(M+1)⁺]: 536.4

### Reference Example 488

### 1-(2-bromo-4-((3-(3-fluoro-4-methoxyphenyl)imidazo[1,2-a]pyrazin-8-yl)amino)benzoyl)-N-(2-(methylamino)ethyl)piperidine-4-carboxamide; formic acid

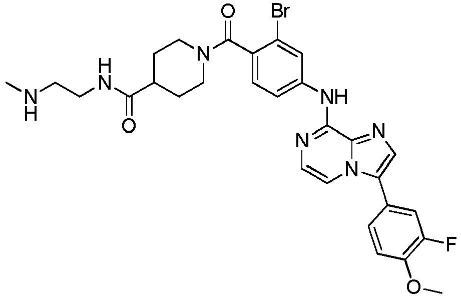

### Step 1: benzyl 4-((2-((tert-butoxycarbonyl)(methyl)amino)ethyl)carbamoyl)piperidine-1-carboxylate; formic acid

To a solution of 1-[(benzyloxy)carbonyl]piperidine-4-carboxylic acid (1.0 g, 3.8 mmol, 1 eq) in DCM (30 mL) was added N-(2-aminoethyl)-N-methyl carbamic acid tert-butyl ester (727.96 mg, 4.18 mmol, 1.1 eq), triethylamine (1.59 mL, 11.39 mmol, 3 eq) and 1-propanephosphonic anhydride (3625.43 mg, 5.7 mmol, 1.5 eq). The mixture was stirred at 25 °C for 6 h. The reaction mixture was washed with aqueous hydrochloric acid, dried over magnesium sulfate, filtered and the filtrate concentrated in vacuo. The residue was purified by prep-HPLC (FA as modifier) to give benzyl 4-[2-[tert-butoxycarbonyl(methyl)amino]ethylcarbamoyl]piperidine-1-carboxylate (1.3 g, 3.1 mmol, 81.59 % yield) as colorless oil. MS (ESI⁺) [M-Boc+H]⁺ : 320

### Step 2: tert-butyl methyl(2-(piperidine-4-carboxamido)ethyl)carbamate

To a solution of benzyl 4-[2-[tert-butoxycarbonyl(methyl)amino]ethylcarbamoyl]piperidine-1-carboxylate (1200.0 mg, 2.86 mmol, 1 eq) in ethyl acetate (30 mL) was added Pd/C (302.92 mg, 0.290 mmol, 0.100 eq). The mixture was stirred at 25 °C for 14 h under H₂ balloon. The mixture was filtered through a Celite pad, and the filtrate was concentrated to give tert-butyl N-methyl-N-[2-(piperidine-4-carbonylamino)ethyl]carbamate (750 mg, 2.63 mmol, 91.88 % yield) as black oil. MS (ESI⁺) [M+H]⁺ : 286

### Step 3: methyl 2-bromo-4-((3-iodoimidazo[1,2-a]pyrazin-8-yl)amino)benzoate

To a solution of 8-chloro-3-iodo-imidazo[1,2-a]pyrazine (2.0 g, 7.16 mmol, 1 eq) in MeCN (18 mL)/acetic acid (2 mL) was added methyl 4-amino-2-bromo-benzoate (1646.4 mg, 7.16 mmol, 1 eq). The mixture was stirred at 90 °C for 14 h. The mixture was filtered and the solid was washed by acetonitrile to give methyl 2-bromo-4-[(3-iodoimidazo[1,2-a]pyrazin-8-yl)amino]benzoate (2.8 g, 5.92 mmol, 73% yield) as white solid. MS (ESI⁺) [M+H]⁺: 474.7

### Step 4: methyl 2-bromo-4-((3-(3-fluoro-4-methoxyphenyl)imidazo[1,2-a]pyrazin-8-yl)amino)benzoate

The title compound was obtained in analogy to step 5 in the preparation of Intermediate 27 using methyl 2-bromo-4-((3-iodoimidazo[1,2-a]pyrazin-8-yl)amino)benzoate and 2-(3-fluoro-4-methoxyphenyl)-4,4,5,5-tetramethyl-1,3,2-dioxaborolane. MS (ESI⁺) [M+H]⁺ : 472.8

### Step 5: 2-bromo-4-((3-(3-fluoro-4-methoxyphenyl)imidazo[1,2-a]pyrazin-8-yl)amino)benzoic acid

A solution of methyl 2-bromo-4-[[3-(3-fluoro-4-methoxy-phenyl)imidazo[l,2-a]pyrazin-8-yl]amino]benzoate (200.0 mg, 0.420 mmol, 1 eq) in methanol (10 mL) was stirred at 80 °C for 10 min. Then 4M aq. sodium hydroxide (5.0 mL, 20 mmol, 47.13 eq) was added. The mixture was stirred at 80 °C for 4 h. The reaction mixture was concentrated *in vacuo,* diluted with water, and acidified with 2 N HCl. The solid was collected and thoroughly dried to give 2-bromo-4-[[3-(3-fluoro-4-methoxy-phenyl)imidazo[1,2-a]pyrazin-8-yl]amino]benzoic acid (180 mg, 0.390 mmol, 64.93 % yield) as off white solid. (ESI⁺) [M+H]⁺ : 458.9

### Step 6: 1-(2-bromo-4-((3-(3-fluoro-4-methoxyphenyl)imidazo[1,2-a]pyrazin-8-yl)amino)benzoyl)-N-(2-(methylamino)ethyl)piperidine-4-carboxamide; formic acid

To a mixture of O-(7-azabenzotriazol-1-yl)-N,N,N',N'-tetramethyluronium hexafluorophosphate (99.79 mg, 0.260 mmol, 1.2 eq) and triethylamine (0.06 mL, 0.440 mmol, 2 eq) in DMF (4 mL) was added 2-bromo-4-[[3-(3-fluoro-4-methoxy-phenyl)imidazo[1,2-a]pyrazin-8-yl]amino]benzoic acid (100.0 mg, 0.220 mmol, 1 eq) and tert-butyl N-methyl-N-[2-(piperidine-4-carbonylamino)ethyl]carbamate (93.62 mg, 0.330 mmol, 1.5 eq) slowly at 25 °C. Then the mixture was stirred at 25 °C for 12 h. Then to the mixture was added HCl indioxane (3 mL). The mixture was stirred at 25°C for 4 h. The mixture was filtered and the filtrate was purified by prep-HPLC (FA as additive) to give 1-[2-bromo-4-[[3-(3-fluoro-4-methoxy-phenyl)imidazo[1,2-a]pyrazin-8-yl]amino]benzoyl]-N-[2-(methylamino)ethyl]piperidine-4-carboxamide (40 mg, 0.060 mmol, 26.73 % yield) as light yellow solid. MS (ESI⁺) [M+H]⁺ : 626

### Reference Example 489

### (2S)-4-[1-[2-chloro-4-[[3-[4-(cyanomethoxy)-2,3-difluoro-phenyl]imidazo[1,2-a]pyrazin-8-yl]amino]benzoyl]piperidine-4-carbonyl]piperazine-2-carboxylic acid; formic acid

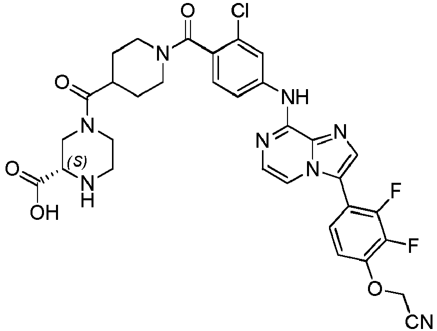

### Step 1: (S)-1-tert-butyl 2-methyl 4-(piperidine-4-carbonyl)piperazine-1,2-dicarboxylate

To a mixture of 1-benzylpiperidine-4-carboxylic acid (300 mg, 1.37 mmol), (S)-1-tert-butyl 2-methyl piperazine-1,2-dicarboxylate (501 mg, 2.05 mmol) and triethylamine (0.57 mL, 4.1 mmol) in DMF (10 mL) was added dropwise1-propanephosphonic anhydride (1295 mg, 2.05 mmol) at 25 °C under N₂. The mixture was stirred at 25 °C for 2 hours. The reaction mixture was poured into water (50 mL), it was extracted by ethyl acetate (50 mLx3), the combined organic layers were washed by brine (50 mL), dried by Na₂SO₄, filtered and concentrated to give the crude intermeidate. A mixture of above residue (400 mg, 0.900 mmol) and palladium hydroxide on carbon (40 mg, 0.900 mmol) in methanol (10 mL) was stirred at 25 °C under a hydrogen balloon for 16 hours. The reaction mixture was filtered and concentrated to afford (S)-1-tert-butyl 2-methyl 4-(piperidine-4-carbonyl)piperazine-1,2-dicarboxylate (220 mg, 0.620 mmol, 69% yield) as a colorless oil. MS (ESI⁺) [M+H]⁺ : 356.1

### Step 2: 2-chloro-4-((3-iodoimidazo[1,2-a]pyrazin-8-yl)amino)benzoic acid

The title compound was obtained in analogy to step 3 of Reference Example 488 using 8-chloro-3-iodoimidazo[1,2-a]pyrazine and 4-amino-2-chlorobenzoic acid. MS (ESI⁺) [M+H]⁺ : 415.0

### Step 3: (S)-1-tert-butyl 2-methyl4-(1-(2-chloro-4-((3-iodoimidazo[1,2-a]pyrazin-8-yl)amino)benzoyl)piperidine-4-carbonyl)piperazine-1,2-dicarboxylate

To a mixture of 2-chloro-4-((3-iodoimidazo[1,2-a]pyrazin-8-yl)amino)benzoic acid (2.0 g, 4.82 mmol), N-hydroxysuccinimide (0.72 g, 6.27 mmol) in DMF (10 mL) and THF (30 mL) was added 1-ethyl-(3-(3-dimethylamino)propyl)-carbodiimide hydrochloride (1.4 mL, 5.31 mmol) at 25 °C under N₂. The mixture was stirred at 25 °C for 16 hours. LC-MS indicated the reaction was completed. The reaction mixture was concentrated and the residue was poured into water (50 mL). The mixture was filtered and the filtrate was concentrated to afford a residue (1.1 g, 2.15 mmol) as a white solid. A mixture of the above residue (303 mg, 0.590 mmol), (S)-1-tert-butyl 2-methyl 4-(piperidine-4-carbonyl)piperazine-1,2-dicarboxylate (220 mg, 0.590 mmol) and triethylamine (0.12 mL, 0.890 mmol) in THF (5 mL) was stirred at 25 °C for 2 h. The reaction mixture was concentrated to afford (S)-1-tert-butyl 2-methyl 4-(1-(2-chloro-4-((3-iodoimidazo[1,2-a]pyrazin-8-yl)amino)benzoyl)piperidine-4-carbonyl)piperazine-1,2-dicarboxylate (350 mg, 0.470 mmol) as a white solid. MS (ESI⁺) [M+H]⁺ : 752.1

### Step 4: (S)-1-tert-butyl 2-methyl 4-(1-(2-chloro-4-((3-(4-(cyanomethoxy)-2,3-difluorophenyl)imidazo[1,2-a]pyrazin-8-yl)amino)benzoyl)piperidine-4-carbonyl)piperazine-1,2-dicarboxylate

The title compound was obtained in analogy to step 5 in the preparation of Intermediate 27 using (S)-1-tert-butyl 2-methyl 4-(1-(2-chloro-4-((3-iodoimidazo[1,2-a]pyrazin-8-yl)amino)benzoyl)piperidine-4-carbonyl)piperazine-1,2-dicarboxylate and 2-(2,3-difluoro-4-(4,4,5,5-tetramethyl-1,3,2-dioxaborolan-2-yl)phenoxy)acetonitrile. (ESI⁺) [(M+H)⁺] : 793.0

### Step 5: (2S)-4-[1-[2-chloro-4-[[3-[4-(cyanomethoxy)-2,3-difluoro-phenyl]imidazo[1,2-a]pyrazin-8-yl]amino]benzoyl]piperidine-4-carbonyl]piperazine-2-carboxylic acid; formic acid

A mixture of (S)-1-tert-butyl 2-methyl 4-(1-(2-chloro-4-((3-(4-(cyanomethoxy)-2,3-difluorophenyl)imidazo[1,2-a]pyrazin-8-yl)amino)benzoyl)piperidine-4-carbonyl)piperazine-1,2-dicarboxylate (100 mg, 0.130 mmol) and lithium chloride (107 mg, 2.52 mmol) in triethylamine (0.1 mL, 0.720 mmol) and DMF (0.5 mL) was stirred at 100 °C under N₂ for 16 hours. To the mixture was added dropwise trifluoroacetic acid (0.2 mL, 2.6 mmol) at 25 °C. The mixture was stirred at 25 °C under N₂ for 16 hours. The reaction mixture was purified directly via prep-HPLC and then lyophilized to afford the title compound (7.5 mg, 0.010 mmol) as a white solid. (ESI⁺) [M+H]⁺: 679.0

### Reference Example 490

### (R)-4-(1-(2-chloro-4-((3-(4-(cyanomethoxy)-2,3-difluorophenyl)imidazo[1,2-a]pyrazin-8-yl)amino)benzoyl)piperidine-4-carbonyl)piperazine-2-carboxylic acid; formic acid

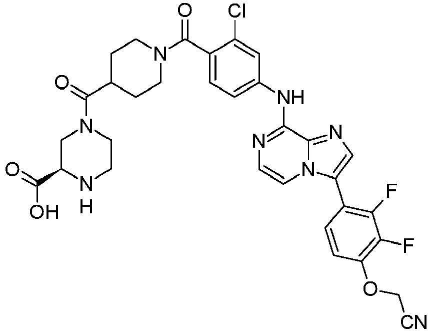

The title compound was obtained in analogy to Reference Example 489 via a 5-step sequence using (R)-1-tert-butyl 2-methyl piperazine-1,2-dicarboxylate instead of (S)-1-tert-butyl 2-methyl piperazine-1,2-dicarboxylate.

(ESI⁺) [M+H]⁺ : 679.0

### Intermediate 137

### 2-[4-[8-[3-chloro-4-[4-(2-chloroethyl)piperazine-1-carbonyl]anilino]imidazo[1,2-a]pyrazin-3-yl]-2,3-difluoro-phenoxy]acetonitrile

### Step 1: 1-(2-chloroethyl)piperazine bis(2,2,2-trifluoroacetate)

To a solution of tert-butyl 4-(2-chloroethyl)piperazine-1-carboxylate (1 g, 4.02 mmol) in DCM (10mL) / TFA (10 mL), the reaction was stirred for two hours at room temperature. The reaction mixture was concentrated in vacuum to give 1-(2-chloroethyl)piperazine bis(2,2,2-trifluoroacetate) (1.48 g).

### Step 2: 2-[4-[8-[3-chloro-4-[4-(2-chloroethyl)piperazine-1-carbonyl]anilino]imidazo[1,2-a]pyrazin-3-yl]-2,3-difluoro-phenoxy]acetonitrile

To a solution of Intermediate 29 (200mg, 439 µmol) in DMF (5 mL) was added 1-(2-chloroethyl)piperazine bis(2,2,2-trifluoroacetate) (165 mg, 439 µmol), triethylamine (178 mg, 245 µL, 1.76 mmol) and 2,4,6-tripropyl-1,3,5,2,4,6-trioxatriphosphinane 2,4,6-trioxide (411 µL, 658 µmol), the reaction was stirred for two hours at room temperature. The mixture was washed with brine and extracted in DCM. The organic layer was concentrated *in vacuo* to give crude 2-(4-(8-((3-chloro-4-(4-(2-chloroethyl)piperazine-1-carbonyl)phenyl)amino)imidazo[1,2-a]pyrazin-3-yl)-2,3-difluorophenoxy)acetonitrile (270mg, 428 µmol, 97.6 % yield). MS (ESI) [M+H]⁺ : 586.1

### Reference Example 491

### 1-[2-[4-[2-chloro-4-[[3-[4-(cyanomethoxy)-2,3-difluoro-phenyl]imidazo[1,2-a]pyrazin-8-yl]amino]benzoyl]piperazin-1-yl]ethyl]pyrrolidine-3-carboxylic acid trifluoroacetate

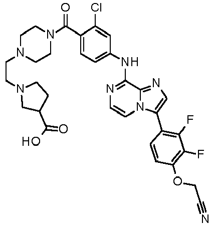

### Step 1: tert-butyl 1-[2-[4-[2-chloro-4-[[3-[4-(cyanomethoxy)-2,3-difluoro-phenyl]imidazo[1,2-a]pyrazin-8-yl]amino]benzoyl]piperazin-1-yl]ethyl]pyrrolidine-3-carboxylate

To a solution of Intermediate 137 (80mg, 136 µmol) in DMSO (3mL) was added sodium carbonate (28.9 mg, 273 µmol) and tert-butyl pyrrolidine-3-carboxylate (46.7 mg, 273 µmol), the reaction was stirred for 15 hours at 60 °C. The reaction mixture was cooled to room temperature and filtered. The filtrate was poured into water and the mixture was filtered. The filter cake was dried in vacuum to give tert-butyl 1-[2-[4-[2-chloro-4-[[3-[4-(cyanomethoxy)-2,3-difluoro-phenyl]imidazo[1,2-a]pyrazin-8-yl]amino]benzoyl]piperazin-1-yl]ethyl]pyrrolidine-3-carboxylate (100 mg, 128 µmol, 93.5 % yield).

### Step2: 1-[2-[4-[2-chloro-4-[[3-[4-(cyanomethoxy)-2,3-difluoro-phenyl]imidazo[1,2-a]pyrazin-8-yl]amino]benzoyl]piperazin-1-yl]ethyl]pyrrolidine-3-carboxylic acid trifluoroacetate

To a solution of tert-butyl 1-[2-[4-[2-chloro-4-[[3-[4-(cyanomethoxy)-2,3-difluorophenyl]imidazo[1,2-a]pyrazin-8-yl]amino]benzoyl]piperazin-1-yl]ethyl]pyrrolidine-3-carboxylate ( 100mg, 128 µmol) in THF ( 5mL) was added 6N HCl ( 2 mL), the reaction mixture was stirred for two hours at room temperature. The mixture was neutralized with ammonium hydroxide. The mixture was extracted in ethyl acetate and the organic layer was concentrated in vacuum. The residue was purified by preparative HPLC to give 1-[2-[4-[2-chloro-4-[[3-[4-(cyanomethoxy)-2,3-difluoro-phenyl]imidazo[1,2-a]pyrazin-8-yl]amino]benzoyl]piperazin-1-yl]ethyl]pyrrolidine-3-carboxylic acid (16 mg). MS (ESI) [M+H]+ : 665.1

The following examples were prepared in analogy to Reference Example 491, the hydrolysis of tert butyl ester step 2 was only applied for intermediates containing a tert butyl ester group.

| Ex. | Name | Structure | ESI [M+H]⁺ | Starting Material |
|---|---|---|---|---|
| REF 492 | 2-[4-[8-[4-[4-[2-(azetidin-1-yl)ethyl]piperazine-1-carbonyl]-3-chloro-anilino]imidazo[1,2-a]pyrazin-3-yl]-2,3-difluoro-phenoxy]acetonitrile trifluoroacetate | 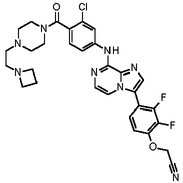 | 607.1 | Intermediate 137 and azetidine hydrochloride |

### Reference Example 493

### N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride

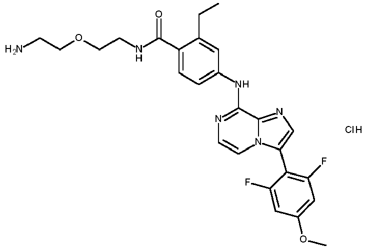

### Step 1:

### tert-butyl N-[2-[2-[[2-ethyl-4-[(3-iodoimidazo[1,2-a]pyrazin-8-yl)amino]benzoyl]amino]ethoxy]ethyl]carbamate (Intermediate 138)

A mixture Intermediate 63 (1.421 g, 3.2 mmol), triethylamine (1.62 g, 2.23 mL, 16 mmol), TBTU (1.22 g, 3.68 mmol) and tert-butyl (2-(2-aminoethoxy)ethyl)carbamate (816 mg, 3.99 mmol) in DMF (20 mL) was stirred at room temperature overnight. The reaction mixture was poured into 150 mL water and extracted with ethyl acetate (2 × 100 mL). The crude material was adsorbed on Isolute and purified by flash chromatography (silica gel, 80 g, 0% to 100% ethyl acetate in heptane) to yield tert-butyl (2-(2-(2-ethyl-4-((3-iodoimidazo[1,2-a]pyrazin-8-yl)amino)benzamido)ethoxy)ethyl)carbamate (1.561 g, 2.63 mmol, 82.2 %). MS (ESI, m/z): 595.4 [M+H]⁺.

### Step 2:

### tert-butyl N-[2-[2-[[4-[[3-(2,6-difluoro-4-methoxy-phenyl)imidazo[1,2-a]pyrazin-8-yl]amino]-2-ethyl-benzoyl]amino]ethoxy]ethyl]carbamate

A mixture of tert-butyl N-[2-[2-[[2-ethyl-4-[(3-iodoimidazo[1,2-a]pyrazin-8-yl)amino]benzoyl]amino]ethoxy]ethyl]carbamate (Intermediate 138) (89.2 mg, 150 µmol), (2,6-difluoro-4-methoxyphenyl)boronic acid (19.7 mg, 225 µmol), 1,1'-bis (diphenylphosphino)ferrocene-palladium(II)dichloride dichloromethane complex (12.2 mg, 15 µmol) and Na₂CO₃ (31.8 mg, 300 µmol) in dioxane (1mL) / water (0.1 mL) was stirred at 110 °C overnight. The mixtures were concentrated in vacuo, pre-purified by passing through a 4 g silica column eluting with 30 mL of a ethyl acetate / MeOH 9/1 solution and concentrated. Purification with preparative HPLC on reversed phase (Gemini 5um C18 75×30) eluting with a gradient formed from water (+0.1% NEt₃) / acetonitrile yielded after evaporation of the product containing fractions tert-butyl N-[2-[2-[[4-[[3-(2,6-difluoro-4-methoxy-phenyl)imidazo[1,2-a]pyrazin-8-yl]amino]-2-ethyl-benzoyl]amino]ethoxy]ethyl]carbamate (7.8 mg, 12.8 µmol, 8.5 %). MS (ESI, m/z): 611.4 [M+H]+.

### Step 3:

### Reference Example 493

### N-[2-(2-aminoethoxy)ethyl]-4-[[3-(2,6-difluoro-4-methoxyphenyl)imidazo[1,2-a]pyrazin-8-yl]amino]-2-ethylbenzamide;hydrochloride

A mixture of tert-butyl N-[2-[2-[[4-[[3-(2,6-difluoro-4-methoxy-phenyl)imidazo[1,2-a]pyrazin-8-yl]amino]-2-ethyl-benzoyl]amino]ethoxy]ethyl]carbamate and excess 4N HCl (dioxane) in DCM (2 mL) was stirred at room temperature for 2 h and evaporated to dryness. The residue was triturated with 2 mL of Et₂O and the product was filtered off to yield after drying N-(2-(2-aminoethoxy)ethyl)-4-((3-(2,6-difluoro-4-methoxyphenyl)imidazo[1,2-a]pyrazin-8-yl)amino)-2-ethylbenzamide hydrochloride (4.8 mg, 9.4 µmol, 73.5 %). MS (ESI, m/z): 509.4 [M+H]⁺.

### Reference Example 494

### N-[2-(2-aminoethoxy)ethyl]-2-ethyl-4-[[3-[4-methoxy-2-(methylsulfamoyl)phenyl]imidazo[1,2-a]pyrazin-8-yl]amino]benzamide;formic acid

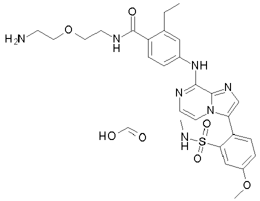

### Step 1:

### tert-butyl N-[2-[2-[[2-ethyl-4-[[3-[4-methoxy-2-(methylsulfamoyl)phenyl]imidazo[1,2-a]pyrazin-8-yl]amino]benzoyl]amino]ethoxy]ethyl]carbamate

In analogy to the procedure described for the synthesis of Reference Example 493 N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl N-[2-[2-[[2-ethyl-4-[(3-iodoimidazo[1,2-a]pyrazin-8-yl)amino]benzoyl]amino]ethoxy]ethyl]carbamate (Intermediate 138) and 5-methoxy-N-methyl-2-(4,4,5,5-tetramethyl-1,3,2-dioxaborolan-2-yl)benzenesulfonamide. MS (ESI, m/z): 668.4 [M+H]+.

### Step 2:

### N-[2-(2-aminoethoxy)ethyl]-2-ethyl-4-[[3-[4-methoxy-2-(methylsulfamoyl)phenyl]imidazo[1,2-a]pyrazin-8-yl]amino]benzamide formate

In analogy the procedure described for the synthesis of Reference Example 493 N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl N-[2-[2-[[2-ethyl-4-[[3-[4-methoxy-2-(methylsulfamoyl)phenyl]imidazo[1,2-a]pyrazin-8-yl]amino]benzoyl]amino]ethoxy]ethyl]carbamate through acidic cleavage of the protecting group followed by reversed phase column chromatography eluting with a gradient formed from water (+0.1% formic acid) / acetonitrile. Evaporation of the product containing fractions yielded the title compound. MS (ESI, m/z): 568.3 [M+H]+.

### Reference Example 495

### N-(2-(2-aminoethoxy)ethyl)-2-ethyl-4-((3-(4-methoxy-2-(trifluoromethyl)phenyl)imidazo[1,2-a]pyrazin-8-yl)amino)benzamide hydrochloride

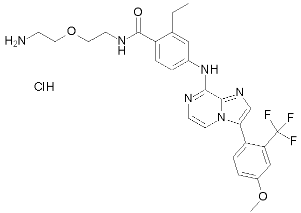

### Step 1:

tert-butyl (2-(2-(2-ethyl-4-((3-(4-methoxy-2-(trifluoromethyl)phenyl)imidazo[1,2-a]pyrazin-8-yl)amino)benzamido)ethoxy)ethyl)carbamate

In analogy to the procedure described for the synthesis of Reference Example 493 N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl N-[2-[2-[[2-ethyl-4-[(3-iodoimidazo[1,2-a]pyrazin-8-yl)amino]benzoyl]amino]ethoxy]ethyl]carbamate (Intermediate 138) and 2-(4-methoxy-2-(trifluoromethyl)phenyl)-4,4,5,5-tetramethyl-1,3,2-dioxaborolane. MS (ESI, m/z): 643.3 [M+H]+.

### Step 2:

### N-(2-(2-aminoethoxy)ethyl)-2-ethyl-4-((3-(4-methoxy-2-(trifluoromethyl)phenyl)imidazo[1,2-a]pyrazin-8-yl)amino)benzamide hydrochloride

In analogy the procedure described for the synthesis of Reference Example 493

N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl (2-(2-(2-ethyl-4-((3-(4-methoxy-2-(trifluoromethyl)phenyl)imidazo[1,2-a]pyrazin-8-yl)amino)benzamido)ethoxy)ethyl)carbamate through acidic cleavage of the protecting group. MS (ESI, m/z): 543.3 [M+H]+.

### Reference Example 496

### 4-((3-(2-amino-4-methoxyphenyl)imidazo[1,2-a]pyrazin-8-yl)amino)-N-(2-(2-aminoethoxy)ethyl)-2-ethylbenzamide hydrochloride

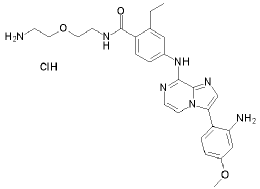

### Step 1:

### tert-butyl (2-(2-(4-((3-(2-((tert-butoxycarbonyl)amino)-4-methoxyphenyl)imidazo[1,2-a]pyrazin-8-yl)amino)-2-ethylbenzamido)ethoxy)ethyl)carbamate

In analogy to the procedure described for the synthesis of Reference Example 493

N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl N-[2-[2-[[2-ethyl-4-[(3-iodoimidazo[1,2-a]pyrazin-8-yl)amino]benzoyl]amino]ethoxy]ethyl]carbamate (Intermediate 138) and tert-butyl (5-methoxy-2-(4,4,5,5-tetramethyl-1,3,2-dioxaborolan-2-yl)phenyl)carbamate. MS (ESI, m/z): 690.5 [M+H]+.

### Step 2:

### 4-((3-(2-amino-4-methoxyphenyl)imidazo[1,2-a]pyrazin-8-yl)amino)-N-(2-(2-aminoethoxy)ethyl)-2-ethylbenzamide hydrochloride

In analogy the procedure described for the synthesis of Reference Example 493

N-[2-(2-aminoethoxy)ethyl]-4-[[3-(2,6-difluoro-4-methoxyphenyl)imidazo[1,2-a]pyrazin-8-yl]amino]-2-ethylbenzamide hydrochloride the title compound was prepared from tert-butyl (2-(2-(4-((3-(2-((tert-butoxycarbonyl)amino)-4-methoxyphenyl)imidazo[1,2-a]pyrazin-8-yl)amino)-2-ethylbenzamido)ethoxy)ethyl)carbamate through acidic cleavage of the protecting group. MS (ESI, m/z): 490.4 [M+H]+.

### Intermediate 139

### 3-(2,3-difluoro-4-methoxyphenyl)imidazo[1,2-a]pyrazin-8-amine

In a sealed pressure tube a suspension of 8-chloro-3-(2,3-difluoro-4-methoxyphenyl)imidazo[1,2-a]pyrazine (Intermediate 21, 0.062 g, 210 µmol, Eq: 1) in isopropanol (839 µl) and 25% aq ammonia (1.31 g, 1.46 mL, 19.3 mmol, Eq: 92) was heated to 115 °C for 19 h. The reaction mixture was diluted with water, the suspension filtered and washed with water. The solid was collected and dried *in vacuo.* The compound 3-(2,3-difluoro-4-methoxyphenyl)imidazo[1,2-a]pyrazin-8-amine (0.046 g, 167 µmol, 79.4 % yield) was obtained as light brown solid. MS ESI (m/z): 277.2 [M+H]⁺

### Intermediate 140

### tert-butyl (2S,4R)-4-hydroxy-2-(piperazine-1-carbonyl)pyrrolidine-1-carboxylate

### Step 1: benzyl 4-[(2S,4R)-1-tert-butoxycarbonyl-4-hydroxy-pyrrolidine-2-carbonyl]piperazine-1-carboxylate

To a clear solution of (2S,4R)-1-(tert-butoxycarbonyl)-4-hydroxypyrrolidine-2-carboxylic acid (1.5 g, 6.29 mmol, Eq: 1) and DIPEA (1.63 g, 2.2 mL, 12.6 mmol, Eq: 2) in dry DMF (15.7 mL) was added HATU (2.39 g, 6.29 mmol, Eq: 1) and the mixture stirred 10 minutes at room temperature. Then a solution of benzyl piperazine-1-carboxylate (1.41 g, 6.29 mmol, Eq: 1) in dry DMF (15.7 mL) was added and stirring at room temperature was continued for 2 h. Then the reaction mixture was concentrated *in vacuo.* The crude material was purified by silica gel chromatography using heptane/ (ethyl acetate/EtOH/NH₄OH 75:25:2) as eluent. The compound benzyl 4-((2S,4R)-1-(tert-butoxycarbonyl)-4-hydroxypyrrolidine-2-carbonyl)piperazine-1-carboxylate (3.177 g, 5.79 mmol, 92 % yield) was obtained as yellow oil with an purity of 79 % (contains 21% DMF acc to NMR) and was used without further purification. MS ESI (m/z): 478.2184 [M+HCOO⁻]⁻

### Step 2: tert-butyl (2S,4R)-4-hydroxy-2-(piperazine-1-carbonyl)pyrrolidine-1-carboxylate

A flask containing a solution of benzyl 4-((2S,4R)-1-(tert-butoxycarbonyl)-4-hydroxypyrrolidine-2-carbonyl)piperazine-1-carboxylate (3.17 g, 5.78 mmol, Eq: 1) in methanol (38.5 mL) was evacuated 3 × (frothing) and flushed with argon. Then 10% palladium on carbon (123 mg, 116 µmol, Eq: 0.02) was added and degassing repeated. Then the apparatus was again 4 × evacuated (frothing) and flushed with hydrogen. The reaction was stirred 5 hours at room temperature under hydrogen. Then the reaction was filtered through a glas fibre filter, washed with MeOH and the obtained solution concentrated *in vacuo.* The obtained material was triturated with heptane/diisopropyl ether, filtered washed and dried in vacuo. The compound tert-butyl (2S,4R)-4-hydroxy-2-(piperazine-1-carbonyl)pyrrolidine-1-carboxylate (1.660 g, 5.38 mmol, 93.1 % yield) was obtained as light yellow solid. MS ESI (m/z): 300.2 [M+H]⁺

### Intermediate 141

### tert-butyl (E)-4-(N,N'-bis(tert-butoxycarbonyl)-1H-pyrazole-1-carboximidamido)butanoate

### Biorg and Med Chem Lett 2014, vol 24 #23 p5525-5529

A solution of tert-butyl (E)-(((tert-butoxycarbonyl)imino)(1H-pyrazol-1-yl)methyl)carbamate (0.155g, 484 µmol, Eq: 1), triphenylphosphine (201 mg, 727 µmol, Eq: 1.5) and tert-butyl 4-hydroxybutanoate (101 mg, 630 µmol, Eq: 1.3) in dry THF (1.86 mL) was cooled to 0 °C. Then DIAD (156 mg, 150 µL, 727 µmol, Eq: 1.5) was added dropwise. Then the cooling bath was removed and the reaction heated to reflux for 16 hours. Then the reaction was quenched with water and diluted with dichloromethane. The mixture was extracted 2 × with dichloromethane and the organic layers were washed 1 × with water. The combined organic layers were dried with sodium sulfate, filtered and concentrated in vacuo. The crude material was purified by silica gel chromatography using heptane/ ethyl acetate as eluent. The compound tert-butyl (E)-4-(N,N'-bis(tert-butoxycarbonyl)-1H-pyrazole-1-carboximidamido)butanoate (52 mg, 107 µmol, 22.1 % yield) was obtained as colorless oil with a purity of 93 % (UV, 220 nm). MS ESI (m/z): 453.4 [M+H]⁺, ¹H NMR (300MHz, chloroform-d) δ = 7.94 (br s, 1H), 7.68 (dd, J=0.6, 1.6 Hz, 1H), 6.41 (dd, J=1.6, 2.8 Hz, 1H), 3.72 (br t, J=7.4 Hz, 2H), 2.32 (t, J=7.5 Hz, 2H), 2.01 (quin, J=7.4 Hz, 2H), 1.50 (s, 9H), 1.43 (s, 9H), 1.27 (s, 9H)

### Reference Example 497

### N-(2-(2-Aminoethoxy)ethyl)-4-((3-(2,3-difluoro-4-methoxyphenyl)imidazo[1,2-a]pyrazin-8-yl)amino)-2-fluoro-6-methylbenzamide formate

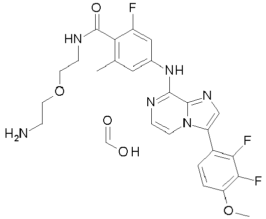

### Step 1: tert-Butyl 4-bromo-2-fluoro-6-methylbenzoate

In a pressure tube to a white suspension of 4-bromo-2-fluoro-6-methylbenzoic acid (700 mg, 3 mmol, Eq: 1) in dry toluene (1.88 mL) was added N,N-dimethylformamide di-tert-butyl acetal (4.41 g, 5.19 mL, 19.5 mmol, Eq: 6.5). The tube was sealed and the mixture heated to 80°C for 3 hours. The reaction mixture was diluted with water, ethyl acetate and sat. aqueous NaHCO₃ solution. The mixture was extracted 2 × with ethyl acetate and the organic layers washed 1 × with sat. aqueous NaHCO₃ solution and 2 × with brine. The combined organic layers were dried with sodium sulfate, filtered and concentrated in vacuo. The crude material (drypack on silica gel) was purified by silica gel chromatography using heptane/ ethyl acetate as eluent. The compound tert-butyl 4-bromo-2-fluoro-6-methylbenzoate (794.9 mg, 2.69 mmol, 89.7 % yield) was obtained as colorless oil and was used without further purification. MS EI (m/z): 290.0 [M]⁺

### Step 2: tert-Butyl 4-((3-(2,3-difluoro-4-methoxyphenyl)imidazo[1,2-a]pyrazin-8-yl)amino)-2-fluoro-6-methylbenzoate

A brown suspension of 3-(2,3-difluoro-4-methoxyphenyl)imidazo[1,2-a]pyrazin-8-amine (Intermediate 139, 300 mg, 1.09 mmol, Eq: 1), tert-butyl 4-bromo-2-fluoro-6-methylbenzoate (628 mg, 2.17 mmol, Eq: 2) and sodium tert-butoxide (157 mg, 1.63 mmol, Eq: 1.5) in THF (10.9 mL) in a pressure tube was sparged with argon for 5 minutes while sonicating the vessel in an ultra-sonic bath. Then 1,1'-bis(diphenylphosphino)ferrocene (72.2 mg, 130 µmol, Eq: 0.12) and tris(dibenzylideneacetone)dipalladium (0) (39.8 mg, 43.4 µmol, Eq: 0.04) were added and degassing continued for 1 minute. The tube was sealed and heated to 130 ° C for 3 hours. Then the mixture was concentrated *in vacuo.* The crude material (drypack on silica gel) was purified by silica gel chromatography using heptane/ ethyl acetate as eluent. The compound tert-butyl 4-((3-(2,3-difluoro-4-methoxyphenyl)imidazo[1,2-a]pyrazin-8-yl)amino)-2-fluoro-6-methylbenzoate (367 mg, 758 µmol, 69.8 % yield) was obtained as yellow solid and was used without further purification. MS ESI (m/z): 485.2 [M+H]⁺

### Step 3: 4-((3-(2,3-Difluoro-4-methoxyphenyl)imidazo[1,2-a]pyrazin-8-yl)amino)-2-fluoro-6-methylbenzoic acid hydrochloride

To a solution of tert-butyl 4-((3-(2,3-difluoro-4-methoxyphenyl)imidazo[1,2-a]pyrazin-8-yl)amino)-2-fluoro-6-methylbenzoate (367 mg, 758 µmol, Eq: 1) in dioxane (1.52 mL) was added 4 M HCl in dioxane (11.4 mL, 45.5 mmol, Eq: 60) and the reaction was heated to 70 °C for 3 hours. Then again 4 M HCl in dioxane (5.68 mL, 22.7 mmol, Eq: 30) was added and the reaction stirred at 70 °C for 1 hour. The mixture was further diluted with dioxane and concentrated in vacuo. The compound 4-((3-(2,3-difluoro-4-methoxyphenyl)imidazo[1,2-a]pyrazin-8-yl)amino)-2-fluoro-6-methylbenzoic acid hydrochloride (410 mg, 750 µmol, 99 % yield) was obtained as light brown solid and was used without further purification. MS ESI (m/z): 429.2 [M+H]⁺

### Step 4: tert-Butyl N-[2-[2-[[4-[[3-(2,3-difluoro-4-methoxy-phenyl)imidazo[1,2-a]pyrazin-8-yl]amino]-2-fluoro-6-methyl-benzoyl]amino]ethoxy]ethyl]carbamate

To a solution of 4-((3-(2,3-difluoro-4-methoxyphenyl)imidazo[1,2-a]pyrazin-8-yl)amino)-2-fluoro-6-methylbenzoic acid hydrochloride (20 mg, 40.4 µmol, Eq: 1) and DIPEA (20.9 mg, 28.3 µl, 162 µmol, Eq: 4) in dry DMF (207 µl) was added HATU (15.4 mg, 40.4 µmol, Eq: 1) and the mixture stirred 10 minutes at room temperature (thick suspension). Then tert-butyl (2-(2-aminoethoxy)ethyl)carbamate hydrochloride (14.6 mg, 60.7 µmol, Eq: 1.5) was added, the reaction diluted with dry DMF (104 µl) and the mixture stirred at room temperature for 1 hour. Then the reaction was concentrated in vacuo. The crude material was purified by silica gel chromatography using dichloromethane/ methanol as eluent. The compound tert-butyl (2-(2-(4-((3-(2,3-difluoro-4-methoxyphenyl)imidazo[1,2-a]pyrazin-8-yl)amino)-2-fluoro-6-methylbenzamido)ethoxy)ethyl)carbamate (0.031 g, 40.3 µmol, 99.8 % yield) was obtained as colorless amorphous solid and was used without further purification. MS ESI (m/z): 615.4 [M+H]⁺

### Step 5: N-(2-(2-Aminoethoxy)ethyl)-4-((3-(2,3-difluoro-4-methoxyphenyl)imidazo[1,2-a]pyrazin-8-yl)amino)-2-fluoro-6-methylbenzamide formate

To a solution of tert-butyl (2-(2-(4-((3-(2,3-difluoro-4-methoxyphenyl)imidazo[1,2-a]pyrazin-8-yl)amino)-2-fluoro-6-methylbenzamido)ethoxy)ethyl)carbamate (31 mg, 40.3 µmol, Eq: 1) in dioxane (202 µl) was added 4 M HCl in dioxane (403 µl, 1.61 mmol, Eq: 40) and the resulting mixture stirred at room temperature (suspension). The reaction was diluted with dichloromethane and basified with 0.5 mL 7 M ammonia in MeOH. Then 1 spoon amine-silica gel was added and the mixture concentrated in vacuo. The crude material (drypack on amine silica gel) was purified by amine silica gel chromatography using dichloromethane/ methanol as eluent. The obtained material was further purified by preparative reversed phase HPLC (Column: Phenomenex Gemini-NX 5u 110A, l: 100 mm, dia: 30 mm) using water containing 0.1% formic acid / acetonitrile as eluent. The compound N-(2-(2-aminoethoxy)ethyl)-4-((3-(2,3-difluoro-4-methoxyphenyl)imidazo[1,2-a]pyrazin-8-yl)amino)-2-fluoro-6-methylbenzamide formate (9 mg, 16.1 µmol, 39.8 % yield) was obtained as white solid. MS ESI (m/z): 515.3 [M+H]⁺, 258.2 [M+2H]²⁺

The following examples were prepared in analogy to Reference Example 497. HCl-salts were isolated in case the compounds were clean after the last step without further purification. The free base was isolated, if the compounds were clean after silica gel chromatography or when a basic eluent was used during preparative HPLC.

| Ex. | Name | Structure | Color and form, analytics | Starting Materials |
|---|---|---|---|---|
| REF 498 | N-(2-(2-aminoethoxy)ethyl)-4-((3-(2,3-difluoro-4-methoxyphenyl)imidaz o[1,2-a]pyrazin-8-yl)amino)-2,6-difluorobenzamide | 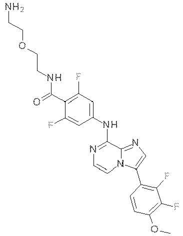 | White lyoph powder, MS ESI (m/z): 519.2 [M+H]⁺, 260.2 [M+2H]²⁺ | 4-bromo-2,6-difluoroben zoic acid |
| REF 499 | N-(2-(2-aminoethoxy)ethyl)-2-chloro-4-((3-(2,3-difluoro-4-methoxyphenyl)imidaz o[1,2-a]pyrazin-8-yl)amino)-6-fluorobenzamide | 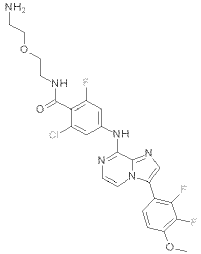 | White lyoph powder, MS ESI (m/z): 535.2, 537.2 [M+H]⁺, 268.2, 269.1 [M+2H]²⁺ | 4-bromo-2-chloro-6-fluorobenz oic acid |
| REF 500 | N-(2-(2-aminoethoxy)ethyl)-4-((3-(2,3-difluoro-4-methoxyphenyl)imidaz o[1,2-a]pyrazin-8-yl)amino)-3-fluoro-2-methylbenzamide dihydrochloride | 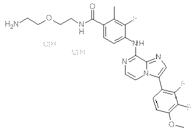 | Off-white solid, MS ESI (m/z): 513.3 [M-H]⁻ | 4-bromo-3-fluoro-2-methylbenz oic acid |
| REF 501 | N-(2-(2-aminoethoxy)ethyl)-2-chloro-4-((3-(2,3-difluoro-4-methoxyphenyl)imidaz o[1,2-a]pyrazin-8-yl)amino)-6-methylbenzamide | 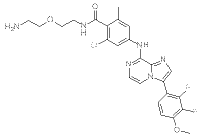 | Off-white solid, MS ESI (m/z): 531.2 [M+H]⁺ | 4-bromo-2-chloro-6-methylbenz oic acid |
| REF 502 | (4-(4-((3-(2,3-difluoro-4-methoxyphenyl)imidaz o[1,2-a]pyrazin-8-yl)amino)-2-fluoro-6-methylbenzoyl)piperazi n-1-yl)((2S,4R)-4-hydroxypyrrolidin-2-yl)methanone dihydrochloride | 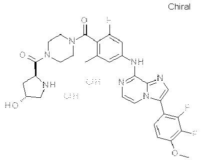 | MS ESI (m/z): 610.2391 [M+H]⁺ | Intermediat e 139 |
| REF 503 | (4-(4-((3-(2,3-difluoro-4-methoxyphenyl)imidaz o[1,2-a]pyrazin-8-yl)amino)-2,6-difluorobenzoyl)piperaz in-1-yl)((2S,4R)-4-hydroxypyrrolidin-2-yl)methanone dihydrochloride | 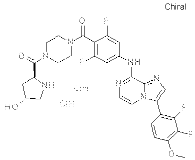 | MS ESI (m/z): 614.3 [M+H]⁺, 307.7 [M+2H]²⁺ | 4-bromo-2,6-difluoroben zoic acid, Intermediat e 139 |
| REF 504 | (4-(2-chloro-4-((3-(2,3-difluoro-4-methoxyphenyl)imidaz o[1,2-a]pyrazin-8-yl)amino)-6-fluorobenzoyl)piperazin -1-yl)((2S,4R)-4-hydroxypyrrolidin-2-yl)methanone dihydrochloride | 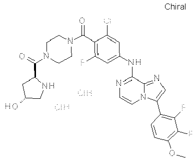 | MS ESI (m/z): 630.3, 632.4 [M+H]⁺, 315.7, 316.5 [M+2H]²⁺ | 4-bromo-2-chloro-6-fluorobenz oic acid, Intermediat e 139 |
| REF 505 | (4-(4-((3-(2,3-difluoro-4-methoxyphenyl)imidaz o[1,2-a]pyrazin-8-yl)amino)-2,5-difluorobenzoyl)piperaz in-1-yl)((2S,4R)-4-hydroxypyrrolidin-2-yl)methanone | 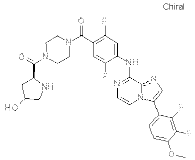 | MS ESI (m/z): 614.3 [M+H]⁺, 307.7 [M+2H]²⁺ | 4-bromo-2,5-difluoroben zoic acid, Intermediat e 139 |
| REF 506 | N-(2-(2-aminoethoxy)ethyl)-4-((3-(2,3-difluoro-4-methoxyphenyl)imidaz o[1,2-a]pyrazin-8-yl)amino)-2,3-difluorobenzamide | 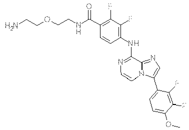 | MS ESI (m/z): 519.3 [M+H]⁺, 260.2 [M+2H]²⁺ | 4-bromo-2,3-difluoroben zoic acid |

### Reference Example 507

### N-(2-((2-Aminoethyl)amino)ethyl)-4-((3-(2,3-difluoro-4-methoxyphenyl)imidazo[1,2-a]pyrazin-8-yl)amino)-2-ethylbenzamide

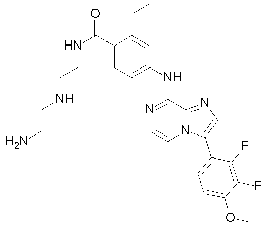

To a solution of 4-((3-(2,3-difluoro-4-methoxyphenyl)imidazo[1,2-a]pyrazin-8-yl)amino)-2-ethylbenzoic acid hydrochloride (Example 86, 100 mg, 217 µmol, Eq: 1) and DIPEA (112 mg, 152 µl, 868 µmol, Eq: 4) in dry DMF (1.08 mL) was added HATU (107 mg, 282 µmol, Eq: 1.3) and the mixture was shaken for 15 minutes at room temperature. Then N1-(2-aminoethyl)ethane-1,2-diamine (89.5 mg, 93.8 µl, 868 µmol, Eq: 4) was added and shaking continued at room temperature for 3 hours. Then the reaction mixture was concentrated in vacuo. The material was purified by preparative reversed phase HPLC (Column: YMC Actus Triart C18 5um, 1:100mm, dia:30mm) using water containing 0.1% triethylamine / acetonitrile as eluent. The compound N-(2-((2-aminoethyl)amino)ethyl)-4-((3-(2,3-difluoro-4-methoxyphenyl)imidazo[1,2-a]pyrazin-8-yl)amino)-2-ethylbenzamide (39 mg, 74.2 µmol, 34.2 % yield) was obtained as white solid. MS ESI (m/z): 510.2433 [M+H]⁺

The following examples were prepared in analogy to Reference Example 507.

| Ex. | Name | Structure | Color and form, MS | Starting Materials |
|---|---|---|---|---|
| 8 | rac-N-((1R,2S)-2-aminocyclohexyl)-4-((3-(2,3-difluoro-4-methoxyphenyl)imidaz o[1,2-a]pyrazin-8-yl)amino)-2-ethylbenzamide | 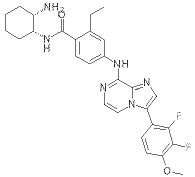 | Brown solid, MS ESI (m/z): 521.2 [M+H]⁺ | rac-(1R,2S)-cyclohexan e-1,2-diamine |
| 9 | N-((1S,2S)-2-aminocyclopentyl)-4-((3-(2,3-difluoro-4-methoxyphenyl)imidaz o[1,2-a]pyrazin-8-yl)amino)-2-ethylbenzamide | 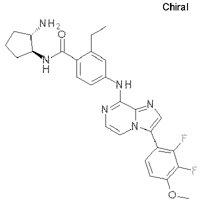 | White solid, MS ESI (m/z): 507.3 [M+H]⁺ | (1S,2S)-cyclopenta ne-1,2-diamine dihydrochl oride |

### Reference Example 508

### N-(2-(2-(2-Aminoethoxy)ethoxy)ethyl)-4-((3-(2,3-difluoro-4-methoxyphenyl)imidazo[1,2-a]pyrazin-8-yl)amino)-2-ethylbenzamide dihydrochloride

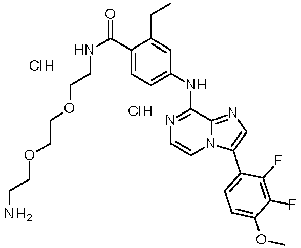

### Step 1: tert-Butyl (2-(2-(2-(4-((3-(2,3-difluoro-4-methoxyphenyl)imidazo[1,2-a]pyrazin-8-yl)amino)-2-ethylbenzamido)ethoxy)ethoxy)ethyl)carbamate

To a solution of 4-((3-(2,3-difluoro-4-methoxyphenyl)imidazo[1,2-a]pyrazin-8-yl)amino)-2-ethylbenzoic acid hydrochloride (Example 86, 40 mg, 86.8 µmol, Eq: 1) and DIPEA (44.9 mg, 60.6 µl, 347 µmol, Eq: 4) in dry DMF (434 µl) was added HATU (36.3 mg, 95.5 µmol, Eq: 1.1) and the mixture was shaken for 10 minutes at room temperature.

Then tert-butyl (2-(2-(2-aminoethoxy)ethoxy)ethyl)carbamate (28 mg, 113 µmol, Eq: 1.3) was added and shaking continued at room temperature for 4 hours. The reaction mixture was diluted with ethyl acetate, sat. aqueous NaHCO₃ solution and brine. The mixture was extracted 2x with ethyl acetate and the organic layers were washed 2x with brine. The combined organic layers were dried with sodium sulfate, filtered and concentrated in vacuo. The crude material was purified by silica gel chromatography using dichloromethane/ methanol as eluent. The compound tert-butyl (2-(2-(2-(4-((3-(2,3-difluoro-4-methoxyphenyl)imidazo[1,2-a]pyrazin-8-yl)amino)-2-ethylbenzamido)ethoxy)ethoxy)ethyl)carbamate (41.1 mg, 62.8 µmol, 72.3 % yield) was obtained as off-white solid . MS ESI (m/z): 655.4 [M+H]⁺

### Step 2: N-(2-(2-(2-aminoethoxy)ethoxy)ethyl)-4-((3-(2,3-difluoro-4-methoxyphenyl)imidazo[1,2-a]pyrazin-8-yl)amino)-2-ethylbenzamide dihydrochloride

In a 5 mL round-bottomed flask, tert-butyl (2-(2-(2-(4-((3-(2,3-difluoro-4-methoxyphenyl)imidazo[1,2-a]pyrazin-8-yl)amino)-2-ethylbenzamido)ethoxy)ethoxy)ethyl)carbamate (41.4 mg, 63.2 µmol, Eq: 1) and 4 M HCl in dioxane (632 µl, 2.53 mmol, Eq: 40) were combined to give a light yellow solution. The reaction mixture was stirred at room temperature for 3 hours. The reaction was diluted with water and directly lyophilized. The compound N-(2-(2-(2-aminoethoxy)ethoxy)ethyl)-4-((3-(2,3-difluoro-4-methoxyphenyl)imidazo[1,2-a]pyrazin-8-yl)amino)-2-ethylbenzamide dihydrochloride (37.3 mg, 59.4 µmol, 94 % yield) was obtained as yellow solid. MS ESI (m/z): 278.3 [M+2H]²⁺

The following examples were prepared in analogy to Reference Example 508. In case the free base was isolated, the obtained material was further purified by silica gel chromatography and/or preparative HPLC.

| Ex. | Name | Structure | Color and form, MS | Starting Materials |
|---|---|---|---|---|
| 10 | N-((1S,2R)-2-aminocyclopentyl)-4-((3-(2,3-difluoro-4-methoxyphenyl)imidaz o[1,2-a]pyrazin-8-yl)amino)-2-ethylbenzamide | 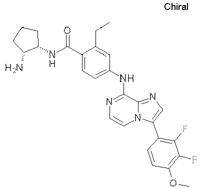 | Light brown solid, MS ESI (m/z): 507.3 [M+H]⁺, 254.3 [M+2H]²⁺ | tert-butyl ((1R,2S)-2-aminocyclo pentyl)carb amate |

### Reference Example 509

### 4-((3-(2,3-difluoro-4-methoxyphenyl)imidazo[1,2-a]pyrazin-8-yl)amino)-2-ethyl-N-(2-(2-(methyl(2-(methylsulfonamido)-2-oxoethyl)amino)ethoxy)ethyl)benzamide dihydrochloride

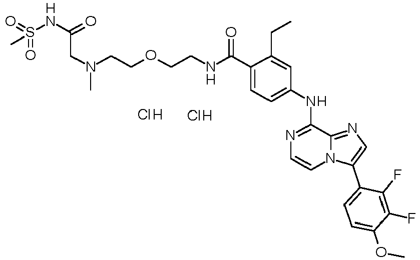

To a solution of N-(2-(2-(4-((3-(2,3-difluoro-4-methoxyphenyl)imidazo[1,2-a]pyrazin-8-yl)amino)-2-ethylbenzamido)ethoxy)ethyl)-N-methylglycine hydrochloride (Reference Example 485, 20 mg, 32.3 µmol, Eq: 1), methanesulfonamide (3.99 mg, 42 µmol, Eq: 1.3) and DMAP (5.13 mg, 42 µmol, Eq: 1.3) in dry dichloromethane (215 µl) was added 1-(3-dimethylaminopropyl)-3-ethylcarbodiimide (6.52 mg, 7.43 µL, 42 µmol, Eq: 1.3) and the mixture stirred at room temperature for 18 hours. Then the reaction was concentrated in vacuo.

The crude material was purified by preparative reversed phase HPLC (Column: YMC Actus Triart C18, 12 nm, 5µm, 1:100mm, dia:30mm) using acetonitrile / water containing 0.1% formic acid as eluent. The obtained solution was lyophilized. The residue was redisolved in 0.1M aq. HCl and again lyophilized.

The compound 4-((3-(2,3-difluoro-4-methoxyphenyl)imidazo[1,2-a]pyrazin-8-yl)amino)-2-ethyl-N-(2-(2-(methyl(2-(methylsulfonamido)-2-oxoethyl)amino)ethoxy)ethyl)benzamide dihydrochloride (16.5 mg, 22.5 µmol, 69.7 % yield) was obtained as light yellow solid. MS ESI (m/z): 660.2417 [M+H]⁺

### Reference Example 510

### (4S)-4-Amino-5-((1-((3-(4-((3-(2,3-difluoro-4-methoxyphenyl)imidazo[1,2-a]pyrazin-8-yl)amino)-2-ethylbenzamido)propyl)amino)-3-guanidino-1-oxopropan-2-yl)amino)-5-oxopentanoic acid trihydrochloride (Epimers 1:1)

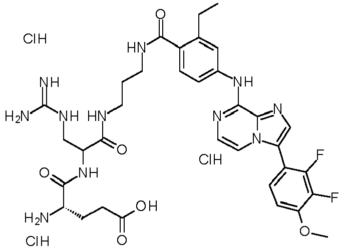

### Step 1: Boc-Glu(OtBu)-Agp(Boc)₂-OH

A solution of Fmoc-Agp(Boc)₂-OH (426 mg, 750 µmol, Eq: 0.625) and DIPEA biotech grade (775 mg, 1.05 mL, 6 mmol, Eq: 5) in dry dichloromethane (6.5 mL) was added to 2-chlorotrityl chloride-resin (Bachem, 1.6 mmol/g, 0.75 g, 1.2 mmol, Eq: 1) in a dried glass bottle and put under argon. The reaction mixture was shaken under argon atmosphere for 16 hours at room temperature. To the mixture was added methanol (596 µl) (0.8 mL per gram resin) and the reaction mixture was shaken for 4 hours at room temperature to cap the remaining chloride. The mixture was filtered and then washed 3 × with 5 mL dichloromethane, followed by 3 × 5 mL DMF. 4-methylpiperidine/DMF/DCM 2:1:1 (4.65 mL) was added to the resin. The reaction mixture was shaken for 30 minutes at room temperature. The resin was filtered and washed 2 x with 5 mL DCM and 2 × with 5 mL DMF. Again 4-methylpiperidine/DMF/DCM 2:1:1 (4.65 mL) was added to the resin and the mixture shaken for 30 minutes at room temperature. The resin was filtered and washed 2 x with 5 mL DCM and 2 × with 2 mL DMF. On the side a solution of Boc-Glu(OtBu)-OH (455 mg, 1.5 mmol, Eq: 1.25) and DIPEA (388 mg, 524 µl, 3 mmol, Eq: 2.5) in DMF/DCM 1:1 (4.65 mL) was treated with HATU (570 mg, 1.5 mmol, Eq: 1.25) and stirred for 10 minutes. The resulting mixture was added to the resin and shaken for 18 hours. The resin was filtered and washed 3 x with 5 mL DMF and 3 × with 5 mL DCM. Then the resin was treated with 5 mL DCM/HFIP 4:1 and shaken 1 hour. The mixture was filtered and washed 3x with DCM. This cleavage procedure was repeated 1 more time. The obtained filtrates were combined and concentrated in vacuo. The obtained oil was redisolved in acetonitrile/water and was lyophilized. The compound Boc-Glu(OtBu)-Agp(Boc)2-OH (156 mg, 247 µmol, 32.9 % yield) was obtained as light brown lyoph powder and was used without further purification. MS ESI (m/z): 632.5 [M+H]⁺

### Step 2: tert-butyl (12S,E)-6,12-bis((tert-butoxycarbonyl)amino)-9-((3-(4-((3-(2,3-difluoro-4-methoxyphenyl)imidazo[1,2-a]pyrazin-8-yl)amino)-2-ethylbenzamido)propyl)carbamoyl)-2,2-dimethyl-4,11-dioxo-3-oxa-5,7,10-triazapentadec-5-en-15-oate (Epimers 1:1)

To a solution of Boc-Glu(OtBu)-Agp(Boc)₂-OH (74.2 mg, 117 µmol, Eq: 1.3) and DIPEA (46.7 mg, 63.1 µl, 361 µmol, Eq: 4) in dry DMF (452 µl) was added HATU (44.7 mg, 117 µmol, Eq: 1.3) and the mixture stirred 10 minutes at room temperature. Then N-(3-aminopropyl)-4-((3-(2,3-difluoro-4-methoxyphenyl)imidazo[1,2-a]pyrazin-8-yl)amino)-2-ethylbenzamide dihydrochloride (Reference Example 332, 50 mg, 90.3 µmol, Eq: 1) was added and stirring at room temperature continued for 1.5 hours, then stored in the fridge for 16 hours. The reaction was concentrated in vacuo at 45 °C. The crude material was purified by silica gel chromatography using dichloromethane/ methanol as eluent. The obtained material was further purified by preparative reversed phase HPLC (Column: Phenomenex Gemini-NX 5u 110A, l: 100 mm, dia: 30 mm) using acetonitrile / water containing 0.1% triethylamine as eluent.

The compound tert-butyl (12S,E)-6,12-bis((tert-butoxycarbonyl)amino)-9-((3-(4-((3-(2,3-difluoro-4-methoxyphenyl)imidazo[1,2-a]pyrazin-8-yl)amino)-2-ethylbenzamido)propyl)carbamoyl)-2,2-dimethyl-4,11-dioxo-3-oxa-5,7,10-triazapentadec-5-en-15-oate (Epimers 1:1, 0.035 g, 30.7 µmol, 34 % yield) was obtained as off-white amorphous with a purity of 96 % (total UV, 210-400 nm). MS ESI (m/z): 1080.5 [M+H]⁺

### Step 3: (4S)-4-amino-5-((1-((3-(4-((3-(2,3-difluoro-4-methoxyphenyl)imidazo[1,2-a]pyrazin-8-yl)amino)-2-ethylbenzamido)propyl)amino)-3-guanidino-1-oxopropan-2-yl)amino)-5-oxopentanoic acid trihydrochloride (Epimers 1:1)

To a solution of tert-butyl (12S,E)-6,12-bis((tert-butoxycarbonyl)amino)-9-((3-(4-((3-(2,3-difluoro-4-methoxyphenyl)imidazo[1,2-a]pyrazin-8-yl)amino)-2-ethylbenzamido)propyl)carbamoyl)-2,2-dimethyl-4,11-dioxo-3-oxa-5,7,10-triazapentadec-5-en-15-oate (0.035 g, 32 µmol, Eq: 1) in dioxane (107 µL) was added 4M HCl in dioxane (480 µL, 1.92 mmol, Eq: 60) and the mixture stirred 16 hours at room temperature. Then the reaction was diluted with more dioxane and directly lyophilized. The compound (4S)-4-amino-5-((1-((3-(4-((3-(2,3-difluoro-4-methoxyphenyl)imidazo[1,2-a]pyrazin-8-yl)amino)-2-ethylbenzamido)propyl)amino)-3-guanidino-1-oxopropan-2-yl)amino)-5-oxopentanoic acid trihydrochloride (30 mg, 29.7 µmol, 93 % yield) was obtained as white lyoph powder with a purity of 84 % (96% by total UV (210-400nm), 13% dioxane acc to NMR). MS ESI (m/z): 738.4 [M+H]⁺]

The following examples were prepared in analogy to Reference Example 510.

| Ex. | Name | Structure | Color and form, analytics | Starting Materials |
|---|---|---|---|---|
| REF 511 | (S)-4-amino-5-(((S)-1-((3-(4-((3-(2,3-difluoro-4-methoxyphenyl)imidaz o[1,2-a]pyrazin-8-yl)amino)-2-ethylbenzamido)propyl) amino)-5-guanidino-1- oxopentan-2-yl)amino)-5-oxopentanoic acid trihydrochloride | 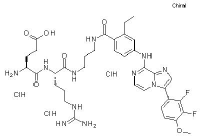 | Off-white lyophilized powder, MS ESI (m/z): 766.4 [M+H]⁺ | Fmoc-Arg(Boc)₂-OH, Boc-Glu-OtBu |

### Reference Example 512

### (S)-7-amino-1-(4-((3-(2,3-difluoro-4-methoxyphenyl)imidazo[1,2-a]pyrazin-8-yl)amino)-2-ethylphenyl)-12-imino-1,6-dioxo-2,5,11,13-tetraazaheptadecan-17-oic acid trihydrochloride

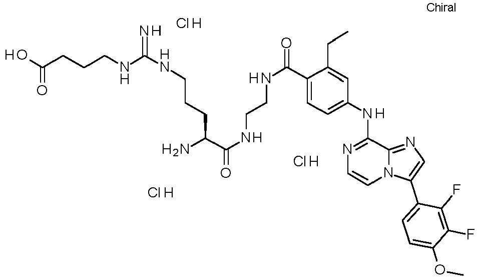

### Step 1: benzyl tert-butyl (5-((2-(4-((3-(2,3-difluoro-4-methoxyphenyl)imidazo[1,2-a]pyrazin-8-yl)amino)-2-ethylbenzamido)ethyl)amino)-5-oxopentane-1,4-diyl)(S)-dicarbamate

To a solution of Boc-Orn(Z)-OH (112 mg, 306 µmol, Eq: 1.1) in dry DMF (1.39 mL) and DIPEA (180 mg, 243 µL, 1.39 mmol, Eq: 5) was added HATU (116 mg, 306 µmol, Eq: 1.1) and the mixture stirred 10 minutes at room temperature. Then N-(2-aminoethyl)-4-((3-(2,3-difluoro-4-methoxyphenyl)imidazo[1,2-a]pyrazin-8-yl)amino)-2-ethylbenzamide dihydrochloride (Reference Example 331, 0.15 g, 278 µmol, Eq: 1) was added and stirring at room temperature continued for 2 hours. Then the reaction mixture was diluted with ethyl acetate, water and sat. aqueous NaHCO₃ solution. The mixture was extracted 2 × with ethyl acetate and the organic layers were washed 2 × with brine. The combined organic layers were dried with sodium sulfate, filtered and concentrated in vacuo. The crude material was purified by silica gel chromatography using dichloromethane/ methanol as eluent. The compound benzyl tert-butyl (5-((2-(4-((3-(2,3-difluoro-4-methoxyphenyl)imidazo[1,2-a]pyrazin-8-yl)amino)-2-ethylbenzamido)ethyl)amino)-5-oxopentane-1,4-diyl)(S)-dicarbamate (0.207 g, 254 µmol, 91.3 % yield) was obtained as light brown amorphous solid. MS ESI (m/z): 815.6 [M+H]⁺

### Step 2: tert-butyl (S)-(5-amino-1-((2-(4-((3-(2,3-difluoro-4-methoxyphenyl)imidazo[1,2-a]pyrazin-8-yl)amino)-2-ethylbenzamido)ethyl)amino)-1-oxopentan-2-yl)

A suspension of benzyl tert-butyl (5-((2-(4-((3-(2,3-difluoro-4-methoxyphenyl)imidazo[1,2-a]pyrazin-8-yl)amino)-2-ethylbenzamido)ethyl)amino)-5-oxopentane-1,4-diyl)(S)-dicarbamate (0.207 g, 254 µmol, Eq: 1) in methanol (2.54 mL) was evacuated 3 × (frothing) and flushed with argon. Then 10% palladium on charcoal (27 mg, 25.4 µmol, Eq: 0.1) was added and degassing repeated. Then again the mixture was evacuated 3 × (frothing) and flushed with hydrogen. The reaction was stirred 3 hours at room temperature under hydrogen. Then the reaction was diluted with ethanol (2.54 mL) and stirring under hydrogen continued for another 20 hours. The reaction mixture was filtered and washed with EtOH and dichloromethane/MeOH 9:1. The obtained solution was concentrated in vacuo. The compound tert-butyl (S)-(5-amino-1-((2-(4-((3-(2,3-difluoro-4-methoxyphenyl)imidazo[1,2-a]pyrazin-8-yl)amino)-2-ethylbenzamido)ethyl)amino)-1-oxopentan-2-yl)carbamate (162 mg, 238 µmol, 93.7 % yield) was obtained as light brown amorphous solid and was used without further purification. MS ESI (m/z): 681.5 [M+H]⁺

### Step 3: tert-butyl (S,Z)-13-(tert-butoxycarbonyl)-7-((tert-butoxycarbonyl)amino)-12-((tert-butoxycarbonyl)imino)-1-(4-((3-(2,3-difluoro-4-methoxyphenyl)imidazo[1,2-a]pyrazin-8-yl)amino)-2-ethylphenyl)-1,6-dioxo-2,5,11,13-tetraazaheptadecan-17-oate

A solution of tert-butyl (E)-4-(N,N'-bis(tert-butoxycarbonyl)-1H-pyrazole-1-carboximidamido)butanoate (Intermediate 141, 21.4 mg, 44.1 µmol, Eq: 1) in acetonitrile (294 µl) was added to tert-butyl (S)-(5-amino-1-((2-(4-((3-(2,3-difluoro-4-methoxyphenyl)imidazo[1,2-a]pyrazin-8-yl)amino)-2-ethylbenzamido)ethyl)amino)-1-oxopentan-2-yl)carbamate (30 mg, 44.1 µmol, Eq: 1). To the resulting suspension DIPEA (12.5 mg, 16.9 µL, 97 µmol, Eq: 2.2) was added and the reaction stirred 16 hours at room temperature. Again tert-butyl (E)-4-(N,N'-bis(tert-butoxycarbonyl)-1H-pyrazole-1-carboximidamido)butanoate (Intermediate 141, 9.97 mg, 22 µmol, Eq: 0.5) in acetonitrile (147 µl) was added and stirring at room temperature continued. Then the reaction was concentrated in vacuo. The crude material was purified by silica gel chromatography using heptane/ (ethyl acetate/EtOH/NH4OH 75:25:2) as eluent. The compound tert-butyl (S,Z)-13-(tert-butoxycarbonyl)-7-((tert-butoxycarbonyl)amino)-12-((tert-butoxycarbonyl)imino)-1-(4-((3-(2,3-difluoro-4-methoxyphenyl)imidazo[1,2-a]pyrazin-8-yl)amino)-2-ethylphenyl)-1,6-dioxo-2,5,11,13-tetraazaheptadecan-17-oate (0.014 g, 13.1 µmol, 29.8 % yield) was obtained as colorless amorphous solid and was used without further purification. MS ESI (m/z): 1065.6 [M+H]⁺

### Step 4: (S)-7-amino-1-(4-((3-(2,3-difluoro-4-methoxyphenyl)imidazo[1,2-a]pyrazin-8-yl)amino)-2-ethylphenyl)-12-imino-1,6-dioxo-2,5,11,13-tetraazaheptadecan-17-oic acid trihydrochloride

To a suspension of tert-butyl (S,Z)-13-(tert-butoxycarbonyl)-7-((tert-butoxycarbonyl)amino)-12-((tert-butoxycarbonyl)imino)-1-(4-((3-(2,3-difluoro-4-methoxyphenyl)imidazo[1,2-a]pyrazin-8-yl)amino)-2-ethylphenyl)-1,6-dioxo-2,5,11,13-tetraazaheptadecan-17-oate (13 mg, 12.2 µmol, Eq: 1) in dioxane (40.7 µL) was added 4M HCl in dioxane (305 µL, 1.22 mmol, Eq: 100) and the resulting solution stirred at room temperature for 4 hours. The reaction was diluted with dioxane and directly lyophilized. The compound (S)-7-amino-1-(4-((3-(2,3-difluoro-4-methoxyphenyl)imidazo[1,2-a]pyrazin-8-yl)amino)-2-ethylphenyl)-12-imino-1,6-dioxo-2,5,11,13-tetraazaheptadecan-17-oic acid trihydrochloride (7 mg, 8.13 µmol, 66.6 % yield) was obtained as white lyoph powder with a purity of 95 % (UV, 265 nm). MS ESI (m/z): 709.3379 [M+H]⁺

### Example 11

### Cis N-(3-aminocyclobutyl)-4-[[3-(2,3-difluoro-4-methoxy-phenyl)imidazo[1,2-a]pyrazin-8-yl]amino]-2-ethyl-benzamide

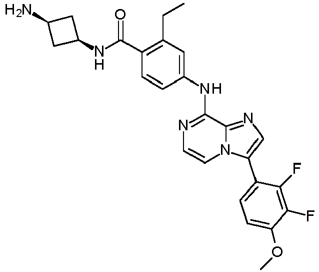

### Step 1:

### Cis-tert-butyl N-[3-[[4-[[3-(2,3-difluoro-4-methoxy-phenyl)imidazo[1,2-a]pyrazin-8-yl]amino]-2-ethyl-benzoyl]amino]cyclobutyl]carbamate

Under Ar, 4-((3-(2,3-difluoro-4-methoxyphenyl)imidazo[1,2-a]pyrazin-8-yl)amino)-2-ethylbenzoic acid, Intermediate 86 (100 mg, 236 µmol, Eq: 1) was suspended in DMF (1 mL). tert-Butyl cis-N-(3-aminocyclobutyl)carbamate [CAS#1212395-34-0] (1.18 mmol, Eq: 5) was added. Additional tert-Butyl cis-N-(3-aminocyclobutyl)carbamate [CAS#1212395-34-0] (283 µmol, Eq: 1.2) and 2-(3H-[1,2,3]triazolo[4,5-b]pyridin-3-yl)-1,1,3,3-tetramethylisouronium hexafluorophosphate(V) (HATU) (179 mg, 471 µmol, Eq: 2) were added and the yellow solution was stirred at RT over 2 h. The solvent was evaporated and the crude material was purified by flash chromatography (silica gel, 20g, 0% to 100% DCM/MeOH/NH4OH (95/5/1)) leading to the target compound (orange foam, 140 mg). MS (ESI, m/z): 593.3 [M+H]+.

### Step 2:

### Cis N-(3-aminocyclobutyl)-4-[[3-(2,3-difluoro-4-methoxy-phenyl)imidazo[1,2-a]pyrazin-8-yl] amino]-2-ethyl-benzamide

Under Ar, cis-tert-butyl N-[3-[[4-[[3-(2,3-difluoro-4-methoxy-phenyl)imidazo[1,2-a]pyrazin-8-yl]amino]-2-ethyl-benzoyl]amino]cyclobutyl] carbamate obtained in step 1 (140 mg) was dissolved in MeOH (1 mL).4M HCl in dioxane (1.07 mL, 4.27 mmol, Eq: 10) was added and the RM was stirred at RT over 3 h. DCM/MeOH/aq. NH₃ was added till the HCl was neutralized and the RM was evaporated. The crude product was purified by flash chromatography (silica gel, 20g, 0% to 100% DCM/MeOH/aq. 25% NH₄OH (90/10/1)) leading to the title compound (white solid, 103 mg). MS (ESI, m/z): 493.2 [M+H]+.

The following examples were prepared in analogy to example 11.

| Ex. | Name | Structure | ESI MS [M+H ]⁺ | Starting Materials |
|---|---|---|---|---|
| 12 | N-(azetidin-3-yl)-4-[[3-(2,3-difluoro-4-methoxy-phenyl)imidazo[1,2-a]pyrazin-8-yl]amino]-2-ethyl-benzamide | 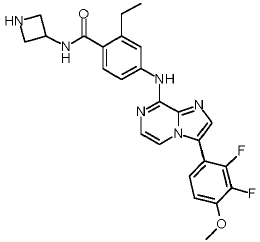 | 479.3 | Intermediate 86 and tert-butyl 3-aminoazetidine-1-carboxylate. Deprotection with TFA/DCM 1:2 at rt for 1 h. |
| 13 | 4-[[3-(2,3-difluoro-4-methoxyphenyl)imidazo[1,2-a]pyrazin-8-yl]amino]-2-ethyl-N-(4-piperidyl)benzamide | 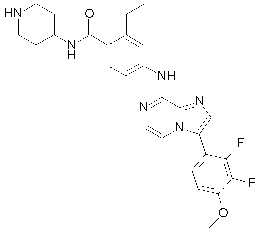 | 507.3 | Intermediate 86 and tert-butyl 4-aminopiperidine-1-carboxylate |
| 6 | Cis-N-(4-aminocyclohexyl)-4-[[3-(2,3-difluoro-4-methoxy-phenyl)imidazo[1,2-a]pyrazin-8-yl]amino]-2-ethyl-benzamide | 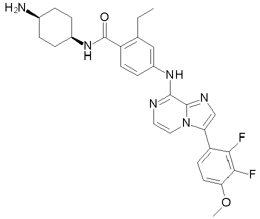 | 521.3 | Intermediate 86 and cis-tert-butyl (-4-aminocyclohexyl)ca rbamate |
| 14 | N-(3 -aminocyclobutyl)-4-[[3-(2,3-difluoro-4-methoxyphenyl)imidazo[1,2-a]pyrazin-8-yl] amino]-2-ethyl-benzamide | 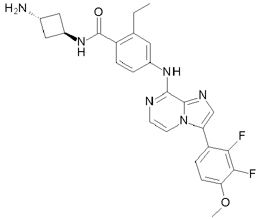 | 493.3 | Intermediate 86 and trans-tert-butyl (3-aminocyclobutyl)ca rbamate |
| REF 543 | N-(3-amino-2-hydroxypropyl)-4-[[3-(2,3-difluoro-4-methoxyphenyl)imidazo[1,2-a]pyrazin-8-yl]amino]-2-ethyl-benzamide;hydrochlori de | 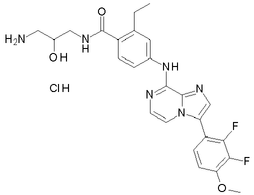 | 495.4 | 86 and tert-butyl (3-amino-2-hydroxypropyl)carb amate [CAS# 144912-84-5]. Product isolated by filtration of reaction mixture following deprotection step. |

### Reference Example 513

### 4-[4-[[3-[4-(difluoromethoxy)phenyl]imidazo[1,2-a]pyrazin-8-yl]amino]-2-methyl-benzoyl]-N-(4-pyridyl)piperazine-1-carboxamide

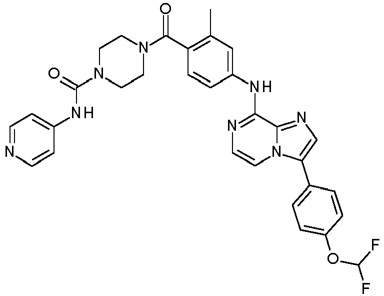

To a solution of [4-[[3-[4-(difluoromethoxy)phenyl]imidazo[1,2-a]pyrazin-8-yl]amino]-2-methyl-phenyl]-piperazin-1-yl-methanone hydrochloride (Reference Example 329) (100.0 mg, 0.210 mmol, 1 eq) in DMF (5 mL) was added phenyl N-(4-pyridyl)carbamate (67.16 mg, 0.310 mmol, 1.5 eq) and N,N-diisopropylethylamine (0.11 mL, 0.630 mmol, 3 eq), then the reaction was stirred at 25 °C for 12 h. The reaction mixture was purified by prep-HPLC (basic) to give product 4-[4-[[3-[4-(difluoromethoxy)phenyl]imidazo[1,2-a]pyrazin-8-yl]amino]-2-methylbenzoyl]-N-(4-pyridyl)piperazine-1-carboxamide (30.5 mg, 0.050 mmol, 24% yield) as a yellow solid. MS (ESI, m/z): 599.1 [M+H]+.

### Reference Example 514

### N-[2-(2-aminoethoxy)ethyl]-4-[[3-(4-but-2-ynoxyphenyl)imidazo[1,2-a]pyrazin-8-yl]amino]-2-methyl-benzamide; 2,2,2-trifluoroacetic acid

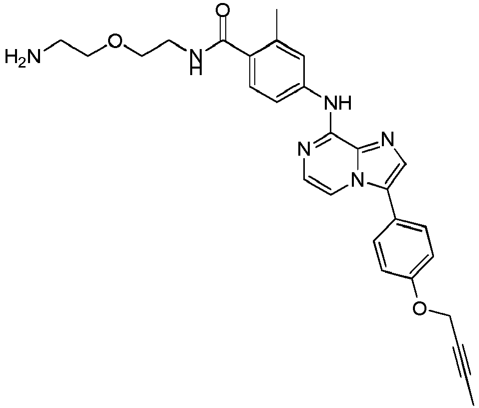

### Step 1: tert-butyl N-[2-[2-[(2-methyl-4-nitro-benzoyl)amino]ethoxy]ethyl]carbamate

The title compound was obtained in analogy to step 1 in the preparation of Reference Example 12 using tert-butyl N-[2-(2-aminoethoxy)ethyl]carbamate and 2-methyl-4-nitro-benzoic acid for condensation. MS (ESI) m/z: 390.1 [M+Na]⁺

### Step 2: tert-butyl N-[2-[2-[(4-amino-2-methyl-benzoyl)amino]ethoxy]ethyl]carbamate

The title compound was obtained in analogy to step 4 in the preparation of Reference Example 486 using tert-butyl N-[2-[2-[(2-methyl-4-nitro-benzoyl)amino]ethoxy]ethyl]carbamate as starting material in hydrogenation. MS (ESI) m/z: 360.2 [M+Na]⁺

### Step 3: 4-(8-chloroimidazo[1,2-a]pyrazin-3-yl)phenol

The title compound was obtained in analogy to step 1 in the preparation of Reference Example 515 using 8-chloro-3-iodo-imidazo[1,2-a]pyrazine and (4-hydroxyphenyl)boronic acid as reaction parteners. MS (ESI) m/z: 245.9 [M+H]⁺

### Step 4: 3-(4-but-2-ynoxyphenyl)-8-chloro-imidazo[1,2-a]pyrazine

A mixture of 4-(8-chloroimidazo[1,2-a]pyrazin-3-yl)phenol (100.0 mg, 0.410 mmol, 1 eq), 1-bromo-2-butyne (81.2 mg, 0.610 mmol, 1.5 eq) and potassium carbonate (112.52 mg, 0.810 mmol, 2 eq) in DMF (3 mL) was stirred at 25 °C for 16 h. The mixture was filtered and the obtained crude product was purified by flash column to afford 78 mg of 3-(4-but-2-ynoxyphenyl)-8-chloro-imidazo[1,2-a]pyrazine as yellow solid. MS (ESI) m/z: 298.0 [M+H]⁺

### Step 5: tert-butyl N-[2-[2-[[4-[[3-(4-but-2-ynoxyphenyl)imidazo[1,2-a]pyrazin-8-yl]amino]-2-methyl-benzoyl]amino]ethoxy]ethyl]carbamate

A mixture of tert-butyl N-[2-[2-[(4-amino-2-methyl-benzoyl)amino]ethoxy] ethyl] carbamate (80.0 mg, 0.240 mmol, 1 eq), 3-(4-but-2-ynoxyphenyl)-8-chloro-imidazo[1,2-a]pyrazine (70.59 mg, 0.240 mmol, 1 eq), cesium carbonate (231.76 mg, 0.710 mmol, 3 eq), tris(dibenzylideneacetone)dipalladium (0) (21.71 mg, 0.020 mmol, 0.100 eq) and 9,9-dimethyl-4,5-bis(diphenylphosphino)xanthene (13.72 mg, 0.020 mmol, 0.100 eq) in 1,4-dioxane (5 mL) was stirred under N₂ at 115 °C under microwave irradiation for 2 h. The mixture was filtered and concentrated to give the crude product, which was purified by prep-TLC (DCM/MeOH/MeCN = 10:1:1) to afford 65 mg of the title compound as light yellow solid. MS (ESI) m/z: 599.3. [M+H]⁺

### Step 6: N-[2-(2-aminoethoxy)ethyl]-4-[[3-(4-but-2-ynoxyphenyl)imidazo[1,2-a]pyrazin-8-yl]amino]-2-methyl-benzamide; 2,2,2-trifluoroacetic acid

A solution of tert-butyl N-[2-[2-[[4-[[3-(4-but-2-ynoxyphenyl)imidazo[1,2-a]pyrazin-8-yl]amino] -2-methyl-benzoyl]amino]ethoxy]ethyl]carbamate (65.0 mg, 0.110 mmol, 1 eq) in DCM (4 mL) was added trifluoroacetic acid (0.5 mL, 6.49 mmol, 59.78 eq) and stirred at 20 °C for 16 h. The mixture was concentrated and the obtained residue was purified by prep-HPLC (TFA) to afford 20.6 mg of the title compound as white solid. MS (ESI) m/z: 499.2. [M+H]⁺

### Reference Example 516

### N-[2-(2-aminoethoxy)ethyl]-4-[[3-[4-(cyanomethoxy)-3-fluoro-phenyl]imidazo[1,2-a]pyrazin-8-yl]amino]-2-methyl-benzamide; formic acid

### Step 1: 4-(8-chloroimidazo[1,2-a]pyrazin-3-yl)-2-fluoro-phenol

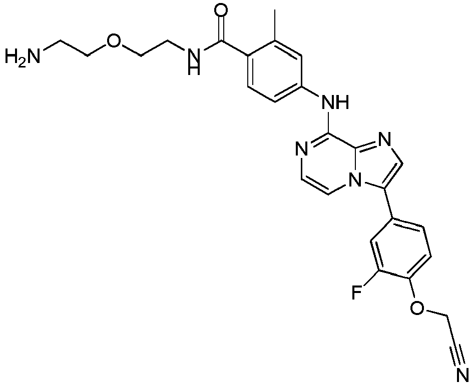

The title compound was obtained in analogy to step 1 in the preparation of Reference Example 515 using 8-chloro-3-iodo-imidazo[1,2-a]pyrazine and 3-fluoro-4-hydroxyphenylboronic acid as starting compounds. MS (ESI) m/z: 264.0 [M+H]⁺

### Step 2: 2-[4-(8-chloroimidazo[1,2-a]pyrazin-3-yl)-2-fluoro-phenoxy]acetonitrile

The title compound was obtained in analogy to step 4 in the preparation of Reference Example 514 using 4-(8-chloroimidazo[1,2-a]pyrazin-3-yl)-2-fluoro-phenol and bromoacetonitrile as reactants. MS (ESI) m/z: 303.0. [M+H]⁺

### Step 3: tert-butyl N-[2-[2-[[4-[[3-[4-(cyanomethoxy)-3-fluoro-phenyl]imidazo[1,2-a]pyrazin-8-yl]amino]-2-methyl-benzoyl]amino]ethoxy]ethyl]carbamate

The title compound was obtained in analogy to step 5 in the preparation of Reference Example 514 using tert-butyl N-[2-[2-[(4-amino-2-methyl-benzoyl)amino]ethoxy]ethyl]carbamate and 2-[4-(8-chloroimidazo[1,2-a]pyrazin-3-yl)-2-fluoro-phenoxy]acetonitrile as coupling reactants. MS (ESI) m/z: 604.5 [M+H]⁺

### Step 4: N-(2-(2-aminoethoxy)ethyl)-4-((3-(4-(cyanomethoxy)-3-fluorophenyl)imidazo[1,2-a]pyrazin-8-yl)amino)-2-methylbenzamide; formic acid

The title compound was obtained in analogy to step 6 in the preparation of Reference Example 514 using tert-butyl N-[2-[2-[[4-[[3-[4-(cyanomethoxy)-3-fluoro-phenyl]imidazo[1,2-a]pyrazin-8-yl]amino]-2-methyl-benzoyl]amino]ethoxy]ethyl]carbamate as starting material. MS (ESI) m/z: 504.2 [M+H]⁺

### Reference Example 517

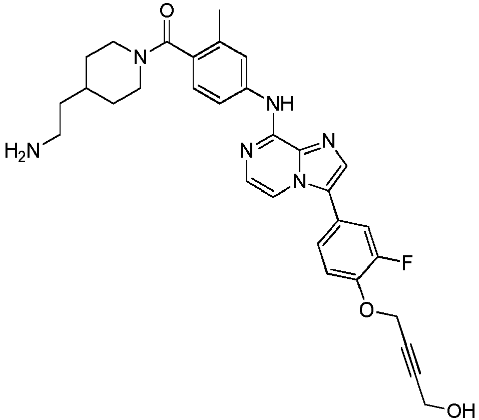

### Step 1: 4-hydroxybut-2-yn-1-yl methanesulfonate

The title compound was obtained in analogy to step 4 in the preparation of REF 518 using but-2-yne-1,4-diol as starting material. The product was directly used in crude form.

### Step 2: 4-[4-(8-chloroimidazo[1,2-a]pyrazin-3-yl)-2-fluoro-phenoxy]but-2-yn-1-ol

The title compound was obtained in analogy to step 7 in the preparation of Reference Example 518 using 4-(8-chloroimidazo[1,2-a]pyrazin-3-yl)-2-fluoro-phenol and 4-hydroxybut-2-ynyl methanesulfonate as starting material. MS (ESI) m/z: 332.0 [M+H]⁺

### Step 3: tert-butyl N-[2-[1-(4-amino-2-methyl-benzoyl)-4-piperidyl]ethyl]carbamate

The title compound was obtained in analogy to step 1 in the preparation of Reference Example 518 using 4-amino-2-methyl benzoic acid and 4-(2-BOC-aminoethyl)piperidine for. MS (ESI) m/z: 362.2 [M+H]⁺

### Step 4: tert-butyl N-[2-[1-[4-[[3-[3-fluoro-4-(4-hydroxybut-2-ynoxy)phenyl]imidazo[1,2-a]pyrazin-8-yl]amino]-2-methyl-benzoyl]-4-piperidyl]ethyl]carbamate

The title compound was obtained in analogy to step 2 in the preparation of Reference Example 515 using tert-butyl N-[2-[1-(4-amino-2-methyl-benzoyl)-4-piperidyl]ethyl]carbamate and 4-[4-(8-chloroimidazo[1,2-a]pyrazin-3-yl)-2-fluoro-phenoxy]but-2-yn-1-ol. MS (ESI) m/z: 657.4 [M+H]⁺

### Step 5: [4-(2-aminoethyl)-1-piperidyl]-[4-[[3-[3-fluoro-4-(4-hydroxybut-2-ynoxy)phenyl]imidazo[1,2-a]pyrazin-8-yl]amino]-2-methyl-phenyl]methanone

The title compound was obtained in analogy to step 2 in the preparation of Reference Example 12 using tert-butyl N-[2-[1-[4-[[3-[3-fluoro-4-(4-hydroxybut-2-ynoxy)phenyl]imidazo[1,2-a]pyrazin-8-yl]amino]-2-methyl-benzoyl]-4-piperidyl]ethyl]carbamate as substrate. MS (ESI) m/z: 557.4 [M+H]⁺

### Reference Example 519

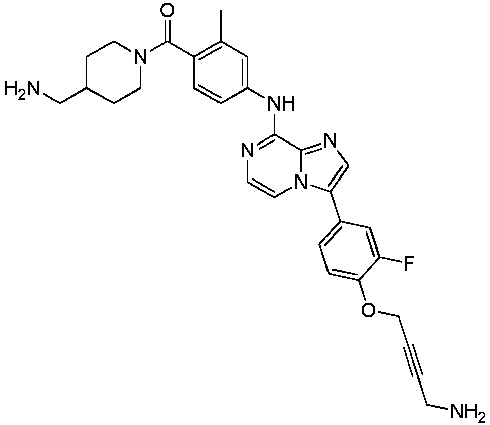

### [4-[[3-[4-(4-aminobut-2-ynoxy)-3-fluoro-phenyl]imidazo[1,2-a]pyrazin-8-yl]amino]-2-methyl-phenyl]-[4-(aminomethyl)-1-piperidyl]methanone; formic acid

### Step 1: 4-((tert-butoxycarbonyl)amino)but-2-yn-1-yl methanesulfonate

The title compound was obtained in analogy to step 4 in the preparation of Reference Example 518 using tert-butyl (4-hydroxybut-2-yn-1-yl)carbamate as starting material. The product was directly used in crude form.

### Step 2: tert-butyl N-[4-[4-(8-chloroimidazo[1,2-a]pyrazin-3-yl)-2-fluoro-phenoxy]but-2-ynyl]carbamate

The title compound was obtained in analogy to step 7 in the preparation of Reference Example 518 using 4-(8-chloroimidazo[1,2-a]pyrazin-3-yl)-2-fluoro-phenol and 4-(tert-butoxycarbonylamino)but-2-ynyl methanesulfonate as starting materials. MS (ESI) m/z: 431.0 [M+H]⁺

### Step 3: tert-butyl ((1-(2-methyl-4-nitrobenzoyl)piperidin-4-yl)methyl)carbamate

The title compound was obtained in analogy to step 1 in the preparation of Reference Example 12 using 4-amino-2-methyl benzoic acid and 4-(tert-butoxycarbonylaminomethyl)piperidine. MS (ESI) m/z: 400.1 [M+Na]⁺

### Step 4: tert-butyl N-[[1-(4-amino-2-methyl-benzoyl)-4-piperidyl]methyl]carbamate

To a stirred suspension mixture of NiCl₂.6H₂O (503.81 mg, 2.12 mmol, 0.500 eq) and sodium borohydride (80.19 mg, 2.12 mmol, 0.500 eq) in methanol (20 mL) was dropwise added a solution of tert-butyl N-[[1-(2-methyl-4-nitro-benzoyl)-4-piperidyl]methyl]carbamate (1.6 g, 4.24 mmol, 1 eq) in THF (6 mL) at 0 °C. Then another batch of sodium borohydride (481.14 mg, 12.72 mmol, 3 eq) was added at 0 °C and the reaction mixture was stirred for 1 h at 10 °C. The solid was filtered and the solvent was removed in vacuum. The residue was treated with a mixture of EA/H₂O (1/1, 200 mL), the organic layer was washed with sat. NH₄Cl (50 mL) and brine (50 mL), dried over sodium sulfate and filtered, concentrated in vacuum to give tert-butyl N-[[1-(4-amino-2-methyl-benzoyl)-4-piperidyl]methyl]carbamate (1.44 g, 4.14 mmol, 97.77 % yield) as white solid. MS (ESI) m/z: 348.1 [M+H]⁺

### Step 5: tert-butylN-[[1-[4-[[3-[4-[4-(tert-butoxycarbonylamino)but-2-ynoxy]-3-fluoro-phenyl]imidazo[1,2-a]pyrazin-8-yl]amino]-2-methyl-benzoyl]-4-piperidyl]methyl]carbamate

The title compound was obtained in analogy to step 2 in the preparation of Reference Example 515 using tert-butyl N-[[1-(4-amino-2-methyl-benzoyl)-4-piperidyl]methyl]carbamate and tert-butyl N-[4-[4-(8-chloroimidazo[1,2-a]pyrazin-3-yl)-2-fluoro-phenoxy]but-2-ynyl]carbamate. MS (ESI) m/z: 742.5 [M+H]⁺

### Step 6: [4-[[3-[4-(4-aminobut-2-ynoxy)-3-fluoro-phenyl]imidazo[1,2-a]pyrazin-8-yl]amino]-2-methyl-phenyl]-[4-(aminomethyl)-1-piperidyl]methanone; formic acid

The title compound was obtained in analogy to step 2 in the preparation of Reference Example 12 using tert-butyl N-[[1-[4-[[3-[4-[4-(tert-butoxycarbonylamino)but-2-ynoxy]-3-fluoro-phenyl]imidazo[1,2-a]pyrazin- 8-yl] amino]-2-methyl-benzoyl] -4-piperidyl] methyl] carbamate. MS (ESI) m/z: 542.3 [M+H]⁺

### Reference Example 520

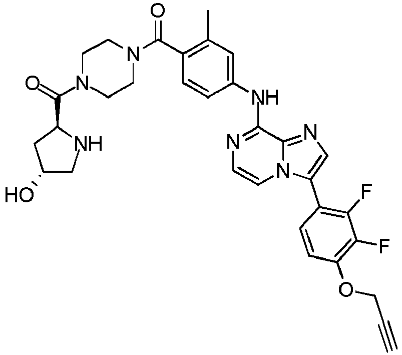

### Step 1: tert-butyl 4-(2-methyl-4-nitrobenzoyl)piperazine-1-carboxylate

The title compound was obtained in analogy to step 3 in the preparation of Reference Example 515 using 2-methyl-4-nitrobenzoic acid and tert-butyl piperazine-1-carboxylate. MS (ESI) m/z: 350.2. [M+H]⁺

### Step 2: tert-butyl 4-(4-amino-2-methylbenzoyl)piperazine-1-carboxylate

The title compound was obtained in analogy to step 4 in the preparation of Reference Example 486 using tert-butyl 4-(2-methyl-4-nitrobenzoyl)piperazine-1-carboxylate as substrate. MS (ESI) m/z: 320.2. [M+H]⁺

### Step 3: tert-butyl 4-[4-[[3-(2,3-difluoro-4-prop-2-ynoxy-phenyl)imidazo[1,2-a]pyrazin-8-yl]amino]-2-methyl-benzoyl]piperazine-1-carboxylate

The title compound was obtained in analogy to step 2 in the preparation of Reference Example 515 using tert-butyl 4-(4-amino-2-methyl-benzoyl)piperazine-1-carboxylate and 8-chloro-3-(2,3-difluoro-4-prop-2-ynoxy-phenyl)imidazo[1,2-a]pyrazine. MS (ESI) m/z: 603.1 [M+H]⁺

### Step 4: [4-[[3-(2,3-difluoro-4-prop-2-ynoxy-phenyl)imidazo[1,2-a]pyrazin-8-yl]amino]-2-methyl-phenyl]-piperazin-1-yl-methanone

The title compound was obtained in analogy to step 2 in the preparation of Reference Example 12 using tert-butyl 4-[4-[[3-(2,3-difluoro-4-prop-2-ynoxy-phenyl)imidazo[1,2-a]pyrazin-8-yl]amino]-2-methyl-benzoyl]piperazine-1-carboxylate as substrate. MS (ESI) m/z: 503.3. [M+H]⁺

### Step 5: tert-butyl (2S,4R)-2-[4-[4-[[3-(2,3-difluoro-4-prop-2-ynoxy-phenyl)imidazo[1,2-a]pyrazin-8-yl]amino]-2-methyl-benzoyl]piperazine-1-carbonyl]-4-hydroxy-pyrrolidine-1-carboxylate

The title compound was obtained in analogy to step 3 in the preparation of Reference Example 515 using [4-[[3-(2,3-difluoro-4-prop-2-ynoxy-phenyl)imidazo[1,2-a]pyrazin-8-yl]amino]-2-methyl-phenyl]-piperazin-1-yl-methanone and (2S,4R)-1-(tert-butoxycarbonyl)-4-hydroxypyrrolidine-2-carboxylic acid. MS (ESI) m/z: 716.3. [M+H]⁺

### Step 6: [4-[[3-(2,3-difluoro-4-prop-2-ynoxy-phenyl)imidazo[1,2-a]pyrazin-8-yl]amino]-2-methyl-phenyl]-[4-[(2S,4R)-4-hydroxypyrrolidine-2-carbonyl]piperazin-1-yl]methanone

The title compound was obtained in analogy to step 2 in the preparation of Reference Example 12 using tert-butyl (2S,4R)-2-[4-[4-[[3-(2,3-difluoro-4-prop-2-ynoxy-phenyl)imidazo[1,2-a]pyrazin-8-yl]amino]-2-methyl-benzoyl]piperazine-1-carbonyl]-4-hydroxy-pyrrolidine-1-carboxylate as substrate. MS (ESI) m/z: 616.3. [M+H]⁺

### Reference Example 521

### [4-(3-aminocyclobutanecarbonyl)piperazin-1-yl]-[4-[[3-[4-(difluoromethoxy)phenyl]imidazo[1,2-a]pyrazin-8-yl]amino]-2-methyl-phenyl]methanone hydrochloride

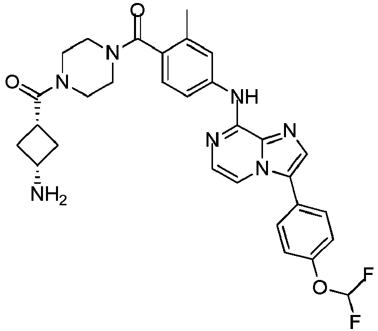

The title compound was obtained in analogy to Reference Example 522 using (1S,3S)-3-((tert-butoxycarbonyl)amino)cyclobutanecarboxylic acid instead of (1R,3R)-3-((tert-butoxycarbonyl)amino)cyclobutanecarboxylic acid. MS (ESI, m/z): 576.2 [M+H]⁺

### Reference Example 522

### [4-(3-aminocyclobutanecarbonyl)piperazin-1-yl]-[4-[[3-[4-(difluoromethoxy)phenyl]imidazo[1,2-a]pyrazin-8-yl]amino]-2-methyl-phenyl]methanone hydrochloride

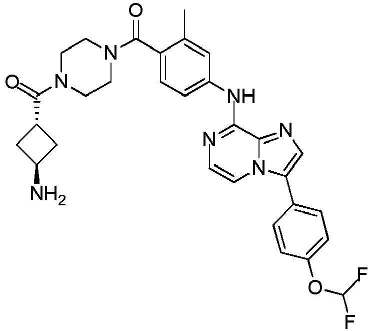

### Step 1: tert-butyl 4-(4-((3-(4-(difluoromethoxy)phenyl)imidazo[1,2-a]pyrazin-8-yl)amino)-2-methylbenzoyl)piperazine-1-carboxylate

The title compound was obtained in analogy to step 2 in the preparation of Reference Example 515 using 8-chloro-3-[4-(difluoromethoxy)phenyl]imidazo[1,2-a]pyrazine and tert-butyl 4-(4-amino-2-methyl-benzoyl)piperazine-1-carboxylate. MS (ESI) m/z: 579.1 [M+H]⁺

### Step 2: [4-[[3-[4-(difluoromethoxy)phenyl]imidazo[1,2-a]pyrazin-8-yl]amino]-2-methyl-phenyl]-piperazin-1-yl-methanone

The title compound was obtained in analogy to step 2 in the preparation of Reference Example 12 using tert-butyl 4-[4-[[3-[4-(difluoromethoxy)phenyl]imidazo[1,2-a]pyrazin-8-yl]amino]-2-methyl-benzoyl]piperazine-1-carboxylate. MS (ESI) m/z: 479.2 [M+H]⁺

### Step 3: [4-(3-aminocyclobutanecarbonyl)piperazin-1-yl]-[4-[[3-[4-(difluoromethoxy)phenyl]imidazo[1,2-a]pyrazin-8-yl]amino]-2-methyl-phenyl]methanone hydrochloride

To a mixture of (1r,3r)-3-((tert-butoxycarbonyl)amino)cyclobutanecarboxylic acid (53.98 mg, 0.250 mmol, 1.5 eq) and [4-[[3-[4-(difluoromethoxy)phenyl]imidazo[1,2-a]pyrazin-8-yl]amino]-2-methyl-phenyl]-piperazin-1-yl-methanone (80.0 mg, 0.170 mmol, 1 eq) in DMF (1 mL) was added triethylamine (0.07 mL, 0.500 mmol, 3 eq) and 1-propanephosphonic anhydride (159.59 mg, 0.250 mmol, 1.5 eq) (50% in DMF solution) slowly at 25 °C. Then the mixture was stirred at 25 °C for 16 h, and conc. HCl (0.5 mL, 6 mmol, 35.89 eq) was added to the mixture and the mixture was stirred at 25 °C for another 48 h. After that the mixture was filtered and purified by prep-HPLC to give [4-(3-aminocyclobutanecarbonyl)piperazin-1-yl]-[4-[[3-[4-(difluoromethoxy)phenyl]imidazo[1,2-a]pyrazin-8-yl]amino]-2-methyl-phenyl]methanone hydrochloride (31.08 mg, 0.050 mmol, 28.62 % yield) as white solid. MS (ESI) m/z: 576.3 [M+H]⁺

### Reference Example 523

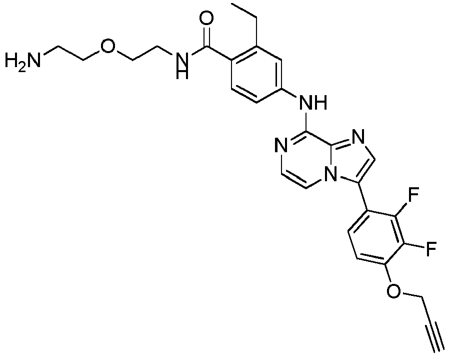

### Step 1: tert-butyl (2-(2-(4-((3-(2,3-difluoro-4-(prop-2-yn-1-yloxy)phenyl)imidazo[1,2-a]pyrazin-8-yl)amino)-2-ethylbenzamido)ethoxy)ethyl)carbamate

The title compound was obtained in analogy to step 3 in the preparation of Reference Example 515 using 4-((3-(2,3-difluoro-4-(prop-2-yn-1-yloxy)phenyl)imidazo[1,2-a]pyrazin-8-yl)amino)-2-ethylbenzoic acid and tert-butyl (2-(2-aminoethoxy)ethyl)carbamate as coupling partners. MS (ESI) m/z: 635.5 [M+H]⁺

### Step 2: N-(2-(2-aminoethoxy)ethyl)-4-((3-(2,3-difluoro-4-(prop-2-yn-1-yloxy)phenyl)imidazo[1,2-a]pyrazin-8-yl)amino)-2-ethylbenzamide

The title compound was obtained in analogy to step 2 in the preparation of Reference Example 12 using tert-butyl (2-(2-(4-((3-(2,3-difluoro-4-(prop-2-yn-1-yloxy)phenyl)imidazo[1,2-a]pyrazin-8-yl)amino)-2-ethylbenzamido)ethoxy)ethyl)carbamate as substrate. MS (ESI) m/z: 535.3 [M+H]⁺

### Reference Example 524

### 2-[2-[2-[[4-[[3-(2,3-difluoro-4-methoxy-phenyl)imidazo[1,2-a]pyrazin-8-yl]amino]-2-ethyl-benzoyl]amino]ethoxy]ethylamino]acetic acid; formic acid

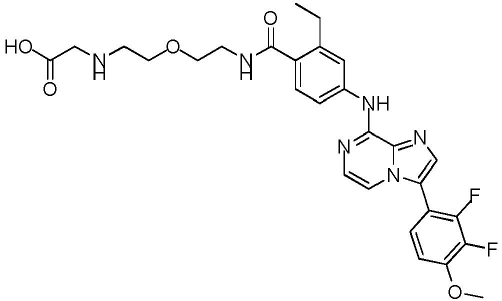

### Step 1: 2,5-dioxopyrrolidin-1-yl 4-((3-(2,3-difluoro-4-methoxyphenyl)imidazo[1,2-a]pyrazin-8-yl)amino)-2-ethylbenzoate

To a solution of 4-((3-(2,3-difluoro-4-methoxyphenyl)imidazo[1,2-a]pyrazin-8-yl)amino)-2-ethylbenzoic acid (2.0 g, 4.71 mmol, 1 eq) in THF (30 mL)/DMF (10 mL) was added N-hydroxysuccinimide (813.55 mg, 7.07 mmol, 1.5 eq) and 1-ethyl-(3-(3-dimethylamino)propyl)-carbodiimide hydrochloride (1084.07 mg, 5.66 mmol, 1.2 eq). The mixture was stirred at 25 °C for 3 h. The solvent was evaporated and water was added. The resulting suspension was filtered and the solid was dried in vacuo to give the title compound (1.8 g, 3.45 mmol, 73 % yield) as light yellow solid. MS (ESI) m/z: 522 [M+H]⁺

### Step 2: N-(2-(2-aminoethoxy)ethyl)-4-((3-(2,3-difluoro-4-methoxyphenyl)imidazo[1,2-a]pyrazin-8-yl)amino)-2-ethylbenzamide

To a solution of 2,5-dioxopyrrolidin-1-yl 4-((3-(2,3-difluoro-4-methoxyphenyl)imidazo[1,2-a]pyrazin-8-yl)amino)-2-ethylbenzoate (1100 mg, 2.11 mmol, 1 eq) in THF (20 mL) was added triethylamine (0.44 mL, 3.16 mmol, 1.5 eq) and 1,5-diamino-3-oxapentane (329 mg, 3.16 mmol, 1.5 eq). The mixture was stirred at 25 °C for 3 h. The solvent was evaporated. The solid was triturated with water and filtered to afford the title compound (912 mg, 1.79 mmol) as off-white solid. MS (ESI) m/z: 511 [M+H]⁺

### Step 3: methyl 2-((2-(2-(4-((3-(2,3-difluoro-4-methoxyphenyl)imidazo[1,2-a]pyrazin-8-yl)amino)-2-ethylbenzamido)ethoxy)ethyl)amino)acetate

To a solution of N-(2-(2-aminoethoxy)ethyl)-4-((3-(2,3-difluoro-4-methoxyphenyl)imidazo[1,2-a]pyrazin-8-yl)amino)-2-ethylbenzamide (500 mg, 0.980 mmol, 1 eq) in DMF (5 mL) was added triethylamine (0.2 mL, 1.47 mmol, 1.5 eq) and methyl chloroacetate (138 mg, 1.27 mmol, 1.3 eq). The mixture was stirred at 25 °C for 4 h. To the mixture was added water and the pH of mixture was adjusted to around 4 by 1 M aq. HCl. The mixture was extracted with ethyl acetate (50 mLx3). The pH of aqueous solution was adjusted to around 8. The resulting suspension was filtered and the residue was dried to give the title compound (500 mg, 0.980 mmol, 1 eq) as a yellow solid. MS (ESI) m/z: 583 [M+H]⁺

### Step 4: 2-((2-(2-(4-((3-(2,3-difluoro-4-methoxyphenyl)imidazo[1,2-a]pyrazin-8-yl)amino)-2-ethylbenzamido)ethoxy)ethyl)amino)acetic acid; formic acid

To a solution of methyl 2-((2-(2-(4-((3-(2,3-difluoro-4-methoxyphenyl)imidazo[1,2-a]pyrazin-8-yl)amino)-2-ethylbenzamido)ethoxy)ethyl)amino)acetate (50 mg, 0.09 mmol, 1 eq) in methanol (2 mL) was added aq. sodium hydroxide solution (0.5 mL, 2 mmol, 23.3 eq, 4 N). The mixture was stirred at 25 °C for 4 h. The pH was adjusted to around 6 by addition of 2 M aq. HCl. The solvent was evaporated and the residue was purified by prep. HPLC to give the title compound (18 mg, 0.030 mmol) as a white solid. MS (ESI) m/z: 569 [M+H]⁺

### Reference Example 525

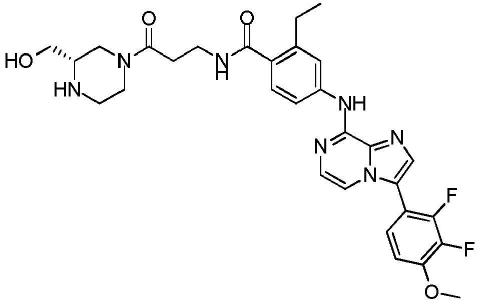

### Step 1: 3-[[4-[[3-(2,3-difluoro-4-methoxy-phenyl)imidazo[1,2-a]pyrazin-8-yl]amino]-2-ethyl-benzoyl]amino]propanoic acid

The title compound was obtained in analogy to step 3 in the preparation of Reference Example 515 using 4-[[3-(2,3-difluoro-4-methoxy-phenyl)imidazo[1,2-a]pyrazin-8-yl]amino]-2-ethyl-benzoic acid and beta-alanine. MS (ESI) m/z: 496.3. [M+H]⁺

### Step 2: tert-butyl (2S)-4-[3-[[4-[[3-(2,3-difluoro-4-methoxy-phenyl)imidazo[1,2-a]pyrazin-8-yl]amino]-2-ethyl-benzoyl]amino]propanoyl]-2-(hydroxymethyl)piperazine-1-carboxylate

The title compound was obtained in analogy to step 3 in the preparation of Reference Example 515 using 3-[[4-[[3-(2,3-difluoro-4-methoxy-phenyl)imidazo[1,2-a]pyrazin-8-yl]amino]-2-ethyl-benzoyl]amino]propanoic acid and tert-butyl (2S)-2-(hydroxymethyl)piperazine-1-carboxylate as reactants. MS (ESI) m/z: 694.2 [M+H]⁺

### Step 3: 4-[[3-(2,3-difluoro-4-methoxy-phenyl)imidazo[1,2-a]pyrazin-8-yl]amino]-2-ethyl-N-[3-[(3S)-3-(hydroxymethyl)piperazin-1-yl]-3-oxo-propyl]benzamide

The title compound was obtained in analogy to step 2 in the preparation of Reference Example 12 using tert-butyl (2S)-4-[3-[[4-[[3-(2,3-difluoro-4-methoxy-phenyl)imidazo[1,2-a]pyrazin-8-yl]amino]-2-ethyl-benzoyl]amino]propanoyl]-2-(hydroxymethyl)piperazine-1-carboxylate as starting material. MS (ESI) m/z: 594.3. [M+H]⁺

### Reference Example 526

### 4-[[3-(2,3-difluoro-4-methoxy-phenyl)imidazo[1,2-a]pyrazin-8-yl]amino]-2-ethyl-N-[4-[(3S)-3-(hydroxymethyl)piperazin-1-yl]-4-oxo-butyl]benzamide

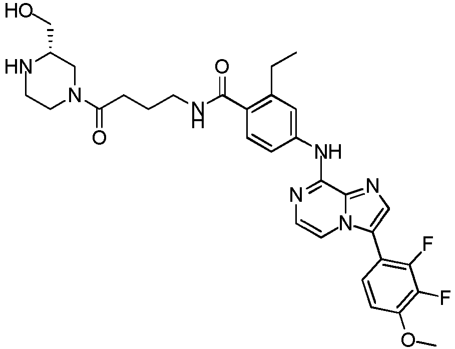

The title compound was obtained in analogy to Reference Example 525 by using 4-aminobutyric acid as starting material instead of beta-alanine. MS (ESI) m/z: 608.4. [M+H]⁺

### Reference Example 527

### 4-[[3-(2,3-difluoro-4-methoxy-phenyl)imidazo[1,2-a]pyrazin-8-yl]amino]-2-ethyl-N-[6-[(3S)-3-(hydroxymethyl)piperazin-1-yl]-6-oxo-hexyl]benzamide

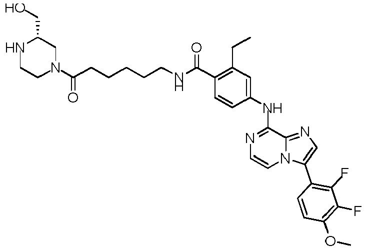

The title compound was obtained in analogy to Reference Example 525 by using 6-aminohexanoic acid as starting material instead of beta-alanine. MS (ESI) m/z: 636.4 [M+H]⁺

### Reference Example 528

### 4-[[3-(2,3-difluoro-4-methoxy-phenyl)imidazo[1,2-a]pyrazin-8-yl]amino]-2-ethyl-N-[5-[(3S)-3-(hydroxymethyl)piperazin-1-yl]-5-oxo-pentyl]benzamide

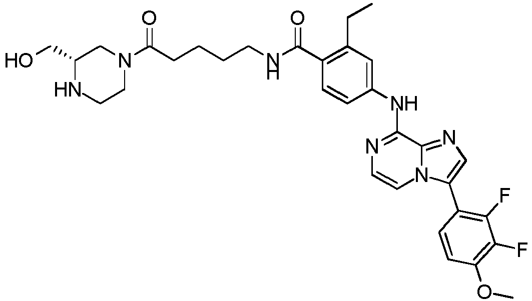

The title compound was obtained in analogy to Reference Example 525 by using 5-aminovaleric acid as starting material instead of beta-alanine. MS (ESI) m/z: 622.4. [M+H]⁺

### Reference Example 529

### N-[2-(2-amino-1,1-dimethyl-ethoxy)ethyl]-4-[[3-(2,3-difluoro-4-methoxy-phenyl)imidazo[1,2-a]pyrazin-8-yl]amino]-2-ethyl-benzamide; formic acid

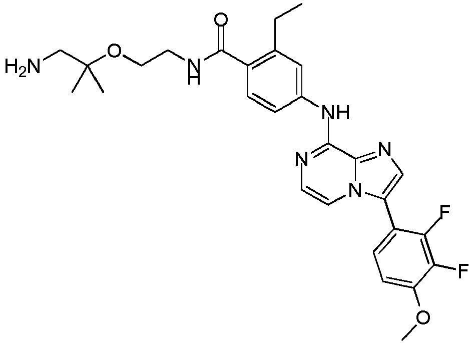

### Step 1: tert-butyl N-(2-allyloxy-2-methyl-propyl)carbamate

The title compound was obtained in analogy to step 2 in the preparation of Reference Example 530 using tert-butyl 2-hydroxy-2-methylpropylcarbamate and allyl tert-butyl carbonate for coupling reaction.

¹H NMR (400 MHz, CHLOROFORM-d) δ = 6.01 - 5.83 (m, 1 H), 5.37 - 5.10 (m, 2 H), 4.86 (br s, 1 H), 3.90 (d, *J* = 5.4 Hz, 2H), 3.18 (d, *J* = 5.9 Hz, 2 H), 1.46 (s, 9 H), 1.20 (s, 6 H) ppm.

### Step 2: tert-butyl N-[2-(2-hydroxyethoxy)-2-methyl-propyl]carbamate

The title compound was obtained in analogy to step 3 in the preparation of Reference Example 530 using tert-butyl N-(2-allyloxy-2-methyl-propyl)carbamate for ozonolysis.

¹H NMR (400 MHz, CHLOROFORM-d) δ = 4.85 (br s, 1 H), 3.64 (t, *J=* 4.3 Hz, 2 H), 3.43 - 3.36 (m, 2 H), 3.10 (d, *J* = 6.0 Hz, 2 H), 2.10 (br s, 1 H), 1.38 (s, 9 H), 1.11 (s, 6 H) ppm.

### Step 3: 2-[2-(tert-butoxycarbonylamino)-1,1-dimethyl-ethoxy]ethyl methanesulfonate

The title compound was obtained in analogy to step 4 in the preparation of Reference Example 518 using tert-butyl N-[2-(2-hydroxyethoxy)-2-methyl-propyl]carbamate as starting material. The product was directly used in crude form.

### Step 4: tert-butyl N-[2-(2-azidoethoxy)-2-methyl-propyl]carbamate

The title compound was obtained in analogy to step 5 in the preparation of Reference Example 530 using 2-[2-(tert-butoxycarbonylamino)-1,1-dimethyl-ethoxy]ethyl methanesulfonate and sodium azide.

¹H NMR (400 MHz, CHLOROFORM-d) δ = 4.94 (br s, 1 H), 3.60 - 3.53 (m, 1 H), 3.60 - 3.53 (m, 1 H), 3.35 (t, *J* = 4.9 Hz, 2 H), 3.19 (d, *J=* 6.0 Hz, 2 H), 1.47 (s, 9 H), 1.21 (s, 6 H) ppm.

### Step 5: tert-butyl N-[2-(2-aminoethoxy)-2-methyl-propyl]carbamate

The title compound was obtained in analogy to step 6 in the preparation of Reference Example 530 using tert-butyl N-[2-(2-azidoethoxy)-2-methyl-propyl]carbamate for reduction, used directly in crude form.

### Step 6: tert-butyl N-[2-methyl-2-[2-[(4-nitro-2-vinyl-benzoyl)amino]ethoxy]propyl]carbamate

The title compound was obtained in analogy to step 3 in the preparation of Reference Example 515 using 4-nitro-2-vinyl-benzoic acid and tert-butyl N-[2-(2-aminoethoxy)-2-methylpropyl]carbamate. MS (ESI) m/z: 420.2 [M+Na]⁺

### Step 7: tert-butyl N-[2-[2-[(4-amino-2-ethyl-benzoyl)amino]ethoxy]-2-methylpropyl]carbamate

The title compound was obtained in analogy to step 4 of the preparation of Reference Example 531 using tert-butyl N-[2-methyl-2-[2-[(4-nitro-2-vinyl-benzoyl)amino]ethoxy]propyl]carbamate as substrate for this hydrogenation. MS (ESI) m/z: 380.1 [M+H]⁺

### Step 8: tert-butyl N-[2-[2-[[4-[[3-(2,3-difluoro-4-methoxy-phenyl)imidazo[1,2-a]pyrazin-8-yl]amino]-2-ethyl-benzoyl]amino]ethoxy]-2-methyl-propyl]carbamate

The title compound was obtained in analogy to step 2 in the preparation of Reference Example 515 using tert-butyl N-[2-[2-[(4-amino-2-ethyl-benzoyl)amino]ethoxy]-2-methylpropyl]carbamate and 8-chloro-3-(2,3-difluoro-4-methoxy-phenyl)imidazo[1,2-a]pyrazine. MS (ESI) m/z: 639.2 [M+H]⁺

### Step 9: N-[2-(2-amino-1,1-dimethyl-ethoxy)ethyl]-4-[[3-(2,3-difluoro-4-methoxy-phenyl)imidazo[1,2-a]pyrazin-8-yl]amino]-2-ethyl-benzamide

The title compound was obtained in analogy to step 2 in the preparation of Reference Example 12 using tert-butyl N-[2-[2-[[4-[[3-(2,3-difluoro-4-methoxy-phenyl)imidazo[1,2-a]pyrazin-8-yl]amino]-2-ethyl-benzoyl]amino]ethoxy]-2-methyl-propyl]carbamate as substrate. MS (ESI) m/z: 539.3 [M+H]⁺

### Reference Example 518

### 4-[[3-[4-[4-(2-aminoethoxy)but-2-ynoxy]-2,3-difluoro-phenyl]imidazo[1,2-a]pyrazin-8-yl]amino]-N-(3-aminopropyl)-2-ethyl-benzamide

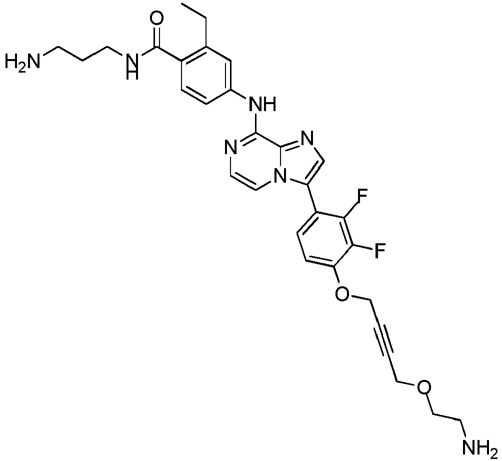

### Step 1: tert-butyl (3-(2-ethyl-4-nitrobenzamido)propyl)carbamate

The title compound was obtained in analogy to step 3 in the preparation of Reference Example 515 using tert-butyl (3-aminopropyl)carbamate and 2-ethyl-4-nitrobenzoic acid. MS (ESI) m/z: 352.2 [M+H]⁺

### Step 2: tert-butyl (3-(4-amino-2-ethylbenzamido)propyl)carbamate

The title compound was obtained in analogy to step 4 in the preparation of Reference Example 531 using tert-butyl (3-(2-ethyl-4-nitrobenzamido)propyl)carbamate as starting material. MS (ESI) m/z: 332.2 [M+H]⁺

### Step 3: tert-butyl N-[3-[[4-[[3-(2,3-difluoro-4-hydroxy-phenyl)imidazo[1,2-a]pyrazin-8-yl]amino]-2-ethyl-benzoyl]amino]propyl]carbamate

The title compound was obtained in analogy to step 2 in the preparation of Reference Example 515 using 4-(8-chloroimidazo[1,2-a]pyrazin-3-yl)-2,3-difluoro-phenol and tert-butyl N-[3-[(4-amino-2-ethyl-benzoyl)amino]propyl]carbamate as reactants. MS (ESI) m/z: 567.2 [M+H]⁺

### Step 4: 2-(tert-butoxycarbonylamino)ethylmethanesulfonate

To a solution of N-BOC-ethanolamine (2.0 g, 12.41 mmol, 1 eq) and triethylamine (3.46 mL, 24.81 mmol, 2 eq) in DCM (5 mL) was added methanesulfonyl chloride (1.44 mL, 18.61 mmol, 1.5 eq) at 20 °C, the mixture was stirred at 20 °C for 2 h. The mixture was quenched with 1 N aq. HCl, and then the mixture was extracted with ethyl acetate (30 mLx2), and dried over Na₂SO₄, filtered and concentrated to get the title compound (2.5 g, 10.45 mmol, 84.21 % yield) as a yellow oil, which was used without further purification.

### Step 5: tert-butyl N-[2-(4-hydroxybut-2-ynoxy)ethyl]carbamate

A mixture of 2-butyne-1,4-diol (1.44 g, 16.72 mmol, 2 eq) and sodium hydroxide in water (8.36 mL, 16.72 mmol, 2 eq) was stirred at 90 °C for 0.5 h, then 2-(tert-butoxycarbonylamino)ethyl methanesulfonate (2.0 g, 8.36 mmol, 1 eq) was added to the mixture at 90 °C and stirred for 5 h. The solution was poured into water and extracted with ethyl acetate (30 mLx2), the combined organic layers were concentrated and the obtained residue was purified by silica gel chromatography (PE/EA = 4:1) to afford tert-butyl N-[2-(4-hydroxybut-2-ynoxy)ethyl]carbamate (500 mg, 2.18 mmol) as colorless oil.

### Step 6: 4-[2-(tert-butoxycarbonylamino)ethoxy]but-2-ynyl methanesulfonate

The title compound was obtained in analogy to step 4 in the preparation of Reference Example 518 using tert-butyl N-[2-(4-hydroxybut-2-ynoxy)ethyl]carbamate as starting material. The product was directly used in crude form.

### Step 7: tert-butyl N-[3-[[4-[[3-[4-[4-[2-(tert-butoxycarbonylamino)ethoxy]but-2-ynoxy]-2,3-difluoro-phenyl]imidazo[1,2-a]pyrazin-8-yl]amino]-2-ethyl-benzoyl]amino]propyl]carbamate

To a solution of tert-butyl N-[3-[[4-[[3-(2,3-difluoro-4-hydroxy-phenyl)imidazo[1,2-a]pyrazin-8-yl]amino]-2-ethyl-benzoyl]amino]propyl]carbamate (50.0 mg, 0.090 mmol, 1 eq) and potassium carbonate (24.39 mg, 0.180 mmol, 2 eq) in acetonitrile (1 mL) was added 4-[2-(tert-butoxycarbonylamino)ethoxy]but-2-ynyl methanesulfonate (40.68 mg, 0.130 mmol, 1.5 eq) at 20 °C, the mixture was stirred at 60 °C for 16 h. The mixture was concentrated and purified by prep-HPLC to get the title compound (30 mg, 0.040 mmol, 43.7% yield) as a white solid. MS (ESI) m/z: 778.2 [M+H]⁺

### Step 8: 4-[[3-[4-[4-(2-aminoethoxy)but-2-ynoxy]-2,3-difluoro-phenyl]imidazo[1,2-a]pyrazin-8-yl]amino]-N-(3-aminopropyl)-2-ethyl-benzamide

The title compound was obtained in analogy to step 2 in the preparation of Reference Example 12 using tert-butyl N-[3-[[4-[[3-[4-[4-[2-(tert-butoxycarbonylamino)ethoxy]but-2-ynoxy]-2,3-difluoro-phenyl]imidazo[1,2-a]pyrazin-8-yl]amino]-2-ethyl-benzoyl]amino]propyl]carbamate as reactant. MS (ESI) m/z: 578.4 [M+H]⁺

### Reference Example 530

### N-[2-(2-amino-2-methyl-propoxy)ethyl]-4-[[3-(2,3-difluoro-4-methoxy-phenyl)imidazo[1,2-a]pyrazin-8-yl]amino]-2-ethyl-benzamide; formic acid

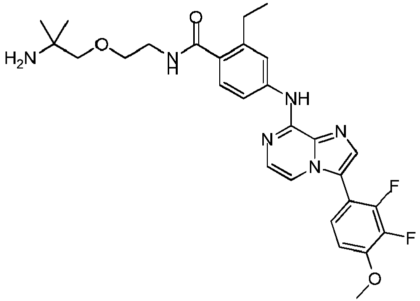

### Step 1: allyl tert-butyl carbonate

To a solution of allyl alcohol (19.96 g, 343.64 mmol, 3 eq) and di-tert-butyldicarbonate (25.0 g, 114.55 mmol, 1 eq) was slowly added 4-dimethylaminopyridine (2.8 g, 22.91 mmol, 0.200 eq). The mixture was stirred at 15 °C for 1 h. The mixture was diluted with MTBE, washed with brine, dried over Na₂SO₄ and concentrated. The residue was purified by silica gel chromatography eluting with PE:EA = 50:1 to afford allyl tert-butyl carbonate (12 g, 75.86 mmol) as colorless oil.

¹H NMR (400 MHz, CHLOROFORM-d) δ = 5.99 - 5.87 (m, 1 H), 5.33 (dd, *J=* 1.4, 17.2 Hz, 1 H), 5.24 (dd, *J* = 1.2, 10.4 Hz, 1 H), 4.55 (td, *J* = 1.2, 5.8 Hz, 2 H), 1.48 (s, 9 H) ppm.

### Step 2: tert-butyl N-(2-allyloxy-1,1-dimethyl-ethyl)carbamate

To a mixture of tert-butyl (1-hydroxy-2-methylpropan-2-yl)carbamate (500.0 mg, 2.64 mmol, 1 eq), allyl tert-butyl carbonate (835 mg, 5.28 mmol, 2 eq) in THF (10 mL) was added tetrakis(triphenylphosphine)palladium(0) (610.6 mg, 0.530 mmol, 0.200 eq). The resulting mixture was stirred at 80 °C for 12 h under nitrogen. The mixture was concentrated and the residue was purified by silica gel chromatography eluting with PE:EA = 50:1 to afford tert-butyl N-(2-allyloxy-1,1-dimethyl-ethyl)carbamate (250 mg, 1.09 mmol, 41.26 % yield) as colorless oil.

¹H NMR (400 MHz, CHLOROFORM-d) δ = 5.90 - 5.75 (m, 1 H), 5.26 - 5.05 (m, 2 H), 4.69 (br s, 1 H), 3.93 (d, *J* = 5.5 Hz, 2 H), 3.30 (s, 2 H), 1.36 (s, 9 H), 1.23 (s, 6 H) ppm.

### Step 3: tert-butyl N-[2-(2-hydroxyethoxy)-1,1-dimethyl-ethyl]carbamate

Through a solution of tert-butyl N-(2-allyloxy-1,1-dimethyl-ethyl)carbamate (250.0 mg, 1.09 mmol, 1 eq) in DCM (20 mL) cooled to -78 °C was bubbled ozone until the mixture turned blue. The mixture was warmed to 0 °C and then sodium borohydride (82.49 mg, 2.18 mmol, 2 eq) was added. The mixture was stirred for 3 h ,quenched with saturated NH₄Cl solution and then the organic phase was separated. The mixture was dried over sodium sulfate and concentrated. The residue was purified by silica gel chromatography eluting with PE:EA from 10:1 to 3:1 to afford tert-butyl N-[2-(2-hydroxyethoxy)-1,1-dimethyl-ethyl]carbamate (80 mg, 0.340 mmol, 31.45 % yield) as colorless oil.

¹H NMR (400 MHz, CHLOROFORM-d) δ = 4.61 (br s, 1 H), 3.69 - 3.65 (m, 2 H), 3.55 - 3.49 (m, 2 H), 3.41 (s, 2 H), 1.37 (s, 9 H), 1.22 (s, 6 H) ppm.

### Step 4: 2-[2-(tert-butoxycarbonylamino)-2-methyl-propoxy]ethyl methanesulfonate

The title compound was obtained in analogy to step 4 in the preparation of Reference Example 518 using tert-butyl N-[2-(2-hydroxyethoxy)-1,1-dimethyl-ethyl]carbamate as starting material. The product was directly used in crude form.

### Step 5: tert-butyl N-[2-(2-azidoethoxy)-1,1-dimethyl-ethyl]carbamate

To a solution of 2-[2-(tert-butoxycarbonylamino)-2-methyl-propoxy] ethyl methanesulfonate (550.0 mg, 1.77 mmol, 1 eq) in DMF (5 mL) was added sodium azide (0.31 mL, 8.83 mmol, 5 eq). The resulting mixture was stirred at 50 °C for 2 h. The mixture was diluted with water, extracted with ethyl acetate, washed with brine, dried over sodium sulfate and concentrated. The residue was purified by silica gel chromatography eluting with PE:EA = 10:1 to afford tert-butyl N-[2-(2-azidoethoxy)-1,1-dimethyl-ethyl]carbamate (380 mg, 1.47 mmol, 83.29 % yield) as colorless oil.

¹H NMR (400 MHz, chloroform-d) δ = 4.63 (br s, 1 H), 3.62 - 3.57 (m, 2 H), 3.40 (s, 2 H), 3.29 (t, *J* = 4.9 Hz, 2 H), 1.36 (s, 9 H), 1.23 (s, 6 H) ppm.

### Step 6: tert-butyl N-[2-(2-aminoethoxy)-1,1-dimethyl-ethyl]carbamate

To a solution of tert-butyl N-[2-(2-azidoethoxy)-1,1-dimethyl-ethyl]carbamate (380.0 mg, 1.47 mmol, 1 eq) in ethyl acetate (5 mL) was added palladium on carbon (38.0 mg, 0.040 mmol, 0.020 eq). The resulting mixture was hydrogenated at 760 mmHg at 15 °C for 2 hand the catalyst was removed by filtration. The filtrate was concentrated to afford tert-butyl N-[2-(2-aminoethoxy)-1,1-dimethyl-ethyl]carbarnate (360 mg, 1.55 mmol, crude) as colorless oil, which was used without further purification.

### Step 7: tert-butyl N-[1,1-dimethyl-2-[2-[(4-nitro-2-vinyl-benzoyl)amino]ethoxy]ethyl]carbamate

The title compound was obtained in analogy to step 3 in the preparation of Reference Example 515 using 4-nitro-2-vinyl-benzoic acid and tert-butyl N-[2-(2-aminoethoxy)-1,1-dimethylethyl]carbamate for condensation. MS (ESI) m/z: 430.3. [M+Na]⁺

### Step 8: tert-butyl N-[2-[2-[(4-amino-2-ethyl-benzoyl)amino]ethoxy]-1,1-dimethyl-ethyl]carbamate

The title compound was obtained in analogy to step 4 in the preparation of Reference Example 486 using tert-butyl N-[1,1-dimethyl-2-[2-[(4-nitro-2-vinyl-benzoyl)amino]ethoxy]ethyl]carbamate as substrate. MS (ESI) m/z: 402.3. [M+Na]⁺

### Step 9: tert-butyl N-[2-[2-[[4-[[3-(2,3-difluoro-4-methoxy-phenyl)imidazo[1,2-a]pyrazin-8-yl]amino]-2-ethyl-benzoyl]amino]ethoxy]-1,1-dimethyl-ethyl]carbamate

The title compound was obtained in analogy to step 2 in the preparation of Reference Example 515 using tert-butyl N-[2-[2-[(4-amino-2-ethyl-benzoyl)amino]ethoxy]-1,1-dimethylethyl]carbamate and 8-chloro-3-(2,3-difluoro-4-methoxy-phenyl)imidazo[1,2-a]pyrazine. MS (ESI) m/z: 639.4. [M+H]⁺

### Step 10: N-[2-(2-amino-2-methyl-propoxy)ethyl]-4-[[3-(2,3-difluoro-4-methoxy-phenyl)imidazo[1,2-a]pyrazin-8-yl]amino]-2-ethyl-benzamide; formic acid

The title compound was obtained in analogy to step 2 in the preparation of Reference Example 12 using tert-butyl N-[2-[2-[[4-[[3-(2,3-difluoro-4-methoxy-phenyl)imidazo[1,2-a]pyrazin-8-yl]amino]-2-ethyl-benzoyl]amino]ethoxy]-1,1-dimethyl-ethyl]carbamate as substrate. MS (ESI) m/z: 539.2 [M+H]⁺

### Reference Example 532

### 4-[[3-(2,3-difluoro-4-prop-2-ynoxy-phenyl)imidazo[1,2-a]pyrazin-8-yl]amino]-2-ethyl-N-[3-(2-hydroxyethylamino)propyl]benzamide

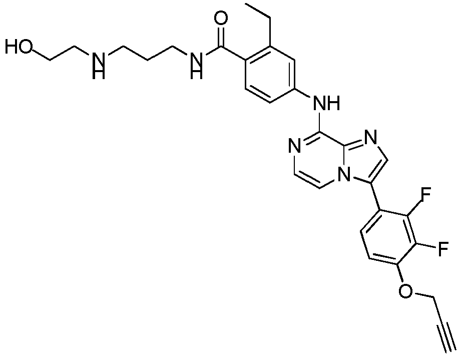

### Step 1: 4-(8-chloroimidazo[1,2-a]pyrazin-3-yl)-2,3-difluoro-phenol

The title compound was obtained in analogy to step 5 in the preparation of Intermediate 27 using 8-chloro-3-iodo-imidazo[1,2-a]pyrazine and 2,3-difluoro-4-(4,4,5,5-tetramethyl-1,3,2-dioxaborolan-2-yl)phenol. MS (ESI) m/z: 282.0 [M+H]⁺

### Step 2: 8-chloro-3-(2,3-difluoro-4-prop-2-ynoxy-phenyl)imidazo[1,2-a]pyrazine

The title compound was obtained in analogy to step 4 in the preparation of Reference Example 514 using 4-(8-chloroimidazo[1,2-a]pyrazin-3-yl)-2,3-difluoro-phenol and propargyl bromide as reactants. MS (ESI) m/z: 320.1. [M+H]⁺

### Step 3: tert-butyl N-[3-(benzyloxycarbonylamino)propyl]-N-(2-hydroxyethyl)carbamate

To a solution of benzyl N-[3-(2-hydroxyethylamino)propyl]carbamate (120.0 mg, 0.480 mmol, 1 eq) and triethylamine (0.07 mL, 0.480 mmol, 1 eq) in DCM (5 mL) was added di-t-butyldicarbonate (103.8 mg, 0.480 mmol, 1 eq), the mixture was stirred at 20 °C for 16 h. The mixture was concentrated and the obtained residue purified by reverse phase flash column chromatography to get the product tert-butyl N-[3-(benzyloxycarbonylamino)propyl]-N-(2-hydroxyethyl)carbamate (80 mg, 0.230 mmol, 47.73 % yield) as a colorless oil. MS (ESI) m/z: 353.2 [M+H]⁺

### Step 4: tert-butyl N-(3-aminopropyl)-N-(2-hydroxyethyl)carbamate

To a solution of tert-butyl N-[3-(benzyloxycarbonylamino)propyl]-N-(2-hydroxyethyl)carbamate (80.0 mg, 0.230 mmol, 1 eq) in methanol (2 mL) was added Pd/C (0.230 mmol, 1 eq) at 20 °C, the mixture was stirred at 20 °C for 24 h, filtered and concentrated to get the product tert-butyl N-(3-aminopropyl)-N-(2-hydroxyethyl)carbamate (30 mg, 0.140 mmol, 40.36 % yield) as a colorless oil, which was used without further purification in the next step.

### Step 5: 4-((3-(2,3-difluoro-4-(prop-2-yn-1-yloxy)phenyl)imidazo[1,2-a]pyrazin-8-yl)amino)-2-ethylbenzoic acid

The title compound was obtained in analogy to step 2 in the preparation of Reference Example 515 using 8-chloro-3-(2,3-difluoro-4-prop-2-ynoxy-phenyl)imidazo[1,2-a]pyrazine and 4-amino-2-ethylbenzoic acid as reactants. MS (ESI) m/z: 449.1 [M+H]⁺

### Step 6: tert-butyl N-[3-[[4-[[3-(2,3-difluoro-4-prop-2-ynoxy-phenyl)imidazo[1,2-a]pyrazin-8-yl]amino]-2-ethyl-benzoyl]amino]propyl]-N-(2-hydroxyethyl)carbamate

The title compound was obtained in analogy to step 3 in the preparation of Reference Example 515 using 4-((3-(2,3-difluoro-4-(prop-2-yn-1-yloxy)phenyl)imidazo[1,2-a]pyrazin-8-yl)amino)-2-ethylbenzoic acid and tert-butyl N-(3-aminopropyl)-N-(2-hydroxyethyl)carbamate as coupling partners. MS (ESI) m/z: 649.3 [M+H]⁺

### Step 7: 4- [[3-(2,3-difluoro-4-prop-2-ynoxy-phenyl)imidazo[1,2-a]pyrazin-8-yl]amino]-2-ethyl-N-[3-(2-hydroxyethylamino)propyl] benzamide

The title compound was obtained in analogy to step 2 in the preparation of Reference Example 12 using tert-butyl N-[3-[[4-[[3-(2,3-difluoro-4-prop-2-ynoxy-phenyl)imidazo[1,2-a]pyrazin-8-yl]amino]-2-ethyl-benzoyl]amino]propyl]-N-(2-hydroxyethyl)carbamate as starting material. MS (ESI) m/z: 549.4 [M+H]⁺

### Reference Example 515

### 4-((3-(2,3-difluoro-4-methoxyphenyl)imidazo[1,2-a]pyrazin-8-yl)amino)-2-ethyl-N-(2-(methyl(2-(methylamino)ethyl)amino)-2-oxoethyl)benzamide; formic acid

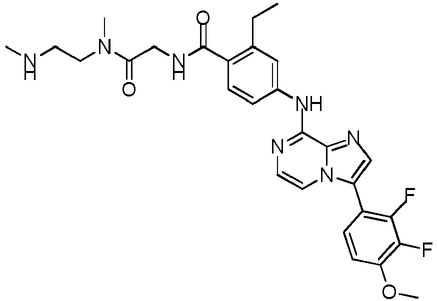

### Step 1: 8-chloro-3-(2,3-difluoro-4-methoxyphenyl)imidazo[1,2-a]pyrazine

A mixture of 8-chloro-3-iodo-imidazo[1,2-a]pyrazine (10.0 g, 35.78 mmol, 1 eq), 2,3-difluoro-4-methoxyphenylboronic acid (8.07 g, 42.94 mmol, 1.2 eq), [1,1'-bis(diphenylphosphino)ferrocene]dichloropalladium(II) (2.62 g, 3.58 mmol, 0.100 eq) and sodium carbonate (7.58 g, 71.56 mmol, 2 eq) in 1,4-dioxane (72 mL) and water (8 mL) was stirred for 15 h at 80 °C under N₂. The mixture was filtered and the filtrate was concentrated in vacuo to give a crude product, which was purified by silica gel chromatography eluting with petroleum ether/ethyl acetate = 2:1 to give product 8-chloro-3-(2,3-difluoro-4-methoxy-phenyl)imidazo[1,2-a]pyrazine (6 g, 20.29 mmol, 50.81 % yield) as a light yellow solid. MS (ESI) m/z: 296.0 [M+H]⁺

### Step 2: 4-((3-(2,3-difluoro-4-methoxyphenyl)imidazo[1,2-a]pyrazin-8-yl)amino)-2-ethylbenzoic acid

A mixture of 4-amino-2-ethyl-benzoic acid (216.91 mg, 1.12 mmol, 1.1 eq) and 8-chloro-3-(2,3-difluoro-4-methoxy-phenyl)imidazo[1,2-a]pyrazine (300.0 mg, 1.01 mmol, 1 eq) in acetonitrile (6.3 mL) and acetic acid (0.700 mL) was stirred for 12 h at 65 °C. The solvent was removed in vacuo to give 4-[[3-(2,3-difluoro-4-methoxy-phenyl)imidazo[l,2-a]pyrazin-8-yl]amino]-2-ethyl-benzoic acid (400 mg, 0.940 mmol, 72.64 % yield) as an off-white solid, which was used without further purification in the next step. MS (ESI) m/z: 425.0 [M+H]⁺

### Step 3: tert-butyl 2-(4-((3-(2,3-difluoro-4-methoxyphenyl)imidazo[1,2-a]pyrazin-8-yl)amino)-2-ethylbenzamido)acetate

To a solution of 4-[[3-(2,3-difluoro-4-methoxy-phenyl)imidazo[1,2-a]pyrazin-8-yl]amino]-2-ethyl-benzoic acid (400.0 mg, 0.940 mmol, 1 eq) in DMF (8 mL) was added O-(7-azabenzotriazol-1-yl)-N,N,N',N'-tetramethyluronium hexafluorophosphate (430 mg, 1.13 mmol, 1.2 eq) and N,N-diisopropylethylamine (0.33 mL, 1.89 mmol, 2 eq), then the mixture was stirred for 0.2 h at 10 °C, tert-butyl glycinate (135.99 mg, 1.04 mmol, 1.1 eq) was added and the mixture was stirred for 15 h at 10 °C. The mixture was diluted with water, filtered and dried to give tert-butyl 2-[[4-[[3-(2,3-difluoro-4-methoxy-phenyl)imidazo[1,2-a]pyrazin-8-yl]amino]-2-ethyl-benzoyl]amino]acetate (536 mg, 1 mmol, 88 % yield) as a light yellow solid. MS (ESI) m/z: 538.1 [M+H]⁺

### Step 4: 2-(4-((3-(2,3-difluoro-4-methoxyphenyl)imidazo[1,2-a]pyrazin-8-yl)amino)-2-ethylbenzamido)acetic acid

To a solution of tert-butyl 2-[[4-[[3-(2,3-difluoro-4-methoxy-phenyl)imidazo[1,2-a]pyrazin-8-yl]amino]-2-ethyl-benzoyl]amino]acetate (536.0 mg, 0.830 mmol, 1 eq) in 1,4-dioxane (6 mL) was added 4 M HCl in dioxane (6.22 mL, 24.89 mmol, 30 eq), and the mixture was stirred for 15 h at 30 °C. The solvent was evaporated to give crude 2-[[4-[[3-(2,3-difluoro-4-methoxyphenyl)imidazo[1,2-a]pyrazin-8-yl]amino]-2-ethyl-benzoyl]amino]acetic acid (455 mg, 0.950 mmol, 91.14 % yield) as an off-white solid, which was used in the next step without further purification. MS (ESI) m/z: 482.2 [M+H]⁺

### Step 5: tert-butyl (2-(2-(4-((3-(2,3-difluoro-4-methoxyphenyl)imidazo[1,2-a]pyrazin-8-yl)amino)-2-ethylbenzamido)-N-methylacetamido)ethyl)(methyl)carbamate

The title compound was obtained in analogy to step 1 in the preparation of Reference Example 12 using 2-(4-((3-(2,3-difluoro-4-methoxyphenyl)imidazo[1,2-a]pyrazin-8-yl)amino)-2-ethylbenzamido)acetic acid and tert-butyl N-methyl-N-[2-(methylamino)ethyl]carbamate as reactants. MS (ESI) m/z: 652.3 [M+H]⁺

### Step 6: 4-((3-(2,3-difluoro-4-methoxyphenyl)imidazo[1,2-a]pyrazin-8-yl)amino)-2-ethyl-N-(2-(methyl(2-(methylamino)ethyl)amino)-2-oxoethyl)benzamide

The title compound was obtained in analogy to step 2 in the preparation of Reference Example 12 using tert-butyl (2-(2-(4-((3-(2,3-difluoro-4-methoxyphenyl)imidazo[1,2-a]pyrazin-8-yl)amino)-2-ethylbenzamido)-N-methylacetamido)ethyl)(methyl)carbamate as reactant. MS (ESI) m/z: 552.1 [M+H]⁺

### Reference Example 533

### N-(3-aminopropyl)-4-[[3-(2,3-difluoro-4-methoxy-phenyl)imidazo[1,2-a]pyrazin-8-yl]amino]-2-(difluoromethyl)benzamide; formic acid

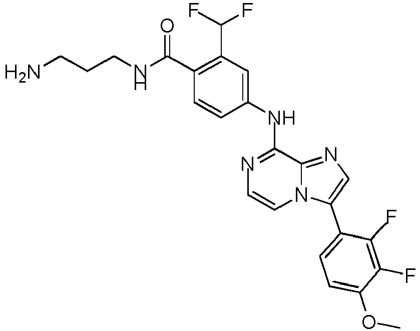

### Step 1: methyl 2-(difluoromethyl)-4-nitrobenzoate

A solution of methyl 2-formyl-4-nitro-benzoate (500.0 mg, 2.39 mmol, 1 eq) in DCM (20 mL) was cooled to -15 °C and diethylaminosulfur trifluoride (1926.64 mg, 11.95 mmol, 5 eq) was added. The resulting mixture was stirred at 10 °C for 15 h. The mixture was quenched with sat. NaHCO₃. The organic separated layer was dried over sodium sulfate and concentrated. The residue was purified by silica gel chromatography eluting with PE:EA = 100:1 to afford methyl 2-(difluoromethyl)-4-nitro-benzoate (380 mg, 1.64 mmol, 68.77 % yield) as yellow oil.

¹H NMR (400 MHz, CHLOROFORM-d) δ = 8.68 (d, *J* = 2.0 Hz, 1 H), 8.41 (dd, *J* = 2.3, 8.5 Hz, 1 H), 8.24 (d, *J* = 8.5 Hz, 1 H), 7.70 - 7.41 (m, 1 H), 4.03 (s, 3 H) ppm.

### Step 2: 2-(difluoromethyl)-4-nitrobenzoic acid

To a solution of methyl 2-(difluoromethyl)-4-nitro-benzoate (380.0 mg, 1.64 mmol, 1 eq) in THF (10 mL) and water (1 mL) was added LiOH.H₂O (137.7 mg, 3.28 mmol, 2 eq). The resulting mixture was stirred at 10 °C for 2 h. The mixture was acidified with IN HCl to pH = 3 and extracted with ethyl acetate (50 mL×2), washed with brine, dried over Na₂SO₄ and concentrated to afford 2-(difluoromethyl)-4-nitro-benzoic acid (350 mg, 1.61 mmol, 98.05% yield) as yellow solid, which was used directly in next step.

### Step 3: tert-butyl N-[3-[[2-(difluoromethyl)-4-nitro-benzoyl]amino]propyl]carbamate

The title compound was obtained in analogy to step 3 in the preparation of Reference Example 515 using 2-(difluoromethyl)-4-nitro-benzoic acid and N-BOC-1,3-diaminopropane. MS (ESI) m/z: 396.3. [M+Na]⁺

### Step 4: tert-butyl N-[3-[[4-amino-2-(difluoromethyl)benzoyl]amino]propyl]carbamate

The title compound was obtained in analogy to step 4 in the preparation of Reference Example 486 using tert-butyl N-[3-[[2-(difluoromethyl)-4-nitro-benzoyl]amino]propyl] carbamate as substrate for hydrogenation. MS (ESI) m/z: 366.1 [M+Na]⁺

### Step 5: tert-butyl N-[3-[[4-[[3-(2,3-difluoro-4-methoxy-phenyl)imidazo[1,2-a]pyrazin-8-yl]amino]-2-(difluoromethyl)benzoyl]amino]propyl]carbamate

A mixture of 8-chloro-3-(2,3-difluoro-4-methoxy-phenyl)imidazo[1,2-a]pyrazine (70.0 mg, 0.240 mmol, 1 eq), tert-butyl N-[3-[[4-amino-2-(difluoromethyl)benzoyl]amino]propyl]carbamate (97.55 mg, 0.280 mmol, 1.2 eq), Brettphos Pd G3 (21.46 mg, 0.020 mmol, 0.100 eq), potassium carbonate (98.16 mg, 0.710 mmol, 3 eq) in tert-butanol (5 mL) were stirred for 15 h at 110 °C under nitrogen protection. The mixture was filtered and the solvent was removed in vacuum to give crude product, which was purified by prep-TLC (DCM/MeOH = 10/1) to give product tert-butyl N-[3-[[4-[[3-(2,3-difluoro-4-methoxyphenyl)imidazo[1,2-a]pyrazin-8-yl]amino]-2-(difluoromethyl)benzoyl]amino]propyl]carbamate (110 mg, 0.180 mmol, 77 % yield). MS (ESI) m/z: 603.2 [M+H]⁺

### Step 6: N-(3-aminopropyl)-4-[[3-(2,3-difluoro-4-methoxy-phenyl)imidazo[1,2-a]pyrazin-8-yl]amino]-2-(difluoromethyl)benzamide

The title compound was obtained in analogy to step 2 in the preparation of Reference Example 12 using tert-butyl N-[3-[[4-[[3-(2,3-difluoro-4-methoxy-phenyl)imidazo[1,2-a]pyrazin-8-yl]amino]-2-(difluoromethyl)benzoyl]amino]propyl]carbamate as substrate. MS (ESI) m/z: 503.3 [M+H]⁺

### Reference Example 534

### 4-[[3-[4-(difluoromethoxy)phenyl]imidazo[1,2-a]pyrazin-8-yl]amino]-2-isopropyl-N-methyl-benzamide

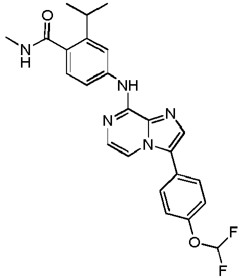

### Step 1: methyl 2-isopropenyl-4-nitro-benzoate

The title compound was obtained in analogy to step 1 in the preparation of Reference Example 535 using 2-bromo-4-nitro-benzoate and 4,4,5,5-tetramethyl-2-(prop-1-en-2-yl)-1,3,2-dioxaborolane.

¹H NMR (400 MHz, chloroform-d) δ = 8.20 - 8.12 (m, 2 H), 7.94 - 7.90 (m, 1 H), 5.25 (quin, *J* = 1.4 Hz, 1 H), 4.99 - 4.94 (m, 1 H), 3.92 (s, 3 H), 2.14 (dd, *J=* 0.9, 1.5 Hz, 3 H) ppm.

### Step 2: 2-isopropenyl-N-methyl-4-nitro-benzamide

The title compound was obtained in analogy to step 4 in the preparation of Reference Example 486 using methyl 2-isopropenyl-4-nitro-benzoate.

### Step 3: 4-amino-2-isopropyl-N-methyl-benzamide

The title compound was obtained in analogy to step 3 in the preparation of Reference Example 535 using 2-isopropenyl-N-methyl-4-nitro-benzamide as substrate. MS (ESI) m/z: 193.2 [M+H]⁺

### Step 4: 4-[[3-[4-(difluoromethoxy)phenyl]imidazo[1,2-a]pyrazin-8-yl]amino]-2-isopropyl-N-methyl-benzamide

The title compound was obtained in analogy to step 2 in the preparation of Reference Example 515 using 8-chloro-3-(4-(difluoromethoxy)phenyl)imidazo[1,2-a]pyrazine and 4-amino-2-isopropyl-N-methyl-benzamide for this substitution reaction. MS (ESI) m/z: 452.2 [M+H]⁺

### Reference Example 536

### N-[2-(2-aminoethoxy)ethyl]-4-[[3-(2,3-difluoro-4-methoxy-phenyl)imidazo[1,2-a]pyrazin-8-yl]amino]-2-(2-fluoroethyl)benzamide

### Step 1: tert-butyl 2-formyl-4-nitrobenzoate

To a solution of tert-butyl 4-nitro-2-vinyl-benzoate (3.2 g, 12.84 mmol, 1 eq) in DCM (50 mL) cooled to -78 was bubbled ozone (6162.24 mg, 128.38 mmol, 10 eq) until the reaction mixture turn blue, and the nitrogen was bubbled for 5 min. Dimethylsulfide (10.0 mL, 12.84 mmol, 1 eq) was added, the resulting mixture was stirred at 25 °C for15 h. The mixture was concentrated and the residue was purified by silica gel chromatography eluting with PE:EA=10:1 to afford tert-butyl 2-formyl-4-nitro-benzoate (1.9 g, 7.56 mmol, 58.91% yield) as white solid. MS (ESI) m/z: 252.1 [M+H]⁺

### Step 2: tert-butyl 2-[(E)-2-bromo-2-fluoro-vinyl]-4-nitro-benzoate

To a solution of tert-butyl 2-formyl-4-nitro-benzoate (0.6 g, 2.39 mmol, 1 eq) and triphenylphosphine (2505.51 mg, 9.55 mmol, 4 eq) in THF (20 mL) was added tribromo(fluoro)methane (1616.3 mg, 5.97 mmol, 2.5 eq). The resulting mixture was stirred at 70 °C under nitrogen for 15 h. The mixture was concentrated and then purified by silica gel chromatography eluting with PE:EA = 20:1 to afford tert-butyl 2-[(E)-2-bromo-2-fluorovinyl]-4-nitro-benzoate (600 mg, 1.73 mmol, 73% yield) as white solid.

### Step 3: tert-butyl 4-amino-2-(2-fluoroethyl)benzoate

The title compound was obtained in analogy to step 4 in the preparation of Reference Example 486 using tert-butyl 2-[(E)-2-bromo-2-fluoro-vinyl]-4-nitro-benzoate. MS (ESI) m/z: 240.1 [M+H]⁺

### Step 4: 4-[[3-(2,3-difluoro-4-methoxy-phenyl)imidazo[1,2-a]pyrazin-8-yl]amino]-2-(2-fluoroethyl)benzoic acid

A solution of tert-butyl 4-amino-2-(2-fluoroethyl)benzoate (194.24 mg, 0.810 mmol, 1.2 eq) and 8-chloro-3-(2,3-difluoro-4-methoxy-phenyl)imidazo[1,2-a]pyrazine (200.0 mg, 0.680 mmol, 1 eq) in ACN (4.5 mL) and acetic acid (0.500 mL) was heated to 80 °C for 15 h. The mixture was purified by prep-HPLC to afford 4-[[3-(2,3-difluoro-4-methoxy-phenyl)imidazo[1,2-a]pyrazin-8-yl]amino]-2-(2-fluoroethyl)benzoic acid (160 mg, 0.360 mmol, 53 % yield) as off-white solid. MS (ESI) m/z: 443.2 [M+H]⁺

### Step 5: tert-butyl N-[2-[2-[[4-[[3-(2,3-difluoro-4-methoxy-phenyl)imidazo[1,2-a]pyrazin-8-yl]amino]-2-(2-fluoroethyl)benzoyl]amino]ethoxy]ethyl]carbamate

The title compound was obtained in analogy to step 3 in the preparation of Reference Example 515 using 4-[[3-(2,3-difluoro-4-methoxy-phenyl)imidazo[1,2-a]pyrazin-8-yl]amino]-2-(2-fluoroethyl)benzoic acid and tert-butyl (2-(2-aminoethoxy)ethyl)carbamate. MS (ESI) m/z: 629.1 [M+H]⁺

### Step 6: N-[2-(2-aminoethoxy)ethyl]-4-[[3-(2,3-difluoro-4-methoxy-phenyl)imidazo[1,2-a]pyrazin-8-yl]amino]-2-(2-fluoroethyl)benzamide

The title compound was obtained in analogy to step 2 in the preparation of Reference Example 12 using tert-butyl N-[2-[2-[[4-[[3-(2,3-difluoro-4-methoxy-phenyl)imidazo[1,2-a]pyrazin-8-yl]amino]-2-(2-fluoroethyl)benzoyl]amino]ethoxy]ethyl]carbamate as. MS (ESI) m/z: 529.3 [M+H]⁺

### Reference Example 535

### 2-cyclopropyl-4-[[3-[4-(difluoromethoxy)phenyl]imidazo[1,2-a]pyrazin-8-yl]amino]-N-methyl-benzamide

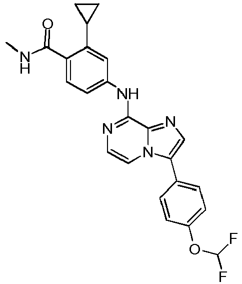

### Step 1: 2-cyclopropyl-4-nitro-benzoate

A solution of methyl 2-bromo-4-nitro-benzoate (600.0 mg, 2.31 mmol, 1 eq), cyclopropylboronic acid (594.49 mg, 6.92 mmol, 3 eq), potassium phosphate (0.96 mL, 11.54 mmol, 5 eq) and 1,1'-bis(di-tert-butylphosphino)ferrocene palladium dichloride (150 mg, 0.230 mmol, 0.100 eq) in toluene (10 mL) and water (0.4 mL) was heated to 100 °C for 15 h under nitrogen. The reaction mixture was concentrated and the residue was purified by silica gel chromatography eluting with PE:EA from 10:1 to 5:1 to afford methyl 2-cyclopropyl-4-nitrobenzoate (450 mg, 2.03 mmol, 88 % yield).

¹H NMR (400 MHz, chloroform-d) δ = 8.04 (dd, *J* = 2.3, 8.5 Hz, 1 H), 7.92 (d, *J=* 8.5 Hz, 1 H), 7.86 (d, *J* = 2.3 Hz, 1 H), 3.99 (s, 3 H), 2.69 (tt, *J* = 5.4, 8.5 Hz, 1 H),, 1.19 - 1.11 (m, 2H), 0.86 - 0.79 (m, 2 H) ppm.

### Step 2: 2-cyclopropyl-N-methyl-4-nitro-benzamide

To a solution of methyl 2-cyclopropyl-4-nitro-benzoate (350.0 mg, 1.58 mmol, 1 eq) in methanol (10 mL) was added methylamine in methanol (10.0 mL). The resulting mixture was heated to 80 °C for 15 h. The mixture was concentrated and the obtained residue was triturated with MTBE (10 mL) to give 2-cyclopropyl-N-methyl-4-nitro-benzamide (220 mg, 1 mmol, 63.14 % yield). MS (ESI) m/z: 222.2 [M+H]⁺

### Step 3: 4-amino-2-cyclopropyl-N-methyl-benzamide

The title compound was obtained in analogy to step 4 in the preparation of Reference Example 486 using 2-cyclopropyl-N-methyl-4-nitro-benzamide. MS (ESI) m/z: 191.2. [M+H]⁺

### Step 4: 8-chloro-3-(4-(difluoromethoxy)phenyl)imidazo[1,2-a]pyrazine

The title compound was obtained in analogy to step 1 in the preparation of Reference Example 515 using 8-chloro-3-iodoimidazo[1,2-a]pyrazine and (4-(difluoromethoxy)phenyl)boronic acid. MS (ESI) m/z: 296.1 [M+H]⁺

### Step 5: 2-cyclopropyl-4-[[3-[4-(difluoromethoxy)phenyl]imidazo[1,2-a]pyrazin-8-yl]amino]-N-methyl-benzamide

The title compound was obtained in analogy to step 2 in the preparation of Reference Example 515 using 8-chloro-3-(4-(difluoromethoxy)phenyl)imidazo[1,2-a]pyrazine and 4-amino-2-cyclopropyl-N-methyl-benzamide as substrates. MS (ESI) m/z: 450.1. [M+H]⁺

### Reference Example 537

### N-[2-(2-aminoethoxy)ethyl]-2-(2,2-difluoroethyl)-4-[[3-(2,3-difluoro-4-methoxy-phenyl)imidazo[1,2-a]pyrazin-8-yl]amino]benzamide; formic acid

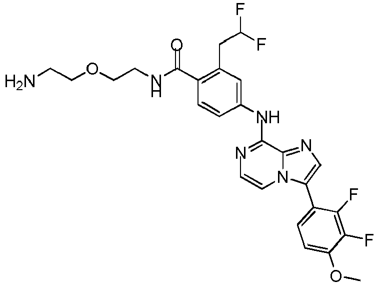

### Step 1: methyl 2-formyl-4-nitrobenzoate

A solution of methyl 4-nitro-2-vinyl-benzoate (3.5 g, 17 mmol, 1 eq) in DCM (20 mL) was stirred under ozone (100 mL, 16.9 mmol, 1 eq) at -40 °C for 0.5 h, the mixture was quenched with dimethylsulfide (10.0 mL, 16.9 mmol, 1 eq) and then concentrated to give the desired product methyl 2-formyl-4-nitro-benzoate (2.8 g, 13 mmol, 79% yield), which was used in the next step without further purification. MS (ESI) m/z: 210.1 [M+H]⁺

### Step 2: methyl 2-(2,2-difluorovinyl)-4-nitro-benzoate

To a solution of methyl 2-formyl-4-nitro-benzoate (647.0 mg, 3.09 mmol, 1 eq) in DMF (5 mL) was added triphenylphosphine (973.6 mg, 3.71 mmol, 1.2 eq) and (2-chloro-2,2-difluoro-acetyl)oxysodium (707.41 mg, 4.64 mmol, 1.5 eq). The resulting suspension was stirred at 100 °C for 0.5 h under nitrogen. The mixture was diluted with water (100 mL), extracted with ethyl acetate (50 mLx2), washed with brine, dried over sodium sulfate and concentrated. The residue was purified by prep-TLC (PE:EA = 5:1) to afford methyl 2-(2,2-difluorovinyl)-4-nitro-benzoate (130 mg, 0.530 mmol, 17.28 % yield) as white solid.

¹H NMR (400 MHz, CHLOROFORM-d) δ = 8.48 - 8.43 (m, 1H), 8.17 - 8.11 (m, 2H), 6.41 - 6.30 (m, 1 H) ppm.

### Step 3: methyl 4-amino-2-(2,2-difluoroethyl)benzoate

The title compound was obtained in analogy to step 4 in the preparation of Reference Example 486 using methyl 2-(2,2-difluorovinyl)-4-nitro-benzoate. MS (ESI) m/z: 216.1 [M+H]⁺

### Step 4: methyl 2-(2,2-difluoroethyl)-4-[[3-(2,3-difluoro-4-methoxy-phenyl)imidazo[1,2-a]pyrazin-8-yl]amino]benzoate

The title compound was obtained in analogy to step 2 in the preparation of Reference Example 515 using methyl 4-amino-2-(2,2-difluoroethyl)benzoate and 1-chloro-6-(2,3-difluoro-4-methoxy-phenyl)pyrrolo[1,2-a]pyrazine. MS (ESI) m/z: 475.2 [M+H]⁺

### Step 5: 2-(2,2-difluoroethyl)-4-[[3-(2,3-difluoro-4-methoxy-phenyl)imidazo[1,2-a]pyrazin-8-yl]amino]benzoic acid

To a solution of methyl 2-(2,2-difluoroethyl)-4-[[3-(2,3-difluoro-4-methoxy-phenyl)imidazo[1,2-a]pyrazin-8-yl]amino]benzoate (60.0 mg, 0.130 mmol, 1 eq) in THF (3 mL) and water (1 mL) was added lithium hydroxide monohydrate (6.37 mg, 0.150 mmol, 1.2 eq). The mixture was acidified with IN aq. HCl to pH = 3 and extracted with ethyl acetate (50 mL), washed with brine, dried over sodium sulfate and concentrated to afford 2-(2,2-difluoroethyl)-4-[[3-(2,3-difluoro-4-methoxy-phenyl)imidazo[1,2-a]pyrazin-8-yl]amino]benzoic acid (50 mg, 0.110 mmol, 85 % yield) as white solid. MS (ESI) m/z: 461.1 [M+H]⁺

### Step 6: tert-butyl N-[2-[2-[[2-(2,2-difluoroethyl)-4-[[3-(2,3-difluoro-4-methoxy-phenyl)imidazo[1,2-a]pyrazin-8-yl]amino]benzoyl]amino]ethoxy]ethyl]carbamate

The title compound was obtained in analogy to step 3 in the preparation of Reference Example 515 using 2-(2,2-difluoroethyl)-4-[[3-(2,3-difluoro-4-methoxy-phenyl)imidazo[1,2-a]pyrazin-8-yl]amino]benzoic acid and N-BOC-2-(2-amino-ethoxy)-ethylamine for this condensation. MS (ESI) m/z: 647.4 [M+H]⁺

### Step 7: N-[2-(2-aminoethoxy)ethyl]-2-(2,2-difluoroethyl)-4-[[3-(2,3-difluoro-4-methoxyphenyl)imidazo [1,2-a] pyrazin-8-yl] amino] benzamide

The title compound was obtained in analogy to step 2 in the preparation of Reference Example 12 using tert-butyl N-[2-[2-[[2-(2,2-difluoroethyl)-4-[[3-(2,3-difluoro-4-methoxyphenyl)imidazo[1,2-a]pyrazin-8-yl]amino]benzoyl]amino]ethoxy]ethyl]carbamate as substrate. MS (ESI) m/z: 547.2 [M+H]⁺

### Reference Example 538

### 4-[[3-[4-(difluoromethoxy)phenyl]imidazo[1,2-a]pyrazin-8-yl]amino]-2-(2-hydroxyethoxymethyl)-N-methyl-benzamide

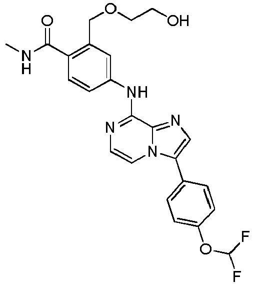

### Step 1: 1-bromo-2-(chloromethyl)-4-nitro-benzene

A mixture of (2-bromo-5-nitro-phenyl)methanol (2.0 g, 8.62 mmol, 1 eq) and SOCl₂ (1.03 g, 8.62 mmol, 1 eq) in 1,4-dioxane (20 mL) was heated to 60 °C for 1 h. The mixture was concentrated and the residue was purified by silica gel chromatography eluting with PE:EA = 20:1 to afford 1-bromo-2-(chloromethyl)-4-nitro-benzene (1.8 g, 7.19 mmol, 83 % yield).

¹H NMR (400 MHz, CHLOROFORM-d) δ = 8.31 (d, *J=* 2.7 Hz, 1 H), 7.99 (dd, *J* = 2.7, 8.8 Hz, 1 H), 7.72 (d, *J* = 8.7 Hz, 1 H), 4.73 - 4.64 (m, 2H) ppm.

### Step 2: 2-[(2-bromo-5-nitro-phenyl)methoxy]ethanol

To an ice-cooled solution of ethylene glycol (1.67 mL, 29.94 mmol, 5 eq) in THF (20 mL) and DMF (20 mL) was added sodium hydride, 60 % in oil (0.36 g, 8.98 mmol, 1.5 eq). The resulting mixture was stirred for 5 min, and then 1-bromo-2-(chloromethyl)-4-nitro-benzene (1.5 g, 5.99 mmol, 1 eq) in THF (5mL) was added. The resulting suspension was stirred at 10 °C for 15 h. The mixture was poured into saturated aq. NH₄Cl (200 mL) solution, extracted with EA (50 mLx2), washed with brine, dried over sodium sulfate and concentrated. The crudematerial was purified by silica gel chromatography eluting with PE:EA from 5:1 to 3:1 to afford 2-[(2-bromo-5-nitro-phenyl)methoxy]ethanol (1 g, 3.62 mmol, 60 % yield).

¹H NMR (400 MHz, CHLOROFORM-d) δ = 8.39 (d, *J=* 2.8 Hz, 1 H), 8.04 (dd, *J* = 2.8, 8.7 Hz, 1 H), 7.75 (d, *J* = 8.7 Hz, 1 H), 4.69 (s, 2 H), 3.93 - 3.88 (m, 2 H), 3.81 - 3.77 (m, 2 H) ppm.

### Step 3: 2-(2-hydroxyethoxymethyl)-4-nitro-benzonitrile

A mixture of 2-[(2-bromo-5-nitro-phenyl)methoxy]ethanol (1.0 g, 3.62 mmol, 1 eq), zinc cyanide (1.28 g, 10.87 mmol, 3 eq) and tetrakis(triphenylphosphine)palladium(0) (418.56 mg, 0.360 mmol, 0.100 eq) in DMF (10 mL) was heated to 110 °C for 15 h under nitrogen protection. The mixture was diluted with water (100 mL), extracted with EA (50 mLx2), dried over sodium sulfate and concentrated. The residue was purified by silica gel chromatography eluting with PE:EA from 10:1 to 5:1 to afford 2-(2-hydroxyethoxymethyl)-4-nitro-benzonitrile (600 mg, 2.7 mmol, 74 % yield).

¹H NMR (400 MHz, CHLOROFORM-d) δ = 8.54 - 8.44 (m, 1 H), 8.28 (dd, *J=* 2.3, 8.5 Hz, 1 H), 7.90 (d, *J* = 8.4 Hz, 1 H), 4.87 (s, 2 H), 3.92 - 3.86 (m, 2 H), 3.83 - 3.77 (m, 2 H) ppm.

### Step 4: 2-(2-hydroxyethoxymethyl)-4-nitro-benzoic acid

A mixture of 2-(2-hydroxyethoxymethyl)-4-nitro-benzonitrile (600 mg, 2.7 mmol, 1 eq) in aq.sodium hydroxide (10.0 mL, 0.270 mmol, 0.100 eq) was heated to 100 °C for 3 h. The mixture was acidified with 2 N HCl aq. to pH = 4 and extracted with EA (100 mLx2), washed with brine, dried over Na₂SO₄ and concentrated. The residue was triturated with DCM to afford 2-(2-hydroxyethoxymethyl)-4-nitro-benzoic acid (450 mg, 1.87 mmol, 69.09 % yield). MS (ESI) m/z: 264.0. [M+Na]⁺

### Step 5: 2-(2-hydroxyethoxymethyl)-N-methyl-4-nitro-benzamide

The title compound was obtained in analogy to step 3 in the preparation of Reference Example 515 using 2-(2-hydroxyethoxymethyl)-4-nitro-benzoic acid and methylamine (2 M in THF). MS (ESI) m/z: 277.0 [M+Na]⁺

### Step 6: 4-amino-2-(2-hydroxyethoxymethyl)-N-methyl-benzamide

The title compound was obtained in analogy to step 4 in the preparation of Reference Example 486 using 2-(2-hydroxyethoxymethyl)-N-methyl-4-nitro-benzamide. MS (ESI) m/z: 225.3 [M+H]⁺

### Step 7: 4-[[3-[4-(difluoromethoxy(phenyl]imidazo[1,2-a]pyrazin-8-yl]amino]-2-(2-hydroxyethoxymethyl)-N-methyl-benzamide

A mixture of 4-amino-2-(2-hydroxyethoxymethyl)-N-methyl-benzamide (120 mg, 0.540 mmol, 1 eq), 8-chloro-3-[4-(difluoromethoxy)phenyl]imidazo[1,2-a]pyrazine (158.21 mg, 0.540 mmol, 1 eq), Brettphos Pd G3 (45.88 mg, 0.050 mmol, 0.100 eq) and K₂CO₃ (147.91 mg, 1.07 mmol, 2 eq) was heated to 110 °C for 15 h under nitrogen. The mixture was diluted with water, and extracted with ethyl acetate (100 mL×2). The combined organic phases were washed with brine, dried over sodium sulfate and concentrated. The residue was purified by prep-TLC (DCM:MeOH = 10:1) to afford 4-[[3-[4-(difluoromethoxy)phenyl]imidazo[1,2-a]pyrazin-8-yl]amino]-2-(2-hydroxyethoxymethyl)-N-methyl-benzamide (55.5 mg, 0.110 mmol, 21 % yield). MS (ESI) m/z: 484.0 [M+H]⁺

### Reference Example 531

### 2-(3-aminopropyl)-6-[[3-(2,3-difluoro-4-methoxy-phenyl)imidazo[1,2-a]pyrazin-8-yl]amino]-3,4-dihydroisoquinolin-1-one

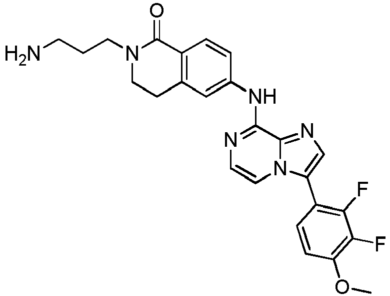

### Step 1: 6-bromo-2-(3-tetrahydropyran-2-yloxypropyl)-3,4-dihydroisoquinolin-1-one

To a mixture of 6-bromo-3,4-dihydro-2H-isoquinolin-1-one (400 mg, 1.77 mmol, 1 eq) in DMF (10 mL) was added sodium hydride (141.55 mg, 3.54 mmol, 2 eq) at 0 °C. Then the mixture was stirred at 0 °C for 0.5 h. Then 2-(3-bromopropoxy)tetrahydro-2H-pyran (1184.29 mg, 5.31 mmol, 3 eq) was added to the mixture at 25 °C and the mixture was stirred at 25 °C for another 15.5 h. Then the mixture was poured into water (30.0 mL) and extracted with ethyl acetate (50.0 mL^{∗}2). The organic phase was dried and concentrated in vacuo to give the crude product as brown oil. The crude product was purified by silica gel column chromatography eluting with PE/EA from 20:1 to 2:1 to give 6-bromo-2-(3-tetrahydropyran-2-yloxypropyl)-3,4-dihydroisoquinolin-1-one (500 mg, 1.36 mmol, 76.73 % yield) as yellow oil. MS (ESI, m/z): 284.0 [M-84+H]⁺, 286.1 [M-84+2+H]⁺

### Step 2: 3-(2,3-difluoro-4-methoxyphenyl)-N-(4-methoxybenzyl)imidazo[1,2-a]pyrazin-8-amine

The title compound was obtained in analogy to step 2 in the preparation of Reference Example 515 using 8-chloro-3-(2,3-difluoro-4-methoxyphenyl)imidazo[1,2-a]pyrazine and (4-methoxyphenyl)methanamine. MS (ESI) m/z: 397.2 [M+H]⁺

### Step 3: 3-(2,3-difluoro-4-methoxyphenyl)imidazo[1,2-a]pyrazin-8-amine

To a stirred solution of 3-(2,3-difluoro-4-methoxyphenyl)-N-(4-methoxybenzyl)imidazo[1,2-a]pyrazin-8-amine (2 g, 5 mmol, 1 eq) in DCM (10 mL) was added TFA (10 mL). The reaction mixture was stirred at 30 °C for 16 hThe mixture was concentrated under reduced pressure to give 1.5 g of the crude product, used directly in the next step without further purification.

### Step 4: 6-[[3-(2,3-difluoro-4-methoxy-phenyl)imidazo[1,2-a]pyrazin-8-yl]amino]-2-(3-tetrahydropyran-2-yloxypropyl)-3,4-dihydroisoquinolin-1-one

To a mixture of 6-bromo-2-(3-tetrahydropyran-2-yloxypropyl)-3,4-dihydroisoquinolin-1-one (440.16 mg, 1.2 mmol, 1.1 eq) and 3-(2,3-difluoro-4-methoxy-phenyl)imidazo[1,2-a]pyrazin-8-amine (300.0 mg, 1.09 mmol, 1 eq) in tert-butanol (10 mL) was added potassium carbonate (300 mg, 2.17 mmol, 2 eq) and BrettPhos-Pd-G3 (197.0 mg, 0.220 mmol, 0.200 eq) at 25 °C. Then the mixture was stirred at 110 °C for 16 h. Then the mixture was poured into water (30.0 mL) and extracted with ethyl acetate (50.0 mL^{∗}2). The organic phase was dried and concentrated in vacuo to give the crude product as brown solid. The crude product was triturated with ethyl acetate (20.0 mL) to give 6-[[3-(2,3-difluoro-4-methoxy-phenyl)imidazo[1,2-a]pyrazin-8-yl]amino]-2-(3-tetrahydropyran-2-yloxypropyl)-3,4-dihydroisoquinolin-1-one (400 mg, 0.710 mmol, 65.32 % yield) as white solid. MS (ESI) m/z: 564.3 [M+H]⁺

### Step 5: 6-((3-(2,3-difluoro-4-methoxyphenyl)imidazo[1,2-a]pyrazin-8-yl)amino)-2-(3-hydroxypropyl)-3,4-dihydroisoquinolin-1(2H)-one

A mixture of 6-[[3-(2,3-difluoro-4-methoxy-phenyl)imidazo[1,2-a]pyrazin-8-yl]amino]-2-(3-tetrahydropyran-2-yloxypropyl)-3,4-dihydroisoquinolin-1-one (400.0 mg, 0.710 mmol, 1 eq) in HCl/MeOH (10.0 mL, 40 mmol, 56.36 eq) was stirred at 25 °C for 16 h. Then the mixture was concentrated in vacuo to give the crude product as grey solid. The crude product was purified by prep-HPLC (FA) to give 250 mg desired product as white solid. MS (ESI) m/z: 480.2 [M+H]⁺

### Step 6: 3-[6-[[3-(2,3-difluoro-4-methoxy-phenyl)imidazo[1,2-a]pyrazin-8-yl]amino]-1-oxo-3,4-dihydroisoquinolin-2-yl]propyl methanesulfonate

The title compound was obtained in analogy to step 4 in the preparation of Reference Example 518 using 6-[[3-(2,3-difluoro-4-methoxy-phenyl)imidazo[1,2-a]pyrazin-8-yl]amino]-2-(3-hydroxypropyl)-3,4-dihydroisoquinolin-1-one as starting material. MS (ESI) m/z: 558.2 [M+H]⁺

### Step 7: 2-(3-aminopropyl)-6-[[3-(2,3-difluoro-4-methoxy-phenyl)imidazo[1,2-a]pyrazin-8-yl]amino]-3,4-dihydroisoquinolin-1-one

To a mixture of NH₃ in THF (2.0 mL, 8 mmol, 111.51 eq) was added 3-[6-[[3-(2,3-difluoro-4-methoxy-phenyl)imidazo[1,2-a]pyrazin-8-yl]amino]-1-oxo-3,4-dihydroisoquinolin-2-yl]propyl methanesulfonate (40.0 mg, 0.070 mmol, 1 eq) at -78 °C. Then the mixture was stirred at -78 °C for 5 h. Then the mixture was concentrated in vacuo and purified by prep-HPLC to give 2-(3-aminopropyl)-6-[[3-(2,3-difluoro-4-methoxy-phenyl)imidazo[1,2-a[pyrazin-8-yl[amino[-3,4-dihydroisoquinolin-1-one (14 mg, 0.030 mmol, 40 % yield) as a white solid. MS (ESI) m/z: 479.3 [M+H]⁺

The following additional Examples have been prepared with the methods described above:

| Ex. | Name | CHEMISTRY | ESI MS [M+H]⁺ |
|---|---|---|---|
| 15 | 4-[[3-(4-methoxyphenyl)imidazo [1,2-a]pyrazin-8-yl]amino]-N,2-dimethyl-N-(1-methylpiperidin-4-yl)benzamide | 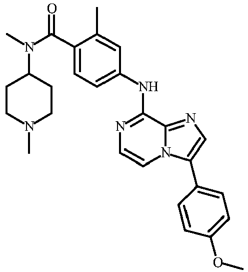 | 484 |
| 16 | 4-[[3-(4-methoxyphenyl)imidazo[1,2-a]pyrazin-8-yl]amino]-N-methyl-N-(1-methylpiperidin-4-yl)benzamide | 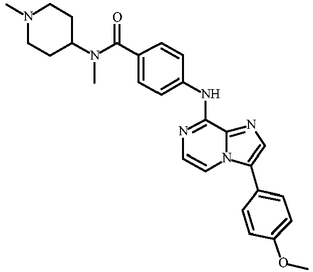 | 470 (M-H)- |
| 17 | 4-[[3-(3-fluoro-4-methoxyphenyl)imidazo[1,2-a]pyrazin-8-yl]amino]-N-[(3R,4R)-4-hydroxypyrrolidin-3-yl]-N,2-dimethyl benzamide;hydrochloride | 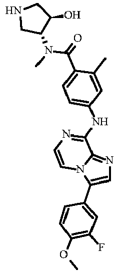 | 491.1 |
| 18 | N-[1-(carbamimidamidomethyl)cyclopropy 1]-4-[[3-(2,3-difluoro-4-methoxyphenyl)imidazo[1,2-a]pyrazin-8-yl]amino]-2-ethylbenzamide | 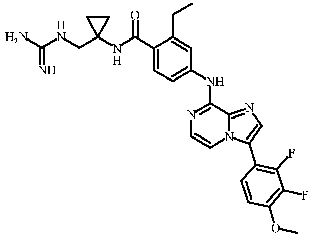 | 535.3 |
| 19 | 4-[[3-(2,3-difluoro-4-methoxyphenyl)imidazo[1,2-a]pyrazin-8-yl]amino]-N-[1-[(ethanimidoylamino)methyl]cyclopr opyl]-2-ethylbenzamide | 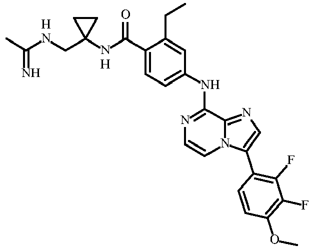 | 534.3 |
| 20 | 2-chloro-4-[[3-(3-fluoro-4-methoxyphenyl)imidazo[1,2-a]pyrazin-8-yl]amino]-N-methyl-N-piperidin-4-ylbenzamide;hydrochloride | 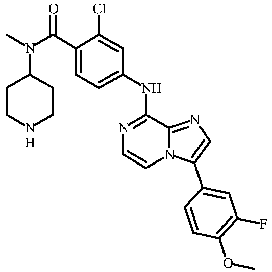 | 510.2 |
| 21 | 4-[[3-(3-fluoro-4-methoxyphenyl)imidazo[1,2-a]pyrazin-8-yl]amino]-N,2-dimethyl-N-piperidin-4-ylbenzamide;hydrochloride | 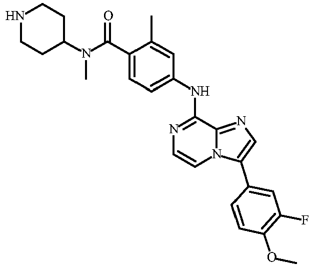 | 489.2 |
| 22 | 4-[[3-[4-(difluoromethoxy)-3-fluorophenyl]imidazo[1,2-a]pyrazin-8-yl]amino]-N,2-dimethyl-N-piperidin-4-ylbenzamide;hydrochloride | 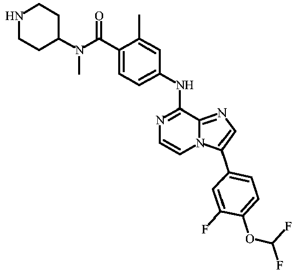 | 523.6 |
| 23 | 4-[[3-[4-(difluoromethoxy)phenyl]imidazo[1,2 -a]pyrazin-8-yl]amino]-2-methyl-N-[(2S,3S,4R,5S,6R)-2,4,5-trihydroxy-6-(hydroxymethyl)oxan-3-yl]benzamide | 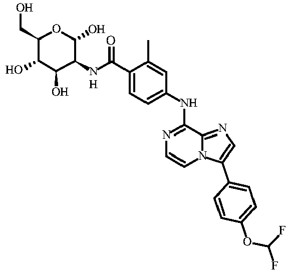 | 572.1 |
| 24 | N-[1-(1,1-dioxothian-4-yl)piperidin-4-yl]-4-[[3-(3-fluoro-4-methoxyphenyl)imidazo[1,2-a]pyrazin-8-yl]amino]-2-methylbenzamide | 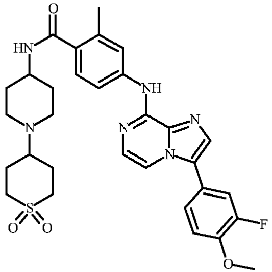 | 607.3 |
| 25 | 4-[[4-[[3-(3-fluoro-4-methoxyphenyl)imidazo[1,2-a]pyrazin-8-yl]amino]-2-methylbenzoyl]amino]benzoic acid | 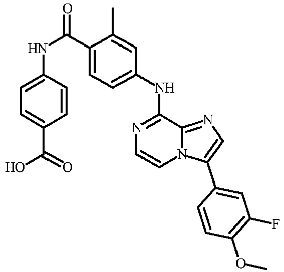 | 512.2 |
| 26 | 4-[[3-(3-fluoro-4-methoxyphenyl)imidazo[1,2-a]pyrazin-8-yl]amino]-N-[(3R,4S)-3-fluoropiperidin-4-yl]-2-methyl benzamide;2,2,2-trifluoroacetic acid | 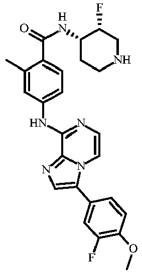 | 491.3 (M-H)⁻ |
| 27 | 4-[[3-(2,3-difluoro-4-methoxyphenyl)imidazo[1,2-a]pyrazin-8-yl]amino]-2-ethyl-N-[3-(hydroxymethyl)oxetan-3- yl]benzamide | 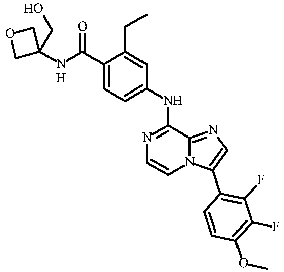 | 510.4 |
| 28 | 4-[[3-(2,3-difluoro-4-methoxyphenyl)imidazo[1,2-a]pyrazin-8-yl]amino]-2-ethyl-N-[(3S)-pyrrolidin-3-yl]benzamide;hydrochloride | 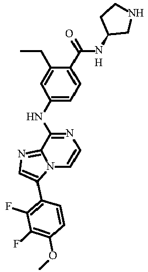 | 493.4 |
| 29 | N-[1-(aminomethyl)cyclopropyl]-4-[[3-[4-(difluoromethoxy)-2,3-difluorophenyl]imidazo[1,2-a]pyrazin-8-yl]amino]-2-ethylbenzamide;hydrochloride | 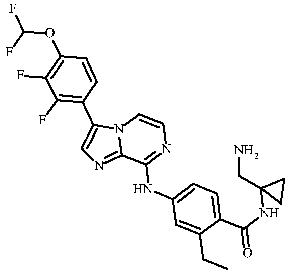 | 529.4 |
| 30 | 4-[[3-(2,3-difluoro-4-methoxyphenyl)imidazo[1,2-a]pyrazin-8-yl]amino]-2-ethyl-N-[(3S)-2-oxopyrrolidin-3-yl]benzamide | 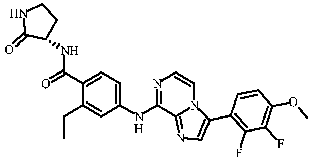 | 507.4 |
| 31 | N-(4-aminocyclohexyl)-4-[[3-(2,3-difluoro-4-methoxyphenyl)imidazo[1,2-a]pyrazin-8-yl]amino]-2-methylbenzamide | 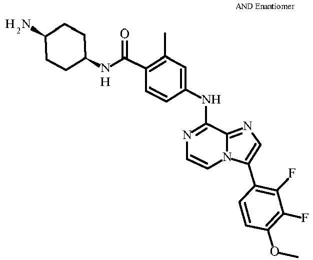 | 507.3 |
| 32 | 4-[[3-(2,3-difluoro-4-methoxyphenyl)imidazo [1,2-a]pyrazin-8-yl]ammo]-N-[1-[(2S,4R)-4-hydroxypyrrolidine-2-carbonyl]piperidin-4-yl]-2-methylbenzamide | 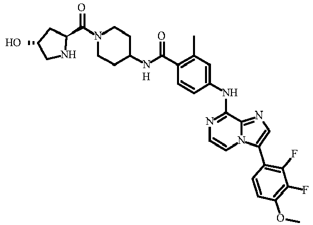 | 606.4 |
| 33 | 4-[[3-(2,3-difluoro-4-methoxyphenyl)imidazo[1,2-a]pyrazin-8-yl]amino]-N-[4-[[1-(dimethylamino)-2-methylprop-1-enyl]amino]cyclohexyl]-2-methylbenzamide;formic acid | 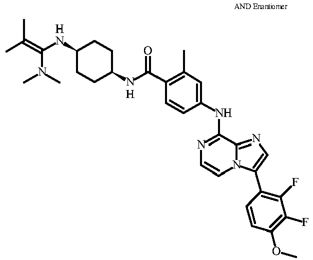 | 604.3 |
| 34 | (2S,4R)-N-[4-[[4-[[3-(2,3-difluoro-4-methoxyphenyl)imidazo[1,2-a]pyrazin-8-yl]amino]-2-methylbenzoyl]amino]cyclohexyl]-4-hydroxypyrrolidine-2-carboxamide | 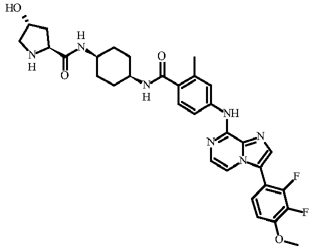 | 620.3 |
| 35 | N-(3-aminocyclobutyl)-4-[[3-(2,3-difluoro-4-methoxyphenyl)imidazo[1,2-a]pyrazin-8-yl]amino]-2-methylbenzamide | 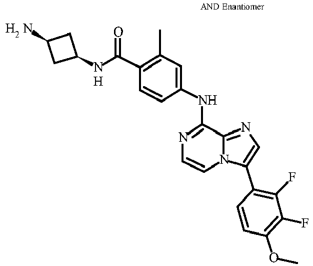 | 479.3 |
| 36 | rac-(2R,4S)-N-[3-[[4-[[3-(2,3-difluoro-4-methoxyphenyl)imidazo[1,2-a]pyrazin-8-yl]amino]-2-methylbenzoyl]amino]cyclopentyl]-4-hydroxypyrrolidine-2-carboxamide | 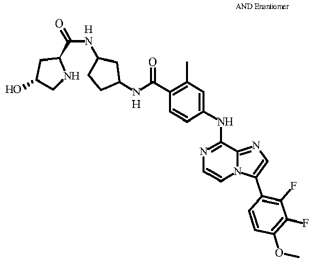 | 606.4 |
| 37 | N-[3-[[(2S)-2-amino-5-carbamimidamidopentanoyl]amino]cy clobutyl]-4-[[3-(2,3-difluoro-4-methoxyphenyl)imidazo[1,2-a]pyrazin-8-yl]amino]-2-ethylbenzamide;2,2,2-trifluoroacetic acid | 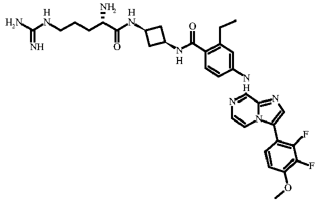 | 649.4 |
| 38 | 4-[[3-(2,3-difluoro-4-methoxyphenyl)imidazo[1,2-a]pyrazin-8-yl]amino]-2-methyl-N-[(3R)-pyrrolidin-3-yl]benzamide | 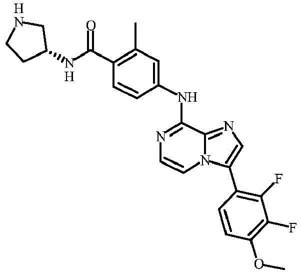 | 479.3 |
| 39 | N-(2-aminocyclobutyl)-4-[[3-(2,3-difluoro-4-methoxyphenyl)imidazo[1,2-a]pyrazin-8-yl]amino]-2-ethylbenzamide | 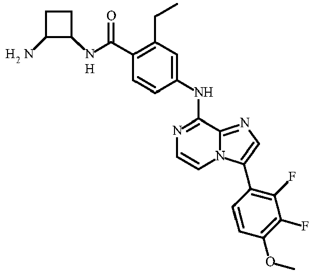 | 493.4 |
| 40 | N-(3-(aminomethyl)oxetan-3-yl)-4-((3-(2,3-difluoro-4-methoxyphenyl)imidazo[1,2-a]pyrazin-8-yl)amino)-2-ethylbenzamidehydrochloride | | 509.3 |
| | | 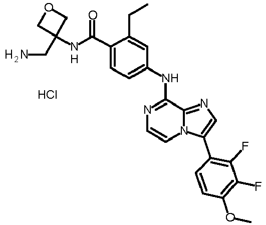 | |
| 41 | 4-((3-(2,3-difluoro-4-methoxyphenyl)imidazo[1,2-a]pyrazin-8-yl)amino)-2-ethyl-N-(piperidin-3-yl)benzamide | 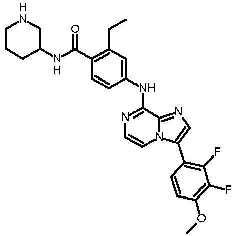 | 507.3 |
| 42 | trans-N-((1r,4r)-4-aminocyclohexyl)-4-((3-(2,3-difluoro-4-methoxyphenyl)imidazo[1,2-a]pyrazin-8-yl)amino)-2-ethylbenzamide | 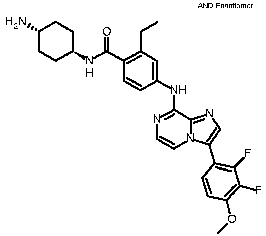 | 521.3 |
| 43 | rac-N-((1R,3R)-3-aminocyclopentyl)-4-((3-(2,3-difluoro-4-methoxyphenyl)imidazo[1,2-a]pyrazin-8-yl)amino)-2-ethylbenzamide | 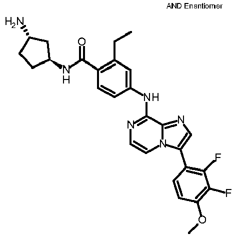 | 507.3 |
| 44 | N-(1-(L-arginyl)piperidin-4-yl)-4-((3-(2,3-difluoro-4-methoxyphenyl)imidazo[1,2-a]pyrazin-8-yl)amino)-2-ethylbenzamide bis(2,2,2-trifluoroacetate) | 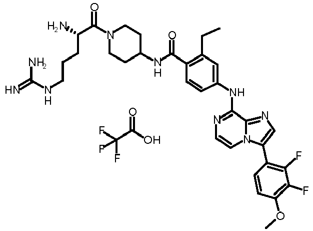 | 663.4 |
| 45 | N-((1S,4r)-4-((S)-2-amino-5-guanidinopentanamido)cyclohexyl)-4-((3-(2,3-difluoro-4-methoxyphenyl)imidazo [1,2-a]pyrazin-8-yl)amino)-2-ethylbenzamide bis(2,2,2-trifluoroacetate) | 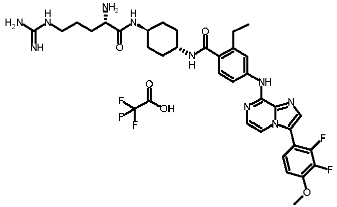 | 677.5 |
| 46 | rac-N-(3-((R)-2-amino-5-guanidinopentanamido)cyclopentyl)-4-((3-(2,3-difluoro-4-methoxyphenyl)imidazo[1,2-a]pyrazin-8-yl)amino)-2-ethylbenzamide bis(2,2,2-trifluoroacetate) | 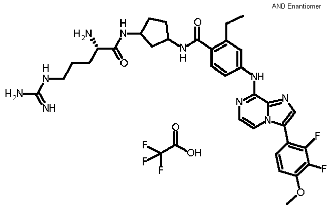 | 663.4 |
| 47 | N-((R)-1-(L-arginyl)pyrrolidin-3-yl)-4-((3-(2,3-difluoro-4-methoxyphenyl)imidazo[1,2-a]pyrazin-8-yl)amino)-2-ethylbenzamide bis(2,2,2-trifluoroacetate) | 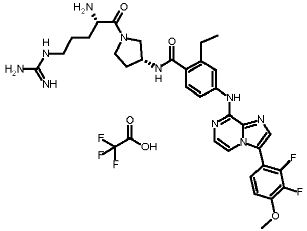 | 649.4 |
| 48 | rac-N-(1-(D-arginyl)piperidin-3-yl)-4-((3-(2,3-difluoro-4-methoxyphenyl)imidazo[1,2-a]pyrazin-8-yl)amino)-2-ethylbenzamide bis(2,2,2-trifluoroacetate) | 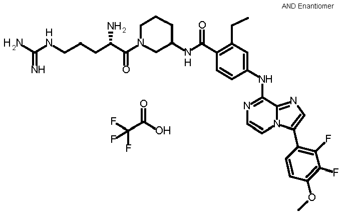 | 663.4 |
| 49 | (2S,4R)-N-(2-(4-((3-(2,3-difluoro-4-methoxyphenyl)imidazo[1,2-a]pyrazin-8-yl)amino)-2-ethylbenzamido)cyclohexyl)-4-hydroxypyrrolidine-2-carboxamide dihydrochloride | 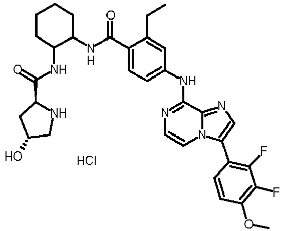 | 634.3 |
| 50 | 4-((3-(2,3-difluoro-4-methoxyphenyl)imidazo[1,2-a]pyrazin-8-yl)amino)-2-methyl-N-(piperidin-4-yl)benzamide | 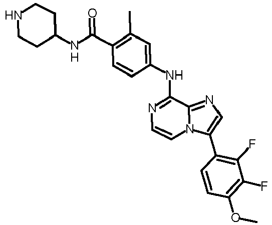 | 493.3 |
| 51 | 4-((3-(2,3-difluoro-4-methoxyphenyl)imidazo[1,2-a]pyrazin-8-yl)amino)-2-methyl-N-(piperidin-3-yl)benzamide | 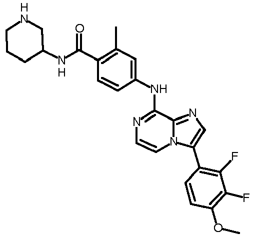 | 493.3 |
| 52 | rac-N-((1r,4r)-4-aminocyclohexyl)-4-((3-(2,3-difluoro-4-methoxyphenyl)imidazo [1,2-a]pyrazin-8-yl)amino)-2-methylbenzamide | 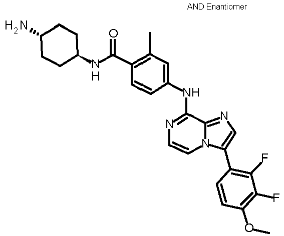 | 507.3 |
| 53 | (2S,4R)-N-((1R,2S)-2-(4-((3-(2,3-difluoro-4-methoxyphenyl)imidazo [1,2-a]pyrazin-8-yl)amino)-2-ethylbenzamido)cyclopentyl)-4-hydroxypyrrolidine-2-carboxamide | 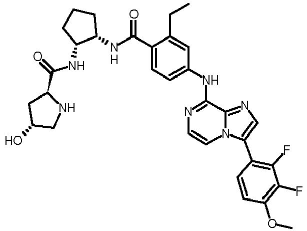 | 620.5 |
| 54 | rac-N-((1R,3R)-3-aminocyclopentyl)-4-((3-(2,3-difluoro-4-methoxyphenyl)imidazo[1,2-a]pyrazin-8-yl)amino)-2-methylbenzamide | 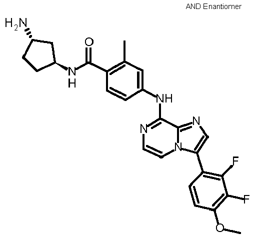 | 493.3 |
| 55 | (S)-4-((3-(2,3-difluoro-4-methoxyphenyl)imidazo [1,2-a]pyrazin-8-yl)amino)-2-methyl-N-(pyrrolidin-3-yl)benzamide (S)-4-((3-(2,3-difluoro-4-methoxyphenyl)imidazo [1,2-a]pyrazin-8-yl)amino)-2-methyl-N-(pyrrolidin-3-yl)benzamide | 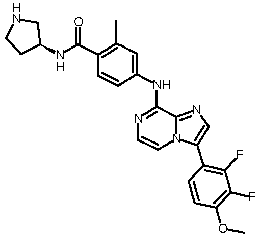 | 479.3 |
| 56 | N-(azetidin-3-yl)-4-((3-(2,3-difluoro-4-methoxyphenyl)imidazo[1,2-a]pyrazin-8-yl)amino)-2-methylbenzamide | 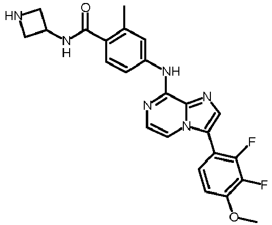 | 465.3 |
| 57 | rac-N-((1r,3r)-3-aminocyclobutyl)-4-((3-(2,3-difluoro-4-methoxyphenyl)imidazo[1,2-a]pyrazin-8-yl)amino)-2-methylbenzamide | 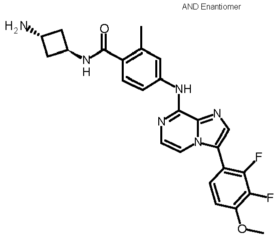 | 479.3 |
| 58 | rac-N-((1R,2R)-2-aminocyclopropyl)-4-((3-(2,3-difluoro-4-methoxyphenyl)imidazo[1,2-a]pyrazin-8-yl)amino)-2-ethylbenzamide | 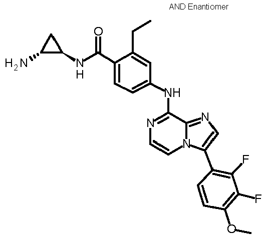 | 479.3 |
| 59 | rac-4-((3-(2,3-difluoro-4-methoxyphenyl)imidazo[1,2-a]pyrazin-8-yl)amino)-N-(1-((2R,4S)-4-hydroxypyrrolidine-2-carbonyl)piperidin-3-yl)-2-methylbenzamide | 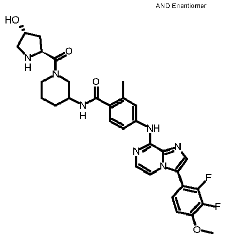 | 606.4 |
| 60 | rac-4-((3-(2,3-difluoro-4-methoxyphenyl)imidazo[1,2-a]pyrazin-8-yl)amino)-N-((1s,4s)-4-isobutyramidocyclohexyl)-2-methylbenzamide | 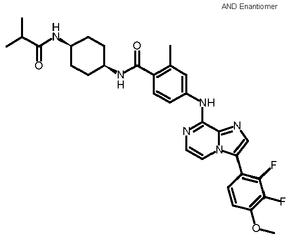 | 577.275 |
| 61 | N-(3-aminobicyclo[1.1.1]pentan-1-yl)-4-((3-(2,3-difluoro-4-methoxyphenyl)imidazo [1,2-a]pyrazin-8-yl)amino)-2-ethylbenzamide dihydrochloride | 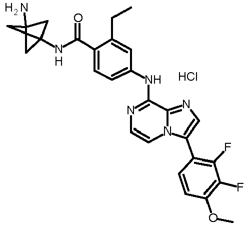 | 505.4 |
| 62 | (2S,4R)-N-((1r,4S)-4-(4-((3-(2,3-difluoro-4-methoxyphenyl)imidazo[1,2-a]pyrazin-8-yl)amino)-2-methylbenzamido)cyclohexyl)-4-hydroxypyrrolidine-2-carboxamide | 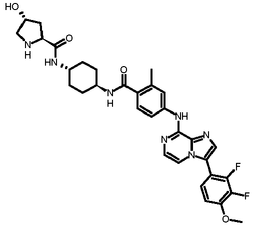 | 620.5 |
| 63 | 4-((3-(2,3-difluoro-4-methoxyphenyl)imidazo[1,2-a]pyrazin-8-yl)amino)-N-((R)-1-((2S,4R)-4-hydroxypyrrolidine-2-carbonyl)pyrrolidin-3-yl)-2-methylbenzamide | 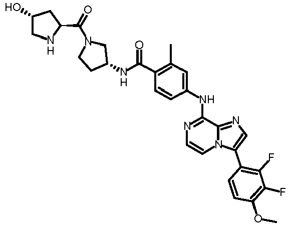 | 592.4 |
| 64 | 4-((3-(2,3-difluoro-4-methoxyphenyl)imidazo[1,2-a]pyrazin-8-yl)amino)-N-((S)-1-((2S,4R)-4-hydroxypyrrolidine-2-carbonyl)pyrrolidin-3-yl)-2-methylbenzamide | 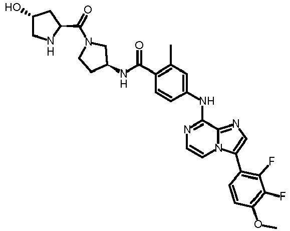 | 592.5 |
| 65 | 4-((3-(2,3-difluoro-4-methoxyphenyl)imidazo[1,2-a]pyrazin-8-yl)amino)-N-(1-((2S,4R)-4-hydroxypyrrolidine-2-carbonyl)azetidin-3-yl)-2-methylbenzamide | 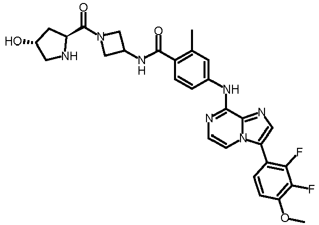 | 578.4 |
| 66 | (2S,4R)-N-((1s,3R)-3-(4-((3-(2,3-difluoro-4-methoxyphenyl)imidazo[1,2-a]pyrazin-8-yl)amino)-2-methylbenzamido)cyclobutyl)-4-hydroxypyrrolidine-2-carboxamide | 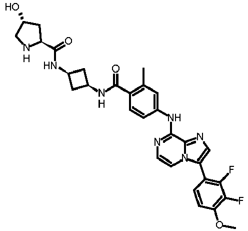 | 592.5 |
| 67 | (2S,4R)-N-((1r,3S)-3-(4-((3-(2,3-difluoro-4-methoxyphenyl)imidazo[1,2-a]pyrazin-8-yl)amino)-2-methylbenzamido)cyclobutyl)-4-hydroxypyrrolidine-2-carboxamide | 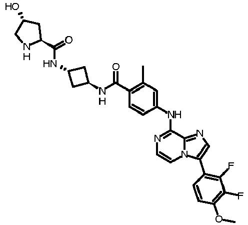 | 592.4 |
| 68 | (2S,4R)-N-[(1RS,2RS)-2-[[4-[[3-(2,3-difluoro-4-methoxyphenyl)imidazo[1,2-a]pyrazin-8-yl]amino]-2-ethyl-benzoyl]amino]cyclopropyl]-4-hydroxy-pyrrolidine-2-carboxamide | 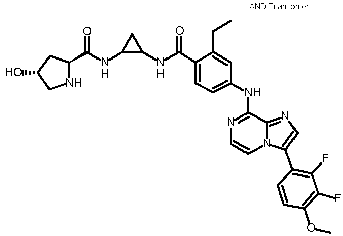 | 592.5 |
| 69 | (2S,4R)-N-(2-(4-((3-(2,3-difluoro-4-methoxyphenyl)imidazo[1,2-a]pyrazin-8-yl)amino)-2-ethylbenzamido)cyclobutyl)-4-hydroxypyrrolidine-2-carboxamide | 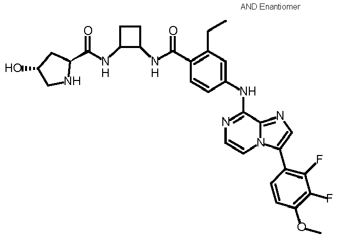 | 606.5 |
| 70 | (2S,4R)-N-(3-(4-((3-(2,3-difluoro-4-methoxyphenyl)imidazo[1,2-a]pyrazin-8-yl)amino)-2-ethylbenzamido)bicyclo[1.1.1]pentan-1-yl)-4-hydroxypyrrolidine-2-carboxamide dihydrochloride | 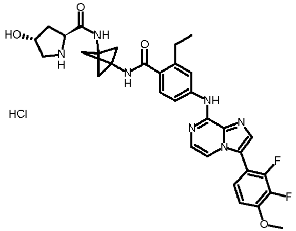 | 618.5 |
| 71 | N-((1R,2S)-2-aminocyclopentyl)-4-((3-(2,3-difluoro-4-methoxyphenyl)imidazo[1,2-a]pyrazin-8-yl)amino)-2-ethylbenzamide dihydrochloride | 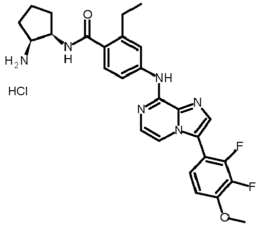 | 507.4 |
| 72 | rac-N-((1R,5S,6s)-3-azabicyclo[3.1.0]hexan-6-yl)-4-((3-(2,3-difluoro-4-methoxyphenyl)imidazo [1,2-a]pyrazin-8-yl)amino)-2-ethylbenzamide hydrochloride | 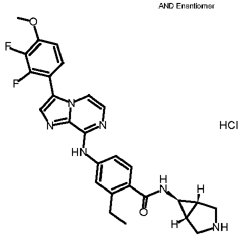 | 503.4 |
| 73 | (2S,4R)-N-[(cis-1SR,2RS)-2-[[4-[[3-(2,3-difluoro-4-methoxyphenyl)imidazo[1,2-a]pyrazin-8-yl]amino]-2-ethyl-benzoyl]amino]cyclopropyl]-4-hydroxy-pyrrolidine-2-carboxamide | 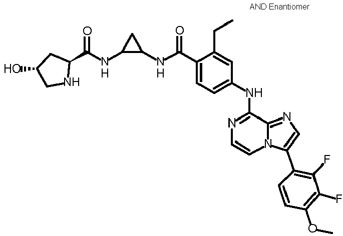 | 592.4 |
| 74 | rac-N-((1R,2S)-2-aminocyclopropyl)-4-((3-(2,3-difluoro-4-methoxyphenyl)imidazo [1,2-a]pyrazin-8-yl)amino)-2-ethylbenzamide | 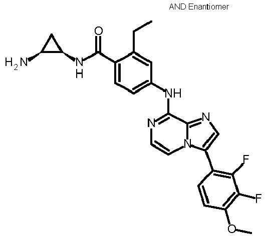 | 497.5 |

### Assay procedures

### Antimicrobial susceptibility testing:

### 90% Growth Inhibitory Concentration (IC90) determination

The in vitro antimicrobial activity of the compounds was determined according to the following procedure:
The assay used a 10-points Iso-Sensitest broth medium to measure quantitatively the *in vitro* activity of the compounds against *Acinetobacter baumannii* ATCC17978 and *Acinetobacter baumannii* ATCC17961.

Stock compounds in DMSO were serially twofold diluted (e.g. range from 50 to 0.097 µM final concentration) in 384 wells microtiter plates and inoculated with 49 µl the bacterial suspension in Iso-Sensitest medium to have a final cell concentration of ~ 5×10⁽⁵⁾ CFU/ml in a final volume/well of 50 ul/well. Microtiter plates were incubated at 35 ± 2 °C.

Bacterial cell growth was determined with the measurement of optical density at λ=600nm each 20 minutes over a time course of 16h. Growth inhibition was calculated during the logarithmic growth of the bacterial cells with determination of the concentration inhibiting 50% (IC50) and 90% (IC90) of the growth.

Table 1 provides the 90% growth inhibitory concentrations (IC90) in micromoles per liter of the compounds of present invention obtained against the strain *Acinetobacter baumannii* ATCC17978 and *Acinetobacter baumannii* ATCC17961.

Particular compounds of the present invention exhibit an IC90 (*A. baumannii* ATCC17978 and *Acinetobacter baumannii* ATCC17961) ≤ 25 µmol/l.

More particular compounds of the present invention exhibit an IC90 (*A. baumannii* ATCC17978 and *Acinetobacter baumannii* ATCC17961) ≤ 5 µmol/l.

Most particular compounds of the present invention exhibit an IC90 (*A. baumannii* ATCC17978 and *Acinetobacter baumannii* ATCC17961) ≤ 1 µmοl/l.

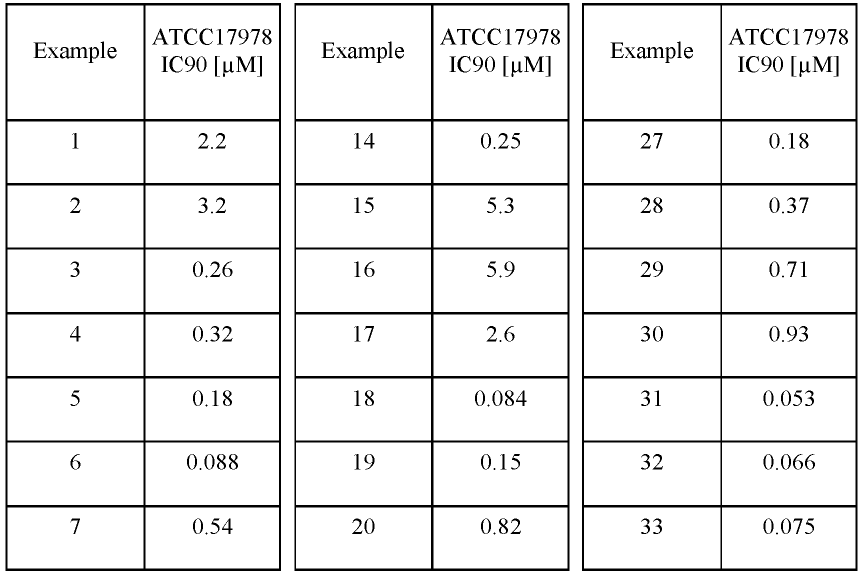

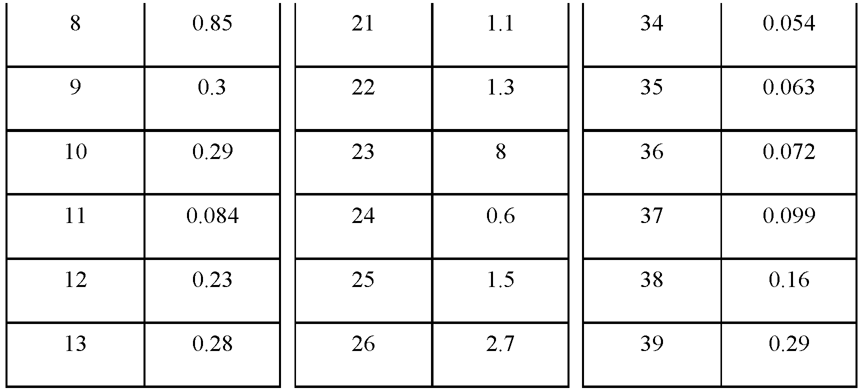

s

| Example | ATCC17961 IC90 [µM] |
|---|---|
| 4 | 0.274 |
| 5 | 0.176 |
| 6 | 0.088 |
| 8 | 0.852 |
| 9 | 0.297 |
| 10 | 0.290 |
| 11 | 0.084 |
| 12 | 0.230 |
| 13 | 0.276 |
| 14 | 0.248 |
| 18 | 0.083 |
| 19 | 0.154 |
| 31 | 0.053 |
| 32 | 0.066 |
| 33 | 0.075 |
| 34 | 0.054 |
| 35 | 0.062 |
| 36 | 0.072 |
| 37 | 0.099 |
| 38 | 0.162 |
| 39 | 0.288 |

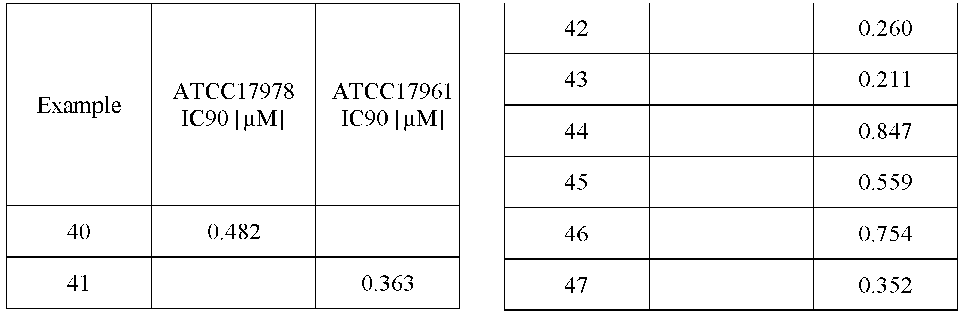

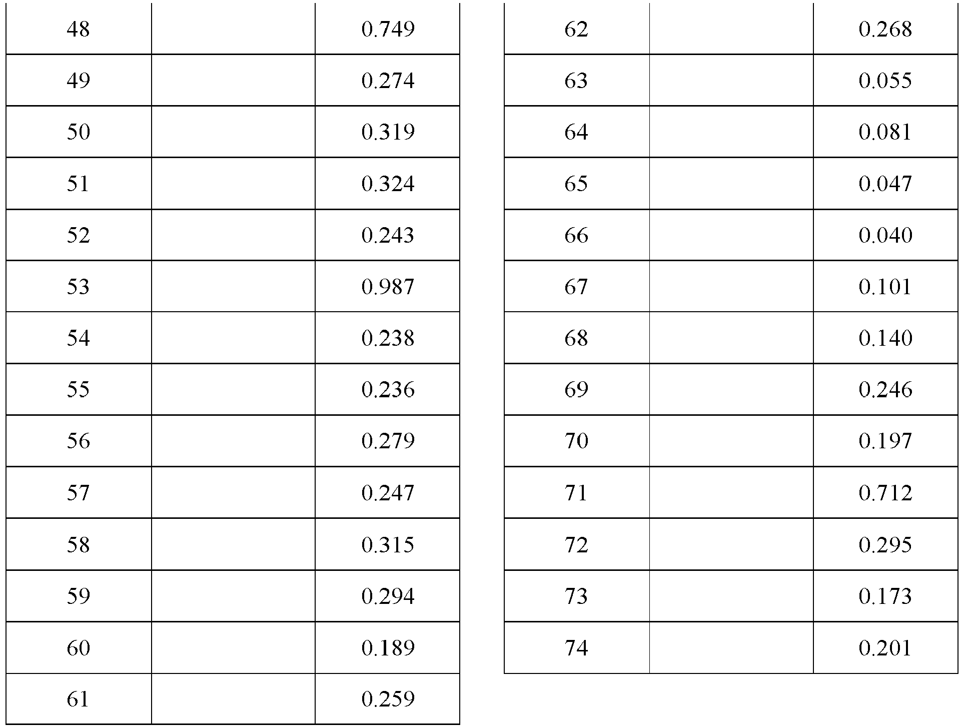

### Example 75

A compound of formula (I) can be used in a manner known per se as the active ingredient for the production of tablets of the following composition:
Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Example 76

A compound of formula (I) can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### Example 77

A compound of formula (I) can be used in a manner known per se as the active ingredient for the production of an infusion solution of the following composition:

| | |
|---|---|
| Active ingredient | 100 mg |
| Lactic acid 90% | 100 mg |

NaOH q.s. or HCl q.s. for adjustment to pH 4.0

Sodium chloride q.s. or glucose q.s. for adjustment of the osmolality to 290 mOsm/kg

| | |
|---|---|
| Water for injection (WFI) | ad 100 ml |

### Example 78

A compound of formula (I) can be used in a manner known per se as the active ingredient for the production of an infusion solution of the following composition:

| | |
|---|---|
| Active ingredient | 100 mg |
| Hydroxypropyl-beta-cyclodextrin | 10 g |

NaOH q.s. or HCl q.s. for adjustment to pH 7.4

Sodium chloride q.s. or glucose q.s. for adjustment of the osmolality to 290 mOsm/kg

| | |
|---|---|
| Water for injection (WFI) | ad 100 ml |

## Claims

1. A compound of formula (I) 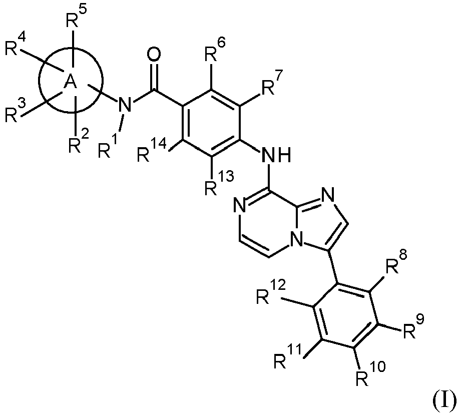 or a pharmaceutically acceptable salt thereof, wherein:
A is C₂-C₉-heterocycloalkyl, C₃-C₁₂-cycloalkyl, C₆-C₁₄-aryl or C₁-C₁₃-heteroaryl;
R¹ is hydrogen, C₁-C₆-alkyl, halo-C₁-C₆-alkyl, cyano-C₁-C₆-alkyl, hydroxy-C₁-C₆-alkyl, amino-C₁-C₆-alkyl, C₁-C₆-alkyl-NH-C₁-C₆-alkyl-, (C₁-C₆-alkyl)₂N-C₁-C₆-alkyl-, amino-C₁-C₆-alkyl-O-C₁-C₆-alkyl, C₁-C₆-alkyl-NH-C₁-C₆-alkyl-O-C₁-C₆-alkyl-, (C₁-C₆-alkyl)₂N-C₁-C₆-alkyl-O-C₁-C₆-alkyl- or C₃-C₁₂-cycloalkyl;
R² is hydrogen, hydroxy, carboxy, amino, C₁-C₆-alkyl-NH-, C₁-C₆-alkyl-C(O)-NH-, (C₁-C₆-alkyl)₂N-, amino-C₁-C₆-alkyl, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-, (C₁-C₆-alkyl)₂N-C₂-C₆-alkenyl-NH-, C₁-C₆-alkyl-NH-C₂-C₆-alkenyl-NH-, amino-C₂-C₆-alkenyl-NH-, C₁-C₆-alkylimidoyl-NH-C₁-C₆-alkyl; C₁-C₆-alkyl or a group 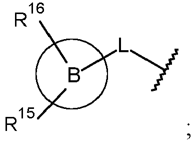
each of R³, R⁴ and R⁵ is independently hydrogen or hydroxy;
R⁶ is hydrogen, halogen, C₁-C₆-alkyl, halo-C₁-C₆-alkyl, hydroxy-C₁-C₆-alkyl, C₂-C₆-alkenyl, NO₂, CN, C₃-C₁₂-cycloalkyl, hydroxy-C₁-C₆-alkoxy-C₁-C₆-alkyl, C₁-C₆-alkoxy or alkoxy-C₁-C₆-alkyl;
R⁷ and R¹³ are each independently hydrogen, halogen or C₁-C₆-alkyl; each of R⁸, R⁹, R¹⁰, R¹¹ and R¹² is independently hydrogen, halogen, C₁-C₆-alkoxycarbonyl-C₁-C₆-alkoxy, amino, C₁-C₆-alkyl-NH-, (C₁-C₆-alkyl)₂N-, hydroxy, C₁-C₆-alkoxy, C₁-C₆-alkylsulfanyl, C₁-C₆-alkylsulfonyloxy, C₃-C₁₂-cycloalkyl-C₁-C₆-alkoxy, halo-C₁-C₆-alkoxy, C₆-C₁₄-aryl-C₁-C₆-alkoxy, C₁-C₁₃-heteroaryloxy, C₁-C₁₃-heteroaryl-C₁-C₆-alkoxy, cyano-C₁-C₆-alkoxy, C₃-C₁₂-cycloalkyloxy, C₂-C₆-alkynyloxy, C₁-C₆-alkoxy-C₂-C₆-alkynyloxy, cyano-C₃-C₁₂-cycloalkyloxy, cyano-C₃-C₁₂-cycloalkyl-C₁-C₆-alkoxy, amino-C₂-C₆-alkynyloxy, hydroxy-C₂-C₆-alkynyloxy, halo-C₁-C₆-alkyl, sulfamoyl, C₁-C₆-alkylsulfamoyl, C₁-C₆-alkyl, amino-C₁-C₆-alkoxy-C₂-C₆-alkynyloxy or amino-C₁-C₆-alkoxy;
R¹⁴ is hydrogen, halogen or C₁-C₆-alkyl;
R¹⁵ and R¹⁶ are each independently hydrogen, oxo or hydroxy;
B is C₂-C₉-heterocycloalkyl; and
L is a covalent bond, carbonyl, -NH-C(O)- or -C(O)-NH-.

2. The compound of formula (I) according to claim 1, or a pharmaceutically acceptable salt thereof, wherein:
A is C₂-C₉-heterocycloalkyl, C₃-C₁₂-cycloalkyl or C₆-C₁₄-aryl;
R¹ is hydrogen or C₁-C₆-alkyl;
R² is hydrogen, hydroxy, carboxy, amino, amino-C₁-C₆-alkyl, C₁-C₆-alkyl-C(O)-NH-, hydroxy-C₁-C₆-alkyl, oxo, halogen, guanidino-C₁-C₆-alkyl, guanidino-C₁-C₆-alkyl-CH(NH₂)-C(O)-, guanidino-C₁-C₆-alkyl-CH(NH₂)-C(O)-NH-(C₁-C₆-alkyl)₂N-C₂-C₆-alkenyl-NH-,C₁-C₆-alkylimidoyl-NH-C₁-C₆-alkyl; C₁-C₆-alkyl or a group 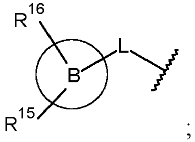 wherein:
R¹⁵ is oxo or hydroxy;
R¹⁶ is hydrogen or oxo;
B is C₂-C₉-heterocycloalkyl; and
L is a covalent bond, carbonyl or -C(O)-NH-; and
R³, R⁴, and R⁵ are all hydrogen.

3. The compound of formula (I) according to claim 1, or a pharmaceutically acceptable salt thereof, wherein:
A is C₂-C₉-heterocycloalkyl or C₃-C₁₂-cycloalkyl;
R¹ is hydrogen;
R² is amino, C₁-C₆-alkylimidoyl-NH-C₁-C₆-alkyl or a group 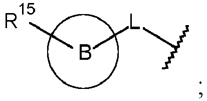 wherein:
R¹⁵ is hydroxy;
B is C₂-C₉-heterocycloalkyl; and
L is carbonyl or -C(O)-NH-; and
R³, R⁴, and R⁵ are all hydrogen.

4. The compound of formula (I) according to claim 1, or a pharmaceutically acceptable salt thereof, wherein:
A is cyclopropyl, cyclobutyl, cyclopentyl or 4-piperidyl;
R¹ is hydrogen;
R² is amino, (ethanimidoylamino)methyl or a group 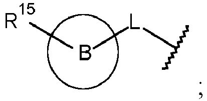 wherein:
R¹⁵ is hydroxy;
B is pyrrolidine; and
L is carbonyl or -C(O)-NH-; and
R³, R⁴, and R⁵ are all hydrogen.

5. The compound of formula (I) according to any one of claims 1 to 4, or a pharmaceutically acceptable salt thereof, wherein:
R⁶ is hydrogen, halogen or C₁-C₆-alkyl; and
R⁷, R¹³ and R¹⁴ are all hydrogen.

6. The compound of formula (I) according to any one of claims 1 to 4, or a pharmaceutically acceptable salt thereof, wherein:
R⁶ is C₁-C₆-alkyl; and
R⁷, R¹³ and R¹⁴ are all hydrogen.

7. The compound of formula (I) according to any one of claims 1 to 4, or a pharmaceutically acceptable salt thereof, wherein:
R⁶ is ethyl or methyl; and
R⁷, R¹³ and R¹⁴ are all hydrogen.

8. The compound of formula (I) according to any one of claims 1 to 7, or a pharmaceutically acceptable salt thereof, wherein:
R⁸ and R⁹ are both hydrogen or halogen;
R¹⁰ is C₁-C₆-alkoxy or halo-C₁-C₆-alkoxy; and
R¹¹ and R¹² are both hydrogen.

9. The compound of formula (I) according to any one of claims 1 to 7, or a pharmaceutically acceptable salt thereof, wherein:
R⁸ and R⁹ are both halogen;
R¹⁰ is C₁-C₆-alkoxy; and
R¹¹ and R¹² are both hydrogen.

10. The compound of formula (I) according to any one of claims 1 to 7, or a pharmaceutically acceptable salt thereof, wherein:
R⁸ and R⁹ are both fluoro;
R¹⁰ is methoxy; and
R¹¹ and R¹² are both hydrogen.

11. The compound of formula (I) according to claim 1, or a pharmaceutically acceptable salt thereof, wherein the compound of formula (I) is selected from:
N-[2-(aminomethyl)-1,3-dioxan-5-yl]-4-[[3-(4-methoxyphenyl)imidazo[1,2-a]pyrazin-8-yl]amino]-2-methyl-benzamide;
2-chloro-4-[[3-(3-fluoro-4-methoxy-phenyl)imidazo[1,2-a]pyrazin-8-yl]amino]-N-methyl-N-(4-piperidyl)benzamide;
4-[[3-(3-fluoro-4-methoxy-phenyl)imidazo[1,2-a]pyrazin-8-yl]amino]-N,2-dimethyl-N-(4-piperidyl)benzamide;
4-[[3-[4-(difluoromethoxy)-3-fluoro-phenyl]imidazo[1,2-a]pyrazin-8-yl]amino]-N,2-dimethyl-N-(4-piperidyl)benzamide;
4-[[3-(4-methoxyphenyl)imidazo[1,2-a]pyrazin-8-yl]amino]-2-methyl-N-(1-methyl-4-piperidyl)benzamide;
4-[[3-[4-(difluoromethoxy)phenyl]imidazo[1,2-a]pyrazin-8-yl]amino]-2-methyl-N-quinuclidin-3-yl-benzamide;
4-[[3-(4-methoxyphenyl)imidazo[1,2-a]pyrazin-8-yl]amino]-N,2-dimethyl-N-(1-methyl-4-piperidyl)benzamide;
4-[[3-(4-methoxyphenyl)imidazo[1,2-a]pyrazin-8-yl]amino]-N-methyl-N-(1-methyl-4-piperidyl)benzamide;
4-[[3-[4-(difluoromethoxy)phenyl]imidazo[1,2-a]pyrazin-8-yl]amino]-2-methyl-N-[(2S,3 S,4R, 5 S,6R)-2,4,5-trihydroxy-6-(hydroxymethyl)tetrahydropyran-3 - yl]benzamide;
N-[1-(1,1-dioxothian-4-yl)-4-piperidyl]-4-[[3-(3-fluoro-4-methoxy-phenyl)imidazo[1,2-a]pyrazin-8-yl]amino]-2-methyl-benzamide;
4-[[4-[[3-(3-fluoro-4-methoxy-phenyl)imidazo[1,2-a]pyrazin-8-yl]amino]-2-methyl-benzoyl]amino]benzoic acid;
4-[[3-(3-fluoro-4-methoxy-phenyl)imidazo[1,2-a]pyrazin-8-yl]amino]-N-[(3R,4R)-4-hydroxypyrrolidin-3-yl]-N,2-dimethyl-benzamide;
4-[[3-(3-fluoro-4-methoxy-phenyl)imidazo[1,2-a]pyrazin-8-yl]amino]-N-[(3R,4S)-3-fluoro-4-piperidyl]-2-methyl-benzamide;
4-[[3-(2,3-difluoro-4-methoxy-phenyl)imidazo[1,2-a]pyrazin-8-yl]amino]-2-ethyl-N-[3 -(hydroxymethyl)oxetan-3-yl]benzamide;
4-[[3-(2,3-difluoro-4-methoxy-phenyl)imidazo[1,2-a]pyrazin-8-yl]amino]-2-ethyl-N-[(3R)-pyrrolidin-3-yl]benzamide;
4-[[3-(2,3-difluoro-4-methoxy-phenyl)imidazo[1,2-a]pyrazin-8-yl]amino]-2-ethyl-N-[(3 S)-pyrrolidin-3-yl]benzamide;
N-[1-(aminomethyl)cyclopropyl]-4-[[3-[4-(difluoromethoxy)-2,3-difluoro-phenyl]imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-[(3 S)-2-oxopyrrolidin-3-yl]benzamide;
N-(azetidin-3-yl)-4-[[3-(2,3-difluoro-4-methoxy-phenyl)imidazo[1,2-a]pyrazin-8-yl]amino]-2-ethyl-benzamide;
N-[(1S,2S)-2-aminocyclopentyl]-4-[[3-(2,3-difluoro-4-methoxy-phenyl)imidazo[1,2-a]pyrazin-8-yl]amino]-2-ethyl-benzamide;
N-[1-(aminomethyl)cyclopropyl]-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-(4-piperidyl)benzamide;
N-(4-aminocyclohexyl)-4-[[3-(2,3-difluoro-4-methoxy-phenyl)imidazo[1,2-a]pyrazin-8-yl]amino]-2-ethyl-benzamide;
N-(3-aminocyclobutyl)-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-[rac-(1R,2S)-2-aminocyclohexyl]benzamide;
N-[(1S,2R)-2-aminocyclopentyl]-4-[[3-(2,3-difluoro-4-methoxy-phenyl)imidazo[1,2-a]pyrazin-8-yl]amino]-2-ethyl-benzamide;
N-[4-[[(2S)-2-amino-5-guanidino-pentanoyl]amino]cyclohexyl]-4-[[3-(2,3-difluoro-4-methoxy-phenyl)imidazo[1,2-a]pyrazin-8-yl]amino]-2-ethyl-benzamide;
N-(4-aminocyclohexyl)-4-[[3-(2,3-difluoro-4-methoxy-phenyl)imidazo[1,2-a]pyrazin-8-yl]amino]-2-methyl-benzamide;
4-[[3-(2,3-difluoro-4-methoxy-phenyl)imidazo[1,2-a]pyrazin-8-yl]amino]-N-[1-[(2S,4R)-4-hydroxypyrrolidine-2-carbonyl]-4-piperidyl]-2-methyl-benzamide;
4-[[3-(2,3-difluoro-4-methoxy-phenyl)imidazo[1,2-a]pyrazin-8-yl]amino]-N-[4-[[1-(dimethylamino)-2-methyl-prop-1-enyl]amino]cyclohexyl]-2-methyl-benzamide;
4-[[3-(2,3-difluoro-4-methoxy-phenyl)imidazo[1,2-a]pyrazin-8-yl]amino]-2-ethyl-N-[1-(guanidinomethyl)cyclopropyl]benzamide;
(2S,4R)-N-[4-[[4-[[3-(2,3-difluoro-4-methoxy-phenyl)imidazo[1,2-a]pyrazin-8-yl]amino]-2-methyl-benzoyl]amino]cyclohexyl]-4-hydroxy-pyrrolidine-2-carboxamide;
N-(3-aminocyclobutyl)-4-[[3-(2,3-difluoro-4-methoxy-phenyl)imidazo[1,2-a]pyrazin-8-yl]amino]-2-methyl-benzamide;
rac-(2S,4R)-N-[3-[[4-[[3-(2,3-difluoro-4-methoxy-phenyl)imidazo[1,2-a]pyrazin-8-yl] amino] -2-methyl-benzoyl] amino] cyclopentyl] -4 -hydroxy-pyrrolidine-2-carboxamide;
N-[3-[[(2S)-2-amino-5-guanidino-pentanoyl]amino]cyclobutyl]-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-N-[1-[(ethanimidoylamino)methyl]cyclopropyl]-2-ethyl-benzamide;
N-(2-aminocyclobutyl)-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-methyl-N-[(3R)-pyrrolidin-3-yl]benzamide;
4-[[3-(2,3-difluoro-4-methoxy-phenyl)imidazo[1,2-a]pyrazin-8-yl]amino]-2-ethyl-N-[rac-(1R,2S)-2-aminocyclopropyl]benzamide;
rac-(2S,4R)-N-[2-[[4-[[3-(2,3-difluoro-4-methoxy-phenyl)imidazo[1,2-a]pyrazin-8-yl]amino]-2-ethyl-benzoyl]amino]cyclopropyl]-4-hydroxy-pyrrolidine-2-carboxamide;
4-[[3-(2,3-difluoro-4-methoxy-phenyl)imidazo[1,2-a]pyrazin-8-yl]amino]-2-ethyl-N-[rac-(1S,5R)-3-azabicyclo[3.1.0]hexan-6-yl]benzamide;
N-[(1R,2S)-2-aminocyclopentyl]-4-[[3-(2,3-difluoro-4-methoxy-phenyl)imidazo[1,2-a]pyrazin-8-yl]amino]-2-ethyl-benzamide;
(2S,4R)-N-[3-[[4-[[3-(2,3-difluoro-4-methoxy-phenyl)imidazo[1,2-a]pyrazin-8-yl]amino]-2-ethyl-benzoyl]amino]-1-bicyclo[1.1.1]pentanyl]-4-hydroxy-pyrrolidine-2-carboxamide;
rac-(2S,4R)-N-[2-[[4-[[3-(2,3-difluoro-4-methoxy-phenyl)imidazo[1,2-a]pyrazin-8-yl] amino] -2-ethyl-benzoyl] amino] cyclobutyl] -4-hydroxy-pyrrolidine-2-carboxamide;
rac-(2S,4R)-N-[2-[[4-[[3-(2,3-difluoro-4-methoxy-phenyl)imidazo[1,2-a]pyrazin-8-yl]amino]-2-ethyl-benzoyl]amino]cyclopropyl]-4-hydroxy-pyrrolidine-2-carboxamide;
(2S,4R)-N-[3-[[4-[[3-(2,3-difluoro-4-methoxy-phenyl)imidazo[1,2-a]pyrazin-8-yl]amino]-2-methyl-benzoyl]amino]cyclobutyl]-4-hydroxy-pyrrolidine-2-carboxamide;
(2S,4R)-N-[3-[[4-[[3-(2,3-difluoro-4-methoxy-phenyl)imidazo[1,2-a]pyrazin-8-yl]amino]-2-methyl-benzoyl]amino]cyclobutyl]-4-hydroxy-pyrrolidine-2-carboxamide;
4-[[3-(2,3-difluoro-4-methoxy-phenyl)imidazo[1,2-a]pyrazin-8-yl]amino]-N-[1-[(2S,4R)-4-hydroxypyrrolidine-2-carbonyl]azetidin-3-yl]-2-methyl-benzamide;
4-[[3-(2,3-difluoro-4-methoxy-phenyl)imidazo[1,2-a]pyrazin-8-yl]amino]-N-[(3S)-1-[(2S,4R)-4-hydroxypyrrolidine-2-carbonyl]pyrrolidin-3-yl]-2-methyl-benzamide;
4-[[3-(2,3-difluoro-4-methoxy-phenyl)imidazo[1,2-a]pyrazin-8-yl]amino]-N-[(3R)-1-[(2S,4R)-4-hydroxypyrrolidine-2-carbonyl]pyrrolidin-3-yl]-2-methyl-benzamide;
(2S,4R)-N-[4-[[4-[[3-(2,3-difluoro-4-methoxy-phenyl)imidazo[1,2-a]pyrazin-8-yl]amino]-2-methyl-benzoyl]amino]cyclohexyl]-4-hydroxy-pyrrolidine-2-carboxamide;
N-(3-amino-1-bicyclo[1.1.1]pentanyl)-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-methyl-N-[4-(2-methylpropanoylamino)cyclohexyl]benzamide;
4-[[3-(2,3-difluoro-4-methoxy-phenyl)imidazo[1,2-a]pyrazin-8-yl]amino]-2-methyl-N-[1-[rac-(2S,4R)-4-hydroxypyrrolidine-2-carbonyl]-3-piperidyl]benzamide;
4-[[3-(2,3-difluoro-4-methoxy-phenyl)imidazo[1,2-a]pyrazin-8-yl]amino]-2-ethyl-N-[rac-(1R,2R)-2-aminocyclopropyl]benzamide;
N-(3-aminocyclobutyl)-4-[[3-(2,3-difluoro-4-methoxy-phenyl)imidazo[1,2-a]pyrazin-8-yl]amino]-2-methyl-benzamide;
N-(azetidin-3-yl)-4-[[3-(2,3-difluoro-4-methoxy-phenyl)imidazo[1,2-a]pyrazin-8-yl]amino]-2-methyl-benzamide;
4-[[3-(2,3-difluoro-4-methoxy-phenyl)imidazo[1,2-a]pyrazin-8-yl]amino]-2-methyl-N-[(3S)-pyrrolidin-3-yl]benzamide;
4-[[3-(2,3-difluoro-4-methoxy-phenyl)imidazo[l,2-a]pyrazin-8-yl]amino]-2-methyl-N-[rac-(1S,3 S)-3-aminocyclopentyl]benzamide;
(2S,4R)-N-[(1R,2S)-2-[[4-[[3-(2,3-difluoro-4-methoxy-phenyl)imidazo[1,2-a]pyrazin-8-yl]amino]-2-ethyl-benzoyl]amino]cyclopentyl]-4-hydroxy-pyrrolidine-2-carboxamide;
N-(4-aminocyclohexyl)-4-[[3-(2,3-difluoro-4-methoxy-phenyl)imidazo[1,2-a]pyrazin-8-yl]amino]-2-methyl-benzamide;
4-[[3-(2,3-difluoro-4-methoxy-phenyl)imidazo[1,2-a]pyrazin-8-yl]amino]-2-methyl-N-(3 -piperidyl)benzamide;
4-[[3-(2,3-difluoro-4-methoxy-phenyl)imidazo[1,2-a]pyrazin-8-yl]amino]-2-methyl-N-(4-piperidyl)benzamide;
(2S,4R)-N-[2-[[4-[[3-(2,3-difluoro-4-methoxy-phenyl)imidazo[1,2-a]pyrazin-8-yl]amino]-2-ethyl-benzoyl]amino]cyclohexyl]-4-hydroxy-pyrrolidine-2-carboxamide;
4-[[3-(2,3-difluoro-4-methoxy-phenyl)imidazo[1,2-a]pyrazin-8-yl]amino]-2-ethyl-N-[1-[rac-(2S)-2-amino-5-guanidino-pentanoyl]-3-piperidyl]benzamide;
N-[(3R)-1-[(2S)-2-amino-5-guanidino-pentanoyl]pyrrolidin-3-yl]-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-[3-[[rac-(2S)-2-amino-5-guanidino-pentanoyl]amino]cyclopentyl]benzamide;
N-[4-[[(2S)-2-amino-5-guanidino-pentanoyl]amino]cyclohexyl]-4-[[3-(2,3-difluoro-4-methoxy-phenyl)imidazo[1,2-a]pyrazin-8-yl]amino]-2-ethyl-benzamide;
N-[1-[(2S)-2-amino-5-guanidino-pentanoyl]-4-piperidyl]-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-[rac-(1S,3 S)-3-aminocyclopentyl]benzamide;
N-(4-aminocyclohexyl)-4-[[3-(2,3-difluoro-4-methoxy-phenyl)imidazo[1,2-a]pyrazin-8-yl]amino]-2-ethyl-benzamide;
4-[[3-(2,3-difluoro-4-methoxy-phenyl)imidazo[1,2-a]pyrazin-8-yl]amino]-2-ethyl-N-(3-piperidyl)benzamide; and
N-[3-(aminomethyl)oxetan-3-yl]-4-[[3-(2,3-difluoro-4-methoxy-phenyl)imidazo[1,2-a]pyrazin-8-yl]amino]-2-ethyl-benzamide.

12. A process of manufacturing the compounds of formula (I) according to any one of claims 1 to 11, comprising:
(i) reacting a carboxylic acid **IVa,** wherein R⁶ to R¹⁴ are as defined in any one of claims 1 to 11, 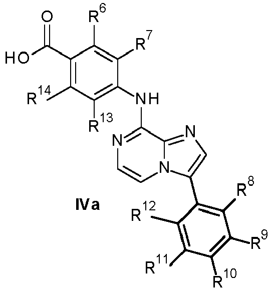 with an amine **V,** wherein A, L and R¹ to R⁵ are as defined in any one of claims 1 to 11, 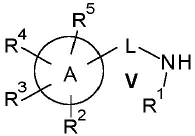 in the presence of a coupling reagent (such as HATU, TBTU, and the like) and a base (such as DIPEA, NEt₃, and the like), to form said compound of formula (I); or
(ii) reacting a compound **VI,** wherein R¹ to R⁷, R¹³, R¹⁴, L and A are as defined in any one of claims 1 to 11 and X is halogen, 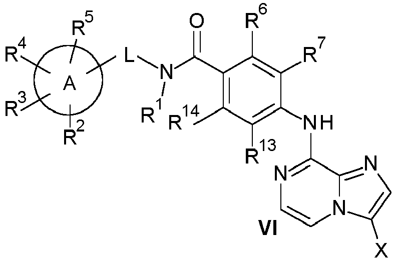 with a boronic acid **VII**, wherein R⁸ to R¹² are as defined in any one of claims 1 to 11 and Y is a boronic acid or a boronic acid ester, 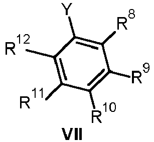 in the presence of a transition metal catalyst (such as PdCl₂(dppf)-CH₂Cl₂ adduct, Pd(PPh₃)₄, and the like) and a base (such as K₃PO₄, NaOtBu, and the like)
to form said compound of formula (I).

13. A compound of formula (I) according to any one of claims 1 to 11, or a pharmaceutically acceptable salt thereof, for use as therapeutically active substance.

14. A pharmaceutical composition comprising a compound of formula (I) according to any one of claims 1 to 11, or a pharmaceutically acceptable salt thereof, and a therapeutically inert carrier.

15. A compound of formula (I) according to any of claims 1 to 11, or a pharmaceutically acceptable salt thereof, for use as antibiotic.

## Patentansprüche

1. Verbindung der Formel (I) 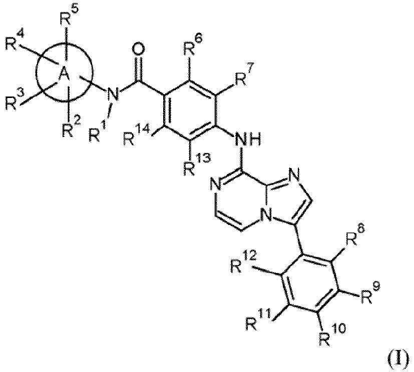 oder ein pharmazeutisch unbedenkliches Salz davon, wobei:
A C₂-C₉-Heterocycloalkyl, C₃-C₁₂-Cycloalkyl, C₆-C₁₄-Aryl oder C₁-C₁₃-Heteroaryl ist;
R¹ Wasserstoff, C₁-C₆-Alkyl, Halogen-C₁-C₆-alkyl, Cyano-C₁-C₆-alkyl, Hydroxy-C₁-C₆-alkyl, Amino-C₁-C₆-alkyl, C₁-C₆-Alkyl-NH-C₁-C₆-alkyl-, (C₁-C₆-Alkyl)₂N-C₁-C₆-alkyl-, Amino-C₁-C₆-alkyl-O-C₁-C₆-alkyl, C₁-C₆-Alkyl-NH-C₁-C₆-alkyl-O-C₁-C₆-alkyl-, (C₁-C₆-Alkyl)₂N-C₁-C₆-alkyl-O-C₁-C₆-alkyl-oder C₃-C₁₂-Cycloalkyl ist;
R² Wasserstoff, Hydroxy, Carboxy, Amino, C₁-C₆-Alkyl-NH-, C₁-C₆-Alkyl-C(O)-NH-, (C₁-C₆-Alkyl)₂N-, Amino-C₁-C₆-alkyl, Hydroxy-C₁-C₆-alkyl, Oxo, Halogen, Guanidino-C₁-C₆-alkyl, Guanidino-C₁-C₆-alkyl-CH(NH₂)-C(O)-, Guanidino-C₁-C₆-alkyl-CH(NH₂)-C(O)-NH-, (C₁-C₆-Alkyl)₂N-C₂-C₆-alkenyl-NH-, C₁-C₆-Alkyl-NH-C₂-C₆-alkenyl-NH-, Amino-C₂-C₆-alkenyl-NH-, C₁-C₆-Akylimidoyl-NH-C₁-C₆-alkyl; C₁-C₆-Alkyl oder eine Gruppe 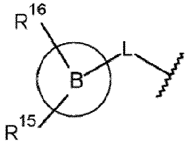 ist; jeder von R³, R⁴ und R⁵ unabhängig voneinander Wasserstoff oder Hydroxy ist;
R⁶ Wasserstoff, Halogen, C₁-C₆-Alkyl, Halogen-C₁-C₆-alkyl, Hydroxy-C₁-C₆-alkyl, C₂-C₆-Alkenyl, NO₂, CN, C₃-C₁₂-Cycloalkyl, Hydroxy-C₁-C₆-alkoxy-C₁-C₆-alkyl, C₁-C₆-Alkoxy oder Alkoxy-C₁-C₆-alkyl ist;
R⁷ und R¹³ jeweils unabhängig voneinander Wasserstoff, Halogen oder C₁-C₆-Alkyl sind;
jeder von R⁸, R⁹, R¹⁰, R¹¹ und R¹² unabhängig voneinander Wasserstoff, Halogen, C₁-C₆-Alkoxycarbonyl-C₁-C₆-alkoxy, Amino, C₁-C₆-Alkyl-NH-, (C₁-C₆-Alkyl)₂N-, Hydroxy, C₁-C₆-Alkoxy, C₁-C₆-Alkylsulfanyl, C₁-C₆-Alkylsulfonyloxy, C₃-C₁₂-Cycloalkyl-C₁-C₆-alkoxy, Halogen-C₁-C₆-alkoxy, C₆-C₄-Aryl-C₁-C₆-alkoxy, C₁-C₁₃-Heteroaryloxy, C₁-C₁₃-Heteroaryl-C₁-C₆-alkoxy, Cyano-C₁-C₆-alkoxy, C₃-C₁₂-Cycloalkyloxy, C₂-C₆-Alkinyloxy, C₁-C₆-Alkoxy-C₂-C₆-alkinyloxy, Cyano-C₃-C₁₂-cycloalkyloxy, Cyano-C₃-C₁₂-cycloalkyl-C₁-C₆-alkoxy, Amino-C₂-C₆-alkinyloxy, Hydroxy-C₂-C₆-alkinyloxy, Halogen-C₁-C₆-alkyl, Sulfamoyl, C₁-C₆-Alkylsulfamoyl, C₁-C₆-Alkyl, Amino-C₁-C₆-alkoxy-C₂-C₆-alkinyloxy oder Amino-C₁-C₆-alkoxy ist;
R¹⁴ Wasserstoff, Halogen oder C₁-C₆-Alkyl ist;
R¹⁵ und R¹⁶ jeweils unabhängig voneinander Wasserstoff, Oxo oder Hydroxy sind;
B C₂-C₉-Heterocycloalkyl ist; und
L eine kovalente Bindung, Carbonyl, -NH-C(O)- oder -C(O)-NH- ist.

2. Verbindung der Formel (I) nach Anspruch 1 oder ein pharmazeutisch unbedenkliches Salz davon, wobei:
A C₂-C₉-Heterocycloalkyl, C₃-C₁₂-Cycloalkyl oder C₆-C₁₄-Aryl ist;
R¹ Wasserstoff oder C₁-C₆-Alkyl ist;
R² Wasserstoff, Hydroxy, Carboxy, Amino, Amino-C₁-C₆-alkyl, C₁-C₆-Alkyl-C(O)-NH-, Hydroxy-C₁-C₆-alkyl, Oxo, Halogen, Guanidino-C₁-C₆-alkyl, Guanidino-C₁-C₆-alkyl-CH(NH₂)-C(O)-, Guanidino-C₁-C₆-alkyl-CH(NH₂)-C(O)-NH-, (C₁-C₆-Alkyl)₂N-C₂-C₆-alkenyl-NH-, C₁-C₆-Alkylimidoyl-NH-C₁-C₆-alkyl; C₁-C₆-Alkyl oder eine Gruppe 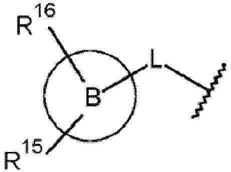 ist; wobei:
R¹⁵ Oxo oder Hydroxy ist;
R¹⁶ Wasserstoff oder Oxo ist;
B C₂-C₉-Heterocycloalkyl ist; und
L eine kovalente Bindung, Carbonyl oder -C(O)-NH- ist; und
R³, R⁴ und R⁵ alle Wasserstoff sind.

3. Verbindung der Formel (I) nach Anspruch 1 oder ein pharmazeutisch unbedenkliches Salz davon, wobei:
A C₂-C₉-Heterocycloalkyl oder C₃-C₁₂-Cycloalkyl ist;
R¹ Wasserstoff ist;
R² Amino, C₁-C₆-Alkylimidoyl-NH-C₁-C₆-alkyl oder eine Gruppe 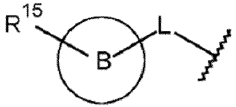 ist; wobei:
R¹⁵ Hydroxy ist;
B C₂-C₉-Heterocycloalkyl ist; und
L Carbonyl oder -C(O)-NH- ist; und
R³, R⁴ und R⁵ alle Wasserstoff sind.

4. Verbindung der Formel (I) nach Anspruch 1 oder ein pharmazeutisch unbedenkliches Salz davon, wobei:
A Cyclopropyl, Cyclobutyl, Cyclopentyl oder 4-Piperidyl ist;
R¹ Wasserstoff ist;
R² Amino, (Ethanimidoylamino)methyl oder eine Gruppe 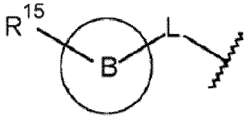 ist; wobei:
R¹⁵ Hydroxy ist;
B Pyrrolidin ist; und
L Carbonyl oder -C(O)-NH- ist; und
R³, R⁴ und R⁵ alle Wasserstoff sind.

5. Verbindung der Formel (I) nach einem der Ansprüche 1 bis 4 oder ein pharmazeutisch unbedenkliches Salz davon, wobei:
R⁶ Wasserstoff, Halogen oder C₁-C₆-Alkyl ist; und
R⁷, R¹³ und R¹⁴ alle Wasserstoff sind.

6. Verbindung der Formel (I) nach einem der Ansprüche 1 bis 4 oder ein pharmazeutisch unbedenkliches Salz davon, wobei:
R⁶ C₁-C₆-Alkyl ist; und
R⁷, R¹³ und R¹⁴ alle Wasserstoff sind.

7. Verbindung der Formel (I) nach einem der Ansprüche 1 bis 4 oder ein pharmazeutisch unbedenkliches Salz davon, wobei:
R⁶ Ethyl oder Methyl ist; und
R⁷, R¹³ und R¹⁴ alle Wasserstoff sind.

8. Verbindung der Formel (I) nach einem der Ansprüche 1 bis 7 oder ein pharmazeutisch unbedenkliches Salz davon, wobei:
R⁸ und R⁹ beide Wasserstoff oder Halogen sind;
R¹⁰ C₁-C₆-Alkoxy oder Halogen-C₁-C₆-alkoxy ist; und
R¹¹ und R¹² beide Wasserstoff sind.

9. Verbindung der Formel (I) nach einem der Ansprüche 1 bis 7 oder ein pharmazeutisch unbedenkliches Salz davon, wobei:
R⁸ und R⁹ beide Halogen sind;
R¹⁰ C₁-C₆-Alkoxy ist; und
R¹¹ und R¹² beide Wasserstoff sind.

10. Verbindung der Formel (I) nach einem der Ansprüche 1 bis 7 oder ein pharmazeutisch unbedenkliches Salz davon, wobei:
R⁸ und R⁹ beide Fluor sind;
R¹⁰ Methoxy ist; und
R¹¹ und R¹² beide Wasserstoff sind.

11. Verbindung der Formel (I) nach Anspruch 1 oder ein pharmazeutisch unbedenkliches Salz davon, wobei die Verbindung der Formel (I) ausgewählt ist aus:
N-[2-(Aminomethyl)-1,3-dioxan-5-yl]-4-[[3-(4-methoxyphenyl)imidazo[1,2-a]pyrazin-8-yl] amino] -2-methylbenzamid;
2-Chlor-4-[[3-(3-fluor-4-methoxyphenyl)imidazo[1,2-a]pyrazin-8-yl]amino]-N-methyl-N-(4-piperidyl)benzamid;
4-[[3-(3-Fluor-4-methoxyphenyl)imidazo[1,2-a]pyrazin-8-yl]amino]-N,2-dimethyl-N-(4-piperidyl)benzamid;
4-[[3-[4-(Difluormethoxy)-3-fluorphenyl]imidazo[1,2-a]pyrazin-8-yl]amino]-N,2-dimethyl-N-(4-piperidyl)benzamid;
4-[[3-(4-Methoxyphenyl)imidazo[1,2-a]pyrazin-8-yl]amino]-2-metbyl-N-(1-metbyl-4-piperidyl)benzamid;
4-[[3-[4-(Difluormethoxy)phenyl]imidazo[1,2-a]pyrazin-8-yl]amino]-2-methyl-N-chinuclidin-3-ylbenzamid;
4-[[3-(4-Methoxyphenyl)imidazo[1,2-a]pyrazin-8-yl]amino]-N,2-dimethyl-N-(1-methyl-4-piperidyl)benzamid;
4-[[3-(4-Methoxyphenyl)imidazo[l,2-a]pyrazin-8-yl]amino]-N-methyl-N-(l-methyl-4-piperidyl)benzamid;
4-[[3-[4-(Difluormethoxy)phenyl]imidazo[l,2-a]pyrazin-8-yl]amino]-2-methyl-N-[(2S,3S,4R,5S,6R)-2,4,5-trihydroxy-6-(hydroxymethyl)tetrahydropyran-3-yl]benzamid;
N-[1-(1,1-Dioxothian-4-yl)-4-piperidyl]-4-[[3-(3-fluor-4-methoxyphenyl)imidazo[1,2-a]pyrazin-8-yl]amino]-2-methylbenzamid;
4-[[4-[[3-(3-Fluor-4-methoxyphenyl)imidazo[1,2-alpyrazin-8-yl]amino]-2-methylbenzoyl]amino]benzoesäure;
4-[[3-(3-Fluor-4-methoxyphenyl)imidazo[1,2-a]pyrazin-8-yl]amino]-N-[(3R,4R)-4-hydroxypyrrolidin-3-yl]-N,2-dimethylbenzamid;
4-[[3-(3-Fluor-4-methoxyphenyl)imidazo[1,2-a]pyrazin-8-yl]amino]-N-[(3R,4S)-3-fluor-4-piperidyl]-2-methylbenzamid;
4-[[3-(2,3-Difluor-4-methoxyphenyl)imidazo[1,2-a]pyrazin-8-yl]amino]-2-ethyl-N-[3-(hydroxymethyl)oxetan-3-yl]benzamid;
4-[[3-(2,3-Difluor-4-methoxyphenyl)imidazo[1,2-a]pyrazin-8-yl]amino]-2-ethyl-N-[(3R)-pyrrolidin-3-yl]benzamid;
4-[[3-(2,3-Difluor-4-methoxyphenyl)imidazo[1,2-a]pyrazin-8-yl]amino]-2-ethyl-N-[(3S)-pyrrolidin-3-yl]benzamid;
N-[1-(Aminomethyl)cyclopropyl]-4-[[3-[4-(difluormethoxy)-2,3-difluorphenyl]imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-[(3S)-2-oxopyrrolidin-3-yl]benzamid;
N-(Azetidin-3-yl)-4-[[3-(2,3-difluor-4-methoxyphenyl)imidazo[1,2-a]pyrazin-8-yl]amino]-2-ethylbenzamid;
N-[(1S,2S)-2-Aminocyclopentyl]-4-[[3-(2,3-difluor-4-methoxyphenyl)imidazo[1,2-a]pyrazin-8-yl]amino]-2-ethylbenzamid;
N-[1-(Aminomethyl)cyclopropyl]-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-(4-piperidyl)benzamid;
N-(4-Aminocyclohexyl)-4-[[3-(2,3-difluor-4-methoxyphenyl)imidazo[1,2-a]pyrazin-8-yl] amino] -2-ethylbenzamid;
N-(3-Aminocyclobutyl)-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-[rac-(1R,2S)-2-aminocyclohexyl]benzamid;
N-[(1S,2R)-2-Aminocyclopentyl]-4-[[3-(2,3-difluor-4-methoxyphenyl)imidazo[1,2-a]pyrazin-8-yl]amino]-2-ethylbenzamid;
N-[4-[[(2S)-2-Amino-5-guanidinopentanoyl]amino]cyclohexyl]-4-[[3-(2,3-difluor-4-methoxyphenyl)imidazo[1,2-a]pyrazin-8-yl]amino]-2-ethylbenzamid;
N-(4-Aminocyclohexyl)-4-[[3-(2,3-difluor-4-methoxyphenyl)imidazo[1,2-a]pyrazin-8-yl]amino]-2-methylbenzamid;
4-[[3-(2,3-Difluor-4-methoxyphenyl)imidazo[1,2-a]pyrazin-8-yl]amino]-N-[1-[(2S,4R)-4-hydroxypyrrolidin-2-carbonyl]-4-piperidyl]-2-methylbenzamid;
4-[[3-(2,3-Difluor-4-methoxyphenyl)imidazo[1,2-a]pyrazin-8-yl]amino]-N-[4-[[1-(dimethylamino)-2-methylprop-1-enyl]amino]cyclohexyl]-2-methylbenzamid;
4-[[3-(2,3-Difluor-4-methoxyphenyl)imidazo[1,2-a]pyrazin-8-yl]amino]-2-ethyl-N-[1-(guanidinomethyl)cyclopropyl]benzamid;
(2S,4R)-N-[4-[[4-[[3-(2,3-Difluor-4-methoxyphenyl)imidazo[1,2-alpyrazin-8-yl]amino]-2-methylbenzoyl]amino]cyclohexyl]-4-hydroxypyrrolidin-2-carboxamid;
N-(3-Aminocyclobutyl)-4-[[3-(2,3-difluor-4-methoxyphenyl)imidazo[1,2-a]pyrazin-8-yl]amino]-2-methylbenzamid;
rac-(2S,4R)-N-[3-[[4-[[3-(2,3-Difluor-4-methoxyphenyl)imidazo[1,2-a]pyrazin-8-yl]amino]-2-methylbenzoyl]amino]cyclopentyl]-4-hydroxypyrrolidin-2-carboxamid;
N-[3-[[(2S)-2-Amino-5-guanidinopentanoyl]amino]cyclobutyl]-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-N-[1-[(ethanimidoylamino)methyl]cyclopropyl]-2-ethylbenzamid;
N-(2-Aminocyclobutyl)-4-[[3-(2,3-difluor-4-methoxyphenyl)imidazo[1,2-a]pyrazin-8-yl] amino] -2-ethylbenzamid;
4-[[3-(2,3-Difluor-4-methoxyphenyl)imidazo[1,2-a]pyrazin-8-yl]amino]-2-methyl-N-[(3R)-pyrrolidin-3-yl]benzamid;
4-[[3-(2,3-Difluor-4-methoxyphenyl)imidazo[1,2-a]pyrazin-8-yl]amino]-2-ethyl-N-[rac-(1R,2S)-2-aminocyclopropyl]benzamid;
rac-(2S,4R)-N-[2-[[4-[[3-(2,3-Difluor-4-methoxyphenyl)imidazo[1,2-a]pyrazin-8-yl]amino]-2-ethylbenzoyl]amino]cyclopropyl]-4-hydroxypyrrolidin-2-carboxamid;
4-[[3-(2,3-Difluor-4-methoxyphenyl)imidazo[1,2-a]pyrazin-8-yl]amino]-2-ethyl-N-[rac-(1S,5R)-3-azabicyclo[3.1.0]hexan-6-yl]benzamid;
N-[(1R,2S)-2-Aminocyclopentyl]-4-[[3-(2,3-difluor-4-methoxyphenyl)imidazo[1,2-a]pyrazin-8-yl]amino]-2-ethylbenzamid;
(2S,4R)-N-[3-[[4-[[3-(2,3-Difluor-4-methoxyphenyl)imidazo[1,2-a]pyrazin-8-yl]amino]-2-ethylbenzoyl]amino]-1-bicyclo[1.1.1]pentanyl]-4-hydroxypyrrolidin-2-carboxamid;
rac-(2S,4R)-N-[2-[[4-[[3-(2,3-Difluor-4-methoxyphenyl)imidazo[1,2-a]pyrazin-8-yl]amino]-2-ethylbenzoyl]amino]cyclobutyl]-4-hydroxypyrrolidin-2-carboxamid;
rac-(2S,4R)-N-[2-[[4-[[3-(2,3-Difluor-4-methoxyphenyl)imidazo[1,2-a]pyrazin-8-yl]amino]-2-ethylbenzoyl]amino]cyclopropyl]-4-hydroxypyrrolidin-2-carboxamid;
(2S,4R)-N-[3-[[4-[[3-(2,3-Difluor-4-methoxyphenyl)imidazo[1,2-a]pyrazin-8-yl]amino]-2-methylbenzoyl]amino]cyclobutyl]-4-hydroxypyrrolidin-2-carboxamid;
(2S,4R)-N-[3-[[4-[[3-(2,3-Difluor-4-methoxyphenyl)imidazo[1,2-a]pyrazin-8-yl]amino]-2-methylbenzoyl]amino]cyclobutyll-4-hydroxypyrrolidin-2-carboxamid;
4-[[3-(2,3-Difluor-4-methoxyphenyl)imidazo[1,2-a]pyrazin-8-yl]amino]-N-[1-[(2S,4R)-4-hydroxypyrrolidin-2-carbonyl]azetidin-3-yl]-2-methylbenzamid;
4-[[3-(2,3-Difluor-4-methoxyphenyl)imidazo[1,2-a]pyrazin-8-yl]amino]-N-[(3S)-1-[(2S,4R)-4-hydroxypyrrolidin-2-carbonyl]pyrrolidin-3-yl]-2-methylbenzamid;
4-[[3-(2,3-Difluor-4-methoxyphenyl)imidazo[1,2-a]pyrazin-8-yl]amino]-N-[(3R)-1-[(2S,4R)-4-hydroxypyrrolidin-2-carbonyl]pyrrolidin-3-yl]-2-methylbenzamid;
(2S,4R)-N-[4-[[4-[[3-(2,3-Difluor-4-methoxyphenyl)imidazo[1,2-a]pyrazin-8-yl]amino]-2-methylbenzoyl]amino]cyclohexyl]-4-hydroxypyrrolidin-2-carboxamid;
N-(3-Amino-1-bicyclo[1.1.1]pentanyl)-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-methyl-N-[4-(2-methylpropanoylamino)cyclohexyl]benzamid;
4-[[3-(2,3-Difluor-4-methoxyphenyl)imidazo[1,2-a]pyrazin-8-yl]amino]-2-methyl-N-[1-[rac-(2S,4R)-4-hydroxypyrrolidin-2-carbonyl]-3-piperidyl]benzamid;
4-[[3-(2,3-Difluor-4-methoxyphenyl)imidazo[1,2-a]pyrazin-8-yl]amino]-2-ethyl-N-[rac-(1R,2R)-2-aminocyclopropyl]benzamid;
N-(3-Aminocyclobutyl)-4-[[3-(2,3-difluor-4-methoxyphenyl)imidazo[1,2-a]pyrazin-8-yl]amino]-2-methylbenzamid;
N-(Azetidin-3-yl)-4-[[3-(2,3-difluor-4-methoxyphenyl)imidazo[1,2-a]pyrazin-8-yl]amino]-2-methylbenzamid;
4-[[3-(2,3-Difluor-4-methoxyphenyl)imidazo[l,2-a]pyrazin-8-yl]amino]-2-methyl-N-[(3S)-pyrrolidin-3-yl]benzamid;
4-[[3-(2,3-Difluor-4-methoxyphenyl)imidazo[1,2-a]pyrazin-8-yl]amino]-2-methyl-N-[rac-(1S,3S)-3-aminocyclopentyl]benzamid;
(2S,4R)-N-[(1R,2S)-2-[[4-[[3-(2,3-Difluor-4-methoxyphenyl)imidazo[1,2-a]pyrazin-8-yl]amino]-2-ethylbenzoyl]amino]cyclopentyl]-4-hydroxypyrrolidin-2-carboxamid;
N-(4-Aminocyclohexyl)-4-[[3-(2,3-difluor-4-methoxyphenyl)imidazo[1,2-a]pyrazin-8-yl]amino]-2-methylbenzamid;
4-[[3-(2,3-Difluor-4-methoxyphenyl)imidazo[1,2-a]pyrazin-8-yl]amino]-2-methyl-N-(3-piperidyl)benzamid;
4-[[3-(2,3-Difluor-4-methoxyphenyl)imidazo[1,2-a]pyrazin-8-yl]amino]-2-methyl-N-(4-piperidyl)benzamid;
(2S,4R)-N-[2-[[4-[[3-(2,3-Difluor-4-methoxyphenyl)imidazo[1,2-a]pyrazin-8-yl]amino]-2-ethylbenzoyl]amino]cyclohexyl]-4-hydroxypyrrolidin-2-carboxamid;
4-[[3-(2,3-Difluor-4-methoxyphenyl)imidazo[1,2-a]pyrazin-8-yl]amino]-2-ethyl-N-[1-[rac-(2S)-2-amino-5-guanidinopentanoyl]-3-piperidyl]benzamid;
N-[(3R)-1-[(2S)-2-Amino-5-guanidinopentanoyl]pyrrolidin-3-yl]-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-[3-[[rac-(2S)-2-amino-5-guanidinopentanoyl]amino]cyclopentyl]benzamid;
N-[4-[[(2S)-2-Amino-5-guanidinopentanoyl]amino]cyclohexyl]-4-[[3-(2,3-difluor-4-methoxyphenyl)imidazo[1,2-a]pyrazin-8-yl]amino]-2-ethylbenzamid;
N-[1-[(2S)-2-Amino-5-guanidinopentanoyl]-4-piperidyl]-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-[rac-(1S,3 S)-3 -aminocyclopentyl]benzamid;
N-(4-Aminocyclohexyl)-4-[[3-(2,3-difluor-4-methoxyphenyl)imidazo[1,2-a]pyrazin-8-yl]amino]-2-ethylbenzamid;
4-[[3-(2,3-Difluor-4-methoxyphenyl)imidazo[1,2-a]pyrazin-8-yl]amino]-2-ethyl-N-(3-piperidyl)benzamid; und
N-[3-(Aminomethyl)oxetan-3-yl]-4-[[3-(2,3-difluor-4-methoxyphenyl)imidazo[1,2-alpyrazin-8-yl]amino]-2-ethylbenzamid.

12. Verfahren zur Herstellung der Verbindungen der Formel (I) nach einem der Ansprüche 1 bis 11, umfassend:
(i) Reagieren einer Carbonsäure **IVa,** wobei R⁶ bis R¹⁴ wie in einem der Ansprüche 1 bis 11 definiert sind, 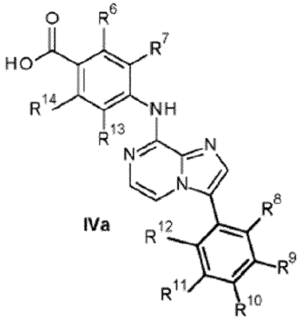 mit einem Amin V, wobei A, L und R¹ bis R⁵ wie in einem der Ansprüche 1 bis 11 definiert sind, 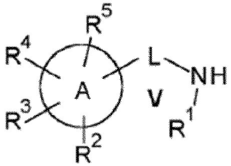 in der Gegenwart eines Kopplungsreagens (wie HATU, TBTU und dergleichen) und einer Base (wie DIPEA, NEt₃ und dergleichen) unter Bildung der Verbindung der Formel (I); oder
(ii) Reagieren einer Verbindung **VI,** wobei R¹ bis R⁷, R¹³, R¹⁴, L und A wie in einem der Ansprüche 1 bis 11 definiert sind und X Halogen ist, 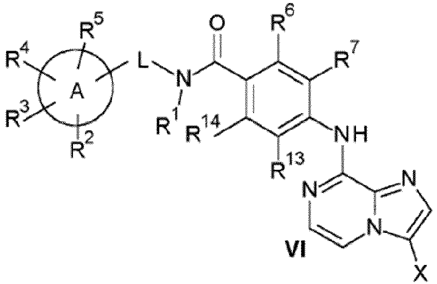 mit einer Boronsäure **VII,** wobei R⁸ bis R¹² wie in einem der Ansprüche 1 bis 11 definiert sind und Y eine Boronsäure oder ein Boronsäureester ist, 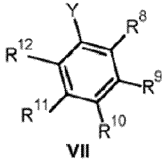 in der Gegenwart eines Übergangsmetallkatalysators (wie PdCl₂(dppf)-CH₂Cl₂-Addukt, Pd(PPh₃)₄ und dergleichen) und einer Base (wie K₃PO₄, NaOtBu und dergleichen)
unter Bildung der Verbindung der Formel (I).

13. Verbindung der Formel (I) nach einem der Ansprüche 1 bis 11 oder ein pharmazeutisch unbedenkliches Salz davon zur Verwendung als eine therapeutisch wirksame Substanz.

14. Pharmazeutische Zusammensetzung, umfassend eine Verbindung der Formel (I) nach einem der Ansprüche 1 bis 11 oder ein pharmazeutisch unbedenkliches Salz davon und einen therapeutisch inerten Träger.

15. Verbindung der Formel (I) nach einem der Ansprüche 1 bis 11 oder ein pharmazeutisch unbedenkliches Salz davon zur Verwendung als Antibiotikum.

## Revendications

1. Composé de formule (I) 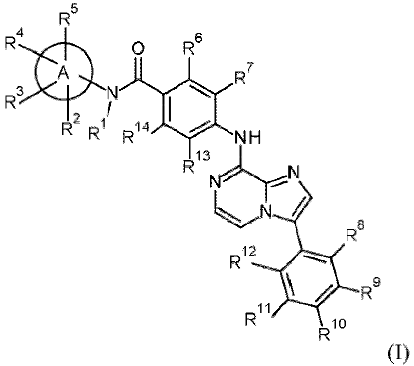 ou un sel pharmaceutiquement acceptable de celui-ci, dans laquelle :
A représente un hétérocycloalkyle en C₂-C₉, un cycloalkyle en C₃-C₁₂, un aryle en C₆-C₁₄ ou un hétéroaryle en C₁-C₁₃ ;
R¹ représente un hydrogène, un alkyle en C₁-C₆, un halogénoalkyle en C₁-C₆, un cyanoalkyle en C₁-C₆, un hydroxyalkyle en C₁-C₆, un aminoalkyle en C₁-C₆, un alkyle en C₁-C₆-NH-alkyle en C₁-C₆-, un (alkyle en C₁-C₆)₂N-alkyle en C₁-C₆-, un aminoalkyle en C₁-C₆-O-alkyle en C₁-C₆, un alkyle en C₁-C₆-NH-alkyle en C₁-C₆-O-alkyle en C₁-C₆-, un (alkyle en C₁-C₆)₂N-alkyle en C₁-C₆-O-alkyle en C₁-C₆- ou un cycloalkyle en C₃-C₁₂ ;
R² représente un hydrogène, un hydroxy, un carboxy, un amino, un alkyle en C₁-C₆-NH-, un alkyle en C₁-C₆-C(O)-NH-, un (alkyle en C₁-C₆)₂N-, un aminoalkyle en C₁-C₆, un hydroxyalkyle en C₁-C₆, un oxo, un halogène, un guanidinoalkyle en C₁-C₆, un guanidinoalkyle en C₁-C₆-CH(NH₂)-C(O)-, un guanidinoalkyle en C₁-C₆-CH(NH₂)-C(O)-NH-, un (alkyle en C₁-C₆)₂N-alcényle en C₂-C₆-NH-, un alkyle en C₁-C₆-NH-alcényle en C₂-C₆-NH-, un aminoalcényle en C₂-C₆-NH-, un alkyle en C₁-C₆-imidoyl-NH-alkyle en C₁-C₆ ; un alkyle en C₁-C₆ ou un groupe 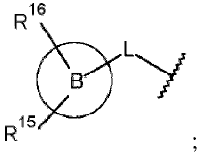 chacun de R³, R⁴ et R⁵ représente indépendamment un hydrogène ou un hydroxy ;
R⁶ représente un hydrogène, un halogène, un alkyle en C₁-C₆, un halogénoalkyle en C₁-C₆, un hydroxyalkyle en C₁-C₆, un alcényle en C₂-C₆, NO₂, CN, un cycloalkyle en C₃-C₁₂, un hydroxyalcoxy en C₁-C₆-alkyle en C₁-C₆, un alcoxy en C₁-C₆ ou un alcoxyalkyle en C₁-C₆ ;
R⁷ etR¹³ représentent chacun indépendamment un hydrogène, un halogène ou un alkyle en C₁-C₆ ;
chacun de R⁸, R⁹, R¹⁰, R¹¹ et R¹² représente indépendamment un hydrogène, un halogène, un alcoxy en C₁-C₆-carbonylalcoxy en C₁-C₆, un amino, un alkyle en C₁-C₆-NH-, un (alkyle en C₁-C₆)₂N-, un hydroxy, un alcoxy en C₁-C₆, un alkyle en C₁-C₆-sulfanyle, un alkyle en C₁-C₆-Sulfonyloxy, un cycloalkyle en C₃-C₁₂-alcoxy en C₁-C₆, un halogénoalcoxy en C₁-C₆, un aryle en C₆-C₁₄-alcoxy en C₁-C₆, un hétéroaryloxy en C₁-C₁₃, un hétéroaryle en C₁-C₁₃-alcoxy en C₁-C₆, un cyanoalcoxy en C₁-C₆, un cycloalkyloxy en C₃-C₁₂, un alcynyloxy en C₂-C₆, un alcoxy en C₁-C₆-alcynyloxy en C₂-C₆, un cyanocycloalkyloxy en C₃-C₁₂, un cyanocycloalkyle en C₃-C₁₂-alcoxy en C₁-C₆, un aminoalcynyloxy en C₂-C₆, un hydroxyalcynyloxy en C₂-C₆, un halogénoalkyle en C₁-C₆, un sulfamoyle, un alkyle en C₁-C₆-sulfamoyle, un alkyle en C₁-C₆, un aminoalcoxy en C₁-C₆-alcynyloxy en C₂-C₆ ou un aminoalcoxy en C₁-C₆ ;
R¹⁴ représente un hydrogène, un halogène ou un alkyle en C₁-C₆ ;
R¹⁵ et R¹⁶ représentent chacun indépendamment un hydrogène, un oxo ou un hydroxy ;
B représente un hétérocycloalkyle en C₂-C₉ ; et
L représente une liaison covalente, un carbonyle, -NH-C(O)- ou -C(O)-NH-.

2. Composé de formule (I) selon la revendication 1, ou un sel pharmaceutiquement acceptable de celui-ci, dans laquelle :
A représente un hétérocycloalkyle en C₂-C₉, un cycloalkyle en C₃-C₁₂ ou un aryle en C₆-C₁₄ ;
R¹ représente un hydrogène ou un alkyle en C₁-C₆ ;
R² représente un hydrogène, un hydroxy, un carboxy, un amino, un aminoalkyle en C₁-C₆, un alkyle en C₁-C₆-C(O)-NH-, un hydroxyalkyle en C₁-C₆, un oxo, un halogène, un guanidinoalkyle en C₁-C₆, un guanidinoalkyle en C₁-C₆-CH(NH₂)-C(O)-, un guanidinoalkyle en C₁-C₆-CH(NH₂)-C(O)-NH-, un (alkyle en C₁-C₆)₂N-alcényle en C₂-C₆-NH-, un alkyle en C₁-C₆-imidoyl-NH-alkyle en C₁-C₆ ; un alkyle en C₁-C₆ ou un groupe 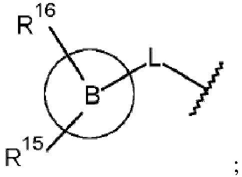 dans lequel :
R¹⁵ représente un oxo ou un hydroxy ;
R¹⁶ représente un hydrogène ou un oxo ;
B représente un hétérocycloalkyle en C₂-C₉ ; et
L représente une liaison covalente, un carbonyle ou -C(O)-NH- ; et
R³, R⁴ et R⁵ représentent tous un hydrogène.

3. Composé de formule (I) selon la revendication 1, ou un sel pharmaceutiquement acceptable de celui-ci, dans laquelle :
A représente un hétérocycloalkyle en C₂-C₉ ou un cycloalkyle en C₃-C₁₂ ;
R¹ représente un hydrogène ;
R² représente un amino, un alkyle en C₁-C₆-imidoyl-NH-alkyle en C₁-C₆ ou un groupe 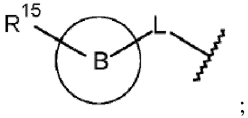 dans lequel :
R¹⁵ représente un hydroxy ;
B représente un hétérocycloalkyle en C₂-C₉ ; et
L représente un carbonyle ou -C(O)-NH- ; et
R³, R⁴ et R⁵ représentent tous un hydrogène.

4. Composé de formule (I) selon la revendication 1, ou un sel pharmaceutiquement acceptable de celui-ci, dans laquelle :
A représente un cyclopropyle, un cyclobutyle, un cyclopentyle ou un 4-pipéridyle ;
R¹ représente un hydrogène ;
R² représente un amino, un (éthanimidoylamino)méthyle ou un groupe 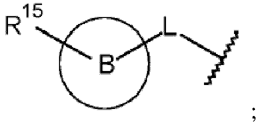 dans lequel :
R¹⁵ représente un hydroxy ;
B représente une pyrrolidine ; et
L représente un carbonyle ou -C(O)-NH- ; et
R³, R⁴ et R⁵ représentent tous un hydrogène.

5. Composé de formule (I) selon l'une quelconque des revendications 1 à 4, ou un sel pharmaceutiquement acceptable de celui-ci, dans laquelle :
R⁶ représente un hydrogène, un halogène ou un alkyle en C₁-C₆ ; et
R⁷, R¹³ et R¹⁴ représentent tous un hydrogène.

6. Composé de formule (I) selon l'une quelconque des revendications 1 à 4, ou un sel pharmaceutiquement acceptable de celui-ci, dans laquelle :
R⁶ représente un alkyle en C₁-C₆ ; et
R⁷, R¹³ et R¹⁴ représentent tous un hydrogène.

7. Composé de formule (I) selon l'une quelconque des revendications 1 à 4, ou un sel pharmaceutiquement acceptable de celui-ci, dans laquelle :
R⁶ représente un éthyle ou un méthyle ; et
R⁷, R¹³ et R¹⁴ représentent tous un hydrogène.

8. Composé de formule (I) selon l'une quelconque des revendications 1 à 7, ou un sel pharmaceutiquement acceptable de celui-ci, dans laquelle :
R⁸ et R⁹ représentent tous deux un hydrogène ou un halogène ;
R¹⁰ représente un alcoxy en C₁-C₆ ou un halogénoalcoxy en C₁-C₆ ; et
R¹¹ et R¹² représentent tous deux un hydrogène.

9. Composé de formule (I) selon l'une quelconque des revendications 1 à 7, ou un sel pharmaceutiquement acceptable de celui-ci, dans laquelle :
R⁸ et R⁹ représentent tous deux un halogène ;
R¹⁰ représente un alcoxy en C₁-C₆ ; et
R¹¹ et R¹² représentent tous deux un hydrogène.

10. Composé de formule (I) selon l'une quelconque des revendications 1 à 7, ou un sel pharmaceutiquement acceptable de celui-ci, dans laquelle :
R⁸ et R⁹ représentent tous deux un fluor ;
R¹⁰ représente un méthoxy ; et
R¹¹ et R¹² représentent tous deux un hydrogène.

11. Composé de formule (I) selon la revendication 1, ou un sel pharmaceutiquement acceptable de celui-ci, dans lequel le composé de formule (I) est choisi parmi :
le N-[2-(aminométhyl)-1,3-dioxan-5-yl]-4-[[3-(4-méthoxyphényl)imidazo[1,2-a]pyrazin-8-yl]amino]-2-méthyl-benzamide ;
le 2-chloro-4-[[3-(3-fluoro-4-méthoxy-phényl)imidazo[1,2-a]pyrazin-8-yl]amino]-N-méthyl-N-(4-pipéridyl)benzamide ;
le 4-[[3-(3-fluoro-4-méthoxy-phényl)imidazo[1,2-a]pyrazin-8-yl]amino]-N,2-diméthyl-N-(4-pipéridyl)benzamide ;
le 4-[[3-[4-(difluorométhoxy)-3-fluoro-phényl]imidazo[1,2-a]pyrazin-8-yl]amino]-N,2-diméthyl-N-(4-pipéridyl)benzamide ;
le 4-[[3-(4-méthoxyphényl)imidazo[1,2-a]pyrazin-8-yl]amino]-2-méthyl-N-(1-méthyl-4-pipéridyl)benzamide ;
le 4-[[3-[4-(difluorométhoxy)phényl]imidazo[1,2-a]pyrazin-8-yl]amino]-2-méthyl-N-quinuclidin-3-yl-benzamide ;
le 4-[[3-(4-méthoxyphényl)imidazo[1,2-a]pyrazin-8-yl]amino]-N,2-diméthyl-N-(1-méthyl-4-pipéridyl)benzamide ;
le 4-[[3-(4-méthoxyphényl)imidazo[1,2-a]pyrazin-8-yl]amino]-N-méthyl-N-(1-méthyl-4-pipéridyl)benzamide ;
le 4-[[3-[4-(difluorométhoxy)phényl]imidazo[1,2-a]pyrazin-8-yl]amino]-2-méthyl-N-[(2S,3S,4R,5S,6R)-2,4,5-trihydroxy-6-(hydroxyméthyl)tétrahydropyran-3-yl]benzamide ;
le N-[1-(1,1-dioxothian-4-yl)-4-pipéridyl]-4-[[3-(3-fluoro-4-méthoxy-phényl)imidazo[1,2-a]pyrazin-8-yl]amino]-2-méthyl-benzamide ;
l'acide 4-[[4-[[3-(3-fluoro-4-méthoxy-phényl)imidazo[1,2-a]pyrazin-8-yl]amino]-2-méthyl-benzoyl]amino]benzoïque ;
le 4-[[3-(3-fluoro-4-méthoxy-phényl)imidazo[1,2-a]pyrazin-8-yl]amino]-N-[(3R,4R)-4-hydroxypyrrolidin-3-yl]-N,2-diméthyl-benzamide ;
le 4-[[3-(3-fluoro-4-méthoxy-phényl)imidazo[1,2-a]pyrazin-8-yl]amino]-N-[(3R,4S)-3-fluoro-4-pipéridyl]-2-méthyl-benzamide ;
le 4-[[3-(2,3-difluoro-4-méthoxy-phényl)imidazo[1,2-a]pyrazin-8-yl]amino]-2-éthyl-N-[3-(hydroxyméthyl)oxétan-3-yl]benzamide ;
le 4-[[3-(2,3-difluoro-4-méthoxy-phényl)imidazo[1,2-a]pyrazin-8-yl]amino]-2-éthyl-N-[(3R)-pyrrolidin-3-yl]benzamide ;
le 4-[[3-(2,3-difluoro-4-méthoxy-phényl)imidazo[1,2-a]pyrazin-8-yl]amino]-2-éthyl-N-[(3S)-pyrrolidin-3-yl]benzamide ;
le N-[1-(aminométhyl)cyclopropyl]-4-[[3-[4-(difluorométhoxy)-2,3-difluoro-phényl]imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-[(3S)-2-oxopyrrolidin-3-yl]benzamide ;
le N-(azétidin-3-yl)-4-[[3-(2,3-difluoro-4-méthoxy-phényl)imidazo[1,2-a]pyrazin-8-yl]amino]-2-éthyl-benzamide ;
le N-[(1S,2S)-2-aminocyclopentyl]-4-[[3-(2,3-difluoro-4-méthoxy-phényl)imidazo[1,2-a]pyrazin-8-yl]amino]-2-éthyl-benzamide ;
le N-[1-(aminométhyl)cyclopropyl]-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-(4-pipéridyl)benzamide ;
le N-(4-aminocyclohexyl)-4-[[3-(2,3-difluoro-4-méthoxy-phényl)imidazo[1,2-a]pyrazin-8-yl]amino]-2-éthyl-benzamide ;
le N-(3-aminocyclobutyl)-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-[rac-(1R,2S)-2-aminocyclohexyl]benzamide ;
le N-[(1S,2R)-2-aminocyclopentyl]-4-[[3-(2,3-difluoro-4-méthoxy-phényl)imidazo[1,2-a]pyrazin-8-yl]amino]-2-éthyl-benzamide ;
le N-[4-[[(2S)-2-amino-5-guanidino-pentanoyl]amino]cyclohexyl]-4-[[3-(2,3-difluoro-4-méthoxy-phényl)imidazo[1,2-a]pyrazin-8-yl]amino]-2-éthyl-benzamide ;
le N-(4-aminocyclohexyl)-4-[[3-(2,3-difluoro-4-méthoxy-phényl)imidazo[1,2-a]pyrazin-8-yl]amino]-2-méthyl-benzamide ;
le 4-[[3-(2,3-difluoro-4-méthoxy-phényl)imidazo[1,2-a]pyrazin-8-yl]amino]-N-[1-[(2S,4R)-4-hydroxypyrrolidine-2-carbonyl]-4-pipéridyl]-2-méthyl-benzamide ;
le 4-[[3-(2,3-difluoro-4-méthoxy-phényl)imidazo[1,2-a]pyrazin-8-yl]amino]-N-[4-[[1-(diméthylamino)-2-méthyl-prop-1-ényl]amino]cyclohexyl]-2-méthyl-benzamide ;
le 4-[[3-(2,3-difluoro-4-méthoxy-phényl)imidazo[1,2-a]pyrazin-8-yl]amino]-2-éthyl-N-[1-(guanidinométhyl)cyclopropyllbenzamide ;
le (2S,4R)-N-[4-[[4-[[3-(2,3-difluoro-4-méthoxy-phényl)imidazo[1,2-a]pyrazin-8-yl]amino]-2-méthyl-benzoyl]amino]cyclohexyl]-4-hydroxy-pyrrolidine-2-carboxamide ;
le N-(3-aminocyclobutyl)-4-[[3-(2,3-difluoro-4-méthoxy-phényl)imidazo[1,2-a]pyrazin-8-yl]amino]-2-méthyl-benzamide ;
le rac-(2S,4R)-N-[3-[[4-[[3-(2,3-difluoro-4-méthoxy-phényl)imidazo[1,2-a]pyrazin-8-yl]amino]-2-méthyl-benzoyl]amino]cyclopentyl]-4-hydroxy-pyrrolidine-2-carboxamide ;
le N-[3-[[(2S)-2-amino-5-guanidino-pentanoyl]amino]cyclobutyl]-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-N-[1-[(éthanimidoylamino)méthyl]cyclopropyl]-2-éthyl-benzamide ;
le N-(2-aminocyclobutyl)-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-méthyl-N-[(3R)-pyrrolidin-3-yl]benzamide ;
le 4-[[3-(2,3-difluoro-4-méthoxy-phényl)imidazo[1,2-a]pyrazin-8-yl]amino]-2-éthyl-N-[rac-(1R,2S)-2-aminocyclopropyl]benzamide ;
le rac-(2S,4R)-N-[2-[[4-[[3-(2,3-difluoro-4-méthoxy-phényl)imidazo[1,2-a]pyrazin-8-yl]amino]-2-éthyl-benzoyl]amino]cyclopropyl]-4-hydroxy-pyrrolidine-2-carboxamide ;
le 4-[[3-(2,3-difluoro-4-méthoxy-phényl)imidazo[1,2-a]pyrazin-8-yl]amino]-2-éthyl-N-[rac-(1S,5R)-3-azabicyclo[3.1.0]hexan-6-yl]benzamide ;
le N-[(1R,2S)-2-aminocyclopentyl]-4-[[3-(2,3-difluoro-4-méthoxy-phényl)imidazo[1,2-a]pyrazin-8-yl]amino]-2-éthyl-benzamide ;
le (2S,4R)-N-[3-[[4-[[3-(2,3-difluoro-4-méthoxy-phényl)imidazo[1,2-a]pyrazin-8-yl]amino]-2-éthyl-benzoyl]amino]-1-bicyclo[1.1.1]pentanyl]-4-hydroxy-pyrrolidine-2-carboxamide ;
le rac-(2S,4R)-N-[2-[[4-[[3-(2,3-difluoro-4-méthoxy-phényl)imidazo[1,2-a]pyrazin-8-yl]amino]-2-éthyl-benzoyl]amino]cyclobutyl]-4-hydroxy-pyrrolidine-2-carboxamide ;
le rac-(2S,4R)-N-[2-[[4-[[3-(2,3-difluoro-4-méthoxy-phényl)imidazo[1,2-a]pyrazin-8-yl]amino]-2-éthyl-benzoyl]amino]cyclopropyl]-4-hydroxy-pyrrolidine-2-carboxamide ;
le (2S,4R)-N-[3-[[4-[[3-(2,3-difluoro-4-méthoxy-phényl)imidazo[1,2-a]pyrazin-8-yl]amino]-2-méthyl-benzoyl]amino]cyclobutyl]-4-hydroxy-pyrrolidine-2-carboxamide ;
le (2S,4R)-N-[3-[[4-[[3-(2,3-difluoro-4-méthoxy-phényl)imidazo[1,2-a]pyrazin-8-yl]amino]-2-méthyl-benzoyl]amino]cyclobutyl]-4-hydroxy-pyrrolidine-2-carboxamide ;
le 4-[[3-(2,3-difluoro-4-méthoxy-phényl)imidazo[1,2-a]pyrazin-8-yl]amino]-N-[1-[(2S,4R)-4-hydroxypyrrolidine-2-carbonyl]azétidin-3-yl]-2-méthyl-benzamide ;
le 4-[[3-(2,3-difluoro-4-méthoxy-phényl)imidazo[1,2-a]pyrazin-8-yl]amino]-N-[(3S)-1-[(2S,4R)-4-hydroxypyrrolidine-2-carbonyl]pyrrolidin-3-yl]-2-méthyl-benzamide ;
le 4-[[3-(2,3-difluoro-4-méthoxy-phényl)imidazo[1,2-a]pyrazin-8-yl]amino]-N-[(3R)-1-[(2S,4R)-4-hydroxypyrrolidine-2-carbonyl]pyrrolidin-3-yl]-2-méthyl-benzamide ;
le (2S,4R)-N-[4-[[4-[[3-(2,3-difluoro-4-méthoxy-phényl)imidazo[1,2-a]pyrazin-8-yl]amino]-2-méthyl-benzoyl]amino]cyclohexyl]-4-hydroxy-pyrrolidine-2-carboxamide ;
le N-(3-amino-1-bicyclo[1.1.1]pentanyl)-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-méthyl-N- [4-(2-méthylpropanoylamino)cyclohexyl]benzamide ;
le 4-[[3-(2,3-difluoro-4-méthoxy-phényl)imidazo[1,2-a]pyrazin-8-yl]amino]-2-méthyl-N-[1-[rac-(2S,4R)-4-hydroxypyrrolidine-2-carbonyl]-3-pipéridyl]benzamide ;
le 4-[[3-(2,3-difluoro-4-méthoxy-phényl)imidazo[1,2-a]pyrazin-8-yl]amino]-2-éthyl-N-[rac-(lR,2R)-2-aminocyclopropyl]benzamide ;
le N-(3-aminocyclobutyl)-4-[[3-(2,3-difluoro-4-méthoxy-phényl)imidazo[1,2-a]pyrazin-8-yl]amino]-2-méthyl-benzamide ;
le N-(azétidin-3-yl)-4-[[3-(2,3-difluoro-4-méthoxy-phényl)imidazo[1,2-a]pyrazin-8-yl]amino]-2-méthyl-benzamide;
le 4-[[3-(2,3-difluoro-4-méthoxy-phényl)imidazo[1,2-a]pyrazin-8-yl]amino]-2-méthyl-N-[(3S)-pyrrolidin-3-yl]benzamide ;
le 4-[[3-(2,3-difluoro-4-méthoxy-phényl)imidazo[1,2-a]pyrazin-8-yl]amino]-2-méthyl-N-[rac-(1S,3S)-3-aminocyclopentyl]benzamide ;
le (2S,4R)-N-[(1R,2S)-2-[[4-[[3-(2,3-difluoro-4-méthoxy-phényl)imidazo[1,2-a]pyrazin-8-yl]amino]-2-éthyl-benzoyl]amino]cyclopentyl]-4-hydroxy-pyrrolidine-2-carboxamide ;
le N-(4-aminocyclohexyl)-4-[[3-(2,3-difluoro-4-méthoxy-phényl)imidazo[1,2-a]pyrazin-8-yl]amino]-2-méthyl-benzamide ;
le 4-[[3-(2,3-difluoro-4-méthoxy-phényl)imidazo[1,2-a]pyrazin-8-yl]amino]-2-méthyl-N-(3-pipéridyl)benzamide ;
le 4-[[3-(2,3-difluoro-4-méthoxy-phényl)imidazo[l,2-a]pyrazin-8-yl]amino]-2-méthyl-N-(4-pipéridyl)benzamide ;
le (2S,4R)-N-[2-[[4-[[3-(2,3-difluoro-4-méthoxy-phényl)imidazo[1,2-a]pyrazin-8-yl]amino]-2-éthyl-benzoyl]amino]cyclohexyl]-4-hydroxy-pyrrolidine-2-carboxamide ;
le 4-[[3-(2,3-difluoro-4-méthoxy-phényl)imidazo[1,2-a]pyrazin-8-yl]amino]-2-éthyl-N-[1-[rac-(2S)-2-amino-5-guanidino-pentanoyl]-3-pipéridyl]benzamide ;
le N-[(3R)-1-[(2S)-2-amino-5-guanidino-pentanoyl]pyrrolidin-3-yl]-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-[3-[[rac-(2S)-2-amino-5-guanidino-pentanoyl]amino]cyclopentyl]benzamide ;
le N-[4-[[(2S)-2-amino-5-guanidino-pentanoyl]amino]cyclohexyl]-4-[[3-(2,3-difluoro-4-méthoxy-phényl)imidazo[1,2-a]pyrazin-8-yl]amino]-2-éthyl-benzamide ;
le N- [1-[(2S)-2-amino-5 -guanidino-pentanoyl] -4-pipéridyl] -4- [[3 -(2,3 -difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-[rac-(1S,3S)-3-aminocyclopentyl]benzamide ;
le N-(4-aminocyclohexyl)-4-[[3-(2,3-difluoro-4-méthoxy-phényl)imidazo[1,2-a]pyrazin-8-yl]amino]-2-éthyl-benzamide ;
le 4-[[3-(2,3-difluoro-4-méthoxy-phényl)imidazo[1,2-a]pyrazin-8-yl]amino]-2-éthyl-N-(3-pipéridyl)benzamide ; et
le N-[3-(aminométhyl)oxétan-3-yl]-4-[[3-(2,3-difluoro-4-méthoxy-phényl)imidazo[1,2-a]pyrazin-8-yl]amino]-2-éthyl-benzamide.

12. Procédé de fabrication des composés de formule (I) selon l'une quelconque des revendications 1 à 11, comprenant :
(i) la réaction d'un acide carboxylique **IVa,** dans lequel R⁶ à R¹⁴ sont tels que définis dans l'une quelconque des revendications 1 à 11, 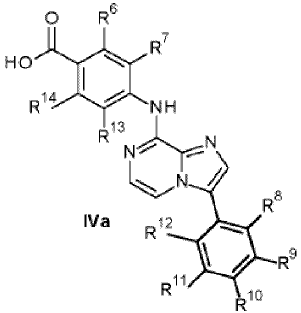 avec une amine **V,** dans laquelle A, L et R¹ à R⁵ sont tels que définis dans l'une quelconque des revendications 1 à 11, 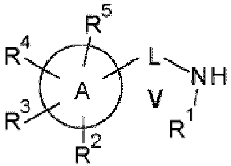 en présence d'un réactif de couplage (tel que HATU, TBTU et similaires) et d'une base (telle que DIPEA, NEt₃ et similaires), pour former ledit composé de formule (I) ; ou
(ii) la réaction d'un composé **VI,** dans lequel R¹ à R⁷, R¹³, R¹⁴, L et A sont tels que définis dans l'une quelconque des revendications 1 à 11 et X représente un halogène, 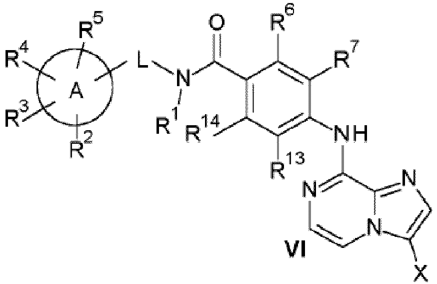 avec un acide boronique **VII,** dans lequel R⁸ à R¹² sont tels que définis dans l'une quelconque des revendications 1 à 11 et Y représente un acide boronique ou un ester d'acide boronique, 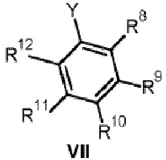 en présence d'un catalyseur à base de métal de transition (tel que le produit d'addition PdCl₂(dppf)-CH₂Cl₂, Pd(PPh₃)₄ et similaires) et d'une base (telle que K₃PO₄, NaOtBu et similaires)
pour former ledit composé de formule (I).

13. Composé de formule (I) selon l'une quelconque des revendications 1 à 11, ou un sel pharmaceutiquement acceptable de celui-ci, pour une utilisation comme substance thérapeutiquement active.

14. Composition pharmaceutique comprenant un composé de formule (I) selon l'une quelconque des revendications 1 à 11, ou un sel pharmaceutiquement acceptable de celui-ci, et un véhicule thérapeutiquement inerte.

15. Composé de formule (I) selon l'une des revendications 1 à 11, ou un sel pharmaceutiquement acceptable de celui-ci, pour une utilisation comme antibiotique.
